# EUROPEAN PATENT APPLICATION

(11) **EP 4 035 684 A1**
(43) Date of publication of application: **03.08.2022**
(21) Application number: 20209541.0
(22) Date of filing: 24.11.2020
(51) Int. Cl.: A61K 47/55, A61K 47/54, A61K 47/64, A61K 47/65, A61K 47/68, A61K 31/122, C07C 49/737, A61P 35/00

(54) **AFFINITY ILLUDOFULVENE CONJUGATES**

(30) Priority: 25.11.2019 US 201962940096 P; 20.11.2020 US 202063116772 P
(71) Applicant: AF Chemical LLC, San Diego, CA 92121 (US)
(72) Inventor: Kelner, Michael, La Jolla, CA92037 (US)
(74) Representative: Schiweck Weinzierl Koch Patentanwälte Partnerschaft mbB

(57) **Abstract**

In an embodiment of the invention, a composition for treating a cell population comprises a medicant. The medicant moiety can be an illudofulvene analog. In an embodiment of the invention, a composition for treating a cell population comprises an Affinity Medicant Conjugate (AMC). The affinity moiety can be an antibody, an antibody fragment, a receptor protein, a peptidic growth factor, an anti-angiogenic protein, a specific binding peptide, protease cleavable peptide, a glycopeptide, a peptide, a peptidic toxin, a protein toxin and an oligonucleotide. The affinity moiety can be covalently bound to the medicant via a linker.

SEQUENCE LISTING

<110>  AF Chemicals, LLC
       Kelner, Michael J.

<120>  AFFINITY ILLUDOFULVENE CONJUGATES

<130>  MKEL-01049US2

<150>  US 62/940096
<151>  2019-11-25

<150>  US 63/116772
<151>  2020-11-20

<160>  926

<170>  PatentIn version 3.5

<210>  1
<211>  1962
<212>  DNA
<213>  Homo sapiens

<400>  1
gctgttccag tcccgcccct gagctctgtc atggcgacgt ccagtgctcc agccggtgtg      60

aggacacgat atgctggggt ttttgtccgc acgccaaacg ggtttggagg accctcttcg     120

ccttcggaga gcagagtcaa cacggagagt tttgggactg gaattaaata aagacagaga     180

tgttgaaaga atccacggcg gtggaattaa cacccttgac attgaacctg ttgaagggag     240

atacatgtta tcaggtggtt cagatggtgt gattgtactt tatgaccttg agaactccag     300

cagacaatct tattacacat gtaaagcagt gtgttccatt ggcagagatc atcctgatgt     360

tcacagatac agtgtggaga ctgtacagtg gtatcctcat gacactggca tgttcacatc     420

aagctcattt gataaaactc tgaaagtatg ggatacaaat acattacaat tggtactaga     480

ggacccaaag tacaactttg tgacttgaag tctggatcct gttctcacat tctacagggt     540

cacagacaag aaatattagc agtttcctgg tctccacgtt atgactatat cttggcaaca     600

gcaagtgctg acagtagagt aaaattatgg gatgtgagaa gagcatcagg atgtttgatt     660

actcttgatc aacataatgg gaaaaagtca caagctgttg aatcagcaaa cactgctcat     720

aatgggaaag ttaatggctt atgttttaca agtgatggac ttcacctcct cactgttggt     780

acagataatc gaatgaggct ctggaatagt tccaatggag aaaacacact tgtgaactat     840

ggaaaagttt gtaataacag taaaaaagga ttgaaattca ctgtctcctg tggctgcagt     900

tcagaatttg tttttgtacc atatggtagc accattgctg tttatacagt ttactcagga     960

gaacagataa ctatgcttaa gggacattat aaaactgttg actgctgtgt atttcagtca    1020

aatttccagg aactttatag tggtagcaga gactgcaaca ttctggcttg ggttccatcc    1080

ttatatgaac cagttcctga tgatgatgag actacaacaa aatcacaatt aaatccggcc    1140

tttgaagatg cctggagcag cagtgatgaa gaaggatgaa tatcatcttt agtacctttt 1200

tgtctctgct gaaacttttt aaatgagact gtgttttttt caactgtatg gtctattcct 1260

gacagctaaa ttagccctaa atgtgggtaa tatttttcct catgttttaa aatgaggtta 1320

atatttgcat aaaatcctaa aacagacttc tgtatagttt atttagtcaa aatgtgttcc 1380

ttgatcccag atgttgtggc ctgggaaagc cctcattgct acagtacaag taacacaagt 1440

cgttgtacct cagttgtgac cttcagcaga ttttatgaac tataagatgc agtctcagag 1500

gatcagcaag tggaggccat cagtattgac tttctcttac ttgctgtact atcagcctgc 1560

tcgtttccac ctttaagaat gattttgcca agaatgatta tatcaaaaat agtagttgaa 1620

atggtaacat caaaattatt ttattctttc ttcttcatgt attcacattt ttcagtggtt 1680

tcatttaatt aaccatgctt tatgttaaac attttggggc tcaatgtctc ctactatcca 1740

aaatgtgcat cacaggaggc ttttaacttt gtgaaaatcc catgtttgct ttattttatt 1800

ttaatgtcag aaggcagttt gcgctaatgc ttgaactctt tttctgtgaa actcattaag 1860

gtatgaccaa atcctgcctc attaattcaa gcagaaaata tcctggcagg gaatctggct 1920

taaacatgaa atgctgtaat aaaatttcta tgttattgtc tc 1962

<210> 2
<211> 396
<212> PRT
<213> Homo sapiens

<400> 2

<210>  3
<211>  3590
<212>  DNA
<213>  Homo sapiens

<400>  3
gcgccacggg cctccccgca ccagcagaag tcggagtcgc tgttgggggc ggtgtctatg      60

gttgagctga gggcgcaggc gccacggccc gtcgagctgg gttccaaggc ggctggcggc     120

ggtagcgtct ctgtttcctt gtgggcgctc gcgcggccct gggtagtctg tagagaatgc     180

caaatgaggg aatcccccac tcaagtcaaa ctcaggagca agactgttta cagagtcaac     240

ctgtcagtaa taatgaagaa atggcaatca agcaagaaag tggtggtgat ggggaggtgg     300

aggagtacct ctcctttcgt tctgtgggtg acgggctgtc cacctctgct gtggggtgcg     360

catcagcagc tccgaggaga gggccagccc tgctgcacat cgaccgacat cagatccagg     420

cagtagagcc tagcgcccag gcccttgagc tgcagggttt gggtgtggac gtctatgacc     480

aggacgtgct ggaacaggga gtgcttcagc aggtggacaa tgccatccat gaggccagcc     540

gtgcctccca gctcgttgac gtggagaagg agtatcggtc ggtcctggat gacctcacgt     600

catgtacgac atccctaagg caaatcaata aaattattga acagcttagc cctcaagctg     660

ccaccagcag agacatcaac aggaaactag attctgtaaa acgacagaag tataataagg     720

aacaacagct aaaaaagatc actgcaaaac aaaagcatct ccaggccatc cttggaggag     780

cagaggtgaa aattgaacta gatcacgcca gtctggagga ggatgcagag ccggggccat     840

ccagtcttgg cagcatgctc atgcctgtcc aggagactgc ctgggaagag ctcatccgca     900

ctggccagat gacacctttt ggtacccaga tccctcagaa acaggagaaa aagcccagaa     960

aaatcatgct taatgaagca tcaggcttcg aaaagtattt ggcagatcaa gcaaaactgt    1020

cttttgaaag gaagaagcaa ggttgtaata aaagagcagc tagaaaagct ccagccccag    1080

tcacgcctcc agccccagtg caaaataaaa acaaaccaaa caagaaagcc agagttctgt    1140

ccaaaaaaga ggagcgtttg aaaaagcaca tcaagaaact ccagaagagg gctttgcagt    1200

tccaggggaa agtgggattg ccaaaggcaa ggagaccttg ggagtcagac atgaggccag    1260

aggcagaggg agactctgag ggtgaagagt ctgagtattt ccccacagag gaggaggaag    1320

aggaggaaga tgacgaggtg gagggggcag aggcggacct gtctggagat ggtactgact    1380

atgagctgaa gcctctgccc aagggcggga aacggcagaa gaaagtgcca gtgcaggaga    1440

ttgatgatga ctttttccca agttctgggg aagaagctga agctgcttct gtaggagaag 1500

gaggaggagg aggtcggaaa gtgggaagat accgagatga tggagatgaa gattattata 1560

agcagcggtt aagtcccaag atgcctcgaa cactaagttt acatgaaata actgaccttt 1620

tagagacaga tgacagcata gaagcaagtg ctatagtgat acaaccacct gaaaatgcta 1680

cagcacctgt ttctgatgag gaatcaggag atgaagaagg tggaacaata aataatctgc 1740

caggttcttt gttgcacaca gctgcgtatc ttattcaaga tggctctgat gctgagtctg 1800

actcagatga tccctcatac gcacctaaag atgactctcc tgatgaagtt ccatctacgt 1860

ttactgtgca gcaacctcca ccatcaagga ggaggaaaat gacaaaaatt ctttgcaaat 1920

ggaaaaaagc cgacctaact gtacaacccg tagcaggtag agttacagca ccaccaaacg 1980

atttcttcac cgtaatgaga actcccacag aaattcttga actttttctt gatgacgagg 2040

tcattgaact cattgtcaag tactccaact tatatgcttg cagtaaaggt gtacatcttg 2100

gcttgactag ctctgaattc aaatgttttc tgggaattat ttttctgagt ggttatgtct 2160

cagttcctag aaggcgtatg ttttgggaac aaagaacaga tgtgcataat gtactggtta 2220

gtgctgccat gagacgtgac cggtttgaaa ctatattttc taatttgcat gttgctgaca 2280

atgcaaattt ggatccagtg gacaaatttt ccaaattgcg acctctcata agcaaactta 2340

atgagagatg catgaaattt gttccaaatg aaacatattt cagctttgat gaattcatgg 2400

ttccttattt tggtcgtcac gggtgcaaac aatttattcg gggaaagccc attcggtttg 2460

gctataagtt ttggtgtggt gccacctgtc tgggctacat ttgctggttt cagccgtatc 2520

agggtaaaaa cccaaatact aaacatgagg aatatggtgt cggtgcgtca cttgtccttc 2580

agtttagtga ggcacttaca gaggcacacc ctggacaata ccattttgta ttcaataact 2640

ttttcaccag tattgcactt cttgataagc tcagttcaat gggacatcag gcaacaggta 2700

cagtgagaaa ggatcacatt gacagagttc cactggaatc agatgtagct ttaaagaaaa 2760

aagaaagagg cacatttgat tatcgaattg atggcaaagg caatattgtc tgcagatgga 2820

atgataacag tgttgtcact gttgcctcat ctggtgctgg tatccatccc ctgtgtcttg 2880

tcagtcgtta ctcccagaaa ctgaaaaaga agatacaagt tcagcagcca aacatgatca 2940

aagtgtataa ccagttcatg ggaggcgtag acagagctga tgaaaacatt gataagtatc 3000

gggcatcaat ccgtggaaag aaatggtatt caagccctct tttgttctgt ttcgaactgg 3060

tcttacaaaa tgcttggcaa ttgcataaaa catatgatga gaaaccagtg gattttctgg 3120

agtttcgtcg acgtgtggta tgccattatc tggagaccca tggtcatcct ccagaacctg 3180

gccaaaaagg aagacctcag aagcgtaaca ttgactcacg ttatgatggc ataaatcatg 3240

tgatagtcaa acagggaaag caaacgcgat gcgctgaatg tcataagaac acaacttttc 3300

gatgtgaaaa atgtgatgtt gccttacatg tgaagtgttc cgttgaatat cacactgaat 3360

agcaggtgtc accacctcct gagataagaa acatagtttt atacattatg tacagtgtag 3420

cagtggtttt gcctagtgtt ccaattttgg aacgtcacat aacaatggaa cataataaat 3480

ttttttttct cttcaaattt ttgttccttg aatttttcta ggtaacatat atgaatttca 3540

tgcaaaaatt caaaaaattg taacctcaga cataaatggg ttaaagatgt 3590

<210> 4
<211> 1493
<212> PRT
<213> Homo sapiens

<400> 4

<210> 5
<211> 3515
<212> DNA
<213> Homo sapiens

<400> 5
ctccttccgc gtggtccctc cccctcaggc cgcggtcgcg attacgctct ctacggcctg 60

cgaccgcagg gccgttgcgg gctggagaca cggcgccgac tggaaccgga ggagctctag 120

gccaaatggt tgggccagcc aggatcccag gacccttcgc cgctcgagac cggagagagg 180

aaacgaaaca ggcgggaacc cgtgggggag ggagggaact agcggaaggt gtcatggcgg 240

ccgcgctctt gagtcacgtg cccagggccc gccttgctac ttccggtcac gtgccctcag 300

actcctcgca gccagcgatg gaggcgagac cccctagtaa cagaggcggt ggctactgct 360

gcggccactg ggtttcggcc tcttcccagc agcggctcta agaagcgcag cggaactcga 420

ccggatccaa cccagttagt tacttcctgt ctagagttgt agcttccacc tgagttctcc 480

tgggaagaag tgtttgccac tcaaggaata cactcaagaa aaacctggaa atcacagaag 540

gcattgtaag atagctacag tgattctgtt aaacctcctg aaattggtcc ttgaacaatt 600

aagaggtgct acagatgacc aggaaaattt accaggcaaa ttagctgtga gacaattgac 660

catttgtgcc caatgttcgc accttctagc caccatggca acctcatctg aagaagtttt 720

gctgattgta aagaaagtgc gtcaaaagaa gcaggatgga gctctgtacc tcatggcaga 780

aagaattgct tgggcacctg aaggcaaaga tagatttaca atcagccata tgtatgcaga 840

tattaaatgc cagaaaatta gtccagaagg aaaagctaaa attcagcttc agctggtcct 900

acatgcaggg gacacaacta acttccattt ttccaatgaa agcacagcag tgaaagagcg 960

agatgcagta aaagaccttc ttcagcagct gctgcccaaa ttcaagagga aagcaaataa 1020

agaactggaa gagaagaaca gaatgctgca agaagatcct gttttgtttc agctttataa 1080

agaccttgtt gtgagtcaag tgatcagtgc tgaggaattc tgggccaatc gtttaaatgt 1140

gaatgcaaca gatagttctt ccacatccaa tcataagcag gatgttggca tttctgctgc 1200

atttctggct gatgtccggc cccaaactga tggctgtaac ggtctaagat ataatttaac 1260

ttctgatatc attgagtcca tatttaggac ctatccagca gtaaaaatga aatatgcaga 1320

aaatgttccc cacaacatga cagagaagga attctggaca cgttttttcc agtcccatta 1380

ttttcacagg gatcggctga atacagggtc aaaggatctc tttgcagaat gtgccaaaat 1440

agatgaaaaa ggcctaaaaa caatggtttc attaggagtg aaaaacccac tactagattt 1500

aacagctttg gaagataaac cattagatga gggctatggc atttcctctg tgccatctgc 1560

ttccaattct aaatccataa aagagaatag taatgctgcc atcatcaaga gatttaacca 1620

tcacagtgcc atggtcctgg cagctggact cagaaaacaa gaagcacaaa atgaacaaac 1680

tagtgagccc agcaacatgg atggaaattc cggagatgca gactgctttc agccagcagt 1740

caaaagggcg aaattacaag agtccattga atatgaagac ttggggaaaa ataattctgt 1800

aaaaacgatt gcactaaacc tcaagaagtc agataggtat tatcatggtc caactccaat 1860

ccagtcacta cagtatgcaa caagtcagga cattattaat tcttttcaaa gtattagaca 1920

agaaatggaa gcttatacac ccaagttaac tcaggttctc tcaagtagtg ctgccagtag 1980

taccatcaca gcactgtcac ctggaggggc acttatgcag ggaggaacac agcaagccat 2040

aaaccagatg gtgccaaatg atattcaatc tgaattgaaa cacttatatg tagctgttgg 2100

agaacttcta cgacatttct ggtcctgctt tcctgttaat acgccattcc tagaagaaaa 2160

ggtagtgaaa atgaaaagta atttggaacg attccaagtt acgaagctct gtccattcca 2220

agaaaagatt cggagacagt atttaagcac aaatttggta agtcacatag aagagatgct 2280

ccagacagcc tacaacaagc tccacacatg gcagtcacgg cgtctgatga agaaaacgtg 2340

aggtggccat gatgcttaca ggttttgtga gattgagaga actatgacct gcagcaactc 2400

tggaaacctg gcctgacaga caagcagatg acctcacagg agtgataaga aacatctgct 2460

ccacgccaac tcccagagct gatgctattg tacttgcaca ttggagactg aaaggaaaga 2520

agggactaaa tgctggggag gtaaattaag acagaaccaa atgagctaag ttgcaaatat 2580

atatatatac acacacacac atatatgtac atgtgtatgt acatatatat tttaaaagac 2640

tgtttactgc agttgctcag gaactgcttt tgattcacat taagctgctt tcagaaatta 2700

aaaaaacact ttttaaaggg tgcattgata aaatctgagg ttttttggtt gtcgtttttt 2760

tctgtgtaca tttttttcct aagtttatgg cacagggtag accttaagta ttcctcctcc 2820

atccttcatt cttcaccctc cattggatcc tcaagtttta atgaattcca attatacctt 2880

acatcagcaa gttaaaaaaa gtactttaaa ataaagcaaa gggagactgt tgctcaacca 2940

tcaggaaaca gttgtcagaa gacatcattg gttctgtgtt tcctacggaa ataagaaacg 3000

ataaatattg cactgaatgt ttgtggtttg gagtccctga ataataaaga gggaatatat 3060

ttgcagaaag tcgcataggg ttttttaatg cagaattttg tcagaagaca atggcgctgc 3120

atgtttttct ttgagtgcaa atgtacattg ctaagatttt tttaagatgg catgtgcttt 3180

gaaaagaaga tattgcattt ttaagagttt aaaaatctta tgagtgagaa atattaaaaa 3240

aatcttattt tcacctcttt agaagaaata aaagatgttt ctcctatctc cttttctcta 3300

gtatttgact gttactgtcc ttggcgaatc gataatcatt gcatagtgac tgaaaagcct 3360

aagtgcaaaa aaaaaaaaaa aagatgttct tgtttctgaa cttcgtgcca tattttgttc 3420

ctgatgggat caacttaatg tttaagactt tagatgtctt gtattaaaaa ttacacaaaa 3480

aaagtaaaac tttttatact taccctttta actct 3515

<210> 6
<211> 548
<212> PRT
<213> Homo sapiens

<400> 6

Met Ala Thr Ser Ser Glu Glu Val Leu Leu Ile Val Lys Lys Val Arg
1               5                   10                  15

Gln Lys Lys Gln Asp Gly Ala Leu Tyr Leu Met Ala Glu Arg Ile Ala
            20                  25                  30

Trp Ala Pro Glu Gly Lys Asp Arg Phe Thr Ile Ser His Met Tyr Ala
        35                  40                  45

Asp Ile Lys Cys Gln Lys Ile Ser Pro Glu Gly Lys Ala Lys Ile Gln
    50                  55                  60

Leu Gln Leu Val Leu His Ala Gly Asp Thr Thr Asn Phe His Phe Ser
65                  70                  75                  80

Asn Glu Ser Thr Ala Val Lys Glu Arg Asp Ala Val Lys Asp Leu Leu
                85                  90                  95

Gln Gln Leu Leu Pro Lys Phe Lys Arg Lys Ala Asn Lys Glu Leu Glu
            100                 105                 110

Glu Lys Asn Arg Met Leu Gln Glu Asp Pro Val Leu Phe Gln Leu Tyr
        115                 120                 125

Lys Asp Leu Val Val Ser Gln Val Ile Ser Ala Glu Glu Phe Trp Ala

130                 135                 140

Asn Arg Leu Asn Val Asn Ala Thr Asp Ser Ser Ser Thr Ser Asn His
145                 150                 155                 160

Lys Gln Asp Val Gly Ile Ser Ala Ala Phe Leu Ala Asp Val Arg Pro
                165                 170                 175

Gln Thr Asp Gly Cys Asn Gly Leu Arg Tyr Asn Leu Thr Ser Asp Ile
            180                 185                 190

Ile Glu Ser Ile Phe Arg Thr Tyr Pro Ala Val Lys Met Lys Tyr Ala
        195                 200                 205

Glu Asn Val Pro His Asn Met Thr Glu Lys Glu Phe Trp Thr Arg Phe
    210                 215                 220

Phe Gln Ser His Tyr Phe His Arg Asp Arg Leu Asn Thr Gly Ser Lys
225                 230                 235                 240

Asp Leu Phe Ala Glu Cys Ala Lys Ile Asp Glu Lys Gly Leu Lys Thr
                245                 250                 255

Met Val Ser Leu Gly Val Lys Asn Pro Leu Leu Asp Leu Thr Ala Leu
            260                 265                 270

Glu Asp Lys Pro Leu Asp Glu Gly Tyr Gly Ile Ser Ser Val Pro Ser
        275                 280                 285

Ala Ser Asn Ser Lys Ser Ile Lys Glu Asn Ser Asn Ala Ala Ile Ile
    290                 295                 300

Lys Arg Phe Asn His His Ser Ala Met Val Leu Ala Ala Gly Leu Arg
305                 310                 315                 320

Lys Gln Glu Ala Gln Asn Glu Gln Thr Ser Glu Pro Ser Asn Met Asp
                325                 330                 335

Gly Asn Ser Gly Asp Ala Asp Cys Phe Gln Pro Ala Val Lys Arg Ala
            340                 345                 350

Lys Leu Gln Glu Ser Ile Glu Tyr Glu Asp Leu Gly Lys Asn Asn Ser
        355                 360                 365

Val Lys Thr Ile Ala Leu Asn Leu Lys Lys Ser Asp Arg Tyr Tyr His
    370                 375                 380

Gly Pro Thr Pro Ile Gln Ser Leu Gln Tyr Ala Thr Ser Gln Asp Ile
385                 390                 395                 400

Ile Asn Ser Phe Gln Ser Ile Arg Gln Glu Met Glu Ala Tyr Thr Pro
                405                 410                 415

Lys Leu Thr Gln Val Leu Ser Ser Ser Ala Ala Ser Ser Thr Ile Thr
            420                 425                 430

Ala Leu Ser Pro Gly Gly Ala Leu Met Gln Gly Gly Thr Gln Gln Ala
        435                 440                 445

Ile Asn Gln Met Val Pro Asn Asp Ile Gln Ser Glu Leu Lys His Leu
    450                 455                 460

Tyr Val Ala Val Gly Glu Leu Leu Arg His Phe Trp Ser Cys Phe Pro
465                 470                 475                 480

Val Asn Thr Pro Phe Leu Glu Glu Lys Val Val Lys Met Lys Ser Asn
                485                 490                 495

Leu Glu Arg Phe Gln Val Thr Lys Leu Cys Pro Phe Gln Glu Lys Ile
            500                 505                 510

Arg Arg Gln Tyr Leu Ser Thr Asn Leu Val Ser His Ile Glu Glu Met
        515                 520                 525

Leu Gln Thr Ala Tyr Asn Lys Leu His Thr Trp Gln Ser Arg Arg Leu
    530                 535                 540

Met Lys Lys Thr
545

<210>  7
<211>  1951
<212>  DNA
<213>  Homo sapiens

<400>  7
ccttctagcg gcgccggtga gtccgcgtgt ggaagtctgt gaggcgcaga ggtggggcag      60

gccgtctggc tagctaggcg gctgggagcg ttttcgtggc ggggaacgga ggttgaattg     120

ccctgcctgg gctcataggg aaggaggatg tgaaggagct tgtgaaggca gaggaagatt     180

attgaataat aaaatacagt tttgaaaaaa atggatgaag aacctgaaag aactaagcga     240

tgggaaggag gctatgaaag aacatggagg attcttaaag aagatgaatc tggatcactt     300

aaagctacaa tagaagacat tctattcaag gcaaagagaa aaagagtatt tgagcaccat     360

ggacaagttc gacttggaat gatgcgccac ctttatgtgg tagtagatgg atcaagaaca     420

atggaagacc aagatttaaa gcctaataga ctgacgtgta ctttaaagtt gttggaatac 480

tttgtagagg aatattttga tcaaaatcct attagtcaga ttggaataat tgtaactaag 540

agtaaaagag ctgaaaaatt gactgaactt tcaggaaacc caagaaaaca tataacgtct 600

ttgaagaaag ctgtggatat gacctgccat ggagagccat ctctttataa ttccctaagc 660

atagctatgc agactctaaa acacatgcct ggacatacaa gtcgagaagt actaatcatc 720

tttagcagcc ttacaacttg cgatccatct aatatttatg atctaatcaa gaccctaaag 780

gcagctaaaa ttagagtatc tgttattgga ttgtctgcag aagttcgcgt ttgcactgta 840

cttgctcgtg aaactggtgg cacgtaccat gttattttag atgaaagcca ttacaaagag 900

ttgctcacac atcatgttag tcctcctcct gctagctcaa gttctgaatg ctcacttatt 960

cgtatgggat ttcctcagca caccattgct tctttatctg accaggatgc aaaaccctct 1020

ttcagcatgg cgcatttgga tggcaatact gagccagggc ttacattagg aggctatttc 1080

tgcccacagt gtcgggcaaa gtactgtgag ctacctgttg agtgtaaaat ctgtggtctt 1140

actttggtgt ctgctcccca cttggcacgg tcttaccatc atttgtttcc tttggatgct 1200

tttcaagaaa ttcccctaga agaatataat ggagaaagat tttgttatgg atgtcagggg 1260

gaattgaaag accaacatgt ttatgtttgt gctgtgtgcc aaaatgtttt ctgtgtggac 1320

tgtgatgttt ttgttcatga ttctctacac tgttgccctg gctgtattca taagattcca 1380

gctccttcag gtgtttgatt ccagcatgta gtatacattg tatgtgttaa aaagaaattt 1440

gcaactgtga ataaaaggac ttctttagaa gaagcttcat ttaaaacatg aaaggataat 1500

ctgacttaag aaactttttg ctaagaaaag gtaatatttt attaaatttt aaatttgtgt 1560

tgtcacagaa atacctgaaa ttcagtagta cttcattcaa ttaattttgt tttctattat 1620

tttgagttat actgttttca aagtcattat gcagtatgta taaacttata agaattaaat 1680

tgatgtgata attttatgtt tttataatta aatatagaat ctttatgatt tatgttaatt 1740

cattaattta gtgtaagaag aaagttaagt ctgaatgtaa attcagtgta agatgaaaat 1800

ttatcaatac ttatgaaatt aggctgggcg ctgtggctca cacctgtaat cccaacactt 1860

tgggaggctg aggtgggcag atcacctgag gtcaggagtt cgagaccagc ctggccaaca 1920

tggtgaaacc ccgtcactac taaaaataca a 1951

<210> 8
<211> 395
<212> PRT
<213> Homo sapiens

<400> 8

Met Asp Glu Glu Pro Glu Arg Thr Lys Arg Trp Glu Gly Gly Tyr Glu
1               5                   10                  15

Arg Thr Trp Glu Ile Leu Lys Glu Asp Glu Ser Gly Ser Leu Lys Ala
            20                  25                  30

Thr Ile Glu Asp Ile Leu Phe Lys Ala Lys Arg Lys Arg Val Phe Glu
        35                  40                  45

His His Gly Gln Val Arg Leu Gly Met Met Arg His Leu Tyr Val Val
    50                  55                  60

Val Asp Gly Ser Arg Thr Met Glu Asp Gln Asp Leu Lys Pro Asn Arg
65                  70                  75                  80

Leu Thr Cys Thr Leu Lys Leu Leu Glu Tyr Phe Val Glu Glu Tyr Phe
                85                  90                  95

Asp Gln Asn Pro Ile Ser Gln Ile Gly Ile Ile Val Thr Lys Ser Lys
            100                 105                 110

Arg Ala Glu Lys Leu Thr Glu Leu Ser Gly Asn Pro Arg Lys His Ile
        115                 120                 125

Thr Ser Leu Lys Lys Ala Val Asp Met Thr Cys His Gly Glu Pro Ser
    130                 135                 140

Leu Tyr Asn Ser Leu Ser Ile Ala Met Gln Thr Leu Lys His Met Pro
145                 150                 155                 160

Gly His Thr Ser Arg Glu Val Leu Ile Ile Phe Ser Ser Leu Thr Thr
                165                 170                 175

Cys Asp Pro Ser Asn Ile Tyr Asp Leu Ile Lys Thr Leu Lys Ala Ala
            180                 185                 190

Lys Ile Arg Val Ser Val Ile Gly Leu Ser Ala Glu Val Arg Val Cys
        195                 200                 205

Thr Val Leu Ala Arg Glu Thr Gly Gly Thr Tyr His Val Ile Leu Asp
    210                 215                 220

Glu Ser His Tyr Lys Glu Leu Leu Thr His His Val Ser Pro Pro Pro
225                 230                 235                 240

Ala Ser Ser Ser Ser Glu Cys Ser Leu Ile Arg Met Gly Phe Pro Gln
                245                 250                 255

His Thr Ile Ala Ser Leu Ser Asp Gln Asp Ala Lys Pro Ser Phe Ser

260                 265                 270

Met Ala His Leu Asp Gly Asn Thr Glu Pro Gly Leu Thr Leu Gly Gly
        275                 280                 285

Tyr Phe Cys Pro Gln Cys Arg Ala Lys Tyr Cys Glu Leu Pro Val Glu
    290                 295                 300

Cys Lys Ile Cys Gly Leu Thr Leu Val Ser Ala Pro His Leu Ala Arg
305                 310                 315                 320

Ser Tyr His His Leu Phe Pro Leu Asp Ala Phe Gln Glu Ile Pro Leu
                325                 330                 335

Glu Glu Tyr Asn Gly Glu Arg Phe Cys Tyr Gly Cys Gln Gly Glu Leu
            340                 345                 350

Lys Asp Gln His Val Tyr Val Cys Ala Val Cys Gln Asn Val Phe Cys
        355                 360                 365

Val Asp Cys Asp Val Phe Val His Asp Ser Leu His Cys Cys Pro Gly
    370                 375                 380

Cys Ile His Lys Ile Pro Ala Pro Ser Gly Val
385                 390                 395

<210>  9
<211>  3434
<212>  DNA
<213>  Homo sapiens

<400>  9
gggggctggc acgagtcaca gcacctcgtg cgtcacttgg cggcgccgga cgttgggctg      60

cgcaggattt gagacgctcc cctgaccacc acttgctctg cgctgaggtg ctgggacagc     120

catggtttca gacgaagatg aattgaatct tctggttatt gtagttgatg ccaacccaat     180

ttggtgggga aagcaagcat taaaggaatc tcagttcact ttatccaaat gcatagatgc     240

cgtgatggtg ctgggaaatt cgcatttatt catgaatcgt tccaacaaac ttgctgtgat     300

agcaagtcac attcaagaaa gccgattctt atatcctgga aagaatggca gacttggaga     360

cttcttcgga gaccctggca accctcctga atttaatccc tctgggagta aagatggaaa     420

atacgaactt ttaacctcag caaatgaagt tattgttgaa gagattaaag atctaatgac     480

caaaagtgac ataaagggtc aacatacaga aactttgctg gcaggatccc tggccaaagc     540

cctttgctac attcatagaa tgaacaagga agttaaagac aatcaggaaa tgaaatcaag     600

gatattggtg attaaggctg cagaagacag tgcgttgcag tatatgaact tcatgaatgt     660

catctttgca gcacagaaac agaatatttt gattgatgcc tgtgttttag actccgactc 720

agggctcctc caacaggctt gtgacatcac gggaggactg tacctgaagg tgcctcagat 780

gccttctctt ctgcagtatt tgctgtgggt gtttcttccc gatcaagatc agagatctca 840

gttaatcctc ccacccccag ttcatgttga ctacagggct gcttgcttct gtcatcgaaa 900

tctcattgaa attggttatg tctgttctgt gtgtttgtca atattctgca atttcagccc 960

catttgtact acgtgcgaga cagcctttaa aatttctctg cctccagtgc tgaaagccaa 1020

gaaaaagaaa ctgaaagtgt ctgcctgagg ataaaatatt ttccccatct tttagagctg 1080

ttaatagaaa ttatatagca gattctttgt tgggaagact gaaaaaaata aagataggta 1140

taggataatt tttaatatgg tgaccttaca gaaaatattt cccaaacatc cttttcatcc 1200

tgtgcttctg gaggactgat ttgtttgagg gaatcattct atgcattata tcctaaaata 1260

ttctatgact ggtttctgtc catgtttgtg gctttcattt ttttaatggg atgactatta 1320

gtcaaagtca gcttgtcatg actcatcata ggctttctaa cctactccct gaatccgggt 1380

cctcattgtg aaatgcatgc catacgaaat ttgaacgtag ctttggaaaa agggactatt 1440

tgtggagtaa tggcattaat caacatagaa catcttattt gaatcaacag ttaacttcag 1500

tagtcatgtg aataaaattc ttattgtcta aattgagaca gcctcagata tttgcagata 1560

tttacttttt gtctgatatc agtacatatt tggacaaagt catctaaata atagtttgtc 1620

accaaataac tacaaaatct cattttaaat gagtaaggag aacgtgtaca gaagcaaatt 1680

ttcttcaaaa tagttgtggg aagagcttat atgtgaaagc ttatgactgg ttttgaggga 1740

gaacttactg gagaaaatgg actctatgtt aagtatggtt ttcagataga attctttcct 1800

tttttaatga ggaaaaaaaa tccacattaa tattgaaact gcacctgtaa tcccagcact 1860

ttgggaggct gaggacagag gattgcttga gcccaggagt tcgagagcag cctgggcagc 1920

aaagtgagac cccatctcta ctaaaaattt aaatgtattt attaaaactg ttctctagaa 1980

gctttggact gaatcccaaa agtgtttata agttcaaaag caaaagtatt tgtaatttca 2040

acaacaaaaa atgtatttct ttatgtaatc ttgaaattat taaaagtcct tttagcttct 2100

agcacatatt tgtacaaaga gtttaaggaa tggtggctgg tttggtttgt tttttaaaaa 2160

tgtttactga cgaggccggg cgtggtggct cacccctgca atcccagcac tttgggaggc 2220

cgaggcaggc agatcacaag gtcaggagtt caagatcagc ctggccagta tggtgaaacc 2280

ctgtctctac taaaaataga aaaattagcc atgcgaagta gcaggtgcct gtagttccag 2340

ctactcggga ggctgaggca agagaattgc ttgaatccag gaggcagagg ttgcagtgag 2400

ccaagatagc gcctctgtac tccagcctgg gtgacagagc gagactctgt atcaaaaaaa 2460

aaaaagatcg ggcacgttgg ctcacgcctg taatcccagc actttgggag gccaaggtgg 2520

gtggatcacg aggtcaggag tgatcaggct gggtttctgt tttgttttgt tttgtgagac 2580

agagtctcgc tctgttgccc aggctggggt gcagtggtgc aagctcggct ctctgcaagc 2640

tctgtctcct gggttcacgc cattctcctg cctcagcctc ccaagtagct gggattacta 2700

ctctactgaa cccatgaggc agaaatttga gaccatcctg cccaacgtgg tgaaaacatg 2760

gtgaaaccct gtctctacta aaaatacaaa aattagctgg gcgtggtggc aggtgcctgt 2820

aatcccagct actcaggaga ctgaggcagg agaattgctt gaacccagga ggtggaggtt 2880

gcagtgagcc aagatcaaga ttgcgccatt gcactccagc ctgggcgaca agaacaaaac 2940

tctgtctcaa aaaaaaaaaa aaaaaaaaag tctgtactga taaaacccat tgtgtacaaa 3000

actttgtatg taaggaagat tttaattttc tctttataca agctgagtca tatttaaata 3060

atttgatgtt ggcttagata atttcagata gattttatat tctggatttg tgtttttgtt 3120

aacaaatata caaagacttt ggtgatcact ttgcaaatat ttgttaatcc ttgagtttga 3180

gaacctgtct tttaaaaata atattttgta tactaattaa gtgtaatgga aatcacaatt 3240

ttaagtctag gaaataaagt attatatata ctttcaaaca agctagcaag gcttttatta 3300

ttactttttt atttgaaata tcttatatat ttggttagtt ctgtttaact tgtttttaac 3360

tgttgccctt atgagttatt ttatataaat ttttacaata aaataatttg attttcaaaa 3420

aaaaaaaaaa aaaa 3434

<210> 10
<211> 308
<212> PRT
<213> Homo sapiens

<400> 10

Met Val Ser Asp Glu Asp Glu Leu Asn Leu Leu Val Ile Val Val Asp
1               5                   10                  15

Ala Asn Pro Ile Trp Trp Gly Lys Gln Ala Leu Lys Glu Ser Gln Phe
            20                  25                  30

Thr Leu Ser Lys Cys Ile Asp Ala Val Met Val Leu Gly Asn Ser His
        35                  40                  45

Leu Phe Met Asn Arg Ser Asn Lys Leu Ala Val Ile Ala Ser His Ile
    50                  55                  60

Gln Glu Ser Arg Phe Leu Tyr Pro Gly Lys Asn Gly Arg Leu Gly Asp
65                  70                  75                  80

Phe Phe Gly Asp Pro Gly Asn Pro Pro Glu Phe Asn Pro Ser Gly Ser
                85                  90                  95

Lys Asp Gly Lys Tyr Glu Leu Leu Thr Ser Ala Asn Glu Val Ile Val
            100                 105                 110

Glu Glu Ile Lys Asp Leu Met Thr Lys Ser Asp Ile Lys Gly Gln His
        115                 120                 125

Thr Glu Thr Leu Leu Ala Gly Ser Leu Ala Lys Ala Leu Cys Tyr Ile
    130                 135                 140

His Arg Met Asn Lys Glu Val Lys Asp Asn Gln Glu Met Lys Ser Arg
145                 150                 155                 160

Ile Leu Val Ile Lys Ala Ala Glu Asp Ser Ala Leu Gln Tyr Met Asn
                165                 170                 175

Phe Met Asn Val Ile Phe Ala Ala Gln Lys Gln Asn Ile Leu Ile Asp
            180                 185                 190

Ala Cys Val Leu Asp Ser Asp Ser Gly Leu Leu Gln Gln Ala Cys Asp
        195                 200                 205

Ile Thr Gly Gly Leu Tyr Leu Lys Val Pro Gln Met Pro Ser Leu Leu
    210                 215                 220

Gln Tyr Leu Leu Trp Val Phe Leu Pro Asp Gln Asp Gln Arg Ser Gln
225                 230                 235                 240

Leu Ile Leu Pro Pro Pro Val His Val Asp Tyr Arg Ala Ala Cys Phe
                245                 250                 255

Cys His Arg Asn Leu Ile Glu Ile Gly Tyr Val Cys Ser Val Cys Leu
            260                 265                 270

Ser Ile Phe Cys Asn Phe Ser Pro Ile Cys Thr Thr Cys Glu Thr Ala
        275                 280                 285

Phe Lys Ile Ser Leu Pro Pro Val Leu Lys Ala Lys Lys Lys Lys Leu
    290                 295                 300

Lys Val Ser Ala
305

<210>  11
<211>  1736
<212>  DNA
<213>  Homo sapiens

<400>  11
cttttccact cctcccctta cctcccttct cttctgaatt ctccattctg ggctcttgcc        60

tgtgaaatct ttctttgctt tccccatctt ttcctcgcat tttttcacca tctttccctc 120

aatctccagg agccaatgcg agactttggc tccgattaag cgacggcccg agactcgggg 180

tgcgcgagga ggatcgacag agtggtgatg gagagcaccc cttcaagggg actgaaccga 240

gtacacctac aatgcaggaa tctgcaggaa ttcttagggg gcctgagccc tggggtattg 300

gaccgattgt atgggcaccc tgccacatgt ctggctgtct tcagggagct cccatccttg 360

gctaagaact gggtgatgcg gatgctcttt ctggagcagc ctttgccaca ggctgctgta 420

gctctgtggg taaagaagga attcagcaag gctcaggagg aaagtacagg gctgctgagc 480

ggcctccgga tctggcacac acagctgctc ccaggcgggc tccagggcct catcctcaac 540

cccattttcc gccagaacct ccgcattgcc cttctgggtg gggggaaggc ctggtctgat 600

gacacaagtc agctgggacc agacaagcat gcccgggacg ttccctccct tgacaagtac 660

gccgaggagc gatgggaggt ggtcttgcac ttcatggtgg gctcccccag tgcagctgtc 720

agccaggact tggctcagct cctcagccag gctgggctca tgaagagtac tgaacctgga 780

gagccgccct gcattacttc cgctggcttc cagttcctgt tgctggacac cccggctcag 840

ctctggtact ttatgttgca gtatttgcag acagcccaga gccggggcat ggacctggta 900

gagattctct ccttcctctt ccagctcagc ttctctactc tgggcaagga ttactctgtg 960

gaaggtatga gtgattctct gttgaacttc ctgcaacatc tgcgtgagtt tgggcttgtt 1020

ttccagagga agaggaaatc tcggcgttac taccccacac gcctggccat caatctctca 1080

tcaggtgtct ctggagctgg gggcactgtg catcagccag gtttcattgt cgtggaaacc 1140

aattaccgac tgtatgccta cacggagtcg gagctgcaga ttgccctcat tgccctcttc 1200

tctgagatgc tctatcggtt ccccaacatg gtggtggcgc aggtgacccg ggagagtgtg 1260

cagcaggcaa tcgccagtgg catcacagcc cagcagataa tccatttcct aaggacaaga 1320

gcccacccag tgatgctcaa acagacacct gtgctgcccc ccaccatcac cgaccagatc 1380

cggctctggg agctggaaag ggacagactc cggttcactg agggtgtcct gtataaccag 1440

ttcctgtcgc aagtggactt tgagctgctg ctggcccacg cgcgggagct gggcgtgctc 1500

gtgttcgaga actcggccaa gcggctcatg gtggtgaccc cggccgggca cagcgacgtc 1560

aagcgctttt ggaagcggca gaaacatagc tcctgagagc gcgggacttg gacacggacc 1620

tcggcgggcg ggactgggcg gggcggggca tcagaactca ggtgtttttt atttacgcgt 1680

cagggctttt cttgtttaat aaagttatga tagctaaaaa aaaaaaaaaa aaaaaa 1736

<210> 12
<211> 462
<212> PRT
<213> Homo sapiens

<400>  12

Met Glu Ser Thr Pro Ser Arg Gly Leu Asn Arg Val His Leu Gln Cys
1               5                   10                  15

Arg Asn Leu Gln Glu Phe Leu Gly Gly Leu Ser Pro Gly Val Leu Asp
            20                  25                  30

Arg Leu Tyr Gly His Pro Ala Thr Cys Leu Ala Val Phe Arg Glu Leu
        35                  40                  45

Pro Ser Leu Ala Lys Asn Trp Val Met Arg Met Leu Phe Leu Glu Gln
    50                  55                  60

Pro Leu Pro Gln Ala Ala Val Ala Leu Trp Val Lys Lys Glu Phe Ser
65                  70                  75                  80

Lys Ala Gln Glu Glu Ser Thr Gly Leu Leu Ser Gly Leu Arg Ile Trp
                85                  90                  95

His Thr Gln Leu Leu Pro Gly Gly Leu Gln Gly Leu Ile Leu Asn Pro
            100                 105                 110

Ile Phe Arg Gln Asn Leu Arg Ile Ala Leu Leu Gly Gly Gly Lys Ala
        115                 120                 125

Trp Ser Asp Asp Thr Ser Gln Leu Gly Pro Asp Lys His Ala Arg Asp
    130                 135                 140

Val Pro Ser Leu Asp Lys Tyr Ala Glu Glu Arg Trp Glu Val Val Leu
145                 150                 155                 160

His Phe Met Val Gly Ser Pro Ser Ala Ala Val Ser Gln Asp Leu Ala
                165                 170                 175

Gln Leu Leu Ser Gln Ala Gly Leu Met Lys Ser Thr Glu Pro Gly Glu
            180                 185                 190

Pro Pro Cys Ile Thr Ser Ala Gly Phe Gln Phe Leu Leu Leu Asp Thr
        195                 200                 205

Pro Ala Gln Leu Trp Tyr Phe Met Leu Gln Tyr Leu Gln Thr Ala Gln
    210                 215                 220

Ser Arg Gly Met Asp Leu Val Glu Ile Leu Ser Phe Leu Phe Gln Leu
225                 230                 235                 240

Ser Phe Ser Thr Leu Gly Lys Asp Tyr Ser Val Glu Gly Met Ser Asp

245                 250                 255

Ser Leu Leu Asn Phe Leu Gln His Leu Arg Glu Phe Gly Leu Val Phe
            260                 265                 270

Gln Arg Lys Arg Lys Ser Arg Arg Tyr Tyr Pro Thr Arg Leu Ala Ile
        275                 280                 285

Asn Leu Ser Ser Gly Val Ser Gly Ala Gly Gly Thr Val His Gln Pro
    290                 295                 300

Gly Phe Ile Val Val Glu Thr Asn Tyr Arg Leu Tyr Ala Tyr Thr Glu
305                 310                 315                 320

Ser Glu Leu Gln Ile Ala Leu Ile Ala Leu Phe Ser Glu Met Leu Tyr
                325                 330                 335

Arg Phe Pro Asn Met Val Val Ala Gln Val Thr Arg Glu Ser Val Gln
            340                 345                 350

Gln Ala Ile Ala Ser Gly Ile Thr Ala Gln Gln Ile Ile His Phe Leu
        355                 360                 365

Arg Thr Arg Ala His Pro Val Met Leu Lys Gln Thr Pro Val Leu Pro
    370                 375                 380

Pro Thr Ile Thr Asp Gln Ile Arg Leu Trp Glu Leu Glu Arg Asp Arg
385                 390                 395                 400

Leu Arg Phe Thr Glu Gly Val Leu Tyr Asn Gln Phe Leu Ser Gln Val
                405                 410                 415

Asp Phe Glu Leu Leu Leu Ala His Ala Arg Glu Leu Gly Val Leu Val
            420                 425                 430

Phe Glu Asn Ser Ala Lys Arg Leu Met Val Val Thr Pro Ala Gly His
        435                 440                 445

Ser Asp Val Lys Arg Phe Trp Lys Arg Gln Lys His Ser Ser
    450                 455                 460

<210>  13
<211>  7503
<212>  DNA
<213>  Homo sapiens

<400>  13
ggcgcactct gccggcaacg ccgaggcgct tctgcatctg tgggccgagc attcttcagg        60

tcatctgaac cttctgagaa aacatggtca acgtcttgaa aggagtgctt atagaatgtg 120

atcctgccat gaagcagttt ctgctgtact tggatgagtc caatgccctg gggaagaagt 180

tcatcattca agacattgat gacactcacg tctttgtaat agcagaattg gttaatgtcc 240

tccaggagcg agtgggtgaa ttaatggacc aaaatgcttt ttcccttacc cagaaatgaa 300

aatactcaat atggaccatt taggaattat aagcagcaac tgtgaaagac ttgccactca 360

atatcttagg tgactgatta gacatagagg gttgttttag gagcatgcca cgggaaagac 420

tgagggatca tgatcatttg ttcagaaaaa aagcccctga actgattttg ttaccataga 480

atttaaaaaa aaaaaaagct ttaacagttg gctgtaattt ggcttttatt atcctttatt 540

aaaatacaaa tgtcaatgct tttcctgcct ttttaatacc atgtcagtgt aacataggta 600

tttattttgc tcatccctgt gatctgtgca tttttgctgc gtggatgtga ttgtttggtt 660

tcagttagaa acgtcataga tttgctgttt gaatatgcca aggtggggac ttagacatta 720

tgtacgtctc acaaatccta cctgcatacc agtcagctct attgaggaag acaatgtaaa 780

actaatgtaa actacagttt gcatttccct gaaaacagaa tattgttttt aagaggttta 840

gaaacaacca gtgggaaagg cacatgctgc tttgtttagt ttttccttgt tcaaactttg 900

ttggtcacat tttcccatct gtattctttt ttattaagaa aacagcttag ggaggctgag 960

gtgggaggat tgcttgagcc caggagtccg aggttgctac aaaaacacca ttgcactctg 1020

gcctggacaa cagagagacc ctgcctcaaa aaaaaaaaaa aaaaaaaaaa aaattctact 1080

tgtaccttat tccctatgag aatacttatc aaacttatct aaaaaagaaa aataggaaca 1140

gccatatgca aagtcagccc aacaggaaag gacatattag attaaaatac attgcggcct 1200

gggcttagtg gctcacacct gtaatcccag cactttggga ggctgaggca ggcagatcgc 1260

ttgagtccag gagtcgaaac cctgtctcta caaaaaaaaa aatacaaaaa tctgcctgtt 1320

gtcccagcca ctggggaggc tgaggtagga ggtcaaggct gcagtgaacc ttgatcattg 1380

ctactgccac tccagcctag gtgacagagt gagaccctgt ctccaaaaaa aaaagtgtat 1440

atgtatgtgt gtatatatag caagagagag ttctgtgatc aattgaaggc aaaaacagta 1500

acactgagag gggctttgct tcccttcctg aagatcatgt cattggggtg ggttcctgta 1560

aagggaattt tccaagagaa aagagaattt tcatgacctg tagactctta caaatccatt 1620

ttacctgctt ccttatggta tgtttattca aagcacctgt gtaccatatt tattcactta 1680

cacagcatta ctgaatctgg aaattttcag ttaggtatat tttacataat tcccacccat 1740

ataactcagt ccatacagtt cacagttttc attccctcat agcaatgcaa aaaattttga 1800

tgagttacta ctactaaaac tagttaagta aaagatgatt cttaagaatt tccaggctgg 1860

gcacggtggc tcacgcctgt aatcccagca ctttgggagg ctgaggcagg cagctcacga 1920

ggtcaggaga tcgagaccat cctgctcggt gaaaccccat ctctattaaa aatacaaaaa 1980

attagccggg cctggtggcg ggtgcctgta gtcccatcta ctcaggaggc tgaggcagga 2040

gaatggcgtg aacccgggag gcggagcttg cagtaagctg agattgtgcc actgcactcc 2100

agcctgggcg acagagggag actccatctc aaaaaaaaaa aaaaaaaaaa atgaatttcc 2160

agctatacca aaatgttact aatgtatctt atatttcagg catgtctaaa tcaactttta 2220

gtatgcctca ggcatttgtc accaaccttt tatattaaca tgaaacttag aaaatttttg 2280

atagaatccc agagttcaaa aggtattagg attttgccca ctgaagtaaa aaagagaaaa 2340

aaagatattt atgagaaaac tattgaacag tgactgtgtt gtacccgagc aagttagagg 2400

aacgccacac tttgagacga atttaaaagt cctttattta gctggcagcc aagaggtggc 2460

tcacacttgg aattctctca gctccgagga aggggcttga ttttccttta tactttggtt 2520

taggaagggg acgggagctc agttgcaaca attctacaga agtaaaaaca tgcaaaaaat 2580

taaaaagaca aatggttaca gagaaacaaa cagttccagg tgcaggggct ctaaatctat 2640

cataagatgt caggtgtggg ggctctgcca gacacaaact caaggcttta tggtgtatct 2700

cttgagcgaa atcctgggaa cttcgtacat tgcttgcttc agtaccttat gagttaattg 2760

gactctttga tatgttgaga gtcagcttac acaagttaac tccttgagga aagggggtgg 2820

gtaaggagtc cttgatgtcc tgtaaatgaa ggaaccaaat ggagttcctc cggctttctc 2880

agctaaggga gagcttattc acatggaaac aaggctaggt gattaaggga gaaagggaca 2940

gtctgaaaac aaggttagta aaaacaaggt taggtattag aactgcaaag agtattaagt 3000

gaaataagtg aaggaaaata tgagctctta tttttaaaaa gttgtgttgg ggcaaatgtt 3060

aaatatcttt ctttctctgg acattattaa gaaagataga gtgatgtgaa tgataggaag 3120

cccacctgta accacttagc tgacatgtcc aactatgggt gaacaggaaa taagcgacat 3180

tcacctaggc tgtttattgg aatctgacta gcattatttg atttggcata ctttacatgt 3240

ccagaacaag gctgttggtg cttggcacca agaaatttat gacttttttt ttttttttaa 3300

ataaagtcct aaaggtagca ttaaaatgac ttaactggag gcaggaggat cactgaagcc 3360

caggagtttg aagctgcagt aagctaagaa ggtgccactg cactgcagcc tggatgacag 3420

agtgaaactt tgtctcttaa tagaactatg gactgaagtt gtgatattga gtgttatatt 3480

tctcaataaa ggttttttct tttaatactg tattaggtca aattcaaggt cttaattcct 3540

tgaattccaa tttccttgtg aaatttcatg tgattaattt aaaattccag ggatcctatc 3600

taacatcaca gttgatggat ataacaatgt attttgagaa atcagttttg gtgaacagta 3660

acagcattac tgataatagg caacaaacac tactgcattt tatttctgat aggcattata 3720

ggtggtaatc caaatgatta atgattttat tgctctttac attttctaga acaggtgatt 3780

tgacataatt gtactttttt ctgagaagtt ggtagtgtac ttactactct attttgcagt 3840

tatacaataa atcttactta gctgacaact aggagtaaaa gctctgacaa aaagcctctg 3900

ctgtaactaa aatactgaga tcattctatc ttaacaaact taacagatgg ggaaggggta 3960

tattttaaag atggagaaat gatgcagttt gcaaatatag tcaataaata ttctcaagtg 4020

tacgtagctc aatttcattt gcagcaaaga taacactgaa atgtctatgg atgagattat 4080

tggataaact tttttaaaaa cactgttctc aagataggca agtgattaag agcactgact 4140

gggccgggcg cggtggctca cgtctataat cctagcactt tgggaggccg aggcgggcaa 4200

atcacaaggt caggagatca aaacaatcct ggctaacaag gtgaaacccc gtctctacta 4260

aaaatacaaa aaaattagct gggcatggtg gtggacgcct gtagtcccag ctactcggga 4320

ggctgaggca ggagaatggt gtaatcccgg gaggtggagg ttgcagtgag ccgagatcgt 4380

gccactgcac tccagccggg gtgacagagc gagactccca cccatctcaa aaaaaataaa 4440

aatgaaaagc actgattgaa tctagattcc ttgagtccaa gtgtcagatc tctgcttact 4500

agctctggga tctcagatgt ttcgttgatt ggatttcttt gtgctcattt gtgtaaacag 4560

gaatgcccgt ctttaaagac tattaaaatg aatgtgagtt aacacacaag aggtcttcaa 4620

aaagttgatg gaaaatgcat attatgaaaa aaattatgca tagatttcaa aaatctttgc 4680

agcaaaataa actcatcatt ataacatgtc tgaacaggat ctagtttgag gcactaagaa 4740

ggatatcagt ttaaatagag cccctgtcag aacaatatga attctgctaa aactgaagca 4800

agaacaaaca tcaaatttat ggcaaagctt aggtggaaga atggtgcaat cattgatgct 4860

ttgcaaaaag tttatgggaa caacgcccca aggaaatcag tttacaaatg gataattcat 4920

tttaagaaag gacaagacaa tgttgaagat gaaacctaca gcagcagacc atgccacttc 4980

gtgaggaaaa aattatctca ttcatgccct acttgaagag gaccaatgat taacagcaga 5040

aacgatagcc aacacaatat gctcctcagt tggttcagct tttataaatc tgactgaaaa 5100

attaaagttg agcacacttt ccactcaatg gataccaaaa ccatccagcc caggtcagct 5160

gcagacaaaa gcagagcttt cgatggaaat gtcaaacaag tgagatcaag atcctgaagc 5220

atttcttcaa agaattgtag caaaaacaga cttttaaaac cttgaattca gagacaggta 5280

taaagactgc ttggtgattt tgaaaggact gagatcattt tgggagagat catcttcaga 5340

aaatgaccag atacaacaag aatttaacat ggcagccaaa ccaacccact aacacacatt 5400

taaataagat acccaaatgc agaaagaaaa gcacagtcgt catgcaaaat agtcactact 5460

cattcaaaaa ctctatagtt gtctgagtcg ttatgctcaa aatatccaat aatatttctt 5520

ttgctgagct gtaaaataat tattttgcaa acataaaaga aaaaaaaaag aattgtaaca 5580

gaaacatggc tttaccagta tgaccctcaa gacaaaatac aaaacaatgg ctaccaagag 5640

gtggaagtgg cccagtcaca gcaagagcag attgatcaag agcaaaggtc atggtaacag 5700

ttttttggat cctcaagtca ttttgcttgt tgactttttg gagggccaaa taaagatatc 5760

atctgcttat tatgagagtg tttcgagaga aatccaaagc ttagcagaga aacgcctggg 5820

aaagcttcac cagagggtct tccaccgtga cagttcccct gcgcattcct ctcataaagc 5880

aagggcagtt tagctagaat ttctatcaaa aatcattagg tatccatctc ccagtcctga 5940

tttggctcct tctgacttct ttttgtttcc caatcttaaa aaaatccctg aagggcacct 6000

gtttttcttc agctaataaa aaagacatgg ttaatttccc aggaccctca gttctttagg 6060

gatagactga atggctggta tcattgcttg aacttaatgg agcttatgtt gagaaataaa 6120

agaaattata ttttattttt atcttttaat tctatttttc catgaacttt ttgaagtccc 6180

ctcagatata aagcagaacg ttatgaaact gagttacctt gaatctagaa gagtacaatg 6240

ataaagggga agatagaaac acacacatat gcgttgtttt ccccactttc acaatgccgt 6300

gcagaaacta acagatgaag gaggagaatc cctaaaaaca gagcattggg tagctagtcc 6360

accagcggtc tgatgctttc attgatctca aaactgtctc tgagtaactc taccaggaaa 6420

gttgtctgga gggttaacat ttctgaaacc atttttatct ttgccttcat tcttgaatga 6480

taattagtct cgaatgtttg gatcacaatc cattgaaaga ctaggctatt ttttcactgt 6540

cctcagacat ttactgagtc cagtttggac tttattttat tttatttttt ttgaaacaag 6600

atctcgctct gttgctcagg ctgaagtgca atagcgcaaa ctcggctcac tgcaacctcc 6660

caggttcaag caattctcgt gcctcagcca tccctatagg catgcgccac cacactttgc 6720

taatcttgta tttttggtag agacggggtt tgcaccatgt tggccaggct ggtctcgaac 6780

tcccaacctc aagtaatctg gcctcccaaa gtggtgggat tacaggcgtg agccagcgcg 6840

cccggcctgg acttaacttc tgaagaactc tttctgttta gattgcagga gcgttctctc 6900

ggctcatctc ttgttttctc acccagctgt tcttcttcta gttggccatc attcttcatc 6960

cctccctccc tttcctgcag ttaattattt ctttgaaatt ttaaaacttc ttaatgttgc 7020

ctggaataca tgagtcttcc tgatctcttc aaacggggtt tgttttgttt tgctttgggg 7080

agaattcttt tgacgttttt gaataagctg ttaacaactt taatacctgt cttgtttgtt 7140

ttcttcagaa atttgttaat tctccatgat tagcaccttt atgtggttat ctcattattt 7200

tcattccttt ttaattctct gttttaggaa tgcttgtcaa attcgtcact aaaattgatt 7260

tactttcttg aaaacctgtt tctgctgctc ccaacatgat ttaatcttgg ctactgagct 7320

tttggttacg ttgcactccc tttaaaatta ctccgtttaa gtacagtgtt cactctttgg 7380

gtaatgtgta caatagaagc ccaatcccca cctataccca atatatccat gggacaagca 7440

tgtacatgca ccccctgagt ctaaaataaa aattttaaaa aaactaaaaa aaaaaaaaaa 7500

aaa 7503

<210> 14

<211>  71
<212>  PRT
<213>  Homo sapiens

<400>  14

Met Val Asn Val Leu Lys Gly Val Leu Ile Glu Cys Asp Pro Ala Met
1               5                   10                  15

Lys Gln Phe Leu Leu Tyr Leu Asp Glu Ser Asn Ala Leu Gly Lys Lys
            20                  25                  30

Phe Ile Ile Gln Asp Ile Asp Asp Thr His Val Phe Val Ile Ala Glu
        35                  40                  45

Leu Val Asn Val Leu Gln Glu Arg Val Gly Glu Leu Met Asp Gln Asn
    50                  55                  60

Ala Phe Ser Leu Thr Gln Lys
65                  70

<210>  15
<211>  5831
<212>  DNA
<213>  Homo sapiens

<400>  15
gacatttcac gccgccgcca ttttgagagc gagccgagcc gagctgccgg gcgccgcgtc      60

cgctgccgga gccccgacga cgacgccgag gaggcggagg ccgcggctct cggaacgcgg      120

ccggcccgtc gcccgcccgc tccgccgctc ccgccggccc cagggcccgc aggcccgccg      180

cggccggcca ggcctcccgt ccgccgcgcc cggcccgagg cggggctgac tccgcggccc      240

ccgaccggcc gccctccgcc cccggccggc ccgcggcccc gcagccccgg ccccggcccc      300

ggcggcggga ggcgggcccc gcggcggcgg cggcggcggc cgcagcgagc gacgaggccg      360

cccggcccgg cccgccggcc gcccgcccgc ctcggcgccg agattgggcg aatcgcgagc      420

aagtacgtgc gcgtctccct gccgccgccg ccgcccgccg cgggccgccc cggggccgcc      480

gtcgccgacg acgcgcggga ggaggaggag gaggccgccc cgccgccgcc gccgccgccg      540

ccgccccggc tcgccgccgc ccgcccgccg ggctcgcagc cccggccccc ggccgcaggc      600

gaggcccagg ccgcggccga catgaaccac cagcagcagc agcagcagca gaaagcgggc      660

gagcagcagt tgagcgagcc cgaggacatg gagatggaag cgggagatac agatgaccca      720

ccaagaatta ctcagaaccc tgtgatcaat gggaatgtgg ccctgagtga tggacacaac      780

accgcggagg aggacatgga ggatgacacc agttggcgct ccgaggcaac ctttcagttc      840

actgtggagc gcttcagcag actgagtgag tcggtcctta gccctccgtg ttttgtgcga      900

aatctgccat ggaagattat ggtgatgcca cgcttttatc cagacagacc acaccaaaaa      960

agcgtaggat tctttctcca gtgcaatgct gaatctgatt ccacgtcatg gtcttgccat 1020

gcacaagcag tgctgaagat aataaattac agagatgatg aaaagtcgtt cagtcgtcgt 1080

attagtcatt tgttcttcca taaagaaaat gattggggat tttccaattt tatggcctgg 1140

agtgaagtga ccgatcctga gaaaggattt atagatgatg acaaagttac ctttgaagtc 1200

tttgtacagg cggatgctcc ccatggagtt gcgtgggatt caaagaagca cacaggctac 1260

gtcggcttaa agaatcaggg agcgacttgt tacatgaaca gcctgctaca gacgttattt 1320

ttcacgaatc agctacgaaa ggctgtgtac atgatgccaa ccgaggggga tgattcgtct 1380

aaaagcgtcc ctttagcatt acaaagagtg ttctatgaat tacagcatag tgataaacct 1440

gtaggaacaa aaaagttaac aaagtcattt gggtgggaaa ctttagatag cttcatgcaa 1500

catgatgttc aggagctttg tcgagtgttg ctcgataatg tggaaaataa gatgaaaggc 1560

acctgtgtag agggcaccat acccaaatta ttccgcggca aaatggtgtc ctatatccag 1620

tgtaaagaag tagactatcg gtctgataga agagaagatt attatgatat ccagctaagt 1680

atcaaaggaa agaaaaatat atttgaatca tttgtggatt atgtggcagt agaacagctc 1740

gatggggaca ataaatacga cgctggggaa catggcttac aggaagcaga gaaaggtgtg 1800

aaattcctaa cattgccacc agtgttacat ctacaactga tgagatttat gtatgaccct 1860

cagacggacc aaaatatcaa gatcaatgat aggtttgaat tcccagagca gttaccactt 1920

gatgaatttt tgcaaaaaac agatcctaag gaccctgcaa attatattct tcatgcagtc 1980

ctggttcata gtggagataa tcatggtgga cattatgtgg tttatctaaa ccccaaaggg 2040

gatggcaaat ggtgtaaatt tgatgacgac gtggtgtcaa ggtgtactaa agaggaagca 2100

attgagcaca attatggggg tcacgatgac gacctgtctg ttcgacactg cactaatgct 2160

tacatgttag tctacatcag ggaatcaaaa ctgagtgaag ttttacaggc ggtcaccgac 2220

catgatattc ctcagcagtt ggtggagcga ttacaagaag agaaaaggat cgaggctcag 2280

aagcggaagg agcggcagga agcccatctc tatatgcaag tgcagatagt cgcagaggac 2340

cagttttgtg gccaccaagg gaatgacatg tacgatgaag aaaaagtgaa atacactgtg 2400

ttcaaagtat tgaagaactc ctcgcttgct gagtttgttc agagcctctc tcagaccatg 2460

ggatttccac aagatcaaat tcgattgtgg cccatgcaag caaggagtaa tggaacaaaa 2520

cgaccagcaa tgttagataa tgaagccgac ggcaataaaa caatgattga gctcagtgat 2580

aatgaaaacc cttggacaat attcctggaa acagttgatc ccgagctggc tgctagtgga 2640

gcgaccttac ccaagtttga taaagatcat gatgtaatgt tatttttgaa gatgtatgat 2700

cccaaaacgc ggagcttgaa ttactgtggg catatctaca caccaatatc ctgtaaaata 2760

cgtgacttgc tcccagttat gtgtgacaga gcaggattta ttcaagatac tagccttatc 2820

ctctatgagg aagttaaacc gaatttaaca gagagaattc aggactatga cgtgtctctt 2880
gataaagccc ttgatgaact aatggatggt gacatcatag tatttcagaa ggatgaccct 2940
gaaaatgata acagtgaatt acccaccgca aaggagtatt tccgagatct ctaccaccgc 3000
gttgatgtca ttttctgtga taaaacaatc cctaatgatc ctggatttgt ggttacgtta 3060
tcaaatagaa tgaattattt tcaggttgca aagacagttg cacagaggct caacacagat 3120
ccaatgttgc tgcagttttt caagtctcaa ggttataggg atggcccagg taatcctctt 3180
agacataatt atgaaggtac tttaagagat cttctacagt tcttcaagcc tagacaacct 3240
aagaaacttt actatcagca gcttaagatg aaaatcacag actttgagaa caggcgaagt 3300
tttaaatgta tatggttaaa cagccaattt agggaagagg aaataacact atatccagac 3360
aagcatgggt gtgtccggga cctgttagaa gaatgtaaaa aggccgtgga gcttggggag 3420
aaagcatcag ggaaacttag gctgctagaa attgtaagct acaaaatcat tggtgttcat 3480
caagaagatg aactattaga atgtttatct cctgcaacga gccggacgtt tcgaatagag 3540
gaaatccctt tggaccaggt ggacatagac aaagagaatg agatgcttgt cacagtggcg 3600
catttccaca aagaggtctt cggaacgttc ggaatcccgt ttttgctgag gatacaccag 3660
ggcgagcatt ttcgagaagt gatgaagcga atccagagcc tgctggacat ccaggagaag 3720
gagtttgaga agtttaaatt tgcaattgta atgatgggcc gacaccagta cataaatgaa 3780
gacgagtatg aagtaaattt gaaagacttt gagccacagc ccggtaatat gtctcatcct 3840
cggccttggc tagggctcga ccacttcaac aaagccccaa agaggagtcg ctacacttac 3900
cttgaaaagg ccattaaaat ccataactga tttccaagct ggtgtgttca aggcgaggac 3960
ggtgtgtggg tggcccctta acagcctaga actttggtgc acgtgccctc tagccgaagt 4020
cttcagcaag aggattcgct gctggtgtta attttatttt attgaggctg ttcagtttgg 4080
cttctctgta tctattgact gccctttttg agcaaaatga agatgttttt ataaagcttg 4140
gatgccaatg agagttattt tatggtaacc acagtgcaag gcaactgtca gcgcaatggg 4200
ggagaagagg ttagtggatc gggggtccct ggctcaaggt ctctgggctg tccctagtgg 4260
gcacgagtgg ctcggctgcc ttcctggggt cccgtgcacc agccctgcag ctagcaagtc 4320
ttgtgtttag gctcgtctga cctatttcct tcagttatac tttcaatgac cttttgtgca 4380
tctgttaagg caaaacagag aaactcacaa cctaataaat agcgctcttc ccttcattgt 4440
gtgcattgtc ggcccttcct cgggttctcc tcctccagct gcctgggggc tttttaataa 4500
acttgtctca cctcgtcagc cactactgtc tgcagcccct ttgcaaagtg gatgcactga 4560
atacagtccg gacagacatt gtgggggtct ttttattaaa tcaagaacat tgttaaattc 4620
aattaaggtt tactctgctg ccttggcaga cttacgatct caacagttca tacgagcagg 4680
tgaaaggatt ataaatagaa tttcgttaaa gtggaacaga cgacaagaaa gccttttagc 4740

aagagggcat gctcactagt ggttagtaag ctgtcgactt tgtaaaaaag ttaaaaatga 4800

aaaaaaaagg aaaaatgaat tgtatattta atgaatgaac atgtacaatt tgccactggg 4860

aggaggttcc tttttgttgg gtgagtctgc aagtgaattt cactgatgtt gatattcatt 4920

gtgtgtagtt ttatttcggt cccagccccg tttcctttta ttttggagct aatgccagct 4980

gcgtgtctag ttttgagtgc agtaaaatag aatcagcaaa tcactcttat ttttcatcct 5040

tttccggtat tttttgggtt gtttctgtgg gagcagtgta caccaactct tcctgtatat 5100

tgcctttttg ctggaaaatg ttgtatgttg aataaaattt tctataaaaa ttataattca 5160

gtgagttacg tggaagtgga ggaagatttc tactctccct ggaaacaggc ctgggaaacc 5220

ttggcatttg taacaaggtt tcactgagat gtacttttcc ttctaattcc gttttgcggg 5280

ggcagggtct cttgtttctt tttttttttt tttttttttt tagcctctaa ctagtcacat 5340

ttactcttaa gaaatgaaag gttttccagg agagaactgt gtacaaataa ggtgactgga 5400

gatgtgacct gatgtgtcac gaggcccttc ggggcggcag gcgctatcgt gggcgtggtc 5460

cttgcaccgt cccatcggcc ttgccttcca gctccgtggc acggtttcct ggtctttggg 5520

ccagtgtgta ccttggagtg acttcctttc tcaacttcca ctgcagtgtg tgtgccttct 5580

gctctgagag ctgccttgtg acccgtgtga tagaaagcag ggagtgaggg tccccgcgga 5640

cctggccctt ccctccttcc tcccccagaa agaggagtta gagcaggggt gcgagagccg 5700

ttcgctgtgg gtttgtcttt gaacaaacat taaggtgtct tgtttttgtt ctgggctggg 5760

ggttggctgt agtcttaggt aactgaaagt tcctactctc ccttaaggta ttaaatgact 5820

ctttttccaa a 5831

<210> 16
<211> 1102
<212> PRT
<213> Homo sapiens

<400> 16

<210> 17
<211> 4674
<212> DNA
<213> Homo sapiens

<400> 17
   gccagggcgt gtcccgtgtg cccgagagcg ccggcggcgg ggccggaggg cgggccctgg 60
   gccgagtgac aggccgcagc cccccgagca ccgtcgaagc cggcgcgccc gcccggtgca 120
   gccgctgtgc gagagcgacc ccgcggtcca agcctcgcgt ggccctgccg ggccccttct 180
   ttccccgagt agggcgcagc ttcccagcct ccgccccggc ctcgcctggc gcttccttcc 240
   gggtccttcg gcctttccct ggcggtgcgg caggccctcg cctcatccca ccaggcacgc 300
   cgcggtgctc ggccctgggt atccgggcag gctgcctccg ttagggccgc ccctgctctc 360
   cggacgcgac ttttcattgg tctcagaatt tcttggctcc tcttggcctc tgcagccttg 420
   ctggaggctg ccctgcggaa tctgaatatg gatcagaaac tttcgaagtt ggtagaagag 480
   ctcacaactt caggagaacc ccgactaaat cctgagaaaa tgaaggaact gaagaaaatt 540
   tgcaagtctt cagaggagca gctgagccgc gcctaccgcc tgctgatagc acagctgacc 600
   caggagcacg ccgagatccg tctctcagcc ttccagattg tggaggaact cttcgtcagg 660
   tctcaccagt tccggatgct ggttgtttcc aacttccagg agttcctgga gctcacgctg 720
   ggcacagacc ccgcacagcc tctgccgccc cccagggagg cggcacagag gctgaggcag 780
   gcgaccaccc gggccgtgga agggtggaat gagaagtttg gggaggccta caagaagctt 840
   gccttgggct accacttctt aagacacaac aaaaaggtgg attttcaaga cacgaatgct 900
   cggagtctgg cagaaaggaa gagagaagag gagaagcaga agcacttgga taaaatttat 960
   caagaaagag ccagccaggc ggagagggag atgcaagaaa tgtctggaga aattgaatcc 1020
   tgcttgacgg aggtagagag ctgctttagg ctgctggtgc cttttgactt tgacccgaac 1080
   ccggagacgg aatcccttgg catggcttct ggcatgtccg atgcccttcg ctcctcctgc 1140
   gcgggccagg tgggcccctg ccggtctggc acccctgacc cccggacggg ggagcagccc 1200
   tgctgcagta gagacctgcc tgcctctgca ggccacccca gagcgggcgg cggggcacag 1260
   ccatcccaga cagccacagg tgacccctca gatgaggacg aggacagcga cctcgaggag 1320
   tttgtgcgga gccacgggct gggctcgcac aagtacacgc tggatgtgga gctctgctca 1380
   gagggcctga aggtgcagga gaacgaggac aaccttgctc tcatccacgc cgcccgcgac 1440
   acactcaagc tcatccggaa caagttcctg ccggctgtgt gctcgtggat ccagcgcttc 1500
   acccgcgtcg ggacccacgg tggatgttta aagcgtgcca ttgacctgaa ggctgaattg 1560
   gagctcgtac tgagaaaata caaggagctg gacatcgagc ctgagggagg ggaaaggcgc 1620
   aggacagaag ccctggggga tgcggaggaa gatgaggacg atgaggactt tgtggaggtc 1680
   cctgagaagg aggggtatga gccacacatc cccgaccact tgcggcctga gtatgggctg 1740
   gaggcagcac cagagaaaga cacagttgtg cggtgcttgc ggacgaggac gaggatggac 1800
   gaggaggtgt cggaccccac ctctgcggct gctcagctgc ggcagctccg ggaccacttg 1860
   cctccaccct catctgccag cccctccaga gcgttgccag agccacagga ggcccagaag 1920
   ctggcagcag agcgggcccg ggcgcctgtg gtgccctacg gcgtggacct gcactactgg 1980
   ggccaggagc tccccacagc cgggaagatt gtcaagtctg actcccagca ccgcttctgg 2040
   aagcccagcg aggtggagga ggaagtggtc aatgccgaca tctccgagat gctccggagc 2100
   cgccacatca cttttgccgg gaagtttgag cctgtgcagc actggtgccg tgccccgagg 2160
   ccagacggcc ggctctgtga gcgccaagac cggctgaagt gccctttcca tgggaagatt 2220
   gttccacggg acgacgaagg acggccgctc gacccggaag acagggctcg tgagcagcgg 2280
   cggcagctgc agaagcagga gcgcccggaa tggcaggacc ctgagttgat gagagacgtg 2340
   gaagcagcca cagggcagga tctcggctca tccaggtaca gcgggaaagg cagggggaag 2400
   aagaggaggt accccagcct caccaacctg aaggctcagg ctgataccgc ccgcgctcgc 2460
   attgggagaa aagtcttcgc caaggcagct gtgcggaggg tagtggcagc catgaaccgg 2520
   atggaccaga agaagcacga gaagttttca aaccagttta actacgcact gaactagaga 2580
   gcggggccca gtgcactggc catcagcact ttctccctct gccagtgtct caggacagca 2640
   gagtgggcgt gggtctgggc agtaaccatg ctttgtctat tactgtgttt gatgtaaaga 2700
   aatggtgtgt tgcaatgccc tgaaggtacg gccgctctgc tgctacaggg ttcggcatcg 2760
   tctggtgatg ggtctggcct cgcagaagag gccctcgggc ctggagatgt gaacacaggc 2820
   agcgaccctg ttccagaggg cttctgcgag tcctcgtgag accagtgctt gtcgtggtgt 2880
   ggggttcagc acggacggaa tgtgtgtgca gctcagcctt cagagcgtgc attccccagc 2940
   caggaggcga ccactcagag gaactctggg aaacccactt ttgtgcaaat gctgttttta 3000
   acacaaacac aaaggctagt gaaccgttca gtcatctgct ttctgtttct ggatgtgcct 3060
   tttcatacat gtgtcctctt gtccctggct tcttacactt ggcgttgttt ttaaggttta 3120
   tccatgcagt tcccctccaa tgtataaaac aaaggaggtg aaaacctgtc catgcggaag 3180
   cttgtgcacc catgttcaca gcagcattgg agaaggatac cgaacatctt ctcgtgggct 3240
   tactggccat ttgtgtatct cctttggaga ggagtctatt caaacctctt gcctattttt 3300
   aattgaatga tttatcttga gttgtaacag ttgttttttt tgtttttcct tttagagatg 3360
   ggcttttgct cttacccagg ctgagtgcag tggcagaatc atagctcagt gcagcctcaa 3420
   actcctgggc tcaagccatc cttccacgtc agcctcccag gtagctggga ctacaggcac 3480
   acaccacaac acctggctaa ttttttaaat tttttgtaga gatgaggtct cactgtgttg 3540
   cccacggtga tctcgaactc ctagcctcaa gcgatcctcc tgcttcggtc tcccaaagtg 3600
   ttgggattac aggcatgagc caccactcct ggccaggagt ttatatgtat actgtggata 3660
   ctagatcctc atgtgatttg caaaaacttt atttatttat ttatttattt ttgaaatgga 3720
   atcttgctct gtcgccaggc tggagtgcaa tggcgtgatc tcggctcact gcaactacca 3780
   cctcctgggt tcaagcgatt cgcctgcgtc agcgtcctga gtagctggga ctacaggcgc 3840
   gcaccaccac gcccagctaa tttttgtatt tttagtagag acgggatttc actgtgttgg 3900
   ctgggatggt cttgatctct tgacctcgtg atccacccac ctcggcctcc catagtgctg 3960
   ggattacagg tgtgagccac cgcactcagc tgatttgcaa aaactttctt ccatactgtt 4020
   gtttcacttt ctggatagtg tcctctgaag cacaaaagtt aattttgatg aagtacagca 4080
   atttatctgt ttttttcttt tgttgcttat gcttttgctg tcacatttca gaagccatca 4140
   cctaatccaa gatcacagag atttacacct agtttctaat aagcatttta taatttcagc 4200
   tcttatgtgt agatctttga tccattgtga attaagtttt gtgtgtggtg taagagccca 4260
   cattcttttg catgtggata tccagttgtc ccagcaccat ttgttgaaaa cattattttt 4320
   ccccattgaa ttatcttcgc accattattg ataatcagtt gaccaaaaat gtacaggctt 4380
   atttctgggc acttgactct attccattga cctgtttaac cttatgccag tgccacagtg 4440
   ccttgatcag tgctgctttg ttgtgagaag ttttactttg ttctgcttca aggctgtttt 4500
   gactcttctg catctcttgc atttccaaat gaattttagg atccactcat cagtttcttc 4560
   aaaaaaaaaa aaaaagcagc tgagattttt gataggaatt acattaaatc tagagattgc 4620
   tttggaatgt attaccgtct taatattaaa tcttctgagt catgaaaaaa aaaa 4674

<210> 18
<211> 709
<212> PRT
<213> Homo sapiens

<400> 18

Met Asp Gln Lys Leu Ser Lys Leu Val Glu Glu Leu Thr Thr Ser Gly
1               5                   10                  15

Glu Pro Arg Leu Asn Pro Glu Lys Met Lys Glu Leu Lys Lys Ile Cys
            20                  25                  30

Lys Ser Ser Glu Glu Gln Leu Ser Arg Ala Tyr Arg Leu Leu Ile Ala
        35                  40                  45

Gln Leu Thr Gln Glu His Ala Glu Ile Arg Leu Ser Ala Phe Gln Ile
    50                  55                  60

Val Glu Glu Leu Phe Val Arg Ser His Gln Phe Arg Met Leu Val Val
65                  70                  75                  80

Ser Asn Phe Gln Glu Phe Leu Glu Leu Thr Leu Gly Thr Asp Pro Ala
                85                  90                  95

Gln Pro Leu Pro Pro Pro Arg Glu Ala Ala Gln Arg Leu Arg Gln Ala
            100                 105                 110

Thr Thr Arg Ala Val Glu Gly Trp Asn Glu Lys Phe Gly Glu Ala Tyr
        115                 120                 125

Lys Lys Leu Ala Leu Gly Tyr His Phe Leu Arg His Asn Lys Lys Val
    130                 135                 140

Asp Phe Gln Asp Thr Asn Ala Arg Ser Leu Ala Glu Arg Lys Arg Glu
145                 150                 155                 160

Glu Glu Lys Gln Lys His Leu Asp Lys Ile Tyr Gln Glu Arg Ala Ser
                165                 170                 175

Gln Ala Glu Arg Glu Met Gln Glu Met Ser Gly Glu Ile Glu Ser Cys
            180                 185                 190

Leu Thr Glu Val Glu Ser Cys Phe Arg Leu Leu Val Pro Phe Asp Phe
        195                 200                 205

Asp Pro Asn Pro Glu Thr Glu Ser Leu Gly Met Ala Ser Gly Met Ser
    210                 215                 220

Asp Ala Leu Arg Ser Ser Cys Ala Gly Gln Val Gly Pro Cys Arg Ser
225                 230                 235                 240

Gly Thr Pro Asp Pro Arg Asp Gly Glu Gln Pro Cys Cys Ser Arg Asp
                245                 250                 255

Leu Pro Ala Ser Ala Gly His Pro Arg Ala Gly Gly Gly Ala Gln Pro
            260                 265                 270

Ser Gln Thr Ala Thr Gly Asp Pro Ser Asp Glu Asp Glu Asp Ser Asp
        275                 280                 285

Leu Glu Glu Phe Val Arg Ser His Gly Leu Gly Ser His Lys Tyr Thr
    290                 295                 300

Leu Asp Val Glu Leu Cys Ser Glu Gly Leu Lys Val Gln Glu Asn Glu
305                 310                 315                 320

Asp Asn Leu Ala Leu Ile His Ala Ala Arg Asp Thr Leu Lys Leu Ile
                325                 330                 335

Arg Asn Lys Phe Leu Pro Ala Val Cys Ser Trp Ile Gln Arg Phe Thr
            340                 345                 350

Arg Val Gly Thr His Gly Gly Cys Leu Lys Arg Ala Ile Asp Leu Lys
        355                 360                 365

Ala Glu Leu Glu Leu Val Leu Arg Lys Tyr Lys Glu Leu Asp Ile Glu
    370                 375                 380

Pro Glu Gly Gly Glu Arg Arg Arg Thr Glu Ala Leu Gly Asp Ala Glu
385                 390                 395                 400

Glu Asp Glu Asp Asp Glu Asp Phe Val Glu Val Pro Glu Lys Glu Gly
                405                 410                 415

Tyr Glu Pro His Ile Pro Asp His Leu Arg Pro Glu Tyr Gly Leu Glu
            420                 425                 430

Ala Ala Pro Glu Lys Asp Thr Val Val Arg Cys Leu Arg Thr Arg Thr
        435                 440                 445

Arg Met Asp Glu Glu Val Ser Asp Pro Thr Ser Ala Ala Ala Gln Leu
    450                 455                 460

Arg Gln Leu Arg Asp His Leu Pro Pro Pro Ser Ser Ala Ser Pro Ser
465                 470                 475                 480

Arg Ala Leu Pro Glu Pro Gln Glu Ala Gln Lys Leu Ala Ala Glu Arg
                485                 490                 495

Ala Arg Ala Pro Val Val Pro Tyr Gly Val Asp Leu His Tyr Trp Gly
            500                 505                 510

Gln Glu Leu Pro Thr Ala Gly Lys Ile Val Lys Ser Asp Ser Gln His
        515                 520                 525

Arg Phe Trp Lys Pro Ser Glu Val Glu Glu Glu Val Val Asn Ala Asp

530                 535                 540

Ile Ser Glu Met Leu Arg Ser Arg His Ile Thr Phe Ala Gly Lys Phe
545                 550                 555                 560

Glu Pro Val Gln His Trp Cys Arg Ala Pro Arg Pro Asp Gly Arg Leu
                565                 570                 575

Cys Glu Arg Gln Asp Arg Leu Lys Cys Pro Phe His Gly Lys Ile Val
            580                 585                 590

Pro Arg Asp Asp Glu Gly Arg Pro Leu Asp Pro Glu Asp Arg Ala Arg
        595                 600                 605

Glu Gln Arg Arg Gln Leu Gln Lys Gln Glu Arg Pro Glu Trp Gln Asp
    610                 615                 620

Pro Glu Leu Met Arg Asp Val Glu Ala Ala Thr Gly Gln Asp Leu Gly
625                 630                 635                 640

Ser Ser Arg Tyr Ser Gly Lys Gly Arg Gly Lys Lys Arg Arg Tyr Pro
                645                 650                 655

Ser Leu Thr Asn Leu Lys Ala Gln Ala Asp Thr Ala Arg Ala Arg Ile
            660                 665                 670

Gly Arg Lys Val Phe Ala Lys Ala Ala Val Arg Arg Val Val Ala Ala
        675                 680                 685

Met Asn Arg Met Asp Gln Lys Lys His Glu Lys Phe Ser Asn Gln Phe
    690                 695                 700

Asn Tyr Ala Leu Asn
705

<210>  19
<211>  2947
<212>  DNA
<213>  Homo sapiens

<400>  19
tgctgtagct gccatgggca aaagagaccg agcggaccgc ggtgagacgt tgcgcgggca      60

cgctcagcca cgactgccct tgccggccct gccccccgct ctgcctcgga gctgctccgg     120

gcctctgcgc cggccgaccc tgctggccct cccgcgcgca ccccgttggg acaggccttt     180

gggcgggaga gatgctggac ctgggcgcag cccagcgaac tcggcctagg ggagacgggt     240

gaggggcgca acgcctgcgg gatgcaggtg gctcttagct aggagtttgc ggcggcgcag     300

acaagaagaa atccaggaag cggcactatg aggatgaaga ggatgatgaa gaggacgccc 360

cggggaacga ccctcaggaa gcggttccct cggcggcggg gaagcaggtg gatgagtcag 420

gcaccaaagt ggatgaatat ggagccaagg actacaggct gcaaatgccg ctgaaggacg 480

accacacctc caggcccctc tgggtggctc ccgatggcca tatcttcttg gaagccttct 540

ctccagttta caaatatgcc caagacttct tggtggctat tgcagagcca gtgtgccgac 600

caacccatgt gcatgagtac aaactaactg cctactcctt gtatgcagct gtcagcgttg 660

ggctgcaaac cagtgacatc accgagtacc tcaggaagct cagcaagact ggagtccctg 720

atggaattat gcagtttatt aagttgtgta ctgtcagcta tggaaaagtc aagctggtct 780

tgaagcacaa cagatacttc gttgaaagtt gccaccctga tgtaatccag catcttctcc 840

aggaccccgt gatccgagaa tgccgcttaa gaaactctga aggggaggcc actgagctca 900

tcacagagac tttcacaagc aaatctgcca tttctaagac tgctgaaagc agtggtgggc 960

cctccacttc ccgagtgaca gatccacagg gtaaatctga catccccatg gacctgtttg 1020

acttctatga gcaaatggac aaggatgaag aagaagaaga agagacacag acagtgtctt 1080

ttgaagtcaa gcaggaaatg attgaggaac tccagaaacg ttgcatccac ctggagtacc 1140

ctctgttggc agaatatgac ttccggaatg attctgtcaa ccctgatatc aacattgacc 1200

taaagcccac agctgtcctc agaccctatc aggagaagag cttgcgaaag atgtttggaa 1260

acgggcgtgc acgttcgggg gtcattgttc ttccctgcgg tgctggaaag tccctggttg 1320

gtgtgactgc tgcatgcact gtcagaaaac gctgtctggt gctgggcaac tcagctgttt 1380

ctgtggagca gtggaaagcc cagttcaaga tgtggtccac cattgacgac agccagatct 1440

gccggttcac ctccgatgcc aaggacaagc ccatcggctg ctccgttgcc attagcacct 1500

actccatgct gggccacacc accaaaaggt cctgggaggc cgagcgagtc atggagtggc 1560

tcaagaccca ggagtggggc ctcatgatcc tggatgaagt gcacaccata ccagccaaga 1620

tgttccgaag ggtgctcacc atcgtgcagg cccactgtaa gctgggtttg actgcgaccc 1680

tcgtccgcga agatgacaaa attgtggatt taaatttctt gattgggcct aagctctacg 1740

aagccaactg gatggagctg cagaataatg gctacatcgc caaagtccag tgtgctgagg 1800

tctggtgccc tatgtctcct gaattttacc gggaatatgt ggcaatcaaa accaagaaac 1860

gaatcttgct gtacaccatg aaccccaaca aatttagagc ttgccagttt ctgatcaagt 1920

ttcatgaaag gaggaatgac aagattattg tctttgctga caatgtgttt gccctaaagg 1980

aatatgccat tcgactgaac aaaccctata tctacggacc tacgtctcag ggggaaagga 2040

tgcaaattct ccagaatttc aagcacaacc ccaaaattaa caccatcttc atatccaagg 2100

taggtgacac ttcgtttgat ctgccggaag caaatgtcct cattcagatc tcatcccatg 2160

gtggctccag gcgtcaggaa gcccaaaggc tagggcgggt gcttcgagct aaaaaaggga 2220

tggttgcaga agagtacaat gcctttttct actcactggt atcccaggac acacaggaaa 2280

tggcttactc aaccaagcgg cagagattct tggtagatca aggttatagc ttcaaggtga 2340

tcacgaaact cgctggcatg gaggaggaag acttggcgtt ttcgacaaaa gaagagcaac 2400

agcagctctt acagaaagtc ctggcagcca ctgacctgga tgccgaggag gaggtggtgg 2460

ctggggaatt tggctccaga tccagccagg catctcggcg ctttggcacc atgagttcta 2520

tgtctggggc cgacgacact gtgtacatgg agtaccactc atcgcggagc aaggcgccca 2580

gcaaacatgt acacccgctc ttcaagcgct ttaggaaatg atgcttaggc agggtacttc 2640

gttcaagacc ggcgcttggc acccttgttg gaaagggatt ttcagcataa catttccctt 2700

ccacctcttt gaccttccct ccagcgttgg ccaaattgtg ctgaggaaga tgcatcaagg 2760

gcttggctgt gccttcatag gtcatctagg gttttataaa ggaggaggag acaatatttt 2820

ttcaaacttt ttggggagtg gggtcatttc tgtatataaa aaatgttaat atttaaggtg 2880

tatttatgtt accgttctga ataaacagaa tggaccattg aaccagtaaa aaaaaaaaaa 2940

aaaaaaa 2947

<210> 20
<211> 718
<212> PRT
<213> Homo sapiens

<400> 20

Met Pro Leu Lys Asp Asp His Thr Ser Arg Pro Leu Trp Val Ala Pro
1               5                   10                  15

Asp Gly His Ile Phe Leu Glu Ala Phe Ser Pro Val Tyr Lys Tyr Ala
            20                  25                  30

Gln Asp Phe Leu Val Ala Ile Ala Glu Pro Val Cys Arg Pro Thr His
        35                  40                  45

Val His Glu Tyr Lys Leu Thr Ala Tyr Ser Leu Tyr Ala Ala Val Ser
    50                  55                  60

Val Gly Leu Gln Thr Ser Asp Ile Thr Glu Tyr Leu Arg Lys Leu Ser
65                  70                  75                  80

Lys Thr Gly Val Pro Asp Gly Ile Met Gln Phe Ile Lys Leu Cys Thr
                85                  90                  95

Val Ser Tyr Gly Lys Val Lys Leu Val Leu Lys His Asn Arg Tyr Phe
            100                 105                 110

Val Glu Ser Cys His Pro Asp Val Ile Gln His Leu Leu Gln Asp Pro
        115                 120                 125

Val Ile Arg Glu Cys Arg Leu Arg Asn Ser Glu Gly Glu Ala Thr Glu
    130                 135                 140

Leu Ile Thr Glu Thr Phe Thr Ser Lys Ser Ala Ile Ser Lys Thr Ala
145                 150                 155                 160

Glu Ser Ser Gly Gly Pro Ser Thr Ser Arg Val Thr Asp Pro Gln Gly
                165                 170                 175

Lys Ser Asp Ile Pro Met Asp Leu Phe Asp Phe Tyr Glu Gln Met Asp
            180                 185                 190

Lys Asp Glu Glu Glu Glu Glu Glu Thr Gln Thr Val Ser Phe Glu Val
        195                 200                 205

Lys Gln Glu Met Ile Glu Glu Leu Gln Lys Arg Cys Ile His Leu Glu
    210                 215                 220

Tyr Pro Leu Leu Ala Glu Tyr Asp Phe Arg Asn Asp Ser Val Asn Pro
225                 230                 235                 240

Asp Ile Asn Ile Asp Leu Lys Pro Thr Ala Val Leu Arg Pro Tyr Gln
                245                 250                 255

Glu Lys Ser Leu Arg Lys Met Phe Gly Asn Gly Arg Ala Arg Ser Gly
            260                 265                 270

Val Ile Val Leu Pro Cys Gly Ala Gly Lys Ser Leu Val Gly Val Thr
        275                 280                 285

Ala Ala Cys Thr Val Arg Lys Arg Cys Leu Val Leu Gly Asn Ser Ala
    290                 295                 300

Val Ser Val Glu Gln Trp Lys Ala Gln Phe Lys Met Trp Ser Thr Ile
305                 310                 315                 320

Asp Asp Ser Gln Ile Cys Arg Phe Thr Ser Asp Ala Lys Asp Lys Pro
                325                 330                 335

Ile Gly Cys Ser Val Ala Ile Ser Thr Tyr Ser Met Leu Gly His Thr
            340                 345                 350

Thr Lys Arg Ser Trp Glu Ala Glu Arg Val Met Glu Trp Leu Lys Thr
        355                 360                 365

Gln Glu Trp Gly Leu Met Ile Leu Asp Glu Val His Thr Ile Pro Ala
    370                 375                 380

Lys Met Phe Arg Arg Val Leu Thr Ile Val Gln Ala His Cys Lys Leu
385                 390                 395                 400

Gly Leu Thr Ala Thr Leu Val Arg Glu Asp Asp Lys Ile Val Asp Leu
                405                 410                 415

Asn Phe Leu Ile Gly Pro Lys Leu Tyr Glu Ala Asn Trp Met Glu Leu
            420                 425                 430

Gln Asn Asn Gly Tyr Ile Ala Lys Val Gln Cys Ala Glu Val Trp Cys
        435                 440                 445

Pro Met Ser Pro Glu Phe Tyr Arg Glu Tyr Val Ala Ile Lys Thr Lys
    450                 455                 460

Lys Arg Ile Leu Leu Tyr Thr Met Asn Pro Asn Lys Phe Arg Ala Cys
465                 470                 475                 480

Gln Phe Leu Ile Lys Phe His Glu Arg Arg Asn Asp Lys Ile Ile Val
                485                 490                 495

Phe Ala Asp Asn Val Phe Ala Leu Lys Glu Tyr Ala Ile Arg Leu Asn
            500                 505                 510

Lys Pro Tyr Ile Tyr Gly Pro Thr Ser Gln Gly Glu Arg Met Gln Ile
        515                 520                 525

Leu Gln Asn Phe Lys His Asn Pro Lys Ile Asn Thr Ile Phe Ile Ser
    530                 535                 540

Lys Val Gly Asp Thr Ser Phe Asp Leu Pro Glu Ala Asn Val Leu Ile
545                 550                 555                 560

Gln Ile Ser Ser His Gly Gly Ser Arg Arg Gln Glu Ala Gln Arg Leu
                565                 570                 575

Gly Arg Val Leu Arg Ala Lys Lys Gly Met Val Ala Glu Glu Tyr Asn
            580                 585                 590

Ala Phe Phe Tyr Ser Leu Val Ser Gln Asp Thr Gln Glu Met Ala Tyr
        595                 600                 605

Ser Thr Lys Arg Gln Arg Phe Leu Val Asp Gln Gly Tyr Ser Phe Lys
    610                 615                 620

Val Ile Thr Lys Leu Ala Gly Met Glu Glu Glu Asp Leu Ala Phe Ser
625                 630                 635                 640

Thr Lys Glu Glu Gln Gln Gln Leu Leu Gln Lys Val Leu Ala Ala Thr
                645                 650                 655

Asp Leu Asp Ala Glu Glu Glu Val Val Ala Gly Glu Phe Gly Ser Arg
            660                 665                 670

Ser Ser Gln Ala Ser Arg Arg Phe Gly Thr Met Ser Ser Met Ser Gly
        675                 680                 685

Ala Asp Asp Thr Val Tyr Met Glu Tyr His Ser Ser Arg Ser Lys Ala
    690                 695                 700

Pro Ser Lys His Val His Pro Leu Phe Lys Arg Phe Arg Lys
705                 710                 715

<210>  21
<211>  2568
<212>  DNA
<213>  Homo sapiens

<400>  21
ttcatgaggg aggcgggtcg accccgctgc acagtccggc cggcgccatg aagctcaacg      60

tggacgggct cctggtctac ttcccgtacg actacatcta ccccgagcag ttctcctaca     120

tgcgggagct caaacgcacg ctggacgcca agggtcatgg agtcctggag atgccctcag     180

gcaccgggaa gacagtatcc ctgttggccc tgatcatggc ataccagaga gcatatccgc     240

tggaggtgac caaactcatc tactgctcaa gaactgtgcc agagattgag aaggtgattg     300

aagagcttcg aaagttgctc aacttctatg agaagcagga gggcgagaag ctgccgtttc     360

tgggactggc tctgagctcc cgcaaaaact tgtgtattca ccctgaggtg acacccctgc     420

gctttgggaa ggacgtcgat gggaaatgcc acagcctcac agcctcctat gtgcgggcgc     480

agtaccagca tgacaccagc ctgccccact gccgattcta tgaggaattt gatgcccatg     540

ggcgtgaggt gcccctcccc gctggcatct acaacctgga tgacctgaag gccctggggc     600

ggcgccaggg ctggtgccca tacttccttg ctcgatactc aatcctgcat gccaatgtgg     660

tggtttatag ctaccactac ctcctggacc ccaagattgc agacctggtg tccaaggaac     720

tggcccgcaa ggccgtcgtg gtcttcgacg aggcccacaa cattgacaac gtctgcatcg     780

actccatgag cgtcaacctc acccgccgga cccttgaccg gtgccagggc aacctggaga     840

ccctgcagaa gacggtgctc aggatcaaag agacagacga gcagcgcctg cgggacgagt     900

accggcgtct ggtggagggg ctgcgggagg ccagcgccgc ccgggagacg gacgcccacc     960

tggccaaccc cgtgctgccc gacgaagtgc tgcaggaggc agtgcctggc tccatccgca 1020

cggccgagca tttcctgggc ttcctgaggc ggctgctgga gtacgtgaag tggcggctgc 1080

gtgtgcagca tgtggtgcag gagagcccgc ccgccttcct gagcggcctg gcccagcgcg 1140

tgtgcatcca gcgcaagccc ctcagattct gtgctgaacg cctccggtcc ctgctgcata 1200

ctctggagat caccgacctt gctgacttct ccccgctcac cctccttgct aactttgcca 1260

cccttgtcag cacctacgcc aaaggcttca ccatcatcat cgagcccttt gacgacagaa 1320

ccccgaccat tgccaacccc atcctgcact tcagctgcat ggacgcctcg ctggccatca 1380

aacccgtatt tgagcgtttc cagtctgtca tcatcacatc tgggacactg tccccgctgg 1440

acatctaccc caagatcctg gacttccacc ccgtcaccat ggcaaccttc accatgacgc 1500

tggcacgggt ctgcctctgc cctatgatca tcggccgtgg caatgaccag gtggccatca 1560

gctccaaatt tgagacccgg gaggatattg ctgtgatccg gaactatggg aacctcctgc 1620

tggagatgtc cgctgtggtc cctgatggca tcgtggcctt cttcaccagc taccagtaca 1680

tggagagcac cgtggcctcc tggtatgagc aggggatcct tgagaacatc cagaggaaca 1740

agctgctctt tattgagacc caggatggtg ccgaaaccag tgtcgccctg gagaagtacc 1800

aggaggcctg cgagaatggc cgcggggcca tcctgctgtc agtggcccgg ggcaaagtgt 1860

ccgagggaat cgactttgtg caccactacg ggcgggccgt catcatgttt ggcgtcccct 1920

acgtctacac acagagccgc attctcaagg cgcggctgga atacctgcgg gaccagttcc 1980

agattcgtga gaatgacttt cttaccttcg atgccatgcg ccacgcggcc cagtgtgtgg 2040

gtcgggccat caggggcaag acggactacg gcctcatggt ctttgccgac aagcggtttg 2100

cccgtgggga caagcggggg aagctgcccc gctggatcca ggagcacctc acagatgcca 2160

acctcaacct gaccgtggac gagggtgtcc aggtggccaa gtacttcctg cggcagatgg 2220

cacagccctt ccaccgggag gatcagctgg gcctgtccct gctcagcctg gagcagctag 2280

aatcagagga gacgctgaag aggatagagc agattgctca gcagctctga gtggggcggg 2340

tggggccata aacggttcct ggtgactcct gagtcttgcc tggccctggt tcccagcggc 2400

ggtggtgcta gaaggtctta tgaagtcagg tgacatttct cactgtcacg tccacagcct 2460

ttaatcgcag gagaaggcag ctatccacca ggtacccaga ggcaaggggg ggccaggaga 2520

tgatagaccc cctctcaccc caccagccca tccctcctgc actgttcc 2568

<210> 22
<211> 760
<212> PRT
<213> Homo sapiens

<400> 22

<210>  23
<211>  6765
<212>  DNA
<213>  Homo sapiens

<400>  23
   agagcttcca tggagtcagg gcagccggct cgacggattg ccatggcgcc gctgctggag      60
   tacgagcgac agctggtgct ggaactgctc gacactgacg ggctagtagt gtgcgcccgc     120
   gggctcggcg cggaccggct cctctaccac tttctccagc tgcactgcca cccagcctgc     180
   ctggtgctgg tgctcaacac gcagccggcc gaggaggagt attttatcaa tcagctgaag     240
   atagaaggag ttgaacacct ccctcgccgt gtaacaaatg aaatcacaag caacagtcgc     300
   tatgaagttt acacacaagg tggtgttata tttgcgacaa gtaggatact tgtggttgac     360
   ttcttgactg atagaatacc ttcagattta attactggca tcttggtgta tagagcccac     420
   agaataatcg agtcttgtca agaagcattc atcttgcgcc tctttcgcca gaaaaacaaa     480
   cgtggtttta ttaaagcttt cacagacaat gctgttgcct ttgatactgg tttttgtcat     540
   gtggaaagag tgatgagaaa tctttttgtg aggaaactgt atctgtggcc aaggttccat     600
   gtagcagtaa actcattttt agaacagcac aaacctgaag ttgtagaaat ccatgtttct     660
   atgacaccta ccatgcttgc tatacagact gctatactgg acattttaaa tgcatgtcta     720
   aaggaactaa aatgccataa cccatcgctt gaagtggaag atttatcttt agaaaatgct     780
   attggaaaac cttttgacaa gacaatccgc cattatctgg atcctttgtg gcaccagctt     840
   ggagccaaga ctaaatcctt agttcaggat ttgaagatat tacgaacttt gctgcagtat     900
   ctctctcagt atgattgtgt cacatttctt aatcttctgg aatctctgag agcaacggaa     960
   aaagcttttg gtcagaattc aggttggctg tttcttgact ccagcacctc gatgtttata    1020
   aatgctcgag caagggttta tcatcttcca gatgccaaaa tgagtaaaaa agaaaaaata    1080
   tctgaaaaaa tggaaattaa agaaggggaa gaaacaaaaa aggaactggt cctagaaagc    1140
   aacccaaagt gggaggcact gactgaagta ttaaaagaaa ttgaggcaga aaataaggag    1200
   agtgaagctc ttggtggtcc aggtcaagta ctgatttgtg caagtgatga ccgaacatgt    1260
   tcccagctga gagactatat cactcttgga gcggaggcct tcttattgag gctctacagg    1320
   aaaacctttg agaaggatag caaagctgaa gaagtctgga tgaaatttag gaaggaagac    1380
   agttcaaaga gaattaggaa atctcacaaa agacctaaag acccccaaaa caaagaacgg    1440
   gcttctacca aagaaagaac cctcaaaaag aaaaaacgga agttgacctt aactcaaatg    1500
   gtaggaaaac ctgaagaact ggaagaggaa ggagatgtcg aggaaggata tcgtcgagaa    1560
   ataagcagta gcccagaaag ctgcccggaa gaaattaagc atgaagaatt tgatgtaaat    1620
   ttgtcatcgg atgctgcttt cggaatcctg aaagaacccc tcactatcat ccatccgctt 1680
   ctgggttgca gcgaccccta tgctctgaca agggtactac atgaagtgga gccaagatac 1740
   gtggttcttt atgacgcaga gctaaccttt gttcggcagc ttgaaattta cagggcgagt 1800
   aggcctggga aacctctgag ggtttacttt cttatatacg gaggttcaac tgaggaacaa 1860
   cgctatctca ctgctttgcg gaaagaaaag gaagcttttg aaaaactcat aagggaaaaa 1920
   gcaagcatgg ttgtccctga agaaagagaa ggcagagatg aaacaaactt agacctagta 1980
   agaggcacag catctgcaga tgtttccact gacactcgga aagccggtgg ccaggaacag 2040
   aatggtacac agcaaagcat agttgtggat atgcgtgaat ttcgaagtga gcttccatct 2100
   ctgatccatc gtcggggcat tgacattgaa cccgtgactt tagaggttgg agattacatc 2160
   ctcactccag aaatgtgcgt ggagcgcaag agtatcagtg atttaatcgg ctctttaaat 2220
   aacggccgcc tctacagcca gtgcatctcc atgtcccgct actacaagcg tcccgtgctt 2280
   ctgattgagt ttgaccctag caagcctttc tctctcactt cccgaggtgc cttgtttcag 2340
   gagatctcca gcaatgacat tagttccaaa ctcactcttc ttacacttca cttccccaga 2400
   ctacggattc tctggtgccc ctctcctcat gcaacggcgg agttgtttga ggagctgaaa 2460
   caaagcaagc cacagcctga tgcggcgaca gcactggcca ttacagcaga ttctgaaacc 2520
   cttcccgagt cagagaagta taatcctggt ccccaagact tcttgttaaa aatgccaggg 2580
   gtgaatgcca aaaactgccg ctccttgatg caccacgtta agaacatcgc agaattagca 2640
   gccctgtcac aagacgagct cacgagtatt ctggggaatg ctgcaaatgc caaacagctt 2700
   tatgatttca ttcacacctc ttttgcagaa gtcgtatcaa aaggaaaagg gaaaaagtga 2760
   acagtgatgg ctgttttctt atcccatgcc tgtacttttc agcggctcct tgccagacat 2820
   cataggtcat tattaattat tggtttgcta tttcattctt ttccaatgct cttaatgatt 2880
   gtacggtgga ccagaagcca ggattcctct ctgaactctg cagttaggca tcacttgaac 2940
   ttgcctgtgc ctgctctttt tcctccctgc accgtctatg ccgggcttag catgtttctt 3000
   tttaaatgag gtttgtcagg atcaggtaaa gttcctacaa gtgattacag aaggtagaaa 3060
   ctttacctga tcctaacaga tctcatttag aaaggaatat gctaagcctg gcatggacgg 3120
   tgcagggagg gaaaagagca ggcacaagaa agctaccatt tttaacagtc cttgttatct 3180
   agtgcaacat aaataacagt cttaattgca cttataccca tgtcctgtgg ctctccaaat 3240
   ctggtctttg ctgttgtgtc tgctggacgc ttgaactgat gtttgtgtag gaaatcatgt 3300
   tctgaccctt tgtctacaaa ggagccttct ggaacactga gaagaaacat ctctttgcca 3360
   ttcctgacca gttctctcta ccacattttc ttcagctcca tacttctgcc tgtctgctct 3420
   aaggaaattt catggagcct tcctactact aattcaagac agtctcctca aaaactggtt 3480
   gactagtctt ctaatgaccc taacatatgt agcatatact ataatttcat tgttccaaat 3540
   tagtattttt aaagcaaaat gaattacctg tttgcaaaag ttaatgatga aggagctctt 3600
   agaattctca atttttgcac atattcagtc tcctaatatc agagatccct aagtccagct 3660
   ggctagttac agagtttttt cagacttcct cgtttctcag ctcttatatc ctaagacacc 3720
   agcatcatat cctctagaaa tacaacctaa ttggcagtga gccgagatcg caccactgca 3780
   cccctgcctg ggcgacagag tgagactttg tctctattac aaaaagaaaa gaaaagaaat 3840
   acaacctaag ctcacctgcc tgtgattcct catttctcac catcctgtgc cagggtggct 3900
   acttctctct gtgaggactc aaatacaagc caatgagtgg cccactaagc ttttaagatt 3960
   tgattttcct gccttgagat aaaaaggagt gtaggtaaat gaaagatcaa tgtatggaat 4020
   atataaaaat acgaaagaaa tatatacgtt taaaaatcca taaagaaaaa aatctcattc 4080
   taaacctgat taagttggct ttttacgtaa gtgtacaaat aggatattca cagcatcttt 4140
   gtgcagtttt taaactttta tatttaaaca ttattaagtt ggcttttgtt cacatgttga 4200
   gtaatgggta gtaaattttc tacctcagga gctgatatag acatcagttc tgctagccat 4260
   atcacatatt ttaatgtttc atcaacatca gctgtttttt tgtttgctac actatttgaa 4320
   ctaatagaca gtggatcatg taacacaaaa ttgtcttcaa ctttaacaaa attgtcattg 4380
   ttattttttt ttgagacaga gtctccctct gttgcccagg ctggagtgca gtggcgcaat 4440
   ctcggctcac tgcaacctcg cctgctgggt tcaagcagtt ctcccacctc agcctcccaa 4500
   gtagctggga ttataggtgt gcaccaccag acccggctaa tttttgtatt tttagtagag 4560
   acggggtttc accatgttgg ccaggctggt ctcaaactac tgacctcagg tgatccaccc 4620
   accttggcct cccaaagtgc tgagataaca ggcgtgagcc accgcaccca cccaacaaaa 4680
   ttatttttaa ctgtcgtttc tgaactgaac ctctcacatt ggcatcttga tttattggta 4740
   cttaacagta tatatggatt ttgaactcta cacaaggtta taaccagaat gaattgaggg 4800
   gtatcaagaa ccagattggt tacagtagaa ctctgtaaga tgatgtggag agtaaggaaa 4860
   aggagaagaa ataaaaatta gtttaagatg gaataaagat ttgggctgct aattttttcc 4920
   caggattcaa aatatacccg ctagaatgga aaacaaaaat ttgaatgatt acaacattat 4980
   cgttaacatt caattttgta tttattgttt tattgaatat agttcagagt atattaaaat 5040
   agacctacca cttcaaatga taatgattat tataatgtct cttcacccta ttctagcact 5100
   tctgcttgca gtatgtggtt tctatttttt tcctcttgta taattccact tgcttttaat 5160
   tgttgtttca ttatataaaa ggaacatctt cccatagcat attctatgaa aggggtttca 5220
   ttccaagttg agttttcaaa aaaaaggtct tcctaaagct accattttca accgtccttg 5280
   ttatctagta caacataaat aacagtctta aaaattgcac taataccagt gcccccctgg 5340
   ctctccaaat ctgttctttg ctcttgtatc tgctggacgc ttgaagacag gtgcactgtc 5400
   tcgtatgtat ttgaattatg aacagtaatt tctaatgaat tctaaaatgg tcattgtaag 5460
   tgaaagcctc tcgctaccac ttcctcttcc aactacataa atatatttca atgtatttcc 5520
   agttttggaa agttttcaat acatacatca agtgtttact tagattttta taaaaatttt 5580
   ttttacaatc taataatctt tggtaaagga actagagatg catgcagttg caaaattaat 5640
   gtatttattt tccagcataa ttttattaac ttcacttttt ttctctctag taaatatcca 5700
   gtgtacttat gaactcatgt ttggctcttt taaaaccttt tctaaaagct agatcagcat 5760
   ttttctattt tacaagtttt ttgtataaaa aggtgaacat atgagatatt gtgagaaatc 5820
   attttaagtt tcatttaaat catggtgcct cttttgatac tttcttaaaa ttgtgcaaga 5880
   agaaatcatt tttagtagtg gtcataaata ttatcctttt ggcagtaagc tattactaat 5940
   tcagcctgaa gctcggtaga caatatgtct acatgtgttt gagtacatcc tggatacagt 6000
   ttcccagctc atgaggaact gaaaatagtc ttattcatca acccactagg atgtgaaggg 6060
   ttaagtctag atttggtcgc attgaaaccc cacaatatag aattaataaa tggccttcag 6120
   taggaaaacc tacactaaag caaatccgaa gaagtggggc agggggaaag aggcattact 6180
   ggtctttcct tttgttttgc aagcataatt tgattttcct ttggctcaga aaactcattt 6240
   ggggaaattc tcttttgtgt tcagtttaac ctagaaaggt cctcttgaaa aaccaacatt 6300
   ttaggaaagt tcttttttca ggatggagta tattaaaatt aagccaggct ttgacgtgaa 6360
   ttatcacttt tctttattat tttgttttct attttggttt atagctattt ctggttcagt 6420
   tctgaacttc agcacttaat catccttatc aaccaggctt ttggtagcct aaaccgctat 6480
   gctgttgttt ttttaattta aagatgtata agccaaaatt tggatgggag tgagacataa 6540
   ctgatttata tgaattttaa cagagttgta tttgtgtgtg tttaataaaa tatatattta 6600
   ttcagtactt tcctcagtat tttatgggca aagtaaaaat aacaatgcat agtgaaaggg 6660
   catatattac cagcagtaat aattcaaaat cctgaaaatg tttcattttt tttgtttttg 6720
   ttatgcagaa taaacaaggc agaaatgctc tttgaaccac taaaa 6765

<210> 24
<211> 916
<212> PRT
<213> Homo sapiens

<400> 24

<210>  25
<211>  4091
<212>  DNA
<213>  Homo sapiens

<400>  25
atttttcatg ggtttgcgga cccaccagcg aaggcgggag gtgtcgcagg gacatcttct      60

ggctgtttcc gtcgcctgcg tggcccttgc accccggtct tccattagcg gcgcagacgt     120

ttgggcctaa gcgctgggcg aggcgaggcc ctgcccctcc ccgccaacgg ccattctctg     180

gacctgtctt tcttccggga ggcggtgaca gctgctgaga cgtgttgcag ccagagtctc     240

tccgctttaa tgcgctccca ttagtgccgt cccccactgg aaaaccgtgg cttctgtatt     300

atttgccatc tttgttgtgt aggagcaggg agggcttcct cccggggtcc taggcggcgg     360

tgcagtccgt cgtagaagaa ttagagtaga agttgtcggg gtccgctctt aggacgcagc     420

cgcctcatgg gggtccaggg gctctggaag ctgctggagt gctccgggcg gcaggtcagc     480

cccgaagcgc tggaagggaa gatcctggct gttgatatta gcatttggtt aaaccaagca     540

cttaaaggag tccgggatcg ccatgggaac tcaatagaaa atcctcatct tctcactttg     600

tttcatcggc tctgcaaact cttatttttt cgaattcgtc ctatttttgt gtttgatggg     660

gatgctccac tattgaagaa acagactttg gtgaagagaa ggcagagaaa ggacttagcg     720

tccagtgact ccaggaaaac gacagagaag cttctgaaaa catttttgaa aagacaagcc 780

atcaaaactg ccttcagaag caaaagagat gaagcactac ccagtcttac ccaagttcga 840

agagaaaacg acctctatgt tttgcctcct ttacaagagg aagaaaaaca cagttcagaa 900

gaggaagatg aaaaagaatg gcaagaaaga atgaatcaaa aacaagcatt acaggaagag 960

ttctttcata atcctcaagc gatagatatt gagtctgagg acttcagcag cctgccccct 1020

gaagtaaagc atgaaatctt gactgatatg aaagagttca ccaagcgcag aagaacatta 1080

tttgaagcaa tgccagagga gtctgatgac ttttcacagt accaactcaa aggcttgctt 1140

aaaaagaact atctgaacca gcatatagaa catgtccaaa aggaaatgaa tcagcaacat 1200

tcaggacaca tccgaaggca gtatgaagat gaagggggct ttctgaagga ggtagagtca 1260

aggagagtgg tctctgaaga cacttcacat tacatcttga taaaaggtat tcaagctaag 1320

acagttgcag aagtggattc agagtctctt ccttcttcca gcaaaatgca cggcatgtct 1380

tttgacgtga agtcatctcc atgtgaaaaa ctgaagacag agaaagagcc tgatgctacc 1440

cctccttctc caagaacttt actagctatg caagctgccc tgctgggaag tagctcagaa 1500

gaggagctgg agagtgaaaa tcgaaggcag gcccgtggga ggaacgcacc tgctgctgta 1560

gacgaaggct ccatatcacc ccggactctt tcagccatta agagagctct tgacgatgac 1620

gaagatgtaa aagtgtgtgc tggggatgat gtgcagacgg gagggccagg agcagaagaa 1680

atgcgtataa acagctccac cgagaacagt gatgaaggac ttaaagtgag agatggaaaa 1740

ggaataccgt ttactgcaac acttgcgtca tctagtgtga actctgcaga ggagcacgta 1800

gccagcacta atgaggggag agagcccaca gactcagttc caaaagaaca aatgtcactt 1860

gttcacgtgg ggactgaagc ctttccgata agtgatgagt ctatgattaa ggacagaaaa 1920

gatcggctgc ctctggagag tgcagtggtt agacatagtg acgcacctgg gctcccgaat 1980

ggaaggaaac tgacaccggc atctccaact tgtacaaatt ctgtgtcaaa gaatgaaaca 2040

catgctgaag tgcttgagca gcagaacgaa ctttgcccat atgagagtaa attcgattct 2100

tctcttcttt caagtgatga tgaaacaaaa tgtaaaccga attctgcttc tgaagtcatt 2160

ggccctgtca gtttgcaaga aacaagtagc atagtaagtg tcccttcaga ggcagtagat 2220

aatgtggaaa atgtggtgtc atttaatgct aaagagcatg agaattttct ggaaaccatc 2280

caagaacagc agaccactga atctgcaggc caggatttaa tttccattcc aaaggccgtg 2340

gaaccaatgg aaattgactc ggaagaaagt gaatctgatg gaagtttcat tgaagtgcaa 2400

agtgtgatta gtgatgagga acttcaagca gaattccctg aaacttccaa acctccctca 2460

gaacaaggcg aagaggaact ggtaggaact agggagggag aagcccctgc tgagtccgag 2520

agcctcctga gggacaactc tgagagggac gacgtggatg gtgagccaca ggaagctgag 2580

aaagatgcgg aagattcgct ccatgaatgg caagatatta atttggagga gttggaaact 2640

ctggagagca acctcttagc acagcagaat tcactgaaag ctcaaaaaca gcagcaagaa 2700

cggatcgctg ctactgtcac cggacagatg ttcctggaaa gccaggaact cctgcgcctg 2760

ttcggcattc cctacatcca ggctcccatg gaagcagagg cgcagtgcgc catcctggac 2820

ctgactgatc agacttccgg aaccatcact gatgacagtg atatctggct gtttggagcg 2880

cggcatgtct atagaaactt ttttaataaa aacaagtttg tagaatatta tcaatatgtg 2940

gactttcaca atcaattggg attggaccgg aataagttaa taaatttggc ttatttgctt 3000

ggaagtgatt ataccgaagg aataccaact gtgggttgtg taaccgccat ggaaattctc 3060

aatgaattcc ctgggcatgg cctggaacct ctcctaaaat tctcagaatg gtggcatgaa 3120

gctcaaaaaa atccaaagat aagacctaat cctcatgaca ccaaagtgaa aaaaaaatta 3180

cggacattgc aactcacccc tggctttcct aacccagctg ttgccgaggc ctacctcaaa 3240

cccgtggtgg atgactcgaa gggatccttt ctgtggggga aacctgatct cgacaaaatt 3300

agagaatttt gtcagcggta tttcggctgg aacagaacga agacagatga atctctgttt 3360

cctgtattaa agcaactcga tgcccagcag acacagctcc gaattgattc cttctttaga 3420

ttagcacaac aggagaaaga agatgctaaa cgtattaaga gccagagact aaacagagct 3480

gtgacatgta tgctaaggaa agagaaagaa gcagcagcca gcgaaataga agcagtttct 3540

gttgccatgg agaaagaatt tgagctactt gataaggcaa aacgaaaaac ccagaagaga 3600

ggcataacaa ataccttaga agagtcatca agcctgaaaa gaaagaggct ttcagattct 3660

aaacgaaaga atacatgcgg tggatttttg ggggagacct gcctctcaga atcatctgat 3720

ggatcttcaa gtgaagatgc tgaaagttca tctttaatga atgtacaaag gagaacagct 3780

gcgaaagagc caaaaaccag tgcttcagat tcgcagaact cagtgaagga agctcccgtg 3840

aagaatggag gtgcgaccac cagcagctct agtgatagtg atgacgatgg agggaaagag 3900

aagatggtcc tcgtgaccgc cagatctgtg tttgggaaga aaagaaggaa actaagacgt 3960

gcgaggggaa gaaaaaggaa aacctaatta aaaaatatgt atcctctata attagttatg 4020

acagccattt gtaatgaatt tgtcgcaaag acgtaataaa attaactggt ggcacggtct 4080

ttgtaaaaaa a 4091

<210> 26
<211> 1186
<212> PRT
<213> Homo sapiens

<400> 26

Met Gly Val Gln Gly Leu Trp Lys Leu Leu Glu Cys Ser Gly Arg Gln
1               5                   10                  15

Val Ser Pro Glu Ala Leu Glu Gly Lys Ile Leu Ala Val Asp Ile Ser
            20                  25                  30

Ile Trp Leu Asn Gln Ala Leu Lys Gly Val Arg Asp Arg His Gly Asn
        35                  40                  45

Ser Ile Glu Asn Pro His Leu Leu Thr Leu Phe His Arg Leu Cys Lys
    50                  55                  60

Leu Leu Phe Phe Arg Ile Arg Pro Ile Phe Val Phe Asp Gly Asp Ala
65                  70                  75                  80

Pro Leu Leu Lys Lys Gln Thr Leu Val Lys Arg Arg Gln Arg Lys Asp
                85                  90                  95

Leu Ala Ser Ser Asp Ser Arg Lys Thr Thr Glu Lys Leu Leu Lys Thr
            100                 105                 110

Phe Leu Lys Arg Gln Ala Ile Lys Thr Ala Phe Arg Ser Lys Arg Asp
        115                 120                 125

Glu Ala Leu Pro Ser Leu Thr Gln Val Arg Arg Glu Asn Asp Leu Tyr
    130                 135                 140

Val Leu Pro Pro Leu Gln Glu Glu Glu Lys His Ser Ser Glu Glu Glu
145                 150                 155                 160

Asp Glu Lys Glu Trp Gln Glu Arg Met Asn Gln Lys Gln Ala Leu Gln
                165                 170                 175

Glu Glu Phe Phe His Asn Pro Gln Ala Ile Asp Ile Glu Ser Glu Asp
            180                 185                 190

Phe Ser Ser Leu Pro Pro Glu Val Lys His Glu Ile Leu Thr Asp Met
        195                 200                 205

Lys Glu Phe Thr Lys Arg Arg Arg Thr Leu Phe Glu Ala Met Pro Glu
    210                 215                 220

Glu Ser Asp Asp Phe Ser Gln Tyr Gln Leu Lys Gly Leu Leu Lys Lys
225                 230                 235                 240

Asn Tyr Leu Asn Gln His Ile Glu His Val Gln Lys Glu Met Asn Gln
                245                 250                 255

Gln His Ser Gly His Ile Arg Arg Gln Tyr Glu Asp Glu Gly Gly Phe
            260                 265                 270

Leu Lys Glu Val Glu Ser Arg Arg Val Val Ser Glu Asp Thr Ser His
        275                 280                 285

Tyr Ile Leu Ile Lys Gly Ile Gln Ala Lys Thr Val Ala Glu Val Asp
    290                 295                 300

Ser Glu Ser Leu Pro Ser Ser Ser Lys Met His Gly Met Ser Phe Asp
305                 310                 315                 320

Val Lys Ser Ser Pro Cys Glu Lys Leu Lys Thr Glu Lys Glu Pro Asp
                325                 330                 335

Ala Thr Pro Pro Ser Pro Arg Thr Leu Leu Ala Met Gln Ala Ala Leu
            340                 345                 350

Leu Gly Ser Ser Ser Glu Glu Glu Leu Glu Ser Glu Asn Arg Arg Gln
        355                 360                 365

Ala Arg Gly Arg Asn Ala Pro Ala Ala Val Asp Glu Gly Ser Ile Ser
    370                 375                 380

Pro Arg Thr Leu Ser Ala Ile Lys Arg Ala Leu Asp Asp Asp Glu Asp
385                 390                 395                 400

Val Lys Val Cys Ala Gly Asp Asp Val Gln Thr Gly Gly Pro Gly Ala
                405                 410                 415

Glu Glu Met Arg Ile Asn Ser Ser Thr Glu Asn Ser Asp Glu Gly Leu
            420                 425                 430

Lys Val Arg Asp Gly Lys Gly Ile Pro Phe Thr Ala Thr Leu Ala Ser
        435                 440                 445

Ser Ser Val Asn Ser Ala Glu Glu His Val Ala Ser Thr Asn Glu Gly
    450                 455                 460

Arg Glu Pro Thr Asp Ser Val Pro Lys Glu Gln Met Ser Leu Val His
465                 470                 475                 480

Val Gly Thr Glu Ala Phe Pro Ile Ser Asp Glu Ser Met Ile Lys Asp
                485                 490                 495

Arg Lys Asp Arg Leu Pro Leu Glu Ser Ala Val Val Arg His Ser Asp
            500                 505                 510

Ala Pro Gly Leu Pro Asn Gly Arg Glu Leu Thr Pro Ala Ser Pro Thr

        515                 520                 525

Cys Thr Asn Ser Val Ser Lys Asn Glu Thr His Ala Glu Val Leu Glu
    530                 535                 540

Gln Gln Asn Glu Leu Cys Pro Tyr Glu Ser Lys Phe Asp Ser Ser Leu
545                 550                 555                 560

Leu Ser Ser Asp Asp Glu Thr Lys Cys Lys Pro Asn Ser Ala Ser Glu
                565                 570                 575

Val Ile Gly Pro Val Ser Leu Gln Glu Thr Ser Ser Ile Val Ser Val
            580                 585                 590

Pro Ser Glu Ala Val Asp Asn Val Glu Asn Val Val Ser Phe Asn Ala
        595                 600                 605

Lys Glu His Glu Asn Phe Leu Glu Thr Ile Gln Glu Gln Gln Thr Thr
    610                 615                 620

Glu Ser Ala Gly Gln Asp Leu Ile Ser Ile Pro Lys Ala Val Glu Pro
625                 630                 635                 640

Met Glu Ile Asp Ser Glu Glu Ser Glu Ser Asp Gly Ser Phe Ile Glu
                645                 650                 655

Val Gln Ser Val Ile Ser Asp Glu Glu Leu Gln Ala Glu Phe Pro Glu
            660                 665                 670

Thr Ser Lys Pro Pro Ser Glu Gln Gly Glu Glu Glu Leu Val Gly Thr
        675                 680                 685

Arg Glu Gly Glu Ala Pro Ala Glu Ser Glu Ser Leu Leu Arg Asp Asn
    690                 695                 700

Ser Glu Arg Asp Asp Val Asp Gly Glu Pro Gln Glu Ala Glu Lys Asp
705                 710                 715                 720

Ala Glu Asp Ser Leu His Glu Trp Gln Asp Ile Asn Leu Glu Glu Leu
                725                 730                 735

Glu Thr Leu Glu Ser Asn Leu Leu Ala Gln Gln Asn Ser Leu Lys Ala
            740                 745                 750

Gln Lys Gln Gln Gln Glu Arg Ile Ala Ala Thr Val Thr Gly Gln Met
        755                 760                 765

Phe Leu Glu Ser Gln Glu Leu Leu Arg Leu Phe Gly Ile Pro Tyr Ile
    770                 775                 780

Gln Ala Pro Met Glu Ala Glu Ala Gln Cys Ala Ile Leu Asp Leu Thr
785                 790                 795                 800

Asp Gln Thr Ser Gly Thr Ile Thr Asp Asp Ser Asp Ile Trp Leu Phe
                805                 810                 815

Gly Ala Arg His Val Tyr Arg Asn Phe Phe Asn Lys Asn Lys Phe Val
            820                 825                 830

Glu Tyr Tyr Gln Tyr Val Asp Phe His Asn Gln Leu Gly Leu Asp Arg
        835                 840                 845

Asn Lys Leu Ile Asn Leu Ala Tyr Leu Leu Gly Ser Asp Tyr Thr Glu
    850                 855                 860

Gly Ile Pro Thr Val Gly Cys Val Thr Ala Met Glu Ile Leu Asn Glu
865                 870                 875                 880

Phe Pro Gly His Gly Leu Glu Pro Leu Leu Lys Phe Ser Glu Trp Trp
                885                 890                 895

His Glu Ala Gln Lys Asn Pro Lys Ile Arg Pro Asn Pro His Asp Thr
            900                 905                 910

Lys Val Lys Lys Lys Leu Arg Thr Leu Gln Leu Thr Pro Gly Phe Pro
        915                 920                 925

Asn Pro Ala Val Ala Glu Ala Tyr Leu Lys Pro Val Val Asp Asp Ser
    930                 935                 940

Lys Gly Ser Phe Leu Trp Gly Lys Pro Asp Leu Asp Lys Ile Arg Glu
945                 950                 955                 960

Phe Cys Gln Arg Tyr Phe Gly Trp Asn Arg Thr Lys Thr Asp Glu Ser
                965                 970                 975

Leu Phe Pro Val Leu Lys Gln Leu Asp Ala Gln Gln Thr Gln Leu Arg
            980                 985                 990

Ile Asp Ser Phe Phe Arg Leu Ala  Gln Gln Glu Lys Glu  Asp Ala Lys
        995                 1000                1005

Arg Ile  Lys Ser Gln Arg Leu  Asn Arg Ala Val Thr  Cys Met Leu
    1010                1015                1020

Arg Lys Glu Lys Glu Ala Ala Ala Ser Glu Ile Glu Ala Val Ser
    1025                1030                1035

Val Ala Met Glu Lys Glu Phe Glu Leu Leu Asp Lys Ala Lys Arg
    1040                1045                1050

Lys Thr Gln Lys Arg Gly Ile Thr Asn Thr Leu Glu Glu Ser Ser
    1055                1060                1065

Ser Leu Lys Arg Lys Arg Leu Ser Asp Ser Lys Arg Lys Asn Thr
    1070                1075                1080

Cys Gly Gly Phe Leu Gly Glu Thr Cys Leu Ser Glu Ser Ser Asp
    1085                1090                1095

Gly Ser Ser Ser Glu Asp Ala Glu Ser Ser Ser Leu Met Asn Val
    1100                1105                1110

Gln Arg Arg Thr Ala Ala Lys Glu Pro Lys Thr Ser Ala Ser Asp
    1115                1120                1125

Ser Gln Asn Ser Val Lys Glu Ala Pro Val Lys Asn Gly Gly Ala
    1130                1135                1140

Thr Thr Ser Ser Ser Ser Asp Ser Asp Asp Asp Gly Gly Lys Glu
    1145                1150                1155

Lys Met Val Leu Val Thr Ala Arg Ser Val Phe Gly Lys Lys Arg
    1160                1165                1170

Arg Lys Leu Arg Arg Ala Arg Gly Arg Lys Arg Lys Thr
    1175                1180                1185

<210> 27
<211> 2492
<212> DNA
<213> Homo sapiens

<400> 27
cactcagaaa ggccgctggg tgcggggagc gcagaggcgg tgcagggcgg ctggctcgcc 60

tcggcgtgca gtgcgcgtgc gtggagctgg gagctaggtc ctcggagtgg gccagagatg 120

gcggcggccg acggggcttt gccggaggcg gcggctttag agcaacccgc ggagctgcct 180

gcctcggtgc gggcgagtat cgagcggaag cggcagcggg cactgatgct gcgccaggcc 240

cggctggctg cccggcccta ctcggcgacg gcggctgcgg ctactggagg tttgggccgc 300

gtccgcgctt tccccttccc tctccccgcc tccccggtcc ccagactggc tcgtgcaagc 360

cgagtcccgg ggcccggggg tcgcgtcaac tccgctggcg tatgtgtgca gattctcccc 420

gagtcggaga gggaatccgc ccagccagcc gccttgtcaa agcgtcctgt ccacgaccac 480

agagcgttcc tctgtcgcac gcgggcctcc tgacccccag ccccgggcct tcttcgctgc 540

acctcggctg ctggcagctt cgatttttcg tttagggatg cagccgcccc ggccgggagg 600

tgtcagccac tgccaggtgt cagggcctca gctgtcccgg aaagaggagt tagactagtt 660

cttgattctg gcctctatac ctagaactag ttgatggaag aggcagatct tggtgtttgt 720

tagggagagc tttcaacaat tagagttgcc aaaagtgcaa tggattgctt ctcatgagct 780

tcccgttact agaagcgctc aaataggaac tggaagaacg cttacttatg tggaggagag 840

gagattcagc aatcagaatc tcctactcag caatcagaag aagggactgg atagtaaacc 900

tttaagattc taatccatag attccatgag tctatttctt ggacatgtga cctctgcgat 960

ttcttggcaa aaactagttg ctcaaaacgt gtttattgat tgaagtaaat taaggactga 1020

tctctcattg cagattattc tcttatagaa tttgccactg gggtcaacta ataatcttta 1080

acaggatact taaaatatag tttggttgac aggcagttct tcagggctgc ctctataaca 1140

tgaagtacat gtgttcctaa agctctatac tgccctttgt cgagcacagt ttagagctgt 1200

attactcttt tttatcagcc agatgtagtg agagtgagaa gagtctgagg ctggcagcat 1260

gtcttgatgt tcatactaac accgaaactg caaattccag ctacttttga tggcatggct 1320

aatgtaaaag cagccccaaa gataattgac acaggaggag gcttcatttt agaagaggaa 1380

gaagaagaag aacagaaaat tggaaaagtt gttcatcaac caggacctgt tatggaattt 1440

gattatgtaa tatgcgaaga atgtgggaaa gaatttatgg attcttatct tatgaaccac 1500

tttgatttgc caacttgtga taactgcaga gatgctgatg ataaacacaa gcttataacc 1560

aaaacagagg caaaacaaga atatcttctg aaagactgtg atttagaaaa aagagagcca 1620

cctcttaaat ttattgtgaa gaagaatcca catcattcac aatggggtga tatgaaactc 1680

tacttaaagt tacagattgt gaagaggtct cttgaagttt ggggtagtca agaagcatta 1740

gaagaagcaa aggaagtccg acaggaaaac cgagaaaaaa tgaaacagaa gaaatttgat 1800

aaaaaagtaa aagaattgcg gcgagcagta agaagcagcg tgtggaaaag ggagacgatt 1860

gttcatcaac atgagtatgg accagaagaa aacctagaag atgacatgta ccgtaagact 1920

tgtactatgt gtggccatga actgacatat gaaaaaatgt gattttttag ttcagtgacc 1980

tgttttatag aattttatat ttaaataaag gaaatttaga ttggtccttt tcaaaattca 2040

aaaaaaaaag caacatcttc atagatgaat gaaacccttg tataagtaat acttcagtaa 2100

taattatgta tgttatggct taaaagcaag tttcagtgaa ggtcacctgg cctggttgtg 2160

tgcacaatgt catgtctgtg attgccttct tacaacagag atgggagctg agtgctagag 2220

taggtgcaga agtggtaggt cagctacaaa tttgaggaca agataccaag gcaaacccta 2280

gattggggta gagggaaaag ggttcaacaa aggctgaact ggattcttaa ccaagaaaca 2340

aataatagca atggtggtgc accactgtac cccaggttct agtcatgtgt tttttaggac 2400

gatttctgtc tccacgatgg tggaaacagt ggggaactac tgctggaaaa agccctaata 2460

gcagaaataa acattgagtt gtacgagtct ga 2492

<210> 28
<211> 273
<212> PRT
<213> Homo sapiens

<400> 28

<210>  29
<211>  5739
<212>  DNA
<213>  Homo sapiens

<400>  29
gacgtaatgc ggtagcgcgg ggaatttcga gtggtgttgg agcgccggag gctagtgggt      60

ggctgacccc cagcatcctc gggagcgacc atggactccc tggccgagtc tcggtggcct     120

ccgggcctgg cagtcatgaa gacaatagat gatttgctgc ggtgtggaat ttgcttcgag     180

tatttcaaca ttgcaatgat aatacctcag tgttcacata actactgctc tctctgtata     240

agaaaatttc tgtcctataa aactcagtgt ccaacttgct gtgtgactgt cacagagccg     300

gatctgaaaa ataaccgcat attagatgaa ctggtaaaaa gcttgaattt tgcacggaat     360

catctgctgc agtttgcttt agagtcacca gccaaatctc ctgcttcttc ctcttcaaag     420

aatcttgctg tcaaagtata tactcctgta gcctccagac agtctttaaa gcaggggagc     480

aggttaatgg ataatttctt gatcagagaa atgagtggtt ctacatcaga gttgttgata     540

aaagaaaata aaagcaaatt cagccctcaa aaagaggcga gccctgctgc aaagaccaaa     600

gagacacgtt ctgtagaaga gatcgctcca gatccctcag aggctaagcg tcctgagcca     660

ccctcgacat ccactttgaa acaagttact aaagtggatt gtcctgtttg cggggttaac     720

attccagaaa gtcacattaa taagcattta gacagctgtt tatcacgcga agagaagaag     780

gaaagcctca gaagttctgt tcacaaaagg aagccgctgc ccaaaactgt atataatttg     840

ctctctgatc gtgatttaaa gaaaaagcta aaagagcatg gattatctat tcaaggaaat     900

aaacaacagc tcattaaaag gcaccaagaa tttgtacaca tgtacaatgc ccaatgcgat     960

gctttgcatc ctaaatcagc tgctgaaata gttcgagaaa tcgaaaatat agagaagact 1020

aggatgcgtc ttgaagctag taaactcaat gaaagtgtaa tggtttttac aaaggaccaa 1080

acagaaaagg aaatagatga aatccacagt aaatatcgta aaaaacataa gagtgaattt 1140

cagcttctgg tggatcaggc tagaaaagga tacaagaaaa ttgctggaat gtcacaaaaa 1200

acagtaacaa taacaaaaga agatgaatct acagaaaagc tatcttctgt atgcatggga 1260

caggaagata atatgacctc agtaacaaac cacttttctc aatcaaagct ggactcccca 1320

gaggaattgg aacctgacag agaagaggat tcttctagct gtattgatat tcaagaagtt 1380

ctttcttcat cagaatcaga ttcatgcaat agttccagtt cagacatcat aagagatctt 1440

ttagaagaag aggaagcctg ggaagcatca cataaaaacg atcttcaaga cacagaaata 1500

agtccaagac agaatcgccg cacaagagcc gctgaaagtg ctgagattga accaagaaac 1560

aagcgtaata ggaattaatg tgggcttttg ctgacttttc aaatgcattg attagaatac 1620

cgtacttttg gttgccacag atagattttc tatttataaa tgcccaagga aagatgctaa 1680

attctaaata ttacggttag ctgatattca ttcttttctg cttttccaga ggggaaaaat 1740

gttacaaaat atccttactt ggtcagttgc ctcctgcctc taaaacatct ctctctaaaa 1800

atactgacat ttcacacagg taccagcttt gcagaggagg tagactcttt ggcactttgg 1860

cacagggatt tggtttggtt tggtttggtt tgataattaa atttcagaat gtccttaggc 1920

cattctcctt ctcttccatg gagaatccag cctcacaaaa ggattctttc aacttcttta 1980

ttgcaagaaa cagttgagtt aaatgtttgt ttttggaaag gcgggatgtc agttcacatc 2040

cttggtgtgt gtaagtaccg atgcacgcca ccaccatgcc tgtgttacag cagctccatg 2100

atggttgttg cccgaggtta atgtagttgt ttgttagacc tgtgtcttac acatttctca 2160

gagtaggata ttagtattat aatttaaagc tacgacagtc acaaagtcac aataacttag 2220

aaacattgcg catattcttc tgaaatagca cttaaaaatg attagtgtca gtattttttc 2280

acttgggtca atcaaatctg taacactgaa tccaagctat taaacaaaaa gtatgcaatg 2340

aatgaatttt gtaaatgaat agagagtatc agtttacaat aatgttctta aaacagtatc 2400

ctctggatag ttgagtatgg gttagaaatc attgaaatgg attggtcaga aattgctatc 2460

tgtgtaaaat gtctaccagt agccagatgc ttccagagtt cttaatgcct ctctggcatt 2520

tcagagccag catcccccaa ctcccacccc tctgccatca cccaacccaa acacatcagc 2580

tttcaaatga gatgatagta aatgcggcaa tgttaagaca agaaatttat gatttgccag 2640

attcaacatt tatgacctcc ccttccaaag actgtctccg ttgaccttgt ctttttggta 2700

tgccttgggg tttctgataa tgtgtggagt ctcattatgg ctgagagttt agtgttttca 2760

cagtgaagtg cagacatttg atttctttat gagttccctg tgttagaaat ggctatagaa 2820

aaatttgtca taataatttc atttgcatga aatcctgagg ggtgcattaa ggaaactaaa 2880

agcaccactt accaaatcta tcggcagaac tgatgtgagg taagtgagca tgtcaaacaa 2940

aataggagct cacatggata tatttatgtc actgagttgt cagaaattat gtcaaaatga 3000

aaactgtttg tttcatgaca aattatatag tctataaatt aaactggaag taattattac 3060

tttaattgca gcaaaaggag tttgtgaggg agcggtgaga cccaagattg ggaaagtagg 3120

cacatgagtt cattcagcaa atatttggtt atctatgtct gtcactgtgc tgacactggg 3180

aatacaaagg tggccaaaga tcatctagaa caatggttcc cagtggggtg ggcagaaaga 3240

ttttgccccc caggagacag ctggcaatgt gtggagacac ttttggaggt ggagggtggt 3300

gaggggtact accagtatca atgggtggag gccagggatg aggctaaaca cccaaccctc 3360

atggattagt ttgccagggc tgccgtcaca agatactgca gacggggaag cttacacaac 3420

agaaatgtgt attctcagaa ttctggaggc tggaagtcca agatcagtag ggtttcttct 3480

tcggcctctc tccttggctt ccactcatgg tgtccttgca tggtcttttc tctgtgtgca 3540

catgactgtg caggactggt gtcccgattt cttgtaggga cagcagtcat tggatcaggg 3600

cccatgcgta tggcctcatt ttacttcagt tacctctttt ttagatagag ggccctatct 3660

ctaaatactg tcacattctg agataccggc agttaagact tccaacatat ttagtggtag 3720

gggttaaggg gcacgcttta gactacaaca ctccacagta aagaattatc cagtctaaca 3780

tgtcagttgt gccaagactg agaaacctgt aatgtaaagg aactcccaag tctacctgaa 3840

aagtaaggtc taaagagcat ttattgagca ctatctgtta gcacttagca tatgtttaat 3900

attcatacat tgaaagaaat gtatgttcat atccatctga caaagattaa ggaacttaga 3960

gtaagtaata gatccaaaat tgaaactgat aaacttctgg ttctaaaatt caccttttag 4020

tgaaaataca gtttctcacc aaacttacag taattcagag ttattaactc tttctacctc 4080

ctcactcccc tcaaagttat aacatctccc atcaacatca gctcagaatg gcagtgatgc 4140

ttatggtttg tgggggtgca ggtgagctgc tggatgcact ttagatggcc tagtatgcca 4200

ggccgtcctt tcatcctcta ctctcgtctt tctttttcga cctcatgttg aggattactg 4260

gtctagttga aaaaagaatt catacacata agaccatgta tataatagag ggaggtatgc 4320

ctttaaaaac aaaattacat ggtaaagact gatgtgtcaa cagggataga caacatagag 4380

aaggctagaa ctgtttctgg aaaaaaaccc aaaaacaatg gatttcaaac ttcttagcat 4440

ccgataaaag aaagtagcaa ttattcaaat gagaaacact tctgtctgtt atgtacatat 4500

tatgaaagta tgaacataag agggaaaacc acaaccatta cattttttac catttcagta 4560

attttttttt ggtttttagt ctgtttttag acatccttaa gaacaactat ctgaggctgt 4620

aaacaagtat ttacattaac acccatgata ctgactttat actccctacc tgcattgaga 4680

ccttaaaggg ggcatttctc cattccttct gtgtatttct gggtatcccc aggttcagat 4740

attcagaata cagatacaag tccatgcttg catatgttgc tgctcccccc gacagacttc 4800

taccagcatc tgccctcctc accgctgctc tgtcacgtgc actgcggctg gccctgctgg 4860

aaagtcctac cttgcctagg acgccatccc cgatatagcc ccaagatgca aacctgccaa 4920

gtgctagggc agccctgcct tcctgtccat ttcagtctct tggccttgct gtgttccaag 4980

tggaaatggg agtgtgcttt cctggtttgc tagtgtacta tcacccggac cacagcgtcc 5040

tgtgcaaaaa tttgcttcct ctctcttccc ctttcactct ttctcttttc tcttccccac 5100

cccctttttt ttctctttcc ccttaccttt ctttcctttc cactcttcct gtccttcctt 5160

ccttcaacta ttaggactat atgtcaagta atgtgcaaaa caaagacagt tcctgccctg 5220

atggagcttg tagatcagaa gggaagatga gcattaagta attatataga ttaatgtaca 5280

actgtgatga gtgcaacaaa agagaggttc atggcactat caaacatgca atggacggat 5340

tttgtttttg cctataaact cttgccatag aaagcttgaa aatcagtcca gggggacagc 5400

attaatcacc atgttctttc cttatccctg tcttccccaa aattcatgtg ttgaaactta 5460

acaagaggtg ggaactttag gatgtgatta agtcgtgagg gcacagctct tatagatggg 5520

gtcacggtcc ttctaaaagg gcttgaggga gtggttttgt ttccttccat cccttccgcc 5580

acatgaggac acagtcttcg ttccctctgg aggactcagc aacaacacac catcttggaa 5640

gcagagagca gtcctcacca gacacggaat ctgccagtgc cttgatcttg gacttaccag 5700

cctccatacc tgtgagaaat aaatttctat tgtctataa 5739

<210> 30
<211> 495
<212> PRT
<213> Homo sapiens

<400> 30

Met Asp Ser Leu Ala Glu Ser Arg Trp Pro Pro Gly Leu Ala Val Met
1               5                   10                  15

Lys Thr Ile Asp Asp Leu Leu Arg Cys Gly Ile Cys Phe Glu Tyr Phe
            20                  25                  30

Asn Ile Ala Met Ile Ile Pro Gln Cys Ser His Asn Tyr Cys Ser Leu
        35                  40                  45

Cys Ile Arg Lys Phe Leu Ser Tyr Lys Thr Gln Cys Pro Thr Cys Cys
    50                  55                  60

Val Thr Val Thr Glu Pro Asp Leu Lys Asn Asn Arg Ile Leu Asp Glu
65                  70                  75                  80

Leu Val Lys Ser Leu Asn Phe Ala Arg Asn His Leu Leu Gln Phe Ala
                85                  90                  95

Leu Glu Ser Pro Ala Lys Ser Pro Ala Ser Ser Ser Ser Lys Asn Leu
            100                 105                 110

Ala Val Lys Val Tyr Thr Pro Val Ala Ser Arg Gln Ser Leu Lys Gln
        115                 120                 125

Gly Ser Arg Leu Met Asp Asn Phe Leu Ile Arg Glu Met Ser Gly Ser
    130                 135                 140

Thr Ser Glu Leu Leu Ile Lys Glu Asn Lys Ser Lys Phe Ser Pro Gln
145                 150                 155                 160

Lys Glu Ala Ser Pro Ala Ala Lys Thr Lys Glu Thr Arg Ser Val Glu
                165                 170                 175

Glu Ile Ala Pro Asp Pro Ser Glu Ala Lys Arg Pro Glu Pro Pro Ser
            180                 185                 190

Thr Ser Thr Leu Lys Gln Val Thr Lys Val Asp Cys Pro Val Cys Gly
        195                 200                 205

Val Asn Ile Pro Glu Ser His Ile Asn Lys His Leu Asp Ser Cys Leu
    210                 215                 220

Ser Arg Glu Glu Lys Lys Glu Ser Leu Arg Ser Ser Val His Lys Arg
225                 230                 235                 240

Lys Pro Leu Pro Lys Thr Val Tyr Asn Leu Leu Ser Asp Arg Asp Leu
                245                 250                 255

Lys Lys Lys Leu Lys Glu His Gly Leu Ser Ile Gln Gly Asn Lys Gln
            260                 265                 270

Gln Leu Ile Lys Arg His Gln Glu Phe Val His Met Tyr Asn Ala Gln
        275                 280                 285

Cys Asp Ala Leu His Pro Lys Ser Ala Ala Glu Ile Val Arg Glu Ile
    290                 295                 300

Glu Asn Ile Glu Lys Thr Arg Met Arg Leu Glu Ala Ser Lys Leu Asn
305                 310                 315                 320

Glu Ser Val Met Val Phe Thr Lys Asp Gln Thr Glu Lys Glu Ile Asp
                325                 330                 335

Glu Ile His Ser Lys Tyr Arg Lys Lys His Lys Ser Glu Phe Gln Leu
                340                 345                 350

Leu Val Asp Gln Ala Arg Lys Gly Tyr Lys Lys Ile Ala Gly Met Ser
        355                 360                 365

Gln Lys Thr Val Thr Ile Thr Lys Glu Asp Glu Ser Thr Glu Lys Leu
    370                 375                 380

Ser Ser Val Cys Met Gly Gln Glu Asp Asn Met Thr Ser Val Thr Asn
385                 390                 395                 400

His Phe Ser Gln Ser Lys Leu Asp Ser Pro Glu Glu Leu Glu Pro Asp
                405                 410                 415

Arg Glu Glu Asp Ser Ser Ser Cys Ile Asp Ile Gln Glu Val Leu Ser
            420                 425                 430

Ser Ser Glu Ser Asp Ser Cys Asn Ser Ser Ser Ser Asp Ile Ile Arg
        435                 440                 445

Asp Leu Leu Glu Glu Glu Glu Ala Trp Glu Ala Ser His Lys Asn Asp
    450                 455                 460

Leu Gln Asp Thr Glu Ile Ser Pro Arg Gln Asn Arg Arg Thr Arg Ala
465                 470                 475                 480

Ala Glu Ser Ala Glu Ile Glu Pro Arg Asn Lys Arg Asn Arg Asn
                485                 490                 495

<210>  31
<211>  4263
<212>  DNA
<213>  Homo sapiens

<400>  31
ctctccacgg cgcacagagc atgcgcgggg cgggagtgag cgaaattcaa gctccaaact      60

ctaagctcca agctccaagc tccaagctcc aagctccaaa ctcccgccgg ggtaactgga     120

acccaatccg agggtcatgg aggcatcccg aaggtttccg gaagccgagg ccttgagccc     180

agagcaggct gctcattacc taagatatgt gaaagaggcc aaagaagcaa ctaagaatgg     240

agacctggaa gaagcattta aacttttcaa tttggcaaag gacatttttc ccaatgaaaa     300

agtgctgagc agaatccaaa aaatacagga agccttggag gagttggcag aacagggaga     360

tgatgaattt acagatgtgt gcaactctgg cttgctactt tatcgagaac tgcacaacca     420

actctttgag caccagaagg aaggcatagc tttcctctat agcctgtata gggatggaag     480

aaaaggtggt atattggctg atgatatggg attagggaag actgttcaaa tcattgcttt 540

cctttccggt atgtttgatg catcacttgt gaatcatgtg ctgctgatca tgccaaccaa 600

tcttattaac acatgggtaa aagaattcat caagtggact ccaggaatga gagtcaaaac 660

ctttcatggt cctagcaagg atgaacggac cagaaacctc aatcggattc agcaaaggaa 720

tggtgttatt atcactacat accaaatgtt aatcaataac tggcagcaac tttcaagctt 780

taggggccaa gagtttgtgt gggactatgt catcctcgat gaagcacata aaataaaaac 840

ctcatctact aagtcagcaa tatgtgctcg tgctattcct gcaagtaatc gcctcctcct 900

cacaggaacc ccaatccaga ataatttaca agaactatgg tccctatttg attttgcttg 960

tcaagggtcc ctgctgggaa cattaaaaac ttttaagatg gagtatgaaa atcctattac 1020

tagagcaaga gagaaggatg ctaccccagg agaaaaagcc ttgggattta aaatatctga 1080

aaacttaatg gcaatcataa aaccctattt tctcaggagg actaaagaag acgtacagaa 1140

gaaaaagtca agcaacccag aggccagact taatgaaaag aatccagatg ttgatgccat 1200

ttgtgaaatg ccttcccttt ccaggaaaaa tgatttaatt atttggatac gacttgtgcc 1260

tttacaagaa gaaatataca ggaaatttgt gtctttagat catatcaagg agttgctaat 1320

ggagacgcgc tcacctttgg ctgagctagg tgtcttaaag aagctgtgtg atcatcctag 1380

gctgctgtct gcacggcttt gttgtttgct aaatcttggg acattctctg ctcaagatgg 1440

aaatgagggg gaagattccc cagatgtgga ccatattgat caagtaactg atgacacatt 1500

gatggaagaa tctggaaaaa tgatattcct aatggaccta cttaagaggc tgcgagatga 1560

gggacatcaa actctggtgt tttctcaatc gaggcaaatt ctaaacatca ttgaacgcct 1620

cttaaagaat aggcacttta agacattgcg aatcgatggg acagttactc atcttttgga 1680

acgagaaaaa agaattaact tattccagca aaataaagat tactctgttt ttctgcttac 1740

cactcaagta ggtggtgtcg gtttaacatt aactgcagca actagagtgg tcatttttga 1800

ccctagctgg aatcctgcaa ctgatgctca agctgtggat agagtttacc gaattggaca 1860

aaaagagaat gttgtggttt ataggctaat cacttgtggg actgtagagg aaaaaatata 1920

cagaagacag gttttcaagg actcattaat aagacaaact actggtgaaa aaaagaaccc 1980

tttccgatat tttagtaaac aagaattaag agagctcttt acaatcgagg atcttcagaa 2040

ctctgtaacc cagctgcagc ttcagtcttt gcatgctgct cagaggaaat ctgatataaa 2100

actagatgaa catattgcct acctgcagtc tttggggata gctggaatct cagaccatga 2160

tttgatgtac acatgtgatc tgtctgttaa agaagagctt gatgtggtag aagaatctca 2220

ctatattcaa caaagggttc agaaagctca attcctcgtt gaattcgagt ctcaaaataa 2280

agagttcctg atggaacaac aaagaactag aaatgagggg gcctggctaa gagaacctgt 2340

atttccttct tcaacaaaga agaaatgccc taaattgaat aaaccacagc ctcagccttc 2400

acctcttcta agtactcatc atactcagga agaagatatc agttccaaaa tggcaagtgt 2460

agtcattgat gatctgccca aagagggtga gaaacaagat ctctccagta taaaggtgaa 2520

tgttaccacc ttgcaagatg gtaaaggtac aggtagtgct gactctatag ctactttacc 2580

aaaggggttt ggaagtgtag aagaactttg tactaactct tcattgggaa tggaaaaaag 2640

ctttgcaact aaaaatgaag ctgtacaaaa agagacatta caagaggggc ctaagcaaga 2700

ggcactgcaa gaggatcctc tggaaagttt taattatgta cttagcaaat caaccaaagc 2760

tgatattggg ccaaatttag atcaactaaa ggatgatgag attttacgtc attgcaatcc 2820

ttggcccatt atttccataa caaatgaaag tcaaaatgca gaatcaaatg tatccattat 2880

tgaaatagct gatgaccttt cagcatccca tagtgcactg caggatgctc aagcaagtga 2940

ggccaagttg gaagaggaac cttcagcatc ttcaccacag tatgcatgtg atttcaatct 3000

tttcttggaa gactcagcag acaacagaca aaatttttcc agtcagtctt tagagcatgt 3060

tgagaaagaa aatagcttgt gtggctctgc acctaattcc agagcagggt ttgtgcatag 3120

caaaacatgt ctcagttggg agttttctga gaaagacgat gaaccagaag aagtagtagt 3180

taaagcaaaa atcagaagta aagctagaag gattgtttca gatggcgaag atgaagatga 3240

ttcttttaaa gatacctcaa gcataaatcc attcaacaca tctctctttc aattctcatc 3300

tgtgaaacaa tttgatgctt caactcccaa aaatgacatc agtccaccag gaaggttctt 3360

ttcatctcaa atacccagta gtgtaaataa gtctatgaac tctagaagat ctctggcttc 3420

taggaggtct cttattaata tggttttaga ccacgtggag gacatggagg aaagacttga 3480

cgacagcagt gaagcaaagg gtcctgaaga ttatccagaa gaaggggtgg aggaaagcag 3540

tggcgaagcc tccaagtata cagaagagga tccttccgga gaaacactgt cttcagaaaa 3600

caagtccagc tggttaatga cgtctaagcc tagtgctcta gctcaagaga cctctcttgg 3660

tgcccctgag cctttgtctg gtgaacagtt ggttggttct ccccaggata aggcggcaga 3720

ggctacaaat gactatgaga ctcttgtaaa gcgtggaaaa gaactaaaag agtgtggaaa 3780

aatccaggag gccctaaact gcttagttaa agcgcttgac ataaaaagtg cagatcctga 3840

agttatgctc ttgactttaa gtttgtataa gcaacttaat aacaattgag aatgtaacct 3900

gtttattgta ttttaaagtg aaactgaata tgagggaatt tttgttccca taattggatt 3960

ctttgggaac atgaagcatt caggcttaag gcaagaaaga tctcaaaaag caacttctgc 4020

cctgcaacgc cccccactcc atagtctggt attctgagca ctagcttaat atttcttcac 4080

ttgaatattc ttatatttta ggcatattct ataaatttaa ctgtgttgtt tcttggaaag 4140

ttttgtaaaa ttattctggt cattcttaat tttactctga aagtgatcat ctttgtatat 4200

aacagttcag ataagaaaat taaagttact tttctcaagt gttttccaaa aaaaaaaaaa 4260

aaa                                                                 4263

<210>  32
<211>  1250
<212>  PRT
<213>  Homo sapiens

<400>  32
   Met Glu Ala Ser Arg Arg Phe Pro Glu Ala Glu Ala Leu Ser Pro Glu
   1               5                   10                  15
   Gln Ala Ala His Tyr Leu Arg Tyr Val Lys Glu Ala Lys Glu Ala Thr
               20                  25                  30
   Lys Asn Gly Asp Leu Glu Glu Ala Phe Lys Leu Phe Asn Leu Ala Lys
           35                  40                  45
   Asp Ile Phe Pro Asn Glu Lys Val Leu Ser Arg Ile Gln Lys Ile Gln
       50                  55                  60
   Glu Ala Leu Glu Glu Leu Ala Glu Gln Gly Asp Asp Glu Phe Thr Asp
   65                  70                  75                  80
   Val Cys Asn Ser Gly Leu Leu Leu Tyr Arg Glu Leu His Asn Gln Leu
                   85                  90                  95
   Phe Glu His Gln Lys Glu Gly Ile Ala Phe Leu Tyr Ser Leu Tyr Arg
               100                 105                 110
   Asp Gly Arg Lys Gly Gly Ile Leu Ala Asp Asp Met Gly Leu Gly Lys
           115                 120                 125
   Thr Val Gln Ile Ile Ala Phe Leu Ser Gly Met Phe Asp Ala Ser Leu
       130                 135                 140
   Val Asn His Val Leu Leu Ile Met Pro Thr Asn Leu Ile Asn Thr Trp
   145                 150                 155                 160
   Val Lys Glu Phe Ile Lys Trp Thr Pro Gly Met Arg Val Lys Thr Phe
                   165                 170                 175
   His Gly Pro Ser Lys Asp Glu Arg Thr Arg Asn Leu Asn Arg Ile Gln
               180                 185                 190
   Gln Arg Asn Gly Val Ile Ile Thr Thr Tyr Gln Met Leu Ile Asn Asn
           195                 200                 205
   Trp Gln Gln Leu Ser Ser Phe Arg Gly Gln Glu Phe Val Trp Asp Tyr
   210                 215                 220
   Val Ile Leu Asp Glu Ala His Lys Ile Lys Thr Ser Ser Thr Lys Ser
   225                 230                 235                 240
   Ala Ile Cys Ala Arg Ala Ile Pro Ala Ser Asn Arg Leu Leu Leu Thr
                   245                 250                 255
   Gly Thr Pro Ile Gln Asn Asn Leu Gln Glu Leu Trp Ser Leu Phe Asp
               260                 265                 270
   Phe Ala Cys Gln Gly Ser Leu Leu Gly Thr Leu Lys Thr Phe Lys Met
           275                 280                 285
   Glu Tyr Glu Asn Pro Ile Thr Arg Ala Arg Glu Lys Asp Ala Thr Pro
       290                 295                 300
   Gly Glu Lys Ala Leu Gly Phe Lys Ile Ser Glu Asn Leu Met Ala Ile
   305                 310                 315                 320
   Ile Lys Pro Tyr Phe Leu Arg Arg Thr Lys Glu Asp Val Gln Lys Lys
                   325                 330                 335
   Lys Ser Ser Asn Pro Glu Ala Arg Leu Asn Glu Lys Asn Pro Asp Val
               340                 345                 350
   Asp Ala Ile Cys Glu Met Pro Ser Leu Ser Arg Lys Asn Asp Leu Ile
           355                 360                 365
   Ile Trp Ile Arg Leu Val Pro Leu Gln Glu Glu Ile Tyr Arg Lys Phe
       370                 375                 380
   Val Ser Leu Asp His Ile Lys Glu Leu Leu Met Glu Thr Arg Ser Pro
   385                 390                 395                 400
   Leu Ala Glu Leu Gly Val Leu Lys Lys Leu Cys Asp His Pro Arg Leu
                   405                 410                 415
   Leu Ser Ala Arg Ala Cys Cys Leu Leu Asn Leu Gly Thr Phe Ser Ala
               420                 425                 430
   Gln Asp Gly Asn Glu Gly Glu Asp Ser Pro Asp Val Asp His Ile Asp
           435                 440                 445
   Gln Val Thr Asp Asp Thr Leu Met Glu Glu Ser Gly Lys Met Ile Phe
       450                 455                 460
   Leu Met Asp Leu Leu Lys Arg Leu Arg Asp Glu Gly His Gln Thr Leu
   465                 470                 475                 480
   Val Phe Ser Gln Ser Arg Gln Ile Leu Asn Ile Ile Glu Arg Leu Leu
                   485                 490                 495
   Lys Asn Arg His Phe Lys Thr Leu Arg Ile Asp Gly Thr Val Thr His
               500                 505                 510
   Leu Leu Glu Arg Glu Lys Arg Ile Asn Leu Phe Gln Gln Asn Lys Asp
           515                 520                 525
   Tyr Ser Val Phe Leu Leu Thr Thr Gln Val Gly Gly Val Gly Leu Thr
       530                 535                 540
   Leu Thr Ala Ala Thr Arg Val Val Ile Phe Asp Pro Ser Trp Asn Pro
   545                 550                 555                 560
   Ala Thr Asp Ala Gln Ala Val Asp Arg Val Tyr Arg Ile Gly Gln Lys
                   565                 570                 575
   Glu Asn Val Val Val Tyr Arg Leu Ile Thr Cys Gly Thr Val Glu Glu
               580                 585                 590
   Lys Ile Tyr Arg Arg Gln Val Phe Lys Asp Ser Leu Ile Arg Gln Thr
           595                 600                 605
   Thr Gly Glu Lys Lys Asn Pro Phe Arg Tyr Phe Ser Lys Gln Glu Leu
       610                 615                 620
   Arg Glu Leu Phe Thr Ile Glu Asp Leu Gln Asn Ser Val Thr Gln Leu
   625                 630                 635                 640
   Gln Leu Gln Ser Leu His Ala Ala Gln Arg Lys Ser Asp Ile Lys Leu
                   645                 650                 655
   Asp Glu His Ile Ala Tyr Leu Gln Ser Leu Gly Ile Ala Gly Ile Ser
               660                 665                 670
   Asp His Asp Leu Met Tyr Thr Cys Asp Leu Ser Val Lys Glu Glu Leu
           675                 680                 685
   Asp Val Val Glu Glu Ser His Tyr Ile Gln Gln Arg Val Gln Lys Ala
       690                 695                 700
   Gln Phe Leu Val Glu Phe Glu Ser Gln Asn Lys Glu Phe Leu Met Glu
   705                 710                 715                 720
   Gln Gln Arg Thr Arg Asn Glu Gly Ala Trp Leu Arg Glu Pro Val Phe
                   725                 730                 735
   Pro Ser Ser Thr Lys Lys Lys Cys Pro Lys Leu Asn Lys Pro Gln Pro
               740                 745                 750
   Gln Pro Ser Pro Leu Leu Ser Thr His His Thr Gln Glu Glu Asp Ile
           755                 760                 765
   Ser Ser Lys Met Ala Ser Val Val Ile Asp Asp Leu Pro Lys Glu Gly
       770                 775                 780
   Glu Lys Gln Asp Leu Ser Ser Ile Lys Val Asn Val Thr Thr Leu Gln
   785                 790                 795                 800
   Asp Gly Lys Gly Thr Gly Ser Ala Asp Ser Ile Ala Thr Leu Pro Lys
                   805                 810                 815
   Gly Phe Gly Ser Val Glu Glu Leu Cys Thr Asn Ser Ser Leu Gly Met
               820                 825                 830
   Glu Lys Ser Phe Ala Thr Lys Asn Glu Ala Val Gln Lys Glu Thr Leu
           835                 840                 845
   Gln Glu Gly Pro Lys Gln Glu Ala Leu Gln Glu Asp Pro Leu Glu Ser
       850                 855                 860
   Phe Asn Tyr Val Leu Ser Lys Ser Thr Lys Ala Asp Ile Gly Pro Asn
   865                 870                 875                 880
   Leu Asp Gln Leu Lys Asp Asp Glu Ile Leu Arg His Cys Asn Pro Trp
                   885                 890                 895
   Pro Ile Ile Ser Ile Thr Asn Glu Ser Gln Asn Ala Glu Ser Asn Val
               900                 905                 910
   Ser Ile Ile Glu Ile Ala Asp Asp Leu Ser Ala Ser His Ser Ala Leu
           915                 920                 925
   Gln Asp Ala Gln Ala Ser Glu Ala Lys Leu Glu Glu Glu Pro Ser Ala
       930                 935                 940
   Ser Ser Pro Gln Tyr Ala Cys Asp Phe Asn Leu Phe Leu Glu Asp Ser
   945                 950                 955                 960
   Ala Asp Asn Arg Gln Asn Phe Ser Ser Gln Ser Leu Glu His Val Glu
                   965                 970                 975
   Lys Glu Asn Ser Leu Cys Gly Ser Ala Pro Asn Ser Arg Ala Gly Phe
               980                 985                 990
   Val His Ser Lys Thr Cys Leu Ser  Trp Glu Phe Ser Glu  Lys Asp Asp
           995                 1000                1005
   Glu Pro  Glu Glu Val Val Val  Lys Ala Lys Ile Arg  Ser Lys Ala
       1010                 1015                 1020
   Arg Arg  Ile Val Ser Asp Gly  Glu Asp Glu Asp Asp  Ser Phe Lys
       1025                 1030                 1035
   Asp Thr  Ser Ser Ile Asn Pro  Phe Asn Thr Ser Leu  Phe Gln Phe
       1040                 1045                 1050
   Ser Ser  Val Lys Gln Phe Asp  Ala Ser Thr Pro Lys  Asn Asp Ile
       1055                 1060                 1065
   Ser Pro  Pro Gly Arg Phe Phe  Ser Ser Gln Ile Pro  Ser Ser Val
       1070                 1075                 1080
   Asn Lys  Ser Met Asn Ser Arg  Arg Ser Leu Ala Ser  Arg Arg Ser
       1085                 1090                 1095
   Leu Ile  Asn Met Val Leu Asp  His Val Glu Asp Met  Glu Glu Arg
       1100                 1105                 1110
   Leu Asp  Asp Ser Ser Glu Ala  Lys Gly Pro Glu Asp  Tyr Pro Glu
       1115                 1120                 1125
   Glu Gly  Val Glu Glu Ser Ser  Gly Glu Ala Ser Lys  Tyr Thr Glu
       1130                 1135                 1140
   Glu Asp  Pro Ser Gly Glu Thr  Leu Ser Ser Glu Asn  Lys Ser Ser
       1145                 1150                 1155
   Trp Leu  Met Thr Ser Lys Pro  Ser Ala Leu Ala Gln  Glu Thr Ser
       1160                 1165                 1170
   Leu Gly  Ala Pro Glu Pro Leu  Ser Gly Glu Gln Leu  Val Gly Ser
       1175                 1180                 1185
   Pro Gln  Asp Lys Ala Ala Glu  Ala Thr Asn Asp Tyr  Glu Thr Leu
       1190                 1195                 1200
   Val Lys  Arg Gly Lys Glu Leu  Lys Glu Cys Gly Lys  Ile Gln Glu
   1205                1210                1215
   Ala Leu Asn Cys Leu Val Lys Ala Leu Asp Ile Lys Ser Ala Asp
       1220                1225                1230
   Pro Glu Val Met Leu Leu Thr Leu Ser Leu Tyr Lys Gln Leu Asn
       1235                1240                1245
   Asn Asn
       1250

<210>  33
<211>  10324
<212>  DNA
<213>  Homo sapiens

<400>  33
caattcggga gcccgttctc gcgggatttt ccaagaccgg gttggaattt gcgggggtta      60

ggagacggaa gtcagagcct aggaagattt gggggtcgcc ttgccggcct cctgtcctcc     120

tccggcggcg gcggagcccg agagaactag gtgaacaccg ctttgccagc ctcacacagc     180

gtcccctggc tctgccgccg ctccggacgt cgccctcccg ttctgcttgg gtccccttag     240

tcgctacctt tgctgggatc cccctcctcc atcctgtggc ttcgggttgc cgaagagcga     300

tgctcggagg gcggccggaa gtggcgttgg ccgccattgg cctgccggcc agccaccttg     360

ctgtcctccg ccgccttccg ggtgttacat gcagccgggc tcggcccctc cccctggccg     420

gatggatccg tcggcgccac agccccgcgc ggaaacctca ggcaaagaca tatggcatcc     480

aggagaaaga tgtcttgccc cttctccaga taatggaaaa ctttgtgaag caagcataaa     540

atctatcaca gtggatgaaa atggcaagtc atttgcagtc gtcttatatg cagattttca     600

agaaaggaaa atacctctta aacagcttca agaagtgaaa tttgttaaag attgccctag     660

gaatcttata tttgatgatg aagatttaga aaaaccttat ttcccaaacc gaaaatttcc     720

atcatcttct gttgctttta aattatctga caatggagac tctattcctt ataccatcaa     780

taggtatttg agagactacc aaagagaagg aacccggttt ctttatggac actacatcca     840

tggaggaggg tgcattctgg gtgatgacat gggacttgga aaaacagtac aggttatttc     900

atttctggct gcagttttgc ataaaaaggg aactcgtgag gatattgaaa ataacatgcc     960

agagttttta ctaagaagta tgaaaaagga acccctttct tctacagcaa aaaagatgtt    1020

cttaatagtt gctcctcttt ctgtcctcta caactggaag gatgaattgg acacctgggg    1080

atatttcaga gtcactgttt tacatggaaa cagaaaagat aatgaattaa ttcgtgtaaa    1140

gcagaggaaa tgtgaaattg ctctaacaac ttatgaaaca ctacgcttat gcctggatga    1200

acttaacagt ttggaatggt cagctgtcat tgtggatgaa gctcatagaa tcaagaatcc    1260

aaaagctaga gtaacagaag ttatgaaagc tttgaaatgt aatgtccgca ttggcctcac 1320

tggaaccatc cttcagaaca acatgaagga actgtggtgt gttatggact gggctgtgcc 1380

aggcctttta gggagtggga cctacttcaa gaagcagttt tctgacccag tagaacatgg 1440

tcagagacac acggcaacaa agagagaact agccactggc cgaaaggcca tgcaaagact 1500

tgccaaaaag atgtctggct ggtttctcag gcgcaccaag actcttatca aggatcagtt 1560

gcctaagaag gaagaccgga tggtgtattg ttctttgaca gatttccaga aagctgtcta 1620

tcaaacagtg ttagaaacag aggacgtgac tttgatactt caatcttctg agccttgtac 1680

ctgtaggagt ggccaaaaaa ggagaaattg ttgttataag accaattctc atggtgaaac 1740

agtgaaaacc ttgtatctca gttaccttac agtccttcag aaggtagcta accatgtcgc 1800

gctactgcaa gctgctagta cttccaaaca acaggaaaca cttatcaaaa ggatatgtga 1860

tcaggtattt tccagattcc cagattttgt gcagaaaagc aaagatgcag cctttgaaac 1920

actttctgac cctaaataca gtggaaaaat gaaggtcctt cagcagcttt taaatcattg 1980

caggaaaaac agagataaag ttcttctctt ttctttttcc accaagttgc ttgacgtgct 2040

acagcagtac tgtatggcgt ctgggcttga ttaccgacga cttgatggaa gtacaaaatc 2100

agaggaaaga ctcaagattg taaaagagtt caacagtaca caagatgtta acatttgcct 2160

tgtctctaca atggctggtg gactaggcct caattttgtc ggtgccaatg ttgttgtatt 2220

atttgatcct acttggaatc cagccaatga tcttcaagcc attgacagag catataggat 2280

tggacaatgt agagatgtca aagtgcttag gctgatatcc ttgggaactg tggaggaaat 2340

catgtattta cgacagatat acaagcagca acttcactgt gtggtggttg gaagtgaaaa 2400

tgccaaacga tattttgaag cagttcaagg atctaaagag catcaaggag agctttttgg 2460

gatccataac ctcttcaaat ttaggtccca agggtcttgt cttacgaagg acatcctgga 2520

gagagaaggc caagtagaag cagggatcat gacagccaca acatggttga aagagggacc 2580

tccagcacac aaactggaaa tgcctagaca gcctgactgt caggaatgca gaggtacaga 2640

acaagctgca gagccactgg caaaggaagc atgtgatctc tgcagtgact tcagtgatga 2700

agagccagtg ggagccacag gaataaagac tgccaaaaac aaagcacccg attcaagtaa 2760

agcttccagc tctccaggac agcttacctt actccagtgt ggtttctcga aattgcttga 2820

aacaaaatgt aaagcagttg aggatagtga tggaaatact gcctctgatg atgaaagttc 2880

tgatgagcag cccacatgcc tttcaacaga agccaaagat gctggttgtg agaaaaatca 2940

ggactctctt ggtacttcaa aacatcagaa attagataac atcctaaatc caaaagaaaa 3000

gcatattttt tataaaagtg agaagatttt agaacagaat atttcttcca agtctgacga 3060

gaaaaaaatt aaaaatacag ataaacattg cattttacag aatgtcacag aatcagaaga 3120

tagtgatgtc atctgtccta cacaatacac aactgagaga ttccccgaca atagtataag 3180

gtttaagcca cccttggaag gatctgagga ttctgaaaca gaacacactg taaaaacaag 3240

aaataatgat aatagtcgaa acactgatga caaaagaaat ggaataattt caaaaaagtt 3300

aagtcctgag aacacaaccc tgaaatctat tttgaaaaga aaaggcacca gtgatatcag 3360

tgatgaatct gatgacattg aaatttcttc caagtcaaga gtaagaaaga gagctagttc 3420

attgaggttt aagagaataa aagaaaccaa aaaagaactt cacaattctc ccaaaacaat 3480

gaacaaaaca aaccaagtgt atgcagcaaa tgaggatcat aactctcagt ttattgatga 3540

ttattcatcc tcagatgaga gtttatccgt cagccacttc agtttctcta aacagagcca 3600

cagaccaaga actataagag acagaactag tttttcttca aaattgccta gccataataa 3660

gaaaaatagc acttttattc caagaaaacc aatgaaatgt tcaaatgaga aagttgttaa 3720

tcaagagcag tcgtatgaat caatggataa atttttagat ggcgttcagg aagtggctta 3780

tattcactca aaccagaatg taattggatc gagcaaagct gaaaatcaca tgagccgatg 3840

ggcagcacat gacgtatttg agttgaagca gttttctcag ctgcctgcta acatagctgt 3900

ttgcagttct aagacatata aagaaaaagt ggatgcagat acattgccac acacaaagaa 3960

aggccagcaa ccgagtgaag gcagcatttc acttcctctt tacatttcaa atcctgtaaa 4020

ccagaagaag aaaaaagtct accatacaaa ccagaccacc ttcataattg gagaaacacc 4080

aaaaggaatc cgcagaaaac aatttgaaga aatggcctct tattttaact cgtcttctgt 4140

aaacgaattt gctaaacata taaccaatgc cacatcagaa gaacgacaga aaatgctaag 4200

agacttttat gcttctcaat atccagaggt aaaagaattt tttgtggatt ctgtgtcaca 4260

attcaacaat tcttcctttg agaaaggaga gcagcgcacc cggaagaaat ctgataaaag 4320

agaatctctt ataaaaccaa ggctgtcaga ttctgaaacc ttgtcattta aagattctac 4380

caacaaaatt tctcaagttt gcagcctaaa aacatataaa agaaaatcag ttaagtttca 4440

gaatcatatt tcctatagag aagaggtgtt ttttaatgat gcagaaacta agaaatcacc 4500

tgttagttct actcaagaga ttgacagtgg gaaaaacagc caggcatccg aagatactgt 4560

gacatcccgt tctctgaaca gtgagtctga aacacgtgag agaaggttag aaaataccat 4620

gaaagaccaa caggacctca caagaacggg catttcaaga aaagaacccc ttctcaaatt 4680

ggaaaacaaa aagatagaaa atccagtgct ggaaaatact tctgtgataa gcttacttgg 4740

tgatacctct attcttgatg acctttttaa aagtcatggg aacagtccca cacaactgcc 4800

aaagaaagtt ctttcagggc ccatggaaaa agcaaaacag agaccaaaag atttctggga 4860

catcttgaat gagcagaatg atgagagtct tagtaaactc acagacttgg cagtaataga 4920

gactctgtgt gaaaaagcac ctctagcagc accctttaaa aggagagaag agccagcaac 4980

ttctctttgg aaatcaaatg agaaattttt atggaagaaa tttagcccaa gtgatacaga 5040

tgaaaacgca accaatacac agagtaccac ataagcatat aaatgaatta ctgcaccagt 5100

aaactgctgc catcactgtt tacggcactg gattccacac tgattctatt atcttgaaca 5160

cagttgttga catatatttt tattaaatta ttgctttagg attttttgaa gtctaaagta 5220

ttgtcatgga tctgtttttc ttgatatttg atttgatctt tcaagaatat gattgtattt 5280

atagtataaa cctctgttat gaattagaaa agattctagg tttgttaata ggagacctgg 5340

gacatctttc ttactatatt acataatgat gtgacacttg ccccggtgag cattgtttcc 5400

cagtatgaaa gatgaagagt ctgtaccgaa tcagcatgag tgtccttcca gtttaaaaaa 5460

gctttgcttc gctctcctaa tggctcatag gctgaatcat gtctgcccct caaatcaggt 5520

gtataccaat gtgtttttta ctagcacttg ggaaagttat taagtatttt ctttttccct 5580

gggcatcatg ttctattatt attttagaaa aaagtcataa ttggtactga atatatggta 5640

tatataatat taaaatggta attttgcaac agctcaaaat taaaaggtta atgttataca 5700

ctttactata tgagctgtga ttactaccat tagccacaga taccagtgcc tcaacttttt 5760

atgtacctat tgtgatttaa tgtaaataaa ggtttgtata gtacttttgt agttcttaag 5820

tatgaagaaa tgggtaaact ttttattttg ttagaaactg ttatattttg agtgtaatat 5880

ttatggttta tagcaaaatg aatgtgctta ttgttgaatg catgtattta gaagccttta 5940

ctcagcccct gtgttctgtg ctaggagctt gagctctaca ggtaaggcag agctaccggt 6000

gaatgaaagg aaatcatgtc agtgaaaaat catggtggaa agcccctggc atcacatgtg 6060

catgctgtag gcaggacctg agctgcctcc gctgcaggtt cagatgcacc gctgcagctg 6120

tccttcagtt agttcacagg gctgcaagag gaggacacat ccctccagaa aacagcctga 6180

gccgggaact ggctgtgcta aagagcactg ctatcaagtt gaggagagag ggcttccgtg 6240

tactcaggat gtagagtcat tgctcagaag tgaacaaaaa atcaaaaaca aaagtcttct 6300

caagggactg atcggccaag tatgcttttc tttagagcaa tgttttgccc tagagaattg 6360

taaaatttat gtcatgactc agtacatatg tgttcgtaca tatatgattg gaataaaatg 6420

tttatgaaat atttactcat aagccatgta acatactttg acatttttct cttctaggat 6480

tgtgtttggt agggcagtgg gtttgtgtgt gtgttaatcc tctcacaacc cactgaattg 6540

aatttcatgg cccatggtta agatccacag tgtgaaaacg ctgttttaaa ttatatgagt 6600

tcgtcatttg tttgctctgt gcagctgagt tgtgtccaga cttaccagat ggtatgtttt 6660

gccattgagg ggccttctac acaatgagtg catgatatgg tccttgatag acttgacttg 6720

tagatgtttt cagcctacaa tgtgatcagc tatctgagga actccagtaa gtagatacca 6780

cttcatttca gtttatatac aagacaatgt agttcaaaca ttttaatacc ttgtaaatta 6840

tgatattcat ataaatatta gctctatagt cttcatatat gtacagtttt tttttttttt 6900

tttttttttt tgagattgag tctcactctg tcacccaggc tggagtacag tggcatgatc 6960

ttggctcact gcaacctcca cctccctccc aaacaactct tatgcctcag cctcctgagt 7020

agctaggatt acaggtgtgt accaccacac tcagctaaac attttttttt ttttttgaga 7080

cggagtctca ctctgtcgcc aggctggagt gcagtggctc gatcttggct cactgcagcc 7140

tccgcccccc gagtcaagca attctcctgc ctcagcctcc cgagtagctg ggactacagg 7200

tgcgtgccac cacgcccagc taattttttt gtgtttttag tagggatggg gtttcaccat 7260

gttggccagg ctggtctcaa tctcctggct gcaagcgatc cacctgcctt gtcctcccaa 7320

agtgctggga ttacaggtgt gagccaccat gcccagccaa taaatgctat taaagaaact 7380

tttaggccgg gcacagtggc tcacgcctgt aatcccagca ctttgggagg ccgaggcagg 7440

cggatcacaa ggtcatgaga tcgagactat cctggctaac gcggtgaaac cccgtctcta 7500

ctaaaaaaaa aaaaaataca aaaattagcc aggcgtggtg gtgggcgcct gtagtcccag 7560

ctactcggga ggctgaggca ggagaatggc gtgaacctgg gaggcagagc ttgcagtgag 7620

ccaggattgt gcccctgcac cccagcctgg gtgacagagc aagactccat ctcaaaaaag 7680

gaaactttta tcaatagtag tcaaaatggg agcaaaaatg cagaactgga gtttaaaaat 7740

tagtttccaa cagttgtcag tgaaatattt atcactatta tgtaggaaag tgtgtcaggt 7800

ggaatgcaga gtccagtatg aaaaggagcc tgtttcagaa cggaaactgt caggtggagt 7860

ggactccctg caatggagtg atggaaagat ttggtacaac cattgatgac ctgagaacag 7920

acactaggaa ggtttgacaa gatccttcct acccccactc cactggtgat gtgtcaccaa 7980

gttctgctaa ctaaatgggt ggcctcaagc aaatccctta cctcctttta gccttagttt 8040

cccctttaaa ttggtgatgg attagatcag tggtggtcag ccattttctt aaagcaactg 8100

aactgtatct taatcagaaa cttaactagt aaaacaatta aaatgcaagt ctgttgcttc 8160

aagcaacacc actgtggctc catggagcac taatttgaaa accacagaga tgagatcatt 8220

tctaagaaaa cctttcaact ttgtttccat gatgctactt gccacaatct tttttactta 8280

agcaatgata ccgtttctgg ctgaacactc accacacagc tattaatatt ggaaactgag 8340

gcaagatacc aagagatttt tgcacttgaa ttcctcaggg ttctcttaca gccctgaaaa 8400

cacctcttca aggaaataaa taacatctgc aaatggtgca gctgccccta agtattcaag 8460

gcaaagaagc cttagtttgc agagatgatg ataaactttt catccttcca cagaaaatga 8520

gaaagccaca ggaaatacac ggttattttc taattaaagt tcaatgtgta cgcttttaaa 8580

ttctgaagtt atccttcttt tatgatatca aaatagccct cttttttgaa atgatggtag 8640

atcatacctt ttggtctttt tcattgtcct tacataaaag ttggtaatca tatgtcagtc 8700

ccagaagttt tatttgacac ttactggtct ataaaatcca aaggaatggg gatcactgcc 8760

tgccttaaag gtttttttgt gtgtttgttt gtttgtttgt ttgcatgtga aggattttta 8820

tgtcacaaat ccagctaaca gtggatctag gatctaggcc tcaccgagga tgtcactgaa 8880

cctgtcagcc ccaatgacag gcacacacct tcaaagagca agagtgccca tcactttcta 8940

ggctccttgc ctgctgctca ttccatcatc acggatacag acttgtttct ggtttcatct 9000

tccaccagag attgcaaagc atcctctgcc cagtgcttct aggaggcctt actgggatgg 9060

ttcataagcc actttcctct tggtgacagc ccctgtacac tgtggcatgt acggctggct 9120

ccacttggta tttgttgcag gagggaagaa catctcactt ttgcttggct aaagggccac 9180

cccaggagag ctattttgcc tcagggtctt gcagggtttc tctaggaaaa cagtgcatct 9240

gatagttact ctctgcccct aggaggaaag acagattcgc ccttccttcc caagtcccag 9300

gctgtgtggt tcccagcccc aagggtagca agtcagaacc tccggagagg cttttaagca 9360

tgcacatgtc cagggtcctc atcaagagac tgaatcaggg ggtctagggc tcagcacagg 9420

cacccttttt gtttttgcag tttcacagga attctgatgt gccagtgatt gtcaggtttg 9480

tccctgggca ggacccacct gcctgacctt ctgtccccct gggacacagt atttccagag 9540

aggtgcttca gttgccccac cacgacttcc agcgcccccc atctctgtat gcagctgagc 9600

tcatgatgga gcccactgtg tgctttttgt tgttgttaca ttcctataaa aatgtccacg 9660

catatttgaa catctgtgtt catagtggca ttattcacaa tggctacaac acgaagaaag 9720

cattccaagt acctatcgat gaataagcaa aatattacac acacaggaat attattcagc 9780

cttgaaagga aattcggaca cgtgctacaa catagatgaa tcttgaggac ggtatgcaaa 9840

gtgaaataaa tcagacacag aaggacaaat actgtatgat tccacttata taaagtacct 9900

agattagtca gattcataga gacaaagtag aatggtggtt gctagacgct ggagggaggg 9960

gagaaagggg agttattaat gggtgtagag tttctgttac acaggatgca aagtggcctg 10020

aagatggatg gtcgtgatgg ttgcacagca atgtgaattt aatgacactg aagtgtacac 10080

ttaaaagtca ttaaaatgct aaattttgta tgtgttttac aattttaaaa atggaaaaaa 10140

agtaagacca aaagcaacac ctctccagaa tgtgtgttat ataccaaatt ttgatgtatg 10200

tgagattgct gactatattt tacttatcaa tttgaaatta tattgttttt atgttgacac 10260

tccatataag ccattatttt gaaaaaaaca ttttccttat taaaagattg gaaaaaaatt 10320

caca 10324

<210> 34
<211> 1561
<212> PRT
<213> Homo sapiens

<400> 34

Met Gln Pro Gly Ser Ala Pro Pro Pro Gly Arg Met Asp Pro Ser Ala
1               5                   10                  15

Pro Gln Pro Arg Ala Glu Thr Ser Gly Lys Asp Ile Trp His Pro Gly
            20                  25                  30

Glu Arg Cys Leu Ala Pro Ser Pro Asp Asn Gly Lys Leu Cys Glu Ala
        35                  40                  45

Ser Ile Lys Ser Ile Thr Val Asp Glu Asn Gly Lys Ser Phe Ala Val
    50                  55                  60

Val Leu Tyr Ala Asp Phe Gln Glu Arg Lys Ile Pro Leu Lys Gln Leu
65                  70                  75                  80

Gln Glu Val Lys Phe Val Lys Asp Cys Pro Arg Asn Leu Ile Phe Asp
                85                  90                  95

Asp Glu Asp Leu Glu Lys Pro Tyr Phe Pro Asn Arg Lys Phe Pro Ser
            100                 105                 110

Ser Ser Val Ala Phe Lys Leu Ser Asp Asn Gly Asp Ser Ile Pro Tyr
        115                 120                 125

Thr Ile Asn Arg Tyr Leu Arg Asp Tyr Gln Arg Glu Gly Thr Arg Phe
    130                 135                 140

Leu Tyr Gly His Tyr Ile His Gly Gly Gly Cys Ile Leu Gly Asp Asp
145                 150                 155                 160

Met Gly Leu Gly Lys Thr Val Gln Val Ile Ser Phe Leu Ala Ala Val
                165                 170                 175

Leu His Lys Lys Gly Thr Arg Glu Asp Ile Glu Asn Asn Met Pro Glu
            180                 185                 190

Phe Leu Leu Arg Ser Met Lys Lys Glu Pro Leu Ser Ser Thr Ala Lys
        195                 200                 205

Lys Met Phe Leu Ile Val Ala Pro Leu Ser Val Leu Tyr Asn Trp Lys
    210                 215                 220

Asp Glu Leu Asp Thr Trp Gly Tyr Phe Arg Val Thr Val Leu His Gly
225                 230                 235                 240

Asn Arg Lys Asp Asn Glu Leu Ile Arg Val Lys Gln Arg Lys Cys Glu
                245                 250                 255

Ile Ala Leu Thr Thr Tyr Glu Thr Leu Arg Leu Cys Leu Asp Glu Leu
            260                 265                 270

Asn Ser Leu Glu Trp Ser Ala Val Ile Val Asp Glu Ala His Arg Ile
        275                 280                 285

Lys Asn Pro Lys Ala Arg Val Thr Glu Val Met Lys Ala Leu Lys Cys
    290                 295                 300

Asn Val Arg Ile Gly Leu Thr Gly Thr Ile Leu Gln Asn Asn Met Lys
305                 310                 315                 320

Glu Leu Trp Cys Val Met Asp Trp Ala Val Pro Gly Leu Leu Gly Ser
                325                 330                 335

Gly Thr Tyr Phe Lys Lys Gln Phe Ser Asp Pro Val Glu His Gly Gln
            340                 345                 350

Arg His Thr Ala Thr Lys Arg Glu Leu Ala Thr Gly Arg Lys Ala Met
        355                 360                 365

Gln Arg Leu Ala Lys Lys Met Ser Gly Trp Phe Leu Arg Arg Thr Lys
    370                 375                 380

Thr Leu Ile Lys Asp Gln Leu Pro Lys Lys Glu Asp Arg Met Val Tyr
385                 390                 395                 400

Cys Ser Leu Thr Asp Phe Gln Lys Ala Val Tyr Gln Thr Val Leu Glu
                405                 410                 415

Thr Glu Asp Val Thr Leu Ile Leu Gln Ser Ser Glu Pro Cys Thr Cys
            420                 425                 430

Arg Ser Gly Gln Lys Arg Arg Asn Cys Cys Tyr Lys Thr Asn Ser His
        435                 440                 445

Gly Glu Thr Val Lys Thr Leu Tyr Leu Ser Tyr Leu Thr Val Leu Gln
    450                 455                 460

Lys Val Ala Asn His Val Ala Leu Leu Gln Ala Ala Ser Thr Ser Lys
465                 470                 475                 480

Gln Gln Glu Thr Leu Ile Lys Arg Ile Cys Asp Gln Val Phe Ser Arg
                485                 490                 495

Phe Pro Asp Phe Val Gln Lys Ser Lys Asp Ala Ala Phe Glu Thr Leu
            500                 505                 510

Ser Asp Pro Lys Tyr Ser Gly Lys Met Lys Val Leu Gln Gln Leu Leu

        515                 520                 525

Asn His Cys Arg Lys Asn Arg Asp Lys Val Leu Leu Phe Ser Phe Ser
    530                 535                 540

Thr Lys Leu Leu Asp Val Leu Gln Gln Tyr Cys Met Ala Ser Gly Leu
545                 550                 555                 560

Asp Tyr Arg Arg Leu Asp Gly Ser Thr Lys Ser Glu Glu Arg Leu Lys
                565                 570                 575

Ile Val Lys Glu Phe Asn Ser Thr Gln Asp Val Asn Ile Cys Leu Val
            580                 585                 590

Ser Thr Met Ala Gly Gly Leu Gly Leu Asn Phe Val Gly Ala Asn Val
        595                 600                 605

Val Val Leu Phe Asp Pro Thr Trp Asn Pro Ala Asn Asp Leu Gln Ala
    610                 615                 620

Ile Asp Arg Ala Tyr Arg Ile Gly Gln Cys Arg Asp Val Lys Val Leu
625                 630                 635                 640

Arg Leu Ile Ser Leu Gly Thr Val Glu Glu Ile Met Tyr Leu Arg Gln
                645                 650                 655

Ile Tyr Lys Gln Gln Leu His Cys Val Val Val Gly Ser Glu Asn Ala
            660                 665                 670

Lys Arg Tyr Phe Glu Ala Val Gln Gly Ser Lys Glu His Gln Gly Glu
        675                 680                 685

Leu Phe Gly Ile His Asn Leu Phe Lys Phe Arg Ser Gln Gly Ser Cys
    690                 695                 700

Leu Thr Lys Asp Ile Leu Glu Arg Glu Gly Gln Val Glu Ala Gly Ile
705                 710                 715                 720

Met Thr Ala Thr Thr Trp Leu Lys Glu Gly Pro Pro Ala His Lys Leu
                725                 730                 735

Glu Met Pro Arg Gln Pro Asp Cys Gln Glu Cys Arg Gly Thr Glu Gln
            740                 745                 750

Ala Ala Glu Pro Leu Ala Lys Glu Ala Cys Asp Leu Cys Ser Asp Phe
        755                 760                 765

Ser Asp Glu Glu Pro Val Gly Ala Thr Gly Ile Lys Thr Ala Lys Asn
    770                 775                 780

Lys Ala Pro Asp Ser Ser Lys Ala Ser Ser Ser Pro Gly Gln Leu Thr
785                 790                 795                 800

Leu Leu Gln Cys Gly Phe Ser Lys Leu Leu Glu Thr Lys Cys Lys Ala
                805                 810                 815

Val Glu Asp Ser Asp Gly Asn Thr Ala Ser Asp Asp Glu Ser Ser Asp
            820                 825                 830

Glu Gln Pro Thr Cys Leu Ser Thr Glu Ala Lys Asp Ala Gly Cys Glu
        835                 840                 845

Lys Asn Gln Asp Ser Leu Gly Thr Ser Lys His Gln Lys Leu Asp Asn
    850                 855                 860

Ile Leu Asn Pro Lys Glu Lys His Ile Phe Tyr Lys Ser Glu Lys Ile
865                 870                 875                 880

Leu Glu Gln Asn Ile Ser Ser Lys Ser Asp Glu Lys Lys Ile Lys Asn
                885                 890                 895

Thr Asp Lys His Cys Ile Leu Gln Asn Val Thr Glu Ser Glu Asp Ser
            900                 905                 910

Asp Val Ile Cys Pro Thr Gln Tyr Thr Thr Glu Arg Phe Pro Asp Asn
        915                 920                 925

Ser Ile Arg Phe Lys Pro Pro Leu Glu Gly Ser Glu Asp Ser Glu Thr
    930                 935                 940

Glu His Thr Val Lys Thr Arg Asn Asn Asp Asn Ser Arg Asn Thr Asp
945                 950                 955                 960

Asp Lys Arg Asn Gly Ile Ile Ser Lys Lys Leu Ser Pro Glu Asn Thr
                965                 970                 975

Thr Leu Lys Ser Ile Leu Lys Arg Lys Gly Thr Ser Asp Ile Ser Asp
            980                 985                 990

Glu Ser Asp Asp Ile Glu Ile Ser Ser Lys Ser Arg Val Arg Lys Arg
        995                 1000                1005

Ala Ser Ser Leu Arg Phe Lys Arg Ile Lys Glu Thr Lys Lys Glu
    1010                1015                1020

Leu His Asn Ser Pro Lys Thr Met Asn Lys Thr Asn Gln Val Tyr
    1025                1030                1035

Ala Ala Asn Glu Asp His Asn Ser Gln Phe Ile Asp Asp Tyr Ser
    1040                1045                1050

Ser Ser Asp Glu Ser Leu Ser Val Ser His Phe Ser Phe Ser Lys
    1055                1060                1065

Gln Ser His Arg Pro Arg Thr Ile Arg Asp Arg Thr Ser Phe Ser
    1070                1075                1080

Ser Lys Leu Pro Ser His Asn Lys Lys Asn Ser Thr Phe Ile Pro
    1085                1090                1095

Arg Lys Pro Met Lys Cys Ser Asn Glu Lys Val Val Asn Gln Glu
    1100                1105                1110

Gln Ser Tyr Glu Ser Met Asp Lys Phe Leu Asp Gly Val Gln Glu
    1115                1120                1125

Val Ala Tyr Ile His Ser Asn Gln Asn Val Ile Gly Ser Ser Lys
    1130                1135                1140

Ala Glu Asn His Met Ser Arg Trp Ala Ala His Asp Val Phe Glu
    1145                1150                1155

Leu Lys Gln Phe Ser Gln Leu Pro Ala Asn Ile Ala Val Cys Ser
    1160                1165                1170

Ser Lys Thr Tyr Lys Glu Lys Val Asp Ala Asp Thr Leu Pro His
    1175                1180                1185

Thr Lys Lys Gly Gln Gln Pro Ser Glu Gly Ser Ile Ser Leu Pro
    1190                1195                1200

Leu Tyr Ile Ser Asn Pro Val Asn Gln Lys Lys Lys Lys Val Tyr
    1205                1210                1215

His Thr Asn Gln Thr Thr Phe Ile Ile Gly Glu Thr Pro Lys Gly
    1220                1225                1230

Ile Arg Arg Lys Gln Phe Glu Glu Met Ala Ser Tyr Phe Asn Ser
    1235                1240                1245

Ser Ser Val Asn Glu Phe Ala Lys His Ile Thr Asn Ala Thr Ser
    1250                1255                1260

Glu Glu Arg Gln Lys Met Leu Arg Asp Phe Tyr Ala Ser Gln Tyr
    1265                1270                1275

Pro Glu Val Lys Glu Phe Phe Val Asp Ser Val Ser Gln Phe Asn
    1280                1285                1290

Asn Ser Ser Phe Glu Lys Gly Glu Gln Arg Thr Arg Lys Lys Ser
    1295                1300                1305

Asp Lys Arg Glu Ser Leu Ile Lys Pro Arg Leu Ser Asp Ser Glu
    1310                1315                1320

Thr Leu Ser Phe Lys Asp Ser Thr Asn Lys Ile Ser Gln Val Cys
    1325                1330                1335

Ser Leu Lys Thr Tyr Lys Arg Lys Ser Val Lys Phe Gln Asn His
    1340                1345                1350

Ile Ser Tyr Arg Glu Glu Val Phe Phe Asn Asp Ala Glu Thr Lys
    1355                1360                1365

Lys Ser Pro Val Ser Ser Thr Gln Glu Ile Asp Ser Gly Lys Asn
    1370                1375                1380

Ser Gln Ala Ser Glu Asp Thr Val Thr Ser Arg Ser Leu Asn Ser
    1385                1390                1395

Glu Ser Glu Thr Arg Glu Arg Arg Leu Glu Asn Thr Met Lys Asp
    1400                1405                1410

Gln Gln Asp Leu Thr Arg Thr Gly Ile Ser Arg Lys Glu Pro Leu
    1415                1420                1425

Leu Lys Leu Glu Asn Lys Lys Ile Glu Asn Pro Val Leu Glu Asn
    1430                1435                1440

Thr Ser Val Ile Ser Leu Leu Gly Asp Thr Ser Ile Leu Asp Asp
    1445                1450                1455

Leu Phe Lys Ser His Gly Asn Ser Pro Thr Gln Leu Pro Lys Lys
    1460                1465                1470

Val Leu Ser Gly Pro Met Glu Lys Ala Lys Gln Arg Pro Lys Asp
    1475                1480                1485

Phe Trp Asp Ile Leu Asn Glu Gln Asn Asp Glu Ser Leu Ser Lys

1490                    1495                    1500

Leu Thr Asp Leu Ala Val Ile Glu Thr Leu Cys Glu Lys Ala Pro
    1505                    1510                    1515

Leu Ala Ala Pro Phe Lys Arg Arg Glu Glu Pro Ala Thr Ser Leu
    1520                    1525                    1530

Trp Lys Ser Asn Glu Lys Phe Leu Trp Lys Lys Phe Ser Pro Ser
    1535                    1540                    1545

Asp Thr Asp Glu Asn Ala Thr Asn Thr Gln Ser Thr Thr
    1550                    1555                    1560

<210>  35
<211>  25
<212>  DNA
<213>  Artificial Sequence

<220>
<223>  Synthetic polynucleotide

<400>  35
ccttgatccc agatgttgtg gcctg                                          25

<210>  36
<211>  25
<212>  DNA
<213>  Artificial Sequence

<220>
<223>  Synthetic polynucleotide

<400>  36
ctgggaaagc cctcattgct acagt                                          25

<210>  37
<211>  25
<212>  DNA
<213>  Artificial Sequence

<220>
<223>  Synthetic polynucleotide

<400>  37
tacctcagtt gtgaccttca gcaga                                          25

<210>  38
<211>  25
<212>  DNA
<213>  Artificial Sequence

<220>
<223>  Synthetic polynucleotide

<400>  38
gtggaggcca tcagtattga ctttc                                              25


<210>  39
<211>  25
<212>  DNA
<213>  Artificial Sequence

<220>
<223>  Synthetic polynucleotide

<400>  39
tattgacttt ctcttacttg ctgta                                              25


<210>  40
<211>  25
<212>  DNA
<213>  Artificial Sequence

<220>
<223>  Synthetic polynucleotide

<400>  40
cttgctgtac tatcagcctg ctcgt                                              25


<210>  41
<211>  24
<212>  DNA
<213>  Artificial Sequence

<220>
<223>  Synthetic polynucleotide

<400>  41
tgctcgtttc cacctttaag aatg                                               24


<210>  42
<211>  25
<212>  DNA
<213>  Artificial Sequence

<220>
<223>  Synthetic polynucleotide

<400>  42
ctcaatgtct cctactatcc aaaat                                              25


<210>  43
<211>  25
<212>  DNA
<213>  Artificial Sequence

<220>
<223>  Synthetic polynucleotide

<400>  43
atgtgcatca caggaggctc ttaac                                              25

<210>  44
<211>  25
<212>  DNA
<213>  Artificial Sequence

<220>
<223>  Synthetic polynucleotide

<400>  44
ttgcgctaat gcttgaactc ttttt                                             25


<210>  45
<211>  25
<212>  DNA
<213>  Artificial Sequence

<220>
<223>  Synthetic polynucleotide

<400>  45
gtatgaccaa atcctgcctc attaa                                             25


<210>  46
<211>  25
<212>  DNA
<213>  Artificial Sequence

<220>
<223>  Synthetic polynucleotide

<400>  46
gctagaaacc acctgattct gccag                                             25


<210>  47
<211>  25
<212>  DNA
<213>  Artificial Sequence

<220>
<223>  Synthetic polynucleotide

<400>  47
tgaaagcggg cacctgcagg aagct                                             25


<210>  48
<211>  25
<212>  DNA
<213>  Artificial Sequence

<220>
<223>  Synthetic polynucleotide

<400>  48
gaacacgatg accttctggt ggaga                                             25


<210>  49
<211>  25

<212>  DNA
<213>  Artificial Sequence

<220>
<223>  Synthetic polynucleotide

<400>  49
tgagaaactt catcgctttc caggc                                          25


<210>  50
<211>  25
<212>  DNA
<213>  Artificial Sequence

<220>
<223>  Synthetic polynucleotide

<400>  50
tccaggccca cactgatggc caggc                                          25


<210>  51
<211>  25
<212>  DNA
<213>  Artificial Sequence

<220>
<223>  Synthetic polynucleotide

<400>  51
caggccagca ccagggagat actgc                                          25


<210>  52
<211>  25
<212>  DNA
<213>  Artificial Sequence

<220>
<223>  Synthetic polynucleotide

<400>  52
aagttatctg catcacagtc ttgtg                                          25


<210>  53
<211>  25
<212>  DNA
<213>  Artificial Sequence

<220>
<223>  Synthetic polynucleotide

<400>  53
cagtcttgtg tcttccgaga actat                                          25


<210>  54
<211>  25
<212>  DNA
<213>  Artificial Sequence

<220>
<223>  Synthetic polynucleotide

<400>  54
atctgtgcac tttccataga acttc                                          25


<210>  55
<211>  25
<212>  DNA
<213>  Artificial Sequence

<220>
<223>  Synthetic polynucleotide

<400>  55
aacaacattg cttcctaaac tttca                                          25


<210>  56
<211>  25
<212>  DNA
<213>  Artificial Sequence

<220>
<223>  Synthetic polynucleotide

<400>  56
ggaagttggc tgcacttgat gtttg                                          25


<210>  57
<211>  25
<212>  DNA
<213>  Artificial Sequence

<220>
<223>  Synthetic polynucleotide

<400>  57
gagactgttg ctcaaccatc aggaa                                          25


<210>  58
<211>  25
<212>  DNA
<213>  Artificial Sequence

<220>
<223>  Synthetic polynucleotide

<400>  58
gagactgttg ctcaaccatc aggaa                                          25


<210>  59
<211>  25
<212>  DNA
<213>  Artificial Sequence

<220>
<223>  Synthetic polynucleotide

<400>  59
gcagaaagtc gcatagggtt tttta                                              25


<210>  60
<211>  25
<212>  DNA
<213>  Artificial Sequence

<220>
<223>  Synthetic polynucleotide

<400>  60
gacaatggcg ctgcatgttt ttctt                                              25


<210>  61
<211>  25
<212>  DNA
<213>  Artificial Sequence

<220>
<223>  Synthetic polynucleotide

<400>  61
tatctccttt tctctagtat ttgac                                              25


<210>  62
<211>  25
<212>  DNA
<213>  Artificial Sequence

<220>
<223>  Synthetic polynucleotide

<400>  62
tttgactgtt actgtccttg gcgaa                                              25


<210>  63
<211>  25
<212>  DNA
<213>  Artificial Sequence

<220>
<223>  Synthetic polynucleotide

<400>  63
gttactgtcc ttggcgaatc gataa                                              25


<210>  64
<211>  25
<212>  DNA
<213>  Artificial Sequence

<220>
<223>  Synthetic polynucleotide

<400>  64
ggcgaatcga taatcattgc atagt                                              25

<210>  65
<211>  25
<212>  DNA
<213>  Artificial Sequence

<220>
<223>  Synthetic polynucleotide

<400>  65
tagtgactga aaagcctaag tgcaa                                          25


<210>  66
<211>  25
<212>  DNA
<213>  Artificial Sequence

<220>
<223>  Synthetic polynucleotide

<400>  66
ttcttgtttc tgaacttcgt gccat                                          25


<210>  67
<211>  25
<212>  DNA
<213>  Artificial Sequence

<220>
<223>  Synthetic polynucleotide

<400>  67
acttcgtgcc atattttgtt cctga                                          25


<210>  68
<211>  25
<212>  DNA
<213>  Artificial Sequence

<220>
<223>  Synthetic polynucleotide

<400>  68
tctattagag acttaccctc ctgac                                          25


<210>  69
<211>  25
<212>  DNA
<213>  Artificial Sequence

<220>
<223>  Synthetic polynucleotide

<400>  69
cttaccctcc tgacctgata aaaag                                          25


<210>  70
<211>  25

<212>  DNA
<213>  Artificial Sequence

<220>
<223>  Synthetic polynucleotide

<400>  70
aaagggatac ccatgtctct attaa                                              25


<210>  71
<211>  25
<212>  DNA
<213>  Artificial Sequence

<220>
<223>  Synthetic polynucleotide

<400>  71
tacccatgtc tctattaaca gcttt                                              25


<210>  72
<211>  25
<212>  DNA
<213>  Artificial Sequence

<220>
<223>  Synthetic polynucleotide

<400>  72
ttagttatgc ttaaggagga gttct                                              25


<210>  73
<211>  25
<212>  DNA
<213>  Artificial Sequence

<220>
<223>  Synthetic polynucleotide

<400>  73
taatgtaaga cagttttggc cttta                                              25


<210>  74
<211>  25
<212>  DNA
<213>  Artificial Sequence

<220>
<223>  Synthetic polynucleotide

<400>  74
attatcatga gagatccttc tgaat                                              25


<210>  75
<211>  25
<212>  DNA
<213>  Artificial Sequence

<220>
<223>  Synthetic polynucleotide

<400>  75
ccttctgaat aggatgtctt tctga                                          25


<210>  76
<211>  25
<212>  DNA
<213>  Artificial Sequence

<220>
<223>  Synthetic polynucleotide

<400>  76
tctttctgag ttccactatt cagtt                                          25


<210>  77
<211>  25
<212>  DNA
<213>  Artificial Sequence

<220>
<223>  Synthetic polynucleotide

<400>  77
cagttacaaa actccttaat gctta                                          25


<210>  78
<211>  25
<212>  DNA
<213>  Artificial Sequence

<220>
<223>  Synthetic polynucleotide

<400>  78
tactgtgtac cttttatatt taata                                          25


<210>  79
<211>  25
<212>  DNA
<213>  Artificial Sequence

<220>
<223>  Synthetic polynucleotide

<400>  79
aagtctgtac tgataaaacc cattg                                          25


<210>  80
<211>  25
<212>  DNA
<213>  Artificial Sequence

<220>
<223>  Synthetic polynucleotide

<400>  80
actgataaaa cccattgtgt acaaa                                              25


<210>  81
<211>  25
<212>  DNA
<213>  Artificial Sequence

<220>
<223>  Synthetic polynucleotide

<400>  81
ttctctttat acaagctgag tcata                                              25


<210>  82
<211>  25
<212>  DNA
<213>  Artificial Sequence

<220>
<223>  Synthetic polynucleotide

<400>  82
gattttatat tctggatttg tgttt                                              25


<210>  83
<211>  25
<212>  DNA
<213>  Artificial Sequence

<220>
<223>  Synthetic polynucleotide

<400>  83
agactttggt gatcactttg caaat                                              25


<210>  84
<211>  25
<212>  DNA
<213>  Artificial Sequence

<220>
<223>  Synthetic polynucleotide

<400>  84
tggtgatcac tttgcaaata tttgt                                              25


<210>  85
<211>  25
<212>  DNA
<213>  Artificial Sequence

<220>
<223>  Synthetic polynucleotide

<400>  85
atatttgtta atccttgagt ttgag                                              25

<210>  86
<211>  25
<212>  DNA
<213>  Artificial Sequence

<220>
<223>  Synthetic polynucleotide

<400>  86
tgagtttgag aacctgtctt ttaaa                                          25


<210>  87
<211>  25
<212>  DNA
<213>  Artificial Sequence

<220>
<223>  Synthetic polynucleotide

<400>  87
atactttcaa acaagctagc aaggc                                          25


<210>  88
<211>  25
<212>  DNA
<213>  Artificial Sequence

<220>
<223>  Synthetic polynucleotide

<400>  88
ttatatattt ggttagttct gttta                                          25


<210>  89
<211>  25
<212>  DNA
<213>  Artificial Sequence

<220>
<223>  Synthetic polynucleotide

<400>  89
ctgttgccct tatgagttat tttat                                          25


<210>  90
<211>  25
<212>  DNA
<213>  Artificial Sequence

<220>
<223>  Synthetic polynucleotide

<400>  90
attaccgact gtatgcctac acgga                                          25


<210>  91
<211>  25

<212>  DNA
<213>  Artificial Sequence

<220>
<223>  Synthetic polynucleotide

<400>  91
tacacggagt cggagctgca gattg                                          25


<210>  92
<211>  25
<212>  DNA
<213>  Artificial Sequence

<220>
<223>  Synthetic polynucleotide

<400>  92
ttccccaaca tggtggtggc gcagg                                          25


<210>  93
<211>  25
<212>  DNA
<213>  Artificial Sequence

<220>
<223>  Synthetic polynucleotide

<400>  93
gcagcaggca atcgccagtg gcatc                                          25


<210>  94
<211>  25
<212>  DNA
<213>  Artificial Sequence

<220>
<223>  Synthetic polynucleotide

<400>  94
cagcccagca gataatccat ttcct                                          25


<210>  95
<211>  25
<212>  DNA
<213>  Artificial Sequence

<220>
<223>  Synthetic polynucleotide

<400>  95
cagactccgg ttcactgagg gtgtc                                          25


<210>  96
<211>  25
<212>  DNA
<213>  Artificial Sequence

<220>
<223>  Synthetic polynucleotide

<400>  96
gagggtgtcc tgtataacca gttcc                                          25


<210>  97
<211>  25
<212>  DNA
<213>  Artificial Sequence

<220>
<223>  Synthetic polynucleotide

<400>  97
aaccagttcc tgtcgcaagt ggact                                          25


<210>  98
<211>  25
<212>  DNA
<213>  Artificial Sequence

<220>
<223>  Synthetic polynucleotide

<400>  98
ttcgagaact cggccaagcg gctca                                          25


<210>  99
<211>  25
<212>  DNA
<213>  Artificial Sequence

<220>
<223>  Synthetic polynucleotide

<400>  99
ggcacagcga cgtcaagcgc ttttg                                          25


<210>  100
<211>  25
<212>  DNA
<213>  Artificial Sequence

<220>
<223>  Synthetic polynucleotide

<400>  100
gctcctgaga gcgcgggact tggac                                          25


<210>  101
<211>  25
<212>  DNA
<213>  Artificial Sequence

<220>
<223>  Synthetic polynucleotide

<400>  101
gagcattctt caggtcatct gaacc                                              25


<210>  102
<211>  25
<212>  DNA
<213>  Artificial Sequence

<220>
<223>  Synthetic polynucleotide

<400>  102
gagaaaacat ggtcaacgtc ttgaa                                              25


<210>  103
<211>  25
<212>  DNA
<213>  Artificial Sequence

<220>
<223>  Synthetic polynucleotide

<400>  103
gtgcttatag aatgtgatcc tgcca                                              25


<210>  104
<211>  25
<212>  DNA
<213>  Artificial Sequence

<220>
<223>  Synthetic polynucleotide

<400>  104
cagtttctgc tgtacttgga tgagt                                              25


<210>  105
<211>  25
<212>  DNA
<213>  Artificial Sequence

<220>
<223>  Synthetic polynucleotide

<400>  105
ttggatgagt ccaatgccct gggga                                              25


<210>  106
<211>  25
<212>  DNA
<213>  Artificial Sequence

<220>
<223>  Synthetic polynucleotide

<400>  106
gatgacactc acgtctttgt aatag                                              25

<210>  107
<211>  25
<212>  DNA
<213>  Artificial Sequence

<220>
<223>  Synthetic polynucleotide

<400>  107
gcagaattgg ttaatgtcct ccagg                                              25


<210>  108
<211>  25
<212>  DNA
<213>  Artificial Sequence

<220>
<223>  Synthetic polynucleotide

<400>  108
tgtcctccag gagcgagtgg gtgaa                                              25


<210>  109
<211>  25
<212>  DNA
<213>  Artificial Sequence

<220>
<223>  Synthetic polynucleotide

<400>  109
aactgtgaaa gacttgccac tcaat                                              25


<210>  110
<211>  25
<212>  DNA
<213>  Artificial Sequence

<220>
<223>  Synthetic polynucleotide

<400>  110
gccactcaat atcttaggtg actga                                              25


<210>  111
<211>  25
<212>  DNA
<213>  Artificial Sequence

<220>
<223>  Synthetic polynucleotide

<400>  111
gggttgtttt aggagcatgc cacgg                                              25


<210>  112
<211>  25

<212>  DNA
<213>  Artificial Sequence

<220>
<223>  Synthetic polynucleotide

<400>  112
agtccggaca gacattgtgg gggtc                                          25


<210>  113
<211>  25
<212>  DNA
<213>  Artificial Sequence

<220>
<223>  Synthetic polynucleotide

<400>  113
attaaggttt actctgctgc cttgg                                          25


<210>  114
<211>  25
<212>  DNA
<213>  Artificial Sequence

<220>
<223>  Synthetic polynucleotide

<400>  114
gccttggcag acttacgatc tcaac                                          25


<210>  115
<211>  25
<212>  DNA
<213>  Artificial Sequence

<220>
<223>  Synthetic polynucleotide

<400>  115
cgatctcaac agttcatacg agcag                                          25


<210>  116
<211>  25
<212>  DNA
<213>  Artificial Sequence

<220>
<223>  Synthetic polynucleotide

<400>  116
gagggcatgc tcactagtgg ttagt                                          25


<210>  117
<211>  25
<212>  DNA
<213>  Artificial Sequence

<220>
<223>  Synthetic polynucleotide

<400>  117
gtggttagta agctgtcgac tttgt                                           25


<210>  118
<211>  25
<212>  DNA
<213>  Artificial Sequence

<220>
<223>  Synthetic polynucleotide

<400>  118
gaacatgtac aatttgccac tggga                                           25


<210>  119
<211>  25
<212>  DNA
<213>  Artificial Sequence

<220>
<223>  Synthetic polynucleotide

<400>  119
aatgccagct gcgtgtctag ttttg                                           25


<210>  120
<211>  25
<212>  DNA
<213>  Artificial Sequence

<220>
<223>  Synthetic polynucleotide

<400>  120
agcaaatcac tcttattttt catcc                                           25


<210>  121
<211>  25
<212>  DNA
<213>  Artificial Sequence

<220>
<223>  Synthetic polynucleotide

<400>  121
gtgggagcag tgtacaccaa ctctt                                           25


<210>  122
<211>  25
<212>  DNA
<213>  Artificial Sequence

<220>
<223>  Synthetic polynucleotide

<400>  122
aactcttcct gtatattgcc ttttt                                          25


<210>  123
<211>  25
<212>  DNA
<213>  Artificial Sequence

<220>
<223>  Synthetic polynucleotide

<400>  123
taacagtaac tgactggccc actga                                          25


<210>  124
<211>  25
<212>  DNA
<213>  Artificial Sequence

<220>
<223>  Synthetic polynucleotide

<400>  124
ccacctggga gagaaccacc cgagg                                          25


<210>  125
<211>  25
<212>  DNA
<213>  Artificial Sequence

<220>
<223>  Synthetic polynucleotide

<400>  125
gtacccggct ctgacgggtg gtcat                                          25


<210>  126
<211>  25
<212>  DNA
<213>  Artificial Sequence

<220>
<223>  Synthetic polynucleotide

<400>  126
ttgctgctgg acttttcgaa gggaa                                          25


<210>  127
<211>  25
<212>  DNA
<213>  Artificial Sequence

<220>
<223>  Synthetic polynucleotide

<400>  127
gagcttgctg atgggcgagt tggtc                                          25

<210>  128
<211>  25
<212>  DNA
<213>  Artificial Sequence

<220>
<223>  Synthetic polynucleotide

<400>  128
ggtccatcgc ctttagggta tgtcc                                          25


<210>  129
<211>  25
<212>  DNA
<213>  Artificial Sequence

<220>
<223>  Synthetic polynucleotide

<400>  129
ctttagggta tgtccacctc tgttc                                          25


<210>  130
<211>  25
<212>  DNA
<213>  Artificial Sequence

<220>
<223>  Synthetic polynucleotide

<400>  130
ctctgttcac ttcctccaag gaaaa                                          25


<210>  131
<211>  25
<212>  DNA
<213>  Artificial Sequence

<220>
<223>  Synthetic polynucleotide

<400>  131
caccctcagg cagccagaga ttcat                                          25


<210>  132
<211>  25
<212>  DNA
<213>  Artificial Sequence

<220>
<223>  Synthetic polynucleotide

<400>  132
aacttcccct cggagggtgg agcat                                          25


<210>  133
<211>  25

<212>  DNA
<213>  Artificial Sequence

<220>
<223>  Synthetic polynucleotide

<400>  133
gattattctg tatcctgtgt ttgta                                             25


<210>  134
<211>  25
<212>  DNA
<213>  Artificial Sequence

<220>
<223>  Synthetic polynucleotide

<400>  134
ggtgatcacg aaactcgctg gcatg                                             25


<210>  135
<211>  25
<212>  DNA
<213>  Artificial Sequence

<220>
<223>  Synthetic polynucleotide

<400>  135
ggaggaagac ttggcgtttt cgaca                                             25


<210>  136
<211>  25
<212>  DNA
<213>  Artificial Sequence

<220>
<223>  Synthetic polynucleotide

<400>  136
ggctggggaa tttggctcca gatcc                                             25


<210>  137
<211>  25
<212>  DNA
<213>  Artificial Sequence

<220>
<223>  Synthetic polynucleotide

<400>  137
gcgctttggc accatgagtt ctatg                                             25


<210>  138
<211>  25
<212>  DNA
<213>  Artificial Sequence

<220>
<223>  Synthetic polynucleotide

<400>  138
ttctatgtct ggggccgacg acact                                          25


<210>  139
<211>  25
<212>  DNA
<213>  Artificial Sequence

<220>
<223>  Synthetic polynucleotide

<400>  139
taccactcat cgcggagcaa ggcgc                                          25


<210>  140
<211>  25
<212>  DNA
<213>  Artificial Sequence

<220>
<223>  Synthetic polynucleotide

<400>  140
acccgctctt caagcgcttt aggaa                                          25


<210>  141
<211>  25
<212>  DNA
<213>  Artificial Sequence

<220>
<223>  Synthetic polynucleotide

<400>  141
gcagggtact tcgttcaaga ccggc                                          25


<210>  142
<211>  25
<212>  DNA
<213>  Artificial Sequence

<220>
<223>  Synthetic polynucleotide

<400>  142
cctccagcgt tggccaaatt gtgct                                          25


<210>  143
<211>  25
<212>  DNA
<213>  Artificial Sequence

<220>
<223>  Synthetic polynucleotide

<400>  143
ggctgtgcct tcataggtca tctag                                          25


<210>  144
<211>  25
<212>  DNA
<213>  Artificial Sequence

<220>
<223>  Synthetic polynucleotide

<400>  144
ggggagtggg gtcatttctg tatat                                          25


<210>  145
<211>  25
<212>  DNA
<213>  Artificial Sequence

<220>
<223>  Synthetic polynucleotide

<400>  145
gaaaccagtg tcgccctgga gaagt                                          25


<210>  146
<211>  25
<212>  DNA
<213>  Artificial Sequence

<220>
<223>  Synthetic polynucleotide

<400>  146
aggaggcctg cgagaatggc cgcgg                                          25


<210>  147
<211>  25
<212>  DNA
<213>  Artificial Sequence

<220>
<223>  Synthetic polynucleotide

<400>  147
tgctgtcagt ggcccggggc aaagt                                          25


<210>  148
<211>  25
<212>  DNA
<213>  Artificial Sequence

<220>
<223>  Synthetic polynucleotide

<400>  148
gagggaatcg actttgtgca ccact                                          25

<210>  149
<211>  25
<212>  DNA
<213>  Artificial Sequence

<220>
<223>  Synthetic polynucleotide

<400>  149
tctacacaca gagccgcatt ctcaa                                          25


<210>  150
<211>  25
<212>  DNA
<213>  Artificial Sequence

<220>
<223>  Synthetic polynucleotide

<400>  150
gcattctcaa ggcgcggctg gaata                                          25


<210>  151
<211>  25
<212>  DNA
<213>  Artificial Sequence

<220>
<223>  Synthetic polynucleotide

<400>  151
gggaccagtt ccagattcgt gagaa                                          25


<210>  152
<211>  25
<212>  DNA
<213>  Artificial Sequence

<220>
<223>  Synthetic polynucleotide

<400>  152
agaatgactt tcttaccttc gatgc                                          25


<210>  153
<211>  25
<212>  DNA
<213>  Artificial Sequence

<220>
<223>  Synthetic polynucleotide

<400>  153
gtcgggccat caggggcaag acgga                                          25


<210>  154
<211>  25

<212>  DNA
<213>  Artificial Sequence

<220>
<223>  Synthetic polynucleotide

<400>  154
ggtctttgcc gacaagcggt ttgcc                                          25


<210>  155
<211>  25
<212>  DNA
<213>  Artificial Sequence

<220>
<223>  Synthetic polynucleotide

<400>  155
tcagcagctc tgagtggggc gggtg                                          25


<210>  156
<211>  25
<212>  DNA
<213>  Artificial Sequence

<220>
<223>  Synthetic polynucleotide

<400>  156
ttagttccaa actcactctt cttac                                          25


<210>  157
<211>  25
<212>  DNA
<213>  Artificial Sequence

<220>
<223>  Synthetic polynucleotide

<400>  157
cgacagcact ggccattaca gcaga                                          25


<210>  158
<211>  25
<212>  DNA
<213>  Artificial Sequence

<220>
<223>  Synthetic polynucleotide

<400>  158
ttacagcaga ttccgaaacc cttcc                                          25


<210>  159
<211>  25
<212>  DNA
<213>  Artificial Sequence

<220>
<223>  Synthetic polynucleotide

<400>  159
gagaagtata atcctggtcc ccaag                                          25


<210>  160
<211>  25
<212>  DNA
<213>  Artificial Sequence

<220>
<223>  Synthetic polynucleotide

<400>  160
ggtgaatgcc aaaaactgcc gctcc                                          25


<210>  161
<211>  25
<212>  DNA
<213>  Artificial Sequence

<220>
<223>  Synthetic polynucleotide

<400>  161
gctccttgat gcaccacgtt aagaa                                          25


<210>  162
<211>  25
<212>  DNA
<213>  Artificial Sequence

<220>
<223>  Synthetic polynucleotide

<400>  162
tagcagccct gtcacaagac gagct                                          25


<210>  163
<211>  25
<212>  DNA
<213>  Artificial Sequence

<220>
<223>  Synthetic polynucleotide

<400>  163
tatgatttca ttcacacctc ttttg                                          25


<210>  164
<211>  25
<212>  DNA
<213>  Artificial Sequence

<220>
<223>  Synthetic polynucleotide

<400>  164
tgatggctgt tttcttatcc catgc                                              25


<210>  165
<211>  25
<212>  DNA
<213>  Artificial Sequence

<220>
<223>  Synthetic polynucleotide

<400>  165
tcccatgcct gtacttttca gcggc                                              25


<210>  166
<211>  25
<212>  DNA
<213>  Artificial Sequence

<220>
<223>  Synthetic polynucleotide

<400>  166
gctccttgcc agacatcata ggtca                                              25


<210>  167
<211>  25
<212>  DNA
<213>  Artificial Sequence

<220>
<223>  Synthetic polynucleotide

<400>  167
gaagcagcag ccagcgaaat agaag                                              25


<210>  168
<211>  25
<212>  DNA
<213>  Artificial Sequence

<220>
<223>  Synthetic polynucleotide

<400>  168
aagaggcttt cagattctaa acgaa                                              25


<210>  169
<211>  25
<212>  DNA
<213>  Artificial Sequence

<220>
<223>  Synthetic polynucleotide

<400>  169
   atacatgcgg tggatttttg gggga                                               25

<210>  170
<211>  25
<212>  DNA
<213>  Artificial Sequence

<220>
<223>  Synthetic polynucleotide

<400>  170
tcatctgatg gatcttcaag tgaac                                              25


<210>  171
<211>  25
<212>  DNA
<213>  Artificial Sequence

<220>
<223>  Synthetic polynucleotide

<400>  171
gccaaaaacc agtgcttcag attcg                                              25


<210>  172
<211>  25
<212>  DNA
<213>  Artificial Sequence

<220>
<223>  Synthetic polynucleotide

<400>  172
gcttcagatt cgcagaactc agtga                                              25


<210>  173
<211>  25
<212>  DNA
<213>  Artificial Sequence

<220>
<223>  Synthetic polynucleotide

<400>  173
gaatggaggt gcgaccacca gcagc                                              25


<210>  174
<211>  25
<212>  DNA
<213>  Artificial Sequence

<220>
<223>  Synthetic polynucleotide

<400>  174
accagcagct ctagtgatag tgatg                                              25


<210>  175
<211>  25

<212>  DNA
<213>  Artificial Sequence

<220>
<223>  Synthetic polynucleotide

<400>  175
agagaagatg gtcctcgtga ccgcc                                          25


<210>  176
<211>  25
<212>  DNA
<213>  Artificial Sequence

<220>
<223>  Synthetic polynucleotide

<400>  176
gtgaccgcca gatctgtgtt tggga                                          25


<210>  177
<211>  25
<212>  DNA
<213>  Artificial Sequence

<220>
<223>  Synthetic polynucleotide

<400>  177
gaaggaaact aagacgtgcg agggg                                          25


<210>  178
<211>  25
<212>  DNA
<213>  Artificial Sequence

<220>
<223>  Synthetic polynucleotide

<400>  178
taagacttgt actatgtgtg gccat                                          25


<210>  179
<211>  25
<212>  DNA
<213>  Artificial Sequence

<220>
<223>  Synthetic polynucleotide

<400>  179
gtgtggccat gaactgacat atgaa                                          25


<210>  180
<211>  25
<212>  DNA
<213>  Artificial Sequence

<220>
<223>  Synthetic polynucleotide

<400>  180
tcagtgaagg tcacctggcc tggtt                                          25


<210>  181
<211>  25
<212>  DNA
<213>  Artificial Sequence

<220>
<223>  Synthetic polynucleotide

<400>  181
gttgtgtgca caatgtcatg tctgt                                          25


<210>  182
<211>  25
<212>  DNA
<213>  Artificial Sequence

<220>
<223>  Synthetic polynucleotide

<400>  182
atgtctgtga ttgccttctt acaac                                          25


<210>  183
<211>  25
<212>  DNA
<213>  Artificial Sequence

<220>
<223>  Synthetic polynucleotide

<400>  183
aggtgcagaa gtggtaggtc agcta                                          25


<210>  184
<211>  25
<212>  DNA
<213>  Artificial Sequence

<220>
<223>  Synthetic polynucleotide

<400>  184
ggacaagata ccaaggcaaa cccta                                          25


<210>  185
<211>  25
<212>  DNA
<213>  Artificial Sequence

<220>
<223>  Synthetic polynucleotide

<400>  185
ggctgaactg gattcttaac caaga                                             25


<210>  186
<211>  25
<212>  DNA
<213>  Artificial Sequence

<220>
<223>  Synthetic polynucleotide

<400>  186
gcaatggtgg tgcaccactg taccc                                             25


<210>  187
<211>  25
<212>  DNA
<213>  Artificial Sequence

<220>
<223>  Synthetic polynucleotide

<400>  187
actgtacccc aggttctagt catgt                                             25


<210>  188
<211>  25
<212>  DNA
<213>  Artificial Sequence

<220>
<223>  Synthetic polynucleotide

<400>  188
ttaggacgat ttctgtctcc acgat                                             25


<210>  189
<211>  25
<212>  DNA
<213>  Artificial Sequence

<220>
<223>  Synthetic polynucleotide

<400>  189
cccttccaaa gactgtctcc gttga                                             25


<210>  190
<211>  25
<212>  DNA
<213>  Artificial Sequence

<220>
<223>  Synthetic polynucleotide

<400>  190
tccaaagact gtctccgttg acctt                                             25

<210>  191
<211>  25
<212>  DNA
<213>  Artificial Sequence

<220>
<223>  Synthetic polynucleotide

<400>  191
tgaccttgtc tttttggtat gcctt                                          25


<210>  192
<211>  25
<212>  DNA
<213>  Artificial Sequence

<220>
<223>  Synthetic polynucleotide

<400>  192
ccttgtcttt ttggtatgcc ttggg                                          25


<210>  193
<211>  25
<212>  DNA
<213>  Artificial Sequence

<220>
<223>  Synthetic polynucleotide

<400>  193
ttttggtatg ccttggggtt tctga                                          25


<210>  194
<211>  25
<212>  DNA
<213>  Artificial Sequence

<220>
<223>  Synthetic polynucleotide

<400>  194
ggtatgcctt ggggtttctg ataat                                          25


<210>  195
<211>  25
<212>  DNA
<213>  Artificial Sequence

<220>
<223>  Synthetic polynucleotide

<400>  195
gccttggggt ttctgataat gtgtg                                          25


<210>  196
<211>  25

<212>  DNA
<213>  Artificial Sequence

<220>
<223>  Synthetic polynucleotide

<400>  196
aggtaagtga gcatgtcaaa caaaa                                          25


<210>  197
<211>  25
<212>  DNA
<213>  Artificial Sequence

<220>
<223>  Synthetic polynucleotide

<400>  197
gtcaaacaaa ataggagctc acatg                                          25


<210>  198
<211>  25
<212>  DNA
<213>  Artificial Sequence

<220>
<223>  Synthetic polynucleotide

<400>  198
aggagctcac atggatatat ttatg                                          25


<210>  199
<211>  25
<212>  DNA
<213>  Artificial Sequence

<220>
<223>  Synthetic polynucleotide

<400>  199
atggatatat ttatgtcact gagtt                                          25


<210>  200
<211>  25
<212>  DNA
<213>  Artificial Sequence

<220>
<223>  Synthetic polynucleotide

<400>  200
gagcctttgt ctggtgaaca gttgg                                          25


<210>  201
<211>  25
<212>  DNA
<213>  Artificial Sequence

<220>
<223>  Synthetic polynucleotide

<400>  201
tctccccagg ataaggcggc agagg                                          25


<210>  202
<211>  25
<212>  DNA
<213>  Artificial Sequence

<220>
<223>  Synthetic polynucleotide

<400>  202
ggcggcagag gctacaaatg actat                                          25


<210>  203
<211>  25
<212>  DNA
<213>  Artificial Sequence

<220>
<223>  Synthetic polynucleotide

<400>  203
aatccaggag gccctaaact gctta                                          25


<210>  204
<211>  25
<212>  DNA
<213>  Artificial Sequence

<220>
<223>  Synthetic polynucleotide

<400>  204
gcagatcctg aagttatgct cttga                                          25


<210>  205
<211>  25
<212>  DNA
<213>  Artificial Sequence

<220>
<223>  Synthetic polynucleotide

<400>  205
gttatgctct tgactttaag tttgt                                          25


<210>  206
<211>  25
<212>  DNA
<213>  Artificial Sequence

<220>
<223>  Synthetic polynucleotide

<400>  206
   ggaatttttg ttcccataat tggat                                            25


<210>  207
<211>  25
<212>  DNA
<213>  Artificial Sequence

<220>
<223>  Synthetic polynucleotide

<400>  207
tcaaaaagca acttctgccc tgcaa                                           25


<210>  208
<211>  25
<212>  DNA
<213>  Artificial Sequence

<220>
<223>  Synthetic polynucleotide

<400>  208
ccccactcca tagtctggta ttctg                                           25


<210>  209
<211>  25
<212>  DNA
<213>  Artificial Sequence

<220>
<223>  Synthetic polynucleotide

<400>  209
gtctggtatt ctgagcacta gctta                                           25


<210>  210
<211>  25
<212>  DNA
<213>  Artificial Sequence

<220>
<223>  Synthetic polynucleotide

<400>  210
gagcactagc ttaatatttc ttcac                                           25


<210>  211
<211>  25
<212>  DNA
<213>  Artificial Sequence

<220>
<223>  Synthetic polynucleotide

<400>  211
tatttagaag cctttactca gcccc                                           25

<210>  212
<211>  25
<212>  DNA
<213>  Artificial Sequence

<220>
<223>  Synthetic polynucleotide

<400>  212
ggagcttgag ctctacaggt aaggc                                              25


<210>  213
<211>  25
<212>  DNA
<213>  Artificial Sequence

<220>
<223>  Synthetic polynucleotide

<400>  213
taaggcagag ctaccggtga atgaa                                              25


<210>  214
<211>  25
<212>  DNA
<213>  Artificial Sequence

<220>
<223>  Synthetic polynucleotide

<400>  214
cccctggcat cacatgtgca tgctg                                              25


<210>  215
<211>  25
<212>  DNA
<213>  Artificial Sequence

<220>
<223>  Synthetic polynucleotide

<400>  215
gcatgctgta ggcaggacct gagct                                              25


<210>  216
<211>  25
<212>  DNA
<213>  Artificial Sequence

<220>
<223>  Synthetic polynucleotide

<400>  216
tccgctgcag gttcagatgc accgc                                              25


<210>  217
<211>  25

<212>  DNA
<213>  Artificial Sequence

<220>
<223>  Synthetic polynucleotide

<400>  217
tgcagctgtc cttcagttag ttcac                                          25


<210>  218
<211>  25
<212>  DNA
<213>  Artificial Sequence

<220>
<223>  Synthetic polynucleotide

<400>  218
agcctgagcc gggaactggc tgtgc                                          25


<210>  219
<211>  25
<212>  DNA
<213>  Artificial Sequence

<220>
<223>  Synthetic polynucleotide

<400>  219
agggcttccg tgtactcagg atgta                                          25


<210>  220
<211>  25
<212>  DNA
<213>  Artificial Sequence

<220>
<223>  Synthetic polynucleotide

<400>  220
gtcttctcaa gggactgatc ggcca                                          25


<210>  221
<211>  25
<212>  DNA
<213>  Artificial Sequence

<220>
<223>  Synthetic polynucleotide

<400>  221
gatcggccaa gtatgctttt cttta                                          25


<210>  222
<211>  4518
<212>  DNA
<213>  Homo sapiens

<400> 222
ggagtttatt cataacgcgc tctccaagta tacgtggcaa tgcgttgctg ggttatttta 60

atcattctag gcatcgtttt cctccttatg cctctatcat tcctccctat ctacactaac 120

atcccacgct ctgaacgcgc gcccattaat acccttcttt cctccactct ccctgggact 180

cttgatcaaa gcgcggccct ttccccagcc ttagcgaggc gccctgcagc ctggtacgcg 240

cgtggcgtgg cggtgggcgc gcagtgcgtt ctcggtgtgg agggcagctg ttccgcctgc 300

gatgatttat actcacagga caaggatgcg gtttgtcaaa cagtactgct acggaggagc 360

agcagagaaa gggagagggt ttgagaggga gcaaaagaaa atggtaggcg cgcgtagtta 420

attcatgcgg ctctcttact ctgtttacat cctagagcta gagtgctcgg ctgcccggct 480

gagtctcctc cccaccttcc ccaccctccc caccctcccc ataagcgccc ctcccgggtt 540

cccaaagcag agggcgtggg ggaaaagaaa aaagatcctc tctcgctaat ctccgcccac 600

cggcccttta taatgcgagg gtctggacgg ctgaggaccc ccgagctgtg ctgctcgcgg 660

ccgccaccgc cgggccccgg ccgtccctgg ctcccctcct gcctcgagaa gggcagggct 720

tctcagaggc ttggcgggaa aaagaacgga gggagggatc gcgctgagta taaaagccgg 780

ttttcggggc tttatctaac tcgctgtagt aattccagcg agaggcagag ggagcgagcg 840

ggcggccggc tagggtggaa gagccgggcg agcagagctg cgctgcgggc gtcctgggaa 900

gggagatccg gagcgaatag ggggcttcgc ctctggccca gccctcccgc tgatccccca 960

gccagcggtc cgcaaccctt gccgcatcca cgaaactttg cccatagcag cgggcgggca 1020

ctttgcactg gaacttacaa cacccgagca aggacgcgac tctcccgacg cggggaggct 1080

attctgccca tttggggaca cttccccgcc gctgccagga cccgcttctc tgaaaggctc 1140

tccttgcagc tgcttagacg ctggattttt ttcgggtagt ggaaaaccag cagcctcccg 1200

cgacgatgcc cctcaacgtt agcttcacca acaggaacta tgacctcgac tacgactcgg 1260

tgcagccgta tttctactgc gacgaggagg agaacttcta ccagcagcag cagcagagcg 1320

agctgcagcc cccggcgccc agcgaggata tctggaagaa attcgagctg ctgcccaccc 1380

cgcccctgtc ccctagccgc cgctccgggc tctgctcgcc ctcctacgtt gcggtcacac 1440

ccttctccct tcggggagac aacgacggcg gtggcgggag cttctccacg gccgaccagc 1500

tggagatggt gaccgagctg ctgggaggag acatggtgaa ccagagtttc atctgcgacc 1560

cggacgacga gaccttcatc aaaaacatca tcatccagga ctgtatgtgg agcggcttct 1620

cggccgccgc caagctcgtc tcagagaagc tggcctccta ccaggctgcg cgcaaagaca 1680

gcggcagccc gaaccccgcc cgcggccaca gcgtctgctc cacctccagc ttgtacctgc 1740

aggatctgag cgccgccgcc tcagagtgca tcgacccctc ggtggtcttc ccctaccctc 1800

tcaacgacag cagctcgccc aagtcctgcg cctcgcaaga ctccagcgcc ttctctccgt 1860

cctcggattc tctgctctcc tcgacggagt cctccccgca gggcagcccc gagcccctgg 1920

tgctccatga ggagacaccg cccaccacca gcagcgactc tgaggaggaa caagaagatg 1980

aggaagaaat cgatgttgtt tctgtggaaa agaggcaggc tcctggcaaa aggtcagagt 2040

ctggatcacc ttctgctgga ggccacagca aacctcctca cagcccactg gtcctcaaga 2100

ggtgccacgt ctccacacat cagcacaact acgcagcgcc tccctccact cggaaggact 2160

atcctgctgc caagagggtc aagttggaca gtgtcagagt cctgagacag atcagcaaca 2220

accgaaaatg caccagcccc aggtcctcgg acaccgagga gaatgtcaag aggcgaacac 2280

acaacgtctt ggagcgccag aggaggaacg agctaaaacg gagctttttt gccctgcgtg 2340

accagatccc ggagttggaa aacaatgaaa aggcccccaa ggtagttatc cttaaaaaag 2400

ccacagcata catcctgtcc gtccaagcag aggagcaaaa gctcatttct gaagaggact 2460

tgttgcggaa acgacgagaa cagttgaaac acaaacttga acagctacgg aactcttgtg 2520

cgtaaggaaa agtaaggaaa acgattcctt ctaacagaaa tgtcctgagc aatcacctat 2580

gaacttgttt caaatgcatg atcaaatgca acctcacaac cttggctgag tcttgagact 2640

gaaagattta gccataatgt aaactgcctc aaattggact ttgggcataa aagaactttt 2700

ttatgcttac catctttttt ttttctttaa cagatttgta tttaagaatt gtttttaaaa 2760

aattttaaga tttacacaat gtttctctgt aaatattgcc attaaatgta aataacttta 2820

ataaaacgtt tatagcagtt acacagaatt tcaatcctag tatatagtac ctagtattat 2880

aggtactata aaccctaatt ttttttattt aagtacattt tgctttttaa agttgatttt 2940

tttctattgt ttttagaaaa aataaaataa ctggcaaata tatcattgag ccaaatctta 3000

agttgtgaat gttttgtttc gtttcttccc cctcccaacc accaccatcc ctgtttgttt 3060

tcatcaattg ccccttcaga gggtggtctt aagaaaggca agagttttcc tctgttgaaa 3120

tgggtctggg ggccttaagg tctttaagtt cttggaggtt ctaagatgct tcctggagac 3180

tatgataaca gccagagttg acagttagaa ggaatggcag aaggcaggtg agaaggtgag 3240

aggtaggcaa aggagataca agaggtcaaa ggtagcagtt aagtacacaa agaggcataa 3300

ggactgggga gttgggagga aggtgaggaa gaaactcctg ttactttagt taaccagtgc 3360

cagtcccctg ctcactccaa acccaggaat tctgcccagt tgatggggac acggtgggaa 3420

ccagcttctg ctgccttcac aaccaggcgc cagtcctgtc catgggttat ctcgcaaacc 3480

ccagaggatc tctgggagga atgctactat taaccctatt tcacaaacaa ggaaatagaa 3540

gagctcaaag aggttatgta acttatctgt agccacgcag ataatacaaa gcagcaatct 3600

ggacccattc tgttcaaaac acttaaccct tcgctatcat gccttggttc atctgggtct 3660

aatgtgctga gatcaagaag gtttaggacc taatggacag actcaagtca taacaatgct 3720

aagctctatt tgtgtcccaa gcactcctaa gcattttatc cctaactcta catcaacccc 3780

atgaaggaga tactgttgat ttccccatat tagaagtaga gagggaagct gaggcacaca 3840

aagactcatc cacatgccca agattcactg atagggaaaa gtggaagcga gatttgaacc 3900

caggctgttt actcctaacc tgtccaagcc acctctcaga cgacggtagg aatcagctgg 3960

ctgcttgtga gtacaggagt tacagtccag tgggttatgt tttttaagtc tcaacatcta 4020

agcctggtca ggcatcagtt cccctttttt tgtgatttat tttgttttta ttttgttgtt 4080

cattgtttaa tttttccttt tacaatgaga aggtcaccat cttgactcct accttagcca 4140

tttgttgaat cagactcatg acggctcctg ggaagaagcc agttcagatc ataaaataaa 4200

acatatttat tctttgtcat gggagtcatt attttagaaa ctacaaactc tccttgcttc 4260

catccttttt tacatactca tgacacatgc tcatcctgag tccttgaaaa ggtatttttg 4320

aacatgtgta ttaattataa gcctctgaaa acctatggcc caaaccagaa atgatgttga 4380

ttatataggt aaatgaagga tgctattgct gttctaatta cctcattgtc tcagtctcaa 4440

agtaggtctt cagctccctg tactttggga ttttaatcta ccaccaccca taaatcaata 4500

aataattact ttctttga 4518

<210> 223
<211> 454
<212> PRT
<213> Homo sapiens

<400> 223

Met Asp Phe Phe Arg Val Val Glu Asn Gln Gln Pro Pro Ala Thr Met
1               5                   10                  15

Pro Leu Asn Val Ser Phe Thr Asn Arg Asn Tyr Asp Leu Asp Tyr Asp
            20                  25                  30

Ser Val Gln Pro Tyr Phe Tyr Cys Asp Glu Glu Glu Asn Phe Tyr Gln
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Thr Pro Phe Ser
                85                  90                  95

Leu Arg Gly Asp Asn Asp Gly Gly Gly Gly Ser Phe Ser Thr Ala Asp
            100                 105                 110

Gln Leu Glu Met Val Thr Glu Leu Leu Gly Gly Asp Met Val Asn Gln
        115                 120                 125

Ser Phe Ile Cys Asp Pro Asp Asp Glu Thr Phe Ile Lys Asn Ile Ile
    130                 135                 140

Ile Gln Asp Cys Met Trp Ser Gly Phe Ser Ala Ala Ala Lys Leu Val
145                 150                 155                 160

Ser Glu Lys Leu Ala Ser Tyr Gln Ala Ala Arg Lys Asp Ser Gly Ser
                165                 170                 175

Pro Asn Pro Ala Arg Gly His Ser Val Cys Ser Thr Ser Ser Leu Tyr
            180                 185                 190

Leu Gln Asp Leu Ser Ala Ala Ala Ser Glu Cys Ile Asp Pro Ser Val
        195                 200                 205

Val Phe Pro Tyr Pro Leu Asn Asp Ser Ser Ser Pro Lys Ser Cys Ala
    210                 215                 220

Ser Gln Asp Ser Ser Ala Phe Ser Pro Ser Ser Asp Ser Leu Leu Ser
225                 230                 235                 240

Ser Thr Glu Ser Ser Pro Gln Gly Ser Pro Glu Pro Leu Val Leu His
                245                 250                 255

Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Ala Pro
        275                 280                 285

Gly Lys Arg Ser Glu Ser Gly Ser Pro Ser Ala Gly Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Val Lys Leu Asp Ser Val Arg Val Leu Arg Gln Ile Ser
            340                 345                 350

Asn Asn Arg Lys Cys Thr Ser Pro Arg Ser Ser Asp Thr Glu Glu Asn
        355                 360                 365

Val Lys Arg Arg Thr His Asn Val Leu Glu Arg Gln Arg Arg Asn Glu
    370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Val Gln Ala Glu Glu Gln Lys Leu Ile Ser Glu Glu
            420                 425                 430

Asp Leu Leu Arg Lys Arg Arg Glu Gln Leu Lys His Lys Leu Glu Gln
        435                 440                 445

Leu Arg Asn Ser Cys Ala
    450

<210>  224
<211>  25
<212>  DNA
<213>  Artificial Sequence

<220>
<223>  Synthetic polynucleotide

<400>  224
gcaacaaccg aaaatgcacc agccc                                          25

<210>  225
<211>  25
<212>  DNA
<213>  Artificial Sequence

<220>
<223>  Synthetic polynucleotide

<400>  225
ccccaggtcc tcggacaccg aggag                                          25

<210>  226
<211>  25
<212>  DNA
<213>  Artificial Sequence

<220>
<223>  Synthetic polynucleotide

<400>  226
cgaacacaca acgtcttgga gcgcc                                          25

<210>  227
<211>  25

<212>  DNA
<213>  Artificial Sequence

<220>
<223>  Synthetic polynucleotide

<400>  227
ggagcgccag aggaggaacg agcta                                          25


<210>  228
<211>  25
<212>  DNA
<213>  Artificial Sequence

<220>
<223>  Synthetic polynucleotide

<400>  228
gctaaaacgg agctttttg ccctg                                           25


<210>  229
<211>  25
<212>  DNA
<213>  Artificial Sequence

<220>
<223>  Synthetic polynucleotide

<400>  229
aaaagccaca gcatacatcc tgtcc                                          25


<210>  230
<211>  25
<212>  DNA
<213>  Artificial Sequence

<220>
<223>  Synthetic polynucleotide

<400>  230
catcctgtcc gtccaagcag aggag                                          25


<210>  231
<211>  25
<212>  DNA
<213>  Artificial Sequence

<220>
<223>  Synthetic polynucleotide

<400>  231
catcctgtcc gtccaagcag aggag                                          25


<210>  232
<211>  25
<212>  DNA
<213>  Artificial Sequence

<220>
<223>  Synthetic polynucleotide

<400>  232
gaggacttgt tgcggaaacg acgag                                              25


<210>  233
<211>  25
<212>  DNA
<213>  Artificial Sequence

<220>
<223>  Synthetic polynucleotide

<400>  233
gaacagctac ggaactcttg tgcgt                                              25


<210>  234
<211>  25
<212>  DNA
<213>  Artificial Sequence

<220>
<223>  Synthetic polynucleotide

<400>  234
gaaatgtcct gagcaatcac ctatg                                              25


<210>  235
<211>  25
<212>  DNA
<213>  Artificial Sequence

<220>
<223>  Synthetic polynucleotide

<400>  235
gcaacctcac aaccttggct gagtc                                              25


<210>  236
<211>  1291
<212>  DNA
<213>  Homo sapiens

<400>  236
gagccgaagg tggaggtcaa aggggcgtgg cgttacagag cctctagcgc tgggtgttgg        60

ggacctgacg ctatggagct ctcggagttt tgtgggggac ggctgtgagt ggggggttcc      120

tgctgcggga tgagaacgta gacgccagtg gctcactcgc tcctggcacc ttccctttca       180

ggctccagat ggaccctggg aaggacaaag agggggtgcc ccagccctca gggccgccag        240

caaggaagaa atttgtgata cccctcgacg aggatgaggt ccctcctgga gtggccaagc       300

ccttattccg atctacacag agccttccca ctgtggacac ctcggcccag gcggcccctc       360

agacctacgc cgaatatgcc atctcacagc ctctggaagg ggctggggcc acgtgcccca       420

cagggtcaga gcccctggca ggagagacgc ccaaccaggc cctgaaaccc ggggcaaaat 480

ccaacagcat cattgtgagc cctcggcaga ggggcaatcc cgtactgaag ttcgtgcgca 540

atgtgccctg ggaatttggc gacgtaattc ccgactatgt gctgggccag agcacctgtg 600

ccctgttcct cagcctccgc taccacaacc tgcacccaga ctacatccat gggcggctgc 660

agagcctggg gaagaacttc gccttgcggg tcctgcttgt ccaggtggat gtgaaagatc 720

cccagcaggc cctcaaggag ctggctaaga tgtgtatcct ggccgactgc acattgatcc 780

tcgcctggag ccccgaggaa gctgggcggt acctggagac ctacaaggcc tatgagcaga 840

aaccagcgga cctcctgatg gagaagctag agcaggactt cgtctcccgg gtgactgaat 900

gtctgaccac cgtgaagtca gtcaacaaaa cggacagtca gaccctcctg accacatttg 960

gatctctgga acagctcatc gccgcatcaa gagaagatct ggccttatgc ccaggcctgg 1020

gccctcagaa agtaagagct ctgggaaaga acccaaggag ttgggggaag gagagagccc 1080

caaataaaca caacctgaga ccccaaagtt ttaaggtgaa aaaagaacca aagaccagac 1140

acagtggctt ccgcctgtaa tcccaacatt ttgggaggcc aaggcgggag gactgcttga 1200

ggccagaagt tggagaccag cctgggcaag tggacacctc atttttacta aaaataaaaa 1260

aaactagctg ggcaaaaaaa aaaaaaaaaa a 1291

<210> 237
<211> 323
<212> PRT
<213> Homo sapiens

<400> 237

Met Asp Pro Gly Lys Asp Lys Glu Gly Val Pro Gln Pro Ser Gly Pro
1               5                   10                  15

Pro Ala Arg Lys Lys Phe Val Ile Pro Leu Asp Glu Asp Glu Val Pro
            20                  25                  30

Pro Gly Val Ala Lys Pro Leu Phe Arg Ser Thr Gln Ser Leu Pro Thr
        35                  40                  45

Val Asp Thr Ser Ala Gln Ala Ala Pro Gln Thr Tyr Ala Glu Tyr Ala
    50                  55                  60

Ile Ser Gln Pro Leu Glu Gly Ala Gly Ala Thr Cys Pro Thr Gly Ser
65                  70                  75                  80

Glu Pro Leu Ala Gly Glu Thr Pro Asn Gln Ala Leu Lys Pro Gly Ala
                85                  90                  95

Lys Ser Asn Ser Ile Ile Val Ser Pro Arg Gln Arg Gly Asn Pro Val

            100                 105                 110

Leu Lys Phe Val Arg Asn Val Pro Trp Glu Phe Gly Asp Val Ile Pro
        115                 120                 125

Asp Tyr Val Leu Gly Gln Ser Thr Cys Ala Leu Phe Leu Ser Leu Arg
    130                 135                 140

Tyr His Asn Leu His Pro Asp Tyr Ile His Gly Arg Leu Gln Ser Leu
145                 150                 155                 160

Gly Lys Asn Phe Ala Leu Arg Val Leu Leu Val Gln Val Asp Val Lys
                165                 170                 175

Asp Pro Gln Gln Ala Leu Lys Glu Leu Ala Lys Met Cys Ile Leu Ala
            180                 185                 190

Asp Cys Thr Leu Ile Leu Ala Trp Ser Pro Glu Glu Ala Gly Arg Tyr
        195                 200                 205

Leu Glu Thr Tyr Lys Ala Tyr Glu Gln Lys Pro Ala Asp Leu Leu Met
    210                 215                 220

Glu Lys Leu Glu Gln Asp Phe Val Ser Arg Val Thr Glu Cys Leu Thr
225                 230                 235                 240

Thr Val Lys Ser Val Asn Lys Thr Asp Ser Gln Thr Leu Leu Thr Thr
                245                 250                 255

Phe Gly Ser Leu Glu Gln Leu Ile Ala Ala Ser Arg Glu Asp Leu Ala
            260                 265                 270

Leu Cys Pro Gly Leu Gly Pro Gln Lys Val Arg Ala Leu Gly Lys Asn
        275                 280                 285

Pro Arg Ser Trp Gly Lys Glu Arg Ala Pro Asn Lys His Asn Leu Arg
    290                 295                 300

Pro Gln Ser Phe Lys Val Lys Lys Glu Pro Lys Thr Arg His Ser Gly
305                 310                 315                 320

Phe Arg Leu

<210> 238
<211> 25
<212> DNA
<213> Artificial Sequence

<220>
<223>  Synthetic polynucleotide

<400>  238
aagatctggc cttatgccca ggcct                                            25


<210>  239
<211>  25
<212>  DNA
<213>  Artificial Sequence

<220>
<223>  Synthetic polynucleotide

<400>  239
ccctcagaaa gcccggaggc tgttt                                            25


<210>  240
<211>  25
<212>  DNA
<213>  Artificial Sequence

<220>
<223>  Synthetic polynucleotide

<400>  240
ctcagaaagc ccggaggctg tttga                                            25


<210>  241
<211>  25
<212>  DNA
<213>  Artificial Sequence

<220>
<223>  Synthetic polynucleotide

<400>  241
gaaagcccgg aggctgtttg atgtc                                            25


<210>  242
<211>  25
<212>  DNA
<213>  Artificial Sequence

<220>
<223>  Synthetic polynucleotide

<400>  242
aagcccggag gctgtttgat gtcct                                            25


<210>  243
<211>  25
<212>  DNA
<213>  Artificial Sequence

<220>
<223>  Synthetic polynucleotide

<400>  243
ggctgtttga tgtcctgcac gagcc                                              25


<210>  244
<211>  25
<212>  DNA
<213>  Artificial Sequence

<220>
<223>  Synthetic polynucleotide

<400>  244
tgcacgagcc cttcttgaaa gtacc                                              25


<210>  245
<211>  25
<212>  DNA
<213>  Artificial Sequence

<220>
<223>  Synthetic polynucleotide

<400>  245
gcccttcttg aaagtaccct gatga                                              25


<210>  246
<211>  25
<212>  DNA
<213>  Artificial Sequence

<220>
<223>  Synthetic polynucleotide

<400>  246
ccttcttgaa agtaccctga tgacc                                              25


<210>  247
<211>  25
<212>  DNA
<213>  Artificial Sequence

<220>
<223>  Synthetic polynucleotide

<400>  247
ttcttgaaag taccctgatg acccc                                              25


<210>  248
<211>  25
<212>  DNA
<213>  Artificial Sequence

<220>
<223>  Synthetic polynucleotide

<400>  248
tcttgaaagt accctgatga cccca                                              25

<210>  249
<211>  1220
<212>  DNA
<213>  Homo sapiens

<400>  249
atgcacacat acgcgcgcgc acatgcacat aggcacacag agatacgcgc acacacacac      60

acaaacgcac tcagatttcc cggaccctgg ttttcctcct gtgacccttt cggggccggg     120

ctctcaccct aaagaagcag ccccgccctg gggtggcccc accctctctt gggacctgtc     180

ataagtcgga ccccgggcgc ccggctgcgc agtcccagcc gccttcccca ggcagtggaa     240

ccttcgggct cctgagcttc aggatggttc gtactaagac atggaccctg aagaagcact     300

ttgttggcta tcctactaat agtgactttg agttgaagac agctgagctc ccacccttaa     360

aaaatggaga ggtcctgctt gaagctttgt tcctcaccgt ggatccctac atgagagtgg     420

cagccaaaag attgaaggaa ggtgatacaa tgatggggca gcaagtggcc aaagttgtgg     480

aaagtaaaaa tgtagcccta ccaaaaggaa ctattgtact ggcttctcca ggctggacaa     540

cgcactccat ttctgatggg aaagatctgg aaaagctgct gacagagtgg ccagacacaa     600

taccactgtc tttggctctg ggacagttgg gcatgccagg cctgactgcc tactttggcc     660

tacttgaaat ctgtggtgtg aagggtggag aaacagtgat ggttaatgca gcagctggag     720

ctgtgggctc agtcgtgggg cagattgcaa agctcaaggg ctgcaaagtt gttggagcag     780

tagggtctga tgaaaaggtt gcctaccttc aaaagcttgg atttgatgtc gtctttaact     840

acaagacggt agagtctttg gaagaaacct tgaagaaagc gtctcctgat ggttatgatt     900

gttattttga taatgtaggt ggagagtttt caaacactgt tatcggccag atgaagaaat     960

ttggaaggat tgccatatgt ggagccatct ctacatataa cagaaccggc ccacttcccc    1020

caggcccacc cccagagatt gttatctatc aggagcttcg catggaagct tttgtcgtct    1080

accgctggca aggagatgcc cgccaaaaag ctctgaagga cttgctgaaa tgggtcttag    1140

agatcaaaag agaaaatgaa gaagattgaa gcttcaaagc agaaaaatga agggaatatg    1200

tatcattcac cattacctta                                                1220


<210>  250
<211>  301
<212>  PRT
<213>  Homo sapiens

<400>  250

Met Val Arg Thr Lys Thr Trp Thr Leu Lys Lys His Phe Val Gly Tyr
1               5                   10                  15


Pro Thr Asn Ser Asp Phe Glu Leu Lys Thr Ala Glu Leu Pro Pro Leu

20                  25                  30

Lys Asn Gly Glu Val Leu Leu Glu Ala Leu Phe Leu Thr Val Asp Pro
        35                  40                  45

Tyr Met Arg Val Ala Ala Lys Arg Leu Lys Glu Gly Asp Thr Met Met
    50                  55                  60

Gly Gln Gln Val Ala Lys Val Val Glu Ser Lys Asn Val Ala Leu Pro
65                  70                  75                  80

Lys Gly Thr Ile Val Leu Ala Ser Pro Gly Trp Thr Thr His Ser Ile
                85                  90                  95

Ser Asp Gly Lys Asp Leu Glu Lys Leu Leu Thr Glu Trp Pro Asp Thr
            100                 105                 110

Ile Pro Leu Ser Leu Ala Leu Gly Thr Val Gly Met Pro Gly Leu Thr
        115                 120                 125

Ala Tyr Phe Gly Leu Leu Glu Ile Cys Gly Val Lys Gly Gly Glu Thr
    130                 135                 140

Val Met Val Asn Ala Ala Ala Gly Ala Val Gly Ser Val Val Gly Gln
145                 150                 155                 160

Ile Ala Lys Leu Lys Gly Cys Lys Val Val Gly Ala Val Gly Ser Asp
                165                 170                 175

Glu Lys Val Ala Tyr Leu Gln Lys Leu Gly Phe Asp Val Val Phe Asn
            180                 185                 190

Tyr Lys Thr Val Glu Ser Leu Glu Glu Thr Leu Lys Lys Ala Ser Pro
        195                 200                 205

Asp Gly Tyr Asp Cys Tyr Phe Asp Asn Val Gly Gly Glu Phe Ser Asn
    210                 215                 220

Thr Val Ile Gly Gln Met Lys Lys Phe Gly Arg Ile Ala Ile Cys Gly
225                 230                 235                 240

Ala Ile Ser Thr Tyr Asn Arg Thr Gly Pro Leu Pro Pro Gly Pro Pro
                245                 250                 255

Pro Glu Ile Val Ile Tyr Gln Glu Leu Arg Met Glu Ala Phe Val Val
            260                 265                 270

Tyr Arg Trp Gln Gly Asp Ala Arg Gln Lys Ala Leu Lys Asp Leu Leu
        275                 280                 285

Lys Trp Val Leu Glu Ile Lys Arg Glu Asn Glu Glu Asp
    290                 295                 300

<210>  251
<211>  25
<212>  DNA
<213>  Artificial Sequence

<220>
<223>  Synthetic polynucleotide

<400>  251
gcttggattt gatgtcgtct ttaac                                          25

<210>  252
<211>  25
<212>  DNA
<213>  Artificial Sequence

<220>
<223>  Synthetic polynucleotide

<400>  252
gaaagcgtct cctgatggtt atgat                                          25

<210>  253
<211>  25
<212>  DNA
<213>  Artificial Sequence

<220>
<223>  Synthetic polynucleotide

<400>  253
ttcaaacact gttatcggcc agatg                                          25

<210>  254
<211>  25
<212>  DNA
<213>  Artificial Sequence

<220>
<223>  Synthetic polynucleotide

<400>  254
atgtggagcc atctctacat ataac                                          25

<210>  255
<211>  25
<212>  DNA
<213>  Artificial Sequence

<220>
<223>  Synthetic polynucleotide

<400>  255
tacatataac agaaccggcc cactt                                              25


<210>  256
<211>  25
<212>  DNA
<213>  Artificial Sequence

<220>
<223>  Synthetic polynucleotide

<400>  256
cccaccccca gagattgtta tctat                                              25


<210>  257
<211>  25
<212>  DNA
<213>  Artificial Sequence

<220>
<223>  Synthetic polynucleotide

<400>  257
gttatctatc aggagcttcg catgg                                              25


<210>  258
<211>  25
<212>  DNA
<213>  Artificial Sequence

<220>
<223>  Synthetic polynucleotide

<400>  258
catggaagct tttgtcgtct accgc                                              25


<210>  259
<211>  25
<212>  DNA
<213>  Artificial Sequence

<220>
<223>  Synthetic polynucleotide

<400>  259
taccgctggc aaggagatgc ccgcc                                              25


<210>  260
<211>  25
<212>  DNA
<213>  Artificial Sequence

<220>
<223>  Synthetic polynucleotide

<400>  260
aggatttgaa aacatgccag ctgca                                              25

<210>  261
<211>  25
<212>  DNA
<213>  Artificial Sequence

<220>
<223>  Synthetic polynucleotide

<400>  261
gaggacacat ggaatctgga ggcca                                          25


<210>  262
<211>  2520
<212>  DNA
<213>  Homo sapiens

<400>  262
gcgcgcctgc gtactgcggt cagagggccg aggcctgggg gcgagctggg gtcgtgcagt      60

acagcctctt tccggcaaat cacgcgagat ttcgttcacc cgggctccac agggagtatt     120

ttatacagaa atcttgtgaa accactgccc aaccagagca atgattgttc aaagagtggt     180

attgaattct cgacctggaa aaaatggtaa tccagtggca gagaatttcc gaatggaaga     240

agtctattta ccagataata ttaatgaagg acaagtacaa gttagaactc tttatctttc     300

tgtggatcct tacatgcgtt gtagaatgaa tgaagacact ggcactgatt atataacacc     360

ttggcagcta tctcaagtcg ttgatggagg aggtattgga attatagaag aaagcaaaca     420

cacaaatttg actaaaggcg attttgtgac ttctttctat tggccctggc aaaccaaggt     480

tattctggat ggaaatagcc ttgaaaaggt agacccacaa cttgtggatg gacacctttc     540

atattttctt ggagctatag gtatgcctgg tttgacttcc ttgattggga tacaggaaaa     600

aggtcatata actgctggat ctaataagac aatggttgtc agtggggccg caggtgcctg     660

tggatctgtg gctgggcaga ttggccattt cttaggttgt tccagagtgg tgggaatttg     720

tggaacacat gagaaatgca tcctcttgac ctcagaactg ggctttgatg ctgcaattaa     780

ttataaaaaa gacaatgtgg cagaacagct ccgtgaatca tgcccagctg gagtggatgt     840

ttattttgac aatgttggtg gtaacatcag tgatacagtg ataagtcaga tgaatgagaa     900

cagccacatc atcctgtgtg gtcaaatttc tcagtacaac aaagatgtgc cttatcctcc     960

cccgctatcc cctgctatag aggcaatcca gaaagaaaga aacatcacaa gggaaagatt    1020

tctggtatta aattataaag acaaatttga gcctggcatt ctacagctga gtcagtggtt    1080

taaagaagga aagctaaaga ttaaagagac ggtaataaat gggttggaaa acatgggagc    1140

tgcattccag tccatgatga caggaggtaa cattggaaag cagatagttt gcatttcaga    1200

agaaatctct ttgtaattgc tgtaaatgtc atcaaggcaa tcatagattt cttttccatt    1260

ttgcatattt tcaaagatat gttaaaaaat ccttagacta tacatagctc ttgatttaaa    1320

tgtgatcata ggtgttattt ttagttgcat agggtatttg atacaatcat taatggatca 1380

tacacaatag gtttttaaaa attaataact tttagtaatt acttttatta atttaaaata 1440

gaacgcttga gaggcacttt gtaaagattt gttaaactgg aaacgtttta catgatctga 1500

tacaaccatt aatgaatcat acacaatagg ttttttaaaa ttaatattaa taacttttat 1560

taatttaaaa tagaatgctt aaaataaaat agaatgcttg agaggcactg agtaaagatt 1620

tgttgaactg gaaatgtttt acatgattct taaactgaaa cttggtgtaa aaatagaatt 1680

gagatggcct ttttttcaca ttgtagactg aaaagagact taatggtatg atgtgtacat 1740

agggactggg ggcaggattg ggggtttcgg agcttgtgta acagtttttg ggataggaga 1800

ccagcggttt tgggttggga ttagtatggg aagataaata acttaggttg gttaagttga 1860

caagatttac tccagaggat catctctttg tatttgccaa ataatttact gtatagccta 1920

aaaactccat atatattgag aaaagcatat gtttatttta ggttagcagg cacatactgt 1980

caagttgtaa agattgagag ggcaaacaga tgtaaacatc acttgtaggt gattaaaaag 2040

attgacagcc gggcctgatg tctcaagcct gtaatcctag cactttggga ggcagaggcg 2100

ggcagatcac ttgaggtcag gagttcaaga ccagcctggc caacacggtg aaaccccatc 2160

tctctaaaaa tacaaaaatt agctgggcgt ggtggcacac gcctgtaatc ccagctactc 2220

aggaggctga ggctggagaa tcatttgaac ctgggagggg gaggttgccg tgagctgagc 2280

ttgcaccatt gcactccagc ctgggcgaca agagtgaaat tccatctcaa aaaacaaaaa 2340

cagattgaca acaaagacag tttcagaaaa tgacaggact gggcaaatta acaaatgttt 2400

gtaaacatga atgttcagga actactgatg tacctcaaaa gtttgtttta ttaattgtac 2460

tcaaccctcg cagaacagta aaactgaaga ttattgtttc tgaaaaaaaa aaaaaaaaaa 2520

<210> 263
<211> 351
<212> PRT
<213> Homo sapiens

<400> 263

Met Ile Val Gln Arg Val Val Leu Asn Ser Arg Pro Gly Lys Asn Gly
1               5                   10                  15

Asn Pro Val Ala Glu Asn Phe Arg Met Glu Glu Val Tyr Leu Pro Asp
            20                  25                  30

Asn Ile Asn Glu Gly Gln Val Gln Val Arg Thr Leu Tyr Leu Ser Val
        35                  40                  45

Asp Pro Tyr Met Arg Cys Arg Met Asn Glu Asp Thr Gly Thr Asp Tyr
    50                  55                  60

Ile Thr Pro Trp Gln Leu Ser Gln Val Val Asp Gly Gly Gly Ile Gly
65                  70                  75                  80

Ile Ile Glu Glu Ser Lys His Thr Asn Leu Thr Lys Gly Asp Phe Val
                85                  90                  95

Thr Ser Phe Tyr Trp Pro Trp Gln Thr Lys Val Ile Leu Asp Gly Asn
            100                 105                 110

Ser Leu Glu Lys Val Asp Pro Gln Leu Val Asp Gly His Leu Ser Tyr
        115                 120                 125

Phe Leu Gly Ala Ile Gly Met Pro Gly Leu Thr Ser Leu Ile Gly Ile
    130                 135                 140

Gln Glu Lys Gly His Ile Thr Ala Gly Ser Asn Lys Thr Met Val Val
145                 150                 155                 160

Ser Gly Ala Ala Gly Ala Cys Gly Ser Val Ala Gly Gln Ile Gly His
                165                 170                 175

Phe Leu Gly Cys Ser Arg Val Val Gly Ile Cys Gly Thr His Glu Lys
            180                 185                 190

Cys Ile Leu Leu Thr Ser Glu Leu Gly Phe Asp Ala Ala Ile Asn Tyr
        195                 200                 205

Lys Lys Asp Asn Val Ala Glu Gln Leu Arg Glu Ser Cys Pro Ala Gly
    210                 215                 220

Val Asp Val Tyr Phe Asp Asn Val Gly Gly Asn Ile Ser Asp Thr Val
225                 230                 235                 240

Ile Ser Gln Met Asn Glu Asn Ser His Ile Ile Leu Cys Gly Gln Ile
                245                 250                 255

Ser Gln Tyr Asn Lys Asp Val Pro Tyr Pro Pro Pro Leu Ser Pro Ala
            260                 265                 270

Ile Glu Ala Ile Gln Lys Glu Arg Asn Ile Thr Arg Glu Arg Phe Leu
        275                 280                 285

Val Leu Asn Tyr Lys Asp Lys Phe Glu Pro Gly Ile Leu Gln Leu Ser
    290                 295                 300

Gln Trp Phe Lys Glu Gly Lys Leu Lys Ile Lys Glu Thr Val Ile Asn

305                 310                 315                 320

Gly Leu Glu Asn Met Gly Ala Ala Phe Gln Ser Met Met Thr Gly Gly
                325                 330                 335

Asn Ile Gly Lys Gln Ile Val Cys Ile Ser Glu Glu Ile Ser Leu
            340                 345                 350

<210>  264
<211>  25
<212>  DNA
<213>  Artificial Sequence

<220>
<223>  Synthetic polynucleotide

<400>  264
ggaaagcaga tagtttgcat ttcag                                          25

<210>  265
<211>  25
<212>  DNA
<213>  Artificial Sequence

<220>
<223>  Synthetic polynucleotide

<400>  265
aaaatcctta gactatacat agctc                                          25

<210>  266
<211>  25
<212>  DNA
<213>  Artificial Sequence

<220>
<223>  Synthetic polynucleotide

<400>  266
agactataca tagctcttga tttaa                                          25

<210>  267
<211>  25
<212>  DNA
<213>  Artificial Sequence

<220>
<223>  Synthetic polynucleotide

<400>  267
gatcataggt gttattttta gttgc                                          25

<210>  268
<211>  25
<212>  DNA
<213>  Artificial Sequence

<220>
<223>  Synthetic polynucleotide

<400>  268
ttagttgcat agggtatttg ataca                                          25


<210>  269
<211>  25
<212>  DNA
<213>  Artificial Sequence

<220>
<223>  Synthetic polynucleotide

<400>  269
gatacaatca ttaatggatc ataca                                          25


<210>  270
<211>  25
<212>  DNA
<213>  Artificial Sequence

<220>
<223>  Synthetic polynucleotide

<400>  270
ggatcataca caataggttt ttaaa                                          25


<210>  271
<211>  25
<212>  DNA
<213>  Artificial Sequence

<220>
<223>  Synthetic polynucleotide

<400>  271
agaacgcttg agaggcactt tgtaa                                          25


<210>  272
<211>  25
<212>  DNA
<213>  Artificial Sequence

<220>
<223>  Synthetic polynucleotide

<400>  272
ggaaacgttt tacatgatct gatac                                          25


<210>  273
<211>  25
<212>  DNA
<213>  Artificial Sequence

<220>
<223>  Synthetic polynucleotide

<400>  273
tgatctgata caaccattaa tgaat                                          25


<210>  274
<211>  25
<212>  DNA
<213>  Artificial Sequence

<220>
<223>  Synthetic polynucleotide

<400>  274
gaatcataca caataggttt tttaa                                          25


<210>  275
<211>  20
<212>  DNA
<213>  Artificial Sequence

<220>
<223>  Synthetic polynucleotide

<400>  275
gcagtctttc ccttgaggct                                                20


<210>  276
<211>  20
<212>  DNA
<213>  Artificial Sequence

<220>
<223>  Synthetic polynucleotide

<400>  276
tcgggaatta cgtcgccaaa                                                20


<210>  277
<211>  23
<212>  DNA
<213>  Artificial Sequence

<220>
<223>  Synthetic polynucleotide

<400>  277
taccccgagc agttctccta cat                                            23


<210>  278
<211>  25
<212>  DNA
<213>  Artificial Sequence

<220>
<223>  Synthetic polynucleotide

<400>  278
gccagttcct tggacaccag gtctg                                          25

<210>  279
<211>  20
<212>  DNA
<213>  Artificial Sequence

<220>
<223>  Synthetic polynucleotide

<400>  279
gcatccacct ggagtaccct                                                    20


<210>  280
<211>  20
<212>  DNA
<213>  Artificial Sequence

<220>
<223>  Synthetic polynucleotide

<400>  280
tcccaggacc ttttggtggt                                                    20


<210>  281
<211>  25
<212>  DNA
<213>  Artificial Sequence

<220>
<223>  Synthetic polynucleotide

<400>  281
tgaggcagaa aataaggaga gtgaa                                              25


<210>  282
<211>  25
<212>  DNA
<213>  Artificial Sequence

<220>
<223>  Synthetic polynucleotide

<400>  282
tggggaagtg aagtgtaaga agagt                                              25


<210>  283
<211>  21
<212>  DNA
<213>  Artificial Sequence

<220>
<223>  Synthetic polynucleotide

<400>  283
aggcagaaaa taaggagagt g                                                  21


<210>  284

<211>  21
<212>  DNA
<213>  Artificial Sequence

<220>
<223>  Synthetic polynucleotide

<400>  284
aagcggatgg atgatagtga g                                                21


<210>  285
<211>  27
<212>  DNA
<213>  Artificial Sequence

<220>
<223>  Synthetic polynucleotide

<400>  285
gcgaggtaga gtcaaggaga gtggtct                                          27


<210>  286
<211>  26
<212>  DNA
<213>  Artificial Sequence

<220>
<223>  Synthetic polynucleotide

<400>  286
gcgcaaagtt cgttctgctg ctcaag                                           26


<210>  287
<211>  25
<212>  DNA
<213>  Artificial Sequence

<220>
<223>  Synthetic polynucleotide

<400>  287
gccgagatga tggagatgaa gatta                                            25


<210>  288
<211>  25
<212>  DNA
<213>  Artificial Sequence

<220>
<223>  Synthetic polynucleotide

<400>  288
gcgcagtagg tatggattta tgtga                                            25


<210>  289
<211>  27
<212>  DNA
<213>  Artificial Sequence

<220>
<223>  Synthetic polynucleotide

<400>  289
gtagtcattg atgatctgcc caaagag                                          27


<210>  290
<211>  27
<212>  DNA
<213>  Artificial Sequence

<220>
<223>  Synthetic polynucleotide

<400>  290
gagccacaca agctattttc tttctca                                          27


<210>  291
<211>  27
<212>  DNA
<213>  Artificial Sequence

<220>
<223>  Synthetic polynucleotide

<400>  291
gcagttcaag gatctaaaga gcatcaa                                          27


<210>  292
<211>  27
<212>  DNA
<213>  Artificial Sequence

<220>
<223>  Synthetic polynucleotide

<400>  292
aacatctgta tgctgaggat gaacaca                                          27


<210>  293
<211>  23
<212>  DNA
<213>  Artificial Sequence

<220>
<223>  Synthetic polynucleotide

<400>  293
tgatgatgag actaaaacaa aat                                              23


<210>  294
<211>  23
<212>  DNA
<213>  Artificial Sequence

<220>
<223>  Synthetic polynucleotide

<400>  294
tgaagaagaa agaataaaat aat                                              23


<210>  295
<211>  25
<212>  DNA
<213>  Artificial Sequence

<220>
<223>  Synthetic polynucleotide

<400>  295
ttttccagtc ccattatttt cacag                                            25


<210>  296
<211>  25
<212>  DNA
<213>  Artificial Sequence

<220>
<223>  Synthetic polynucleotide

<400>  296
agtcccttct ttcctttcag tctcc                                            25


<210>  297
<211>  25
<212>  DNA
<213>  Artificial Sequence

<220>
<223>  Synthetic polynucleotide

<400>  297
tcaaggcaaa gagaaaaaga gtatt                                            25


<210>  298
<211>  25
<212>  DNA
<213>  Artificial Sequence

<220>
<223>  Synthetic polynucleotide

<400>  298
agacaccaaa gtaagaccac agatt                                            25


<210>  299
<211>  25
<212>  DNA
<213>  Artificial Sequence

<220>
<223>  Synthetic polynucleotide

<400>  299
agacttggag acttcttcgg agacc                                            25

<210>  300
<211>  25
<212>  DNA
<213>  Artificial Sequence

<220>
<223>  Synthetic polynucleotide

<400>  300
taaaacacag gcatcaatca aaata                                          25


<210>  301
<211>  21
<212>  DNA
<213>  Artificial Sequence

<220>
<223>  Synthetic polynucleotide

<400>  301
gaactgggtg atgcggatgc t                                              21


<210>  302
<211>  21
<212>  DNA
<213>  Artificial Sequence

<220>
<223>  Synthetic polynucleotide

<400>  302
gctggaagag gaaggagaga a                                              21


<210>  303
<211>  27
<212>  DNA
<213>  Artificial Sequence

<220>
<223>  Synthetic polynucleotide

<400>  303
acaaggtgaa accccgtctc tactaaa                                        27


<210>  304
<211>  27
<212>  DNA
<213>  Artificial Sequence

<220>
<223>  Synthetic polynucleotide

<400>  304
ttcttccacc taagctttgc cataaat                                        27


<210>  305

<211>  27
<212>  DNA
<213>  Artificial Sequence

<220>
<223>  Synthetic polynucleotide

<400>  305
atcaaaaaca tcatcatcca ggactgt                                          27


<210>  306
<211>  27
<212>  DNA
<213>  Artificial Sequence

<220>
<223>  Synthetic polynucleotide

<400>  306
tctcaggact ctgacactgt ccaactt                                          27


<210>  307
<211>  27
<212>  DNA
<213>  Artificial Sequence

<220>
<223>  Synthetic polynucleotide

<400>  307
caggatggtt cgtactaaga catggac                                          27


<210>  308
<211>  27
<212>  DNA
<213>  Artificial Sequence

<220>
<223>  Synthetic polynucleotide

<400>  308
gctttcttca aggtttcttc caaagac                                          27


<210>  309
<211>  27
<212>  DNA
<213>  Artificial Sequence

<220>
<223>  Synthetic polynucleotide

<400>  309
catgcgttgt agaatgaatg aagacac                                          27


<210>  310
<211>  27
<212>  DNA
<213>  Artificial Sequence

<220>
<223>  Synthetic polynucleotide

<400>  310
tgtactgaga aatttgacca cacagga                                          27


<210>  311
<211>  26
<212>  DNA
<213>  Artificial Sequence

<220>
<223>  Synthetic polynucleotide

<400>  311
agcaggttaa tggataattt cttgat                                           26


<210>  312
<211>  26
<212>  DNA
<213>  Artificial Sequence

<220>
<223>  Synthetic polynucleotide

<400>  312
ggtcctttgt aaaaaccatt acactt                                           26


<210>  313
<211>  27
<212>  DNA
<213>  Artificial Sequence

<220>
<223>  Synthetic polynucleotide

<400>  313
cgtttccttt tattttggag ctaatgc                                          27


<210>  314
<211>  27
<212>  DNA
<213>  Artificial Sequence

<220>
<223>  Synthetic polynucleotide

<400>  314
aaggaagtca ctccaaggta cacactg                                          27


<210>  315
<211>  27
<212>  DNA
<213>  Artificial Sequence

<220>
<223>  Synthetic polynucleotide

<400>  315
atgcaagaaa tgtctggaga aattgaa                                          27


<210>  316
<211>  27
<212>  DNA
<213>  Artificial Sequence

<220>
<223>  Synthetic polynucleotide

<400>  316
ccttgtattt tctcagtacg agctcca                                          27


<210>  317
<211>  25
<212>  DNA
<213>  Artificial Sequence

<220>
<223>  Synthetic polynucleotide

<400>  317
gcgacggcgg ctgcggctac tggag                                            25


<210>  318
<211>  25
<212>  DNA
<213>  Artificial Sequence

<220>
<223>  Synthetic polynucleotide

<400>  318
ttcttctaat gcttcttgac taccc                                            25


<210>  319
<211>  25
<212>  DNA
<213>  Artificial Sequence

<220>
<223>  Synthetic polynucleotide

<400>  319
tgaaaggaag gagaaggaga aaaag                                            25


<210>  320
<211>  25
<212>  DNA
<213>  Artificial Sequence

<220>
<223>  Synthetic polynucleotide

<400>  320
agaggcagtg agggcagcat tagta                                            25

<210>  321
<211>  25
<212>  DNA
<213>  Artificial Sequence

<220>
<223>  Synthetic polynucleotide

<400>  321
taaatggaga ggaggtagaa gaaac                                          25


<210>  322
<211>  25
<212>  DNA
<213>  Artificial Sequence

<220>
<223>  Synthetic polynucleotide

<400>  322
ggtggtagaa agagaacgaa aagtc                                          25


<210>  323
<211>  23
<212>  DNA
<213>  Artificial Sequence

<220>
<223>  Synthetic polynucleotide

<400>  323
gcgtgacatt aaggagaagc tgt                                            23


<210>  324
<211>  23
<212>  DNA
<213>  Artificial Sequence

<220>
<223>  Synthetic polynucleotide

<400>  324
cttgttttct gcgcaagtta ggt                                            23


<210>  325
<211>  18
<212>  DNA
<213>  Artificial Sequence

<220>
<223>  Synthetic polynucleotide

<400>  325
aggagccagg agaggact                                                  18


<210>  326

<211>  18
<212>  DNA
<213>  Artificial Sequence

<220>
<223>  Synthetic polynucleotide

<400>  326
gcagggacga aaggtatc                                                    18


<210>  327
<211>  23
<212>  DNA
<213>  Artificial Sequence

<220>
<223>  Synthetic polynucleotide

<400>  327
gtatcgtgga aggactcatg acc                                              23


<210>  328
<211>  23
<212>  DNA
<213>  Artificial Sequence

<220>
<223>  Synthetic polynucleotide

<400>  328
tctcttcctc ttgtgctctt gct                                              23


<210>  329
<211>  120
<212>  DNA
<213>  Artificial Sequence

<220>
<223>  Synthetic polynucleotide

<400>  329
gcacacagaa gcgctcagta agggctttga aacttaacag tttgggagcc agatcctcag      60

gccacatctc tctcctccca cgacctccgc ggtcctccag aaccatagag agttgtacag     120


<210>  330
<211>  120
<212>  DNA
<213>  Artificial Sequence

<220>
<223>  Synthetic polynucleotide

<400>  330
agatcgccct gctctatgct ctactctcct ggggagcggg gccagagagg ccggaagtgc      60

tgcgagccct gggccacgct ggccgtgctg gcagtgggcc gcctcgatcc ctctgcagtc     120

<210>  331
<211>  120
<212>  DNA
<213>  Artificial Sequence

<220>
<223>  Synthetic polynucleotide

<400>  331
tttcccttga ggctccaaga ccagcaggtg aggcctcgcg gcgctgaaac cgtgaggccc      60

ggaccacagg tgcgggaggc ggagactgcg ggtggagatt ggcgccgcgg aagccaatca      120


<210>  332
<211>  120
<212>  DNA
<213>  Artificial Sequence

<220>
<223>  Synthetic polynucleotide

<400>  332
gcgatgggac ttgtggacct gtaaggggcg gggcgagccg aaggtggagg tcaaaggggc      60

gtggcgttac agagcctcta gcgctgggtg ttggggacct gacgctatgg agctctcgga      120


<210>  333
<211>  120
<212>  DNA
<213>  Artificial Sequence

<220>
<223>  Synthetic polynucleotide

<400>  333
gttttgtggg ggacggctgt gagtgggggg ttcctgctgc gggatgagaa cgtagacgcc      60

agtggctcac tcgctcctgg caccttccct ttcaggctcc agatggaccc tgggaaggac      120


<210>  334
<211>  120
<212>  DNA
<213>  Artificial Sequence

<220>
<223>  Synthetic polynucleotide

<400>  334
aaagaggggg tgccccagcc ctcagggccg ccagcaagga agaaatttgt gatacccctc      60

gacgaggatg aggtccctcc tggagtggta ggacaaggag atgcggggcc cctgggaggc      120


<210>  335
<211>  120
<212>  DNA
<213>  Artificial Sequence

<220>
<223>  Synthetic polynucleotide

<400>  335
ttctccccac aggccaagcc cttattccga tctacacaga gccttcccac tgtggacacc      60

tcggcccagg cggcccctca gacctacgcc gaatatgcca tctcacagcc tctggaaggg     120


<210>  336
<211>  120
<212>  DNA
<213>  Artificial Sequence

<220>
<223>  Synthetic polynucleotide

<400>  336
gctggggcca cgtgccccac agggtcagag cccctggcag gagagacgcc caaccaggcc      60

ctgaaacccg gggcaaaatc caacagcatc attgtgagcc ctcggcaggt gaggagggag     120


<210>  337
<211>  120
<212>  DNA
<213>  Artificial Sequence

<220>
<223>  Synthetic polynucleotide

<400>  337
ctccccagag gggcaatccc gtactgaagt tcgtgcgcaa tgtgccctgg gaatttggcg      60

acgtaattcc cgactatgtg ctgggccaga gcacctgtgc cctgttcctc aggtgagctc     120


<210>  338
<211>  120
<212>  DNA
<213>  Artificial Sequence

<220>
<223>  Synthetic polynucleotide

<400>  338
cccccaccag cctccgctac cacaacctgc acccagacta catccatggg cggctgcaga      60

gcctggggaa gaacttcgcc ttgcgggtcc tgcttgtcca ggtggatgtg gtaagcaggg     120


<210>  339
<211>  120
<212>  DNA
<213>  Artificial Sequence

<220>
<223>  Synthetic polynucleotide

<400>  339
aattctgatt ttctcctcca gaaagatccc cagcaggccc tcaaggagct ggctaagatg      60

tgtatcctgg ccgactgcac attgatcctc gcctggaggt gagatgaggg cttccctgcc     120

<210>  340
<211>  120
<212>  DNA
<213>  Artificial Sequence

<220>
<223>  Synthetic polynucleotide

<400>  340
gttatcccag ccccgaggaa gctgggcggt acctggagac ctacaaggcc tatgagcaga      60

aaccagcgga cctcctgatg gagaagctag agcaggactt cgtctcccgg gtgaggccac      120


<210>  341
<211>  120
<212>  DNA
<213>  Artificial Sequence

<220>
<223>  Synthetic polynucleotide

<400>  341
cactcctgcc tccacccttt ccaggtgact gaatgtctga ccaccgtgaa gtcagtcaac      60

aaaacggaca gtcagaccct cctgaccaca tttggagtaa ggaatggctc ccctgcccca      120


<210>  342
<211>  120
<212>  DNA
<213>  Artificial Sequence

<220>
<223>  Synthetic polynucleotide

<400>  342
ggaggctttt gtgctcaact gccctgaccc ctcgctttca cctttcagtc tctggaacag      60

ctcatcgccg catcaagaga agatctggcc ttatgcccag gcctgggccc tcagaaagta      120


<210>  343
<211>  119
<212>  DNA
<213>  Artificial Sequence

<220>
<223>  Synthetic polynucleotide

<400>  343
agagctctgg gaaagaaccc aaggagttgg gggaaggaga gagccccaaa taaacacaac      60

ctgagacccc aaagttttaa ggtgaaaaaa gaaccaaaga ccagacacag tggcttccg      119


<210>  344
<211>  120
<212>  DNA
<213>  Artificial Sequence

<220>
<223>  Synthetic polynucleotide

<400>  344
ggcgggtcga ccccgctgca cagtccggcc ggcgccatga agtgagaagg gggctggggg      60

tcgcgctcgc tagcgggcgc ggggggtctt gaagatgggg tcatcggtgg gcgcgcctgg      120


<210>  345
<211>  120
<212>  DNA
<213>  Artificial Sequence

<220>
<223>  Synthetic polynucleotide

<400>  345
gtccccaagg gggcgagggg agggtgaagg ggtgggacgg gggcagccgc agggagcagc      60

agtgatagcg aggagacact gagggggccc cgaggctcct gaggacctga gggttaccgg      120


<210>  346
<211>  120
<212>  DNA
<213>  Artificial Sequence

<220>
<223>  Synthetic polynucleotide

<400>  346
gggcgccggg cccgtcaccc ttctctgggc tcgacgaccg ggcactgtgg aggcgggaga      60

ggggctgagg ggacgggaac tgacccagca gcccctgccg ccaggctcaa cgtggacggg      120


<210>  347
<211>  120
<212>  DNA
<213>  Artificial Sequence

<220>
<223>  Synthetic polynucleotide

<400>  347
ctcctggtct acttcccgta cgactacatc taccccgagc agttctccta catgcgggag      60

ctcaaacgca cgctggacgc caaggtgggt ggccggtggg cccgacccgc ccactcgacc      120


<210>  348
<211>  120
<212>  DNA
<213>  Artificial Sequence

<220>
<223>  Synthetic polynucleotide

<400>  348
cctctggtcc ccaacatgca gggtcatgga gtcctggaga tgccctcagg caccgggaag      60

acagtatccc tgttggccct gatcatggca taccagagag tgagtgatgc gctgaacccg      120

<210>  349
<211>  120
<212>  DNA
<213>  Artificial Sequence

<220>
<223>  Synthetic polynucleotide

<400>  349
agggactgag tccgcttgtt atcggcaggc atatccgctg gaggtgacca aactcatcta      60

ctgctcaaga actgtgccag agattgagaa ggtaagctgg gactcatcct ggtgctccag      120


<210>  350
<211>  120
<212>  DNA
<213>  Artificial Sequence

<220>
<223>  Synthetic polynucleotide

<400>  350
agtaggtgat tgaagagctt cgaaagttgc tcaacttcta tgagaagcag gagggcgaga      60

agctgccgtt tctgggactg gctctgagct cccgcaaaaa cttgtgtatt caccctgagg      120


<210>  351
<211>  120
<212>  DNA
<213>  Artificial Sequence

<220>
<223>  Synthetic polynucleotide

<400>  351
aggtgacacc cctgcgcttt gggaaggacg tcgatgggaa atgccacagc ctcacagcct      60

cctatgtgcg ggcgcagtac cagcatgaca ccagcctgcc ccactgccga ttctatgagg      120


<210>  352
<211>  120
<212>  DNA
<213>  Artificial Sequence

<220>
<223>  Synthetic polynucleotide

<400>  352
aggaatttga tgcccatggg cgtgaggtgc ccctccccgc tggcatctac aacctggatg      60

acctgaaggc cctggggcgg cgccagggct ggtgcccata cttccttgct cgatactcag      120


<210>  353
<211>  120
<212>  DNA
<213>  Artificial Sequence

<220>
<223>  Synthetic polynucleotide

<400>  353
ggtaggggta ggggttgggg tggcagggcc ctggtaaccc tgctccccgg ccccccagat      60

cctgcatgcc aatgtggtgg tttatagcta ccactacctc ctggacccca agattgcaga     120


<210>  354
<211>  120
<212>  DNA
<213>  Artificial Sequence

<220>
<223>  Synthetic polynucleotide

<400>  354
cctggtgtcc aaggaactgg cccgcaaggc cgtcgtggtc ttcgacgagg cccacaacat      60

tggtgagggg ggcgccaggg gccaaaggga tgccagcccc tctgagtgag gcccctgcag     120


<210>  355
<211>  120
<212>  DNA
<213>  Artificial Sequence

<220>
<223>  Synthetic polynucleotide

<400>  355
ccccttctcc agacaacgtc tgcatcgact ccatgagcgt caacctcacc cgccggaccc      60

ttgaccggtg ccagggcaac ctggagaccc tgcagaagac ggtgctcagg tggggccggg     120


<210>  356
<211>  120
<212>  DNA
<213>  Artificial Sequence

<220>
<223>  Synthetic polynucleotide

<400>  356
acttgtcccc agggttgcca gccccctccc agccccagct catctctccg caggatcaaa      60

gagacagacg agcagcgcct gcgggacgag taccggcgtc tggtggaggg gctgcgggag     120


<210>  357
<211>  120
<212>  DNA
<213>  Artificial Sequence

<220>
<223>  Synthetic polynucleotide

<400>  357
gccagcgccg cccgggagac ggacgcccac ctggccaacc ccgtgctgcc cgacgaagtg      60

ctgcagggtg agccccgacc ccccgctgcc ccccagtccc tttcccgcct ccccgtcgcc     120

<210>  358
<211>  120
<212>  DNA
<213>  Artificial Sequence

<220>
<223>  Synthetic polynucleotide

<400>  358
cgcctccccg tcgccgctga tagcgtctcc tcgcagaggc agtgcctggc tccatccgca      60

cggccgagca tttcctgggc ttcctgaggc ggctgctgga gtacgtgaag tggcggctgc      120


<210>  359
<211>  120
<212>  DNA
<213>  Artificial Sequence

<220>
<223>  Synthetic polynucleotide

<400>  359
gtgtgcagca tgtggtgcag gagagcccgc ccgccttcct gagcggcctg gcccagcgcg      60

tgtgcatcca gcgcaagccc ctcaggtgcg gccccagaca gcgcgcgggg tgggggcccg      120


<210>  360
<211>  120
<212>  DNA
<213>  Artificial Sequence

<220>
<223>  Synthetic polynucleotide

<400>  360
attctgtgct gaacgcctcc ggtccctgct gcatactctg gagatcaccg accttgctga      60

cttctccccg ctcaccctcc ttgctaactt tgccaccctt gtcagcacct acgccaaagg      120


<210>  361
<211>  120
<212>  DNA
<213>  Artificial Sequence

<220>
<223>  Synthetic polynucleotide

<400>  361
acccctcctt gcctgccctc tgcaggcttc accatcatca tcgagccctt tgacgacaga      60

accccgacca ttgccaaccc catcctgcac ttcaggtggg accctgcccg gtgagggtgt      120


<210>  362
<211>  120
<212>  DNA
<213>  Artificial Sequence

<220>
<223>  Synthetic polynucleotide

<400>  362
cctcactgcc ctcatctctc tccagctgca tggacgcctc gctggccatc aaacccgtat      60

ttgagcgttt ccagtctgtc atcatcacat ctggggtaag gacccttccc cgtcccctcc     120


<210>  363
<211>  120
<212>  DNA
<213>  Artificial Sequence

<220>
<223>  Synthetic polynucleotide

<400>  363
ccgccacaga cactgtcccc gctggacatc taccccaaga tcctggactt ccaccccgtc      60

accatggcaa ccttcaccat gacgctggca cgggtctgcc tctgccctat ggtgagtggg     120


<210>  364
<211>  120
<212>  DNA
<213>  Artificial Sequence

<220>
<223>  Synthetic polynucleotide

<400>  364
gcttatcgtg acctctgttg ctctccagat catcggccgt ggcaatgacc aggtggccat      60

cagctccaaa tttgagaccc gggaggatat tggtatgctg ccagtggggc tggtttctgt     120


<210>  365
<211>  120
<212>  DNA
<213>  Artificial Sequence

<220>
<223>  Synthetic polynucleotide

<400>  365
agacagagaa gggaggagga cctgggccag gagagggccc aacctctgac cccttgcagc      60

tgtgatccgg aactatggga acctcctgct ggagatgtcc gctgtggtcc ctgatggcat     120


<210>  366
<211>  120
<212>  DNA
<213>  Artificial Sequence

<220>
<223>  Synthetic polynucleotide

<400>  366
cgtggccttc ttcaccagct accagtacat ggagagcacc gtggcctcct ggtatgagca      60

ggtacgcctg gccaccccct ccctgcacct gctctcctca gtccctggca ctgcaccgct     120

<210>  367
<211>  120
<212>  DNA
<213>  Artificial Sequence

<220>
<223>  Synthetic polynucleotide

<400>  367
gggtccttcc ccaggggatc cttgagaaca tccagaggaa caagctgctc tttattgaga      60

cccaggatgg tgccgaaacc agtgtcgccc tggagaagta ccaggaggtg ggtgtgcgag      120


<210>  368
<211>  120
<212>  DNA
<213>  Artificial Sequence

<220>
<223>  Synthetic polynucleotide

<400>  368
tgcgtctcgg tctccccacc tcaggcctgc gagaatggcc gcggggccat cctgctgtca      60

gtggcccggg gcaaagtgtc cgagggaatc gactttggtg agtggactgg gctcttcctc      120


<210>  369
<211>  120
<212>  DNA
<213>  Artificial Sequence

<220>
<223>  Synthetic polynucleotide

<400>  369
cgccccctct cctctgccca ccagtgcacc actacgggcg ggccgtcatc atgtttggcg      60

tcccctacgt ctacacacag agccgcattc tcaaggtgag tagctctgtc tcccagggag      120


<210>  370
<211>  120
<212>  DNA
<213>  Artificial Sequence

<220>
<223>  Synthetic polynucleotide

<400>  370
aggagtggct cccccacctc cccagcttct catcctccgt atctgcaggc gcggctggaa      60

tacctgcggg accagttcca gattcgtgag aatgactttc ttaccttcga tgccatgcgc      120


<210>  371
<211>  120
<212>  DNA
<213>  Artificial Sequence

<220>
<223>  Synthetic polynucleotide

<400>  371
cacgcggccc agtgtgtggg tcgggccatc aggggcaaga cggactacgg cctcatggtc        60

tttgccgaca aggtgcagct tcaggggtgc ccctgtgctc ccatcccagg ctccaagaac       120


<210>  372
<211>  120
<212>  DNA
<213>  Artificial Sequence

<220>
<223>  Synthetic polynucleotide

<400>  372
aggctccccg aatgaccttc tgtccctggc ctgcgcttct gcccacagcg gtttgcccgt        60

ggggacaagc gggggaagct gccccgctgg atccaggagc acctcacaga tgccaacctc       120


<210>  373
<211>  120
<212>  DNA
<213>  Artificial Sequence

<220>
<223>  Synthetic polynucleotide

<400>  373
aacctgaccg tggacgaggg tgtccaggtg gccaagtact tcctgcggca gatggcacag        60

cccttccacc gggtgaggcc tgcgtccccc tcccggcacc ctcccaggct gagcttctcc       120


<210>  374
<211>  120
<212>  DNA
<213>  Artificial Sequence

<220>
<223>  Synthetic polynucleotide

<400>  374
tctgttctct gcaggaggat cagctgggcc tgtccctgct cagcctggag cagctagaat        60

cagaggagac gctgaagagg atagagcaga ttgctcagca gctctgagtg gggcgggtgg        120


<210>  375
<211>  120
<212>  DNA
<213>  Artificial Sequence

<220>
<223>  Synthetic polynucleotide

<400>  375
gagcggggtc atcttctctc tgctgctgta gctgccatgg gcaaaagaga ccgagcggac        60

cgcggtgaga cgttgcgcgg gcacgctcag ccacgactgc ccttgccggc cctgcccccc       120

<210>  376
<211>  120
<212>  DNA
<213>  Artificial Sequence

<220>
<223>  Synthetic polynucleotide

<400>  376
gctctgcctc ggagctgctc cgggcctctg cgccggccga ccctgctggc cctcccgcgc      60

gcaccccgtt gggacaggcc tttgggcggg agagatgctg gacctgggcg cagcccagcg      120


<210>  377
<211>  120
<212>  DNA
<213>  Artificial Sequence

<220>
<223>  Synthetic polynucleotide

<400>  377
aactcggcct aggggagacg ggtgaggggc gcaacgcctg cgggatgcag gtggctctta      60

gctaggagtt tgcggcggcg caggtgaaat gctgccaagc ggtgcggagg gaaccctgaa      120


<210>  378
<211>  120
<212>  DNA
<213>  Artificial Sequence

<220>
<223>  Synthetic polynucleotide

<400>  378
gtgttggtat cttgcagaca agaagaaatc caggaagcgg cactatgagg atgaagagga      60

tgatgaagag gacgccccgg ggaacgaccc tcaggaagcg gttccctcgg cggcggggaa      120


<210>  379
<211>  120
<212>  DNA
<213>  Artificial Sequence

<220>
<223>  Synthetic polynucleotide

<400>  379
gcaggtggat gagtcaggca ccaaagtgga tgaatatgga gccaaggact acaggctgca      60

aatgccgctg aaggacgacc acacctccag gcccctctgg gtggtaagca tgcccttagc      120


<210>  380
<211>  120
<212>  DNA
<213>  Artificial Sequence

<220>
<223>  Synthetic polynucleotide

<400>  380
aggctcccga tggccatatc ttcttggaag ccttctctcc agtttacaaa tatgcccaag      60

acttcttggt ggctattgca gagccagtgt gccgaccaac ccatgtgcat gagtacaaac     120


<210>  381
<211>  120
<212>  DNA
<213>  Artificial Sequence

<220>
<223>  Synthetic polynucleotide

<400>  381
taactgccta ctccttgtat gcagctgtca gcgttgggct gcaaaccagt gacatcaccg      60

agtacctcag gaagctcagc aagactggag tccctgatgg aattatgcag tttattaagg     120


<210>  382
<211>  120
<212>  DNA
<213>  Artificial Sequence

<220>
<223>  Synthetic polynucleotide

<400>  382
cacatttacc tgttggatgt tatctgtatt tgcagttgtg tactgtcagc tatggaaaag      60

tcaagctggt cttgaagcac aacaggtaag agattccatg acaggcctgt cccaaggcac     120


<210>  383
<211>  120
<212>  DNA
<213>  Artificial Sequence

<220>
<223>  Synthetic polynucleotide

<400>  383
tccaggaggt acctgctggt cagtagaccc cttcttcctc ccctgcttgc agatacttcg      60

ttgaaagttg ccaccctgat gtaatccagc atcttctcca ggaccccgtg atccgagaat     120


<210>  384
<211>  120
<212>  DNA
<213>  Artificial Sequence

<220>
<223>  Synthetic polynucleotide

<400>  384
gccgcttaag aaactctgaa ggggaggcca ctgagctcat cacagagact ttcacaagca      60

aatctgccgt atgtggactc ctgggccacc cttgggtggg ggcaggcatt taggatttca     120

<210>  385
<211>  120
<212>  DNA
<213>  Artificial Sequence

<220>
<223>  Synthetic polynucleotide

<400>  385
aatgttgtgt gcacgttcag cacctacctc tctcacagat ttctaagact gctgaaagca      60

gtggtgggcc ctccacttcc cgagtgacag atccacaggg taaatctgac atccccatgg      120


<210>  386
<211>  120
<212>  DNA
<213>  Artificial Sequence

<220>
<223>  Synthetic polynucleotide

<400>  386
acctgtttga cttctatgag caaatggaca aggatgaaga agaagaagaa gagacacaga      60

cagtgtcttt tgaagtcaag caggttagtg aatgtacctt cctcctggtt ccttcactga      120


<210>  387
<211>  120
<212>  DNA
<213>  Artificial Sequence

<220>
<223>  Synthetic polynucleotide

<400>  387
tttgtgccct ctttccagga aatgattgag gaactccaga aacgttgcat ccacctggag      60

taccctctgt tggcagaata tgacttccgg aatgattctg tcaaccctga tatcaacatt      120


<210>  388
<211>  120
<212>  DNA
<213>  Artificial Sequence

<220>
<223>  Synthetic polynucleotide

<400>  388
gacctaaagc ccacagctgt cctcagaccc tatcaggaga agagcttgcg aaagatgttt      60

ggaaacgggc gtgcacgttc gggggtcatt gttcttccct gcggtaagtg gtaccagagt      120


<210>  389
<211>  120
<212>  DNA
<213>  Artificial Sequence

<220>
<223>  Synthetic polynucleotide

<400>  389
attgcactct tgtttcttgt aggtgctgga aagtccctgg ttggtgtgac tgctgcatgc        60

actgtcagaa aacgctgtct ggtgctgggc aactcagctg tttctgtgga gcagtggaaa       120


<210>  390
<211>  120
<212>  DNA
<213>  Artificial Sequence

<220>
<223>  Synthetic polynucleotide

<400>  390
gcccagttca agatgtggtc caccattgac gacagccaga tctgccggtt cacctccgat        60

gccaaggaca agcccatcgg ctgctccgtt gccattagca cctactccat gctgggccac       120


<210>  391
<211>  120
<212>  DNA
<213>  Artificial Sequence

<220>
<223>  Synthetic polynucleotide

<400>  391
accaccaaaa ggtcctggga ggccgagcga gtcatggagt ggctcaagac ccaggagtgg        60

ggcctcatga tcctggatga agtgcacacc ataccaggta agcaggctgg agctgagctg       120


<210>  392
<211>  120
<212>  DNA
<213>  Artificial Sequence

<220>
<223>  Synthetic polynucleotide

<400>  392
agtgtgaatg ctcccattgt ttcctcagcc aagatgttcc gaagggtgct caccatcgtg        60

caggcccact gtaagctggg tttgactgcg accctcgtcc gcgaagatga caaaattgtg       120


<210>  393
<211>  120
<212>  DNA
<213>  Artificial Sequence

<220>
<223>  Synthetic polynucleotide

<400>  393
gatttaaatt ttctgattgg gcctaagctc tacgaagcca actggatgga gctgcagaat        60

aatggctaca tcgccaaagt ccagtgtgct gaggtagctg ggcctgggct gggggcgtct       120

<210>  394
<211>  120
<212>  DNA
<213>  Artificial Sequence

<220>
<223>  Synthetic polynucleotide

<400>  394
gttcttactg tattacaggt ctggtgccct atgtctcctg aattttaccg ggaatatgtg      60

gcaatcaaaa ccaagaaacg aatcttgctg tacaccatga accccaacaa atttagagct      120


<210>  395
<211>  120
<212>  DNA
<213>  Artificial Sequence

<220>
<223>  Synthetic polynucleotide

<400>  395
tgccagtttc tgatcaagtt tcatgaaagg aggaatgaca agattattgt ctttgctgac      60

aatgtgtttg ccctaaagga atatgccatt cgactgaaca agtaagaatt gaaaacttgg      120


<210>  396
<211>  120
<212>  DNA
<213>  Artificial Sequence

<220>
<223>  Synthetic polynucleotide

<400>  396
attcttctct agaccctata tctacggacc tacgtctcag ggggaaagga tgcaaattct      60

ccagaatttc aagcacaacc ccaaaattaa caccatcttc atatccaagg tttgtgtggc      120


<210>  397
<211>  120
<212>  DNA
<213>  Artificial Sequence

<220>
<223>  Synthetic polynucleotide

<400>  397
ggtaggtgac acttcgtttg atctgccgga agcaaatgtc ctcattcaga tctcatccca      60

tggtggctcc aggcgtcagg aagcccaaag gctagggcgg gtgcttcgag ctaaaaaagg      120


<210>  398
<211>  120
<212>  DNA
<213>  Artificial Sequence

<220>
<223>  Synthetic polynucleotide

<400>  398
ggatggttgc agaagagtac aatgcctttt tctactcact ggtatcccag gacacacagg      60

aaatggctta ctcaaccaag cggcagagat tcttggtaga tcaaggttat agcttcaagg     120


<210>  399
<211>  120
<212>  DNA
<213>  Artificial Sequence

<220>
<223>  Synthetic polynucleotide

<400>  399
agagagagca gaaaggggca tgtctgctgg cttgggcttt gcaggtgatc acgaaactcg      60

ctggcatgga ggaggaagac ttggcgtttt cgacaaaaga agagcaacag cagctcttac     120


<210>  400
<211>  120
<212>  DNA
<213>  Artificial Sequence

<220>
<223>  Synthetic polynucleotide

<400>  400
agaaagtcct ggcagccact gacctggatg ccgaggagga ggtggtggct ggggaatttg      60

gctccagatc cagccaggtg agtaaatggg tgaggaggtt ccaggtgtca gtgctctcac     120


<210>  401
<211>  120
<212>  DNA
<213>  Artificial Sequence

<220>
<223>  Synthetic polynucleotide

<400>  401
aggagtatct tttcttctgg agactaacat gggctggttc ccccttcccg gcaggcatct      60

cggcgctttg gcaccatgag ttctatgtct ggggccgacg acactgtgta catggagtac     120


<210>  402
<211>  120
<212>  DNA
<213>  Artificial Sequence

<220>
<223>  Synthetic polynucleotide

<400>  402
cactcatcgc ggagcaaggc gcccagcaaa catgtacacc cgctcttcaa gcgctttagg      60

aaatgatgct taggcagggt acttcgttca agaccggcgc ttggcaccct tgttggaaag     120

<210>  403
<211>  120
<212>  DNA
<213>  Artificial Sequence

<220>
<223>  Synthetic polynucleotide

<400>  403
acccggaaga gcttccatgg agtcagggca gccggctcga cggattgcca tggcgccgct      60

gctggagtac gagcgacagc tggtgctgga actgctcgac actgacgggc tagtagtgtg      120


<210>  404
<211>  120
<212>  DNA
<213>  Artificial Sequence

<220>
<223>  Synthetic polynucleotide

<400>  404
cgcccgcggg ctcggcgcgg accggctcct ctaccacttt ctccagctgc actgccaccc      60

agcctgcctg gtgctggtgc tcaacacgca gccggccgag gaggtgcggc cgcgctggcg      120


<210>  405
<211>  120
<212>  DNA
<213>  Artificial Sequence

<220>
<223>  Synthetic polynucleotide

<400>  405
agcctactaa tcaagtttga tttgatttag gagtatttta tcaatcagct gaagatagaa      60

ggagttgaac acctccctcg ccgtgtaaca aatgaaatca caagcaacag tcgctatgaa      120


<210>  406
<211>  120
<212>  DNA
<213>  Artificial Sequence

<220>
<223>  Synthetic polynucleotide

<400>  406
gtttacacac aaggtggtgt tatatttgcg acaagtagga tacttgtggt tgacttcttg      60

actgatagaa taccttcaga tttaattact ggtaagaatt tgaaatctta ttattagtat      120


<210>  407
<211>  120
<212>  DNA
<213>  Artificial Sequence

<220>
<223>  Synthetic polynucleotide

<400>  407
aaattatgtt tctccccctc aggcatcttg gtgtatagag cccacagaat aatcgagtct      60

tgtcaagaag cattcatctt gcgcctcttt cgccagaaaa acaaacgtgg ttttattaaa     120


<210>  408
<211>  120
<212>  DNA
<213>  Artificial Sequence

<220>
<223>  Synthetic polynucleotide

<400>  408
gctttcacag acaatgctgt tgcctttgat actggttttt gtcatgtgga aagagtgatg      60

agaaatcttt ttgtgaggaa actgtatctg tggccaaggt aaagaacatt atgtgacaaa     120


<210>  409
<211>  120
<212>  DNA
<213>  Artificial Sequence

<220>
<223>  Synthetic polynucleotide

<400>  409
atatttgtct ctttaggttc catgtagcag taaactcatt tttagaacag cacaaacctg      60

aagttgtaga aatccatgtt tctatgacac ctaccatgct tgctatacag actgctatac     120


<210>  410
<211>  120
<212>  DNA
<213>  Artificial Sequence

<220>
<223>  Synthetic polynucleotide

<400>  410
tggacatttt aaatgcatgt ctaaaggaac taaaatgcca taacccatcg cttgaagtgg      60

aagatttatc tttagaaaat gctattggaa aaccttttga caaggtactc tttttccttt     120


<210>  411
<211>  120
<212>  DNA
<213>  Artificial Sequence

<220>
<223>  Synthetic polynucleotide

<400>  411
tagcaatacc aaattttatt cttgttttag acaatccgcc attatctgga tcctttgtgg      60

caccagcttg gagccaagac taaatcctta gttcaggatt tgaagatatt acgaactttg     120

<210>  412
<211>  120
<212>  DNA
<213>  Artificial Sequence

<220>
<223>  Synthetic polynucleotide

<400>  412
ctgcagtatc tctctcagta tgattgtgtc acatttctta atcttctgga atctctgaga      60

gcaacggaaa aagcttttgg tcagaattca ggtgggagat taaaatacta ataatattct     120


<210>  413
<211>  120
<212>  DNA
<213>  Artificial Sequence

<220>
<223>  Synthetic polynucleotide

<400>  413
ctgttttaaa ttaaccataa attgatggca ctttttcttt taacttttcg tattaggttg      60

gctgtttctt gactccagca cctcgatgtt tataaatgct cgagcaaggg tttatcatct     120


<210>  414
<211>  120
<212>  DNA
<213>  Artificial Sequence

<220>
<223>  Synthetic polynucleotide

<400>  414
tccagatgcc aaaatgagta aaaaagaaaa aatatctgaa aaaatggaaa ttaaagaagg      60

ggaaggtatc ttgtggggtt aagtctttaa atgtgttttt tatttcggta tttggtatgg     120


<210>  415
<211>  120
<212>  DNA
<213>  Artificial Sequence

<220>
<223>  Synthetic polynucleotide

<400>  415
acagaaacaa aaaaggaact ggtcctagaa agcaacccaa agtgggaggc actgactgaa      60

gtattaaaag aaattgaggc agaaaataag gagagtgaag ctcttggtgg tccaggtagg     120


<210>  416
<211>  120
<212>  DNA
<213>  Artificial Sequence

<220>
<223>  Synthetic polynucleotide

<400>  416
ggtcaagtac tgatttgtgc aagtgatgac cgaacatgtt cccagctgag agactatatc      60

actcttggag cggaggcctt cttattgagg ctctacagga aaacctttga gaaggatagc     120


<210>  417
<211>  120
<212>  DNA
<213>  Artificial Sequence

<220>
<223>  Synthetic polynucleotide

<400>  417
aaagctgaag aagtctggat gaaatttagg aaggaagaca gttcaaagag aattaggaaa      60

tctcacaaaa gacctaaaga cccccaaaac aaagaacggg cttctaccaa agaaagaacc     120


<210>  418
<211>  120
<212>  DNA
<213>  Artificial Sequence

<220>
<223>  Synthetic polynucleotide

<400>  418
ctcaaaaaga aaaaacggaa gttgacctta actcaaatgg taggaaaacc tgaagaactg      60

gaagaggaag gagatgtcga ggaaggatat cgtcgagaaa taagcagtag cccagaaagc     120


<210>  419
<211>  120
<212>  DNA
<213>  Artificial Sequence

<220>
<223>  Synthetic polynucleotide

<400>  419
tgcccggaag aaattaagca tgaagaattt gatgtaaatt tgtcatcgga tgctgctttc      60

ggaatcctga aagaacccct cactatcatc catccgcttc tgggttgcag cgacccctat     120


<210>  420
<211>  120
<212>  DNA
<213>  Artificial Sequence

<220>
<223>  Synthetic polynucleotide

<400>  420
gctctgacaa gggtactaca tgaagtggag ccaagatacg tggttcttta tgacgcagag      60

ctaacctttg ttcggcagct tgaaatttac agggcgagta ggcctgggaa acctctgagg     120

<210>  421
<211>  120
<212>  DNA
<213>  Artificial Sequence

<220>
<223>  Synthetic polynucleotide

<400>  421
gtcttaacat gcagggttta ctttcttata tacggaggtt caactgagga acaacgctat      60

ctcactgctt tgcggaaaga aaaggaagct tttgaaaaac tcataaggta atacatagaa      120


<210>  422
<211>  120
<212>  DNA
<213>  Artificial Sequence

<220>
<223>  Synthetic polynucleotide

<400>  422
acagggaaaa agcaagcatg gttgtccctg aagaaagaga aggcagagat gaaacaaact      60

tagacctagt aagaggcaca gcatctgcag atgtttccac tgacactcgg aaagccggtg      120


<210>  423
<211>  120
<212>  DNA
<213>  Artificial Sequence

<220>
<223>  Synthetic polynucleotide

<400>  423
attccttctt tgagagttct tccccagtga cattacttac tttttctctg taggtggcca      60

ggaacagaat ggtacacagc aaagcatagt tgtggatatg cgtgaatttc gaagtgagct      120


<210>  424
<211>  120
<212>  DNA
<213>  Artificial Sequence

<220>
<223>  Synthetic polynucleotide

<400>  424
tccatctctg atccatcgtc ggggcattga cattgaaccc gtgactttag aggttggaga      60

ttacatcctc actccagaaa tgtgcgtgga gcgcaagagt atcagtgatt taatcggctc      120


<210>  425
<211>  120
<212>  DNA
<213>  Artificial Sequence

<220>
<223>  Synthetic polynucleotide

<400>  425
tttaaataac ggccgcctct acagccagtg catctccatg tcccgctact acaagcgtcc      60

cgtgcttctg attgagtttg accctagcaa gcctttctct ctcacttccc gaggtgcctt     120


<210>  426
<211>  120
<212>  DNA
<213>  Artificial Sequence

<220>
<223>  Synthetic polynucleotide

<400>  426
gtttcaggag atctccagca atgacattag ttccaaactc actcttctta cacttcactt      60

ccccagacta cggattctct ggtgcccctc tcctcatgca acggcggagt tgtttgagga     120


<210>  427
<211>  120
<212>  DNA
<213>  Artificial Sequence

<220>
<223>  Synthetic polynucleotide

<400>  427
gctgaaacaa agcaagccac agcctgatgc ggcgacagca ctggccatta cagcagattc      60

tgaaaccctt cccgagtcag agaagtataa tcctggtccc caagacttct tgttaaaaat     120


<210>  428
<211>  120
<212>  DNA
<213>  Artificial Sequence

<220>
<223>  Synthetic polynucleotide

<400>  428
gccaggggtg aatgccaaaa actgccgctc cttgatgcac cacgttaaga acatcgcaga      60

attagcagcc ctgtcacaag acgagctcac gagtattctg gggaatgctg caaatgccaa     120


<210>  429
<211>  120
<212>  DNA
<213>  Artificial Sequence

<220>
<223>  Synthetic polynucleotide

<400>  429
acagctttat gatttcattc acacctcttt tgcagaagtc gtatcaaaag gaaaagggaa      60

aaagtgaaca gtgatggctg ttttcttatc ccatgcctgt acttttcagc ggctccttgc     120

<210>  430
<211>  120
<212>  DNA
<213>  Artificial Sequence

<220>
<223>  Synthetic polynucleotide

<400>  430
aggacgcagc cgcctcatgg gggtccaggg gctctggaag ctgctggagt gctccgggcg      60

gcaggtcagc cccgaagcgc tggaagggaa gatcctggct gttggtatcc ttaacgccgc      120


<210>  431
<211>  120
<212>  DNA
<213>  Artificial Sequence

<220>
<223>  Synthetic polynucleotide

<400>  431
cccggagttt tttccattaa caattctccc agatattagc atttggttaa accaagcact      60

taaaggagtc cgggatcgcc atgggaactc aatagaaaat cctcatcttc tcactttgtt      120


<210>  432
<211>  120
<212>  DNA
<213>  Artificial Sequence

<220>
<223>  Synthetic polynucleotide

<400>  432
tcatcggctc tgcaaactct tattttttcg aattcgtcct atttttgtgt ttgatgggga      60

tgctccacta ttgaagaaac agactttggt aagtgtcgta tagtttttag taagtgtcaa      120


<210>  433
<211>  120
<212>  DNA
<213>  Artificial Sequence

<220>
<223>  Synthetic polynucleotide

<400>  433
aggtgaagag aaggcagaga aaggacttag cgtccagtga ctccaggaaa acgacagaga      60

agcttctgaa aacatttttg aaaagacaag ccatcaaaac tgccttcaga agcaaaaggc      120


<210>  434
<211>  120
<212>  DNA
<213>  Artificial Sequence

<220>
<223>  Synthetic polynucleotide

<400>  434
tttgtgtact ttccagagat gaagcactac ccagtcttac ccaagttcga agagaaaacg      60

acctctatgt tttgcctcct ttacaagagg aagaaaaaca caggtaaatg tttaactatt     120


<210>  435
<211>  120
<212>  DNA
<213>  Artificial Sequence

<220>
<223>  Synthetic polynucleotide

<400>  435
agatatcgta aaagtatgtt tgactttcag ttcagaagag gaagatgaaa aagaatggca      60

agaaagaatg aatcaaaaac aagcattaca ggtatttaga tcatttttga attcagaatg     120


<210>  436
<211>  120
<212>  DNA
<213>  Artificial Sequence

<220>
<223>  Synthetic polynucleotide

<400>  436
aagtgtatga aatgtaaatt tcatggtgct gtgattttat ctttacagga agagttcttt      60

cataatcctc aagcgataga tattgagtct gaggacttca gcagcctgcc ccctgaagta     120


<210>  437
<211>  120
<212>  DNA
<213>  Artificial Sequence

<220>
<223>  Synthetic polynucleotide

<400>  437
aagcatgaaa tcttgactga tatgaaagag ttcaccaagc gcagaagaac attatttgaa      60

gcaatgccag aggtgaaata tgcaacagta cattcatgct tagaattaag aacttcagca     120


<210>  438
<211>  120
<212>  DNA
<213>  Artificial Sequence

<220>
<223>  Synthetic polynucleotide

<400>  438
tttattttgc ctttaggagt ctgatgactt ttcacagtac caactcaaag gcttgcttaa      60

aaagaactat ctgaaccagc atatagaaca tgtccaaaag gaaatgaatc agcaacattc     120

<210> 439
<211> 120
<212> DNA
<213> Artificial Sequence

<220>
<223> Synthetic polynucleotide

<400> 439
aggacacatc cgaaggcagt atgaagatga aggggctttt ctgaaggagg tagagtcaag 60

gagagtggtc tctgaagaca cttcacatta catcttgata aaaggtatca ggcaccatca 120


<210> 440
<211> 120
<212> DNA
<213> Artificial Sequence

<220>
<223> Synthetic polynucleotide

<400> 440
aaggtattca agctaagaca gttgcagaag tggattcaga gtctcttcct tcttccagca 60

aaatgcacgg catgtctttt gacgtgaagt catctccatg tgaaaaactg aagacagaga 120


<210> 441

<400> 441
000

<210> 442

<400> 442
000

<210> 443

<400> 443
000

<210> 444

<400> 444
000

<210> 445

<400> 445
000

<210> 446

<400> 446
000

<210> 447

<400> 447
000

<210>  448

<400>  448
000

<210>  449

<400>  449
000

<210>  450

<400>  450
000

<210>  451
<211>  120
<212>  DNA
<213>  Artificial Sequence

<220>
<223>  Synthetic polynucleotide

<400>  451
aagagcctga tgctacccct ccttctccaa gaactttact agctatgcaa gctgccctgc      60

tgggaagtag ctcagaagag gagctggaga gtgaaaatcg aaggcaggcc cgtgggagga      120


<210>  452
<211>  120
<212>  DNA
<213>  Artificial Sequence

<220>
<223>  Synthetic polynucleotide

<400>  452
acgcacctgc tgctgtagac gaaggctcca tatcaccccg gactctttca gccattaaga      60

gagctcttga cgatgacgaa gatgtaaaag tgtgtgctgg ggatgatgtg cagacgggag      120


<210>  453
<211>  120
<212>  DNA
<213>  Artificial Sequence

<220>
<223>  Synthetic polynucleotide

<400>  453
ggccaggagc agaagaaatg cgtataaaca gctccaccga gaacagtgat gaaggactta      60

aagtgagaga tggaaaagga ataccgttta ctgcaacact tgcgtcatct agtgtgaact      120


<210>  454
<211>  120
<212>  DNA
<213>  Artificial Sequence

<220>

<223>  Synthetic polynucleotide

<400>  454
ctgcagagga gcacgtagcc agcactaatg aggggagaga gcccacagac tcagttccaa      60

aagaacaaat gtcacttgtt cacgtgggga ctgaagcctt tccgataagt gatgagtcta      120


<210>  455
<211>  120
<212>  DNA
<213>  Artificial Sequence

<220>
<223>  Synthetic polynucleotide

<400>  455
tgattaagga cagaaaagat cggctgcctc tggagagtgc agtggttaga catagtgacg      60

cacctgggct cccgaatgga agggaactga caccggcatc tccaacttgt acaaattctg      120


<210>  456
<211>  120
<212>  DNA
<213>  Artificial Sequence

<220>
<223>  Synthetic polynucleotide

<400>  456
tgtcaaagaa tgaaacacat gctgaagtgc ttgagcagca gaacgaactt tgcccatatg      60

agagtaaatt cgattcttct cttctttcaa gtgatgatga aacaaaatgt aaaccgaatt      120


<210>  457
<211>  120
<212>  DNA
<213>  Artificial Sequence

<220>
<223>  Synthetic polynucleotide

<400>  457
ctgcttctga agtcattggc cctgtcagtt tgcaagaaac aagtagcata gtaagtgtcc      60

cttcagaggc agtagataat gtggaaaatg tggtgtcatt taatgctaaa gagcatgaga      120


<210>  458
<211>  120
<212>  DNA
<213>  Artificial Sequence

<220>
<223>  Synthetic polynucleotide

<400>  458
attttctgga aaccatccaa gaacagcaga ccactgaatc tgcaggccag gatttaattt      60

ccattccaaa ggccgtggaa ccaatggaaa ttgactcgga agaaagtgaa tctgatggta      120

<210>  459
<211>  120
<212>  DNA
<213>  Artificial Sequence

<220>
<223>  Synthetic polynucleotide

<400>  459
tgcaggatca ttttaatgtt ttgattgtag atgaagtgac cttttaattt tggtacagga      60

agtttcattg aagtgcaaag tgtgattagt gatgaggaac ttcaagcaga attccctgaa      120


<210>  460
<211>  120
<212>  DNA
<213>  Artificial Sequence

<220>
<223>  Synthetic polynucleotide

<400>  460
acttccaaac ctccctcaga acaaggcgaa gaggaactgg taggaactag ggagggagaa      60

gcccctgctg agtccgagag cctcctgagg gacaactctg agagggacga cgtggatggt      120


<210>  461
<211>  120
<212>  DNA
<213>  Artificial Sequence

<220>
<223>  Synthetic polynucleotide

<400>  461
gagccacagg aagctgagaa agatgcggaa gattcgctcc atgaatggca agatattaat      60

ttggtaatac cgtaacattg tgtttcgact tcttgctgag gaagccaggt taagtaggtt      120


<210>  462
<211>  120
<212>  DNA
<213>  Artificial Sequence

<220>
<223>  Synthetic polynucleotide

<400>  462
tgtaaacaaa actcatttgg attattaata taaatctata aatgaaaaaa cattttatag      60

gaggagttgg aaactctgga gagcaacctc ttagcacagc agaattcact gaaagctcaa      120


<210>  463
<211>  120
<212>  DNA
<213>  Artificial Sequence

<220>

<223>  Synthetic polynucleotide

<400>  463
aaacagcagc aagaacggat cgctgctact gtcaccggac agatgttcct ggaaagccag      60

gtgggtgcag gcagcttggg tttcctttac caccttcttc agacccctgg gggaatgcac      120


<210>  464
<211>  120
<212>  DNA
<213>  Artificial Sequence

<220>
<223>  Synthetic polynucleotide

<400>  464
cctcactctg caggaactcc tgcgcctgtt cggcattccc tacatccagg ctcccatgga      60

agcagaggcg cagtgcgcca tcctggacct gactgatcag acttccggaa ccatcactga      120


<210>  465
<211>  120
<212>  DNA
<213>  Artificial Sequence

<220>
<223>  Synthetic polynucleotide

<400>  465
tgacagtgat atctggctgt ttggagcgcg gcatgtctat agaaactttt ttaataaaaa      60

caagtttgta gaatattatc aatatgtgga ctttcacaat caattgggta agacttcaga      120


<210>  466
<211>  120
<212>  DNA
<213>  Artificial Sequence

<220>
<223>  Synthetic polynucleotide

<400>  466
tcatataaga aatcttgata aaaattaaaa aatattgtta ctctttagga ttggaccgga      60

ataagttaat aaatttggct tatttgcttg gaagtgatta taccgaagga ataccaactg      120


<210>  467
<211>  120
<212>  DNA
<213>  Artificial Sequence

<220>
<223>  Synthetic polynucleotide

<400>  467
tgggttgtgt aaccgccatg gaaattctca atgaattccc tgggcatggc ctggaacctc      60

tcctaaaatt ctcgtaaggt cttttatttc tttaatttgg ataattgtgt aaatacccaa      120

<210>  468
<211>  120
<212>  DNA
<213>  Artificial Sequence

<220>
<223>  Synthetic polynucleotide

<400>  468
ttgtttcctt tattttacag agaatggtgg catgaagctc aaaaaaatcc aaagataaga      60

cctaatcctc atgacaccaa agtgaaaaaa aaattacgga cattgcaact cacccctggc      120


<210>  469
<211>  120
<212>  DNA
<213>  Artificial Sequence

<220>
<223>  Synthetic polynucleotide

<400>  469
tttcctaacc cagctgttgc cgaggcctac ctcaaacccg tggtggatga ctcgaaggga      60

tcctttctgt gggggaaacc tgatctcgac aaaattagag aatatccttt gcttcttaaa      120


<210>  470
<211>  120
<212>  DNA
<213>  Artificial Sequence

<220>
<223>  Synthetic polynucleotide

<400>  470
agtcttaact gcatgcatat tttgtcagcg gtatttcggc tggaacagaa cgaagacaga      60

tgaatctctg tttcctgtat taaagcaact cgatgcccag caggtaatca tggtggaccc      120


<210>  471
<211>  120
<212>  DNA
<213>  Artificial Sequence

<220>
<223>  Synthetic polynucleotide

<400>  471
agacacagct ccgaattgat tccttcttta gattagcaca acaggagaaa gaagatgcta      60

aacgtattaa gagccagaga ctaaacagag ctgtgacatg tatgctaagg aaagagaaag      120


<210>  472
<211>  120
<212>  DNA
<213>  Artificial Sequence

<220>

<223>  Synthetic polynucleotide

<400>  472
aagcagcagc cagcgaaata gaagcagttt ctgttgccat ggagaaagaa tttgagctac      60

ttgataaggc aaaaggaaaa acccagaaga gaggcataac aaatacctta gaagagtcat      120


<210>  473
<211>  120
<212>  DNA
<213>  Artificial Sequence

<220>
<223>  Synthetic polynucleotide

<400>  473
caagcctgaa aagaaagagg ctttcagatt ctaaaggaaa gaatacatgc ggtggatttt      60

tgggggagac ctgcctctca gaatcatctg atggatcttc aagtgaagat gctgaaagtt      120


<210>  474
<211>  120
<212>  DNA
<213>  Artificial Sequence

<220>
<223>  Synthetic polynucleotide

<400>  474
catctttaat gaatgtacaa aggagaacag ctgcgaaaga gccaaaaacc agtgcttcag      60

attcgcagaa ctcagtgaag gaagctcccg tgaagaatgg aggtgcgacc accagcagct      120


<210>  475
<211>  120
<212>  DNA
<213>  Artificial Sequence

<220>
<223>  Synthetic polynucleotide

<400>  475
ctagtgatag tgatgacgat ggagggaaag agaagatggt cctcgtgacc gccagatctg      60

tgtttgggaa gaaaagaagg aaactaagac gtgcgagggg aagaaaaagg aaaacctaat      120


<210>  476
<211>  120
<212>  DNA
<213>  Artificial Sequence

<220>
<223>  Synthetic polynucleotide

<400>  476
tttttttcct tttaggtagt ctgtagagaa tgccaaatga gggaatcccc cactcaagtc      60

aaactcagga gcaagactgt ttacagagtc aacctgtcag taataatgaa gaaatggcaa      120

<210>  477
<211>  120
<212>  DNA
<213>  Artificial Sequence

<220>
<223>  Synthetic polynucleotide

<400>  477
tcaagcaaga aagtggtggt gatggggagg tggaggagta cctctccttt cgttctgtgg      60

gtgacgggct gtccacctct gctgtggggt gcgcatcagc agctccgagg agagggccag      120


<210>  478
<211>  120
<212>  DNA
<213>  Artificial Sequence

<220>
<223>  Synthetic polynucleotide

<400>  478
ccctgctgca catcgaccga catcagatcc aggcagtaga gcctagcgcc caggcccttg      60

agctgcaggg tttgggtgtg gacgtctatg accaggacgt gctggaacag ggagtgcttc      120


<210>  479
<211>  120
<212>  DNA
<213>  Artificial Sequence

<220>
<223>  Synthetic polynucleotide

<400>  479
agcaggtgga caatgccatc catgaggcca gccgtgcctc ccagctcgtt gacgtggaga      60

aggagtatcg gtcggtcctg gatgacctca cgtgagtgca gcccatcttc ttcctttcaa      120


<210>  480
<211>  120
<212>  DNA
<213>  Artificial Sequence

<220>
<223>  Synthetic polynucleotide

<400>  480
tatattatgc tataaaaatg ttatcagagt gcaaaataga caatttatct tatttttcag      60

gtcatgtacg acatccctaa ggcaaatcaa taaaattatt gaacagctta gccctcaagc      120


<210>  481
<211>  120
<212>  DNA
<213>  Artificial Sequence

<220>

<223>  Synthetic polynucleotide

<400>  481
tgccaccagc agagacatca acaggaaact agattctgta aaacgacaga agtataataa      60

ggtgattcag aataacattc agtattgcat ttttgaaaaa gtcatcattt aggggcactt      120


<210>  482
<211>  120
<212>  DNA
<213>  Artificial Sequence

<220>
<223>  Synthetic polynucleotide

<400>  482
ttcaaggaac aacagctaaa aaagatcact gcaaaacaaa agcatctcca ggccatcctt      60

ggaggagcag aggtgaaaat tgaactagat cacgccagtc tggaggagga tgcaggtgag      120


<210>  483
<211>  120
<212>  DNA
<213>  Artificial Sequence

<220>
<223>  Synthetic polynucleotide

<400>  483
ggtggagggg gcagaggcgg acctgtctgg agatggtact gactatgagc tgaagcctct      60

gcccaagggc gggaaacggc agaagaaagt gccagtgcag gagattgatg atgacttttt      120


<210>  484
<211>  120
<212>  DNA
<213>  Artificial Sequence

<220>
<223>  Synthetic polynucleotide

<400>  484
cccaagttct ggggaagaag ctgaagctgc ttctgtagga gaaggaggag gaggaggtcg      60

gaaagtggga agataccgag atgatggaga tgaagattat tataagcagc ggttaaggtc      120


<210>  485
<211>  120
<212>  DNA
<213>  Artificial Sequence

<220>
<223>  Synthetic polynucleotide

<400>  485
atttgtagct gcctaatatt tcttgaaaga gtgagcattt cttttgtacc ctcggaaagt      60

ttcatgctag tgcgaaacgc attgctattg ttctttcaga gccggggcca tccagtcttg      120

<210>  486
<211>  120
<212>  DNA
<213>  Artificial Sequence

<220>
<223>  Synthetic polynucleotide

<400>  486
gcagcatgct catgcctgtc caggagactg cctgggaaga gctcatccgc actggccaga      60

tgacaccttt tggtacccag atccctcaga aacaggagaa aaagcccaga aaaatcatgc      120


<210>  487
<211>  120
<212>  DNA
<213>  Artificial Sequence

<220>
<223>  Synthetic polynucleotide

<400>  487
ttaatgaagc atcaggcttc gaaaagtatt tggcagatca agcaaaactg tcttttgaaa      60

ggaagaagca aggttgtaat aaaagagcag ctagaaaagc tccagcccca gtcacgcctc      120


<210>  488
<211>  120
<212>  DNA
<213>  Artificial Sequence

<220>
<223>  Synthetic polynucleotide

<400>  488
cagccccagt gcaaaataaa aacaaaccaa acaagaaagc cagagttctg tccaaaaaag      60

aggagcgttt gaaaaagcac atcaagaaac tccagaagag ggctttgcag ttccagggga      120


<210>  489
<211>  119
<212>  DNA
<213>  Artificial Sequence

<220>
<223>  Synthetic polynucleotide

<400>  489
aagtgggatt gccaaaggca aggagacctt gggagtcaga catgaggcca gaggcagagg      60

gagactctga gggtgaagag tctgagtatt tccccacaga ggaggaggaa gaggaggaa      119


<210>  490
<211>  120
<212>  DNA
<213>  Artificial Sequence

<220>

<223>  Synthetic polynucleotide

<400>  490
tggaacacta ggcaaaacca ctaccactag gactaataat accttttttc cgttttagtc      60

ccaagatgcc tcgaacacta agtttacatg aaataactga ccttttagag acagatgaca      120


<210>  491
<211>  120
<212>  DNA
<213>  Artificial Sequence

<220>
<223>  Synthetic polynucleotide

<400>  491
ttacagaggc acaccctgga caataccatt ttgtattcaa taactttttc accagtattg      60

cacttcttga taagctcagt tcaatgggac atcaggcaac aggtacagtg agaaaggatc      120


<210>  492
<211>  120
<212>  DNA
<213>  Artificial Sequence

<220>
<223>  Synthetic polynucleotide

<400>  492
acattgacag agttccactg gaatcagatg tagctttaaa gaaaaaagaa agaggcacat      60

ttgattatcg aattgatggc aaaggcaata ttgtctgcag atggaatgat aacagtgttg      120


<210>  493
<211>  120
<212>  DNA
<213>  Artificial Sequence

<220>
<223>  Synthetic polynucleotide

<400>  493
tcactgttgc ctcatctggt gctggtatcc atcccctgtg tcttgtcagt cgttactccc      60

agaaactgaa aaagaagata caagttcagc agccaaacat gatcaaagtg tataaccagt      120


<210>  494
<211>  120
<212>  DNA
<213>  Artificial Sequence

<220>
<223>  Synthetic polynucleotide

<400>  494
tcatgggagg cgtagacaga gctgatgaaa acattgataa gtatcgggca tcaatccgtg      60

gaaagaaatg gtattcaagc cctcttttgt tctgtttcga actggtctta caaaatgctt      120

<210>  495
<211>  120
<212>  DNA
<213>  Artificial Sequence

<220>
<223>  Synthetic polynucleotide

<400>  495
ggcaattgca taaaacatat gatgagaaac cagtggattt tctggagttt cgtcgacgtg      60

tggtatgcca ttatctggag acccatggtc atcctccaga acctggccaa aaaggaagac      120


<210>  496
<211>  120
<212>  DNA
<213>  Artificial Sequence

<220>
<223>  Synthetic polynucleotide

<400>  496
ctcagaagcg taacattgac tcacgttatg atggcataaa tcatgtgata gtcaaacagg      60

gaaagcaaac gcgatgcgct gaatgtcata agaacacaac ttttcgatgt gaaaaatgtg      120


<210>  497
<211>  120
<212>  DNA
<213>  Artificial Sequence

<220>
<223>  Synthetic polynucleotide

<400>  497
atgttgcctt acatgtgaag tgttccgttg aatatcacac tgaatagcag gtgtcaccac      60

ctcctgagat aagaaacata gttttataca ttatgtacag tgtagcagtg gttttgccta      120


<210>  498
<211>  120
<212>  DNA
<213>  Artificial Sequence

<220>
<223>  Synthetic polynucleotide

<400>  498
gtgttccaat tttggaacgt cacataacaa tggaacataa taaatttttt tttctcttca      60

aatttttgtt ccttgaattt ttctaggtaa catatatgaa tttcatgcaa aaattcaaaa      120


<210>  499
<211>  120
<212>  DNA
<213>  Artificial Sequence

<220>

<223>  Synthetic polynucleotide

<400>  499
gcatagaagc aagtgctata gtgatacaac cacctgaaaa tgctacagca cctgtttctg      60

atgaggaatc aggagatgaa gaaggtggaa caataaataa tctgccaggt tctttgttgc     120


<210>  500
<211>  120
<212>  DNA
<213>  Artificial Sequence

<220>
<223>  Synthetic polynucleotide

<400>  500
acacagctgc gtatcttatt caagatggct ctgatgctga gtctgactca gatgatccct      60

catacgcacc taaagatgac tctcctgatg aagttccatc tacgtttact gtgcagcaac     120


<210>  501
<211>  120
<212>  DNA
<213>  Artificial Sequence

<220>
<223>  Synthetic polynucleotide

<400>  501
ctccaccatc aaggaggagg aaaatgacaa aaattctttg caaatggaaa aaagccgacc      60

taactgtaca acccgtagca ggtagagtta cagcaccacc aaacgatttc ttcaccgtaa     120


<210>  502
<211>  120
<212>  DNA
<213>  Artificial Sequence

<220>
<223>  Synthetic polynucleotide

<400>  502
tgagaactcc cacagaaatt cttgaacttt ttcttgatga cgaggtcatt gaactcattg      60

tcaagtactc caacttatat gcttgcagta aaggtgtaca tcttggcttg actagctctg     120


<210>  503
<211>  120
<212>  DNA
<213>  Artificial Sequence

<220>
<223>  Synthetic polynucleotide

<400>  503
aattcaaatg ttttctggga attatttttc tgagtggtta tgtctcagtt cctagaaggc      60

gtatgttttg ggaacaaaga acagatgtgc ataatgtact ggttagtgct gccatgagac     120

<210>  504
<211>  120
<212>  DNA
<213>  Artificial Sequence

<220>
<223>  Synthetic polynucleotide

<400>  504
gtgaccggtt tgaaactata ttttctaatt tgcatgttgc tgacaatgca aatttggatc      60

cagtggacaa attttccaaa ttgcgacctc tcataagcaa acttaatgag agatgcatga      120


<210>  505
<211>  120
<212>  DNA
<213>  Artificial Sequence

<220>
<223>  Synthetic polynucleotide

<400>  505
aatttgttcc aaatgaaaca tatttcagct ttgatgaatt catggttcct tattttggtc      60

gtcacgggtg caaacaattt attcggggaa agcccattcg gtttggctat aagttttggt      120


<210>  506
<211>  120
<212>  DNA
<213>  Artificial Sequence

<220>
<223>  Synthetic polynucleotide

<400>  506
gtggtgccac ctgtctgggc tacatttgct ggtttcagcc gtatcagggt aaaaacccaa      60

atactaaaca tgaggaatat ggtgtcggtg cgtcacttgt ccttcagttt agtgaggcac      120


<210>  507
<211>  120
<212>  DNA
<213>  Artificial Sequence

<220>
<223>  Synthetic polynucleotide

<400>  507
tttaaatctg tcttgtgatc aaaataatgg aaatgtgatt tttattttca tggtaggaga      60

tggaataaac tgagactgca ggacaaagag aaacgtctga agctggagga cgattctgag      120


<210>  508
<211>  120
<212>  DNA
<213>  Artificial Sequence

<220>

<223>  Synthetic polynucleotide

<400>  508
gaaagtgatg ctgaatttga cgaaggtttt aaagtgccag gttttctgtt caaaaagctt      60

tttaagtatg taccatatgt tctttccttt ctatttgcta tcaagccatt atgtacaaat      120


<210>  509
<211>  120
<212>  DNA
<213>  Artificial Sequence

<220>
<223>  Synthetic polynucleotide

<400>  509
ttgccatttt ctcttttctt gttggtgttt gttgtcatag gtaccagcag acaggtgtta      60

ggtggctgtg ggaattgcac tgccagcagg caggaggaat tctgggagat gaaatgggat      120


<210>  510
<211>  120
<212>  DNA
<213>  Artificial Sequence

<220>
<223>  Synthetic polynucleotide

<400>  510
tgggcaagac catccagata attgccttct tggcaggtct gagctacagc aagatcagga      60

ctcgtggttc aaattacagg caagtgctcc tctgcagact gtcagtctgt ggagctcaat      120


<210>  511
<211>  120
<212>  DNA
<213>  Artificial Sequence

<220>
<223>  Synthetic polynucleotide

<400>  511
tgactgtata tgctctttca tggttgtttt cttcttggac gtttggatgc aggtttgagg      60

ggttgggtcc aactgtaatt gtctgtccaa caacagtgat gcatcagtgg gtgaaggaat      120


<210>  512
<211>  120
<212>  DNA
<213>  Artificial Sequence

<220>
<223>  Synthetic polynucleotide

<400>  512
ttcacacgtg gtggcctccg ttcagagtgg caattctaca tgaaaccggt tcctataccc      60

acaaaaaggt aacacaatat ttcagtacct ttttgttttg ttttaatgcc ctccatttta      120

<210>  513
<211>  120
<212>  DNA
<213>  Artificial Sequence

<220>
<223>  Synthetic polynucleotide

<400>  513
taaaactgac tttaccattt tattgtggtc tcaggagaaa ctaattcgag atgttgctca      60

ttgtcatgga attttgatca catcttactc ctacattcga ttgatgcagg atgacattag      120


<210>  514
<211>  120
<212>  DNA
<213>  Artificial Sequence

<220>
<223>  Synthetic polynucleotide

<400>  514
caggtatgac tggcactatg tgatcttgga cgaaggacac aaaattcgaa atccaaatgc      60

tgctgtcacc cttgcttgca aacaggtatg acctctttta acaagggaga tttccaagtg      120


<210>  515
<211>  120
<212>  DNA
<213>  Artificial Sequence

<220>
<223>  Synthetic polynucleotide

<400>  515
ttaataaaaa ggtgcttcct ttcctcaaat agtttcgcac ccctcatcgg atcattctgt      60

ctggctcacc gatgcaaaat aacctccgag agctgtggtc gctctttgac ttcatcttcc      120


<210>  516
<211>  120
<212>  DNA
<213>  Artificial Sequence

<220>
<223>  Synthetic polynucleotide

<400>  516
cgggaaagtt aggcacgttg cctgtgttta tggagcagtt ctccgtcccc atcaccatgg      60

ggggatattc aaatgcttcc ccagtacagg taaaatatta ggatgataat acttttggca      120


<210>  517
<211>  120
<212>  DNA
<213>  Artificial Sequence

<220>

<223>  Synthetic polynucleotide

<400>  517
aggtcaaaac tgcttacaag tgtgcatgtg tcttacgaga taccataaat ccatacctac      60

tgcggagaat gaagtcagat gtcaagatga gcctttcttt gccagataaa aatgaacagg     120


<210>  518
<211>  120
<212>  DNA
<213>  Artificial Sequence

<220>
<223>  Synthetic polynucleotide

<400>  518
tcccgtccgt aggtcttatt ttgccgtctt acagatgagc agcataaagt ctaccaaaat      60

ttcgttgatt ccaaagaagt ttacaggatt ctcaatggag agatgcaggt cagctaaaaa     120


<210>  519
<211>  120
<212>  DNA
<213>  Artificial Sequence

<220>
<223>  Synthetic polynucleotide

<400>  519
ttcttaccac agattttctc cggacttata gccctaagaa aaatttgcaa ccaccctgat      60

ctcttttctg gaggtcccaa gaatctcaaa ggtcttcctg atgatgaact agaagaagat     120


<210>  520
<211>  120
<212>  DNA
<213>  Artificial Sequence

<220>
<223>  Synthetic polynucleotide

<400>  520
cagtttgggt actggaaacg ttctgggaaa atgattgttg ttgagtcttt gttgaaaata      60

tggcacaagc agggtcagcg agtattgctg ttttctcagt caaggcaggt gagtgcacag     120


<210>  521
<211>  120
<212>  DNA
<213>  Artificial Sequence

<220>
<223>  Synthetic polynucleotide

<400>  521
acagatgctg gacatacttg aagtattcct tagagcccaa aagtatacct atctcaagat      60

ggatggtacc actacaatag cttcaagaca gccactgatt acgagataca atgaggtaac     120

<210>  522
<211>  120
<212>  DNA
<213>  Artificial Sequence

<220>
<223>  Synthetic polynucleotide

<400>  522
ggctgagaac tgtaactggt cttaagtgtg tgtgctcagt gttgtgtgtc ttacctctag      60

gacacatcca tatttgtgtt tcttctgacc acgcgggtgg gcggcttagg tgtcaacctg      120


<210>  523
<211>  120
<212>  DNA
<213>  Artificial Sequence

<220>
<223>  Synthetic polynucleotide

<400>  523
acgggggcaa acagagttgt catctatgac ccagactgga acccaagcac ggacacgcag      60

gtttgttttt attttttttt taaaagaatg tattagtaga aatatagttt catggttagc      120


<210>  524
<211>  120
<212>  DNA
<213>  Artificial Sequence

<220>
<223>  Synthetic polynucleotide

<400>  524
tctctgttgc aggcccggga gcgagcatgg agaataggcc agaagaagca agtgactgtg      60

tacaggctcc tgactgcggg caccattgaa gaaaagatct accaccggtc agtgcacaca      120


<210>  525
<211>  120
<212>  DNA
<213>  Artificial Sequence

<220>
<223>  Synthetic polynucleotide

<400>  525
gtagagctac acattgtttt ataccagctt atcttttatt tttttagaca aatcttcaag      60

cagtttttga caaatagagt gctaaaagac ccaaaacaaa ggcggttttt caaatccaat      120


<210>  526
<211>  120
<212>  DNA
<213>  Artificial Sequence

<220>

<223>  Synthetic polynucleotide

<400>  526
gatctctatg agctatttac tctgactagt cctgatgcat cccagagcac tgaaacaagt      60

gcaatttttg caggtattac ataaaatcat ttaatttaaa gtaatcaatt gcacaagatg      120


<210>  527
<211>  120
<212>  DNA
<213>  Artificial Sequence

<220>
<223>  Synthetic polynucleotide

<400>  527
ttacaggaac tggatcagat gttcagacac ccaaatgcca tctaaaaaga aggattcaac      60

cagcctttgg agcagaccat gatgttccaa aacgcaagaa gttccctgct tctaacatat      120


<210>  528
<211>  120
<212>  DNA
<213>  Artificial Sequence

<220>
<223>  Synthetic polynucleotide

<400>  528
ctgtaaatga tgccacatca tctgaagaga aatctgaggc taaaggagct gaagtaaatg      60

cagtaacttc taatcgaagt gatcctttga aagatgaccc tcacatgagt agtaatgtaa      120


<210>  529
<211>  120
<212>  DNA
<213>  Artificial Sequence

<220>
<223>  Synthetic polynucleotide

<400>  529
ctagcaatga taggcttgga gaagagacaa atgcagtatc tggaccagaa gagttgtcag      60

tgattagtgg aaatggggaa tgttcaaatt cttcaggaac aggcaaaact tctatgccat      120


<210>  530
<211>  120
<212>  DNA
<213>  Artificial Sequence

<220>
<223>  Synthetic polynucleotide

<400>  530
ctggtgatga aagcattgat gaaaagttag gtctttctta caaaagagaa agacccagcc      60

aggctcaaac agaagctttt tgggagaata aacaaatgga aaataatttt tataagcaca      120

<210>  531
<211>  120
<212>  DNA
<213>  Artificial Sequence

<220>
<223>  Synthetic polynucleotide

<400>  531
agtcaaaaac aaaacatcat agtgtggcag aagaagagac cctggagaaa catctgagac      60

caaagcaaaa gcctaagaac tctaagcatt gcagagacgc caagtttgaa ggaactcgaa      120


<210>  532
<211>  120
<212>  DNA
<213>  Artificial Sequence

<220>
<223>  Synthetic polynucleotide

<400>  532
ttccacacct ggtgaagaaa aggcgttacc agaagcaaga cagtgaaaac aagagtgagg      60

ccaaggaaca gagcaatgac gattatgttt tggaaaagct tttcaaaaaa tcaggtaatc      120


<210>  533
<211>  120
<212>  DNA
<213>  Artificial Sequence

<220>
<223>  Synthetic polynucleotide

<400>  533
tatttctttt cttgctagtt ggcgtgcaca gtgtcatgaa gcacgatgcc atcatggatg      60

gagccagccc agattatgta ctggtggagg cagaagccaa ccgagtggcc caggatgccc      120


<210>  534
<211>  120
<212>  DNA
<213>  Artificial Sequence

<220>
<223>  Synthetic polynucleotide

<400>  534
tgaaagcact gaggctctct cgtcagcggt gtctgggagc agtgtctggt gttcccacct      60

ggactggcca cagggggatt tctggtgcac cagcaggaaa aaagtaagag attgctgcat      120


<210>  535
<211>  120
<212>  DNA
<213>  Artificial Sequence

<220>

<223>  Synthetic polynucleotide

<400>  535
tgatgctttt ccttttttag gagtagattt ggtaagaaaa ggaattctaa cttctctgtg      60

cagcatcctt catcaacatc tccaacagag aagtgccagg taatatagat aacctttttg      120


<210>  536
<211>  120
<212>  DNA
<213>  Artificial Sequence

<220>
<223>  Synthetic polynucleotide

<400>  536
tatatcagta tagtggtctt ctttttatag gatggcatca tgaaaaagga gggaaaagat      60

aatgtccctg agcattttag tggaagagca gaagatgcag actcttcatc cgggcccctc      120


<210>  537
<211>  120
<212>  DNA
<213>  Artificial Sequence

<220>
<223>  Synthetic polynucleotide

<400>  537
gcttcctcct cactcttggc taaaatgaga gctagaaacc acctgattct gccagagcgt      60

ttagaaagtg aaagcgggca cctgcaggaa gcttctgccc tgctgcccac cacagaacac      120


<210>  538
<211>  120
<212>  DNA
<213>  Artificial Sequence

<220>
<223>  Synthetic polynucleotide

<400>  538
gatgaccttc tggtggagat gagaaacttc atcgctttcc aggcccacac tgatggccag      60

gccagcacca gggagatact gcaggagttt gaatccaagt tatctgcatc acagtcttgt      120


<210>  539
<211>  120
<212>  DNA
<213>  Artificial Sequence

<220>
<223>  Synthetic polynucleotide

<400>  539
gtcttccgag aactattgag aaatctgtgc actttccata gaacttctgg tggtgaagga      60

atttggaaac tcaagccaga atactgctaa acaacattgc ttcctaaact ttcaagtccc      120

<210>  540
<211>  120
<212>  DNA
<213>  Artificial Sequence

<220>
<223>  Synthetic polynucleotide

<400>  540
ggtaactgga acccaatccg agggtcatgg aggcatcccg aaggtttccg gaagccgagg      60

ccttgagccc agagcaggct gctcattacc taaggtatgt ctggaccgct gggcgggtct      120


<210>  541
<211>  120
<212>  DNA
<213>  Artificial Sequence

<220>
<223>  Synthetic polynucleotide

<400>  541
tctttttttt tctatagata tgtgaaagag gccaaagaag caactaagaa tggagacctg      60

gaagaagcat ttaaactttt caatttggca aaggacattt ttcccaatga aaaagtgctg      120


<210>  542
<211>  120
<212>  DNA
<213>  Artificial Sequence

<220>
<223>  Synthetic polynucleotide

<400>  542
gaagaaatat acaggaaatt tgtgtcttta gatcatatca aggagttgct aatggagacg      60

cgctcacctt tggctgagct aggtgtctta aagaagctgt gtgatcatcc taggctgctg      120


<210>  543
<211>  120
<212>  DNA
<213>  Artificial Sequence

<220>
<223>  Synthetic polynucleotide

<400>  543
tctgcacggg cttgttgttt gctaaatctt gggacattct ctgctcaaga tggaaatgag      60

ggggaagatt ccccagatgt ggaccatatt gatcaagtaa ctgatgacac attgatggaa      120


<210>  544
<211>  120
<212>  DNA
<213>  Artificial Sequence

<220>

<223>  Synthetic polynucleotide

<400>  544
gaatctggaa aaatgatatt cctaatggac ctacttaaga ggctgcgaga tgagggacat      60

caaactctgg tgttttctca atcgaggcaa attctaaaca tcattgaacg cctcttaaag      120


<210>  545
<211>  120
<212>  DNA
<213>  Artificial Sequence

<220>
<223>  Synthetic polynucleotide

<400>  545
aataggcact ttaagacatt gcgaatcgat gggacagtta ctcatctttt ggaacgagaa      60

aaaagaatta acttattcca gcaaaataaa gattactctg tttttctgct taccactcaa      120


<210>  546
<211>  120
<212>  DNA
<213>  Artificial Sequence

<220>
<223>  Synthetic polynucleotide

<400>  546
gtaggtggtg tcggtttaac attaactgca gcaactagag tggtcatttt tgaccctagc      60

tggaatcctg caactgatgc tcaagctgtg gatagagttt accgaattgg acaaaaagag      120


<210>  547
<211>  120
<212>  DNA
<213>  Artificial Sequence

<220>
<223>  Synthetic polynucleotide

<400>  547
aatgttgtgg tttataggct aatcacttgt gggactgtag aggaaaaaat atacagaaga      60

caggttttca aggactcatt aataagacaa actactggtg aaaaaaagaa ccctttccga      120


<210>  548
<211>  120
<212>  DNA
<213>  Artificial Sequence

<220>
<223>  Synthetic polynucleotide

<400>  548
tattttagta aacaagaatt aagagagctc tttacaatcg aggatcttca gaactctgta      60

acccagctgc agcttcagtc tttgcatgct gctcagagga aatctgatat aaaactagat      120

<210>  549
<211>  120
<212>  DNA
<213>  Artificial Sequence

<220>
<223>  Synthetic polynucleotide

<400>  549
gaacatattg cctacctgca gtctttgggg atagctggaa tctcagacca tgatttgatg      60

tacacatgtg atctgtctgt taaagaagag cttgatgtgg tagaagaatc tcactatatt      120


<210>  550
<211>  120
<212>  DNA
<213>  Artificial Sequence

<220>
<223>  Synthetic polynucleotide

<400>  550
caacaaaggg ttcagaaagc tcaattcctc gttgaattcg agtctcaaaa taaagagttc      60

ctgatggaac aacaaagaac tagaaatgag ggggcctggc taagagaacc tgtatttcct      120


<210>  551
<211>  120
<212>  DNA
<213>  Artificial Sequence

<220>
<223>  Synthetic polynucleotide

<400>  551
tcttcaacaa agaagaaatg ccctaaattg aataaaccac agcctcagcc ttcacctctt      60

ctaagtactc atcatactca ggaagaagat atcagttcca aaatggcaag tgtagtcatt      120


<210>  552
<211>  120
<212>  DNA
<213>  Artificial Sequence

<220>
<223>  Synthetic polynucleotide

<400>  552
agcagaatcc aaaaaataca ggaagccttg gaggagttgg cagaacaggg agatgatgaa      60

tttacagatg tgtgcaactc tggcttgcta ctttatcgag aactgcacaa ccaactcttt      120


<210>  553
<211>  120
<212>  DNA
<213>  Artificial Sequence

<220>

<223>  Synthetic polynucleotide

<400>  553
gatgatctgc ccaaagaggg tgagaaacaa gatctctcca gtataaaggt gaatgttacc      60

accttgcaag atggtaaagg tacaggtagt gctgactcta tagctacttt accaaagggg     120


<210>  554
<211>  120
<212>  DNA
<213>  Artificial Sequence

<220>
<223>  Synthetic polynucleotide

<400>  554
tttggaagtg tagaagaact ttgtactaac tcttcattgg gaatggaaaa aagctttgca      60

actaaaaatg aagctgtaca aaaagagaca ttacaagagg ggcctaagca agaggcactg     120


<210>  555
<211>  120
<212>  DNA
<213>  Artificial Sequence

<220>
<223>  Synthetic polynucleotide

<400>  555
caagaggatc ctctggaaag ttttaattat gtacttagca aatcaaccaa agctgatatt      60

gggccaaatt tagatcaact aaaggatgat gagattttac gtcattgcaa tccttggccc     120


<210>  556
<211>  120
<212>  DNA
<213>  Artificial Sequence

<220>
<223>  Synthetic polynucleotide

<400>  556
attatttcca taacaaatga aagtcaaaat gcagaatcaa atgtatccat tattgaaata      60

gctgatgacc tttcagcatc ccatagtgca ctgcaggatg ctcaagcaag tgaggccaag     120


<210>  557
<211>  120
<212>  DNA
<213>  Artificial Sequence

<220>
<223>  Synthetic polynucleotide

<400>  557
ttggaagagg aaccttcagc atcttcacca cagtatgcat gtgatttcaa tcttttcttg      60

gaagactcag cagacaacag acaaaatttt tccagtcagt ctttagagca tgttgagaaa     120

<210>  558
<211>  120
<212>  DNA
<213>  Artificial Sequence

<220>
<223>  Synthetic polynucleotide

<400>  558
gaaaatagct tgtgtggctc tgcacctaat tccagagcag ggtttgtgca tagcaaaaca      60

tgtctcagtt gggagttttc tgagaaagac gatgaaccag aagaagtagt agttaaagca      120


<210>  559
<211>  120
<212>  DNA
<213>  Artificial Sequence

<220>
<223>  Synthetic polynucleotide

<400>  559
aaaatcagaa gtaaagctag aaggattgtt tcagatggcg aagatgaaga tgattctttt      60

aaagatacct caagcataaa tccattcaac acatctctct ttcaattctc atctgtgaaa      120


<210>  560
<211>  120
<212>  DNA
<213>  Artificial Sequence

<220>
<223>  Synthetic polynucleotide

<400>  560
caatttgatg cttcaactcc caaaaatgac atcagtccac caggaaggtt cttttcatct      60

caaataccca gtagtgtaaa taagtctatg aactctagaa gatctctggc ttctaggagg      120


<210>  561
<211>  120
<212>  DNA
<213>  Artificial Sequence

<220>
<223>  Synthetic polynucleotide

<400>  561
tctcttatta atatggtttt agaccacgtg gaggacatgg aggaaagact tgacgacagc      60

agtgaagcaa agggtcctga agattatcca gaagaagggg tggaggaaag cagtggcgaa      120


<210>  562
<211>  120
<212>  DNA
<213>  Artificial Sequence

<220>

<223>  Synthetic polynucleotide

<400>  562
gcctccaagt atacagaaga ggatccttcc ggagaaacac tgtcttcaga aaacaagtcc      60

agctggttaa tgacgtctaa gcctagtgct ctagctcaag agacctctct tggtgcccct      120


<210>  563
<211>  120
<212>  DNA
<213>  Artificial Sequence

<220>
<223>  Synthetic polynucleotide

<400>  563
gagcaccaga aggaaggcat agctttcctc tatagcctgt atagggatgg aagaaaaggt      60

ggtatattgg ctgatgatat gggattaggg aagactgttc aaatcattgc tttcctttcc      120


<210>  564
<211>  120
<212>  DNA
<213>  Artificial Sequence

<220>
<223>  Synthetic polynucleotide

<400>  564
gagcctttgt ctggtgaaca gttggttggt tctccccagg ataaggcggc agaggctaca      60

aatgactatg agactcttgt aaagcgtgga aaagaactaa aagagtgtgg aaaaatccag      120


<210>  565
<211>  120
<212>  DNA
<213>  Artificial Sequence

<220>
<223>  Synthetic polynucleotide

<400>  565
gaggccctaa actgcttagt taaagcgctt gacataaaaa gtgcagatcc tgaagttatg      60

ctcttgactt taagtttgta taagcaactt aataacaatt gagaatgtaa cctgtttatt      120


<210>  566
<211>  120
<212>  DNA
<213>  Artificial Sequence

<220>
<223>  Synthetic polynucleotide

<400>  566
ggtatgtttg atgcatcact tgtgaatcat gtgctgctga tcatgccaac caatcttatt      60

aacacatggg taaaagaatt catcaagtgg actccaggaa tgagagtcaa aacctttcat      120

<210>  567
<211>  120
<212>  DNA
<213>  Artificial Sequence

<220>
<223>  Synthetic polynucleotide

<400>  567
ggtcctagca aggatgaacg gaccagaaac ctcaatcgga ttcagcaaag gaatggtgtt      60

attatcacta cataccaaat gttaatcaat aactggcagc aactttcaag ctttaggggc      120


<210>  568
<211>  120
<212>  DNA
<213>  Artificial Sequence

<220>
<223>  Synthetic polynucleotide

<400>  568
caagagtttg tgtgggacta tgtcatcctc gatgaagcac ataaaataaa aacctcatct      60

actaagtcag caatatgtgc tcgtgctatt cctgcaagta atcgcctcct cctcacagga      120


<210>  569
<211>  120
<212>  DNA
<213>  Artificial Sequence

<220>
<223>  Synthetic polynucleotide

<400>  569
accccaatcc agaataattt acaagaacta tggtccctat ttgattttgc ttgtcaaggg      60

tccctgctgg gaacattaaa aacttttaag atggagtatg aaaatcctat tactagagca      120


<210>  570
<211>  120
<212>  DNA
<213>  Artificial Sequence

<220>
<223>  Synthetic polynucleotide

<400>  570
agagagaagg atgctacccc aggagaaaaa gccttgggat ttaaaatatc tgaaaactta      60

atggcaatca taaaacccta ttttctcagg aggactaaag aagacgtaca gaagaaaaag      120


<210>  571
<211>  120
<212>  DNA
<213>  Artificial Sequence

<220>

<223>  Synthetic polynucleotide

<400>  571
tcaagcaacc cagaggccag acttaatgaa aagaatccag atgttgatgc catttgtgaa      60

atgccttccc tttccaggaa aaatgattta attatttgga tacgacttgt gcctttacaa      120


<210>  572
<211>  120
<212>  DNA
<213>  Artificial Sequence

<220>
<223>  Synthetic polynucleotide

<400>  572
cgccgccttc cgggtgttac atgcagccgg gctcggcccc tccccctggc cggatggatc      60

cgtcggcgcc acagccccgc gcggaaacct caggcaaagg taccagctcc gcgctcgccc      120


<210>  573
<211>  120
<212>  DNA
<213>  Artificial Sequence

<220>
<223>  Synthetic polynucleotide

<400>  573
ggaaacttta ttttctttat tcttgcagac atatggcatc caggagaaag atgtcttgcc      60

ccttctccag ataatggaaa actttgtgaa gcaagcataa aatctatcac agtggatgaa      120


<210>  574
<211>  120
<212>  DNA
<213>  Artificial Sequence

<220>
<223>  Synthetic polynucleotide

<400>  574
aatggcaagt catttgcagt cgtcttatat gcagattttc aagaaaggaa aatacctctt      60

aaacagcttc aagaagtgaa atttgttaaa gattgcccta ggaatcttat atttgatgat      120


<210>  575
<211>  120
<212>  DNA
<213>  Artificial Sequence

<220>
<223>  Synthetic polynucleotide

<400>  575
gaagatttag aaaaacctta tttcccaaac cgaaaatttc catcatcttc tgttgctttt      60

aaattatctg acaatggaga ctctattcct tataccatca ataggtattt gagagactac      120

<210>  576
<211>  120
<212>  DNA
<213>  Artificial Sequence

<220>
<223>  Synthetic polynucleotide

<400>  576
caaagagaag gaacccggtt tctttatgga cactacatcc atggaggagg gtgcattctg      60

ggtgatgaca tgggacttgg aaaaacagta caggtattta attatgttat aacagtaaag     120


<210>  577
<211>  120
<212>  DNA
<213>  Artificial Sequence

<220>
<223>  Synthetic polynucleotide

<400>  577
aaattttgta cgtcatgata cattatacac agtgattgaa aaagtctttt ttccccaggt      60

tatttcattt ctggctgcag ttttgcataa aaagggaact cgtgaggata ttgaaaataa     120


<210>  578
<211>  120
<212>  DNA
<213>  Artificial Sequence

<220>
<223>  Synthetic polynucleotide

<400>  578
catgccagag tttttactaa gaagtatgaa aaaggaaccc ctttcttcta cagcaaaaaa      60

ggtaaaatct ctagacaatg tatattctac tcatgatgct ggtattacaa aagcatatag     120


<210>  579
<211>  120
<212>  DNA
<213>  Artificial Sequence

<220>
<223>  Synthetic polynucleotide

<400>  579
aaattttttt attcttgttt tagatgttct taatagttgc tcctctttct gtcctctaca      60

actggaagga tgaattggac acctggggat atttcagagt cactgtttta catggaaaca     120


<210>  580
<211>  120
<212>  DNA
<213>  Artificial Sequence

<220>

<223>  Synthetic polynucleotide

<400>  580
gaaaagataa tgaattaatt cgtgtaaagc agaggaaatg tgaaattgct ctaacaactt      60

atgaaacact acgcttatgc ctggatgaac ttaacaggta atgggaataa taggaatagg      120


<210>  581
<211>  120
<212>  DNA
<213>  Artificial Sequence

<220>
<223>  Synthetic polynucleotide

<400>  581
gttttctcaa cctcaccctt cttttttctt actttatagt ttggaatggt cagctgtcat      60

tgtggatgaa gctcatagaa tcaagaatcc aaaagctaga gtaacagaag ttatgaaagc      120


<210>  582
<211>  120
<212>  DNA
<213>  Artificial Sequence

<220>
<223>  Synthetic polynucleotide

<400>  582
tttgaaatgt aatgtccgca ttggcctcac tggaaccatc cttcagaaca acatgaagga      60

actgtggtgt gttatggact ggtgagagaa aacacttttt aaaaaattgt ttaatagttc      120


<210>  583
<211>  120
<212>  DNA
<213>  Artificial Sequence

<220>
<223>  Synthetic polynucleotide

<400>  583
tattgtcttt ataaagggct gtgccaggcc ttttagggag tgggacctac ttcaagaagc      60

agttttctga cccagtagaa catggtcaga gacacacggc aacaaagaga gaactagcca      120


<210>  584
<211>  120
<212>  DNA
<213>  Artificial Sequence

<220>
<223>  Synthetic polynucleotide

<400>  584
ctggccgaaa ggccatgcaa agacttgcca aaaagatgtc tggctggttt ctcaggcgca      60

ccaagactct tatcaaggat cagttgccta agaaggaaga ccgggtaaga accgcatttg      120

<210>  585
<211>  120
<212>  DNA
<213>  Artificial Sequence

<220>
<223>  Synthetic polynucleotide

<400>  585
tattgttata aacaaaatac taataactac cttgtatttt atcttggcag atggtgtatt      60

gttctttgac agatttccag aaagctgtct atcaaacagt gttagaaaca gaggacgtga      120


<210>  586
<211>  120
<212>  DNA
<213>  Artificial Sequence

<220>
<223>  Synthetic polynucleotide

<400>  586
ctttgatact tcaatcttct gagccttgta cctgtaggag tggccaaaaa aggagaaatt      60

gttgttataa ggcaagcatt tcaatatatc tttataatca tgcttttgat tacatagtac      120


<210>  587
<211>  120
<212>  DNA
<213>  Artificial Sequence

<220>
<223>  Synthetic polynucleotide

<400>  587
cagaccaatt ctcatggtga aacagtgaaa accttgtatc tcagttacct tacagtcctt      60

cagaaggtag ctaaccatgt cgcgctactg caagctgcta gtacttccaa acaacaggtt      120


<210>  588
<211>  120
<212>  DNA
<213>  Artificial Sequence

<220>
<223>  Synthetic polynucleotide

<400>  588
attatttcaa tttatacagg tttgaatgtg ttaagtggaa atatcttttc ttctgccttt      60

tcccttcaag gaaacactta tcaaaaggat atgtgatcag gtattttcca gattcccaga      120


<210>  589
<211>  119
<212>  DNA
<213>  Artificial Sequence

<220>

<223>  Synthetic polynucleotide

<400>  589
ttttgtgcag aaaagcaaag atgcagcctt tgaaacactt tctgacccta aatacagtgg      60

aaaaatgaag gtaagtgctc ctctttcagg ttgcatacag acatgataca caaaatatt      119


<210>  590
<211>  120
<212>  DNA
<213>  Artificial Sequence

<220>
<223>  Synthetic polynucleotide

<400>  590
tgattttctg tggttcattt tcaggtcctt cagcagcttt taaatcattg caggaaaaac      60

agagataaag ttcttctctt ttctttttcc accaaggtga gttcatctaa agtatatcct      120


<210>  591
<211>  120
<212>  DNA
<213>  Artificial Sequence

<220>
<223>  Synthetic polynucleotide

<400>  591
tacttaacca agattcatgt ttgcccatct tcatacctct cgtctagttg cttgacgtgc      60

tacagcagta ctgtatggcg tctgggcttg attaccgacg acttgatgga agtacaaaat      120


<210>  592
<211>  120
<212>  DNA
<213>  Artificial Sequence

<220>
<223>  Synthetic polynucleotide

<400>  592
cagaggaaag actcaagatt gtaaaagagt tcaacagtac acaagatgtt aacatttgcc      60

ttgtctctac aatgtaagaa aattaaattt aataactaga tttttatcca attgtttttg      120


<210>  593
<211>  120
<212>  DNA
<213>  Artificial Sequence

<220>
<223>  Synthetic polynucleotide

<400>  593
tctttcctcc agggctggtg gactaggcct caattttgtc ggtgccaatg ttgttgtatt      60

atttgatcct acttggaatc cagccaatga tcttcaagcc attgacaggt ataatactga      120

<210>  594
<211>  120
<212>  DNA
<213>  Artificial Sequence

<220>
<223>  Synthetic polynucleotide

<400>  594
ccttttacag agcatatagg attggacaat gtagagatgt caaagtgctt aggctgatat      60

ccttgggaac tgtggaggaa atcatgtatt tacgacagat atacaagcag gtaaatatgt      120


<210>  595
<211>  120
<212>  DNA
<213>  Artificial Sequence

<220>
<223>  Synthetic polynucleotide

<400>  595
gcaaacactt caaaaatgtc ttgtgttttt tctgttttag caacttcact gtgtggtggt      60

tggaagtgaa aatgccaaac gatattttga agcagttcaa ggatctaaag agcatcaagg      120


<210>  596
<211>  120
<212>  DNA
<213>  Artificial Sequence

<220>
<223>  Synthetic polynucleotide

<400>  596
agagcttttt gggatccata acctcttcaa atttaggtcc caagggtctt gtcttacgaa      60

ggacatcctg gaggtgtgaa cttcttctct gaccttttca ataatatttt aaatacagtt      120


<210>  597
<211>  120
<212>  DNA
<213>  Artificial Sequence

<220>
<223>  Synthetic polynucleotide

<400>  597
ttttcttcat cttttttttc tctcttagca ccttctagcc accatggcaa cctcatctga      60

agaagttttg ctgattgtaa agaaagtgcg tcaaaagaag caggatggag ctctgtacct      120


<210>  598
<211>  120
<212>  DNA
<213>  Artificial Sequence

<220>

<223>  Synthetic polynucleotide

<400>  598
catggcagaa agaattgctt gggcacctga aggcaaagat agatttacaa tcagccatat      60

gtatgcagat attaaatgta agtcagctat actaagttct gatgtatttg tatgtcatag      120


<210>  599
<211>  120
<212>  DNA
<213>  Artificial Sequence

<220>
<223>  Synthetic polynucleotide

<400>  599
aactgccctc atgcttcttt ttaggccaga aaattagtcc agaaggaaaa gctaaaattc      60

agcttcagct ggtcctacat gcaggggaca caactaactt ccatttttcc aatgaaagca      120


<210>  600
<211>  120
<212>  DNA
<213>  Artificial Sequence

<220>
<223>  Synthetic polynucleotide

<400>  600
cagcagtgaa agagcgagat gcagtaaaag accttcttca gcagctgctg cccaaattca      60

agaggaaagc aaataaagaa ctggaagaga agaacaggtg ggaggaaaag aatagccttt      120


<210>  601
<211>  120
<212>  DNA
<213>  Artificial Sequence

<220>
<223>  Synthetic polynucleotide

<400>  601
agaagttcag ttatattcag tatattctgc tttacagaat gctgcaagaa gatcctgttt      60

tgtttcagct ttataaagac cttgttgtga gtcaagtgat cagtgctgag gaattctggg      120


<210>  602
<211>  120
<212>  DNA
<213>  Artificial Sequence

<220>
<223>  Synthetic polynucleotide

<400>  602
ccaatcgttt aaatgtgaat gcaacagata gttcttccac atccaatcat aagcaggatg      60

ttggcatttc tgctgcattt ctggtatgtg agccttctag atttctgaag aaaataaaaa      120

<210>  603
<211>  120
<212>  DNA
<213>  Artificial Sequence

<220>
<223>  Synthetic polynucleotide

<400>  603
ggctttgttt taggctgatg tccggcccca aactgatggc tgtaacggtc taagatataa      60

tttaacttct gatatcattg agtccatatt taggacctat ccagcaggta agaagaatca      120


<210>  604
<211>  120
<212>  DNA
<213>  Artificial Sequence

<220>
<223>  Synthetic polynucleotide

<400>  604
aaaatggaaa ttcagtatat aatatttgtg ggtttttttc cacagtaaaa atgaaatatg      60

cagaaaatgt tccccacaac atgacagaga aggaattctg gacacgtttt ttccagtccc      120


<210>  605
<211>  120
<212>  DNA
<213>  Artificial Sequence

<220>
<223>  Synthetic polynucleotide

<400>  605
attattttca cagggatcgg ctgaatacag ggtcaaagga tctctttgca gaatgtgcca      60

aaatagatga aaaaggtaac tgtttatctc tgatagacac tggtatttaa cttgccaccc      120


<210>  606
<211>  120
<212>  DNA
<213>  Artificial Sequence

<220>
<223>  Synthetic polynucleotide

<400>  606
ttattttgtt gattttctag gcctaaaaac aatggtttca ttaggagtga aaaacccact      60

actagattta acagctttgg aagataaacc attagatgag gtaagaagca ataaaagaag      120


<210>  607
<211>  120
<212>  DNA
<213>  Artificial Sequence

<220>

<223>  Synthetic polynucleotide

<400>  607
tataataaga aaagcagaac ctttttaaaa aatttttaat ctattatttc tcacagggct      60

atggcatttc ctctgtgcca tctgcttcca attctaaatc cataaaagag aatagtaatg      120


<210>  608
<211>  120
<212>  DNA
<213>  Artificial Sequence

<220>
<223>  Synthetic polynucleotide

<400>  608
ctgccatcat caagagattt aaccatcaca gtgccatggt cctggcagct ggactcagaa      60

aacagttaag tataaatgca gaggtgcagt aactgggctt ttcaggatat ccagatggag      120


<210>  609
<211>  120
<212>  DNA
<213>  Artificial Sequence

<220>
<223>  Synthetic polynucleotide

<400>  609
tcccctttat tttaagagaa gcacaaaatg aacaaactag tgagcccagc aacatggatg      60

gaaattccgg agatgcagac tgctttcagc cagcagtcaa aagggtatgg gcaaaaaaat      120


<210>  610
<211>  120
<212>  DNA
<213>  Artificial Sequence

<220>
<223>  Synthetic polynucleotide

<400>  610
gtgttaataa ttataggcga aattacaaga gtccattgaa tatgaagact tggggaaaaa      60

taattctgta aaaacgattg cactaaacct caagaagtca gataggtaag tttggtcaat      120


<210>  611
<211>  120
<212>  DNA
<213>  Artificial Sequence

<220>
<223>  Synthetic polynucleotide

<400>  611
ggtattatca tggtccaact ccaatccagt cactacagta tgcaacaagt caggacatta      60

ttaattcttt tcaaagtatt agacaagaaa tggaagctta tacacccaag ttaactcagg      120

<210> 612
<211> 120
<212> DNA
<213> Artificial Sequence

<220>
<223> Synthetic polynucleotide

<400> 612
ttcatttttt tcaggttctc tcaagtagtg ctgccagtag taccatcaca gcactgtcac 60

ctggaggggc acttatgcag ggaggaacac agcaagccat aaaccgtatg tgccgggcca 120


<210> 613
<211> 120
<212> DNA
<213> Artificial Sequence

<220>
<223> Synthetic polynucleotide

<400> 613
agagatggtg ccaaatgata ttcaatctga attgaaacac ttatatgtag ctgttggaga 60

acttctacga catttctggt cctgctttcc tgttaatacg ccattcctag aagaaaaggt 120


<210> 614
<211> 120
<212> DNA
<213> Artificial Sequence

<220>
<223> Synthetic polynucleotide

<400> 614
ctcatctttt ttaggtagtg aaaatgaaaa gtaatttgga acgattccaa gttacgaagc 60

tctgtccatt ccaagaaaag attcggagac agtatttaag cacaaatgta aggcagcaat 120


<210> 615
<211> 120
<212> DNA
<213> Artificial Sequence

<220>
<223> Synthetic polynucleotide

<400> 615
ttgctgtgtt tttcagttgg taagtcacat agaagagatg ctccagacag cctacaacaa 60

gctccacaca tggcagtcac ggcgtctgat gaagaaaacg tgaggtggcc atgatgctta 120


<210> 616
<211> 120
<212> DNA
<213> Artificial Sequence

<220>

<223>  Synthetic polynucleotide

<400>  616
ttttacagat tattgaataa taaaatacag ttttgaaaaa aatggatgaa gaacctgaaa      60

gaactaagcg atgggaagga ggctatgaaa gaacatggta aggagagctt tattgccctg      120


<210>  617
<211>  120
<212>  DNA
<213>  Artificial Sequence

<220>
<223>  Synthetic polynucleotide

<400>  617
aacaaataat tatgacttat agggagattc ttaaagaaga tgaatctgga tcacttaaag      60

ctacaataga agacattcta ttcaaggcaa agagaaaaag gtatgtaacc ttcctatgta      120


<210>  618
<211>  120
<212>  DNA
<213>  Artificial Sequence

<220>
<223>  Synthetic polynucleotide

<400>  618
agtatatgga attacattaa aatgtttgta taattttaac agagtatttg agcaccatgg      60

acaagttcga cttggaatgg tatgtcatta tttttctttt tactagtaca gaactagttt      120


<210>  619
<211>  120
<212>  DNA
<213>  Artificial Sequence

<220>
<223>  Synthetic polynucleotide

<400>  619
ttcttttttc cgatagatgc gccaccttta tgtggtagta gatggatcaa gaacaatgga      60

agaccaagat ttaaagccta atagactgac gtgtacttta aaggtaaaat ttaagtttat      120


<210>  620
<211>  120
<212>  DNA
<213>  Artificial Sequence

<220>
<223>  Synthetic polynucleotide

<400>  620
cctttatcta gtatgtcttt ttttgtttaa acagttgttg gaatactttg tagaggaata      60

ttttgatcaa aatcctatta gtcaggtacg tatctaagtg atagaattca gaattagatt      120

<210>  621
<211>  120
<212>  DNA
<213>  Artificial Sequence

<220>
<223>  Synthetic polynucleotide

<400>  621
atattaataa taatttttgt ttttatttca agattggaat aattgtaact aagagtaaaa      60

gagctgaaaa attgactgaa ctttcaggta tgcataaaat tacctttaca tgactcaagg      120


<210>  622
<211>  120
<212>  DNA
<213>  Artificial Sequence

<220>
<223>  Synthetic polynucleotide

<400>  622
attttaggaa acccaagaaa acatataacg tctttgaaga aagctgtgga tatgacctgc      60

catggagagc catctcttta taattcccta agcatagcta tgcagactct aaagttagta      120


<210>  623
<211>  120
<212>  DNA
<213>  Artificial Sequence

<220>
<223>  Synthetic polynucleotide

<400>  623
atctttcttt taagacacat gcctggacat acaagtcgag aagtactaat catctttagc      60

agccttacaa cttgcgatcc atctaatatt tatgatctaa tcaaggtaga ccaaaaaatc      120


<210>  624
<211>  120
<212>  DNA
<213>  Artificial Sequence

<220>
<223>  Synthetic polynucleotide

<400>  624
cttggatttt atgaagaccc taaaggcagc taaaattaga gtatctgtta ttggattgtc      60

tgcagaagtt cgcgtttgca ctgtacttgc tcgtgaaact ggtggtatat atataattta      120


<210>  625
<211>  120
<212>  DNA
<213>  Artificial Sequence

<220>

<223>  Synthetic polynucleotide

<400>  625
tgttaggcac gtaccatgtt attttagatg aaagccatta caaagagttg ctcacacatc      60

atgttagtcc tcctcctgct agctcaagtt ctgaatgctc acttattcgt atgggtaagt     120


<210>  626
<211>  120
<212>  DNA
<213>  Artificial Sequence

<220>
<223>  Synthetic polynucleotide

<400>  626
agaaagtaat acattttctt ctatgaagga tttcctcagc acaccattgc ttctttatct      60

gaccaggatg caaaaccctc tttcagcatg gcgtaagtaa agaccttgaa aatatcagtg     120


<210>  627
<211>  120
<212>  DNA
<213>  Artificial Sequence

<220>
<223>  Synthetic polynucleotide

<400>  627
ccctacaggc atttggatgg caatactgag ccagggctta cattaggagg ctatttctgc      60

ccacagtgtc gggcaaagta ctgtgagcta cctgttgagt gtaaaatctg tggtaagaaa     120


<210>  628
<211>  120
<212>  DNA
<213>  Artificial Sequence

<220>
<223>  Synthetic polynucleotide

<400>  628
tgtgttaggt cttactttgg tgtctgctcc ccacttggca cggtcttacc atcatttgtt      60

tcctttggat gcttttcaag aaattcccct agaagaatat aatggagaaa ggtatttcag     120


<210>  629
<211>  120
<212>  DNA
<213>  Artificial Sequence

<220>
<223>  Synthetic polynucleotide

<400>  629
gtaaattcta agaaaaaaca ttgttaaact tttttttcag attttgttat ggatgtcagg      60

gggaattgaa agaccaacat gtaagttctt tggctttcta aatattaagt aatgtacaag     120

<210>  630
<211>  120
<212>  DNA
<213>  Artificial Sequence

<220>
<223>  Synthetic polynucleotide

<400>  630
tagctgtgct gtaattaact tttggaaact agtttatcac ttttcttctt ttcactacag      60

gtttatgttt gtgctgtgtg ccaaaatgtt ttctgtgtgg actgtgatgt ttttgttcat      120


<210>  631
<211>  120
<212>  DNA
<213>  Artificial Sequence

<220>
<223>  Synthetic polynucleotide

<400>  631
gattctctac actgttgccc tggctgtatt cataagattc cagctccttc aggtgtttga      60

ttccagcatg tagtatacat tgtatgtgtt aaaaagaaat ttgcaactgt gaataaaagg      120


<210>  632
<211>  120
<212>  DNA
<213>  Artificial Sequence

<220>
<223>  Synthetic polynucleotide

<400>  632
tttgagacgc tcccctgacc accacttgct ctgcgctgag gtgctgggac agccatggtt      60

tcagacggtg aggaccctgc agggcgggac ttcgactccg gggctcggct gtctcggtcc      120


<210>  633
<211>  120
<212>  DNA
<213>  Artificial Sequence

<220>
<223>  Synthetic polynucleotide

<400>  633
tttcttgtta atattttcag aagatgaatt gaatcttctg gttattgtag ttgatgccaa      60

cccaatttgg tggggaaagc aagcattaaa ggaatctcag gtaagactgc ttgaggaggc      120


<210>  634
<211>  120
<212>  DNA
<213>  Artificial Sequence

<220>

<223>  Synthetic polynucleotide

<400>  634
caacagttca ctttatccaa atgcatagat gccgtgatgg tgctgggaaa ttcgcattta      60

ttcatgaatc gttccaacaa acttgctgtg atagcaagtc acattcaaga aaggtatgac      120


<210>  635
<211>  120
<212>  DNA
<213>  Artificial Sequence

<220>
<223>  Synthetic polynucleotide

<400>  635
ggcctggtgt aaccaggttt tttcccctgc tgtttcagcc gattcttata tcctggaaag      60

aatggcagac ttggagactt cttcggagac cctggcaacc ctcctgaatt taatccctct      120


<210>  636
<211>  120
<212>  DNA
<213>  Artificial Sequence

<220>
<223>  Synthetic polynucleotide

<400>  636
gggagtaaag atggaaaata cgaactttta acctcagcaa atgaagttat tgttgaagag      60

attaaagatc taatgaccaa aagtaacaac ttttaaacat tgttattttg caaatagtgt      120


<210>  637
<211>  120
<212>  DNA
<213>  Artificial Sequence

<220>
<223>  Synthetic polynucleotide

<400>  637
cccttttaat gttttctgtt atttgcaggt gacataaagg gtcaacatac agaaactttg      60

ctggcaggat ccctggccaa agccctttgc tgtatccttg gtgtctgaat catttagaag      120


<210>  638
<211>  120
<212>  DNA
<213>  Artificial Sequence

<220>
<223>  Synthetic polynucleotide

<400>  638
ttatgaagca aaataatatt tttgtattcc ttaatgttaa gaaattaaga cattcataga      60

atgaacaagg aagttaaagg taagagccta cgttttccta tgagaactgt aatcctactt      120

<210>  639
<211>  120
<212>  DNA
<213>  Artificial Sequence

<220>
<223>  Synthetic polynucleotide

<400>  639
ttagtaagat ttagttaaat ttttttctgc ttttttctct ttgtagacaa tcaggaaatg      60

aaatcaagga tattggtaag ataaaaaaat cattactttt ttatatgaca actataatta      120


<210>  640
<211>  120
<212>  DNA
<213>  Artificial Sequence

<220>
<223>  Synthetic polynucleotide

<400>  640
ttaacatgac tgtgattttt aggtgattaa ggctgcagaa gacagtgcgt tgcagtatat      60

gaacttcatg aatgtcatct ttgcagcaca gaaacaggtg aactgagagc ctgccgttta      120


<210>  641
<211>  120
<212>  DNA
<213>  Artificial Sequence

<220>
<223>  Synthetic polynucleotide

<400>  641
attcctgtaa tattttcaaa atgtttcttt tagaatattt tgattgatgc ctgtgtttta      60

gactccgact caggctcctc ccaacaggta tgttttaaaa ccatgctttg tgtcggtggt      120


<210>  642
<211>  120
<212>  DNA
<213>  Artificial Sequence

<220>
<223>  Synthetic polynucleotide

<400>  642
ggcagcaagc cgcctgtgtc ttgcaggctt gtgacatcac gggaggactg tacctgaagg      60

tgcctcagat gccttctctt ctgcagtatt tgctggtaag gagacagcag cggcgaccct      120


<210>  643
<211>  120
<212>  DNA
<213>  Artificial Sequence

<220>

<223>  Synthetic polynucleotide

<400>  643
atttttttcc catgttttaa ataaaagttt cttttgtttg tttgttttac agtgggtgtt      60

tcttcccgat caagatcaga gatctcagtt aatcctccca cccccagttc atgttgacta      120


<210>  644
<211>  120
<212>  DNA
<213>  Artificial Sequence

<220>
<223>  Synthetic polynucleotide

<400>  644
cagggctgct tgcttctgtc atcgaaatct cattgaaatt ggttatgtct gttctgtgtg      60

tttgtcaagt aagttaatgt acctagtttt tctttttttt ctatgttggg gggcaggaaa      120


<210>  645
<211>  120
<212>  DNA
<213>  Artificial Sequence

<220>
<223>  Synthetic polynucleotide

<400>  645
ttttcagcca ccctattgtt tctttttttt ttttaattac agtattctgc aatttcagcc      60

ccatttgtac tacgtgcgag taagtatctt tgagattgtg tgggtggcta atacttcaca      120


<210>  646
<211>  120
<212>  DNA
<213>  Artificial Sequence

<220>
<223>  Synthetic polynucleotide

<400>  646
aatgttttcc tctctgtaat ttcaggacag cctttaaaat ttctctgcct ccagtgctga      60

aagccaagaa aaagaaactg aaagtgtctg cctgaggata aaatattttc cccatctttt      120


<210>  647
<211>  120
<212>  DNA
<213>  Artificial Sequence

<220>
<223>  Synthetic polynucleotide

<400>  647
acactggcat ggatggtgag gttgcacttc tgacgtttgc attcctcagg tgatggagag      60

caccccttca aggggactga accgagtaca cctacaatgc aggaatctgc aggaattctt      120

<210>  648
<211>  120
<212>  DNA
<213>  Artificial Sequence

<220>
<223>  Synthetic polynucleotide

<400>  648
agggggcctg agccctgggg tattggaccg attgtatggg caccctgcca catgtctggc      60

tgtcttcagg tgagaagccc cttcatggca gggaaatgta atggggtctg cggagtggaa      120


<210>  649
<211>  120
<212>  DNA
<213>  Artificial Sequence

<220>
<223>  Synthetic polynucleotide

<400>  649
ctgtacaggg agctcccatc cttggctaag aactgggtga tgcggatgct ctttctggag      60

cagcctttgc cacaggctgc tgtagctctg tgggtaaaga aggaattcag caagtaagtc      120


<210>  650
<211>  120
<212>  DNA
<213>  Artificial Sequence

<220>
<223>  Synthetic polynucleotide

<400>  650
gggcctcctt tttgttttcc aaatacccta ctcacctctc tgcttctgtt ccagggctca      60

ggaggaaagt acagggctgc tgagcggcct ccggatctgg cacacacagc tgctcccagg      120


<210>  651
<211>  120
<212>  DNA
<213>  Artificial Sequence

<220>
<223>  Synthetic polynucleotide

<400>  651
cgggctccag ggcctcatcc tcaaccccat tttccgccag aacctccgca ttgcccttct      60

gggtgggtat gtcacttctc tctcttccta agctagggca ggggaactgc tgcttattaa      120


<210>  652
<211>  120
<212>  DNA
<213>  Artificial Sequence

<220>

<223>  Synthetic polynucleotide

<400>  652
cctggccgta gggggaaggc ctggtctgat gacacaagtc agctgggacc agacaagcat      60

gcccgggacg ttccctccct tgacaagtac gccgaggagc gatgggaggt aagcacttgg      120


<210>  653
<211>  120
<212>  DNA
<213>  Artificial Sequence

<220>
<223>  Synthetic polynucleotide

<400>  653
ctcttctgtt cttcaggtgg tcttgcactt catggtgggc tcccccagtg cagctgtcag      60

ccaggacttg gctcagctcc tcagccaggc tgggctcatg aagaggtgag gaagccggag      120


<210>  654
<211>  120
<212>  DNA
<213>  Artificial Sequence

<220>
<223>  Synthetic polynucleotide

<400>  654
ctagtactga acctggagag ccgccctgca ttacttccgc tggcttccag ttcctgttgc      60

tggacacccc ggctcagctc tggtacttta tgttgcagta tttgcagaca gcccaggtga      120


<210>  655
<211>  120
<212>  DNA
<213>  Artificial Sequence

<220>
<223>  Synthetic polynucleotide

<400>  655
tttgtctctg cctctttctc cctagagccg gggcatggac ctggtagaga ttctctcctt      60

cctcttccag ctcagcttct ctactctggg caaggtaagc agggggctga aaggtataga      120


<210>  656
<211>  120
<212>  DNA
<213>  Artificial Sequence

<220>
<223>  Synthetic polynucleotide

<400>  656
gaaacctctg ttcctcagga ttactctgtg gaaggtatga gtgattctct gttgaacttc      60

ctgcaacatc tgcgtgagtt tgggcttgtt ttccagagga aggtatgagc gcctagataa      120

<210>  657
<211>  120
<212>  DNA
<213>  Artificial Sequence

<220>
<223>  Synthetic polynucleotide

<400>  657
agatgtaagg cagtgacttc tgagacaagg catctgcctt tctattcttt tcagaggaaa      60

tctcggcgtt actaccccac acgcctggcc atcaatctct catcaggtgt ctctggagct      120


<210>  658
<211>  120
<212>  DNA
<213>  Artificial Sequence

<220>
<223>  Synthetic polynucleotide

<400>  658
gggggcactg tgcatcagcc aggtttcatt gtcgtggaaa ccaattaccg actgtatgcc      60

tacacgggtg aggcgggaca gagggcccct ggaagaggag gttgggggtg agggaatgcc      120


<210>  659
<211>  120
<212>  DNA
<213>  Artificial Sequence

<220>
<223>  Synthetic polynucleotide

<400>  659
agggagtctg ggtgtggggg tggcctcctc atcctctttc tatccctggc tcagagtcgg      60

agctgcagat tgccctcatt gccctcttct ctgagatgct ctatcggttc cccaacatgg      120


<210>  660
<211>  120
<212>  DNA
<213>  Artificial Sequence

<220>
<223>  Synthetic polynucleotide

<400>  660
tggtggcgca ggtgacccgg gagagtgtgc agcaggcaat cgccagtggc atcacagccc      60

agcaggtatt cccacttggg agaggtggag caggaagaca ggctgcactt gggctgcggg      120


<210>  661
<211>  120
<212>  DNA
<213>  Artificial Sequence

<220>

<223>  Synthetic polynucleotide

<400>  661
tcttgctgga gccctcatgc cattcttgtc tgttttccta gataatccat ttcctaagga      60

caagagccca cccagtgatg ctcaaacagg tatagacagg ctccaagatg tcagaggctg      120


<210>  662
<211>  120
<212>  DNA
<213>  Artificial Sequence

<220>
<223>  Synthetic polynucleotide

<400>  662
gcagctggtg atgacatgat ggaaaagaaa aaggggcatc caaatctggg gaagaaacag      60

agggccgggt tgtctggggc agtattctga gtccctacag tcaacccttg ctccttgcag      120


<210>  663
<211>  120
<212>  DNA
<213>  Artificial Sequence

<220>
<223>  Synthetic polynucleotide

<400>  663
acacctgtgc tgccccccac catcaccgac cagatccggc tctgggagct ggaaagggac      60

agactccggt tcactgaggg tgagtagctt ctggtggcca agtcttggtc attggccaga      120


<210>  664
<211>  120
<212>  DNA
<213>  Artificial Sequence

<220>
<223>  Synthetic polynucleotide

<400>  664
tctccccgcg cccctcccgt cctgccgacc ccaggtgtcc tgtataacca gttcctgtcg      60

caagtggact ttgagctgct gctggcccac gcgcgggagc tgggcgtgct cgtgttcgag      120


<210>  665
<211>  120
<212>  DNA
<213>  Artificial Sequence

<220>
<223>  Synthetic polynucleotide

<400>  665
aactcggcca agcggctcat ggtggtgacc ccggccgggc acagcgacgt caagcgcttt      60

tggaagcggc agaaacatag ctcctgagag cgcgggactt ggacacggac ctcggcgggc      120

<210>  666
<211>  120
<212>  DNA
<213>  Artificial Sequence

<220>
<223>  Synthetic polynucleotide

<400>  666
tttattagca ttcttcaggt catctgaacc ttctgagaaa acatggtcaa cgtcttgaaa      60

ggagtgctta tagaatggtt agtagttttg atactgcatg agttttaagt ttaaatattg      120


<210>  667
<211>  120
<212>  DNA
<213>  Artificial Sequence

<220>
<223>  Synthetic polynucleotide

<400>  667
tgtcttacaa tcatgtgttt gtctttacag tgatcctgcc atgaagcagt ttctgctgta      60

cttggatgag tccaatgccc tggggaagaa gttcatcatt caagacattg atgacactca      120


<210>  668
<211>  120
<212>  DNA
<213>  Artificial Sequence

<220>
<223>  Synthetic polynucleotide

<400>  668
cgtctttgta atagcagaat tggttaatgt cctccaggag cgagtgggtg aattaatgga      60

ccaaaatgct ttttccctta cccagaaatg aaaatactca atatggacca tttaggaatt      120


<210>  669
<211>  120
<212>  DNA
<213>  Artificial Sequence

<220>
<223>  Synthetic polynucleotide

<400>  669
caggacccgc ttctctgaaa ggctctcctt gcagctgctt agacgctgga tttttttcgg      60

gtagtggaaa accaggtaag caccgaagtc cacttgcctt ttaatttatt tttttatcac      120


<210>  670
<211>  120
<212>  DNA
<213>  Artificial Sequence

<220>

<223>  Synthetic polynucleotide

<400>  670
aagactgcct cccgctttgt gtgccccgct ccagcagcct cccgcgacga tgcccctcaa      60

cgttagcttc accaacagga actatgacct cgactacgac tcggtgcagc cgtatttcta      120


<210>  671
<211>  120
<212>  DNA
<213>  Artificial Sequence

<220>
<223>  Synthetic polynucleotide

<400>  671
ctgcgacgag gaggagaact tctaccagca gcagcagcag agcgagctgc agcccccggc      60

gcccagcgag gatatctgga agaaattcga gctgctgccc accccgcccc tgtcccctag      120


<210>  672
<211>  120
<212>  DNA
<213>  Artificial Sequence

<220>
<223>  Synthetic polynucleotide

<400>  672
ccgccgctcc gggctctgct cgccctccta cgttgcggtc acacccttct cccttcgggg      60

agacaacgac ggcggtggcg ggagcttctc cacggccgac cagctggaga tggtgaccga      120


<210>  673
<211>  120
<212>  DNA
<213>  Artificial Sequence

<220>
<223>  Synthetic polynucleotide

<400>  673
gctgctggga ggagacatgg tgaaccagag tttcatctgc gacccggacg acgagacctt      60

catcaaaaac atcatcatcc aggactgtat gtggagcggc ttctcggccg ccgccaagct      120


<210>  674
<211>  120
<212>  DNA
<213>  Artificial Sequence

<220>
<223>  Synthetic polynucleotide

<400>  674
cgtctcagag aagctggcct cctaccaggc tgcgcgcaaa gacagcggca gcccgaaccc      60

cgcccgcggc cacagcgtct gctccacctc cagcttgtac ctgcaggatc tgagcgccgc      120

<210>  675
<211>  120
<212>  DNA
<213>  Artificial Sequence

<220>
<223>  Synthetic polynucleotide

<400>  675
cgcctcagag tgcatcgacc cctcggtggt cttcccctac cctctcaacg acagcagctc      60

gcccaagtcc tgcgcctcgc aagactccag cgccttctct ccgtcctcgg attctctgct      120


<210>  676
<211>  120
<212>  DNA
<213>  Artificial Sequence

<220>
<223>  Synthetic polynucleotide

<400>  676
ctcctcgacg gagtcctccc cgcagggcag ccccgagccc ctggtgctcc atgaggagac      60

accgcccacc accagcagcg actctggtaa gcgaagcccg cccaggcctg tcaaaagtgg      120


<210>  677
<211>  120
<212>  DNA
<213>  Artificial Sequence

<220>
<223>  Synthetic polynucleotide

<400>  677
ctatttcctt tcttaaagag gaggaacaag aagatgagga agaaatcgat gttgtttctg      60

tggaaaagag gcaggctcct ggcaaaaggt cagagtctgg atcaccttct gctggaggcc      120


<210>  678
<211>  120
<212>  DNA
<213>  Artificial Sequence

<220>
<223>  Synthetic polynucleotide

<400>  678
acagcaaacc tcctcacagc ccactggtcc tcaagaggtg ccacgtctcc acacatcagc      60

acaactacgc agcgcctccc tccactcgga aggactatcc tgctgccaag agggtcaagt      120


<210>  679
<211>  120
<212>  DNA
<213>  Artificial Sequence

<220>

<223>  Synthetic polynucleotide

<400>  679
tggacagtgt cagagtcctg agacagatca gcaacaaccg aaaatgcacc agccccaggt      60

cctcggacac cgaggagaat gtcaagaggc gaacacacaa cgtcttggag cgccagagga      120


<210>  680
<211>  120
<212>  DNA
<213>  Artificial Sequence

<220>
<223>  Synthetic polynucleotide

<400>  680
ggaacgagct aaaacggagc ttttttgccc tgcgtgacca gatcccggag ttggaaaaca      60

atgaaaaggc ccccaaggta gttatcctta aaaaagccac agcatacatc ctgtccgtcc      120


<210>  681
<211>  120
<212>  DNA
<213>  Artificial Sequence

<220>
<223>  Synthetic polynucleotide

<400>  681
aagcagagga gcaaaagctc atttctgaag aggacttgtt gcggaaacga cgagaacagt      60

tgaaacacaa acttgaacag ctacggaact cttgtgcgta aggaaaagta aggaaaacga      120


<210>  682
<211>  120
<212>  DNA
<213>  Artificial Sequence

<220>
<223>  Synthetic polynucleotide

<400>  682
cttcaggatg gttcgtacta agacatggac cctgaagaag cactttgttg gctatcctac      60

taatagtgac tttgagttga agacagctga gctcccaccc ttaaaaaatg gaggtaagtc      120


<210>  683
<211>  120
<212>  DNA
<213>  Artificial Sequence

<220>
<223>  Synthetic polynucleotide

<400>  683
gagtttctaa ttgcctatgg aatgctttat tttgtagagg tcctgcttga agctttgttc      60

ctcaccgtgg atccctacat gaggtattat ttatttcgcc cctcaaaatg ctaatgctgt      120

<210>  684
<211>  120
<212>  DNA
<213>  Artificial Sequence

<220>
<223>  Synthetic polynucleotide

<400>  684
tttttttttt tttttttttt tgcagagtgg cagccaaaag attgaaggaa ggtgatacaa      60

tgatggggca gcaagtggcc aagtaattat ttccatttgt cccagtgctt ggaaaatatg      120


<210>  685
<211>  119
<212>  DNA
<213>  Artificial Sequence

<220>
<223>  Synthetic polynucleotide

<400>  685
tctcttaaaa aaaccaaaca tctcattctt tattttttta aattttgtgt ctcaatgact      60

agccagttat tataatacca ttggtagaaa tcgtctatgg atctttactg gaatttcag      119


<210>  686
<211>  120
<212>  DNA
<213>  Artificial Sequence

<220>
<223>  Synthetic polynucleotide

<400>  686
acatgcctta atgccttaat gtgctttatc tttcagagtt gtggaaagta aaaatgtagc      60

cctaccaaaa ggaactattg tactggcttc tccaggctgg acaacgcact ccatttctga      120


<210>  687
<211>  120
<212>  DNA
<213>  Artificial Sequence

<220>
<223>  Synthetic polynucleotide

<400>  687
tgggaaagat ctggaaaagc tgctgacaga gtggccagac acaataccac tgtctttggc      60

tctggggaca gttggcatgc cagggtgagt ttcatggata tattccattt gtttgaatgt      120


<210>  688
<211>  120
<212>  DNA
<213>  Artificial Sequence

<220>

<223>  Synthetic polynucleotide

<400>  688
gcctgactgc ctactttggc ctacttgaaa tctgtggtgt gaagggtgga gaaacagtga      60

tggttaatgc agcagctgga gctgtgggct cagtcgtggg gcagattgca aagctcaagg      120


<210>  689
<211>  120
<212>  DNA
<213>  Artificial Sequence

<220>
<223>  Synthetic polynucleotide

<400>  689
tgacttattc atggtttcct tttttttttt ttttctactt agggctgcaa agttgttgga      60

gcagtagggt ctgatgaaaa ggttgcctac cttcaaaagc ttggatttga tgtcgtcttt      120


<210>  690
<211>  120
<212>  DNA
<213>  Artificial Sequence

<220>
<223>  Synthetic polynucleotide

<400>  690
aactacaaga cggtagagtc tttggaagaa accttgaaga aagcgtctcc tgatggttat      60

gattgttatt ttgataatgt aagtacaaac tggacttaat atctaattta ttatggaagt      120


<210>  691
<211>  120
<212>  DNA
<213>  Artificial Sequence

<220>
<223>  Synthetic polynucleotide

<400>  691
gtttaggtag gtggagagtt ttcaaacact gttatcggcc agatgaagaa atttggaagg      60

attgccatat gtggagccat ctctacatat aacagaaccg gcccacttcc cccaggtaat      120


<210>  692
<211>  120
<212>  DNA
<213>  Artificial Sequence

<220>
<223>  Synthetic polynucleotide

<400>  692
gcccaccccc agagattgtt atctatcagg agcttcgcat ggaagctttt gtcgtctacc      60

gctggcaagg agatgcccgc caaaaagctc tgaaggactt gctgaaatgg gtcttagagg      120

<210>  693
<211>  120
<212>  DNA
<213>  Artificial Sequence

<220>
<223>  Synthetic polynucleotide

<400>  693
ttgagaatat taaattaagt tttaaaattt gttgttgctt tgatacaggg taaaatccag      60

tacaaggaat atatcattga aggatttgaa aacatgccag ctgcatttat gggaatgctg      120


<210>  694
<211>  120
<212>  DNA
<213>  Artificial Sequence

<220>
<223>  Synthetic polynucleotide

<400>  694
aaaggagata atttggggaa gacaatagtg aaagcatgaa aaagaggaca catggaatct      60

ggaggccatt tagatgatta gttaatttgt ttttcaccat ttagcaaaaa tgtatactac      120


<210>  695
<211>  120
<212>  DNA
<213>  Artificial Sequence

<220>
<223>  Synthetic polynucleotide

<400>  695
cttaaatgtc ttaagaaata gtactcataa tgagtttgag ctacttaata aaatacattt      60

aagtggtatg taattagtga tggaggatgg aagtttcaaa gtcaacaaca accagtcacc      120


<210>  696
<211>  120
<212>  DNA
<213>  Artificial Sequence

<220>
<223>  Synthetic polynucleotide

<400>  696
gatcaaaaga gaaaatgaag aagattgaag cttcaaagca gaaaaatgaa gggaatatgt      60

atcattcacc attaccttag aaggcaaggg tccactttac aaaaggacta caaacattta      120


<210>  697
<211>  120
<212>  DNA
<213>  Artificial Sequence

<220>

<223>  Synthetic polynucleotide

<400>  697
gggtggctga cccccagcat cctcgggagc gaccatggac tccctggccg agtctcggtg      60

gcctccgggc ctggcagtca tgaaggtgag tgcttcgggg agtttgggag ctcccgggtg      120


<210>  698
<211>  120
<212>  DNA
<213>  Artificial Sequence

<220>
<223>  Synthetic polynucleotide

<400>  698
atgcatattt gcatttcaga caatagatga tttgctgcgg tgtggaattt gcttcgagta      60

tttcaacatt gcaatgataa tacctcagtg ttcacataac tgtaagtatg ttttgttcct      120


<210>  699
<211>  120
<212>  DNA
<213>  Artificial Sequence

<220>
<223>  Synthetic polynucleotide

<400>  699
aacattatgt tgcactttgt gttttgcaga ctgctctctc tgtataagaa aatttctgtc      60

ctataaaact cagtgtccaa cttgctgtgt ggtgagtttt tgtttccttt ttttaaaact      120


<210>  700
<211>  120
<212>  DNA
<213>  Artificial Sequence

<220>
<223>  Synthetic polynucleotide

<400>  700
ccatctgttg tttgtttttt ctagactgtc acagagccgg atctgaaaaa taaccgcata      60

ttagatgaac tggtaaaaag cttgaatttt gcacggtatg atttaatttt gtgactaacc      120


<210>  701
<211>  120
<212>  DNA
<213>  Artificial Sequence

<220>
<223>  Synthetic polynucleotide

<400>  701
tgtgcttcaa ggaatcatct gctgcagttt gctttagagt caccagccaa atctcctgct      60

tcttcctctt caaagaatct tgctgtcaaa gtatatactc ctgtagcctc cagacagtct      120

<210>  702
<211>  120
<212>  DNA
<213>  Artificial Sequence

<220>
<223>  Synthetic polynucleotide

<400>  702
ttaaagcagg ggagcaggtt aatggataat ttcttgatca gagaaatgag tggttctaca      60

tcagagttgt tgataaaaga aaataaaagc aaattcagcc ctcaaaaaga ggcgagccct      120


<210>  703
<211>  120
<212>  DNA
<213>  Artificial Sequence

<220>
<223>  Synthetic polynucleotide

<400>  703
gctgcaaaga ccaaagagac acgttctgta gaagagatcg ctccagatcc ctcagaggct      60

aagcgtcctg agccaccctc gacatccact ttgaaacaag ttactaaagg taggaagtta      120


<210>  704
<211>  120
<212>  DNA
<213>  Artificial Sequence

<220>
<223>  Synthetic polynucleotide

<400>  704
aaaaatacag tggattgtcc tgtttgcggg gttaacattc cagaaagtca cattaataag      60

catttagaca gctgtttatc acgcgaagag aagaaggaaa gcctcagaag gtaaggaagt      120


<210>  705
<211>  120
<212>  DNA
<213>  Artificial Sequence

<220>
<223>  Synthetic polynucleotide

<400>  705
atcatcaggt atcttccccc cttttcagtt ctgttcacaa aaggaagccg ctgcccaaaa      60

ctgtatataa tttgctctct gatcgtgatt taaagaaaaa gctaaaagag catggattat      120


<210>  706
<211>  120
<212>  DNA
<213>  Artificial Sequence

<220>

<223>  Synthetic polynucleotide

<400>  706
ctattcaagg aaataaacaa cagctcatta aaaggcacca agaatttgta cacatgtaca      60

atgcccaatg cgatgctttg catcctaaat caggtaaagt aataaaacag tgagttatgc      120


<210>  707
<211>  120
<212>  DNA
<213>  Artificial Sequence

<220>
<223>  Synthetic polynucleotide

<400>  707
gttattttct tattttaaca gctgctgaaa tagttcgaga aatcgaaaat atagagaaga      60

ctaggatgcg tcttgaagct agtaaactca atgaaagtgt atgttaatgg gctatttcag      120


<210>  708
<211>  120
<212>  DNA
<213>  Artificial Sequence

<220>
<223>  Synthetic polynucleotide

<400>  708
tttattaagg tctttttttg attattttag gtaatggttt ttacaaagga ccaaacagaa      60

aaggaaatag atgaaatcca cagtaaatat cgtaagttgc acgactttat ttaatttgta      120


<210>  709
<211>  120
<212>  DNA
<213>  Artificial Sequence

<220>
<223>  Synthetic polynucleotide

<400>  709
ggttaacttt catagtccta gctttactga gaagatcttt tgaatttcag gtaaaaaaca      60

taagagtgaa tttcagcttc tggtggatca ggctagaaaa ggatacaaga aaattgctgg      120


<210>  710
<211>  120
<212>  DNA
<213>  Artificial Sequence

<220>
<223>  Synthetic polynucleotide

<400>  710
aatgtcacaa aaaacagtaa caataacaaa agaagatgaa tctacagaaa agctatcttc      60

tgtatgcatg ggtaagagac tataattaaa aacagacata tgtcgaattt cattatttac      120

<210>  711
<211>  120
<212>  DNA
<213>  Artificial Sequence

<220>
<223>  Synthetic polynucleotide

<400>  711
aagttgtaga ttttaaggta ctcttaaatt tattttttcc taggacagga agataatatg      60

acctcagtaa caaaccactt ttctcaatca aagctggact ccccagagga attggaacct      120


<210>  712
<211>  120
<212>  DNA
<213>  Artificial Sequence

<220>
<223>  Synthetic polynucleotide

<400>  712
gacagagaag aggattcttc tagctgtatt gatattcaag aagttctttc ttcatcagaa      60

tcagattcat gcaataggta agaaacatgc agtagttgat tattttaaat atctacttca      120


<210>  713
<211>  120
<212>  DNA
<213>  Artificial Sequence

<220>
<223>  Synthetic polynucleotide

<400>  713
tgtcaatact gatgtacata tccttttagt tccagttcag acatcataag agatctttta      60

gaagaagagg aagcctggga agcatcacat aagtgagttc tcataatcat gaaataagca      120


<210>  714
<211>  120
<212>  DNA
<213>  Artificial Sequence

<220>
<223>  Synthetic polynucleotide

<400>  714
gtcttcagaa acgatcttca agacacagaa ataagtccaa gacagaatcg ccgcacaaga      60

gccgctgaaa gtgctgagat tgaaccaaga aacaagcgta ataggaatta atgtgggctt      120


<210>  715
<211>  120
<212>  DNA
<213>  Artificial Sequence

<220>

<223>  Synthetic polynucleotide

<400>  715
ttttatacag aaatcttgtg aaaccactgc ccaaccagag caatgattgt tcaaagagtg      60

gtattgaatt ctcgacctgg tatgtatttg cgatgaatga acaactctga tctttgataa      120


<210>  716
<211>  120
<212>  DNA
<213>  Artificial Sequence

<220>
<223>  Synthetic polynucleotide

<400>  716
gaaaaaatgg taatccagtg gcagagaatt tccgaatgga agaagtctat ttaccagata      60

atattaatga aggacaagta caagttagaa ctctttatct ttctgtggat ccttacatgg      120


<210>  717
<211>  120
<212>  DNA
<213>  Artificial Sequence

<220>
<223>  Synthetic polynucleotide

<400>  717
tatcttaatg ttttcttttt tcagcgttgt agaatgaatg aagacactgg cactgattat      60

ataacacctt ggcagctatc tcaagtcgtt gatggaggag gtattggaat tatagaagaa      120


<210>  718
<211>  120
<212>  DNA
<213>  Artificial Sequence

<220>
<223>  Synthetic polynucleotide

<400>  718
agcaaacaca caaatttgac taaaggcgat tttgtgactt ctttctattg gccctggcaa      60

accaaggtta ttctggatgg aaatagcctt gaaaaggtga tatatatatg aacatatctg      120


<210>  719
<211>  120
<212>  DNA
<213>  Artificial Sequence

<220>
<223>  Synthetic polynucleotide

<400>  719
cctagtattt ttaaagggta tatattttat tttaggtaga cccacaactt gtggatggac      60

acctttcata ttttcttgga gctataggta tgcctggttt gacttccttg attgggatac      120

<210>  720
<211>  120
<212>  DNA
<213>  Artificial Sequence

<220>
<223>  Synthetic polynucleotide

<400>  720
aggaaaaagg tcatataact gctggatcta ataagacaat ggttgtcagt ggggccgcag      60

gtgcctgtgg atctgtggct gggcaggtaa actttctgag aattatttga ttttctgatt      120


<210>  721
<211>  120
<212>  DNA
<213>  Artificial Sequence

<220>
<223>  Synthetic polynucleotide

<400>  721
aaatttcccc cctagattgg ccatttctta ggttgttcca gagtggtggg aatttgtgga      60

acacatgaga aatgcatcct cttgacctca gaactgggct ttgatgctgc aattaattat      120


<210>  722
<211>  120
<212>  DNA
<213>  Artificial Sequence

<220>
<223>  Synthetic polynucleotide

<400>  722
aaaaaagaca atgtggcaga acagctccgt gaatcatgcc cagctggagt ggatgtttat      60

tttgacaatg ttggtggtaa catcagtgat acagtgataa gtcaggttgt ttgctgattt      120


<210>  723
<211>  120
<212>  DNA
<213>  Artificial Sequence

<220>
<223>  Synthetic polynucleotide

<400>  723
agccataaaa tcagtaaaca taggaaaggg atattttatc attatttttc tctgtgcaga      60

tgaatgagaa cagccacatc atcctgtgtg gtcaaatttc tcagtacaac aaagatgtgc      120


<210>  724
<211>  120
<212>  DNA
<213>  Artificial Sequence

<220>

<223>  Synthetic polynucleotide

<400>  724
cttatcctcc cccgctatcc cctgctatag aggcaatcca gaaagaaaga aacatcacaa      60

ggtgtgttct tcctctttgc ccttattacc aggttcatgg ggttttaaat atcatagatg      120


<210>  725
<211>  120
<212>  DNA
<213>  Artificial Sequence

<220>
<223>  Synthetic polynucleotide

<400>  725
ttcttcctca ctgcagggaa agatttctgg tattaaatta taaagacaaa tttgagcctg      60

gcattctaca gctgagtcag tggtttaaag aaggaaagct aaaggtagaa cttctattct      120


<210>  726
<211>  120
<212>  DNA
<213>  Artificial Sequence

<220>
<223>  Synthetic polynucleotide

<400>  726
aagtttaaag actattaaat ctagctattt tgatttacag attaaagaga cggtaataaa      60

tgggttggaa aacatgggag gtaagatgaa tgtagactta ttatatacac atgctcagca      120


<210>  727
<211>  120
<212>  DNA
<213>  Artificial Sequence

<220>
<223>  Synthetic polynucleotide

<400>  727
aacctacttt ctctttttcc agctgcattc cagtccatga tgacaggagg taacattgga      60

aagcagatag tttgcatttc agaagaaatc tctttgtaat tgctgtaaat gtcatcaagg      120


<210>  728
<211>  120
<212>  DNA
<213>  Artificial Sequence

<220>
<223>  Synthetic polynucleotide

<400>  728
   gagctgggag ctaggtcctc ggagtgggcc agagatggcg gcggccgacg gggctttgcc      60
   ggaggcggcg gctttagagc aacccgcgga gctgcctgcc tcggtgcggg cgagtatcga      120

<210>  729
<211>  120
<212>  DNA
<213>  Artificial Sequence

<220>
<223>  Synthetic polynucleotide

<400>  729
gcggaagcgg cagcgggcac tgatgctgcg ccaggcccgg ctggctgccc ggccctactc      60

ggcgacggcg gctgcggcta ctggaggttt gggccgcgtc cgcgctttcc ccttccctct      120


<210>  730
<211>  120
<212>  DNA
<213>  Artificial Sequence

<220>
<223>  Synthetic polynucleotide

<400>  730
gtaggcatgg ctaatgtaaa agcagcccca aagataattg acacaggagg aggcttcatt      60

ttagaagagg aagaagaaga agaacagaaa attggaaaag ttgttcatca accaggtaaa      120


<210>  731
<211>  120
<212>  DNA
<213>  Artificial Sequence

<220>
<223>  Synthetic polynucleotide

<400>  731
ttcttaggac ctgttatgga atttgattat gtaatatgcg aagaatgtgg gaaagaattt      60

atggattctt atcttatgaa ccactttgat ttgccaactt gtgataactg caggtactta      120


<210>  732
<211>  120
<212>  DNA
<213>  Artificial Sequence

<220>
<223>  Synthetic polynucleotide

<400>  732
ataactaatt tatataatgg acttaatctg ttttcagaga tgctgatgat aaacacaagc      60

ttataaccaa aacagaggca aaacaagaat atcttctgaa agactgtgat ttagaaaaaa      120


<210>  733
<211>  120
<212>  DNA
<213>  Artificial Sequence

<220>

<223>  Synthetic polynucleotide

<400>  733
gagagccacc tcttaaattt attgtgaaga agaatccaca tcattcacaa tggggtgata      60

tgaaactcta cttaaagtta caggtctcta ataagttgta tttattattt ttcactctgg      120


<210>  734
<211>  120
<212>  DNA
<213>  Artificial Sequence

<220>
<223>  Synthetic polynucleotide

<400>  734
gattgtgaag aggtctcttg aagtttgggg tagtcaagaa gcattagaag aagcaaagga      60

agtccgacag gaaaaccgag aaaaaatgaa acagaagaaa tttgataaaa aagtaaaagg      120


<210>  735
<211>  120
<212>  DNA
<213>  Artificial Sequence

<220>
<223>  Synthetic polynucleotide

<400>  735
cctgaatagc accactgaaa agatgacttt aaaatttttt tttcagaatt gcggcgagca      60

gtaagaagca gcgtgtggaa aagggagacg attgttcatc aacatgagta tggaccagaa      120


<210>  736
<211>  120
<212>  DNA
<213>  Artificial Sequence

<220>
<223>  Synthetic polynucleotide

<400>  736
gaaaacctag aagatgacat gtaccgtaag acttgtacta tgtgtggcca tgaactgaca      60

tatgaaaaaa tgtgattttt tagttcagtg acctgtttta tagaatttta tatttaaata      120


<210>  737
<211>  120
<212>  DNA
<213>  Artificial Sequence

<220>
<223>  Synthetic polynucleotide

<400>  737
caagcaacat ggctcggaaa cgcgcggccg gcggggagcc gcggggacgc gaactgcgca      60

gccagaaatc caaggccaag agcaaggccc ggcgtgagga ggaggaggag ggtgagagcg      120

<210>  738
<211>  120
<212>  DNA
<213>  Artificial Sequence

<220>
<223>  Synthetic polynucleotide

<400>  738
tatgttctgt gttgtcacct agatgccttt gaagatgaga aacccccaaa gaagagcctt      60

ctctccaaag tttcacaagg aaagaggaaa agaggctgca gtcatcctgg gggttcagca      120


<210>  739
<211>  120
<212>  DNA
<213>  Artificial Sequence

<220>
<223>  Synthetic polynucleotide

<400>  739
gatggtccag caaaaaagaa agtggccaag gtgactgtta aatctgaaaa cctcaaggtt      60

ataaaggatg aagccctcag cgatggggat gacctcaggt gagatgtctg caaagctttg      120


<210>  740
<211>  120
<212>  DNA
<213>  Artificial Sequence

<220>
<223>  Synthetic polynucleotide

<400>  740
acagggactt tccaagtgac ctcaagaagg cacaccatct gaagagaggg gctaccatga      60

atgaagacag caatgaagaa gaggaagaaa gtgaaaatga ttgggaagag gttgaaggtg      120


<210>  741
<211>  120
<212>  DNA
<213>  Artificial Sequence

<220>
<223>  Synthetic polynucleotide

<400>  741
acagtagcta ttattattgt tattactatt actgattttt aaaaatgctt gttgatagaa      60

cttagtgagc ctgtgctggg tgacgtgaga gaaagtacag ccttctctcg atctcttctg      120


<210>  742
<211>  120
<212>  DNA
<213>  Artificial Sequence

<220>

<223>  Synthetic polynucleotide

<400>  742
cctgtgaagc cagtggagat agagattgaa acgccagagc aggcgaagac aagagaaaga      60

aggtaagctt aggcccttgc ttctaggctc ctgtcacatg cagtaggcaa cagctgctgc      120


<210>  743
<211>  120
<212>  DNA
<213>  Artificial Sequence

<220>
<223>  Synthetic polynucleotide

<400>  743
tttcactatt tgttgcagtg aaaagataaa actggagttt gagacatatc ttcggagggc      60

gatgaaacgt ttcaataaag gggtccatga ggacacacac aaggtaaggg caaggaatga      120


<210>  744
<211>  120
<212>  DNA
<213>  Artificial Sequence

<220>
<223>  Synthetic polynucleotide

<400>  744
ccttccttcc atgctgcccc ttcctccttt cctcttcaca ggttcacctt ctctgcctgc      60

tagcaaatgg cttctatcga aataacatct gcagccagcc agatctgcat gctattggcc      120


<210>  745
<211>  120
<212>  DNA
<213>  Artificial Sequence

<220>
<223>  Synthetic polynucleotide

<400>  745
tgtccatcat cccagcccgc tttaccagag tgctgcctcg agatgtggac acctactacc      60

tctcaaacct ggtgaagtgg taaggccctc cgcttgtcct gcagagctgg ggagtgtagg      120


<210>  746
<211>  120
<212>  DNA
<213>  Artificial Sequence

<220>
<223>  Synthetic polynucleotide

<400>  746
gaaatgaaaa ttcccctttg ccctgacctc tgacacaagg aatgcctgct ttctccccag      60

gttcattgga acatttacag ttaatgcaga actttcagcc agtgaacaag ataacctgca      120

<210>  747
<211>  120
<212>  DNA
<213>  Artificial Sequence

<220>
<223>  Synthetic polynucleotide

<400>  747
gactacattg gaaaggagat ttgctattta ctctgctcga gatgatgagg aattggtcca      60

tgtaagtgat cctcccggat cactgttttt tatcagtact gttaactaat gataatggca      120


<210>  748
<211>  120
<212>  DNA
<213>  Artificial Sequence

<220>
<223>  Synthetic polynucleotide

<400>  748
tccttttcat catagatatt cttactgatt ctccgggctc tgcagctctt gacccggctg      60

gtattgtctc tacagccaat tcctctgaag tcagcaacag caaaggtgag gtgcgcaggg      120


<210>  749
<211>  120
<212>  DNA
<213>  Artificial Sequence

<220>
<223>  Synthetic polynucleotide

<400>  749
gggggagaaa aaaaagcaaa atttcttatt ctgtttaagg gaaagaaacc ttccaaggaa      60

agattgactg cggatccagg aggctcctca gaaacttcca gccaagttct agaaaaccac      120


<210>  750
<211>  120
<212>  DNA
<213>  Artificial Sequence

<220>
<223>  Synthetic polynucleotide

<400>  750
accaaaccaa agaccagcaa aggaaccaaa caagaggaaa cctttgctaa gggcacctgc      60

aggccaagtg ccaaagggaa gaggaacaag ggaggcagaa agaaacggag caagccctcc      120


<210>  751
<211>  120
<212>  DNA
<213>  Artificial Sequence

<220>

<223>  Synthetic polynucleotide

<400>  751
tccagcgagg aagatgaggg cccaggagac aagcaggaga aggcaaccca gcgacgtccg      60

catggccggg agcggcgggt ggcctccagg gtgtcttata aagaggagag tgggagtgat     120


<210>  752
<211>  120
<212>  DNA
<213>  Artificial Sequence

<220>
<223>  Synthetic polynucleotide

<400>  752
gaggctggca gcggctctga ttttgagctc tccagtggag aagcctctga tccctctgat      60

gaggattccg aacctggccc tccaaagcag aggaaagccc ccgctcctca gaggacaaag     120


<210>  753
<211>  120
<212>  DNA
<213>  Artificial Sequence

<220>
<223>  Synthetic polynucleotide

<400>  753
gctgggtcca agagtgcctc caggacccat cgtgggagcc atcgtaagga cccaagcttg      60

ccagcggcat cctcaagctc ttcaagcagt aaaagaggca agaaaatgtg cagcgatggt     120


<210>  754
<211>  120
<212>  DNA
<213>  Artificial Sequence

<220>
<223>  Synthetic polynucleotide

<400>  754
gagaaggcag aaaaaagaag catagctggt atagaccagt ggctagaggt gttctgtgag      60

caggaggaaa agtgggtatg tgtagactgt gtgcacggtg tggtgggcca gcctctgacc     120


<210>  755
<211>  120
<212>  DNA
<213>  Artificial Sequence

<220>
<223>  Synthetic polynucleotide

<400>  755
tgttacaagt acgccaccaa gcccatgacc tatgtggtgg gcattgacag tgacggctgg      60

gtccgagatg tcacacagag gtacgaccca gtctggatga cagtgacccg caagtgccgg     120

<210>  756
<211>  120
<212>  DNA
<213>  Artificial Sequence

<220>
<223>  Synthetic polynucleotide

<400>  756
gttgatgctg agtggtgggc cgagaccttg agaccatacc agagcccatt tatggacagg      60

gagaagaaag aagacttgga ggtaaggcct tggctgccag gggctccaag acacagtcag      120


<210>  757
<211>  120
<212>  DNA
<213>  Artificial Sequence

<220>
<223>  Synthetic polynucleotide

<400>  757
ctaaataaca tgctcaacct tgtctgtctg gcctttgcag tttcaggcta aacacatgga      60

ccagcctttg cccactgcca ttggcttata taagaaccac cctctgtatg ccctgaagcg      120


<210>  758
<211>  120
<212>  DNA
<213>  Artificial Sequence

<220>
<223>  Synthetic polynucleotide

<400>  758
gcatctcctg aaatatgagg ccatctatcc cgagacagct gccatccttg ggtattgtcg      60

tggagaagcg gtctactcca ggtgcgtgag gcagcctggt tggcctcagg ggcttcctga      120


<210>  759
<211>  120
<212>  DNA
<213>  Artificial Sequence

<220>
<223>  Synthetic polynucleotide

<400>  759
tcctgtgttg gttccacagg gattgtgtgc acactctgca ttccagggac acgtggctga      60

agaaagcaag agtggtgagg cttggagaag taccctacaa ggtaactgga gctggggggt      120


<210>  760
<211>  120
<212>  DNA
<213>  Artificial Sequence

<220>

<223>  Synthetic polynucleotide

<400>  760
ctgaggaact ggatgccttt gttgtaaacg gtggccgtgt cctctggtgc agatggtgaa      60

aggcttttct aaccgtgctc ggaaagcccg acttgctgag ccccagctgc gggaagaaaa     120


<210>  761
<211>  120
<212>  DNA
<213>  Artificial Sequence

<220>
<223>  Synthetic polynucleotide

<400>  761
tgacctgggc ctgtttggct actggcagac agaggagtat cagcccccag tggccgtgga      60

cgggaaggta agggcagcat cagaagggct caggaccagg ccgccttgtt cccctgctgg     120


<210>  762
<211>  120
<212>  DNA
<213>  Artificial Sequence

<220>
<223>  Synthetic polynucleotide

<400>  762
tgctggccaa atgctgactt gctcacccga cacaggtgcc ccggaacgag tttgggaatg      60

tgtacctctt cctgcccagc atgatgccta ttggctgtgt ccagctgaac ctgcccaatc     120


<210>  763
<211>  120
<212>  DNA
<213>  Artificial Sequence

<220>
<223>  Synthetic polynucleotide

<400>  763
tacaccgcgt ggcccgcaag ctggacatcg actgtgtcca ggccatcact ggctttgatt      60

tccatggcgg ctactcccat cccgtgtgcg tgagggcctt tcgatggagg ctaaacacag     120


<210>  764
<211>  120
<212>  DNA
<213>  Artificial Sequence

<220>
<223>  Synthetic polynucleotide

<400>  764
tgtgtcccca caggactgat ggatacatcg tctgcgagga attcaaagac gtgctcctga      60

ctgcctggga aaatgagcag gcagtcattg aaaggaagga gaaggaggta agcgcatatg     120

<210>  765
<211>  120
<212>  DNA
<213>  Artificial Sequence

<220>
<223>  Synthetic polynucleotide

<400>  765
catttttgcc tgcagaaaaa ggagaagcgg gctctaggga actggaagtt gctggccaaa      60

ggtctgctca tcagggagag gctgaagcgt cgctacgggc ccaaggtcag tgcaggttct      120


<210>  766
<211>  120
<212>  DNA
<213>  Artificial Sequence

<220>
<223>  Synthetic polynucleotide

<400>  766
acctgtccag agtgaggcag cagctcccca cacagatgca ggaggtggac tctcttctga      60

tgaagaggag gggaccagct ctcaagcaga agcggccagg atactggctg cctcctggcc      120


<210>  767
<211>  120
<212>  DNA
<213>  Artificial Sequence

<220>
<223>  Synthetic polynucleotide

<400>  767
tcaaaaccga gaagatgaag aaaagcagaa gctgaagggt gggcccaaga agaccaaaag      60

ggaaaagaaa gcagcagctt cccacctgtt cccatttgag cagctgtgag ctgagcgccc      120


<210>  768
<211>  120
<212>  DNA
<213>  Artificial Sequence

<220>
<223>  Synthetic polynucleotide

<400>  768
cagaaagcgg gcgagcagca gttgagcgag cccgaggaca tggagatgga aggtgaggcc      60

cgagggccgg ccgcggcggg gccggggggc gcctttcatt gtggccgggg gagccgcggg      120


<210>  769
<211>  120
<212>  DNA
<213>  Artificial Sequence

<220>

<223>  Synthetic polynucleotide

<400>  769
tgaatgtttc tggtcctaaa gacaaggctt ctgtgggatg ctttaaaaaa taaaaaagaa      60

tgctgaggta gagctcctag agattctatg cagtcactct gatgtgggtg agagaacctc      120


<210>  770
<211>  120
<212>  DNA
<213>  Artificial Sequence

<220>
<223>  Synthetic polynucleotide

<400>  770
ccccatcccc cagcagaggc cactgcagag ccatgcagag atggctggga accacaggct      60

tgggctggaa ggtgagtcag gttttcaggt tgagctcctt atgctggtga tggatggaga      120


<210>  771
<211>  120
<212>  DNA
<213>  Artificial Sequence

<220>
<223>  Synthetic polynucleotide

<400>  771
ctttaaagcg ggagatacag atgacccacc aagaattact cagaaccctg tgatcaatgg      60

gaatgtggcc ctgagtgatg gacacaacac cgcggaggag gacatggagg atggtaagtg      120


<210>  772
<211>  120
<212>  DNA
<213>  Artificial Sequence

<220>
<223>  Synthetic polynucleotide

<400>  772
atgatgtgtt ttttttgcag acaccagttg gcgctccgag gcaacctttc agttcactgt      60

ggagcgcttc agcagactga gtgagtcggt ccttagccct ccgtgttttg tgcgaaatct      120


<210>  773
<211>  120
<212>  DNA
<213>  Artificial Sequence

<220>
<223>  Synthetic polynucleotide

<400>  773
gccatggaag attatggtga tgccacgctt ttatccagac agaccacacc aaaaaagcgt      60

aggattcttt ctccagtgca atgctgaatc tgattccacg taagacagta tttcattact      120

<210>  774
<211>  120
<212>  DNA
<213>  Artificial Sequence

<220>
<223>  Synthetic polynucleotide

<400>  774
aacatctggt aaataatgtc aactaaggct cagattattc ttttaaacag gtcatggtct      60

tgccatgcac aagcagtgct gaagataata aattacagag atgatgaaaa gtcgttcagt      120


<210>  775
<211>  120
<212>  DNA
<213>  Artificial Sequence

<220>
<223>  Synthetic polynucleotide

<400>  775
cgtcgtatta gtcatttgtt cttccataaa gaaaatgatt ggggattttc caattttatg      60

gcctggagtg taagtaacag tgtatttagt ctgaacaatt ggtgcattac tgcttgttgt      120


<210>  776
<211>  120
<212>  DNA
<213>  Artificial Sequence

<220>
<223>  Synthetic polynucleotide

<400>  776
tatttatgtt ttataggaag tgaccgatcc tgagaaagga tttatagatg atgacaaagt      60

tacctttgaa gtctttgtac aggcggatgc tccccatgga gttgcgtaag tttttcttta      120


<210>  777
<211>  120
<212>  DNA
<213>  Artificial Sequence

<220>
<223>  Synthetic polynucleotide

<400>  777
tttcaggtgg gattcaaaga agcacacagg ctacgtcggc ttaaagaatc agggagcgac      60

ttgttacatg aacagcctgc tacagacgtt atttttcacg aatcagctac gaaaggtaaa      120


<210>  778
<211>  120
<212>  DNA
<213>  Artificial Sequence

<220>

<223>  Synthetic polynucleotide

<400>  778
taaattcccc tgcatcttct cagagtaaaa attcttattt cttaattgtt tcaggctgtg      60

tacatgatgc caaccgaggg ggatgattcg tctaaaagcg tccctttagc attacaaaga      120


<210>  779
<211>  120
<212>  DNA
<213>  Artificial Sequence

<220>
<223>  Synthetic polynucleotide

<400>  779
gtgttctatg aattacagca tagtgataaa cctgtaggaa caaaaaagtt aacaaagtca      60

tttgggtatg tattagacat ctgcctttgc cattctgctt cctctttttt tttttttttt      120


<210>  780
<211>  120
<212>  DNA
<213>  Artificial Sequence

<220>
<223>  Synthetic polynucleotide

<400>  780
ttaaatggag ttgtttgttt tatctttgaa aggtgggaaa ctttagatag cttcatgcaa      60

catgatgttc aggagctttg tcgagtggta agtttcaagc aatacttgta tatttcttac      120


<210>  781
<211>  120
<212>  DNA
<213>  Artificial Sequence

<220>
<223>  Synthetic polynucleotide

<400>  781
gccaaaacat tctttttcag ttgctcgata atgtggaaaa taagatgaaa ggcacctgtg      60

tagagggcac catacccaaa ttattccgcg gcaaaatggt ggtatgtggc taccagttct      120


<210>  782
<211>  120
<212>  DNA
<213>  Artificial Sequence

<220>
<223>  Synthetic polynucleotide

<400>  782
ttccttattt gcagtcctat atccagtgta aagaagtaga ctatcggtct gatagaagag      60

aagattatta tgatatccag ctaagtatca aaggaaagaa aaatagtaag tgttgtgtgt      120

<210>  783
<211>  120
<212>  DNA
<213>  Artificial Sequence

<220>
<223>  Synthetic polynucleotide

<400>  783
ctctctctct ctttaaagta tttgaatcat ttgtggatta tgtggcagta gaacagctcg      60

atggggacaa taaatacgac gctggggaac atggcttaca ggtaaattga gtgttttgtg     120


<210>  784
<211>  120
<212>  DNA
<213>  Artificial Sequence

<220>
<223>  Synthetic polynucleotide

<400>  784
tttaggaagc agagaaaggt gtgaaattcc taacattgcc accagtgtta catctacaac      60

tgatgagatt tatgtatgac cctcagacgg accaaaatat caagatcaat gataggtaat     120


<210>  785
<211>  120
<212>  DNA
<213>  Artificial Sequence

<220>
<223>  Synthetic polynucleotide

<400>  785
tgtaaattaa tactgaattt cttttaaaat gttttctaat aggtttgaat tcccagagca      60

gttaccactt gatgaatttt tgcaaaaaac agatcctaag gaccctgcaa attatattct     120


<210>  786
<211>  120
<212>  DNA
<213>  Artificial Sequence

<220>
<223>  Synthetic polynucleotide

<400>  786
tcatgcagtc ctggttcata gtggagataa tcatggtgga cattatgtgg tttatctaaa      60

ccccaaaggg gatggcaaag taagtggtgg gacccagggc tgaatgcaag ctcatcagaa     120


<210>  787
<211>  120
<212>  DNA
<213>  Artificial Sequence

<220>

<223>  Synthetic polynucleotide

<400>  787
cttgtcacag tgcttgatcg ctgctggtgt gtgttgtttt ccttgcagtg gtgtaaattt      60

gatgacgacg tggtgtcaag gtgtactaaa gaggaagcaa ttgagcacaa ttatgggggt      120


<210>  788
<211>  120
<212>  DNA
<213>  Artificial Sequence

<220>
<223>  Synthetic polynucleotide

<400>  788
cacgatgacg acctgtctgt tcgacactgc actaatgctt acatgttagt ctacatcagg      60

gaatcaaaac tgagtgagta gtgttcactt ttgttctgtt ctttactgtg gtggacttgg      120


<210>  789
<211>  120
<212>  DNA
<213>  Artificial Sequence

<220>
<223>  Synthetic polynucleotide

<400>  789
ctaagagggg aaagtccatc tgcaggggtc aaagaggatg cctttgtcct gcaggtgaag      60

ttttacaggc ggtcaccgac catgatattc ctcagcagtt ggtggagcga ttacaagaag      120


<210>  790
<211>  120
<212>  DNA
<213>  Artificial Sequence

<220>
<223>  Synthetic polynucleotide

<400>  790
agaaaaggat cgaggctcag aagcggaagg agcggcagga agcccatctc tatatgcaag      60

tgcaggtcag cccccgccct tctgggactc cgggtcacca tgcagccggg cacccatctc      120


<210>  791
<211>  120
<212>  DNA
<213>  Artificial Sequence

<220>
<223>  Synthetic polynucleotide

<400>  791
tcattttttg tcagttgtca agtcttttct gtgctttcat acatattttc agatagtcgc      60

agaggaccag ttttgtggcc accaagggaa tgacatgtac gatgaagaaa aagtgaaata      120

<210>  792
<211>  120
<212>  DNA
<213>  Artificial Sequence

<220>
<223>  Synthetic polynucleotide

<400>  792
cactgtgttc aaagtattga agaactcctc gcttgctgag tttgttcaga gcctctctca      60

gaccatggtg cgtaccggtc ccgtggatat accccaccgt ggctcccaac gtccaccttc      120


<210>  793
<211>  120
<212>  DNA
<213>  Artificial Sequence

<220>
<223>  Synthetic polynucleotide

<400>  793
ttttttcagg gatttccaca agatcaaatt cgattgtggc ccatgcaagc aaggagtaat      60

ggaacaaaac gaccagcaat gttagataat gaagccgacg gcaataaaac agtaaatatt      120


<210>  794
<211>  120
<212>  DNA
<213>  Artificial Sequence

<220>
<223>  Synthetic polynucleotide

<400>  794
gaaatagatg attgagctca gtgataatga aaacccttgg acaatattcc tggaaacagt      60

tgatcccgag ctggctgcta gtggagcgac cttacccaag tttgataaag atcgtaagtg      120


<210>  795
<211>  120
<212>  DNA
<213>  Artificial Sequence

<220>
<223>  Synthetic polynucleotide

<400>  795
tattaacctt gtagatgatg taatgttatt tttgaagatg tatgatccca aaacgcggag      60

cttgaattac tgtgggcata tctacacacc aatatcctgt aaaatacgta agtccttcgt      120


<210>  796
<211>  120
<212>  DNA
<213>  Artificial Sequence

<220>

<223>  Synthetic polynucleotide

<400>  796
catttaaaat atcttctttc tcctaggtga cttgctccca gttatgtgtg acagagcagg      60

atttattcaa gatactagcc ttatcctcta tgaggtttgg atggtttatt tttccataat      120


<210>  797
<211>  120
<212>  DNA
<213>  Artificial Sequence

<220>
<223>  Synthetic polynucleotide

<400>  797
ttcactgtag gaagttaaac cgaatttaac agagagaatt caggactatg acgtgtctct      60

tgataaagcc cttgatgaac taatggatgg tgacatcata gtatttcaga agtatgtact      120


<210>  798
<211>  120
<212>  DNA
<213>  Artificial Sequence

<220>
<223>  Synthetic polynucleotide

<400>  798
ccatggactt tgattcagac tgcaaacttt ctccctccac cagggatgac cctgaaaatg      60

ataacagtga attacccacc gcaaaggagt atttccgaga tctctaccac cgcgttgatg      120


<210>  799
<211>  120
<212>  DNA
<213>  Artificial Sequence

<220>
<223>  Synthetic polynucleotide

<400>  799
tcattttctg tgataaaaca atccctaatg atcctggatt tgtggttacg ttatcaaata      60

gaatgaatta ttttcaggta tttaataaat gctcctttcc tcttcagatc ccactccaga      120


<210>  800
<211>  120
<212>  DNA
<213>  Artificial Sequence

<220>
<223>  Synthetic polynucleotide

<400>  800
tgtgaacctt cctttctgtg tcttaggttg caaagacagt tgcacagagg ctcaacacag      60

atccaatgtt gctgcagttt ttcaagtctc aagggtaggt cacatgtgtg gacacttgct      120

<210>  801
<211>  120
<212>  DNA
<213>  Artificial Sequence

<220>
<223>  Synthetic polynucleotide

<400>  801
gtgtagttat agggatggcc caggtaatcc tcttagacat aattatgaag gtactttaag      60

agatcttcta cagttcttca agcctagaca acctaagaaa ctttactatc agcaggtatg      120


<210>  802
<211>  120
<212>  DNA
<213>  Artificial Sequence

<220>
<223>  Synthetic polynucleotide

<400>  802
gaatctttct tttcttaata gcttaagatg aaaatcacag actttgagaa caggcgaagt      60

tttaaatgta tatggttaaa cagccaattt agggaagagg taagtttttt taatactatg      120


<210>  803
<211>  120
<212>  DNA
<213>  Artificial Sequence

<220>
<223>  Synthetic polynucleotide

<400>  803
cattacttag gaaataacac tatatccaga caagcatggg tgtgtccggg acctgttaga      60

agaatgtaaa aaggccgtgg agcttgggga gaaagcatca gggaaactta ggcaagtatt      120


<210>  804
<211>  120
<212>  DNA
<213>  Artificial Sequence

<220>
<223>  Synthetic polynucleotide

<400>  804
ctctgttcag gctgctagaa attgtaagct acaaaatcat tggtgttcat caagaagatg      60

aactattaga atgtttatct cctgcaacga gccggacgtt tcgaatagag gtatctgtcc      120


<210>  805
<211>  120
<212>  DNA
<213>  Artificial Sequence

<220>

<223>  Synthetic polynucleotide

<400>  805
tggacacttt gaccacatca cttgcactta cagaactgtg tctgttgtcc gtgtccccag      60

gaaatccctt tggaccaggt ggacatagac aaagagaatg agatgcttgt cacagtggcg      120


<210>  806
<211>  120
<212>  DNA
<213>  Artificial Sequence

<220>
<223>  Synthetic polynucleotide

<400>  806
catttccaca aagaggtctt cggaacgttc ggaatcccgt ttttgctgag gatacaccag      60

gtatgctgtt gtggttggcc gagtggctca cgtcaaaacc tggagccttt gagtggccgc      120


<210>  807
<211>  120
<212>  DNA
<213>  Artificial Sequence

<220>
<223>  Synthetic polynucleotide

<400>  807
agcacatgtc ctttgccatt ctagggcgag cattttcgag aagtgatgaa gcgaatccag      60

agcctgctgg acatccagga gaaggagttt gagaaggtgt gcagctgggg cctcctgggg      120


<210>  808
<211>  120
<212>  DNA
<213>  Artificial Sequence

<220>
<223>  Synthetic polynucleotide

<400>  808
gtttaaattt gcaattgtaa tgatgggccg acaccagtac ataaatgaag acgagtatga      60

agtaaatttg aaagactttg agccacagcc cggtaagggt tctccccccc ccgggggctg      120


<210>  809
<211>  120
<212>  DNA
<213>  Artificial Sequence

<220>
<223>  Synthetic polynucleotide

<400>  809
caccaggtaa tatgtctcat cctcggcctt ggctagggct cgaccacttc aacaaagccc      60

caaagaggag tcgctacact taccttgaaa aggccattaa aatccataac tgatttccaa      120

<210>  810
<211>  120
<212>  DNA
<213>  Artificial Sequence

<220>
<223>  Synthetic polynucleotide

<400>  810
tatttctaga tatggatcag aaactttcga agttggtaga agagctcaca acttcaggag      60

aaccccgact aaatcctgag aaaatgaagg aactgaagaa aatttgcaag tatgtcttag     120


<210>  811
<211>  120
<212>  DNA
<213>  Artificial Sequence

<220>
<223>  Synthetic polynucleotide

<400>  811
gctcgcatcc ccaggtcttc agaggagcag ctgagccgcg cctaccgcct gctgatagca      60

cagctgaccc aggagcacgc cgagatccgt ctctcagcct tccagattgt ggaggaactc     120


<210>  812
<211>  120
<212>  DNA
<213>  Artificial Sequence

<220>
<223>  Synthetic polynucleotide

<400>  812
ttcgtcaggt ctcaccagtt ccggatgctg gttgtttcca acttccagga gttcctggag      60

ctcacgctgg gcacagaccc cgcacagcct ctgccgcccc ccagggaggc ggcacagagg     120


<210>  813
<211>  120
<212>  DNA
<213>  Artificial Sequence

<220>
<223>  Synthetic polynucleotide

<400>  813
ctgaggcagg cgaccacccg ggccgtggaa gggtggaatg agaagtttgg ggaggcctac      60

aagaagcttg ccttgggcta ccacttctta agacacaaca aaaaggtagg tgggcctggc     120


<210>  814
<211>  120
<212>  DNA
<213>  Artificial Sequence

<220>

<223>  Synthetic polynucleotide

<400>  814
gtgagccacc gcgcctgtcc cctagtcttt attttcaatt gctcaggtgg attttcaaga      60

cacgaatgct cggagtctgg cagaaaggaa gagagaagag gagaagcaga agcacttgga      120


<210>  815
<211>  120
<212>  DNA
<213>  Artificial Sequence

<220>
<223>  Synthetic polynucleotide

<400>  815
taaaatttat caagaaagag ccagccaggc ggagagggag atgcaaggca agtgtccagg      60

acggagggag gggcccacgc ctctgagggt tgcccgtggt ccagcagttc atcctcctgg      120


<210>  816
<211>  120
<212>  DNA
<213>  Artificial Sequence

<220>
<223>  Synthetic polynucleotide

<400>  816
cttttgctcc acatagcgca gcaaacaggt gtcttgatct tccggcagaa atgtctggag      60

aaattgaatc ctgcttgacg gaggtagaga gctgctttag gctgctggtg ccttttgact      120


<210>  817
<211>  120
<212>  DNA
<213>  Artificial Sequence

<220>
<223>  Synthetic polynucleotide

<400>  817
ttgacccgaa cccggagacg gaatcccttg gcatggcttc tggcatgtcc gatgcccttc      60

gctcctcctg cgcgggccag gtgggcccct gccggtctgg cacccctgac ccccgggacg      120


<210>  818
<211>  120
<212>  DNA
<213>  Artificial Sequence

<220>
<223>  Synthetic polynucleotide

<400>  818
gggagcagcc ctgctgcagt agagacctgc ctgcctctgc aggccacccc agagcgggcg      60

gcggggcaca gccatcccag acagccacag gtgacccctc agatgaggac gaggacagcg      120

<210> 819
<211> 120
<212> DNA
<213> Artificial Sequence

<220>
<223> Synthetic polynucleotide

<400> 819
acctcgagga gtttgtgcgg agccacgggc tgggctcgca caagtacacg ctggatgtgg 60

agctctgctc aggtaactgc cttcgcgggg tctctgtggc gccaccctgc cccggctccc 120


<210> 820
<211> 120
<212> DNA
<213> Artificial Sequence

<220>
<223> Synthetic polynucleotide

<400> 820
ccagagggcc tgaaggtgca ggagaacgag gacaaccttg ctctcatcca cgccgcccgc 60

gacacactca agctcatccg gaacaagttc ctgccggctg tgtgctcgtg gatccaggtg 120


<210> 821
<211> 120
<212> DNA
<213> Artificial Sequence

<220>
<223> Synthetic polynucleotide

<400> 821
gtgtgccagg gaccccccgg gccagtggac ctggcatgtg cctcccagca ggacagcttc 60

ccaggtcctg cccggccggc ccctgagctg ttcgcacccc cgtttcgcag cgcttcaccc 120


<210> 822
<211> 120
<212> DNA
<213> Artificial Sequence

<220>
<223> Synthetic polynucleotide

<400> 822
gcgtcgggac ccacggtgga tgtttaaagc gtgccattga cctgaaggct gaattggagc 60

tcgtactgag aaaatacaag gagctggaca tcgagcctga gggaggggaa aggcgcaggg 120


<210> 823
<211> 120
<212> DNA
<213> Artificial Sequence

<220>

<223>  Synthetic polynucleotide

<400>  823
acagacagaa gccctggggg atgcggagga agatgaggac gatgaggact ttgtggaggt      60

ccctgagaag gaggggtatg agccacacat ccccgaccac ttgcggcctg agtatggtga      120


<210>  824
<211>  120
<212>  DNA
<213>  Artificial Sequence

<220>
<223>  Synthetic polynucleotide

<400>  824
ggcgggttgt gggagtggtg gcagggagtg gacacgtgtc tgtttcaggg ctggaggcag      60

caccagagaa agacacagtt gtgcggtgct tgcggacgag gacgaggatg gacgaggagg      120


<210>  825
<211>  120
<212>  DNA
<213>  Artificial Sequence

<220>
<223>  Synthetic polynucleotide

<400>  825
tgtcggaccc cacctctgcg gctgctcagc tgcggcagct ccgggaccac ttgcctccac      60

cctcatctgc caggtgactc ccagtgtcct gtgtgctgag ccccctgccc ggcgctgcca      120


<210>  826
<211>  120
<212>  DNA
<213>  Artificial Sequence

<220>
<223>  Synthetic polynucleotide

<400>  826
gctgtgggtg gcaccagcag caccgtcagg ctgtcccact ctgctcctgt agcccctcca      60

gagcgttgcc agagccacag gaggcccaga agctggcagc agagcgggcc cgggcgcctg      120


<210>  827
<211>  120
<212>  DNA
<213>  Artificial Sequence

<220>
<223>  Synthetic polynucleotide

<400>  827
tggtgcccta cggcgtggac ctgcactact ggggccagga gctccccaca gccgggaaga      60

ttgtcaagtg agtccccatg tgtctgaagt cggccagggc acacaaccag ggtcccagcc      120

<210> 828
<211> 120
<212> DNA
<213> Artificial Sequence

<220>
<223> Synthetic polynucleotide

<400> 828
ctatgagggc ctctggctgt gtctgcaggt ctgactccca gcaccgcttc tggaagccca 60

gcgaggtgga ggaggaagtg gtcaatgccg acatctccga gatgctccgg agccgccaca 120


<210> 829
<211> 120
<212> DNA
<213> Artificial Sequence

<220>
<223> Synthetic polynucleotide

<400> 829
tcacttttgc cgggaagttt gagcctgtgc agcactggtg ccgtgccccg aggccagacg 60

gccggctctg tgagcgccaa gaccggctga aggtgaggcc gtggcccgag ggcgggggtg 120


<210> 830
<211> 120
<212> DNA
<213> Artificial Sequence

<220>
<223> Synthetic polynucleotide

<400> 830
gactttccac agctgcccaa gaccttccgg ctccagagga gggacggagc catccccacg 60

tccctgtggc tctcaggacg ccctcctctg aggggcgcgt gattccaggt tgtgtacact 120


<210> 831
<211> 120
<212> DNA
<213> Artificial Sequence

<220>
<223> Synthetic polynucleotide

<400> 831
ttggctctgt gataccaccc accctggtcg ctgtttgtgc ccaagtcgtg gtggtggggg 60

gaggtggtca aggcaagcgg acccctcccc gccatcagcc accgtgtcct cgctgtgcag 120


<210> 832
<211> 120
<212> DNA
<213> Artificial Sequence

<220>

<223>  Synthetic polynucleotide

<400>  832
tgccctttcc atgggaagat tgttccacgg gacgacgaag gacggccgct cgacccggaa      60

gacagggctc gtgagcagcg gcggcagctg cagaagcagg agcgcccggg taggtctggg      120


<210>  833
<211>  120
<212>  DNA
<213>  Artificial Sequence

<220>
<223>  Synthetic polynucleotide

<400>  833
cgtctcctga agtgcttgag ttttgtttgc agaatggcag gaccctgagt tgatgagaga      60

cgtggaagca gccacagggc aggatctcgg ctcatccagg tacagcggga aaggcagggg      120


<210>  834
<211>  120
<212>  DNA
<213>  Artificial Sequence

<220>
<223>  Synthetic polynucleotide

<400>  834
gaagaagagg aggtacccca gcctcaccaa cctgaaggct caggctgata ccgcccgcgc      60

tcgcattggg agaaaagtct tcgccaagta agagtggctg ctgggtcacc tcccaccgcg      120


<210>  835
<211>  120
<212>  DNA
<213>  Artificial Sequence

<220>
<223>  Synthetic polynucleotide

<400>  835
tgtttcccca cagggcagct gtgcggaggg tagtggcagc catgaaccgg atggaccaga      60

agaagcacga gaagttttca aaccagttta actacgcact gaactagaga gcggggccca      120


<210>  836
<211>  120
<212>  DNA
<213>  Artificial Sequence

<220>
<223>  Synthetic polynucleotide

<400>  836
tttttcttta tctttttttt ttttagacta caacaaaatc acaattaaat ccggcctttg      60

aagatgcctg gagcagcagt gatgaagaag gatgaatatc atctttagta cctttttgtc      120

<210>  837
<211>  120
<212>  DNA
<213>  Artificial Sequence

<220>
<223>  Synthetic polynucleotide

<400>  837
ctttgccttc tatgttatag gaactttata gtggtagcag agactgcaac attctggctt      60

gggttccatc cttatatgaa ccagttcctg atgatgatga ggtaaatatt attttcacaa      120


<210>  838
<211>  120
<212>  DNA
<213>  Artificial Sequence

<220>
<223>  Synthetic polynucleotide

<400>  838
accattgctg tttatacagt ttactcagga gaacagataa ctatgcttaa gggacattat      60

aaaactgttg actgctgtgt atttcagtca aatttccagg taagagtgat acaggaattt      120


<210>  839
<211>  120
<212>  DNA
<213>  Artificial Sequence

<220>
<223>  Synthetic polynucleotide

<400>  839
cttattattt taatcctaca ggtgaactat ggaaaagttt gtaataacag taaaaaagga      60

ttgaaattca ctgtctcctg tggctgcagt tcagaatttg tttttgtacc atatggtagc      120


<210>  840
<211>  120
<212>  DNA
<213>  Artificial Sequence

<220>
<223>  Synthetic polynucleotide

<400>  840
ctcactgttg gtacagataa tcgaatgagg ctctggaata gttccaatgg agaaaacaca      60

cttgtaagag atttttaagt atatatttgg gtataaatta agaaatggat ctgtgacata      120


<210>  841
<211>  120
<212>  DNA
<213>  Artificial Sequence

<220>

<223>  Synthetic polynucleotide

<400>  841
acattaaata tgtcctgaat acaagaacag ataaactgaa ctatgttttt cattgcagca      60

aacactgctc ataatgggaa agttaatggc ttatgtttta caagtgatgg acttcacctc      120


<210>  842
<211>  120
<212>  DNA
<213>  Artificial Sequence

<220>
<223>  Synthetic polynucleotide

<400>  842
ttcttctcag tgctgacagt agagtaaaat tatgggatgt gagaagagca tcaggatgtt      60

tgattactct tgatcaacat aatgggaaaa agtcacaagc tgttgaatca ggtaaggaag      120


<210>  843
<211>  120
<212>  DNA
<213>  Artificial Sequence

<220>
<223>  Synthetic polynucleotide

<400>  843
gacttttcat atagttgtat ttgcaggtca cagacaagaa atattagcag tttcctggtc      60

tccacgttat gactatatct tggcaacagc aaggtaaaat ttaacttatt tgcttttgaa      120


<210>  844
<211>  120
<212>  DNA
<213>  Artificial Sequence

<220>
<223>  Synthetic polynucleotide

<400>  844
aatgtctttt gattaccaga ctgcagatgt atttaatttt gaggaaacag tttatagtca      60

tcatatgtct ccagtctcca ccaagcactg tttggtagca ggtttgtaag tgtattcttt      120


<210>  845
<211>  120
<212>  DNA
<213>  Artificial Sequence

<220>
<223>  Synthetic polynucleotide

<400>  845
tttattttta agagttttgg gactggaatt aaataaagac agagatgttg aaagaatcca      60

cggcggtgga attaacaccc ttgacattga acctgttgaa gggagatagt aagtttatta      120

<210>  846
<211>  120
<212>  DNA
<213>  Artificial Sequence

<220>
<223>  Synthetic polynucleotide

<400>  846
ccagccggtg tgaggacacg atatgctggg gtttttgtcc gcacgccaaa cgggtttgga      60

ggaccctctt cgccttcgga gagcagagtc aacacggagg taaagaaaac cttacttttt      120


<210>  847
<211>  120
<212>  DNA
<213>  Artificial Sequence

<220>
<223>  Synthetic polynucleotide

<400>  847
gcatgttcac atcaagctca tttgataaaa ctctgaaagt atgggataca aatacattac      60

aagtaagtac attaaaacat ttgcagattg ctttgtagga taaaggagaa tttaatccta      120


<210>  848
<211>  120
<212>  DNA
<213>  Artificial Sequence

<220>
<223>  Synthetic polynucleotide

<400>  848
taccagtttt gaaactcaca taagtcagtt aatgtaaatt ttgattgctg ttttgtagag      60

atcatcctga tgttcacaga tacagtgtgg agactgtaca gtggtatcct catgacactg      120


<210>  849
<211>  120
<212>  DNA
<213>  Artificial Sequence

<220>
<223>  Synthetic polynucleotide

<400>  849
tattatcagc atgttatcag gtggttcaga tggtgtgatt gtactttatg accttgagaa      60

ctccagcaga caatcttatt acacatgtaa agcagtgtgt tccattggca ggtatgtatt      120


<210>  850
<211>  120
<212>  DNA
<213>  Artificial Sequence

<220>

<223>  Synthetic polynucleotide

<400>  850
ggtattttta ttttatttca aacggcaact actttgagta aacgattcaa taaaaagaaa      60

cgttactaac agtgtattct ttgtaagtga catgactaat gtactttgtg ctggttgttg      120


<210>  851
<211>  120
<212>  DNA
<213>  Artificial Sequence

<220>
<223>  Synthetic polynucleotide

<400>  851
ttttgatgat gacttatgat aaaattatct taaaccggtt ctgtttttac agttggtact      60

agaggaccca aagtacaact ttgtgacttg aagtctggat cctgttctca cattctacag      120


<210>  852
<211>  21
<212>  DNA
<213>  Artificial Sequence

<220>
<223>  Synthetic polynucleotide

<400>  852
   aatttgtgat acccctcgac g                                               21


<210>  853
<211>  24
<212>  DNA
<213>  Artificial Sequence

<220>
<223>  Synthetic polynucleotide

<400>  853
tgggaaggct ctgtgtagat cgga                                             24


<210>  854
<211>  20
<212>  DNA
<213>  Artificial Sequence

<220>
<223>  Synthetic polynucleotide

<400>  854
tgtgagatgg catattcggc                                                  20


<210>  855
<211>  20
<212>  DNA
<213>  Artificial Sequence

<220>
<223>  Synthetic polynucleotide

<400>  855
tccttgctaa ctttgccacc                                                    20


<210>  856
<211>  21
<212>  DNA
<213>  Artificial Sequence

<220>
<223>  Synthetic polynucleotide

<400>  856
tgcttccaca gtgctgagcc t                                                  21


<210>  857
<211>  22
<212>  DNA
<213>  Artificial Sequence

<220>
<223>  Synthetic polynucleotide

<400>  857
catctttgct actggttttc cc                                                 22


<210>  858
<211>  22
<212>  DNA
<213>  Artificial Sequence

<220>
<223>  Synthetic polynucleotide

<400>  858
gtccgcgaag atgacaaaat tg                                                 22


<210>  859
<211>  24
<212>  DNA
<213>  Artificial Sequence

<220>
<223>  Synthetic polynucleotide

<400>  859
agcacactgg actttggcga tgta                                               24


<210>  860
<211>  21
<212>  DNA
<213>  Artificial Sequence

<220>
<223>  Synthetic polynucleotide

<400>  860
aattcaggag acatagggca c                                                21

<210>  861
<211>  22
<212>  DNA
<213>  Artificial Sequence

<220>
<223>  Synthetic polynucleotide

<400>  861
tctttatgac gcagagctaa cc                                               22

<210>  862
<211>  25
<212>  DNA
<213>  Artificial Sequence

<220>
<223>  Synthetic polynucleotide

<400>  862
ttcggcagct tgaaatttac agggc                                            25

<210>  863
<211>  20
<212>  DNA
<213>  Artificial Sequence

<220>
<223>  Synthetic polynucleotide

<400>  863
tccgcaaagc agtgagatag                                                  20

<210>  864
<211>  21
<212>  DNA
<213>  Artificial Sequence

<220>
<223>  Synthetic polynucleotide

<400>  864
gactccagga aaacgacaga g                                                21

<210>  865
<211>  28
<212>  DNA
<213>  Artificial Sequence

<220>
<223>  Synthetic polynucleotide

<400>  865
agcaaaagag atgaagcact acccagtc                                         28

<210>  866
<211>  21
<212>  DNA
<213>  Artificial Sequence

<220>
<223>  Synthetic polynucleotide

<400>  866
ggaggcaaaa catagaggtc g                                                21


<210>  867
<211>  21
<212>  DNA
<213>  Artificial Sequence

<220>
<223>  Synthetic polynucleotide

<400>  867
ggaaagtggg aagataccga g                                                21


<210>  868
<211>  27
<212>  DNA
<213>  Artificial Sequence

<220>
<223>  Synthetic polynucleotide

<400>  868
tgcaggacaa agagaaacgt ctgaagc                                          27


<210>  869
<211>  22
<212>  DNA
<213>  Artificial Sequence

<220>
<223>  Synthetic polynucleotide

<400>  869
ccttcgtcaa attcagcatc ac                                               22


<210>  870
<211>  20
<212>  DNA
<213>  Artificial Sequence

<220>
<223>  Synthetic polynucleotide

<400>  870
agtagaaata cggctgcacc                                                  20


<210>  871
<211>  20

<212>  DNA
<213>  Artificial Sequence

<220>
<223>  Synthetic polynucleotide

<400>  871
tccggaaggc cgaggccttg                                                   20


<210>  872
<211>  22
<212>  DNA
<213>  Artificial Sequence

<220>
<223>  Synthetic polynucleotide

<400>  872
ccaggtctcc attcttagtt gc                                                22


<210>  873
<211>  22
<212>  DNA
<213>  Artificial Sequence

<220>
<223>  Synthetic polynucleotide

<400>  873
agccattgac agagcatata gg                                                22


<210>  874
<211>  26
<212>  DNA
<213>  Artificial Sequence

<220>
<223>  Synthetic polynucleotide

<400>  874
cctccacagt tcccaaggat atcagc                                            26


<210>  875
<211>  21
<212>  DNA
<213>  Artificial Sequence

<220>
<223>  Synthetic polynucleotide

<400>  875
ggcattttca cttccaacca c                                                 21


<210>  876
<211>  20
<212>  DNA
<213>  Artificial Sequence

<220>
<223>  Synthetic polynucleotide

<400>  876
gtgaggacac gatatgctgg                                                   20


<210>  877
<211>  26
<212>  DNA
<213>  Artificial Sequence

<220>
<223>  Synthetic polynucleotide

<400>  877
aacacggaga gttttgggac tggaat                                            26


<210>  878
<211>  21
<212>  DNA
<213>  Artificial Sequence

<220>
<223>  Synthetic polynucleotide

<400>  878
gccgtggatt ctttcaacat c                                                 21


<210>  879
<211>  22
<212>  DNA
<213>  Artificial Sequence

<220>
<223>  Synthetic polynucleotide

<400>  879
tccacatcca atcataagca gg                                                22


<210>  880
<211>  23
<212>  DNA
<213>  Artificial Sequence

<220>
<223>  Synthetic polynucleotide

<400>  880
cggacatcag ccagaaatgc agc                                               23


<210>  881
<211>  22
<212>  DNA
<213>  Artificial Sequence

<220>
<223>  Synthetic polynucleotide

<400>  881
cttagaccgt tacagccatc ag                                                22


<210>  882
<211>  21
<212>  DNA
<213>  Artificial Sequence

<220>
<223>  Synthetic polynucleotide

<400>  882
caaaaccctc tttcagcatg g                                                 21


<210>  883
<211>  25
<212>  DNA
<213>  Artificial Sequence

<220>
<223>  Synthetic polynucleotide

<400>  883
tggctcagtg ttgctatcca gatgc                                             25


<210>  884
<211>  21
<212>  DNA
<213>  Artificial Sequence

<220>
<223>  Synthetic polynucleotide

<400>  884
agcagacacc aaagtaagac c                                                 21


<210>  885
<211>  20
<212>  DNA
<213>  Artificial Sequence

<220>
<223>  Synthetic polynucleotide

<400>  885
agccatggtt tcagacgaag                                                   20


<210>  886
<211>  26
<212>  DNA
<213>  Artificial Sequence

<220>
<223>  Synthetic polynucleotide

<400>  886
ttcctttaat gcttgctttc cccacc                                            26

<210>  887
<211>  18
<212>  DNA
<213>  Artificial Sequence

<220>
<223>  Synthetic polynucleotide

<400>  887
tttcccagca ccatcacg                                                     18


<210>  888
<211>  22
<212>  DNA
<213>  Artificial Sequence

<220>
<223>  Synthetic polynucleotide

<400>  888
ccatctttcc ctcaatctcc ag                                                22


<210>  889
<211>  25
<212>  DNA
<213>  Artificial Sequence

<220>
<223>  Synthetic polynucleotide

<400>  889
cgtcgcttaa tcggagccaa agtct                                             25


<210>  890
<211>  21
<212>  DNA
<213>  Artificial Sequence

<220>
<223>  Synthetic polynucleotide

<400>  890
ctgcattgta ggtgtactcg g                                                 21


<210>  891
<211>  21
<212>  DNA
<213>  Artificial Sequence

<220>
<223>  Synthetic polynucleotide

<400>  891
tggtcaacgt cttgaaagga g                                                 21


<210>  892
<211>  26

<212>  DNA
<213>  Artificial Sequence

<220>
<223>  Synthetic polynucleotide

<400>  892
tgaatgatga acttcttccc cagggc                                          26


<210>  893
<211>  25
<212>  DNA
<213>  Artificial Sequence

<220>
<223>  Synthetic polynucleotide

<400>  893
aaccaattct gctattacaa agacg                                           25


<210>  894
<211>  20
<212>  DNA
<213>  Artificial Sequence

<220>
<223>  Synthetic polynucleotide

<400>  894
ttcgggtagt ggaaaaccag                                                 20


<210>  895
<211>  25
<212>  DNA
<213>  Artificial Sequence

<220>
<223>  Synthetic polynucleotide

<400>  895
cccctcaacg ttagcttcac caaca                                           25


<210>  896
<211>  20
<212>  DNA
<213>  Artificial Sequence

<220>
<223>  Synthetic polynucleotide

<400>  896
agtagaaata cggctgcacc                                                 20


<210>  897
<211>  20
<212>  DNA
<213>  Artificial Sequence

<220>
<223>  Synthetic polynucleotide

<400>  897
atataacaga accggcccac                                                   20


<210>  898
<211>  23
<212>  DNA
<213>  Artificial Sequence

<220>
<223>  Synthetic polynucleotide

<400>  898
catctccttg ccagcggtag acg                                               23


<210>  899
<211>  21
<212>  DNA
<213>  Artificial Sequence

<220>
<223>  Synthetic polynucleotide

<400>  899
ccctctaaga cccatttcag c                                                 21


<210>  900
<211>  21
<212>  DNA
<213>  Artificial Sequence

<220>
<223>  Synthetic polynucleotide

<400>  900
ccctctaaga cccatttcag c                                                 21


<210>  901
<211>  24
<212>  DNA
<213>  Artificial Sequence

<220>
<223>  Synthetic polynucleotide

<400>  901
tcttgtgaaa ccactgccca acca                                              24


<210>  902
<211>  21
<212>  DNA
<213>  Artificial Sequence

<220>
<223>  Synthetic polynucleotide

<400>  902
attctctgcc actggattac c                                                 21


<210>  903
<211>  20
<212>  DNA
<213>  Artificial Sequence

<220>
<223>  Synthetic polynucleotide

<400>  903
gcaaagacca aagagacacg                                                   20


<210>  904
<211>  23
<212>  DNA
<213>  Artificial Sequence

<220>
<223>  Synthetic polynucleotide

<400>  904
tcaaagtgga tgtcgagggt ggc                                               23


<210>  905
<211>  20
<212>  DNA
<213>  Artificial Sequence

<220>
<223>  Synthetic polynucleotide

<400>  905
ggaatgttaa ccccgcaaac                                                   20


<210>  906
<211>  22
<212>  DNA
<213>  Artificial Sequence

<220>
<223>  Synthetic polynucleotide

<400>  906
ccaggagaag gagtttgaga ag                                                22


<210>  907
<211>  25
<212>  DNA
<213>  Artificial Sequence

<220>
<223>  Synthetic polynucleotide

<400>  907
tttgaaagac tttgagccac agccc                                             25

<210>  908
<211>  22
<212>  DNA
<213>  Artificial Sequence

<220>
<223>  Synthetic polynucleotide

<400>  908
ggccgaggat gagacatatt ac                                                22


<210>  909
<211>  22
<212>  DNA
<213>  Artificial Sequence

<220>
<223>  Synthetic polynucleotide

<400>  909
gatgaggacg atgaggactt tg                                                22


<210>  910
<211>  23
<212>  DNA
<213>  Artificial Sequence

<220>
<223>  Synthetic polynucleotide

<400>  910
cgaccacttg cggcctgagt atg                                               23


<210>  911
<211>  20
<212>  DNA
<213>  Artificial Sequence

<220>
<223>  Synthetic polynucleotide

<400>  911
tgtgtctttc tctggtgctg                                                   20


<210>  912
<211>  18
<212>  DNA
<213>  Artificial Sequence

<220>
<223>  Synthetic polynucleotide

<400>  912
gagtatcgag cggaagcg                                                     18


<210>  913
<211>  23

<212>  DNA
<213>  Artificial Sequence

<220>
<223>  Synthetic polynucleotide

<400>  913
attagccatg cctccagtag ccg                                              23


<210>  914
<211>  23
<212>  DNA
<213>  Artificial Sequence

<220>
<223>  Synthetic polynucleotide

<400>  914
ctcctgtgtc aattatcttt ggg                                              23


<210>  915
<211>  22
<212>  DNA
<213>  Artificial Sequence

<220>
<223>  Synthetic polynucleotide

<400>  915
gtctctacag ccaattcctc tg                                               22


<210>  916
<211>  26
<212>  DNA
<213>  Artificial Sequence

<220>
<223>  Synthetic polynucleotide

<400>  916
tggatccgca gtcaatcttt ccttgg                                           26


<210>  917
<211>  21
<212>  DNA
<213>  Artificial Sequence

<220>
<223>  Synthetic polynucleotide

<400>  917
cctttgctgg tctttggttt g                                                21


<210>  918
<211>  20
<212>  DNA
<213>  Artificial Sequence

<220>
<223>  Synthetic polynucleotide

<400>  918
accttctaca atgagctgcg                                                    20


<210>  919
<211>  24
<212>  DNA
<213>  Artificial Sequence

<220>
<223>  Synthetic polynucleotide

<400>  919
atctgggtca tcttctcgcg gttg                                               24


<210>  920
<211>  21
<212>  DNA
<213>  Artificial Sequence

<220>
<223>  Synthetic polynucleotide

<400>  920
cctggatagc aacgtacatg g                                                  21


<210>  921
<211>  20
<212>  DNA
<213>  Artificial Sequence

<220>
<223>  Synthetic polynucleotide

<400>  921
acttgcccag atgttctcag                                                    20


<210>  922
<211>  24
<212>  DNA
<213>  Artificial Sequence

<220>
<223>  Synthetic polynucleotide

<400>  922
accaaccgat ccaaagtctc cagc                                               24


<210>  923
<211>  22
<212>  DNA
<213>  Artificial Sequence

<220>
<223>  Synthetic polynucleotide

<400>  923
gtatccctct agccattcag tg                                                22

<210>  924
<211>  19
<212>  DNA
<213>  Artificial Sequence

<220>
<223>  Synthetic polynucleotide

<400>  924
acatcgctca gacaccatg                                                    19

<210>  925
<211>  25
<212>  DNA
<213>  Artificial Sequence

<220>
<223>  Synthetic polynucleotide

<400>  925
aaggtcggag tcaacggatt tggtc                                             25

<210>  926
<211>  22
<212>  DNA
<213>  Artificial Sequence

<220>
<223>  Synthetic polynucleotide

<400>  926
tgtagttgag gtcaatgaag gg                                                22

## Description

### REFERENCE TO A "SEQUENCE LISTING," A TABLE. OR A COMPUTER PROGRAM LISTING APPENDIX SUBMITTED AS AN ASCII TEXT FILE

The Sequence Listing written in file MKEL-01049US1_ST25.TXT, created November 19, 2020, 446,918 bytes, machine format IBM-PC, MS-Windows operating system, is hereby incorporated by reference.

### TECHNICAL FIELD

The present invention relates to compositions and methods for treating target molecules including cell populations with either a medicant or an affinity medicant conjugate including an antibody drug conjugate or a small molecule medicant conjugate.

### BACKGROUND ART

The present invention is directed to a medicant or an Affinity Medicant Conjugate (AMC) including illudofulvene based medicants, illudofulvene based Affinity Medicant Linker Conjugates (AMLC), illudofulvene based antibody-drug conjugates (ADC) and illudofulvene based medicant-linker (ML) compounds, as well as to compositions of the same, and to methods for their use in treating cancer, an autoimmune to methods of using illudofulvene based Ligand Linker Medicant (LLM) conjugates and illudofulvene based ML compounds in vitro, in situ, and in vivo for the detection, diagnosis or treatment of cells and associated pathological conditions.

### SUMMARY OF INVENTION

There exists a continuing need for chemotherapeutic agents for treating cancer. In particular there is a need for chemotherapeutic agents that have activity against cancers with resistant phenotypes and which can inhibit tumor growth and which have an adequate therapeutic index to be effective for in vivo treatment. In an embodiment of the invention, the chemotherapeutic agents can be delivered to tumors as a stand alone treatment. In an alternative embodiment of the invention, the chemotherapeutic agents can be delivered to a specific patient population having a specific tumors as a stand alone treatment. In another embodiment of the invention, the chemotherapeutic agents can be conjugated with an antibody to form an effective treatment. In another embodiment of the invention, the chemotherapeutic agents can be conjugated with an antibody, an antibody fragment, a receptor protein, a peptidic growth factor, an anti-angiogenic protein, a specific binding peptide, protease cleavable peptide, a glycopeptide, a peptide, a peptidic toxin, a protein toxin and an oligonucleotide and delivered to a specific patient population having a specific tumors to form an effective treatment. In various embodiments of the invention, the therapeutic treatment can be delivered in humans as well as in animals. For example, such therapeutic applications can include: cancer, adenocarcinoma, carcinoma, breast cancer, prostate cancer, ovarian cancer, endometrial cancer, neuroendocrine tumors, infertility, polycystic ovary syndrome, endometriosis, and precocious puberty. For example, veterinary and agricultural applications can include treatment of cancer, adenocarcinoma, carcinoma, ovarian cancer, endometrial cancer, neuroendocrine tumors, and endometriosis in farmyard and/or companion animals.

### BRIEF DESCRIPTION OF THE DRAWINGS

This invention is described with respect to specific embodiments thereof. Additional features can be appreciated from the FIG.s in which:

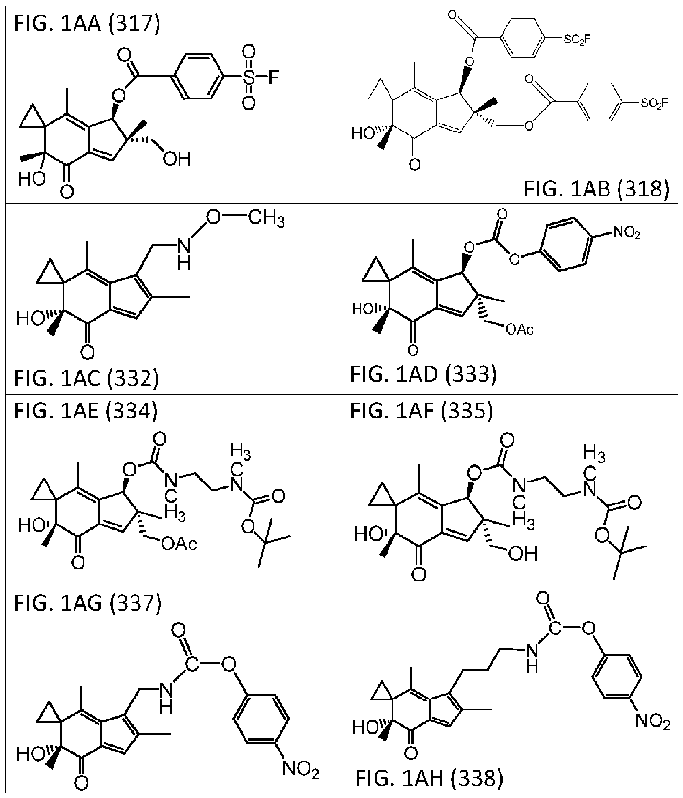

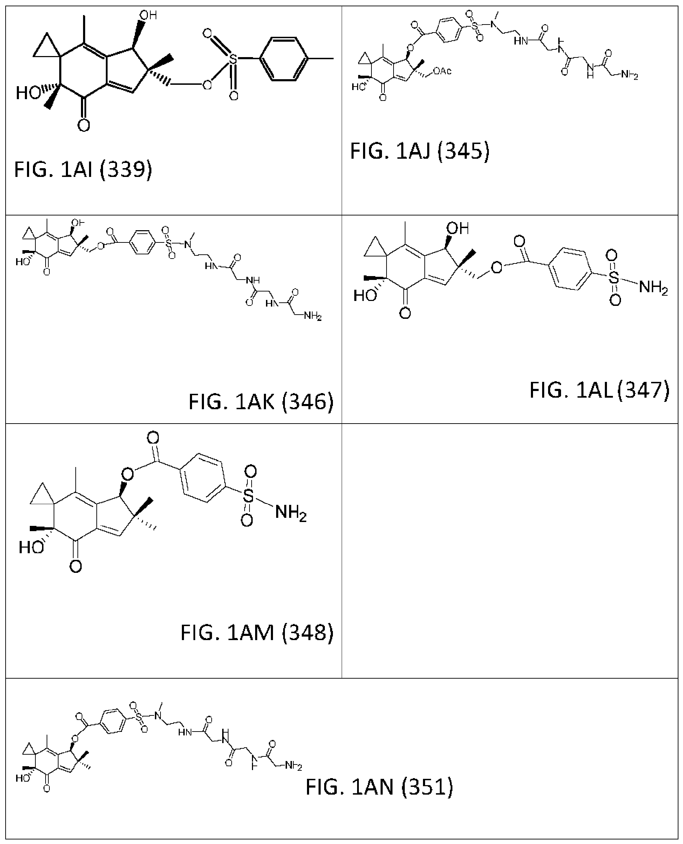

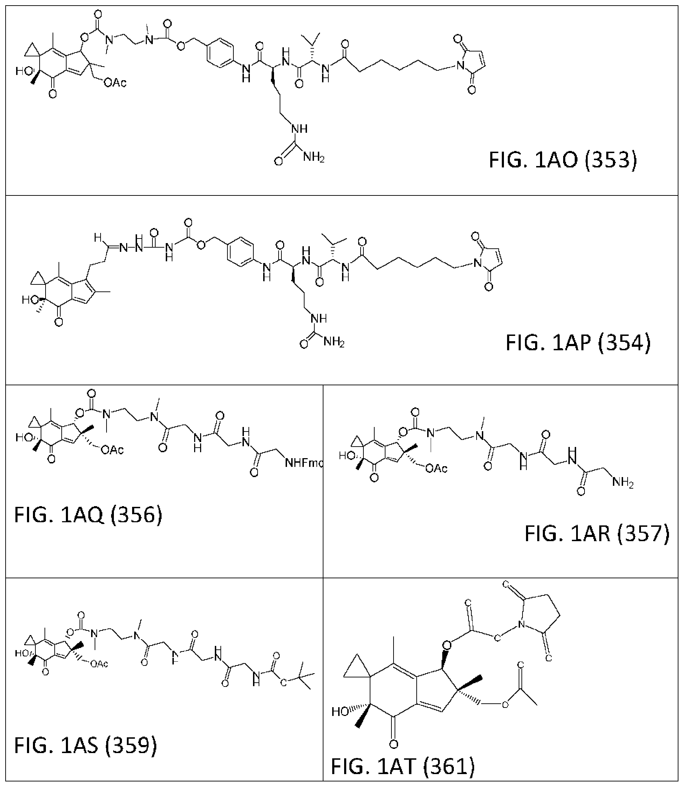

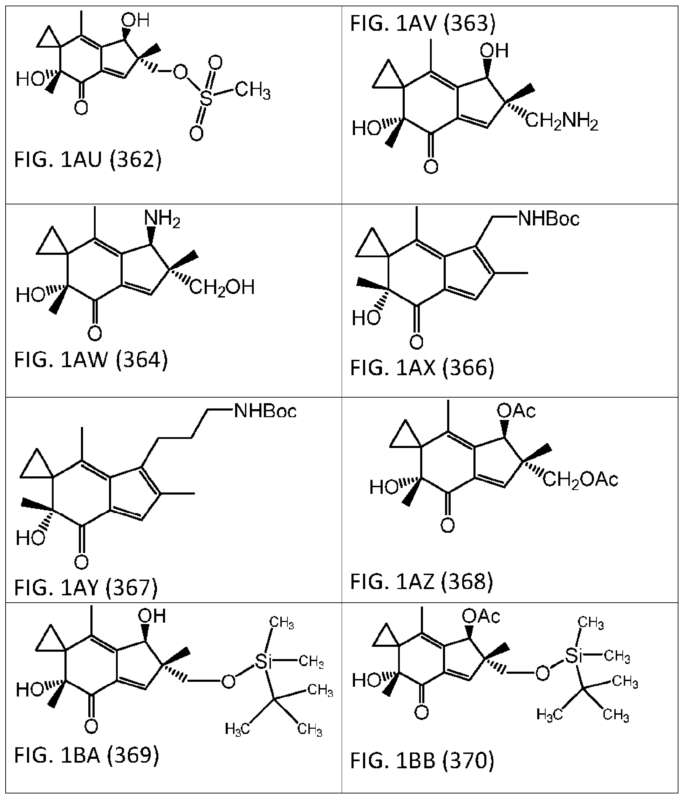

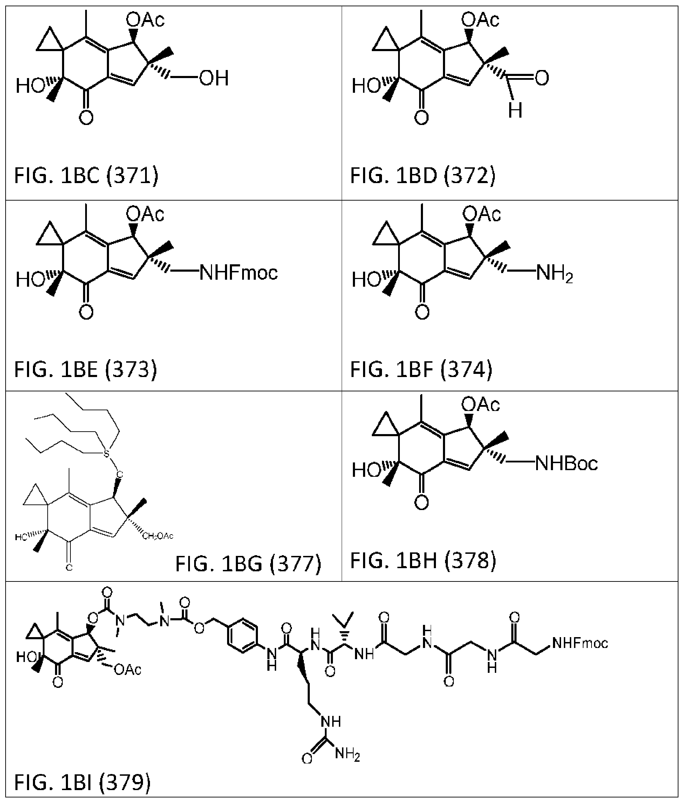

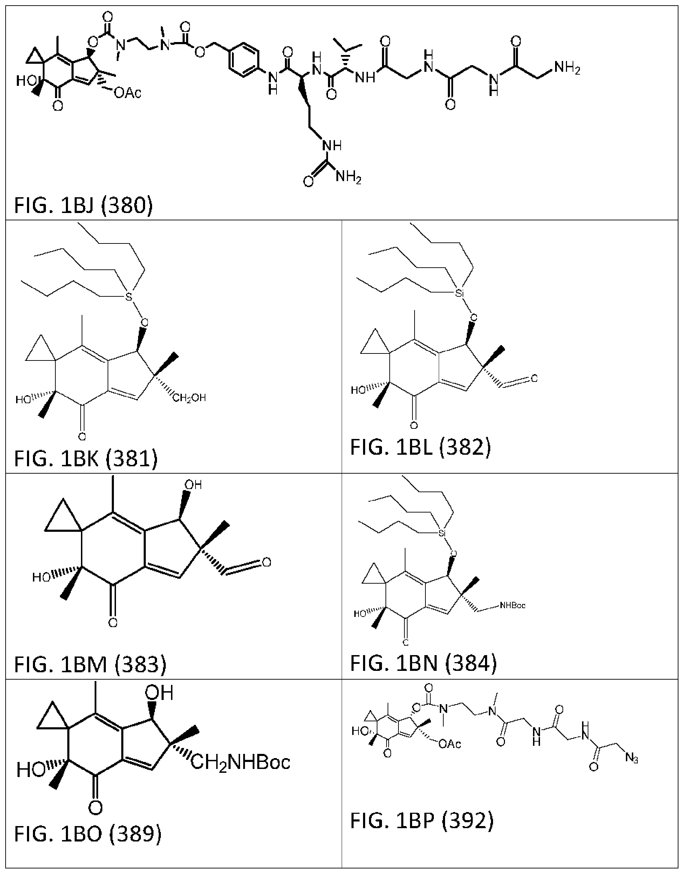

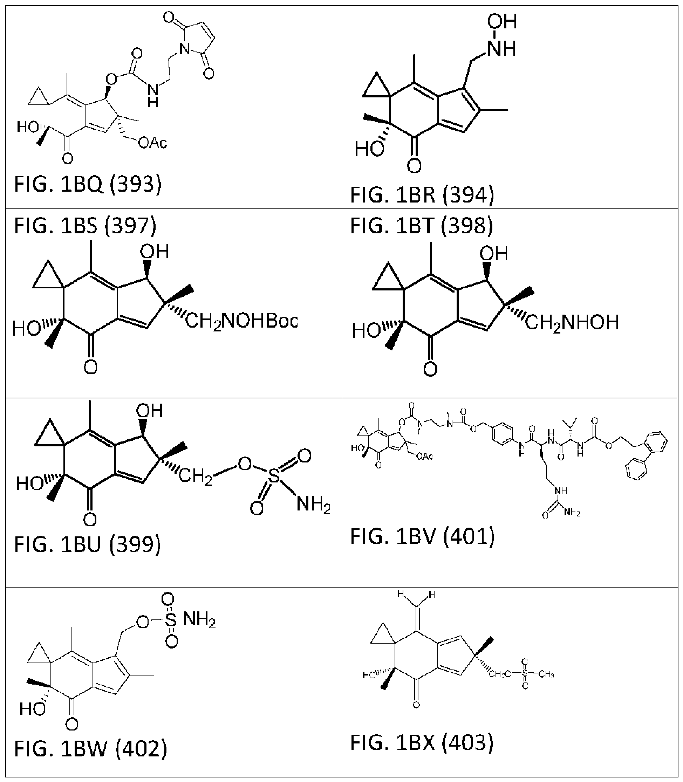

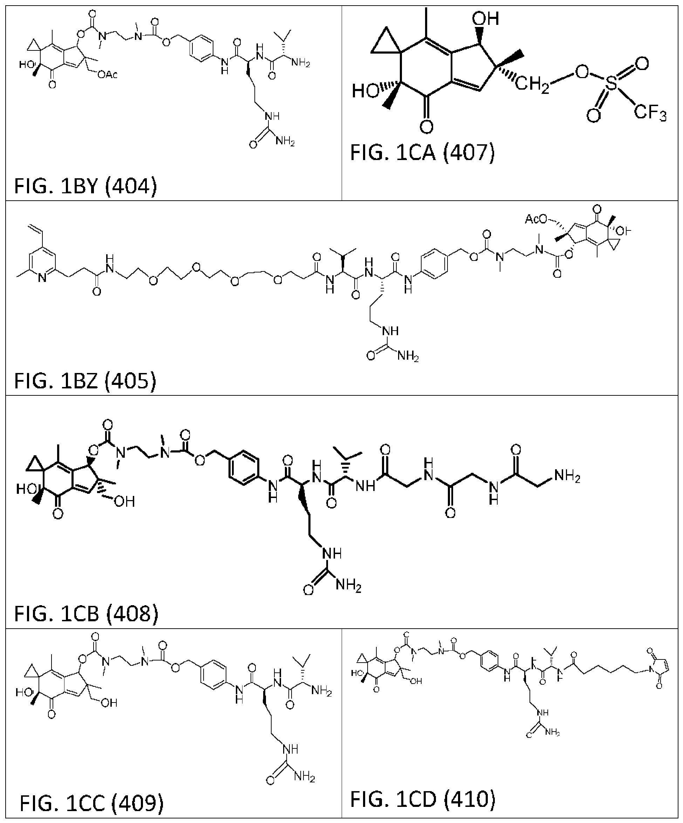

**FIG. 1AA** shows **analog 317** ; **FIG. 1AB** shows **analog 318;** **FIG. 1AC** shows **analog 332;** **FIG.** 1AD shows **analog 333;** **FIG. 1AE** shows **analog 334;** **FIG. 1AF** shows **analog 335;** **FIG. 1AG** shows **analog 337;** **FIG. 1AH** shows **analog 338;** **FIG. 1AI** shows **analog 339;** **FIG. 1AJ** shows **analog 345;** **FIG. 1AK** shows **analog 346;** **FIG. 1AL** shows **analog 347;** **FIG. 1AM** shows **analog 348;** **FIG. 1AN** shows **analog 351;** **FIG. 1AO** shows **analog 353;** **FIG. 1AP** shows **analog 354;** **FIG. 1AQ** shows **analog 356;** **FIG. 1AR** shows **analog 357;** **FIG. 1AS** shows **analog 359;** **FIG. 1AT** shows **analog 361;** **FIG. 1AU** shows **analog 362;** **FIG. 1AV** shows **analog 363;** **FIG. 1AW** shows **analog 364;** **FIG. 1AX** shows **analog 366;** **FIG. 1AY** shows **analog 367;** **FIG. 1AZ** shows **analog 368;** **FIG. 1BA** shows **analog 369;** **FIG. 1BB** shows **analog 370;** **FIG. 1BC** shows **analog 371;** **FIG. 1BD** shows **analog 372;** **FIG. 1BE** shows **analog 373;** **FIG. 1BF** shows **analog 374;** **FIG. 1BG** shows **analog 377;** **FIG. 1BH** shows **analog 378;** **FIG. 1BI** shows **analog 379;** **FIG. 1BJ** shows **analog 380;** **FIG. 1BK** shows **analog 381;** **FIG. 1BL** shows **analog 382;** **FIG. 1BM** shows **analog 383;** **FIG. 1BN** shows **analog 384;** **FIG. 1BO** shows **analog 389;** **FIG. 1BP** shows **analog 392;** **FIG. 1BQ** shows **analog 393;** **FIG. 1BR** shows **analog 394;** **FIG. 1BS** shows **analog 397;** **FIG. 1BT** shows **analog 398;** **FIG. 1BU** shows **analog 399;** **FIG. 1BV** shows **analog 401;** **FIG. 1BW** shows **analog 402;** **FIG. 1BX** shows **analog 403;** **FIG. 1BY** shows **analog 404;** **FIG. 1BZ** shows **analog 405;** **FIG. 1CA** shows **analog 407;** **FIG. 1CB** shows **analog 408;** **FIG. 1CC** shows **analog 409** and **FIG. 1CD** shows **analog 410,** according to various embodiments of the invention.

### DETAILED DESCRIPTION OF THE INVENTION

**Definitions.** As used herein, the term "receptor for a biologically active polypeptide" means a receptor which can bind a biologically active peptide conjugate.

As used herein, the term "cell population" is used to describe a set or subset of cells expressing a molecule such as a receptor.

The phrase 'Other Drugs' means docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, and rituximab.

'Acylfulvene' means an Illudofulvene subgroup with the following structural molecular formula: 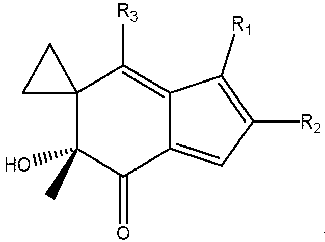 where R₁, R₂, R₃, R₈, R₉, R₁₀, R₁₁, are as set forth in Formula P1, as given herein.

'Formula P1' means R₁ represents -H, -CR₉R₈OH, -CHR₉CHR₈OH, - CHR₁₀CHR₉CHR₈OH, -CH₂CHR₁₀CHR₉CHR₈OH, -CR₉R₈OH, -CHR₉CHR₈OH, _ CHR₁₀CHR₉CHR₈OH, -CH₂CHR₁₀CHR₉CHR₈OH, -C(=O)H, -CH₂(C=O)H, -CH₂CH₂(C=O)H, - CH₂CH₂CH₂(C=O)H, -CH₂CH₂CH₂CH₂(C=O)H, -CR₉R₈(C=O)H, -CHR₉CHR₈(C=O)H, - CH₂CHR₉CHR₈(C=O)H, -CR₉R₈(C=O)R₁₀, -CHR₉CHR₈(C=O)R₁₀, -CH₂CHR₉CHR₈(C=O)R₁₀, - CH₂CH₂CHR₉CHR₈(C=O)R₁₀, -CO₂H, -CHR₉CO₂H, -CHR₈CHR₉CO₂H, - CHR₁₀CHR₈CHR₉CO₂H, -CO₂R₁₀, -CHR₉CO₂R₁₀, -CHR₈CHR₉CO₂R₁₀, - CH₂CHR₈CHR₉CO₂R₁₀, -CHR₉CH₂CH₂CHR₈CO₂H, -CHR₉CH₂CH₂CHR₈CO₂R₁₀, -CR₈₌CH₂, - CHR₈CH=CH₂, -CH₂CHR₈CH=CH₂, -CH₂CH₂CHR₈CH=CH₂, -CR₈₌CHR₉, -CHR₈CR₉=CH₂, - CH₂CHR₈CR₉=CH₂, -CH₂CH₂CHR₈CR₉=CH₂, -CR₈=CR₉R₁₀, -CHR-₈CH=CR₉R₁₀, - CH₂CHR₈CH=CHR₉R₁₀, -CH₂CH₂CHR₈CH=CHR₉R₁₀, -Cl, -Br, -I, -F, -NO₂, -NR₈R₉, -CH₂Cl, -CH₂Br, -CH₂I, -CH₂F, -CR₈Cl₂, -CR₈Br₂, -CR₈I₂, -CR₈F₂, -CCl₃, -CBr₃, -Cl₃, -CF₃, -CHR₈Cl, - CR₈R₉Cl, -CHR₈CHR₉Cl, -CHR₁₀CHR₈CHR₉Cl, -CH₂CHR₁₀CHR₈CHR₉Cl, -CHR₈Br, - CR₈R₉Br, -CHR₈CHR₉Br, -CHR₁₀CHR₈CHR₉Br, -CH₂CHR₁₀CHR₈CHR₉Br, -CHR₈I, -CR₈R₉I, - CHR₈CHR₉Br, -CHR₁₀CHR₈CHR₉I, -CH₂CHR₁₀CHR₈CHR₉I, -CR₈R₉NH₂, -CHR₈CHR₉NH₂, - CHR₁₀CHR₈CHR₉NH₂ -CH₂CH R₁₀CHR₈CHR₉NH₂, -CR₉R₁₀NHR₈, -CHR₉CHR₁₀NHR₈, - CH₂CHR₉CHR₁₀NHR₈, - CH₂CH₂CHR₉CHR₁₀NHR₈, -CHR₉NHR₈R₁₀, -CHR₉CH₂NHR₈R₁₀, - CH₂CH₂CHR₉NR₁₀R₈, -CH₂CH₂CH₂CH₂NHR₈, -CR₉R₈R₁₀, -CH₂CR₈R₉OR₁₀, - CH₂CH₂CR₈R₉OR₁₀, -CH₂CH₂CH₂R₈R₉OR₁₀, -CH₂CH₂CH₂CH₂R₈R₉OR₁₀, - CHR₈OC(=O)CHR₉R₁₀, -CHR8OC(=O)CHR₉R₁₀, -CH₂CH₂OC(=O)CHR₉R₁₀, - CH₂CH₂CHR₈OC(=O)CHR₉R₁₀, -CH₂CH₂CH₂CH₂OC(=O)CHR₉R₁₀, -CHR₈(=O)OCHR₉R₁₀, - CHR₈(=O)OCHR₉R₁₀, -CH₂CHR₈(=O)OCHR₉R₁₀, -CH₂CH₂CHR₈(=O)OCHR₉R₁₀, - CH₂CH₂CH₂CHR₈(=O)OCHR₉R₁₀, -R₁₂C=CR₉CH₂OH, -R₁₂C=CR₉C(=O)H, - R₁₂C=CR₉CH₂OR₁₀, -R₁₂C=CR₉C(=O) R₁₀, -CH₃, -CH₂CH₂, -CHR₈CH₂, -CHR₈CH₂CH₂, - CHR₈CHR₉CH₃, -OCH₃, -OCR₈R₉R₁₀, -OCH₂CR₈R₉R₁₀, -OCR₉R₈CHR₁₀, -OCHR₈CH₂CH₃, - OCHR₈CHR₉CH₃, -NR₈CH₃, -NR₈CH₂CH₃, -NR₉CHR₈CH₃, -NR₉CHR₈CH₂CH₃, - NR₁₀CHR₈CHR₉CH₃, -OCH₂OR₈, -OCHR₈OR₉, -OCHR₈CH₂OR₉, -OCHR₈CHR₉OR₁₀, - OC(=O)OR₈, -OCH₂C(=O)OR₈, -OCHR₉C(=O)OR₈, -CR₈(=N)H, -CH₂CR₈(=N)H, - CH₂CR₈(=N)H, -CH₂CH₂CR₈ (=N)H, -CH₂CH₂CH₂ CR₈(=N)H, -CH₂CH₂CH₂CH₂ CR₈(=N)H, - CR₈(=N)OH, -CH₂CR₈(=N)OH, -CH₂CR₈(=N)OH, -CH₂CH₂CR₈(=N)OH, -CH₂CH₂CH₂ CR₈(=N)OH, -CH₂CH₂CH₂CH₂ CR₈ (=N)OH, -CR₈(=N)R₉, -CH₂CR₈(=N)R₉, -CH₂CR₈(=N)R₉, -CH₂CH₂CR₈ (=N)R₉, -CH₂CH₂CH₂ CR₈(=N)R₉, -CH₂CH₂CH₂CH₂ CR₈(=N)R₉, -CR₈(=N)OR₉, -CH₂CR₈(=N)OR₉, -CH₂CR₈(=N)O R₉, -CH₂CH₂CR₈ (=N)OR₉, -CH₂CH₂CH₂ CR₈(=N)OR₉, - CH₂CH₂CH₂CH₂ CR₈ (=N)OR₉, -CR₈(=N)NR₉, -CH₂CR₈(=N)NR₉" -CH₂CR₈(=N)NR₉,, - CH₂CH₂CR₈ (=N)NR₉, -CH₂CH₂CH₂-CR₈(=N)NR₉,, -CH₂CH₂CH₂CH₂CR₈ (=N)NR₉, - CR₈(=N)NR₉S(=O)₂R₁₀, -CH₂C(R₈)(=N)NR₉S(=O)₂R₁₀, -CH₂CH₂ C(R₈)(=N)NR₉S(=O)₂R₁₀, - CH₂CH₂CH₂C(R₈)(=N)NR₉S(=O)₂R₁₀, -CH₂CH₂CH₂CH₂, -C(R₈)(=N)NR₉S(=O)₂R₁₀, - R₁₂N(R₈)C(=O)NR₉R₁₀, -R₁₂N(R₈)C(=S)NR₉R₁₀, -R₁₂N(OR₈)C(=O)NR₉R₁₀, - R₁₂N(OR₈)C(=S)NR₉R₁₀, -R₁₂OS(O₂)NH₂, -R₁₂NHS(O₂)NH₂, -R₁₂OS(O₂)NR₈R₉, - R₁₂NHS(O₂)NR₈R₉, -CH₂N(R₈)S(O2)NR₉R₁₀, -CH₂CH₂N(R₈)S(O2)NR₉R₁₀, - CH₂CH₂CH₂N(R₈)S(O2)NR₉R₁₀, -CH₂N(R₈)S(O2)CR₉R₁₀R₁₁, -CH₂CH₂N(R₈)S(O2) CR₉R₁₀R₁₁, -CH₂CH₂CH₂N(R₈)S(O2)CR₉R₁₀R₁₁, -N(R₈)C(=O)R₉, -CH₂N(R₈)C(=O)R₉, - CH₂CH₂N(R₈)C(=O)R₉, -CH₂CH₂CH₂N(R₈)C(=O)R₉, -CH₂N(R₈)(C=O)NR₉R₁₀, - CH₂CH₂N(R₈)(C=O)NR₉R₁₀, -CH₂CH₂N(R₈)(C=O)NR₉R₁₀, -CH₂N(R₈)(C=O)CR\₉R₁₀R₁₁, - CH₂CH₂N(R₈)(C=O) CR₉R₁₀R₁₁, -CH₂CH₂N(R₈)(C=O) CR₉R₁₀R₁₁, -R₁₂N(OH)C(=O)NHOH, - R₁₂N(OH)C(=S)NHOH, -R₁₂N(OR₈)C(=O)NHOR₉, -R₁₂N(OR₈)C(=S)NHOR₉, - R₁₂OS(O₂)NHOH, -R₁₂NHS(O₂)NHOH, -R₁₂OS(O₂)NHOR₉, -R₁₂OS(O₂)N(R₈)OR₉, - R₁₂NR₈S(O₂)NHOR₉, -CR₉(=N)OR₈, -NH(OR₈), -C(C=O)NHR₈, -C(C=O)NR₉R₈, - NR₁₀(OR₈)C(=O)R9, -N(OR₈)C(=O)NR₉, -NR₈(R₉)SR₁₀, -N(R₈)S(=O)R₉, -NR(R₈)S(=O)₂R₉, - OC(=O)NR₈, -N(OR₈)C(=O)OR₉, -N(R₈)C(=S)R₉, -O(S(=O)₂R₈, -R₁₂O(S(=O)₂R₈, - O(S(=O)₂NR₈, -R₁₂O(S(=O)₂NR₈, -S(=O)R₈, -R₁₂S(=O)R₈, -S(=O)₂R₈, -R₁₂S(=O)₂R₈, - NR₁₀(R₉)S(=O)₂NHR₈, -NR₉(C=O)R₈, -NR₉(C=O)OR₈, -NR₉O(C=O)OR₈, -NR₉(C=O)NR₈R₁₀, - R₁₂N(R₉)S(=O)₂NHR₈, -R₁₂N(R₉)(C=O)R₈, -R₁₂N(R₉)(C=O)OR₈, -R₁₂N(R₉)(C=O)NR₈, - N(=NR₁₀)R₈, -R₉-N(=NR₁₀)R₈, -C(R₁₀)(=N-N=)CR₈R₉; -N₃, -CH₂N₃, -CH₂CH₂N₃, - CH₂CH₂CH₂N₃, -CH₂CH₂CH₂CH₂N₃, -CHR₈N₃, -CR₉R₈N₃, -CHR₉CHR₈N₃, - CH₂CHR₉CHR₈N₃, -CH₂CH₂CH₂CHR₈N₃, -C(R₈)=N-R₉, -CH₂C(R₈)=N-R₉, - CH₂CH₂C(R₈)=N-R₉, -CH₂CH₂CH₂C(R₈)=N-R₉, -N₃, -CH₂CH₂N₃, -CH₂CH₂CH₂N₃, -CH₂CH₂CH₂CH₂N₃; - CH₂NHC(=O)OC(CH₃)₃, -CH₂NOHC(=O)OC(CH₃)_{3.} -CH₂CH₂NHC(=O)OC(CH₃)₃, -CH₂ CH₂NOHC(=O)OC(CH₃)₃, -CH₂CH₂CH₂NHC(=O)OC(CH₃)₃, -CH₂CH₂ CH₂NOHC(=O)OC(CH₃)₃, -CH₂NHFmoc; -CH₂NOHFmoc; -CH₂CH₂NHFmoc; - CH₂CH₂NOHFmoc; -CH₂CH₂CH₂NH-Fmoc; -CH₂CH₂CH₂NOH-Fmoc, and R2 and R3 each independently represent -H, -OH, -CH₃, -OCH₃, -C(=O)CH₃, -OC(=O)CH₃, -C(=O)OCH₃, - C(=O)CH₂CH₃, -OC(=O)CH₂CH₃, -C(=O)OCH₂CH₃, -CH₂CH₃, -CH(CH₃)₂, -C(CH₃) ₃, - CH₂CH₂CH₃, -CH₂CH(CH₃)₂, -CHC(CH₃)₃, -CH₂OH, -NH₂, -NHOH, -CH₂NH₂, -CH₂NHOH, - N₃, and (C 1-C4)alkyl; where R₈ R₉, R₁₀, R₁₁ each independently represent -H, -OH, -CH₃, - CH₂CH₃, -CH₂₌CH₂, -CH₂CH₂=CH₂, -CH₂CH₂CH₂=CH₂, -C(H)=O, -CH₂C(H)=O, - CH₂CH₂C(H)=O, -CH(CH₃)CH₃, -CH₂CH₂CH₃, -CH₂CH(CH₃)CH₃, -CHC(CH₃)₂CH₃, - C(=O)CH₃, -C(=O)CH₂CH₃,-C(=O)NH₂, -CH₂OH, -CH₂CH₂OH, -C(H)(OH)C(H₂)(OH), - OCH₃, -OC(CH₃)₃, -OCH₂CH₃, -OCH(CH₃)₂, -CO₂H, -C(=O)OCH_{3.} -C(=O)OCH₂CH_{3.} - OC(=O)CH_{3.} -OC(=O)CH₂CH_{3.} -OC(=O)OCH_{3.} -OC(=O)OCH₂CH₃, -C(CH₃)₃, - CH₂CH₂(CH₃)₂, -CHC(CH₃)₃, -CH₂CH₂OH, CH₂CH₂CH₂OH, -Cl, -Br, -I, -F, -CH₂Cl, -CH₂Br, -CH₂I, -CH₂F, -CHCl₂, -CHBr₂, -CHI₂, -CHF₂, -CCl₃, -CBr₃, -CI₃, -CF₃, -NH₂, -CH₂NH₂, - NH(OH), -CH₂N(OH), -NH(OCH₃), -N(OCH₂CH₃), -CH₂NH(OCH₃), -CH₂N(OCH₂CH₃), -N₃, -CH₂N₃, -CH₂CH₂N₃, -CH=NH, -CH=NOH, -CH=NOCH₃, -SH, -SCH₃, -SCH₂CH₃, -NO₂, - CN, cyclopropane ring, saturated or unsaturated cyclobutane ring, saturated or unsaturated cyclopentane ring, saturated or unsaturated cyclohexane ring, benzene ring, phenolic ring, xylene ring, an amino acid(s), and (C₁-C₄)alkyl, and R12 represents -CH₂-, -CH₂CH₂-, -CH(CH₃)CH₂-, -C(CH₃)CH₂-, -CH=CH-, -O-, -S-, -O(C=O)-, -(C=O)O-, -NH-, -N(R₈)-, -N(OH)-, -CH2-O-, -O-CH₂-, -CH₂CH₂-O-, -O-CH₂CH₂-, -S(=O)-, -(S=O)₂-, -NH(S=O)₂-, -N(OH)S(=O)₂-, -S(=O)₂NH-, -S(=O)₂N(OH)-, -NHS(=O)₂-, -N(OH)S(=O)₂-, -CH₂NH-, -CH₂N(R₈)-, -CH₂N(OH)-, -NHCH₂-, -N(R₈)CH₂-, -N(OH)CH₂-, -OC(C=O)O-, -OC(=O)NR₈-, -NR₈C(=O)O-.

'Illudin' means an Illudofulvene subgroup with the following structural molecular formula: 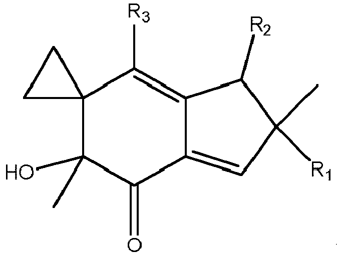 where R₁, R₂, R₃, are as set forth in Formula P2, as given herein.

'Formula P2' means where R₁, R₂, and R₃ each independently represent -H, -OH, - CH₃, -OCH₃, -C(=O)CH₃, -OC(=O)CH₃, -C(=O)OCH₃, -C(=O)CH₂CH₃, -OC(=O)CH₂CH₃, - C(=O)OCH₂CH₃,-CH₂CH₃, -CH(CH₃)₂, -C(CH₃)₃, -CH₂CH₂CH₃, -CH₂CH(CH₃)₂, -CHC(CH₃)₃, -CH₂OH, -NH₂, -CH₂NH₂, -N₃, -CH₂NHOH, -NHOH, -CH₂NHC(=O)OC(CH₃)₃, - CH₂NOHC(=O)OC(CH₃)₃, -C(=O)H, -C(=O)OH, -CH₂OSi(CH₃)₂C(CH₃)₃, -OS(=O)₂CH₃, - CH₂OS(=O)₂(C₄H₆)CH₃, -OC(=O)O(C₆H₄)NO₂, - OC(=O)N(CH₃)CH₂CH₂N(CH₃)C(=O)OC(CH₃)₃, -OC(C=O)N(CH₃)CH₂CH₂N(CH₃)H, - OC(=O)NHCH₂CH₂NH₂, -NH(FMOC), -NOH(FMOC), -CH₂NH(FMOC), -CH₂NOH(FMOC), -OSi(CH₂CH₂CH₂CH₃)₃, -OSi(CH₃)₂C(CH₃)₃, and (C1-C4)alkyl.

**Table V** is a listing of IUPAC names of the Illudofulvene Analog according to various embodiments of the present invention. **FIGS. 1AA**

- **1CY** show the structure of analogs **317, 318, 332, 333, 334, 335, 337, 338, 339, 345, 346, 347, 348, 351, 353, 354, 356, 357, 359, 361, 362, 363, 364, 366, 367, 368, 369, 370, 371, 372, 373, 374, 377, 378, 379, 380, 381, 382, 383, 384, 389, 392, 393, 394, 397, 398, 399, 401, 402, 403, 404, 405, 407, 408, 409, and 410** respectively.

As used herein, the terms "analog", "medicant" and "medicant moiety" are used interchangeably and comprise synthetic and naturally occurring drugs, toxins, nutraceuticals and other cytoactive, anti-inflammatory and bioactive molecules including Doxorubicin (Immunomedics), auristatins E (Seattle Genetics), auristatins F (Celdex), monomethyl auristatin E (MMAE) (Amgen), monomethyl auristatin F (MMAF) (Astelles), maytanasines (Immunogen), DM1 (Biotest), DM4 (Amgen), calicheamicin (CellTech), irinotecan, folate, SN38 (Immunomedics), Pyrrolobenzodiazepines (Seattle Genetics), MGBA a duocarmycin derivative (Medarex), thalidomides, taxanes, penicillins, Trastuzumab emtansine (Genentech for Breast cancer uses maytanasine derive DM-1). Some of the above analogs are stand alone drugs, but can be used as a medicant moiety in an affinity drug conjugate according to various embodiments of the invention.

As used herein, the phrase "peptide receptor" includes peptide hormone receptors, protein hormone receptors, chemotactic receptors and chemokine receptors.

As used herein, the term "receptor" includes growth factor receptors, peptide hormone receptors, peptide receptors, steroid hormone receptors, steroid receptors and lipid receptors.

As used herein, phrase "affinity medicant conjugate" is an Affinity Moiety covalently bound to a medicant moiety, and includes antibody medicant conjugates, where the antibody is directed to a specific receptor. As used herein the phrase 'Affinity Moiety' includes antibodies, antibody fragments, peptides, proteins, growth factors, steroids, and lipids, where the antibodies, antibody fragments, peptides, proteins, growth factors, steroids, folate or lipids have an affinity for a specific receptor, receptors, is processed by an enzyme to produce a ligand that has an affinity for a specific receptor or otherwise directs the Affinity Moiety to a specific subset of cells. A 'medicant moiety' includes a group bound to an Affinity Moiety, which when released acts as a medicant.

As used herein, the term "Affinity Moiety" (AM) is used to describe a chemical group or molecule that can bind a receptor or proteins. An AM is understood to have a minimum binding affinity greater than approximately 1 x 10⁻³ M affinity. As used herein, the term AM includes "ligands", "ligand moieties", "affinity unit" and an AM modified to include a linker. As used herein, the phrase "an affinity moiety directed to a peptide receptor" is used to describe a molecule or a portion of a molecule which has a binding affinity to the peptide receptor greater than approximately 1 x 10⁻¹⁰ M. In this range approximately means 1 × 10⁻⁹ M to 1 × 10⁻¹¹ M. In an embodiment of the invention, an AM directed to a peptide receptor has a binding affinity to the peptide receptor greater than approximately 1 × 10⁻¹² M. In this range approximately means 1 × 10⁻¹¹ M to 1 × 10⁻¹³ M.

As used herein, the term "cytoactive" (which is abbreviated as "CA") is used to describe a small molecule that disrupts a cellular process, modulates a cellular process or otherwise affects the normal function of the cell. As used herein, the term "toxin" or "toxic" is used to describe a small molecule which interferes with RNA or DNA synthesis, causes RNA or DNA strand scission, blocks cell cycling, division, replication or is otherwise cytotoxic to the cell. As used herein, the term "toxin moiety" is used to describe a toxin modified to include a linker. As used herein, the phrase "a moiety possessing cell cytotoxicity" is used to describe a toxin moiety which when given in the concentration range of approximately 1 × 10⁻³ M to approximately 1 × 10⁻⁹ M results in inhibition of DNA synthesis or proliferation in an appropriate cultured cell line and/or when administered intravenously to an animal in the dosage approximately 1 × 10⁻⁴ g to approximately 1 × 10⁻⁹ g of the compound per kilogram of body weight of the animal results in *in vivo* cell death. As used herein, the term "ablated" is used to describe a reduction in the cell population of between approximately 50% and approximately 95%. In this range approximately means plus or minus five (5) per cent. In an embodiment of the invention, a toxin moiety ablating a cell population reduces the cell population by approximately 100 per cent. As used herein, the term "impaired" is used to describe a reduction in the cell population of between approximately 30% and approximately 50%. In this range approximately means plus or minus ten (10) per cent.

As used herein, the term "linker" is used to describe one or more covalently bonded groups of atoms that are covalently bonded to a medicant moiety and an AM For example a linker can be covalently bound to both an illudofulvene moiety and to an antibody or other ligand moiety with an affinity for a receptor.

As used herein, the term "non releasable linker" is used to describe a linker covalently bound to an AM and a medicant moiety in which the AM and the medicant moiety remain covalently bound to the linker after internalization and exposure to both reducing and acidic environments of vesicles within the cell. As used herein, the term "membrane permeability" is used to describe a compound comprising a linker covalently bound to an AM and an illudofulvene moiety, where the compound can diffuse across membranes within the cell.

As used herein, the term "transmembrane receptor" means a protein that spans the plasma membrane of a cell with the extracellular domain of the protein having the ability to bind an AM and the intracellular domain having an activity such as activation of G protein signaling which is induced upon the AM binding.

As used herein, the term "seven transmembrane receptor" is a transmembrane receptor including a transmembrane domain where the protein spans the cell membrane in seven (7) regions.

As used herein, the term "G-protein coupled receptor" means a seven transmembrane domain receptor which transduces a biological signal via G-protein coupling.

As used herein, the term "conjugated" or "conjugate" means a chemical compound that is formed by joining two or more compounds with one or more chemical bonds or linkers. In an embodiment of the invention, an antibody and a medicant form a conjugate.

As used herein, the term "antibody" herein is used in the broadest sense and specifically covers intact antibodies, monoclonal antibodies, polyclonal antibodies, monospecific antibodies, multi-specific antibodies (e.g., bi-specific antibodies), and antibody fragments that exhibit the desired biological activity, including those antibodies directed against Alk, Alk fusion proteins, CD 2 (SEQ. ID. 001), CD3epsilon (SEQ. ID. 002), CD5 (SEQ. ID. 003), CD7 (SEQ. ID. 004), CD19 (SEQ. ID. 005), CD20 (SEQ. ID. 006), CD22 (SEQ. ID. 007), CD25 (SEQ. ID. 008), CD30 (SEQ. ID. 009), CD33 (SEQ. ID. 010), CD37 (SEQ. ID. 011), CD44 (SEQ. ID. 012), CD44v6 (SEQ. ID. 013), CD56 (SEQ. ID. 014), CD70 (SEQ. ID. 015), CD74 (SEQ. ID. 016), CD79 (SEQ. ID. 017), CD79b (SEQ. ID. 018), CD 80 (SEQ. ID. 019), CD 86 (SEQ. ID. 020), CD138 (syndecan 1) (SEQ. ID. 021), CAIX (SEQ. ID. 022), Integrin alphaVbeta 3 (SEQ. ID. 023), EphA2 (SEQ. ID. 024), Crypto1 (SEQ. ID. 025), CanAg (SEQ. ID. 026), ENPP3 (SEQ. ID. 027), Nectin-4 (SEQ. ID. 028), Mesothelin (SEQ. ID. 029), Lewis Y (SEQ. ID. 030), EGFRvIII (SEQ. ID. 031), SLC44A4 (SEQ. ID. 032), EBTR (endothelin) (SEQ. ID. 033), erbB2/neu/HER2 (SEQ. ID. 034), Transferrin receptor (SEQ. ID. 035), 55kDa breast cancer antigen, 72 kDa TAA, GPNMB (osteoactivin) (SEQ. ID. 038), CA-IX (SEQ. ID. 039), CEA (CD66e) (SEQ. ID. 040), CEACAM5 (SEQ. ID. 041), PSMA (SEQ. ID. 042), CA125 (MUC16) (SEQ. ID. 043), Muc1 (CA6) (SEQ. ID. 044), Melanoma glycoprotein NMB (SEQ. ID. 045), IL-2R (SEQ. ID. 166 and 046), IL13R (SEQ. ID. 047), TACSTD2 (TROP2 or EGP1) (SEQ. ID. 048), Folate receptor 1 (SEQ. ID. 049), Mucin 16 (SEQ. ID. 050), Endothelin receptor ETB (SEQ. ID. 051), STEAP1 (SEQ. ID. 052), SLC44A4 (AGS-5) (SEQ. ID. 053), AGS-16 (SEQ. ID. 054), and Guanylyl cyclase C (SEQ. ID. 055). An intact antibody has primarily two regions: a variable region and a constant region. The variable region binds to and interacts with a target antigen. The variable region includes a complementary determing region (CDR) that recognizes and binds to a specific binding site on a particular antigen. The constant region may be recognized by and interact with the immune system. An antibody can be of any type or class (e.g., IgG, IgE, IgM, IgD, and IgA) or subclass (e.g., IgG1, IgG2, IgG3, IgG4, IgA1 and IgA2). The antibody can be derived from any suitable species. In some embodiments, the antibody is of human or murine origin. An antibody can be, for example, human, humanized or chimeric.

As used herein, the terms "specifically binds" and "specific binding" refer to antibody binding to a predetermined antigen. Typically, the antibody binds with an affinity of at least about 1 ×10⁷ M, and binds to the predetermined antigen with an affinity that is at least two-fold greater than its affinity for binding to a non-specific antigen (e.g., Bovine Serum Albumin, casein) other than the predetermined antigen or a closely-related antigen.

As used herein, the term "monoclonal antibody" as used herein refers to an antibody obtained from a population of substantially homogeneous antibodies, i.e., the individual antibodies comprising the population are identical except for possible naturally-occurring mutations that may be present in minor amounts and includes "chimeric" antibodies in which a portion of the heavy and/or light chain is identical to or homologous with the corresponding sequence of antibodies derived from a particular species or belonging to a particular antibody class or subclass, while the remainder of the chain(s) is identical to or homologous with the corresponding sequences of antibodies derived from another species or belonging to another antibody class or subclass, as well as fragments of such antibodies, so long as they exhibit the desired biological activity. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

As used herein, an "intact antibody" is one which comprises an antigen-binding variable region as well as a light chain constant domain (C_{L}) and heavy chain constant domains, C_{H1}, C_{H2}, C_{H3} and C_{H4}, as appropriate for the antibody class. The constant domains may be native sequence constant domains (e.g., human native sequence constant domains) or amino acid sequence variants thereof.

As used herein, the term an "intact antibody" may have one or more "effector functions", which refers to those biological activities attributable to the Fc region (e.g., a native sequence Fc region or amino acid sequence variant Fc region) of an antibody. Examples of antibody effector functions include complement dependent cytotoxicity, antibody-dependent cell-mediated cytotoxicity (AMCC) and antibody-dependent cell-mediated phagocytosis.

As used herein, the term an "antibody fragment" comprises a portion of an intact antibody, preferably comprising the antigen-binding or variable region thereof. Examples of antibody fragments include Fab, Fab', F(ab')₂, and Fv fragments, di-abodies, tri-abodies, tetrabodies, linear antibodies, single-chain antibody molecules, scFv, scFv-Fc, multi-specific antibody fragments formed from antibody fragment(s), a fragment(s) produced by a Fab expression library, or an epitope-binding fragments of any of the above which immuno specifically bind to a target antigen (e.g., a cancer cell antigen, a viral antigen or a microbial antigen).

As used herein, the term "variable" in the context of an antibody refers to certain portions of the variable domains of the antibody that differ extensively in sequence and are used in the binding and specificity of each particular antibody for its particular antigen. This variability is concentrated in three segments called "hypervariable regions" in the light chain and the heavy chain variable domains. The more highly conserved portions of variable domains are called the Framework Regions (FRs). The variable domains of native heavy and light chains each comprise four FRs connected by three hypervariable regions.

As used herein, the term "hypervariable region" when used herein refers to the amino acid residues of an antibody which are responsible for antigen-binding. The hypervariable region generally comprises amino acid residues from a "complementarity determining region" or "CDR" (e.g., residues 24-34 (L1), 50-56 (L2) and 89-97 (L3) in the light chain variable domain and 31-35 (HI), 50-65 (H2) and 95-102 (L3) in the heavy chain variable domain, and/or those residues from a "hypervariable loop" (e.g., residues 26-32 (L1), 50-52 (L2) and 91-96 (L3) in the light chain variable domain and 26-32 (HI), 53-55 (142) and 96-101 (H3) in the heavy chain variable domain. FR residues are those variable domain residues other than the hypervariable region residues as herein defined.

As used herein, a "single-chain Fv" or "scFv" antibody fragment comprises the V_{H} and V_{L} domains of an antibody, wherein these domains are present in a single polypeptide chain. Typically, the Fv polypeptide further comprises a polypeptide linker between the V_{H} and V_{L} domains which enables the scFv to form the desired structure for antigen binding.

As used herein, the term "di-abody" refers to small antibody fragments with two antigen-binding sites, which fragments comprise a variable heavy domain (V_{H}) connected to a variable light domain (V_{L}) in the same polypeptide chain. By using a linker that is too short to allow pairing between the two domains on the same chain, the domains are forced to pair with the complementary domains of another chain and create two antigen-binding sites.

As used herein, "Humanized" forms of non-human (e.g., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin.

As used herein, "isolated" means separated from other components of (a) a natural source, such as a plant or animal cell or cell culture, or (b) a synthetic organic chemical reaction mixture. As used herein, "purified" means that when isolated, the isolate contains at least 95%, and in another aspect at least 98%, of a compound (e.g., a conjugate) by weight of the isolate.

As used herein, an "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or non-proteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or non-reducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

As used herein, an antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies). The cell is a tumor cell, e.g., a breast, ovarian, stomach, endometrial, salivary gland, lung, kidney, colon, thyroid, pancreatic or bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells.

As used herein, the term "therapeutically effective amount" refers to an amount of a medicant effective to treat a disease or disorder in a mammal. In the case of cancer, the therapeutically effective amount of the medicant may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the medicant may inhibit the growth of and/or kill existing cancer cells, it may be cytostatic and/or cytotoxic. For cancer therapy, efficacy can, for example, be measured by assessing the time to disease progression (TTP) and/or determining the response rate (RR).

As used herein, the term "substantial amount" refers to a majority, i.e. greater than approximately fifty per cent (50%) of a population, of a mixture or a sample. In this range approximately means plus or minus ten per cent (10%).

As used herein, the term "intracellular metabolite" refers to a compound resulting from a metabolic process or reaction inside a cell on an Affinity Medicant Linker conjugate (e.g., an Antibody Drug Conjugate (AMC)). The metabolic process or reaction may be an enzymatic process such as proteolytic cleavage of a peptide linker of the AMC. Intracellular metabolites include, but are not limited to, antibodies and free medicant which have undergone intracellular cleavage after entry, diffusion, uptake or transport into a cell.

As used herein, the terms "intracellularly cleaved" and "intracellular cleavage" refer to a metabolic process or reaction inside a cell on an Affinity Medicant Linker conjugate (e.g., an Antibody Medicant conjugate (AMC) or the like), whereby the covalent attachment, e.g., the linker, between the Medicant moiety (M) and the Affinity unit (e.g., an antibody (Ab)) is broken, resulting in the free Medicant, or other metabolite of the conjugate dissociated from the antibody inside the cell. The cleaved moieties of the Affinity Medicant Linker conjugate are thus intracellular metabolites.

As used herein, the term "bioavailability" refers to the systemic availability (i.e., blood/plasma levels) of a given amount of a medicant administered to a patient. Bioavailability is an absolute term that indicates measurement of both the time (rate) and total amount (extent) of medicant that reaches the general circulation from an administered dosage form.

As used herein, the term "cytotoxic activity" refers to a cell-killing, a cytostatic or an anti-proliferative effect of an Affinity Medicant Linker conjugate or an intracellular metabolite of an Affinity Medicant Linker conjugate. Cytotoxic activity may be expressed as the IC₅₀ value, which is the concentration (molar or mass) per unit volume at which half the cells survive.

As used herein, the term "cytotoxic agent" as used herein refers to a substance that inhibits or inhibits the function of cells and/or causes destruction of cells. The term is intended to include radioactive isotopes (e.g., ²¹¹At, ¹³¹I, ¹²⁵I, ⁹⁰Y, ¹⁸⁶Re, ¹⁸⁸Re, ¹⁵³Sm, ²¹²Bi, ³²P, ⁶⁰C, and radioactive isotopes of Lu), chemotherapeutic agents, and toxins such as small molecule toxins or enzymatically active toxins of bacterial, fungal, plant or animal origin, including synthetic analogs and derivatives thereof. In one aspect, the term does not include a radioactive isotope(s).

As used herein, a "disorder" is any condition that would benefit from treatment with an Affinity Medicant Linker Conjugate. This includes chronic and acute disorders or diseases including those pathological conditions which predispose a mammal to the disorder in question. Non-limiting examples of disorders to be treated herein include benign and malignant cancers; leukemia and lymphoid malignancies, neuronal, glial, astrocytal, hypothalamic and other glandular, macrophagal, epithelial, stromal and blastocoelic disorders; and inflammatory, angiogenic and immunologic disorders.

As used herein, the terms "cancer" and "cancerous" refer to or describe the physiological condition or disorder in mammals that is typically characterized by unregulated cell growth. A "tumor" comprises one or more cancerous cells.

As used herein, an "autoimmune disease" herein is a disease or disorder arising from and directed against an individual's own tissues or a co-segregate or manifestation thereof or resulting condition therefrom.

As used herein, an example of a "patient" includes, but is not limited to, a human, rat, mouse, guinea pig, monkey, pig, goat, cow, horse, dog, cat, bird and fowl. In an exemplary embodiment, the patient is a human.

As used herein, the terms "treat" or "treatment," unless otherwise indicated by context, refer to therapeutic treatment and prophylactic measures to prevent relapse, wherein the object is to inhibit or slow down (lessen) an undesired physiological change or disorder, such as the development or spread of cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already having the condition or disorder as well as those prone to have the condition or disorder.

As used herein, in the context of cancer, the term "treating" includes any or all of inhibiting growth of tumor cells, cancer cells, or of a tumor; inhibiting replication of tumor cells or cancer cells, lessening of overall tumor burden or decreasing the number of cancerous cells, and ameliorating one or more symptoms associated with the disease.

As used herein, in the context of an autoimmune disease, the term "treating" includes any or all of inhibiting replication of cells associated with an autoimmune disease state including, but not limited to, cells that produce an autoimmune antibody, lessening the autoimmune-antibody burden and ameliorating one or more symptoms of an autoimmune disease.

As used herein, in the context of an infectious disease, the term "treating" includes any or all of: inhibiting the growth, multiplication or replication of the pathogen that causes the infectious disease and ameliorating one or more symptoms of an infectious disease.

As used herein, the term "package insert" is used to refer to instructions customarily included in commercial packages of therapeutic products, that contain information about the indication(s), usage, dosage, administration, contraindications and/or warnings concerning the use of such therapeutic products.

As used herein, a "native sequence" polypeptide is one which has the same amino acid sequence as a polypeptide, e.g., a tumor-associated antigen receptor, derived from nature. Such native sequence polypeptides can be isolated from nature or can be produced by recombinant or synthetic means. Thus, a native sequence polypeptide can have the amino acid sequence of a naturally-occurring human polypeptide, a murine polypeptide, or a polypeptide from any other mammalian species.

As used herein, an "isolated" nucleic acid molecule is a nucleic acid molecule that is identified and separated from at least one contaminant nucleic acid molecule with which it is ordinarily associated in the natural source of the nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the nucleic acid where, for example, the nucleic acid molecule is in a chromosomal location different from that of natural cells.

As used herein, the expression "control sequences" refers to nucleic acid sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

As used herein, a nucleic acid is "operably linked" when it is placed into a functional relationship with another nucleic acid sequence. For example, DNA for a pre-sequence or secretory leader is operably linked to DNA encoding a polypeptide if it is expressed as a pre-protein that participates in the secretion of the polypeptide; a promoter or enhancer is operably linked to a coding sequence, for example, if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking can be accomplished by ligation at convenient restriction sites. If such sites do not exist, the synthetic oligonucleotide adaptors or linkers can be used in accordance with conventional practice.

As used herein, the terms "cell," "cell line," and "cell culture" are used interchangeably and all such designations include progeny. The words "transformants" and "transformed cells" include the primary subject cell and cultures or progeny derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, it will be clear from the context.

As used herein, the term "chiral" refers to molecules which have the property of non-superimposability of the mirror image partner, while the term "achiral" refers to molecules which are superimposable on their mirror image partner.

As used herein, the term "stereoisomers" refers to compounds which have identical chemical constitution, but differ with regard to the arrangement of the atoms or groups in space.

As used herein, "diastereomer" refers to a stereoisomer with two or more centers of chirality and whose molecules are not mirror images of one another. Diastereomers have different physical properties, e.g., melting points, boiling points, spectral properties, and reactivities. Mixtures of diastereomers may separate under high resolution analytical procedures such as electrophoresis and chromatography.

The abbreviations used herein have their conventional meaning within the chemical and biological arts. The chemical structures and formulae set forth herein are constructed according to the standard rules of chemical valency known in the chemical arts.

Where substituent groups are specified by their conventional chemical formulae, written from left to right, they equally encompass the chemically identical substituents that would result from writing the structure from right to left, e.g., -CH₂O- is equivalent to -OCH₂-.

The term "alkyl," by itself or as part of another substituent, means, unless otherwise stated, a straight (i.e., unbranched) or branched chain, or combination thereof, which may be fully saturated, mono- or polyunsaturated and can include di- and multivalent radicals, having the number of carbon atoms designated (i.e., C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, (cyclohexyl)methyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. An alkoxy is an alkyl attached to the remainder of the molecule via an oxygen linker (-O-).

The term "alkylene," by itself or as part of another substituent, means, unless otherwise stated, a divalent radical derived from an alkyl, as exemplified, but not limited by, -CH₂CH₂CH₂CH₂-. Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "heteroalkyl," by itself or in combination with another term, means, unless otherwise stated, a stable straight or branched chain, or combinations thereof, consisting of at least one carbon atom and at least one heteroatom selected from the group consisting of O, N, P, Si, and S, and wherein the nitrogen and sulfur atoms may optionally be oxidized, and the nitrogen heteroatom may optionally be quaternized. The heteroatom(s) O, N, P, S, and Si may be placed at any interior position of the heteroalkyl group or at the position at which the alkyl group is attached to the remainder of the molecule. Examples include, but are not limited to: -CH₂-CH₂-O-CH₃, -CH₂-CH₂-NH-CH₃, -CH₂-CH₂-N(CH₃)-CH₃, -CH₂-S-CH₂-CH₃, -CH₂-CH ₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃, and -CN. Up to two heteroatoms may be consecutive, such as, for example, -CH₂-NH-OCH₃.

Similarly, the term "heteroalkylene," by itself or as part of another substituent, means, unless otherwise stated, a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR', and/or -SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl," by themselves or in combination with other terms, mean, unless otherwise stated, cyclic versions of "alkyl" and "heteroalkyl," respectively. Additionally, for heterocycloalkyl, a heteroatom can occupy the position at which the heterocycle is attached to the remainder of the molecule. Examples of cycloalkyl include, but are not limited to, cyclopropyl, cyclobutyl, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and a "heterocycloalkylene," alone or as part of another substituent, means a divalent radical derived from a cycloalkyl and heterocycloalkyl, respectively.

The terms "halo" or "halogen," by themselves or as part of another substituent, mean, unless otherwise stated, a fluorine, chlorine, bromine, or iodine atom. Additionally, terms such as "haloalkyl" are meant to include monohaloalkyl and polyhaloalkyl. For example, the term "halo(C₁-C₄)alkyl" includes, but is not limited to, fluoromethyl, difluoromethyl, trifluoromethyl, 2,2,2-trifluoroethyl, 4-chlorobutyl, and 3-bromopropyl.

The term "acyl" means, unless otherwise stated, -C(O)R where R is a substituted or unsubstituted alkyl, substituted or unsubstituted cycloalkyl, substituted or unsubstituted heteroalkyl, substituted or unsubstituted heterocycloalkyl, substituted or unsubstituted aryl, or substituted or unsubstituted heteroaryl.

The term "aryl" means, unless otherwise stated, a polyunsaturated, aromatic, hydrocarbon substituent, which can be a single ring or multiple rings (preferably from 1 to 3 rings) that are fused together (i.e., a fused ring aryl) or linked covalently. A fused ring aryl refers to multiple rings fused together wherein at least one of the fused rings is an aryl ring. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. Thus, the term "heteroaryl" includes fused ring heteroaryl groups (i.e., multiple rings fused together wherein at least one of the fused rings is a heteroaromatic ring). A 5,6-fused ring heteroarylene refers to two rings fused together, wherein one ring has 5 members and the other ring has 6 members, and wherein at least one ring is a heteroaryl ring. Likewise, a 6,6-fused ring heteroarylene refers to two rings fused together, wherein one ring has 6 members and the other ring has 6 members, and wherein at least one ring is a heteroaryl ring. And a 6,5-fused ring heteroarylene refers to two rings fused together, wherein one ring has 6 members and the other ring has 5 members, and wherein at least one ring is a heteroaryl ring. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. An "arylene" and a "heteroarylene," alone or as part of another substituent, mean a divalent radical derived from an aryl and heteroaryl, respectively.

For brevity, the term "aryl" when used in combination with other terms (e.g., aryloxy, arylthioxy, arylalkyl) includes both aryl and heteroaryl rings as defined above. Thus, the term "arylalkyl" is meant to include those radicals in which an aryl group is attached to an alkyl group (e.g., benzyl, phenethyl, pyridylmethyl, and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like).

The term "oxo," as used herein, means an oxygen that is double bonded to a carbon atom.

The term "alkylsulfoxide" as used herein, means a moiety having the formula R-S(O)-R', where R and R' are alkyl groups as defined above. R and R' may have a specified number of carbons (e.g., "C₁-C₄ alkylsulfoxide").

Each of the above terms (e.g., "alkyl," "heteroalkyl," "aryl," and "heteroaryl") includes both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to, -OR', =O, =NR', =N-OR', -NR'R", -SR', -halogen, -SiR'R"R‴, -OC(O)R', -C(O)R', -CO₂R', -CONR'R", -OC(O)NR 'R", -NR"C(O)R', -NR'-C(O)NR"R‴, -NR"C(O)₂R', -NR-C(NR'R"R‴)=NR"", -NR-C(NR'R")=NR ‴, -S(O)R', -S(O)₂R', -S(O)₂NR'R", -NRSO₂R', -OS(O)₂NR'R", -NRS(O)₂NR'R", -CN, and -NO₂ in a number ranging from zero to (2m'+1), where m' is the total number of carbon atoms in such radical. R', R", R‴, and R"" each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy, or thioalkoxy groups, or arylalkyl groups. When a compound of the invention includes more than one R group, for example, each of the R groups is independently selected as are each R', R", R‴, and R"" group when more than one of these groups is present. When R' and R" are attached to the same nitrogen atom, they can be combined with the nitrogen atom to form a 4-, 5-, 6-, or 7-membered ring. For example, -NR'R" includes, but is not limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are varied and are selected from, for example: -OR', -NR'R", -SR', -halogen, -SiR'R"R‴, -OC(O)R', -C(O)R', -CO₂R', -CONR'R", -OC (O)NR'R", -NR"C(O)R', -NR'-C(O)NR"R‴, -NR"C(O)₂R', -NR-C(NR'R"R‴)=NR"", -NR-C(NR' R")=NR‴, -S(O)R', -S(O)₂R', -S(O)₂NR'R", -NRSO₂R', -OS(O)₂NR'R", - NRS(O)₂NR'R", -CN, -NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R‴, and R"" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl. When a compound of the invention includes more than one R group, for example, each of the R groups is independently selected as are each R', R", R‴, and R"" groups when more than one of these groups is present.

Two or more substituents may optionally be joined to form aryl, heteroaryl, cycloalkyl, or heterocycloalkyl groups. Such so-called ring-forming substituents are typically, though not necessarily, found attached to a cyclic base structure. In one embodiment, the ring-forming substituents are attached to adjacent members of the base structure. For example, two ring-forming substituents attached to adjacent members of a cyclic base structure create a fused ring structure. In another embodiment, the ring-forming substituents are attached to a single member of the base structure. For example, two ring-forming substituents attached to a single member of a cyclic base structure create a spirocyclic structure. In yet another embodiment, the ring-forming substituents are attached to non-adjacent members of the base structure.

Two of the substituents on adjacent atoms of the aryl or heteroaryl ring may optionally form a ring of the formula -T-C(O)-(CRR')_{q}-U-, wherein T and U are independently -NR-, -O-, -CRR'-, or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O) -, -S(O)₂-, -S(O)₂NR'-, -OS(O)₂NR'-, - NRS(O)₂NR'-, or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X'- (C"R‴)_{d}-, where s and d are independently integers of from 0 to 3, and X' is -O-, -NR'-, -S-, -S(O)-, -S(O)₂-, -OS(O)₂NR'-, -NRS(O)₂NR'-, or -S(O)₂NR'-. The substituents R, R', R", and R‴ are preferably independently selected from hydrogen, substituted or unsubstituted alkyl, substituted or unsubstituted cycloalkyl, substituted or unsubstituted heterocycloalkyl, substituted or unsubstituted aryl, and substituted or unsubstituted heteroaryl.

As used herein, the terms "heteroatom" or "ring heteroatom" are meant to include oxygen (O), nitrogen (N), sulfur (S), phosphorus (P), and silicon (Si).

A "substituent group," as used herein, means a group selected from the following moieties: (A) -OH, -NH₂, -SH, -CN, -CF₃, -NO₂, oxo, halogen, unsubstituted alkyl, unsubstituted heteroalkyl, unsubstituted cycloalkyl, unsubstituted heterocycloalkyl, unsubstituted aryl, unsubstituted heteroaryl, and (B) alkyl, heteroalkyl, cycloalkyl, heterocycloalkyl, aryl, and heteroaryl, substituted with at least one substituent selected from: (i) oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, unsubstituted heteroaryl, and (ii) alkyl, heteroalkyl, cycloalkyl, heterocycloalkyl, aryl, and heteroaryl, substituted with at least one substituent selected from: (a) oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, unsubstituted heteroaryl, and (b) alkyl, heteroalkyl, cycloalkyl, heterocycloalkyl, aryl, or heteroaryl, substituted with at least one substituent selected from: oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, and unsubstituted heteroaryl.

A "size-limited substituent" or " size-limited substituent group," as used herein, means a group selected from all of the substituents described above for a "substituent group," wherein each substituted or unsubstituted alkyl is a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₄-C₈ cycloalkyl, and each substituted or unsubstituted heterocycloalkyl is a substituted or unsubstituted 4 to 8 membered heterocycloalkyl.

A "lower substituent" or " lower substituent group," as used herein, means a group selected from all of the substituents described above for a "substituent group," wherein each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₅-C₇ cycloalkyl, and each substituted or unsubstituted heterocycloalkyl is a substituted or unsubstituted 5 to 7 membered heterocycloalkyl.

The term "pharmaceutically acceptable salts" is meant to include salts of the active compounds that are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, oxalic, methanesulfonic, and the like. Also included are salts of amino acids such as arginate, and salts of organic acids like glucuronic or galacturonic acids. Certain specific compounds of the present invention contain both basic and acidic functionalities that allow the compounds to be converted into either base or acid addition salts.

Thus, the compounds of the present invention may exist as salts, such as with pharmaceutically acceptable acids. The present invention includes such salts. Examples of such salts include hydrochlorides, hydrobromides, sulfates, methanesulfonates, nitrates, maleates, acetates, citrates, fumarates, tartrates (e.g., (+)-tartrates, (-)-tartrates, or mixtures thereof including racemic mixtures), succinates, benzoates, and salts with amino acids such as glutamic acid. These salts may be prepared by methods known to those skilled in the art.

The neutral forms of the compounds are preferably regenerated by contacting the salt with a base or acid and isolating the parent compound in the conventional manner. The parent form of the compound differs from the various salt forms in certain physical properties, such as solubility in polar solvents.

In addition to salt forms, the present invention provides compounds in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an *ex vivo* environment. For example, prodrugs can be slowly converted to the compounds of the present invention when placed in a transdermal patch reservoir with a suitable enzyme or chemical reagent.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centers) or double bonds; the racemates, diastereomers, tautomers, geometric isomers, and individual isomers are encompassed within the scope of the present invention. The compounds of the present invention do not include those that are known in the art to be too unstable to synthesize and/or isolate.

The compounds of the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I), carbon-13 (¹³C), or carbon-14 (¹⁴C). All isotopic variations of the compounds of the present invention, whether radioactive or not, are encompassed within the scope of the present invention.

Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

As used herein, an amino acid "derivative" includes an amino acid having substitutions or modifications by covalent attachment of a parent amino acid, such as, e.g., by alkylation, glycosylation, acetylation, phosphorylation, and the like. Further included within the definition of "derivative" is, for example, one or more analogs₁ of an amino acid with substituted linkages, as well as other modifications known in the art.

As used herein, a "natural amino acid" refers to arginine, glutamine, phenylalanine, tyrosine, tryptophan, lysine, glycine, alanine, histidine, serine, proline, glutamic acid, aspartic acid, threonine, cysteine, methionine, leucine, asparagine, isoleucine, and valine, unless otherwise indicated by context.

As used herein, a "protecting group" refers to a moiety that when attached to a reactive group in a molecule masks, reduces or prevents that reactivity. Representative hydroxy protecting groups include acyl groups, benzyl and trityl ethers, tetrahydropyranyl ethers, trialkylsilyl ethers and allyl ethers. Representative amino protecting groups include formyl, acetyl, trifluoroacetyl, benzyl, benzyloxycarbonyl (CBZ), tert-butoxycarbonyl (Boc), trimethyl silyl (TMS), 2-trimethylsilyl-ethanesulfonyl (SES), trityl and substituted trityl groups, allyloxycarbonyl, 9-fluorenylmethyloxycarbonyl (FMOC), nitro-veratryloxycarbonyl (NVOC), and the like. Examples of a "hydroxyl protecting group" include, but are not limited to, methoxymethyl ether, 2-methoxyethoxymethyl ether, tetrahydropyranyl ether, benzyl ether, p-methoxybenzyl ether, trimethylsilyl ether, triethylsilyl ether, triisopropyl silyl ether, t-butyldimethyl silyl ether, triphenylmethyl silyl ether, acetate ester, substituted acetate esters, pivaloate, benzoate, methanesulfonate and p-toluenesulfonate.

As used herein, a "leaving group" refers to a functional group that can be substituted by another functional group. Such leaving groups are well known in the art, and examples include, but are not limited to, a halide (e.g., chloride, bromide, and iodide), methanesulfonyl (mesyl), p-toluenesulfonyl (tosyl), trifluoromethylsulfonyl (triflate), and trifluoromethylsulfonate.

The phrase "pharmaceutically acceptable salt," as used herein, refers to pharmaceutically acceptable organic or inorganic salts of a compound (e.g., a Medicant Linker compound, or an Affinity Medicant Linker conjugate). The compound typically contains at least one amino group, and accordingly acid addition salts can be formed with this amino group. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and palmoate (i.e., 1,1'-methylene-bis-(2-hydroxy-3-naphthoate)) salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

As used herein, a "pharmaceutically acceptable solvate" or "solvate" refer to an association of one or more solvent molecules and a compound of the invention, e.g., an Affinity Medicant Linker conjugate or a Medicant Linker compound. Examples of solvents that form pharmaceutically acceptable solvates include, but are not limited to, water, isopropanol, ethanol, methanol, DMSO, ethyl acetate, acetic acid, and ethanolamine.

The following abbreviations are used herein and have the indicated definitions: Boc is N-(t-butoxycarbonyl), cit is citrulline, dap is dolaproine, DCM is dichloromethane, DIEA is N,N-diisopropylethylamine, dil is dolaisoleuine, DMF is N,N-dimethylformamide, DMSO is dimethylsulfoxide, doe is dolaphenine, dov is N,N-dimethylvaline, DTNB is 5,5'-dithiobis(2-nitrobenzoic acid), DTPA is diethylenetriaminepentaacetic acid, DTT is dithiothreitol, Fmoc is N-(9-fluorenylmethoxycarbonyl), gly is glycine, HATU is O-(7-azabenzotriazol-1-yl)-N,N,N',N'-tetramethyluronium hexafluorophosphate, HBTU is 2-[1H-benzotriazole-1-yl]-1,1,3,3-tetramethylaminium hexafluorophosphate; HOBt is 1-hydroxybenzotriazole, HPLC is high pressure liquid chromatography, ile is isoleucine, lys is lysine, MeOH is methanol, MeVal is N-methyl-valine, PAB is p-aminobenzyl, PBS is phosphate-buffered saline (pH 7.4), Ph is phenyl, phe is L-phenylalanine, PyBrop is bromo tris-pyrrolidino phosphonium hexafluorophosphate, TFA is trifluoroacetic acid, UV is ultraviolet, and val is valine.

The following LU abbreviations are used herein and have the indicated definitions: Val Cit or vc is a valine-citrulline dipeptide site in protease cleavable linker; PABC is p-aminobenzylcarbamoyl; (Me)vc is N-methyl-valine citrulline, where the linker peptide bond has been modified to prevent its cleavage by cathepsin B; and MC(PEG)₆-OH is maleimidocaproyl-polyethylene glycol.

As used herein, a "pegylated compound" refers to a compound conjugated with two or more polyethylene glycol moieties or two or more polypropylene glycol moieties or a combination thereof.

As used herein, a "pro-peptide" includes pro-peptide, pre-peptide, pro-protein and pre-protein amino acid sequences including those amino acid sequences cleaved by enzymes disclosed in **Table III**.

Malignant neoplasia is the second most common cause of death in the United States behind cardiovascular disease. Chemotherapy has exerted a predominant role in increasing life spans for patients with a variety of tumors including Burkitt's lymphoma, acute lymphocytic leukemia and Hodgkin's disease. Further, new cancer chemotherapeutic agents and methods of care combined with early detection and treatment have resulted in decreases in the overall incidence of cancer and decreases in the death rates from all cancers combined. Responsive tumors represent only a small fraction of the various types of cancer. Further, agents such as cyclophosphamide, adriamycin, 5-fluorouracil and hexamethylmelamine, which are highly active against clinical solid tumors, are limited. Thus, patients with many types of malignancies remain at significant risk for relapse and mortality. After relapse, some patients can be re-induced into remission with their initial treatment regimen. However, higher doses of the initial chemotherapeutic agent or the use of additional agents are frequently required, indicating the development of at least partial medicant resistance. Evidence indicates medicant resistance can develop simultaneously to several agents, including medicant resistance to treatments to which the patient was not exposed. The development of multiple-medicant resistant tumors may be a function of tumor mass and constitutes a major cause of treatment failure. To overcome this medicant resistance, high-dose chemotherapy with or without radiation and allogenic or autologous bone marrow transplantation can be employed. The high-dose chemotherapy may employ the original medicant(s) or be altered to include additional agents. As a result, there remain many cancer patients for whom no or minimally effective therapy exists. Accordingly, there is a need for the development of novel chemotherapeutics with greater efficacy or safety, either as monotherapy or in combination with other chemotherapeutic agents, and such agents with the potential to overcome medicant resistance in cancer cells.

Illudins are toxic natural products produced by mushrooms of the genus Omphalotus. Syn-Illudins are semi-synthetic derivatives of Illudins. Acylfulvenes are also semi-synthetic derivatives of Illudins. Syn-Illudins and Acylfulvenes have each been chemically modified at select sites to allow their use as medicants. The modifications in the Syn-Illudins do not alter any of the cyclic rings (cyclopropane, cyclopentane, cyclohexane) of the basic Illudin chemical structure. The modifications of Acylfulvenes differ from Syn-Illudins in that an additional double bond (an unsaturated bond) has been created in the 5 membered (cyclopentane) ring.

Illudins function as alkylating agents that damage DNA and thereby block transcription. The blockage can be repaired through nucleotide excision. The toxicity of the illudins has prevented any applications in human tumor therapy. Acylfulvenes have been developed which exhibit promising antitumor activity with a better safety profile, as described in U.S. Patent Nos. 5,439,936; 5,523490 and 6,380,403 which are each herein expressly incorporated by reference in their entireties. (6'*R*)-6'-hydroxy-3'-(hydroxymethyl)-2',4',6'-tiimethylspiro[cyclopropane-1,5'-inden]-7'(6'*H*)-one **(analog 002, Table V)** is an illudofulvene analog of illudin S which has demonstrated clinical activity with an acceptable safety profile in hormone-refractory prostate cancer. Most relevant to clinical applications, irofulven activity is independent of common resistance mechanisms such as the multi-medicant resistance phenotype, anti-apoptotic B-cell lymphoma 2 (Bcl-2) (SEQ. ID. 056) over expression, as well as tumor protein 53 (p53) (SEQ. ID. 057) and cyclin dependent kinase inhibitor 1 (p21 / WAF1) (SEQ. ID. 058) mutations.

Growth factors, including peptides and proteins are critical mediators of a wide range of cell-cell communication. They are important endocrine, paracrine and autocrine messengers. Growth factors function as neurotransmitters and neuromodulators, regulate chemotaxis, immune function, development, cell growth, and can influence tumor cells. The receptors that recognize growth factors are highly selective and define specific cell populations. As a result, growth factor receptors are a large and important class of medicant (including drug) targets. In addition to physiologic noncancerous cell populations, these receptors can also be expressed in various cancer cell populations.

A polypeptide is a long, continuous, and unbranched chain of amino acids. A glycol-peptide is a peptide that contains one or more carbohydrate moieties covalently attached to the side chains of specific amino acids. A pro-peptide, is an inactive peptide that can be turned into an active form through a post translational modification that enzymatically cleaves the pro-peptide. Examples include pro-insulin (SEQ. ID. 059) and pro-opiomelanocortin (SEQ. ID. 060). Enzymatically cleaving the pro-peptide, allows for the peptide to be available on short notice and/or in large quantities. Some pro-peptides are secreted from the cell. Many of these are synthesized with an N-terminal signal peptide that targets the pro-peptide for secretion.

Cytokines are small proteins (approximately 5 to 20 kDa) that affect the behavior of other cells, and sometimes the releasing cell itself and are thereby important in cell signaling. Many specific cytokines can be released by a variety of different kinds of cells, e.g., macrophages, B lymphocytes, T lymphocytes, mast cells, endothelial cells, fibroblasts, and various stromal cells. Cytokines act through specific receptors, and are important in the humoral and cell-based immune responses. Cytokines also regulate the maturation, growth, and responsiveness of specific cell populations. Cytokines circulate in much higher concentrations than hormones and in contrast with hormones are made by a variety of different kinds of cells. Cytokines are important in host responses to infection, immune responses, inflammation, trauma, sepsis, cancer, and reproduction. As a result, cytokine receptors are upregulated in many forms of cancers.

A steroid is an organic compound that contains four cycloalkane rings joined to each other. Examples of steroids include the dietary lipid cholesterol and the sex hormones estradiol and testosterone. The core of a steroid molecule is composed of seventeen carbon atoms bonded together that take the form of four fused rings: three six-carbon atom rings and one five-carbon atom ring. A variety of functional groups can be attached to the four-ring core. Steroids can also vary depending on the oxidation state of the rings. A steroid hormone is a steroid that acts as a hormone. Steroid hormones can be grouped into five groups (glucocorticoids, mineralocorticoids, androgens, estrogens, and progesterones) based on the receptors to which they bind. Steroid hormones, particularly androgens, are essential not only for growth and development but also in the progression of many forms of cancer. As a result, steroid hormone receptors are upregulated in many forms of cancers.

The retinoic acid receptor (RAR) is a nuclear receptor which can also act as a transcription factor. The RAR can be activated by either all-trans retinoic acid or 9-cis retinoic acid. There are three RAR isoforms (alpha (SEQ. ID. 061), beta (SEQ. ID. 062), and gamma (SEQ. ID. 063)), each encoded by separate genes, where splice variants generate still further diversity in the expressed receptor. The retinoid X receptor (RXR) is a nuclear receptor activated by 9-cis retinoic acid. There are also three RXR isoforms (alpha (SEQ. ID. 064), beta (SEQ. ID. 065), and gamma (SEQ. ID. 066)), each encoded by separate genes. RXR hetero-dimerizes with subfamily 1 nuclear receptors including RAR. In the absence of ligand, the RAR/RXR dimer binds to retinoic acid response elements complexes with a co-repressor protein. Binding of agonist ligands to RAR results in dissociation of the co-repressor and recruitment of a coactivator protein that, in turn, promotes transcription of the downstream target gene into mRNA and thereby protein or other RNA signaling mechanisms.

Lipid metabolism is altered in many forms of cancer, including upregulation of de novo lipid synthesis. Cancer cells can also use alternative enzymes and pathways to facilitate the production of fatty acids. These newly synthesized lipids may support a number of cellular processes to promote cancer cell proliferation and survival. Elaidic acid or (E)-octadec-9-enoic acid is the trans isomer of oleic acid and is found in small quantities in caprine milk, bovine milk and some meats. It increases Cholesteryl Ester Transfer Protein (CETP) (SEQ. ID. 067) activity, which in turn raises levels of very low density lipoprotein and lowers levels of high density lipoprotein (HDL) cholesterol. CETP is found in plasma, where it is involved in the transfer of cholesteryl ester from HDL to other lipoproteins. Defects in the CETP gene are a cause of hyperalphalipoproteinemia 1.

An antibody is a protein made up of four peptide chains disulfide linked together to form a "Y"-shape. Antibodies are produced by plasma cells and are used by the immune system to identify and neutralize foreign antigens such as bacteria and viruses. The antibody recognizes a unique part of the antigen using each FAB portion of the protein (i.e., the tip of the "Y" portion of the antibody), allowing a specific high affinity binding interaction to occur. The binding interaction of different antibodies can target specific antigen epitopes. An antibody fragment containing one or both FAB portions can also target specific antigen epitopes.

The ability of the Illudofulvene analogs to inhibit tumor cell growth is shown in **Table VII** and **Table VIII. Table VII** shows the ability of Illudofulvene analogs to inhibit tumor cell growth. **Table VIII** shows screening data with the National Cancer Institute (NCI) Developmental Therapeutics Program (DTP) NCI 60 cell line screen assay comparing conventional anti cancer drugs Epothilone A, MMA,DM1 with the previously reported illudofulvene analogs (**analog 002, analog 142, analog 159, analog 176)** and with the illudofulvene analogs **(analog 334** (FIG. 1AE), **analog 362** (FIG. 1AU), **analog 371** (FIG. 1BC), **analog 383** (FIG. IBM), and **analog 394** (FIG. 1AE)). In **Table VIII,** 'Cytotoxicity' is defined as the ability to actually kill tumor cells was detected versus only inhibiting growth of tumor cells, while 'MDR activity' is the ability to equally kill a drug resistant daughter cell as compared to the non-drug resistant parent cell. The conventional anticancer drugs, Epothilone A, MMA and DM1 were able to inhibit the growth of cells in the NCI 60 cell line screen but were not able to actually kill timor cells. That is they were not cytotoxic. Further, they were not MDR active. All of the illudofulvene analogs in **Table VIII (analog 334, analog 362, analog 371, analog 383 and analog 394)** were cytotoxic in the NCI 60 cell line assay. The illudofulvene **analog 371** is the most potent compound at lysing the NCI 60 tumor cell line. The MV522 cell line is a lung-derived adenocarcinoma cell line. In various embodiments of the invention, the MV522 cell line represents a "target" cell line. That is an illudofulvene analog that exhibits toxicity against this solid tumor cell line shows a desirable result. The 8392B cell line represents a hematopoietic (non-solid) cell line. In various embodiments of the invention, the 8392B cell line is considered a "nontarget" cell line. The two hour toxicity data represents the concentration of a given analog for which a two hour exposure will inhibit 50% of the DNA synthesis activity in a given cell line. The 48 hour exposure data represents the concentration at which a given analog with a 48 hour exposure will inhibit the growth or viability in a given cell line as defined by the standard Trypan Blue Exclusion assay. As an example, **analog 002** will inhibit the target MV522 cell line at 110 nM with only a 2 hour exposure but has no inhibitory effect on the nontarget 8392B cell line at 26,000 nM (26 µM). Analog **002** with a prolonged exposure period (e.g. 48 hours) can eventually inhibit the nontarget cell line. In contrast, Analog **201** will inhibit the target MV522 cell line with only a 2 hour exposure (IC50 = 360 nM) but has minimal effect on the 8392B cell nontarget line with even a 48 hour exposure (IC50 = 26,000 nM) indicating superior anticancer activity as a monotherapeutic agent. In contrast to these two analogs, **analog 224** displayed minimal toxicity as well as no differential toxicity between the target and nontarget cell line indicating it would have minimal properties as a monotherapeutic anticancer agent.

As used herein, a "growth factor" or an "anti-angiogenic protein" includes Adrenomedullin (SEQ. ID. 068), Angiopoietin (Ang) (SEQ. ID. 069, 106, 111, and 145), Autocrine motility factor (SEQ. ID. 070), Bone morphogenetic proteins (BMPs) (SEQ. ID. 071), Brain-derived neurotrophic factor (BDNF) (SEQ. ID. 072), Endostatin (SEQ. ID. 073), Endostar (SEQ. ID. 074), Epidermal growth factor (EGF) (SEQ. ID. 075), Erythropoietin (EPO) (SEQ. ID. 076), Fibroblast growth factor (FGF) (SEQ. ID. 077), Glial cell line-derived neurotrophic factor (GDNF) (SEQ. ID. 078), Granulocyte colony-stimulating factor (G-CSF) (SEQ. ID. 079), Granulocyte macrophage colony-stimulating factor (GM-CSF) (SEQ. ID. 080), Growth differentiation factor-9 (GDF9) (SEQ. ID. 081), Hepatocyte growth factor (HGF) (SEQ. ID. 082), Hepatoma-derived growth factor (HDGF) (SEQ. ID. 083), Insulin-like growth factor (IGF) (SEQ. ID. 084), Migration-stimulating factor (SEQ. ID. 085), Myostatin (GDF-8) (SEQ. ID. 086), Nerve growth factor (NGF) (SEQ. ID. 087) and other neurotrophins (SEQ. ID. 144), Platelet-derived growth factor (PDGF A) (SEQ. ID. 088), PDGF B (SEQ. ID 168), PDGF C (SEQ. ID. 036), PDGF D (SEQ. ID. 037), Thrombopoietin (TPO) (SEQ. ID. 089), Transforming growth factor alpha(TGF-α) (SEQ. ID. 090), Transforming growth factor beta(TGF-β) (SEQ. ID. 091), Tumor necrosis factor-alpha(TNF-a) (SEQ. ID. 092), Vascular endothelial growth factor (VEGF) (SEQ. ID. 093), and placental growth factor (P1GF) (SEQ. ID. 094).

As used herein, a "protein toxin" includes ricin A chain (SEQ. ID. 095), ricin B chain (SEQ. ID. 096), diphtheria toxin (SEQ. ID. 097), Pseudomonas aeurginosa exotoxin A (SEQ. ID. 098), r-gelonin (SEQ. ID. 099), saporin (SEQ. ID. 100), glycosylated protein toxins, deglcosylated protein toxins and protein toxin fragments which includes deglycosylated ricin A, deglycosylated ricin B, Pseudomonas aeurginosa exotoxin A PE40 fragment (SEQ. ID. 101) and Pseudomonas aeurginosa exotoxin A PE38 fragment (SEQ. ID. 102).

As used herein, a "steroid" includes cholesterol (5-cholesten-3beta-ol), pregnenolone (3beta-hydroxy-5-pregnen-20-one), 17-hydroxyprenenolone (3-beta,17-dihydroxy-5-pregnen-20-one), progesterone (4-pregnene-3,20-dione), 17-hydroxyprogesterone (17-hydroxy-4-pregnene-3,20-dione), androstenedione (4-androstene-3,17-dione), 4-hydroxyandrostenedione (4-hydroxy-4-androstene-3,17-dione), 11-beta-hydroxyandostenedione (11beta-4-androstene-3,17-dione), androstanediol (3-beta,17-beta-Androstanediol), androsterone (3-alpha-hydroxy-5alpha-androstan-17-one), epiandrosterone (3-beta-hydroxy-5alpha-androstan-17-one ), adrenosterone (4-androstene-3,11,17-trione), dehydroepiandrosterone (3beta-hydroxy-5-androsten-17-one), dehydroepiandrosterone sulfate (3-beta-sulfooxy-5-androsten-17-one), testosterone (17beta-hydroxy-4-androsten-3-one ), epitestosterone (17-alpha-hydroxy-4-androsten-3-one), 5-alpha-dihydrotesterone (17-beta-hydroxy-5alpha-androstan-3-one), 5-beta-dihydrotestosterone (17-beta-hydroxy-5beta-androstan-3-one), 11-beta-hydroxytesosterone (11-beta,17beta-dihydroxy-4-androsten-3-one), 11-ketotesosterone (17-beta-hydroxy-4-androsten-3,17-dione), estrogen (including: estrone (3-hydroxy-1,3,5(10)-estratrien-17-one), estradiol (1,3,5(10)-estratriene-3,17beta-diol), and estriol (1,3,5(10)-estratriene-3,16alpha,17beta-triol)), corticosterone (11-beta,21-dihydroxy-4-pregnene-3,20-dione), deoxycorticosterone (21-hydroxy-4-pregnene-3,20-dione), cortisol (11-beta,17,21-trihydroxy-4-pregnene-3,20-dione), 11-deoxycortisol (17,21-dihydroxy-4-pregnene-3,20-dione), cortisone (17,21-dihydroxy-4-pregnene-3,11,20-trione), 18-hydroxycorticosterone (11-beta,18,21-trihydroxy-4-pregnene-3,20-dione), 1-alpha-hydroxycorticosterone (1-alpha,11-beta,21-trihydroxy-4-pregnene-3,20-dione), and aldosterone (18,11-hemiacetal of 11beta,21-dihydroxy-3,20-dioxo-4-pregnen-18-al).

As used herein, a "Specific Binding Peptide" includes an "anti-angiogenic peptide" (SEQ. ID. 146) and an "integrin binding peptide" (SEQ. ID. 147). A "Specific Binding Peptide" includes integrin binding peptide RGD4C = CDCRGDFC (SEQ. ID. 147), integrin binding peptide RGD10 (SEQ. ID. 148), c(RGDyK) (SEQ. ID. 149), integrin binding peptide c(RGDfK) (SEQ. ID. 150), integrin binding peptide [c(RGDyK)]2 (SEQ. ID. 151), integrin binding peptide CAGKNFFWKTFTSC (SEQ. ID. 152), cilengitide (cyclic RGD pentapeptide) (SEQ. ID. 153), ATN-161 (peptide antagonist of integrin alpha5beta1) (SEQ. ID. 154), ATN-454 (Ac-PHSCN-NH₂) (peptide antagonist of integrin alpha5beta1) (SEQ. ID. 155), tumstatin T7 peptide TMPFLFCNVNDVCNFASRNDYSYWL (SEQ. ID. 156), tumstatin sequence 1 YSNS (SEQ. ID. 157), tumstatin sequence 2 YSNSG (SEQ. ID. 158), endostatin motif FLSSRLQDLYSIVRRADRAA (SEQ. ID. 159), endostatin motif IVRRADRAAVP (SEQ. ID. 160), laminin peptide A13 (RQVFQVAYIIIKA) (SEQ. ID. 161), laminin peptide C16 (KAFDITYVRLKF) (SEQ. ID. 162), laminin peptide C16S (DFKLFAVTIKYR) (SEQ. ID. 163), and VEGFR1 peptide (CPQPRPLC) (SEQ. ID. 164).

As used herein, a traditional linker includes linkers that can be formed from those reagents disclosed in Tables IA-ID, IIA-IID, IIIA-IIIC, IVA-IVC, VA-VB, and VIA-VID of U.S. Patent No. 9,381,178.

As used herein, a "FSB linker" includes those linkers selected from the group consisting of 4-fluorosulfonyl benzoyl, 3-fluorosulfonyl benzoyl and 2-fluorosulfonyl benzoyl as depicted in FIG. 15 of U.S. Patent No. 9,381,178.

As used herein, a "Mall" linker includes a malonic linker and a maleimide linker covalently attached to an illudofulvene analog.

As used herein, a "protease" includes those enzymes disclosed in **Table III.**

As used herein, a "cytokine" includes chemokines, interferons, interleukins, lymphokines, tumor necrosis factor, neutrophil activating protein-2, monocyte chemotactic protein-1 and the like.

Despite recent advances in therapy, many patients with cancer invariably relapse and require additional treatments. Most of these patient's cancers become refractory to standard chemotherapy and/or radiation treatment regimens. The prognosis for these patients is poor and long term survival rates for metastatic solid tumor cancers remain very low. Thus, there is a need for the development of novel agents and treatment regimens that specifically target these recurring tumor cells and also produce less systemic toxicity. Target therapies, such as monoclonal antibodies, now provide a promising alternative to the conventional cytotoxic chemotherapy approach.

Monoclonal antibody based therapy has recently achieved considerable success in oncology and there are currently nine monoclonal antibodies (without a medicant attached) approved by the FDA as cancer therapeutics. As an example, HERCEPTIN^{®} and RITUXAN^{®} (both produced by Genentech, South San Francisco, California), are used to successfully treat breast cancer and non-Hodgkin's lymphoma, respectively. HERCEPTIN^{®} is a recombinant DNA-derived humanized monoclonal antibody selectively binding to the extracellular domain of the Human Epidermal growth factor Receptor 2 (HER2) proto-oncogene whereas RITUXAN^{®} is a genetically engineered chimeric murine/human monoclonal antibody directed against the CD20 antigen overexpressed on the surface of normal and malignant B lymphocytes.

Recent clinical evidence indicates that while the monoclonal antibody based therapies are effective at inducing remission, they do not always produce a complete cure, and relapses eventually occur in most patients. There is now a tremendous interest in the use of antibody medicant conjugates as a class of therapeutics that utilize the antigen-selectivity of monoclonal antibodies to deliver potent cytotoxic medicants to specific tumor cells. Antibody medicant conjugates are produced by attaching a cytotoxic agent to an antibody that binds specifically to a tumor-associated antigen.

In theory, antibody medicant conjugates can confer an increased therapeutic index to highly potent medicants by improving therapeutic efficacy and reducing systemic toxicity (by minimizing damage to normal tissues), although this goal has been elusive in achieving. The basis for the efficacy of antibody medicant conjugates is that they target tumor cells that preferentially express an antigen that is recognized by the associated antibody. In contrast, non-tumor cells either fail to express this antigen, or express the antigen at a very low level. In theory, only the tumor cells expressing the associated antibody are recognized and destroyed by the AMC, and other cells are left untouched and undamaged.

While different medicant classes have been tried for delivery via antibodies, only a few have proved efficacious for use as antibody medicant conjugates. The two main medicant classes used to date to produce antibody medicant conjugates are the auristatins (MMAE/N-methylvaline-valine-dolaisoleuine-dolaproine-norephedrine or MMAF/N-methylvaline-valine-dolaisoleuine-dolaproine-phenylalanine) and the maytansines (DM1 or DM4). Currently only two antibody medicant conjugates are approved by the U.S.F.D.A. and marketed; brentuximab vedotin (auristatin based) and ado-trastuzumab emtansine (maytansine based).

Illudofulvenes have several unique properties over agents traditional used to make medicants and/or antibody drug conjugates (ADCs). Firstly, these are the only agents known to function by inhibition of the DNA transcription-coupled repair pathway. No other toxin, drug or medicant inhibits this pathway. The result is that illudofulvenes are true cytotoxic agents whereas other agents traditionally used to produce ADCs (pyrrolobenzodiazepines, maytansines, fumagillols, dolstatins, auristatins, enadiynes, halichondrins, and tubulysins) are only cytostatic. In the NCI-DTP 60 cell line panel these other agents were capable of inhibiting tumor cell growth (IC₅₀ value), had some ability to block tumor cell growth (TGI value) but none were capable of actually causing tumor cell death or cytotoxicity **(Table VI).** The illudin derivatives, however, are capable of killing tumor cells at nanomolar concentrations **(Table VI).** This means that while ADCs developed using other toxins can stall tumor cell growth, they cannot actually kill the tumor cell. Once the effect of the drug has worn off the tumor cells will again grow and kill the patient. In contrast, the illudofulvenes actually kill the tumor cell with as little as a 2 hour exposure. Secondly, whereas tumor cells will undergo apoptosis or cell death with hours once the DNA transcription-coupled repair pathway is blocked, normal diploid non-tumor cells can survive for hours. This translates into a wide therapeutic window for ADCs developed with illudofulvenes. The two ADC agents currently FDA approved for administration deliver a dose of the associated toxin that is 300% higher than a lethal dose which is why these agents have severe systemic toxicity. In contrast, the comparable ADC developed with illudofulvenes will deliver a dose of the associated toxin that is 40% of a known non-toxic dose (estimated at 28% of a toxic dose and only 12% of a lethal dose). Thus, ADCS developed with illudofulvenes will have minimal systemic toxicity as compared to current agents. Thirdly, these agents are stable down to a pH of 2.0. An ADC is engulfed by a tumor cell, transported to the endosomes (pH < 6.0) and then into the lysozomes (pH < 4). Many agents used for ADCs will degrade in these low pH environment, whereas illudofulvenes are stable. 4) Cancer cells can become resistant to various toxins and drugs through the development of what is termed multi-drug resistance. This process is known to occur through several different mechanisms. Whereas other toxins and drugs are substrates for the most common MDR mechanisms (MDR1/gp170 and MRP/gp180), and cancer cells can become resistant to these agents, the illudofulvenes remain active against all MDR phenotypes regardless of the mechanism (see **Table IV).** Hence, if tumor cells have already developed multi-drug resistance prior to ADC with a conventional toxin, or during the administration of a course of the ADC, the ADC will have no efficacy. In contrast, ADCs developed with illudofulvenes will continue to kill cancer cells.

The present invention is based on the discovery that illudofulvene are active as medicant delivery agents in vitro and in vivo and can be conjugated directly to a linker, via a variety of peptide or non-peptide bonds, and are active as medicant delivery agents in vitro and in vivo. Similar to other medicant classes used to produce antibody medicant conjugates, the illudofulvenes can be conjugated to a linker that allows subsequent coupling to a monoclonal antibody. Unlike previous medicant classes such as the auristatins (MMAE, MMAF, dolstatin-10), the maytansines (DM1 or DM4), the irinotecans and their metabolites (SN38), the calicheamicins (17-DMAG), the pyrrolobenzodiazepines (SJG-136), the duocarmycins (CC-1065), many of the illudofulvenes compounds do not require a linker and can be directly attached to a monoclonal antibody or fragment thereof by a variety of simple chemical reactions. In this sense, the lack of requirement for a linker or a spacer, the illudofulvenes compounds are unique. They will directly form covalent bonds with reactive groups on an AM such as a monoclonal antibody. In addition, because of their very small size and extreme cytotoxicity the illudofulvenes can be coupled directly to very small molecular weight entities (or affinity moieties) that allow tumor specific cytotoxicity without the concomitant requirement of use of a monoclonal antibody. Examples include the ability to link illudofulvenes directly to steroids which allow the medicant-affinity complex to kill cells overexpressing a specific steroid receptor (such as estrogen- or progesterone-positive breast cancer cells) or even to be chemically coupled to various lipids. The small size and extreme cytotoxicity illudofulvenes allows direct coupling to peptides which can preferentially bind to tumor cells (integrin binding peptides) or display anti-angiogenic properties to hinder tumor invasion. The illudofulvenes can also be coupled to specific peptides which actually renders the medicant-affinity complex non-toxic until the peptide is cleaved by a protease secreted by tumor cells. An example includes PSA (prostate specific antigen) secreted by prostate adenocarcinoma cells. Again, unlike previous medicant classes such as the auristatins (MMAE, MMAF, dolstatin-10), the maytansines (DM1 or DM4), the irinotecans and their metabolites (SN38), the calicheamicins (17-DMAG), the pyrrolobenzodiazepines (SJG-136), the duocarmycins (CC-1065), the illudofulvenes compounds do not require a linker and can be directly attached to a steroid or a peptide that will subsequently function as an AM and direct the associated complex to specific tumor cells. In an embodiment of the invention, an illudofulvenes is attached to either a Specific Binding Peptide or a peptide which when cleaved by a specific protease (see **Table III)** such as PSA generates an entity which is cytotoxic (see **Table II).**

Trastuzumab emtansine (Genentech for Breast cancer) uses maytanasine derive DM-1, a stable non-cleavable linker. Brentuximab vedotin (Seattle Genetics/Takeda for Hodgkin's Lymphoma) uses auristatin MMAE to anti-CD30, an enzyme sensitive cleavable linker.

The malonic linker, maleimide linker and SMCC [succinimidyl 4-(N-maleimidomethyl)-cyclohexane-1-carboxylate] linker can form active intermediates that react with sulfhydryl groups on an antibody. SMCC has been used to bind maytansine derivative DM1 to the monoclonal antibody Herceptin. The AMC was internalized where the Herceptin was degraded by proteases and DM1 was released into the cytosol. Further, Sulfo-SMCC [sulfosuccinimidyloxycarbonyl-a-methyl-a-(2-pyridyldithio)toluene] forms an active intermediate that reacts with sulfhydryl groups on an antibody. The resulting Sulfo-SMCC AMC is more water soluble than the SMCC AMC.

Compounds and Conjugates. The present invention is drawn to a series of compounds and conjugates containing a Medicant moiety (M) linked via its C terminus to a LU (LU). The LU can operate to provide a suitable release of M.

In one group of embodiments, the invention provides Medicant Linker compounds having Formula I: LU-M (I) or a pharmaceutically acceptable salt or solvate thereof where the medicant loading is represented by p, the average number of medicant molecules per affinity (e.g., an antibody) (e.g. of Formula II, IIa, IIa'). Medicant loading may range from 1 to 20 Medicant units (M) per Affinity unit (e.g., Ab or in Ab). Compositions of Formula IIa and Formula IIa' include mixtures of antibodies conjugated with a range of medicants, from 1 to 20.

In some embodiments, p is from about 1 to about 8 Medicant units per Affinity unit. In some embodiments, p is 1. In some embodiments, p is from about 2 to about 8 Medicant units per Affinity unit. In some embodiments, p is from about 2 to about 6, 2 to about 5, or 2 to about 4 Medicant units per LU. In some embodiments, p is about 2, about 4, about 6 or about 8 Medicant units per Affinity unit.

The average number of Medicants units per Affinity unit in a preparation from a conjugation reaction may be characterized by conventional means such as mass spectroscopy, ELISA assay, and HPLC. The quantitative distribution of Affinity Medicant Linker conjugates in terms of p may also be determined. In some instances, separation, purification, and characterization of homogeneous Affinity Medicant Linker conjugates, where p is a certain value from Affinity Medicant Linker conjugates with other medicant loadings may be achieved by means such as reverse phase HPLC or electrophoresis.

Returning to Formula IIa', the conjugates comprise an antibody covalently attached to one or more Medicant units (moieties) via a LU: A, a, W and w are as described above. The antibody medicant conjugate include pharmaceutically acceptable salts or solvates thereof.

The medicant loading is represented by p, the average number of Medicant units per antibody in a molecule of Formula II. Medicant loading may range from 1 to 20 medicants (M) per Ab or mAb. Compositions of the AMC of Formula IIa' include mixtures of antibodies conjugated with a range of medicants, from 1 to 20. In some embodiments, p is from about 1 to about 8 Medicant units per antibody. In some embodiments, p is 1. In some embodiments, p is from about 2 to about 8 Medicant units per antibody. In some embodiments, p is from about 2 to about 6, 2 to about 5, or 2 to about 4 Medicant units per antibody. In some embodiments, p is about 2, about 4, about 6 or about 8 Medicant units per antibody.

The average number of medicants per antibody in preparations of AMCs from conjugation reactions may be characterized by conventional means such as UV/visible spectroscopy, mass spectrometry, ELISA assay, and HPLC. The quantitative distribution of AMCs in terms of p may also be determined. In some instances, separation, purification, and characterization of homogeneous AMCs where p is a certain value from AMC with other medicant loadings may be achieved by means such as reverse phase HPLC or electrophoresis.

For some antibody medicant conjugates, p may be limited by the number of attachment sites on the antibody. For example, where the attachment is a cysteine thiol, an antibody may have only one or several cysteine thiol groups, or may have only one or several sufficiently reactive thiol groups through which a LU may be attached. In some embodiments, the cysteine thiol is a thiol group of a cysteine residue that forms an interchain disulfide bond. In some embodiments, the cysteine thiol is a thiol group of a cysteine residue that does not form an interchain disulfide bond.

Typically, less than the theoretical maximums of medicant moieties are conjugated to an antibody during a conjugation reaction. An antibody may contain, for example, many lysine residues that do not react with the Medicant Linker compound intermediate or LU reagent. Only the most reactive lysine groups may react with an amine-reactive LU reagent. Generally, antibodies do not contain many, if any, free and reactive cysteine thiol groups which may be linked to a Medicant moiety via a LU. Most cysteine thiol residues in the antibodies exist as disulfide bridges and must be reduced with a reducing agent such as dithiothreitol (DTT). The antibody may be subjected to denaturing conditions to reveal reactive nucleophilic groups such as lysine or cysteine. The loading (medicant/ antibody ratio) of an AMC may be controlled in several different manners, including: (i) limiting the molar excess of Medicant Linker compound intermediate or LU reagent relative to antibody, (ii) limiting the conjugation reaction time or temperature, and (iii) partial or limiting reductive conditions for cysteine thiol modification.

Where more than one nucleophilic group reacts with a Medicant Linker compound intermediate, or LU reagent followed by Medicant moiety reagent, then the resulting product is a mixture of Affinity Medicant Linker Conjugates (e.g., AMCs) with a distribution of one or more Medicant moieties per Affinity unit (e.g., an antibody). The average number of medicants per Affinity unit (e.g., antibody) may be calculated from the mixture by, for example, dual enzyme linked immune serum assay (ELISA) antibody assay, specific for antibody and specific for the medicant. Individual Affinity Medicant Linker Conjugate molecules may be identified in the mixture by mass spectroscopy, and separated by high performance liquid chromatography (HPLC), e.g., hydrophobic interaction chromatography. Thus, a homogeneous conjugate with a single loading value may be isolated from the conjugation mixture by electrophoresis or chromatography.

A "Linker Unit" (LU) is a bifunctional compound which can be used to link a Medicant unit and/or an Affinity unit to form an Affinity Medicant Linker conjugate. Such conjugates are useful, for example, in the formation of immuno conjugates directed against tumor associated antigens. Such conjugates allow the selective delivery of cytotoxic drugs to tumor cells. A LU includes a traditional linker, a 4-fluorosulfonyl benzoyl (4-FSB) linker, a 3-fluorosulfonyl benzoyl (3-FSB) linker a 2-fluorosulfonyl benzoyl (2-FSB) linker, a maleimide (Mall) linker, an azlactone linker and a bridging amino acid.

A traditional linker is as defined in U.S. Patent No. 9,381,178. A Stretcher Unit includes two or more Linker Units.

A bridging amino acid means -NH-C(R')H-CO- or -N(R")-C(R')H-CO- including glycine, L-alanine, L-serine, L-threonine, L-cysteine, L-valine, L-leucine, L-isoleucine, L-methionine, L-proline, L-phenylalanine, L-tyrosine, L-tryptophan, L-aspartic acid, L-glutamic acid, L-asparagine, L-glutamine, L-histidine, L-lysine, L-arginine, L-homocysteine, L-selenocysteine, L-pyrrolysine, L-carnitine, L-hypusine, 2-aminoisobutyric acid, dehydroalanine, L-gamma-aminobutyric acid, L-ornithine, L-citrulline, L-α-Amino-n-butyric acid, L-Norvaline, L-Norleucine, L-Pipecolic acid, L-Alloisoleucine, L-α,β-diaminopropionic acid, L-α,γ-diaminobutyric acid, L-Allothreonine, L-α-Amino-n-heptanoic acid, L-Homoserine, β-Amino-n-butyric acid, β-Aminoisobutyric acid, γ-Aminobutyric acid, L-isovaline, L-Sarcosine, N-ethyl glycine, N-propyl glycine, N-isopropyl glycine, L-N-methyl alanine, L-N-ethyl alanine, N-methyl β-alanine, N-ethyl β-alanine, Isoserine, L-α-hydroxy-γ-aminobutyric acid, L-diaminopimelic acid, cystathione, L-aminoisobutyric acid, dehydroalanine, delta- aminolevulinic acid, 4-aminobenzoic acid, L-Hydroxyproline, Formylmethioinine, L-lanthionine, djenkolic acid, L-Pyroglutamic acid, Hypusine, L-carboxyglutamic acid, penicillamine, L-thialysine, quisqualic acid, L-canavine, L-azetidine-2-carboxylic acid, D-alanine, D-serine, D-threonine, D-cysteine, D-valine, D-leucine, D-isoleucine, D-methionine, D-proline, D-phenylalanine, D-tyrosine, D-tryptophan, D-aspartic acid, D-glutamic acid, D-asparagine, D-glutamine, D-histidine, D-lysine, D-arginine, D-homocysteine, D-selenocysteine, D-pyrrolysine, D-carnitine, D-hypusine, D-gamma-aminobutyric acid, D-ornithine, D-citrulline, D-α-Amino-n-butyric acid, D-Norvaline, D-Norleucine, D-Pipecolic acid, D-Alloisoleucine, D-α,β-diaminopropionic acid, D-α,γ-diaminobutyric acid, D-Allothreonine, D-α-Amino-n-heptanoic acid, D-Homoserine, D-isovaline, D-Sarcosine, D-N-methyl alanine, D-N-ethyl alanine, D-α-hydroxy-γ-aminobutyric acid, D-diaminopimelic acid, D-aminoisobutyric acid, D-Hydroxyproline, D-lanthionine, D-Pyroglutamic acid, D-carboxyglutamic acid, D-thialysine, quisqualic acid, D-canavine, D-azetidine-2-carboxylic acid. A 'modified bridging amino acid' means a bridging amino acid with R' including a hydroxyl group that has been esterified, a bridging amino acid with R' including a sulphur atom where the sulphur atom has been reacted with an alkyl or other organic group and/or a bridging amino acid with R' including a primary amino group that has been converted into a secondary or tertiary amino group.

In one embodiment, the LU of the Medicant Linker compound and Affinity Medicant Linker conjugate has the formula: --W_{w}-Aₐ wherein -A- is a Stretcher Unit; a is 1 or 2; eachW-- is independently an Amino Acid unit; w is independently an integer ranging from 1 to 20. In the Affinity Medicant Linker conjugate, the LU serves to attach the Medicant moiety and the AM

The Affinity Moiety (AM) includes within its scope an Affinity Unit (AU) that specifically binds or reactively associates or complexes with a receptor, antigen or other receptive moiety associated with a given target-cell population. An AU is a molecule that binds to, complexes with, or reacts with a receptor, antigen or other receptive moiety of a cell population sought to be therapeutically or otherwise biologically modified. In one aspect, the AM acts to deliver the Medicant unit to the particular target cell population with which the AM interacts. Such AM's include, but are not limited to, proteins, polypeptides and peptides and include, antibodies, binding proteins, smaller molecular weight proteins, polypeptides, peptides, lectins, glycoproteins, non-peptides, vitamins, nutrient-transport molecules (such as, but not limited to, transferrin), or any other cell binding molecule or substance.

In an embodiment of the invention, an AM can form a bond to a Stretcher Unit. In an alternative embodiment of the invention, an AM can form a bond to the Stretcher Unit of the LU via a heteroatom of the AM. Heteroatoms that may be present on an AM include sulfur (in one embodiment, from a sulfhydryl group of an AM), oxygen (in one embodiment, from a carbonyl, carboxyl or hydroxyl group of an AM) and nitrogen (in one embodiment, from a primary or secondary amino group of an AM). These hetero atoms can be present on the AM in the AM's natural state, for example a naturally-occurring antibody, or can be introduced into the AM via chemical modification.

In one embodiment, an AM unit has a sulfhydryl group and the AM bonds to the LU via the sulfhydryl group's sulfur atom. In another embodiment, the AM has lysine residues that can react with activated esters (such esters include, but are not limited to, N-hydroxysuccinimide, pentafluorophenyl, and p-nitrophenyl esters) of the Stretcher Unit of the AM and thus form an amide bond consisting of the primary nitrogen atom of the AM and the carboxyl group of the AM. In yet another aspect, the AM has one or more lysine residues that can be chemically modified to introduce one or more sulfhydryl groups. The AM bonds to the LU via the sulfhydryl group's sulfur atom. The reagents that can be used to modify lysines include, but are not limited to, N-succinimidyl S-acetylthioacetate (SATA) and 2-Iminothiolane hydrochloride (Traut's Reagent).

In another embodiment, the AM can have one or more carbohydrate groups that can be chemically modified to have one or more sulfhydryl groups. The AM bonds to the LU (or a Stretcher Unit) via the sulfhydryl group's sulfur atom. In yet another embodiment, the AM can have one or more carbohydrate groups that can be oxidized to provide an aldehyde (-CHO) group. The corresponding aldehyde can form a bond with a reactive site on a Stretcher Unit. Reactive sites on a Stretcher Unit that can react with a carbonyl group on an AM include, but are not limited to, hydrazine and hydroxylamine.

Useful non-immunoreactive protein, polypeptide, or peptide affinity moieties include, but are not limited to, transferrin, epidermal growth factors ("EGF"), bombesin, gastrin, gastrin-releasing peptide, platelet-derived growth factor, IL-2, IL-6, transforming growth factors ("TOP"), such as TGF-.alpha. and TGF-.beta., vaccinia growth factor ("VGF"), insulin and insulin-like growth factors I and II, somatostatin, lectins and apoprotein from low density lipoprotein.

Useful polyclonal antibodies are heterogeneous populations of antibody molecules derived from the sera of immunized animals. Useful monoclonal antibodies are homogeneous populations of antibodies to a particular antigenic determinant (e.g., a cancer cell antigen, a viral antigen, a microbial antigen, a protein, a peptide, a carbohydrate, a chemical, nucleic acid, or fragments thereof). A monoclonal antibody (mAb) to an antigen-of-interest can be prepared by using any technique known in the art which provides for the production of antibody molecules by continuous cell lines in culture.

Useful monoclonal antibodies include, but are not limited to, human monoclonal antibodies, humanized monoclonal antibodies, antibody fragments, or chimeric monoclonal antibodies. Human monoclonal antibodies may be made by any of numerous techniques known in the art.

The antibody can also be a bispecific antibody. Methods for making bispecific antibodies are known in the art and are discussed infra.

The antibody can be a functionally active fragment, derivative or analog of an antibody that immunospecifically binds to target cells (e.g., cancer cell antigens, viral antigens, or microbial antigens) or other antibodies that bind to tumor cells or matrix. In this regard, "functionally active" means that the fragment, derivative or analog is able to elicit anti-anti-idiotype antibodies that recognize the same antigen that the antibody from which the fragment, derivative or analog is derived recognized. Specifically, in an exemplary embodiment the antigenicity of the idiotype of the immunoglobulin molecule can be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art (e.g., the BIA core assay).

Other useful antibodies include fragments of antibodies such as, but not limited to, F(ab')₂ fragments, Fab fragments, Fvs, single chain antibodies, diabodies, triabodies, tetrabodies, scFv, scFv-FV, or any other molecule with the same specificity as the antibody.

Additionally, recombinant antibodies, such as chimeric and humanized monoclonal antibodies, comprising both human and non-human portions, which can be made using standard recombinant DNA techniques, are useful antibodies. A chimeric antibody is a molecule in which different portions are derived from different animal species, such as for example, those having a variable region derived from a murine monoclonal and human immunoglobulin constant regions. Humanized antibodies are antibody molecules from non-human species having one or more complementarity determining regions (CDRs) from the non-human species and a framework region from a human immunoglobulin molecule. Such chimeric and humanized monoclonal antibodies can be produced by recombinant DNA techniques known in the art.

Completely human antibodies are particularly desirable and can be produced using transgenic mice that are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. Other human antibodies can be obtained commercially from, for example, Abgenix, Inc. (now Amgen, Freemont, Calif.) and Medarex (Princeton, N.J.).

Completely human antibodies that recognize a selected epitope can be generated using a technique referred to as "guided selection." In this approach a selected non-human monoclonal antibody, e.g., a mouse antibody, is used to guide the selection of a completely human antibody recognizing the same epitope. Human antibodies can also be produced using various techniques known in the art, including phage display libraries.

In other embodiments, the antibody is a fusion protein of an antibody, or a functionally active fragment thereof, for example in which the antibody is fused via a covalent bond (e.g., a peptide bond), at either the N-terminus or the C-terminus to an amino acid sequence of another protein (or portion thereof, preferably at least 10, 20 or 50 amino acid portion of the protein) that is not from an antibody. Preferably, the antibody or fragment thereof is covalently linked to the other protein at the N-terminus of the constant domain.

Antibodies include analogs and derivatives that are either modified, i.e., by the covalent attachment of any type of molecule as long as such covalent attachment permits the antibody to retain its antigen binding immunospecificity. For example, but not by way of limitation, derivatives and analogs of the antibodies include those that have been further modified, e.g., by glycosylation, acetylation, pegylation, phosphorylation, amidation, derivatization by known protecting/blocking groups, proteolytic cleavage, linkage to a cellular antibody unit or other protein, etc. Any of numerous chemical modifications can be carried out by known techniques including, but not limited to, specific chemical cleavage, acetylation, formylation, metabolic synthesis in the presence of tunicamycin, etc. Additionally, the analog or derivative can contain one or more unnatural amino acids.

Antibodies can have modifications (e.g., substitutions, deletions or additions) in amino acid residues that interact with Fc receptors. In particular, antibodies can have modifications in amino acid residues identified as involved in the interaction between the anti-Fc domain and the FcRn receptor.

Antibodies immunospecific for a cancer cell antigen can be obtained commercially or produced by any method known to one of skill in the art such as, e.g., chemical synthesis or recombinant expression techniques. The nucleotide sequence encoding antibodies immunospecific for a cancer cell antigen can be obtained, e.g., from the GenBank database or a database like it, literature publications, or by routine cloning and sequencing.

In a specific embodiment, known antibodies for the treatment of cancer can be used. Antibodies immunospecific for a cancer cell antigen can be obtained commercially or produced by any method known to one of skill in the art such as, e.g., recombinant expression techniques. The nucleotide sequence encoding antibodies immunospecific for a cancer cell antigen can be obtained, e.g., from the GenBank database or a database like it, the literature publications, or by routine cloning and sequencing. Examples of antibodies available for the treatment of cancer include, but are not limited to, RITUXAN^{®} (rituximab; Genentech) which is a chimeric anti-CD20 monoclonal antibody for the treatment of patients with non-Hodgkin's lymphoma; OVAREX which is a murine antibody for the treatment of ovarian cancer; PANOREX (Glaxo Wellcome, N.C.) which is a murine IgG₂ₐ antibody for the treatment of colorectal cancer; Cetuximab ERBITUX (Imclone Systems Inc., NY) which is an anti-EGFR IgG chimeric antibody for the treatment of epidermal growth factor positive cancers, such as head and neck cancer; Vitaxin (MedImmune, Inc., MD) which is a humanized antibody for the treatment of sarcoma; CAMPATH I/H (Leukosite, MA) which is a humanized IgG₁ antibody for the treatment of chronic lymphocytic leukemia (CLL); SMART MI95 (Protein Design Labs, Inc., CA) and SGN-33 (Seattle Genetics, Inc., WA) which is a humanized anti-CD33 IgG antibody for the treatment of acute myeloid leukemia (AML); LYMPHOCIDE (Immunomedics, Inc., NJ) which is a humanized anti-CD22 IgG antibody for the treatment of non-Hodgkin's lymphoma; SMART ID10 (Protein Design Labs, Inc., CA) which is a humanized anti-HLA-DR antibody for the treatment of non-Hodgkin's lymphoma; ONCOLYM (Techniclone, Inc., CA) which is a radiolabeled murine anti-HLA-DrlO antibody for the treatment of non-Hodgkin's lymphoma; ALLOMUNE (BioTransplant, CA) which is a humanized anti-CD2 mAb for the treatment of Hodgkin's Disease or non-Hodgkin's lymphoma; AVASTIN (Genentech, Inc., CA) which is an anti-VEGF humanized antibody for the treatment of lung and colorectal cancers; Epratuzamab (Immunomedics, Inc., NJ and Amgen, Calif.) which is an anti-CD22 antibody for the treatment of non-Hodgkin's lymphoma; and CEACIDE (Immunomedics, N.J.) which is a humanized anti-CEA antibody for the treatment of colorectal cancer.

Other antibodies useful in the treatment of cancer include, but are not limited to, antibodies against the following antigens (where exemplary cancers that can be treated with the antibody are in parentheses): Alk (adrenocarcinomas) (SEQ. ID. 103), CA125 (ovarian) (SEQ. ID. 104), CA15-3 (carcinomas) (SEQ. ID. 105), CA19-9 (carcinomas), L6 (carcinomas) (SEQ. ID. 107), Lewis Y (carcinomas) (SEQ. ID. 108), Lewis X (carcinomas) (SEQ. ID. 109), alpha fetoprotein (carcinomas) (SEQ. ID. 110), CA 242 (colorectal), placental alkaline phosphatase (carcinomas) (SEQ. ID. 112), prostate specific antigen (prostate) (SEQ. ID. 113), prostate specific membrane antigen (prostate) (SEQ. ID. 114), prostatic acid phosphatase (prostate) (SEQ. ID. 115), epidermal growth factor (carcinomas), MAGE-1 (carcinomas) (SEQ. ID. 117), MAGE-2 (carcinomas) (SEQ. ID. 118), MAGE-3 (carcinomas) (SEQ. ID. 119), MAGE -4 (carcinomas) (SEQ. ID. 120), anti-transferrin receptor (carcinomas) (SEQ. ID. 121), p97 (melanoma) (SEQ. ID. 122), MUC1 (breast cancer) (SEQ. ID. 123), CEA (colorectal) (SEQ. ID. 124), gp100 (melanoma) (SEQ. ID. 125), MART-1 (melanoma) (SEQ. ID. 126), IL-2 receptor (T-cell leukemia and lymphomas), CD2 (buccal mucosa) (SEQ. ID. 128), CD20 (non-Hodgkin's lymphoma) (SEQ. ID. 129), CD52 (leukemia) (SEQ. ID. 130), CD33 (leukemia), CD22 (lymphoma), beta human chorionic gonadotropin (carcinoma) (SEQ. ID. 133), CD38 (multiple myeloma) (SEQ. ID. 134), CD40 (lymphoma) (SEQ. ID. 135), CD80 (colorectal), CD86 (colorectal), mucin (carcinomas), P21 (carcinomas), MPG (melanoma) (SEQ. ID. 140), Neu oncogene product (carcinomas) and STEAP-1 (prostate).

Compositions and Methods of Administration. In other embodiments, described is a pharmaceutical composition including an effective amount of an Affinity Medicant Linker conjugate and/or a Medicant Linker compound and a pharmaceutically acceptable carrier or vehicle. The compositions are suitable for veterinary or human administration.

The present pharmaceutical compositions can be in any form that allows for the composition to be administered to a patient. For example, the composition can be in the form of a solid or liquid. Typical routes of administration include, without limitation, parenteral, ocular and intra-tumor. Parenteral administration includes subcutaneous injections, intravenous, intramuscular or intrasternal injection or infusion techniques. In one aspect, the compositions are administered parenterally. In a specific embodiment, the compositions are administered intravenously.

Pharmaceutical compositions can be formulated so as to allow an Affinity Medicant Linker conjugate and/or a Medicant Linker compound to be bioavailable upon administration of the composition to a patient. Compositions can take the form of one or more dosage units, where for example, a tablet can be a single dosage unit, and a container of an Affinity Medicant Linker conjugate and/or a Medicant Linker compound in liquid form can hold a plurality of dosage units.

Materials used in preparing the pharmaceutical compositions can be non-toxic in the amounts used. It will be evident to those of ordinary skill in the art that the optimal dosage of the active ingredient(s) in the pharmaceutical composition will depend on a variety of factors. Relevant factors include, without limitation, the type of animal (e.g., human), the particular form of the Affinity Medicant Linker conjugate and/or a Medicant Linker compound, the manner of administration, and the composition employed.

The pharmaceutically acceptable carrier or vehicle can be solid or particulate, so that the compositions are, for example, in tablet or powder form. The carrier(s) can be liquid. In addition, the carrier(s) can be particulate.

The composition can be in the form of a liquid, e.g., a solution, emulsion or suspension. In a composition for administration by injection, one or more of a surfactant, preservative, wetting agent, dispersing agent, suspending agent, buffer, stabilizer and isotonic agent can also be included.

The liquid compositions, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates, phosphates or amino acids and agents for the adjustment of tonicity such as sodium chloride or dextrose. A parenteral composition can be enclosed in ampoule, a disposable syringe or a multiple-dose vial made of glass, plastic or other material. Physiological saline is an exemplary adjuvant. An injectable composition is preferably sterile.

The amount of the Affinity Medicant Linker conjugate and/or a Medicant Linker compound that is effective in the treatment of a particular disorder or condition will depend on the nature of the disorder or condition, and can be determined by standard clinical techniques. In addition, in vitro or in vivo assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The compositions comprise an effective amount of an Affinity Medicant Linker conjugate and/or a Medicant Linker compound such that a suitable dosage will be obtained. Typically, this amount is at least about 0.01% of an Affinity Medicant Linker conjugate and/or a Medicant Linker compound by weight of the composition. In an exemplary embodiment, pharmaceutical compositions are prepared so that a parenteral dosage unit contains from about 0.01% to about 2% by weight of the Affinity Medicant Linker conjugate and/or a Medicant Linker compound.

For intravenous administration, the composition can comprise from about 0.01 to about 100 mg of an Affinity Medicant Linker conjugate and/or a Medicant Linker compound per kg of the patient's body weight. In one aspect, the composition can include from about 1 to about 100 mg of an Affinity Medicant Linker conjugate and/or a Medicant Linker compound per kg of the patient's body weight. In another aspect, the amount administered will be in the range from about 0.1 to about 25 mg/kg of body weight of the Affinity Medicant Linker conjugate and/or a Medicant Linker compound.

Prior art ADC's such as Kadcyla or Adcetris deliver a dose of the associated toxin (auristatins MMAE or emtansine DM-1) that is three or more times the lethal dose (for that toxin) which results in severe systemic (or non-target) toxicity. In contrast, illudofulvenes ADC's (such as **analog 189, analog 190, analog 217, analog 218, analog 219, analog 222 ,** or **analog 316** deliver less than one third (i.e., < 1/3) of a lethal dose, minimizing the risk and severity of systemic toxicity. Illudofulvenes are true cytotoxic agents whereas other toxic agents used in prior art ADC's (e.g., pyrrolobenzodiazepines, maytansines, fumagillols, dolstatins, auristatins, enadiynes, halichondrins, and tubulysins) are only cytostatic. See **Table VI** (based on the NCI-DTP 60 cell line). Hence, other payloads, such as those used in Herceptin, Adcetris or Rituxin only stall tumor cell growth and do not actually kill the tumor cells. Other payloads (e.g., pyrrolobenzodiazepines, maytansines, fumagillols, dolstatins, auristatins, enadiynes, halichondrins, and tubulysins) are not active against multidrug phenotypes, notably the MDR1/GP170 and MRP/GP180 transport mechanisms (see **Table IV).** Illudofulvenes show the excellent effect of remaining active against all MDR phenotypes known regardless of the mechanism of resistance (see **Table IV).** Hence, if tumor cells have already developed multidrug resistance to a prior art ADC with a prior art toxin, or develop multi-drug resistance during the administration of a course of the prior art ADC with a prior art toxin, then the ADC will have no efficacy. In contrast, ADCs developed with illudofulvenes have the advantageous effect that they will continue to kill cancer cells.

Generally, the dosage of an Affinity Medicant Linker conjugate and/or a Medicant Linker compound administered to a patient is typically about 0.01 mg/kg to about 20 mg/kg of the patient's body weight. In one aspect, the dosage administered to a patient is between about 0.01 mg/kg to about 10 mg/kg of the patient's body weight. In another aspect, the dosage administered to a patient is between about 0.1 mg/kg and about 10 mg/kg of the patient's body weight. In yet another aspect, the dosage administered to a patient is between about 0.1 mg/kg and about 5 mg/kg of the patient's body weight. In yet another aspect the dosage administered is between about 0.1 mg/kg to about 3 mg/kg of the patient's body weight. In yet another aspect, the dosage administered is between about 1 mg/kg to about 3 mg/kg of the patient's body weight.

The Affinity Medicant Linker conjugate and/or a Medicant Linker compound can be administered by any convenient route, for example by infusion or bolus injection. Administration can be systemic or local. Various delivery systems are known, e.g., encapsulation in liposomes, micro-particles, microcapsules, capsules, etc., and can be used to administer an Affinity Medicant Linker conjugate and/or a Medicant Linker compound. In certain embodiments, more than one Affinity Medicant Linker conjugate and/or a Medicant Linker compound is administered to a patient.

In specific embodiments, it can be desirable to administer one or more Affinity Medicant Linker conjugates and/or a Medicant Linker compound locally to the area in need of treatment. This can be achieved, for example, and not by way of limitation, by local infusion during surgery; topical application, e.g., in conjunction with a wound dressing after surgery; by injection; by means of a catheter; or by means of an implant, the implant being of a porous, nonporous, or gelatinous material, including membranes, such as sialastic membranes, or fibers. In one embodiment, administration can be by direct injection at the site (or former site) of a cancer, tumor or neoplastic or pre-neoplastic tissue. In another embodiment, administration can be by direct injection at the site (or former site) of a manifestation of an autoimmune disease.

In yet another embodiment, the Affinity Medicant Linker conjugate and/or a Medicant Linker compound can be delivered in a controlled release system, such as but not limited to, a pump or various polymeric materials can be used. In yet another embodiment, a controlled-release system can be placed in proximity of the target of the Linker Affinity conjugate and/or a Medicant Linker compound, e.g., the liver, thus requiring only a fraction of the systemic dose.

The term "carrier" refers to a diluent, adjuvant or excipient, with which an Affinity Medicant Linker conjugate and/or a Medicant Linker compound is administered. Such pharmaceutical carriers can be liquids, such as water and oils, including those of petroleum, animal, vegetable or synthetic origin. The carriers can be saline, and the like. In addition, auxiliary, stabilizing and other agents can be used. In one embodiment, when administered to a patient, the Affinity Medicant Linker conjugate and/or the Medicant Linker compound and pharmaceutically acceptable carriers are sterile. Water is an exemplary carrier when the Affinity Medicant Linker conjugate and/or a Medicant Linker compound are administered intravenously. Saline solutions and aqueous dextrose and glycerol solutions can also be employed as liquid carriers, particularly for injectable solutions. The present compositions, if desired, can also contain minor amounts of wetting or emulsifying agents, or pH buffering agents.

The present compositions can take the form of solutions, pellets, powders, sustained-release formulations, or any other form suitable for use.

In an embodiment, the Affinity Medicant Linker conjugates and/or Medicant Linker compounds are formulated in accordance with routine procedures as a pharmaceutical composition adapted for intravenous administration to animals, particularly human beings. Typically, the carriers or vehicles for intravenous administration are sterile isotonic aqueous buffer solutions. Where necessary, the compositions can also include a solubilizing agent. Compositions for intravenous administration can optionally comprise a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where an Affinity Medicant Linker conjugate and/or Medicant Linker compound is to be administered by infusion, it can be dispensed, for example, with an infusion bottle containing sterile pharmaceutical grade water or saline. Where the Affinity Medicant Linker conjugate and/or Medicant Linker compound is administered by injection, an ampoule of sterile water for injection or saline can be provided so that the ingredients can be mixed prior to administration.

The composition can include various materials that modify the physical form of a solid or liquid dosage unit. For example, the composition can include materials that form a coating shell around the active ingredients. The materials that form the coating shell are typically inert, and can be selected from, for example, sugar, shellac, and other enteric coating agents. Alternatively, the active ingredients can be encased in a gelatin capsule.

Whether in solid or liquid form, the present compositions can include a pharmacological agent used in the treatment of cancer, an autoimmune disease or an infectious disease.

Treatment of Cancer. The Affinity Medicant Linker conjugates and Medicant Linker compounds are useful for inhibiting the multiplication of a tumor cell or cancer cell, causing apoptosis in a tumor or cancer cell, or for treating cancer in a patient. The Affinity Medicant Linker conjugates and/or Medicant Linker compounds can be used accordingly in a variety of settings for the treatment of animal cancers. The Affinity Medicant Linker Conjugates can be used to deliver a Medicant or Medicant unit to a tumor cell or cancer cell. Without being bound by theory, in one embodiment, the AM of an Affinity Medicant Linker conjugate binds to or associates with a cancer-cell or a tumor-cell-associated antigen, and the Affinity Medicant Linker conjugate can be taken up (internalized) inside a tumor cell or cancer cell through receptor-mediated endocytosis or other internalization mechanism. The antigen can be attached to a tumor cell or cancer cell or can be an extracellular matrix protein associated with the tumor cell or cancer cell. Once inside the cell, one or more specific peptide sequences within or at the Medicant unit's proximal end of the LU are hydrolytically cleaved by one or more tumor cell or cancer cell-associated proteases, resulting in release of the Medicant unit. The released Medicant unit is then free to migrate within the cell and induce cytotoxic or cytostatic activities. The Affinity Medicant Linker conjugate also can be cleaved by an intracellular protease to release the Medicant moiety. In an alternative embodiment, the Medicant or Medicant unit is cleaved from the Affinity Medicant Linker conjugate outside the tumor cell or cancer cell, and the Medicant or Medicant unit subsequently penetrates the cell.

The Affinity Medicant Linker conjugates provide conjugation-specific tumor or cancer medicant targeting, thus reducing general toxicity of the Medicant. The LUs stabilize the Affinity Medicant Conjugates in blood, yet are cleavable by tumor-specific proteases within the cell, liberating a Medicant unit.

In one embodiment, the AM binds to the tumor cell or cancer cell. In another embodiment, the AM binds to a tumor cell or cancer cell antigen which is on the surface of the tumor cell or cancer cell. In another embodiment, the AM binds to a tumor cell or cancer cell antigen which is an extracellular matrix protein associated with the tumor cell or cancer cell.

The specificity of the AM for a particular tumor cell or cancer cell can be important for determining those tumors or cancers that are most effectively treated. For example, an Affinity Medicant Linker conjugate and/or Medicant Linker compound having a BR96 AM can be useful for treating antigen positive carcinomas including those of the lung, breast, colon, ovaries, and pancreas. Affinity Medicant Linker conjugates having an anti-CD30 or an anti-CD70 binding affinity moiety can be useful for treating hematologic malignancies.

Other particular types of cancers that can be treated with an Affinity Medicant Linker conjugate and/or a Medicant Linker compound include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, retinoblastoma blood-borne cancers, including but not limited to: acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", hairy cell leukemia, multiple myeloma acute and chronic leukemias: lymphoblastic, myelogenous, lymphocytic, myelocytic leukemias Lymphomas: Hodgkin's disease, non-Hodgkin's Lymphoma, Multiple myeloma, Waldenstrom's macroglobulinemia, Heavy chain disease, Polycythemia vera.

Multi-Modality Therapy for Cancer. Cancers, including, but not limited to, a tumor, metastasis, or other disease or disorder characterized by uncontrolled cell growth, can be treated or inhibited by administration of an Affinity Medicant Linker conjugate or Medicant Linker compound.

In other embodiments, methods for treating cancer are provided, including administering to a patient in need thereof an effective amount of an Affinity Medicant Linker conjugate and a chemotherapeutic agent. In one embodiment the chemotherapeutic agent is that with which treatment of the cancer has not been found to be refractory. In another embodiment, the chemotherapeutic agent is that with which the treatment of cancer has been found to be refractory. The Affinity Medicant Linker conjugates can be administered to a patient that has also undergone surgery as treatment for the cancer.

In some embodiments, the patient also receives an additional treatment, such as radiation therapy. In a specific embodiment, the Affinity Medicant Linker conjugate is administered concurrently with the chemotherapeutic agent or with radiation therapy. In another specific embodiment, the chemotherapeutic agent or radiation therapy is administered prior or subsequent to administration of an Affinity Medicant Linker conjugate.

A chemotherapeutic agent can be administered over a series of sessions. Any one or a combination of the chemotherapeutic agents, such a standard of care chemotherapeutic agent(s), can be administered.

Additionally, methods of treatment of cancer with an Affinity Medicant Linker conjugate and/or a Medicant Linker compound are provided as an alternative to chemotherapy or radiation therapy where the chemotherapy or the radiation therapy has proven or can prove too toxic, e.g., results in unacceptable or unbearable side effects, for the subject being treated. The patient being treated can, optionally, be treated with another cancer treatment such as surgery, radiation therapy or chemotherapy, depending on which treatment is found to be acceptable or bearable.

Example 1. Synthesis of Medicant **113.** The Wittig reaction was performed on **analog 010.** First 65 mg CH₃PPh₃Br (0.185 mmol) in anhydrous THF was cooled to -75°C and stirred for 1 hour. Then 200 µL of n-butyl lithium (0.183 mmol) was added very slowly to the flask while maintaining temperature at -75°C, and a yellow precipitate formed. It was stirred for another 1.5 hours then **analog 010** (50 mg, 0.183 mmol) was slowly added while maintaining temperature at -75°C, followed by stirring for 2.0 hours. The reaction was quenched with ammonium chloride, extracted with CH₂Cl₂, washed with water, NaHCO₃, and saline. Dried over Na₂SO₄ and concentrated. The residue was eluted through a column (10% ethyl acetate in hexane) to give **analog 113** as a solid.

Example 2. Synthesis of Medicant-Estrone **107.** Analog **106** (see **Example 13)** (139 mg 0.384 mmol, 1 equiv.), DMAP (4 mg, 0.03 mmol, 0.08 equiv.) and estrone (104.4 mg, 0.384 mmol, 1 equiv.) were dissolved in CH₂Cl₂ (14 mL) at 0 °C. To this solution was added CH₂Cl₂ solution of DCC (460 µL, 1 M, 0.46 mmol, 1.2 equiv.) through a syringe. After 0.5 hours the solution was raised to RT. After 2 hours the mixture was filtered and the filtrate was washed with dilute HCl (1.5%), saturated NaHCO₃ and brine in sequence. The organic phase was then dried and evaporated. The residue was eluted through a column (CH₂Cl₂/Methanol, 10:0.25) to give **analog 107** (100 mg, 42%) as semisolid. **Analog 107** can be subsequently linked to estrone.

Example 3. Preparation of Medicant-Estradiol **108. Analog 038** (58.5 mg, 0.2035 mmol), beta-estradiol (58.0 mg, 0.2150 mmol) and DMAP (5 mg. 0.048 mmol) were dissolved in CH₂Cl₂ (5.6 mL) at 0 °C. To this solution was added CH₂Cl₂ solution of DCC (250 µL, 1 M, 0.244 mmol), stirred for 30 minutes, allowed to warm to RT then stirred for 1.5 hours. The filtrate was washed with dilute HCl (1.5%), saturated NaHCO₃ and brine in sequence. The organic phase was dried over Na₂SO₄, and evaporated. The residue was eluted through a column (100% CH₂Cl₂), fractions collected then eluted through a second column (CH₂Cl₂ plus 0.5% methanol), to give **analog 108** (45 mg) as a solid.

**Table I** shows the cytotoxic data IC₅₀ values (micromolar, 2 hour exposure, N = 3, mean + SD) for **108.** MCF7 over express estrogen alpha-receptors. MCF7 cells are preferentially killed by **110** the acylfulvene-estrone analog and to a lesser extent **108** the acylfulvene-estradiol analog because estrone preferentially binds to alpha-receptor.

Example 4. Preparation of Medicant-Estradiol **109.** Analog **106** (54.5 mg, 0.15 mmol, 1 equiv.), β-estradiol (40.5 mg, 0.15 mmol), and DMAP (1.8 mg, 0.015 mmol, 0.1 equiv.) were dissolved in CH₂Cl₂ (5 mL) at 0 °C. To this solution was added CH₂Cl₂ solution of DCC (165 µL, 1 M, 0.165 mmol, 1.1 equiv.). The mixture was raised to RT after 0.5 h. After another 2 h, the mixture was filtered. The filtrate was washed with dilute HCl (1.5%), saturated NaHCO₃ and brine in sequence. The organic phase was dried and evaporated. The residue was eluted through a column (CH₂Cl₂/Methanol 10:0.25) to give **analog 109** (55 mg, 60%) as semisolid.

Example 5. Preparation of Medicant-Estrone **110.** Analog **038** (68 mg, 0.2365 mmol), estrone (68.0 mg, 0.2160 mmol) and DMAP (5 mg. 0.048 mmol) were dissolved in CH₂Cl₂ (8.0 mL) at 0 °C. To this solution was added CH₂Cl₂ solution of DCC (300 µL, 1 M, 0.283 mmol), stirred for 30 minutes, allowed to warm to RT then stirred for 0.5 hours. The filtrate was washed with dilute HCl (1.5%), saturated NaHCO₃ and brine in sequence. The organic phase was dried over Na₂SO₄, and evaporated. The residue was eluted through a column (100% CH₂Cl₂), fractions collected then eluted through a second column (CH₂Cl₂ plus 0.5% methanol), to give **analog 110** (40 mg) as a solid.

**Table I** shows the cytotoxic data IC₅₀ values (micromolar, 2 hour exposure, N =3, mean + SD) for **110.** MCF7 cells over express estrogen alpha-receptors. MCF7 cells are preferentially killed by the acylfulvene-estrone **analog 110** and to a lesser extent by the acylfulvene-estradiol **analog 108** because estrone preferentially binds to alpha-receptor. In contrast, illudin M killed both ER negative and ER positive cells to the same extent. The data in **Table I** demonstrates that **analog 108** and **analog 110** are preferentially cytotoxic to cells expressing large numbers of estrogen receptors on their surface.

Example 6. Preparation of Medicant-Testosterone **111.** Analog **038** (52.5 mg, 0.182 mmol), testosterone (50.0 mg, 0.173 mmol) and DMAP (5 mg. 0.048 mmol) were dissolved in CH₂Cl₂ (8.0 mL) at 0 °C. To this solution was added CH₂Cl₂ solution of DCC (250 µL, 1 M), stirred for 30 minutes, allowed to warm to RT then stirred for 2 hours. The filtrate was washed with dilute HCl (1.5%), saturated NaHCO₃ and brine in sequence. The organic phase was dried over Na₂SO₄, and evaporated. The residue was eluted through a column (100% CH₂Cl₂ plus 0.5% methanol), to give **analog 111** (15 mg) as a solid.

Example 7. Preparation of Medicant-Androsterone **112. Analog 038** (29 mg), androsterone (25.0 mg) and DMAP (5 mg. 0.048 mmol) were dissolved in CH₂Cl₂ (5.0 mL) at 0 °C. To this solution was added CH₂Cl₂ solution of DCC (150 µL, 1 M), stirred for 30 minutes, allowed to warm to RT then stirred for 2 hours. The filtrate was washed with dilute HCl (1.5%), saturated NaHCO₃ and brine in sequence. The organic phase was dried over Na₂SO₄, and evaporated. The residue was eluted through a column (2:3 ethyl acetate: hexane) to give **analog 112** (15 mg) as a solid.

In an embodiment of the present invention, an illudin2 moiety **1302** linked via a traditional linker **1240** to a steroid **1140** bind to receptors for the steroid and directs the illudin2 moiety **1302** to cell populations expressing the receptor. In an embodiment of the present invention, a steroid **1140** linked via a traditional linker **1240** to an illudin2 moiety **1302** acts as an AM for the steroid hormone receptor and directs the illudin2 moiety **1302** to tissues containing cells expressing the receptor. In an embodiment of the present invention, a steroid **1140** linked via a traditional linker **1240** to an illudin2 moiety **1302** acts as an AM for the steroid hormone receptor and directs the illudin2 moiety **1302** to tumors containing cells over-expressing the receptor compared to non tumor cells.

Example 4. Preparation of Medicant-Estradiol **109.** Analog **106** (54.5 mg, 0.15 mmol, 1 equiv.), β-estradiol (40.5 mg, 0.15 mmol), and DMAP (1.8 mg, 0.015 mmol, 0.1 equiv.) were dissolved in CH₂Cl₂ (5 mL) at 0 °C. To this solution was added CH₂Cl₂ solution of DCC (165 µL, 1 M, 0.165 mmol, 1.1 equiv.). The mixture was raised to RT after 0.5 h. After another 2 h, the mixture was filtered. The filtrate was washed with dilute HCl (1.5%), saturated NaHCO₃ and brine in sequence. The organic phase was dried and evaporated. The residue was eluted through a column (CH₂Cl₂/Methanol 10:0.25) to give **analog 109** (55 mg, 60%) as semisolid.

Example 5. Preparation of Medicant-Estrone **110.** Analog **038** (68 mg, 0.2365 mmol), estrone (68.0 mg, 0.2160 mmol) and DMAP (5 mg. 0.048 mmol) were dissolved in CH₂Cl₂ (8.0 mL) at 0 °C. To this solution was added CH₂Cl₂ solution of DCC (300 µL, 1 M, 0.283 mmol), stirred for 30 minutes, allowed to warm to RT then stirred for 0.5 hours. The filtrate was washed with dilute HCl (1.5%), saturated NaHCO₃ and brine in sequence. The organic phase was dried over Na₂SO₄, and evaporated. The residue was eluted through a column (100% CH₂Cl₂), fractions collected then eluted through a second column (CH₂Cl₂ plus 0.5% methanol), to give **analog 110** (40 mg) as a solid.

**Table I** shows the cytotoxic data IC₅₀ values (micromolar, 2 hour exposure, N =3, mean + SD) for 110. MCF7 cells over express estrogen alpha-receptors. MCF7 cells are preferentially killed by the acylfulvene-estrone **analog 110** and to a lesser extent by the acylfulvene-estradiol **analog 108** because estrone preferentially binds to alpha-receptor. In contrast, illudin M killed both ER negative and ER positive cells to the same extent. The data in **Table I** demonstrates that compounds **108** and **110** are preferentially cytotoxic to cells expressing large numbers of estrogen receptors on their surface.

Example 6. Preparation of Medicant-Testosterone **111.** Analog **038** (52.5 mg, 0.182 mmol), testosterone (50.0 mg, 0.173 mmol) and DMAP (5 mg. 0.048 mmol) were dissolved in CH₂Cl₂ (8.0 mL) at 0 °C. To this solution was added CH₂Cl₂ solution of DCC (250 µL, 1 M), stirred for 30 minutes, allowed to warm to RT then stirred for 2 hours. The filtrate was washed with dilute HCl (1.5%), saturated NaHCO₃ and brine in sequence. The organic phase was dried over Na₂SO₄, and evaporated. The residue was eluted through a column (100% CH₂Cl₂ plus 0.5% methanol), to give **analog 111** (15 mg) as a solid.

Example 7. Preparation of Medicant-Androsterone **112.** Analog **038** (29 mg), androsterone (25.0 mg) and DMAP (5 mg. 0.048 mmol) were dissolved in CH₂Cl₂ (5.0 mL) at 0 °C. To this solution was added CH₂Cl₂ solution of DCC (150 µL, 1 M), stirred for 30 minutes, allowed to warm to RT then stirred for 2 hours. The filtrate was washed with dilute HCl (1.5%), saturated NaHCO₃ and brine in sequence. The organic phase was dried over Na₂SO₄, and evaporated. The residue was eluted through a column (2:3 ethyl acetate: hexane) to give **analog** 112 (15 mg) as a solid.

Example 13. Synthesis of Medicant 106. Illudin M (450 mg, 1.845 mmol, 1 equiv.), glutaric anhydride (2.10 g, 18.45 mmol, 10 equiv.) and DMAP (171 mg, 1.4 mmol, 0.76 equiv.) were dissolved in CH₂Cl₂ (5 mL) at RT. After 3.5 hours the mixture was taken up by CH₂Cl₂, which was washed with water, and brine in sequence. It was then dried and evaporated. The residue was eluted through a column (Hexane/EtOAc 4:1) to give **analog 106** (365 mg, 55%) as a liquid. UV (CHCl₃) λ nm (ε): 309 (3387).

Analog **106** was generated from illudin M as outlined in **Example 13.** The carboxylic acid derivative was activated using DCC/DMAP to synthesize steroid AFC's **107** and **109.** In addition, Irofulven carboxylic acid derivative, **analog 038** was activated using DCC/DMAP to produce analogs **108, 110, 111,** and **112.** In general, carboxylate group containing compounds can be activated using a carbodiimide in the presence of an amino acid to form an azlactone. The azlactone formed will react spontaneously with primary amine groups on an amino acid, a peptide, an antibody, a protein, or another drug, and undergo ring opening with the formation of an amide bond. For proteins, antibodies and peptides the amino acids capable of reacting with the azlactone derivative includes arginine and lysine.

To form an Illudin derived azlactone active drug-linker moiety, either **analog 106** or **analog 038** can be activated by DCC/DMAP in the presence of a small amino acid such as glycine to form the azlactone. DCC cannot be added without the presence of an amine containing target (such as the glycine) or the activated carboxylate reacts with another carboxylate to form a symmetrical anhydride. The azlactone formed will react spontaneously with primary amine groups on a peptide, an antibody, a protein, or a medicant.

Example 14. Activation of **analog 038** by DCC to form medicant-azlactone. Part A: Production of Azlactone from carboxylate Acylfulvene analog: Analog **038** (58.5 mg, 0.2035 mmol), and DMAP (5 mg. 0.048 mmol) were dissolved in CH₂Cl₂ (5.6 mL) at 0°C. The desired amino acid (such as glycine) was added in an equimolar amount. Note that amino acids having substitutions on the C4 carbon (such as alpha-methyl glycine or 2-dimethylglycine) are preferred over conventional amino acids as substitution cannot occur at the C4 position after ring-opening and all nucleophilic coupling reactions must occur at the C5 position, resulting only in the desired amide-bond formation with the amine-containing molecule. To this solution was added CH₂Cl₂ solution of DCC (250 µL, 1 M, 0.244 mmol), stirred for 30 minutes, allowed to warm to RT then stirred for 1.5 hours. The filtrate was washed with dilute HCl (1.5%), saturated NaHCO₃ and brine in sequence. The organic phase was dried over Na₂SO₄, and evaporated. The residue was eluted through a column (100% CH₂Cl₂ plus 0.5% methanol), to give the desired azlactone analog as a solid. Part B: Coupling of Azlactone to the protein component (reacting with primary amines on amino acids such as the one on lysine): The typical protein coupling reaction consists of the Azlactone suspended in buffer [25 mM sodium phosphate, 150 mM NaCl (pH 7.5)] and the desired amount of protein (20 µg to 5.0 mg) was added. The mixture was gently rocked for 60 minutes, then the reaction terminated by the addition of the blocking reagent, 1.0 ml of 1.0 M ethanolamine in 25 mM sodium pyrophosphate (titrated to pH 9.0 with HCI) Sample rocked gently for 5 minutes then the residual ethanolamine removed by dialysis or chromatography using pH 7.5 phosphate-NaCl buffer.

Example 15. Reaction of the medicant-azlactone product with an antibody. The azlactone derivative generated in Example 14 (note that other amino acids can be used in place of glycine) was then reacted with the desired peptide or protein or other compound containing a primary amino group at a 1:1 ratio in buffer (25 mM sodium phosphate, 150 mM sodium chloride, pH 7.5) with gentle rocking at RT for 60 minutes. The reaction was terminated by the addition of 1.0 mL of 25 mM ethanolamine (titrated to pH 9.00) with rocking for 5 minutes at RT). The drug-azlactone-ligand product can be purified by column chromatography or dialysis to remove the ethanolamine by-product.

Example 16. Synthesis of Medicant **114.** (CH₃) ₃S(O)I (110 mg, 0.4 mmol) and tBuOK(50 mg, 0.4 mmol) were dissolved in anhydrous DMSO (1 mL) and stirred at RT for 40 minutes at RT. Then **analog 010** (50 mg, 0.2 mmol) in 1.0 mL of DMSO was added via syringe, and stirred for 3 hours. Reaction quenched with saturated NH₄Cl (1 mL), extracted with CH₂Cl₂, dried over Na₂SO₄, concentrated then chromatographed (2:3 hexane : ethyl acetate) to yield **analog 114** (20 mg., 50% yield).

Example 17. Synthesis of Medicant **115. Analog 010** (40 mg) and NAHCO₃ (50 mg) are dissolved in 10 mL of 1:1 Ethanol and water mixture, then hydroxylamine hydrochloride (20 mg) was added, stirred for 30 minutes at RT. Water and ethyl acetate (1:1 mixture) was added, stirred, the organic layer was recovered, washed with saturated NaHCO₃ and then brine, dried over Na₂SO₄, concentrated then chromatographed (2:3 ethyl acetate: hexane) to yield **analog 115.**

Example 18. Synthesis of Medicant **116.** SeO₂ (45 mg) and 500 mg SiO2 transferred into a dried RB flask, 5 mL of CH₂Cl₂ added, and stirred for 1 hour under nitrogen. Then 250 µL of tBuO₂H added and stirred for 15minutes. Then 100 mg of Irofulven in 1 mL CH₂Cl₂ was added, and stirred for 3 hours at RT under a nitrogen atmosphere. Product was filtered, wash twice with water (25 mL), twice with brine (25 mL), dried over Na₂SO₄ and concentrated then chromatographed (4:1 hexane: ethyl acetate) to yield **analog 116.**

Example 19. Synthesis of Medicant **116. Analog 117:** Illudin S (100 mg, 0.378 mmol) and glutaric anhydride (215.46 mg, 1.89 mmol) are dissolved in 5 mL of CH₂Cl₂, and DMAP added (92.23 mg, 0.756 mmol), and stirred for 2 hours at RT. The CH₂Cl₂ was evaporated, 5 mL of water was added, and stirred for 1 hour. The solution was extracted with 10 mL of CH₂Cl₂, washed with water, dried over Na₂SO₄ and concentrated to yield **analog 117** (120 mg).

Example 20. **Synthesis of Analog 118:** Analog **302** (75 mg), glutaric anhydride (20 mg) are dissolved in 5 mL of CH₂Cl₂, and DMAP added (42 mg), and stirred for 2 hours at RT. The CH₂Cl₂ was evaporated, 5 mL of water added, and stirred for 1 hour. Solution was extracted with 10 mL of CH₂Cl₂, washed with water, dried over Na₂SO₄ and concentrated to yield **analog 118** (120 mg).

Example 21. **Synthesis of Analog 119:** Analog **114** (10 mg) was dissolved in 1.5 mL of acetone with 1.0 mL of 4N H₂SO₄, and contents stirred for 1.5 hours at RT. Then 10 mL of CH₂Cl₂ and 10 mL of water are added, extracted, and the organic layer recovered which was then washed with saturated NaHCO₃ and saline, dried over Na₂SO₄ and concentrated, and **analog 119** recovered **(analog 128** was a byproduct).

Example 22. **Synthesis of Analog** 120: **Analog 010** (50 mg), NaHCO₃ are dissolved in 10 mL of 1:1 mixture of water and ethanol, then NH₂NH₂ (0.5 mL added with stirring at RT for one hour. The solution was extracted with CH₂Cl₂ twice, the organic layer recovered, washed with water, then NaHCO₃ solution, dried over Na₂SO₄, and evaporated to yield **analog 120** (30 mg).

Example 23. **Synthesis of Analog 121: Analog 010** (50 mg) and NaCO₂CH₃ (75 mg) are dissolved in 10 mL of 1:1 mixture of water and ethanol 1:1, then semicarbazide hydrochloride salt (H₂NNHCONH₂·HCl, 50 mg) added, and stirred for 2 hours at RT. The solution was extracted with CH₂Cl₂ twice, the organic layer recovered, washed with water, then NaHCO₃ solution, dried over Na₂SO₄, and evaporated then chromatographed (5% methanol in ethyl acetate) to yield **analog 121.**

Example 24. **Synthesis of Analog 122: Analog 010** (50 mg) and NaCO₂CH₃ (75 mg) are dissolved in 5 mL of ethanol, then phenylhydrazide (50 mg) was added, stirred for 1 hour at RT. Then 5 mL of water was added, followed by extraction with ethyl acetate, washed with water, dried over Na₂SO₄ and concentrated and chromatographed (5% methanol in ethyl acetate) to yield **analog 122.**

Example 25. **Synthesis of Analog 123: Analog 010** (50 mg) and NaCO₂CH₃ (75 mg) are dissolved in 10 mL of 1:1 water and ethanol, then H₂NNHTS ( H₂NNHS(=O)₂(phenyl)methyl, 50 mg) was added, stirred for 2 hour at RT. Then 5 mL of water was added, followed by extraction with ethyl acetate, washed with water, dried over Na₂SO₄ and concentrated and chromatographed (5% methanol in ethyl acetate) to yield **analog 123.**

Example 26. **Synthesis of Analog 124: Analog 115** (15 mg) and NaOAc(15 mg) are dissolved in acetic anhydride (1 mL) and stirred for 2 hours, then sodium acetate (300 mg) was added with stirring for 1 hour. Then the mixture was chromatographed (10% ethyl acetate in hexane) to give **analog 124.**

Example 27. **Synthesis of Analog 125: Analog 010** (50 mg) and NaCO₂CH₃ (75 mg) are dissolved in 5 mL of ethanol, then the dinitrophenylhydrazide (50 mg) was added, stirred for 1 hour at RT. Then 5 mL of water was added, followed by extraction with ethyl acetate, washed with water, dried over Na₂SO₄ and concentrated and chromatographed (5% methanol in ethyl acetate) to yield **analog 125.**

Example 28. **Synthesis of Analog 126: Analog 011** (40 mg), hydroxylamine (20 mg), NaHCO₃ (50 mg) are dissolved in 10 mL of ethanol and water (1:1) then stirred at RT for 90 minutes. Then the mixture was extracted with water (10 mL) and ethyl acetate (20 mL), the organic layer washed with saturated NaHCO₃ then brine, dried over Na₂SO₄ and concentrated, then chromatographed (2:3 ethyl acetate:hexane) to give **analog 126.**

Example 29. **Synthesis of Analog 127: Analog 010** (100 mg) and NH₄Cl (1.5 equivalent) are dissolved in 1,4-dioxane (5mL) and water (0.2 mL), then NaCN added (1.3 equivalents), stirred for 1 hour at RT. Then ethyl ether (20 mL) was added, the organic layer recovered, washed with water, washed with brine, then dried over Na₂SO₄, then chromatographed (2:3 ethyl acetate: hexane) to yield **analog 127.**

Example 30. **Synthesis of Analog 128:** Analog **114** (10 mg) was dissolved in 1.5 mL of acetone with 1.0 mL of 4N H₂SO₄, and contents stirred for 1.5 hours at RT. Then 10 mL of CH₂Cl₂ and 10 mL of water are added, extracted, and the organic layer recovered which was then washed with saturated NaHCO₃ and saline, dried over Na₂SO₄ and concentrated, and **analog 128** recovered **(analog 119** was a byproduct).

Example 31. **Synthesis of Analog** **129:** Analog 001 (200 mg) was dissolved in anhydrous THF (10 mL) at RT then NaBH₄ (100 mg) was added slowly for 30 minutes. Reaction was quenched with 1 mL of water then extracted with ethyl acetate (10 mL), washed with saturated NaHCO₃, and dried over Na₂SO₄, then concentrated to yield **analog 129.** If need be the compound can be purified by chromatography (1:1 ethyl acetate: hexane).

Example 32. **Analog 141:** Analog **129** (200 mg) was dissolved in CH₂Cl₂ at RT, then 1,4-dimethyl but-2-ynedioate (1.1 equivalent) was added slowly and mixture allowed to react for one hour, then evaporated to yield **analog 141.** If need be the compound can be purified by chromatography (1:1 ethyl acetate: hexane).

Example 33. **Synthesis of Analog 142:** Analog **141** (100 mg) was dissolved in CH₂Cl₂ at RT then Dess-Martin Periodinane reagent (200 mg) added with stirring for 1 hour to yield **analog 142.** If need be the compound can be purified by chromatography (1:1 ethyl acetate: hexane).

Example 34. **Synthesis of Analog 146:** Analog **127** (35 mg, 0.117 mmol), DMAP (5 mg), and diimidazole (22 mg, 1.2 eq) were dissolved in anhydrous CH₂Cl₂ under an argon atmosphere, and stirred for 30 minutes. The solution was cooled to 20°C then tributyl tin hydride (Bu₃SnH, 0.6 mL) and azobis isobutylnitrite (4 mg) were added with stirring for 30 minutes. The mixture was filtered then chromatographed (1:10 ethyl acetate: hexane) to remove impurities and starting materials, then chromatographed (2:3 ethyl acetate: hexane) to yield **analog** 146.

Example 35. **Synthesis of Analog 147:** Irofulven (10 mg) was dissolved in 3 mL of acetone and 1 M H₂SO₄ solution (1:1) with stirring at RT and 2-Mercaptobenzothiazole (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 147.**

Example 36. **Synthesis of Analog 148:** Irofulven (10 mg) was dissolved in 3 mL of acetone and 1 M H₂SO₄ solution (1:1) with stirring at RT and 2-Mercaptobenzoxazole (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 Ethyl acetate: hexane) to give **analog 148.**

Example 37. **Synthesis of Analog 149:** Irofulven (10 mg) was dissolved in 4 mL of acetone and 1 M H₂SO₄ solution (1:1) with stirring at RT and thiol-imidazole (1 equivalent) was added, stirred for 24 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 149.**

Example 38. **Synthesis of Analog 150:** Irofulven (10 mg) was dissolved in 4 mL of acetone and 1 M H₂SO₄ solution (1:1) with stirring at RT and 2-mercapto-5-methylbenzimidazole (1 equivalent) was added, stirred for 12 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 150.**

Example 39. **Synthesis of Analog 151:** Irofulven (10 mg) was dissolved in 3 mL of acetone and 1 M H₂SO₄ solution (1:1) with stirring at RT and 1-phenyl-1,2,3,4-tetraazole-5-thiol (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 151.**

Example 40. **Synthesis of Analog 152:** Irofulven (10 mg) was dissolved in 3 mL of acetone and 1 M H₂SO₄ solution (1:1) with stirring at RT and 2-mercapto-5-nitro benzimidazole (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 152.**

Example 41. **Synthesis of Analog 153:** Irofulven (10 mg) was dissolved in 3 mL of acetone and 1 M H₂SO₄ solution (1:1) with stirring at RT and 1, 2, 4-Triazole-3-thiol (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 153.**

Example 42. **Synthesis of Analog 154:** Irofulven (10 mg) was dissolved in 3 mL of acetone and 1 M H₂SO₄ solution (1:1) with stirring at RT and 2-sulfanylpteridin-4-ol (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 154.**

Example 43. **Synthesis of Analog 155:** Irofulven (10 mg) was dissolved in 3 mL of acetone and 1 M H₂SO₄ solution (1:1) with stirring at RT and 4-(5-sulfanyl-1H-1,2,3,4-tetrazol-1-yl)phenol (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 155.**

Example 44. **Synthesis of Analog 156:** Irofulven (10 mg) was dissolved in 3 mL of acetone and 1 M H₂SO₄ solution (1:1) with stirring at RT and 4-(5-sulfanyl-1-1,2,3,4-tetrazol-1-yl)benzoic acid (1 equivalent) was added, stirred for 2 hours, then partitioned between ethyl acetate and water. The organic extract was washed with saturated NaHCO₃ and saline until neutral, dried over MgSO₄, concentrated then chromatographed (1:1 ethyl acetate:hexane) to give **analog 156.**

Example 45. **Synthesis of Analog 159:** Illudin S (300 mg) was dissolved acetic anhydride (6 mL) and stirred for 15 minutes, then sodium acetate (300 mg) was added with stirring for 1 hour. Water (6 mL) was added, ethyl acetate extraction performed, washed with sodium bicarbonate solution, dried over Na₂SO₄, concentrated then chromatographed (2:3 ethyl acetate:hexane) to give **analog 159.**

Example 46. **Synthesis of Analog 160:** Analog **159** (60 mg) was dissolved in dry CH₂Cl₂ (6 mL) under nitrogen at RT and glutaric anhydride (100 mg) with DMAP (20 mg) was added with stirring for 30 minutes. The solvent was removed, water added, extracted with CH₂Cl₂, washed with water, dried over Na₂SO₄, concentrated then chromatographed (2:3 ethyl acetate: hexane) to give **analog 160.**

Example 47. **Synthesis of Analog 161:** Dehydroilludin S (300 mg) was dissolved acetic anhydride (6 mL) and stirred for 15 minutes, then sodium acetate (300 mg) was added with stirring for 1 hour. Water (6 mL) was added, ethyl acetate extraction performed, washed with sodium bicarbonate solution, dried over Na₂SO₄, concentrated then chromatographed (2:3 ethyl acetate:hexane) to give **analog 161.**

Example 48. **Synthesis of Analog 162:** Dehydroilludin S (60 mg) was dissolved in dry CH₂Cl₂ (6 mL) under nitrogen at RT and glutaric anhydride (150 mg) with DMAP (50 mg) was added with stirring for 30 minutes. The solvent was removed, water added, extracted with CH₂Cl₂, washed with water, dried over Na₂SO₄, concentrated then chromatographed (2:3 ethyl acetate:hexane) to give **analog 162.**

Example 49. **Synthesis of Analog 163:** Analog 159 (20.25 mg), DMAP (20 mg) are dissolved in dry CH₂Cl₂ (6 mL) at 0°C under nitrogen atmosphere and stirred for 10 minutes. Then chloroacetyl chloride (0.2 mL) was added slowly and the mixture stirred for 30 minutes, warmed to RT with stirring over 1 5minutes. Then water (6 mL) was added, mixed, and then extracted with CH₂Cl₂. The organic layer was washed with saturated NaHCO₃ followed by a saline wash, dried over Na₂SO₄ then chromatographed (2:3 ethyl acetate: hexane) to yield **analog 163** (60% yield).

Example 50. **Synthesis of Analog 164:** Irofulven (50 mg), DMAP (40 mg) are dissolved in dry CH₂Cl₂ (6 mL) at 0°C under nitrogen atmosphere and stirred for 10 minutes. Then chloroacetyl chloride (0.2 mL) was added slowly and the mixture stirred for 30 minutes, warmed to RT with stirring over 1 5minutes. Then water (6 mL) was added, mixed, and then extracted with CH₂Cl₂. The organic layer was washed with saturated NaHCO₃ followed by a saline wash, dried over Na₂SO₄ then chromatographed (2:3 ethyl acetate: hexane) to yield **analog 164** (60% yield).

Example 51. **Synthesis of Analog 165:** Analog **164** (40 mg) was dissolved in dry CH₂Cl₂ (6 mL) at RT under nitrogen atmosphere and stirred for 10 minutes. Then 1 mL of morpholine was added drop wise, with stirring for 30 minutes. The reaction was diluted with water (6 mL), extracted with CH₂Cl₂ (12 mL). The organic layer was washed with saturated NaHCO₃ then washed with saline, dried over Na₂SO₄ and chromatographed (2:3 ethyl acetate: hexane) to yield **165** (35% yield).

Example 52. **Synthesis of Analog 166 and analog 167 (prepared together):** Analog **160** (30 mg) was dissolved in methanol (4 mL) at 0°C, and 1N H₂SO₄ (1 mL) was added with stirring for 1 hour. Water (6 mL) was added, extracted with ethyl acetate, washed with NaHCO₃ then a brine solution, dried over MgSO₄, concentrated and then chromatographed (1:1 ethyl acetate:hexane) to yield analogs **166** and **167** in equal amounts.

Example 53. **Synthesis of Analog 168:** Analog **162** (20 mg) was dissolved in methanol (5 mL) at 0°C and stirred for 10 minutes, then 1 mL of 1N H₂SO₄ in methanol was slowly added, followed by stirring for 30 minutes. Water was added, followed by an ethyl acetate extraction, washed with NaHCO₃ then a brine solution, dried over Na₂SO₄, concentrated then chromatographed (1:1 ethyl acetate:hexane) to yield **analog 168.**

Example 54. **Synthesis of Analog 169:** Dehydroilludin S (20 mg), DMAP (20 mg) are dissolved in dry CH₂Cl₂ (6 mL) at 0°C under nitrogen atmosphere and stirred for 10 minutes. Then chloroacetyl chloride (0.2 mL) was added slowly and the mixture stirred for 30 minutes, warmed to RT with stirring over 15 minutes. Then water (6 mL) was added, mixed, then extracted with CH₂Cl₂. The organic layer was washed with saturated NaHCO₃ followed by a saline wash, dried over Na₂SO₄ then chromatographed (2:3 ethyl acetate: hexane) to yield **analog 169** (60% yield).

Example 55. **Synthesis of Analog 176:** To a solution of **analog 00 9** (266 umol), Boc protected leucine amino acid (300 umol) and DMAP (dimethylaminopyridine, 110 umol) in CH₂Cl₂ (2.5 mL) at 0°C was added DCC (dicyclohexylcarbodiimide; 1.0M in CH₂Cl₂, 300 umol)/. The mixture was stirred for 35 minutes then 5 µL of water added to quench the reaction. The mixture was diluted with hexane and precipitate filtered off, solvent evaporated off and crude product chromatographed (2:1 hexanes-ethyl acetate) to give the desired Boc-protected derivative of 176 at 80% yield. The Boc group was removed by dissolving the Boc-protected derivative in a 1:1 mixture (2.0 mL) of 1,4-dioxane and 2M H₂SO₄, stirred for 18 hours, then partitioned between ethyl acetate and water. Aqueous layer was extracted with ethyl acetate and extracts discarded. Aqueous layer was neutralized with saturated NaHCO₃ and extracted again with ethyl acetate. Organic layer was washed with brine, dried with MgSO₃, solvent evaporated to yield the **analog 009** amino acid derivative. As the amine derivative was unstable over prolonged periods of time it can be converted to the very stable trifluoroacetate salt by dissolving in CH₂Cl₂ adding the equal molar amount of trifluoroacetic acid and concentrating to dryness.

Example 56. **Synthesis of Analog 178: Analog 009** (15 mg) was dissolved in CH₂Cl₂ (2.0 mL) under a nitrogen atmosphere at RT, succinic anhydride (1 equivalent) was added, followed by DMAP (10 mg) and stirring for 30 minutes. Solvent was removed and product recrystallized to give **analog 178.**

Example 57. **Synthesis of Analog 179:** To a solution of **Analog 009** (266 µmol), Boc protected glycine amino acid (300 umol) and DMAP (dimethylaminopyridine, 110 umol) in CH₂Cl₂ (2.5 mL) at 0°C was added DCC (dicyclohexylcarbodiimide; 1.0M in CH₂Cl₂, 300 umol)/. The mixture was stirred for 35 minutes then 5 µL of water added to quench the reaction. The mixture was diluted with hexane and precipitate filtered off, solvent evaporated off and crude product chromatographed (2:1 hexanes-ethyl acetate) to give the desired Boc-protected derivative of 179 at 80% yield. The Boc group was removed by dissolving the Boc-protected derivative in a 1:1 mixture (2.0 mL) of 1,4-dioxane and 2M H₂SO₄, stirred for 18 hours, then partitioned between ethyl acetate and water. Aqueous layer was extracted with ethyl acetate and extracts discarded. Aqueous layer was neutralized with saturated NaHCO₃ and extracted again with ethyl acetate. Organic layer was washed with brine, dried with MgSO₃, solvent evaporated to yield the **analog 009** amino acid derivative. As the amine derivative was unstable over prolonged periods of time it can be converted to the very stable trifluoroacetate salt by dissolving in CH₂Cl₂ adding the equal molar amount of trifluoroacetic acid and concentrating to dryness.

Example 58. **Synthesis of Analog 180:** Illudin M (50 mg) was dissolved in dry benzene (10 mL) under a nitrogen atmosphere, and vanadyl acetylacetonate (VO(acac)₂, 1.2 mg) was added. Then t-butyl hydroperoxide (t-BuO₂H, 0.5 mL) in benzene was added drop wise with stirring for 30minutes. A saturated solution of Na₂S₂O₃ was added (10 mL), then extraction with ethyl acetate, and the organic layer was dried over Na₂SO₄, concentrated then chromatographed) (1:1 ethyl acetate:hexane) to give **analog 180.**

Example 59. **Synthesis of Analog 181:** Analog **159** (40 mg) was dissolved in dry benzene (8 mL) under a nitrogen atmosphere, and vanadyl acetylacetonate (VO(acac)₂, 2 mg) was added. Then t-butyl hydroperoxide (t-BuO₂H, 0.5 mL) in benzene was added drop wise with stirring for 30 minutes. A saturated solution of Na₂S₂O₃ was added (10 mL), then extraction with ethyl acetate, followed by a brine wash, and the organic layer was then dried over Na₂SO₄, concentrated then chromatographed) (1:1 ethyl acetate:hexane) to give **analog 181.**

Example 60. **Synthesis of Analog 189:** To a solution of Irofulven (1.00 equivalent), maleimide (1.71 equivalent), triphenylphosphine (PPh₃, 1.71 equivalent) in 1.5 mL of THF at - 40°C, was added DEAD (diethylazodicarboxylate; 1.68 equivalent). The mixture was stirred for 30 minutes then water (20 µL) added to quench the reaction. The mixture was concentrated on a rotary evaporator and crude product was chromatographed on a silica column (10:3 hexanes: ethyl acetate) to yield an orange compound (20% yield).

Example 61. **Synthesis of Analog 190:** To a solution of **analog 009** (6-hydroxy-n-propylacylfulvene - structure below, 1.00 equivalent), maleimide (1.23 equivalent), triphenylphosphine (PPh₃, 1.13 equivalent) in 2.5 mL of THF at -40°C, was added DIAD (diisopropylcarbodiimide; 1.44 equivalent). The mixture was stirred for 1 hour then water (10 µL) added to quench the reaction. The mixture was concentrated on a rotary evaporator and crude product was chromatographed on a silica column (5:1 → 10:3 hexanes:ethyl acetate) to yield an orange compound (15% yield).

Example 62. **Synthesis of Analog 196:** To a solution of **analog 009** (266 umol), Boc protected proline amino acid (300 umol) and DMAP (dimethylaminopyridine, 110 umol) in CH₂Cl₂ (2.5 mL) at 0°C was added DCC (dicyclohexylcarbodiimide; 1.0M in CH₂Cl₂, 300 umol)/. The mixture was stirred for 35 minutes then 5 µL of water added to quench the reaction. The mixture was diluted with hexane and precipitate filtered off, solvent evaporated off and crude product chromatographed (2:1 hexanes-ethyl acetate) to give the desired Boc-protected derivative of 196 at 80% yield. The Boc group was removed by dissolving the Boc-protected derivative in a 1:1 mixture (2.0 mL) of 1,4-dioxane and 2M H₂SO₄, stirred for 18 hours, then partitioned between ethyl acetate and water. Aqueous layer was extracted with ethyl acetate and extracts discarded. Aqueous layer was neutralized with saturated NaHCO₃ and extracted again with ethyl acetate. Organic layer was washed with brine, dried with MgSO₃, solvent evaporated to yield the **analog 009** amino acid derivative. As the amine derivative was unstable over prolonged periods of time it can be converted to the very stable trifluoroacetate salt by dissolving in CH₂Cl₂ adding the equal molar amount of trifluoroacetic acid and concentrating to dryness.

Example 63. **Synthesis of Analog 198:** Irofulven (26.3 mg, 107 umol), p-nitrophenol (16.2 mg, 116 umol) and PPh3 (30.8 mg, 117 umol) were dissolved in anhydrous THF (1.5 mL) at -40°C, the DEAD (25 µL, 160 umol) was added, followed by stirring for 30 minutes, then diluted with hexane. The precipitate was filtered off, solvent evaporated, and crude product chromatographed (6:1-> 2:1 hexane: ethyl acetate) to give **analog 198** as a yellow product (18.5 mg, 47%).

Example 64. **Analogs 199 and 200 (prepared together):** : Irofulven (25.2 mg, 102 umol), phenol (11.5 mg, 122 umol) and PPh₃ (29.1 mg, 117 µmol) were dissolved in anhydrous THF (1.0 mL) at -40°C, the DEAD (25 µL, 192 µmol) was added, followed by stirring for 30 minutes, then diluted with hexane. The precipitate was filtered off, solvent evaporated, and crude product chromatographed (6:1-> 3:1 hexane: ethyl acetate) to give **analog 199** (8.2 mg, 25%) and **analog 200** (14.6 mg, 44%) as a yellow products.

Example 65. **Synthesis of Analog 201** [6-(acetamidopropyl)acylfulvene]: To a solution of **analog 195** (49.1 umol) and water (20 µL in THF (0.5 ml) was added a solution of O-acetyl-2-(diphenylphosphino)phenol (39.0 umol) in THF (0.5 mL). The mixture was stirred for 3 days at RT then concentrated. The crude product was chromatographed (100% ethyl acetate) to yield 8.2 mg of **analog 201.**

Example 66. **Synthesis of Analog 202** ( i.e., **analog 211** linked to proline): Prepared via Staudinger ligation. To a solution of **analog 195** (94 umol) in THF (1.2 mL), water (40 µL) was added, the was added N-Boc-proline, 2-(diphenylphosphino)phenyl ester (101 µmol) in THF (0.8 mL). The mixture was stirred for 3days at RT then concentrated. The crude product was chromatographed (5:1 - >1:2 hexanes-ethyl acetate) to yield 31.4 mg (66.7 umol) of **analog 202** - Boc (71%). The **analog** 202-Boc was dissolved (66.7 umol) in dioxane (2.0 mL) and 2.0 mL of 2M H₂SO₄ was added, and the mixture was stirred overnight. Water and ethyl acetate was added, orange color appeared in the aqueous. The aqueous was extracted again with ethyl acetate and organic layer discarded. Sodium bicarbonate was added to aqueous until basic, re-extracted with ethyl acetate. The solution was dried with magnesium sulphate, concentrated to dryness, dissolved in CH₂Cl₂ and 8 mg of TFA added (1 drop). Analog **202** was obtained in an amount of 22.2 mg (69%).

Example 67. **Synthesis of Analog 203: Analog 208** (9.2 mg, 16.5 umol) was dissolved in CH₂Cl₂ (1.5 mL), 1 drop of anisole added, then 0.5 mL of trifluoro acetic acid for 15 minutes. The mixture was concentrated, dissolved in water, then re-extracted with CH₂Cl₂,and the orange color remains in the aqueous phase, which was concentrated to give **analog 203** as the orange colored TFA salt (10.0 mg).

Example 68. **Synthesis of Analog 204:** Although the Fmoc-Pro-OH would preferentially react with the primary hydroxyl group on Illudin S, the resulting ester linkage was not stable, as illudin S was recovered after storage in CDCl₃ for several days at RT. The secondary hydroxy group of illudin S was therefore used for coupling with peptides. The primary hydroxy group of illudin S first protected with a TBS group (TBSCl, Imidazole, and DMF, 92%) to produce **analog 204.**

Example 69. **Synthesis of Analog 205:** Analog **309** (20 mg, 0.050 mmol, 1 equiv.), triphenylphosphine (40 mg, 0.1525 mmol, 3 equiv.) was dissolved in THF (1 mL) at RT. After 20 hours a few drops of water was added and the mixture was heated up at 70 °C. After 5 hours the solution was cooled down and evaporated. The residue was chromatographed (hexane/EtOAc/Et₃N 4:1:0.1) to give **analog 205** (5.3 mg, 29%) as an oil.

Example 70. **Synthesis of Analog_206: Analog 205** (14 mg, 0.037 mmol, 1 equiv.) was dissolved in CH₃CN (0.5 mL) and pyridine (0.1 mL) at 0 °C. To this solution was added HF·Pyridine (7 µL, 0.245 mmol, 35 M, 6.6 equiv.). After 10 min K₂CO₃ (0.5 mL, 0.5 M) was added and this mixture was chromatographed (CH₂Cl₂/Methanol/Et₃N 5:0.5:0.1) to give analog **206** (10 mg, 68%) as an oil.

Example 71. **Synthesis of Analog_207 (211** -leucine): Prepared via Staudinger ligation. To a solution of **analog 195** (101 umol) in THF (1.0 mL), water (40 µL) was added, then was added N-Boc-leucine,2-(diphenylphosphino)phenyl ester (95.9 µmol) in THF (1.2 mL). The mixture was stirred for 6 days at RT then concentrated. The crude product was chromatographed (1:1 hexanes-ethyl acetate) to yield 27.3 mg of **analog 207** -Boc. The **analog 207**-Boc was dissolved (16 µmol) in CH₂Cl₂ with 3 drops of anisole, TFA was added (0.3 mL), and the mixture was stirred for 15 minutes then concentrated. The crude material was dissolved in water then extracted with CH₂Cl₂. The aqueous layer was recovered and concentrated to yield 17.4 mg of the **analog 207**TFA salt (87%).

Example 72. **Analog 208:** The TFA salt of **analog 196** (13.7 mg, 28.2 µmol) was dissolved in anhydrous DMF (2.5 mL), Boc-Serine-OH (9.6 mg, 47 umol) was added, ODHBT (13.0 mg, 79.4 umol), cooled to 0°C under a nitrogen atmosphere. Next EDC (15.1 mg) was added followed by NMM (10 µL) to adjust pH, and the mixture stirred at 0°C for 3 hours. The reaction was added to ethyl acetate/water mixture, and the orange product appeared in the organic layer. The aqueous layer was re-extracted with ethyl acetate, organic layers combined, washed with dilute NaHSO₄, water, saturated NaHCO₃, brine, then dried with MgSO₄. The organic layer was concentrated then chromatographed (1:3 hexane: ethyl acetate) to yield **analog 208** as an orange residue (63% yield).

Example 73. **Synthesis of Analog 209:** The TFA salt of **analog** 196 (12.5 mg, 25.7 µmol) was dissolved in anhydrous DMF (2.5 mL), Boc-Serine-Ser OH (88.6 umol) was added, ODHBT (33.9 mg, 205 umol), cooled to 0°C under a nitrogen atmosphere. Next EDC (142 umol) was added followed by NMM (10 µL) to adjust pH, and the mixture stirred at 0°C but allowed to gradually warm as the ice melts. The mixture was stirred a total of 16 hour then 1 mL water added followed by stirring for 50 minutes. The reaction was added to ethyl acetate/water mixture, and the orange product appeared in the organic layer. The aqueous layer was re-extracted with ethyl acetate, organic layers combined, washed with dilute NaHSO₄, water, saturated NaHCO₃, brine, and then dried with MgSO₄. The organic layer was concentrated then chromatographed (10:1 ethyl acetate: methanol) to give **analog 209** as an orange residue (5.9 mg, 36% yield).

Example 74. **Synthesis of Analog 210** (Ac-Hyp-Ser-Ser-Chg-Gln-Ser-Ser-Pro-O-(CH₂)₃-acylfulvene): To a mixture of **Analog 196** TFA salt (21.6 umol), the peptide Ac-Hyp-Ser-Ser-Chg-Gln-Ser-Ser-OH (30.3 umol), ODHBt (3,4,-dihydroxy-4-oxo-1,2,3-benzo-triazine-3-yl ester, 71.7 µmol) and NMM (N-methylmorpholine; 7.5 ul) in DMF (2.0 ml) at RT was added EDC (1-(3-dimethylaminopropyl)-3-ethylcarbodiimide hydrochloride, 68 µmol), the mixture stirred for 2 hours at RT, then diluted with 10 mL of water. Solution was directly chromatographed on a reverse phase C18 column (4:1 - > 2:1, water/acetonitrile gradient) to yield 69% of **analog 210.**

Example 75. **Synthesis of Analog 212** (Illudin M-proline) Illudin M (20 mg, 0.081 mmol, 1 equivalent), DMAP (1 mg, 0.008 mmol, 0.1 equiv.) and Fmoc-Pro-OH (33 mg, 0.097 mmol, 1.2 equiv.) were dissolved in CH₂Cl₂ (1mL) at 0°C, to which was added a CH₂Cl₂ solution of DCC (100 µL, 0.1 mmol, 1 M, 1.2 equiv.). The temperature of the mixture gradually rose to 5°C in 1.5 hours and then the mixture was filtered through a pad of Celite. The filtrate was concentrated and the residue was chromatographed (CH₂Cl₂/EtOAc 5:0.1-5:0.4) to give Illudin-M-proline-Fmoc protected **analog** (36 mg, 79%) as oil. The proton spectra of this oil showed that it was a mixture of two isomers (rotamers). And then this oil was dissolved in CH₂Cl₂ (4 mL) and treated with piperidine (1 mL) at 0°C. After 0.5 hours the solution was concentrated and the concentrate was chromatographed (CH₂Cl₂/Methanol 5:0.4) to give **analog 212** (15 mg, 54%) as oil.

Example 76. **Synthesis of Analog 213:** Analog **204** was coupled with Fmoc-Pro-H (DMAP, CH₂Cl₂, DCC, 0 °C, 85%), followed by deprotection of Fmoc group with 20% piperidine in CH₂Cl₂ to produce **analog 213** in 78% yield.

Example 77. **Synthesis of Analog 214** (Illudin S-Pro-Ser-Ser-HHOAc): The Fmoc protected peptide of H-Ser-Ser-OH was prepared by taking H-Ser-Ser-OH (50 mg, 0.26 mmol, 1 equiv.) and K₂CO₃ (89.7 mg, 0.65 mmol, 2.5 equiv.), dissolving in a mixture of water (4 mL) and dioxane (3 mL) at 0°C. To this solution Fmoc (67.3 mg, 0.26 mmol, 1 equiv.) was added in several portions. After 18 hours the mixture was acidified by KHSO₄ and the pH raised to 2.5. Then this mixture was taken up by ethyl acetate, which was washed with brine, dried, filtered and evaporated. The residue was chromatographed (CH₂Cl₂/Methanol/HOAc 5:1:0.1) to give 3.27 (75 mg, 70%) as a white solid. The **analog 212** (Illudin S tosylate-Pro) (42.8 mg 0.09 mmol, 0.9 equiv.), and the Fmoc protected H-Ser-Ser-OH peptide (41.2 mg, 0.1 mmol, 1 equiv.) were dissolved in DMF (1.5 mL) at 0°C. To this solution was added NMM (22 µL, 0.2 mmol, 2 equiv.), ODHBt (29.4 mg, 0.18 mmol, 1.8 equiv.), and EDC (31.1 mg, 0.16 mmol, 1.6 equiv.). The solution temperature was then raised to RT and kept for 3 hours before it was taken up by ethyl acetate. The mixture was then washed with saturated sodium bicarbonate and brine. It was then dried, filtered and evaporated. The residue was chromatographed (CH₂Cl₂/Methanol 5:0.3) to give **analog 214** (50.5 mg, 67%) as an oil.

Example 78. **Synthesis of Analog 215:** (Illudin S-Pro-Ser-Ser-Gln-Chg-Ser-Ser-Hyp-Ac) **Analog 204** was coupled with Fmoc-Pro-H (DMAP, CH₂Cl₂, DCC, 0 °C, 85%), followed by deprotection of Fmoc group with 20% piperidine in CH₂Cl₂ to produce **analog 213** in 78% yield. Peptide conjugate, **analog 215** was obtained from further coupling with hepta-peptide Ac-Hyp-Ser-Ser-Chg-Gln-Ser-Ser-OH (ODHBt, NMM, DMF, 0 °C, 47%).

Example 79. **Synthesis of Analog 216:** (Illudin M-Pro-Ser-Ser-Gln-Chg-Ser-Ser-Hyp-Ac). **Analog 212** was further coupled with the commercially available hepta-peptide Ac-Hyp-Ser-Ser-Chg-Gln-Ser-Ser-OH (ODHBt, NMM, DMF, EDC, 0°C) to yield **analog 216** at 33%. The low yield resulted from repeated chromatographic purification as the purity of the final raw product was estimated by HPLC to be only 70%.

Example 80. **Synthesis of Analog 217:** To a solution of Irofulven (1.00 equivalent), epsilon-maleimidocaproic acid (1.27 equivalent), DMAP (0.15 equivalent) in 1.0 mL of methylene chloride (CH₂Cl₂) at 0°C, was added DCC (dicyclohexylcarbodiimide; 1.27 equivalent) in methylene chloride (CH₂Cl₂). The mixture was stirred for 1.25 hours, diluted with hexane and precipitated was filtered. Residual solvent was evaporated off, and oil residue was chromatographed on a silica column (2:1 hexanes:ethyl acetate) to yield **analog 217,**an orange compound (77% yield).

Example 81. **Synthesis of Analog 218:** To a solution of Illudin M (1.00 equivalent), epsilon-maleimidocaproic acid (1.33 equivalent), DMAP (0.18 equivalent) in 1.0 mL of methylene chloride (CH₂Cl₂) at 0°C, was added DCC (dicyclohexylcarbodiimide; 1.33 equivalent) in methylene chloride (CH₂Cl₂). The mixture was stirred for 2.25 hours, diluted with hexane and precipitated was filtered. Residual solvent was evaporated off, and oil residue was chromatographed on a silica column (2:1 hexane : ethyl acetate) to yield **analog 218,** an orange compound (83% yield).

Example 82. **Synthesis of Analog 219:** Analog 204 (33.4 mg) was dissolved in 1.0 mL of anhydrous pyridine under a nitrogen atmosphere, then DMAP (5.1 mg) was added, followed by 4-fluorosulfonyl-benzoyl chloride (86.1 mg). The mixture was stirred for 90 minutes at RT The mixture was diluted with ethyl acetate, washed once with saturated copper sulfate solution, washed twice with water, then dried over MgSO₄, concentrated then chromatographed (20% ethyl acetate: hexane) to give **analog 219.**

Example 83. **Synthesis of Analog 221:** Prepared from **Analog 207** by coupling with Mu-His-Ser-Ser-Lys(Fmoc)-Leu-Gln-OH in DIC/HOBt for 5 minutes, then 5% piperidine/DMF for 1 minute. Followed by TFA quenching to yield **analog 221** at 21% yield.

Example 84. **Synthesis of Analog 222:** : Illudin M (63 mg) was dissolved in 1.0 mL of anhydrous pyridine under a nitrogen atmosphere, then DMAP (6.4 mg) was added, followed by 4-fluorosulfonyl-benzoyl chloride (86 mg). The mixture was stirred for 35 minutes at RT then chromatographed (20% ethyl acetate: hexane) to give **analog 222** (70.9 mg).

Example 85. **Synthesis of Analog 223:** The disulfhydryl peptide CNGRC was first converted to a cyclic disulfide peptide by dissolving 355 mg in 3.0 mL DMSO, adding 9 mL of water, allowing to sit overnight at RT, followed by water removal on a rotoevaporator then DMSO removal under high vacuum. The TFA salt of **analog 179** (14.5 mg) was dissolved in DMF (2.0 mL) and the CNGRC disulfide peptide added (19.0 mg), 60 µL of DIPEA was added, followed by gradual addition of a solution of Py-BOP (19.6 mg) and HOBt (8.9 mg) in DMF (2.0 mL) over 150 minutes at RT. The reaction was stopped by adding two drops of TFA and water. The mixture was applied to a reverse phase column and **analog 223** was eluted with acetonitrile: water (1:4).

Example 86. **Synthesis of Analog 224: Analog 001** (116 mg) was dissolved in ethanol (4.0 mL) with stirring, hydroxylamine hydrochloride (84.2 mg) added, Sodium acetate (233 mg) added, then refluxed for 70 minutes at 85°C. The ethanol was removed, then ethyl acetate (10 mL) added to dissolve crude product, then water (10 mL) added, the organic layer was washed with brine, dried over Na₂SO₄, concentrated then chromatographed (20% ethyl acetate: hexane) to give **analog 224** (63.7 mg, 54% yield).

Example 87. **Synthesis of Analog 225:** Illudin S (439 mg) was dissolved in ethanol (15 mL) with stirring, hydroxylamine hydrochloride (233 mg) added, sodium acetate (933 mg) added, then refluxed for 130 minutes at 85°C. The solution was cooled to RT, filtered, ethanol was removed, then ethyl acetate (30 mL) added to dissolve crude product, then water (30 mL) added, the organic layer was washed with brine, dried over Na₂SO₄, concentrated then chromatographed (30% - >50%, acetone: hexane) to give **analog 225** (372 mg, 80% yield).

Example 88. **Synthesis of Analog 226:** Irofulven (37.6 mg) was dissolved with stirring in CH₂Cl₂,elaidic acid (180 mg. 1.3 equivalents) added, DMAP (15 mg) added, cooled to 0°C, then DCC (180 µL) in CH₂Cl₂ (640 µL) added. Reaction mixture stirred at 0°C for 1 hour, then additional DCC (120 µL) added, and stirred for 2 more hours. Mixture chromatographed (20% ethyl acetate: hexane) to give **analog 226** as a yellow oil (50.5 mg, 48% yield).

Example 89. **Synthesis of Analog 227:** Analog **009** (87 mg) was dissolved with stirring in CH₂Cl₂, elaidic acid (108 mg) added, DMAP (15.4 mg) added, cooled to 0°C, then DCC (0.5 mL) in CH₂Cl₂ (1.5 mL) added. Reaction mixture stirred at 0°C for 3 hours, then the mixture directly chromatographed (20% ethyl acetate: hexane) to give **analog 227** as a yellow oil (105 mg, 61% yield).

Example 90. **Synthesis of Analog 228:** Illudin S (86 mg) was dissolved with stirring in CH₂Cl₂,elaidic acid (202 mg) added, DMAP (15.4 mg) added, cooled to 0°C, then DCC (1.0 mL ) in CH₂Cl₂ (3.0 mL) added. Reaction mixture stirred at 0°C for 3 hours, then the mixture directly chromatographed (20% ethyl acetate: hexane) to give **analog 228** as a yellow oil (198 mg, 77% yield).

Example 91. **Synthesis of Analog 229:** The elaidic ester of 0-diphenylphosphine phenol was first prepared by dissolving with stirring in 3.0 mL of CH₂Cl₂ the O-diphenylphosphine phenol (91.3 mg), elaidic acid (94.5 mg, 1 equivalent), DMAP (9.4 mg). The solution was cooled to 0°C then DDC (0.44 mL, 1.0 M in CH₂Cl₂) was added with stirring for 3.5 hours. The precipitate was filtered off and discarded. The elaidic ester was chromatographed and concentrated to dryness then dissolved in THF (1.0 mL). **Analog 195** (26.1 mg) was dissolved in THF (1.0 mL) and water (80 µL) added. The elaidic ester solution was slowly added to the **analog 195** solution with stirring, and reacted for 22 hours at RT. The mixture was directly chromatographed (30% acetone: hexane) to give **analog 229** (22.2 mg, 47% yield).

Example 92. **Synthesis of Analog 230: Analog 308** (22 mg) was dissolved in anhydrous CH₂Cl₂ (1.5 mL), diisopropylethylamine (20 µL) added, and the mixture cooled to 0°C, then methylsulfonyl chloride added (15 µL), mixture stirred at 0°C for 1 hour, and allowed to warm to RT while being stirred for an additional hour. The mixture was chromatographed (30% ethyl acetate in hexane) to yield **analog 230** (35% yield).

Example 93. **Synthesis of Analog 231: Analog 308** (16 mg) was dissolved in anhydrous CH₂Cl₂ (1.5 mL), diisopropylethylamine (20 µL) added, and the mixture cooled to 0°C, then tosyl chloride added (18.4 mg), mixture stirred at 0°C for 1 hour, and allowed to warm to RT while being stirred for an additional 3 hours. The mixture was chromatographed (30% ethyl acetate in hexane) to yield **analog 231** (8.6 mg).

Example 94. **Synthesis of Analog 240: Analog 232** (25.1 mg) was dissolved in anhydrous CH₂Cl₂ (2.0 mL), 15 µL of acetic anhydride added, and the mixture cooled to RT, then DMAP added (5 mg), and stirred for 25 minutes. The mixture was partially concentrated then chromatographed (30% ethyl acetate in hexane) to yield **analog 240** (26.6 mg, 93% yield).

Example 95. **Synthesis of Analog 254: Analog 009** (51.4 mg), 4-carboxybenzene sulfonamide (59.4 mg), and DCC (39.6 mg) were dissolved in anhydrous DMF (1.0 mL) at RT, stirred, then DMAP (15 mg) added. The mixture was stirred for 2 hours at RT then solid material was filtered off. The mixture was then chromatographed (1:1 ethyl acetate: hexane) to give **analog 254** (38.6 mg, 45% yield).

Example 96. **Synthesis of Analog 255: Analog 009** (244.3 mg) and sulfamoyl chloride (157 mg) were dissolved in anhydrous DMAP (2.0 mL) at RT, and stirred for 3.5 hours. The mixture was concentrated under high vacuum then chromatographed (30% ethyl acetate in hexane) to give **analog 255.**

Example 97. **Synthesis of Analog 259: Analog 255** (64.7 mg), (diacetoxyiodo)benzene (64.7 mg), dirhodiumtetraacetate or Rh₂(OAc)₄ and magnesium (16.8) dissolved in 5.0 mL of CH₂Cl₂ are heated to 70°C and stirred for 7 hours. The mixture was filtered, concentrated, then chromatographed (1:1 ethyl acetate: hexane) to give **analog 259.**

Example 98. **Synthesis of Analog 262 and 263 (prepared together): Analog 025** (44.7 mg) was dissolved in methanol (1.0 mL), Oxone^{®} reagent (246 mg, 3 equivalents) was dissolved in water (1.0 mL). The oxone solution was slowly added to the methanol solution with stirring at RT for 3.5 hours, then an additional amount of Oxone reagent added followed by stirring for 1.5 hours. Then 2 mL of saturated sodium sulfite solution was added, followed by ethyl acetate extraction, dried over Na₂SO₄, concentrated then chromatographed (1:1 Ethyl acetate:hexane) to yield first **analog 263** (21.4 mg) and then **analog 262** (14.3 mg).

Example 99. **Synthesis of Analog 284 and 289 (prepared together): Analog 034** (174 mg) and uracil (227 mg) are dissolved in CH₂Cl₂ with stirring and the mixture cooled to 0°C. Then SnCL₄ (148.8 µL) was slowly added. The mixture was stirred at 0°C for 80 minutes, then concentrated, chromatographed (2->5% methanol: CH₂Cl₂) to give **analog 284** (68.9 mg, 33% yield) and **analog 289** (21.6 mg, 10% yield).

Example 100. **Synthesis of Analog 285: Analog 034** (25 mg) was dissolved in ethanol, and O-(tert-Butyldimethylsilyl) hydroxylamine (25 mg) was added followed by stirring for 2 hours at RT. The secondary amine intermediate (9 mg) was recovered by chromatography (30% ethyl acetate:hexane), dissolved in CH₂Cl₂, and reacted with sulfamoyl chloride (ClSO₂NH₂, 5 mg) and DABCO (2 mg) with stirring for one hour, then additional sulfamoyl chloride (6 mg) was added with stirring for another 1.5 hours. The TPS blocked product was recovered by chromatography (30% ethyl acetate:hexane), and the TPS group was removed in THF by adding TBAF (Tetra-n-butylammonium fluoride).The TPS group can also be removed by dissolving the TPS product in pyridine and THF at 0°C, then adding HF-pyridine overnight. After TPS deblocking the mixture was chromatographed (50% ethyl acetate: hexane) to give **analog 285.**

Example 101. **Synthesis of Analog 286** and **analog 287 (prepared together):** The ketone groups on 5-fluorouracil are first blocked with TMS groups by dissolving 5-fluorouracil (610 mg) and (NH₄)₂SO₄ in HMDS (10 mL) under a nitrogen atmosphere. The solution was refluxed at 142°C for 2.5 hours, cooled to 60°C and excess HMDS distilled off, then concentrated to dryness under high vacuum. **Analog 034** (180 mg) and the di-TMS 5-fluorouracil are dissolved in CH₂Cl₂ (5.0 mL) with stirring and the mixture cooled to 0°C. Then SnCL₄ (120 µL) was slowly added drop wise. The mixture was stirred at 0°C for 3.5 hours, then concentrated, chromatographed (80% ethyl acetate: hexane) to give **analog 286** (18.9 mg, 9 % yield) and **analog 287** (84 mg, 38% yield).

Example 102. **Synthesis of Analog 289:** See the preparation of **analog 284** for the preparation of **analog 289 (284** and **289** prepared simultaneously then separated by chromatography).

Example 103. **Analogs 299 and 300 (prepared together):** Analogs **299** and **300** are prepared in equal amounts from Illudin S using the Mitsunobu reaction. Illudin S was directly reacted with HN₃ (PPh3, DEAD, benzene) at 0°C under nitrogen for 45 minutes. Mitsunobu, O. Synthesis 1:1-28, 1981.

Example 104. **Synthesis of Analog 301:** Irofulven (31.6 mg, 0.128 mmol), 5-benzoylvaleric acid (35.8 mg, 0.174 mmol) and DMAP (4.7 mg) was dissolved in CH₂Cl₂ (2 mL) under a nitrogen atmosphere, cooled to 0°C, the DCC added (170 µL of 1.0M solution in CH₂Cl₂). The mixture was stirred for 60 minutes then diluted with hexane (10 mL) and filtered. The organic layer was further diluted with CH₂Cl₂, washed with water, then saturated NaHCO₃ then brine, dried with MgSO₄, concentrated, then dissolved in CH₂Cl₂, filtered and chromatographed (10:3 hexane:ethyl acetate), appropriate fractions collected, pooled, concentrated then chromatographed (3:1 hexane:ethyl acetate) to give **analog 301** (23.2 mg, 42% yield).

Example 105. **Analogs 302** and **analog 303 (prepared together):** Illudin S (100 mg, 0.378 mmol) was benzoylated by dissolving in pyridine (1.0 mL) then adding 3, 5-dintirobenzoyl chloride (110 mg, 0.5 mmol) at RT and stirring for 24 hours. The mixture was poured onto crushed ice then extracted with CH₂Cl₂ (10 mL), which was washed twice with water (20 mL). The organic layer was dried over Na₂SO₄ and concentrated to yield analogs **302** and **303.** The two analogs can be separated by column chromatography (1:1 hexane:ethyl acetate).

Example 106. **Synthesis of Analog 304: Analog 009** (84.6 mg) was dissolved in anhydrous CH₂Cl₂ (3.0 mL), DCC added (81.2 mg), mixture cooled to 0°C, propiolic acid (35 µL) added, then the reaction started with DMAP (15 mg), stirred and allowed to warm to RT over 1 hour. The mixture was filtered to remove solids then chromatographed (30% ethyl acetate in hexane) to give **analog 304** (60% yield).

Example 107. **Synthesis of Analog 305: Analog 009** (99.1 mg) was dissolved in anhydrous CH₂Cl₂ (3.0 mL), pyridine (150 µL) added, then p-nitrophenylchloroformate and stirred for 3.5 hours at RT. The mixture was concentrated, hexane (20 mL) added, and precipitate filtered before chromatographing (50% ethyl acetate in hexane) to give **analog 305** (50% yield).

Example 108. **Synthesis of Analog 306: Analog 009** (244 mg) was dissolved in anhydrous CH₂Cl₂ (4.0 mL), tosyl chloride (181 mg) added, the mixture cooled to 0°C, to which an aliquot of pyridine (80 µL) was added. The mixture stirred at 0°C for 1 hour, and allowed to warm to RT while being stirred for an additional 20 hours. The mixture was concentrated then chromatographed (50% ethyl acetate in hexane) to yield **analog 306.**

Example 109. **Synthesis of Analog 307:** A solution of 1.0 M N₃H in benzene was first prepared by mixing 654 mg N₃H, 0.65 mL water, in 10 mL of benzene. The mixture was cooled to 0°C, 0.5 mL of concentrated H₂SO₄ added, and allowed to warm slowly to RT and then stirred for 80 minutes. Next PPh₃ (590 mg) was dissolved in anhydrous THF (1.5 mL) and cooled to 0°C. Then 2.1 mL of N₃H 1.0 M solution was added, followed by DEAD (0.475 mL) then Illudin S (282 mg in 1.0 mL anhydrous THF). The mixture was stirred for 3 hours at 0°C, warmed, concentrated, followed by chromatography (30% ethyl acetate in hexane) to give **analog 307.**

Example 110. **Synthesis of Analog 308: Analog 307** (100 mg) was dissolved in anhydrous THF (3.0 mL) at RT and PPH3 added **(306** mg, 3 equivalents). The mixture was stirred for 5 hours at RT, then the reaction stooped by adding water (0.15 mL). The mixture was heated to 85°C for 30 minutes, then concentrated and chromatographed (20% methanol in ethyl acetate) to give **analog 308.**

Example 111. **Synthesis of Analog 309: Analog 204** was reacted with HN₃ (DEAD, THF) to yield the azide **analog 309** at 68% yield.

Example 112. **Synthesis of Analog 310:** Irofulven (42.9 mg), 4-carboxybenzene sulfonamide (41.4 mg), and DCC (38.4 mg) were dissolved in anhydrous DMF (1.0mL) at RT, stirred and then DMAP (10 mg) added. The mixture was stirred for 75 minutes at RT then solid material was filtered off. The mixture was then chromatographed (1:1 ethyl acetate: hexane) to give **analog 310** (40% yield).

Example 113. **Synthesis of Analog 311:** Illudin M (32.4 mg), 4-carboxybenzene sulfonamide (39.7 mg), and DCC (24.4 mg) were dissolved in anhydrous DMF (1.0mL) at RT, stirred, then DMAP (15 mg) added. The mixture was stirred for 75 minutes at RT, allowed to warm to RT, then stirred for 22 hours. The solid material was filtered off and the mixture was then chromatographed (1:1 ethyl acetate: hexane) to give **analog 311** (35% yield).

Example 114. **Synthesis of Analog 312:** Irofulven (1.18 grams) was dissolved in anhydrous CH₂Cl₂ (4.0 mL), tosyl chloride (1.1 equivalent) added, the mixture cooled to 0°C, then pyridine (0.4 mL) added. The mixture stirred at 0°C for 1 hour, and allowed to warm to RT while being stirred for an additional 3 hours. The mixture was concentrated then chromatographed (50% ethyl acetate in hexane) to yield **analog 312.**

Example 115. **Synthesis of Analog 313: Analog 308** (31 mg) was dissolved in anhydrous CH₂Cl₂, cooled to 0°C, with stirring then diisopropylethylamine added (45 µL), then fluorophenylsulfonyl chloride added (36 µL) for 3 hours at 0°C. Mixture was directly chromatographed (20% ethyl acetate: hexane) to give **analog 313** (23.3 mg).

Example 116. **Synthesis of Analog 314: Analog 009 was** dissolved in anhydrous CH₂Cl₂ (4.0 mL), tosyl chloride (1.1 equivalent) added, the mixture cooled to 0°C, then pyridine (0.4 mL) added. The mixture stirred at 0°C for 1 hour, and allowed to warm to RT while being stirred for an additional 3 hours. The mixture was concentrated then chromatographed (50% ethyl acetate in hexane) to yield **analog 314.**

Example 117. **Synthesis of Analog 315:** Irofulven was dissolved in a solution of 2,5 dimethylpyrrole (4 fold excess molar solution) in 5 mL of dry CH₂Cl₂ at -78°C . Boron trifluoride (equivalent molar amount to the irofulven) was slowly added with stirring. The reaction was allowed to stir for 2 more hours at -78°C, then water slowly added. The mixture was extracted twice with 2 fold equivalent volumes of ethyl acetate, the organic extracts combined, washed with saturated NaHCO₃, water, brine, then dried over MgSO₄. The solution was concentrated under vacuum until a red residue remained, which was chromatographed on silica gel (50% ethyl acetate in hexane) to yield **analog 315** (30% yield).

Example 118. **Synthesis of Analog 316: Analog 316** was prepared by dissolving Illudin S (20 mg) in pyridine (0.5 mL) and then 4-fluorosulfonylbenzoly chloride (equivalent molar amount) was added to the mixture in an ice bath. The solution was allowed to warm slowly and then react overnight. The liquid was then removed under reduced pressure until a crude residue remained. Rather than recrystallize from chloroform, the residue was instead chromatographed on a standard silica gel column using hexane-ethyl acetate (1:1). The mono-adduct **(analog 316),** a di-adduct and a small amount of unreacted Illudin S were recovered in separate eluates.

Example 119. **N₃H 1.0 M Solution:** A solution of 1.0 M N3H in benzene was first prepared by mixing 654 mg N₃H, 0.65 mL water, in 10 mL of benzene. The mixture was cooled to 0°C, 0.5 mL of concentrated H₂SO₄ added, and allowed to warm slowly to RT and then stirred for 80 minutes.

Example 120. **Synthesis of Analog 193:** Irofulven (221 mg, 0.897 umol) was dissolved in anhydrous THF (1.5 mL), then PPh₃ (261 mg, 0.995 umol) was added, then 1.0 M N₃H solution (1.0 mL, 1.0 mmol) under nitrogen atmosphere. The solution was cooled to -40°C, and then DIAD (0.21 mL, 1.013 umol) added and stirred for 30 minutes at 0°C then diluted with hexane, and filtered to remove precipitate. The mixture was concentrated then chromatographed (30% ethyl acetate: hexane) to give **analog 193** (171 mg, 71%).

Example 121. **Synthesis of Analog 195: Analog 009** (31.9 mg, 116 umol) was dissolved in anhydrous THF (3.0 mL), then PPh₃ (33 mg, 126 umol) was added, then 1.0 M N₃H solution (0.3061 mL) under nitrogen atmosphere. The solution was cooled to 0°C, DIAD (30 µL, 145 umol) added and stirred for 30 minutes at 0°C then water (5 µL) was added to destroy the PPh₃. The mixture was concentrated then chromatographed (30% ethyl acetate: hexane) to give **analog 195** (24.9mg, 72%).

Example 122. **Synthesis of Analog 317: Analog 219** (33 mg) was dissolved in anhydrous acetonitrile (0.5 mL) and pyridine (0.1 mL) at 0°C under nitrogen. Then add HF-pyridine solution (7 uL, 0.245 mmol, 35M, 6.6 equiv). After 10 minutes K₂CO₃ was added (0.5 mL, 0.5M) and the mixture concentrated then chromatographed using CH₂Cl₂, methanol, triethylamine (5: 0.5 : 0.1) to yield **analog 317.**

Example 123. **Synthesis of Analog 318:** Illudin S (30 mg) was dissolved in 1.0 mL of anhydrous pyridine under a nitrogen atmosphere, then DMAP (5.0 mg) was added, followed by excess 4-fluorosulfonyl-benzoyl chloride (190 mg). The mixture was stirred for 90 minutes at RT then diluted with5.0 mL of ethyl acetate, washed once with saturated copper sulfate solution, washed twice with water, then dried over MgSO₄, concentrated then chromatographed (20% ethyl acetate: hexane) to give **analog 318.**

Example 124. **Synthesis of Analog 332: Analog 034** (200 mg) was dissolved in ethanol (95%) at RT, then 300 mg of O-methylhydroxylamine hydrochloride (NH₂OMe HCl; Note O-methyl not the N-methyl compound) was added with sodium acetate (300 mg), stirred for 6 hours, concentrated, then chromatographed (15% ethyl acetate/hexanes) to yield the **analog 332.**

Example 125. **Synthesis of Analog 333:** Prepared by dissolving **analog 159** and p-nitrophenylchloroformate in anhydrous CH₂Cl₂ under nitrogen for 5 hours at 0°C with 1.5 equivalents of DIPEA and 1.0 equivalents of DMAP. The CH₂Cl₂ was removed and **analog 333** was purified using a 7% ethyl acetate/hexane column. Yield was 70%.

Example 126. **Synthesis of Analog 334:** Prepared by dissolving **analog 159** and p-nitrophenylchloroformate in anhydrous CH₂Cl₂ under nitrogen at 0°C with 1.5 equivalents of DIPEA and 1.0 equivalents of DMAP. After 5 hours the Boc-N,N'-dimethylaminoethane was added and the mixture kept at 0°C under nitrogen for 2 hours then allowed to warm up to RT and react overnight. The CH₂Cl₂ was removed and **analog 334** purified using a 15% ethyl acetate/hexane column.

Example 127. **Synthesis of Analog 335: Analog 334** (19 µmole) was dissolved in 1 mL of methanol and then sodium methoxide in methanol (0.5 M, 100 µmole) was added at RT with stirring for 30 minutes. The reaction was quenched by addition of excess acetic acid (40 µmole). The methanol was removed and **analog 335** purified using a 25% ethyl acetate/hexane column.

Example 128. **Synthesis of Analog 336: Analog 002** (200 mg) was placed in a dry round bottom, add 10 mL anhydrous CH₂Cl₂, stir at 0°C under nitrogen, add aluminum trichloride (AlCl3). The mixture will turn dark orange, stir additional 10 min at 0oC, add 20 mL of ethylene oxide/ CH₂Cl₂ solution at 0°C, stir for 30 minutes at 0°C. Wash twice with 10% HCl/ice cold water (2 x 50 mL), then twice with water (2 x 25 mL), dry over Na₂SO₄, concentrate under reduced pressure and chromatograph using ethyl acetate/hexane mixture.

Example 129. **Synthesis of Analog 337: Analog 270** (244 mg) was dissolved in 5.0 mL of CH₂Cl₂ and DABCO added (153 mg), and solution cooled to 0°C. Then p-nitrophenylchloroformate (162 mg) was added with stirring for 3 hours at 0°C. Mixture was concentrated and chromatographed (50% ethyl acetate 50% hexanes) to yield 201 mg of **analog** 337 as a yellow liquid.

Example 130. **Synthesis of Analog 338: Analog 211** (82 mg) was dissolved in 5.0 mL of CH₂Cl₂ and DABCO added (42 mg), and solution cooled to 0°C. Then p-nitrophenylchloroformate (51 mg) was added with stirring for 5 hours at 0°C. Mixture was concentrated and chromatographed (50% ethyl acetate 50% hexanes) to yield 63 mg of **analog 338** as a yellow liquid.

Example 131. **Synthesis of Analog 339:** Illudin S is dissolved in anhydrous CH₂Cl₂ (4.0 mL), tosyl chloride (1.1 equivalent) added, the mixture cooled to 0C, then pyridine (400 uL) added. The mixture stirred at 0C for 1 hour, and allowed to warm to RT while being stirred for an additional 3 hours. The mixture is concentrated then chromatographed (50% ethyl acetate in hexane) to yield **analog 339.**

Example 132. **Synthesis of Analog 340: Analog 270** (146 mg), DABCO (90 mg) was dissolved in DMF/ CH₂Cl₂ (1:3; 5.0 mL), cooled to 0°C, then methane sulfonyl chloride (55 uL; Cl- SO₂-CH₃) added with stirring for 2 hours at 0°C, concentrated then chromatographed using ethyl acetate/hexane= (1:1) to yield **analog 340** as a yellow liquid.

Example 133. **Synthesis of Analog 347:** Illudin S (35 mg) was dissolved in 1.0 mL of anhydrous pyridine under a nitrogen atmosphere, then DMAP (5.0 mg) was added, followed by 4-sulfamidobenzoyl chloride (90 mg). The mixture was stirred for 90 minutes at RT then diluted with ethyl acetate, washed once with saturated copper sulfate solution, washed twice with water, then dried over MgSO₄, concentrated, then chromatographed (20% ethyl acetate: hexane) to give **analog 347.**

Example 134. **Synthesis of Analog 348:** Illudin M (15 mg) was dissolved in 1.0 mL of anhydrous pyridine under a nitrogen atmosphere, then DMAP (3.0 mg) was added, followed by 4-sulfamidobenzoyl chloride (33 mg). The mixture was stirred for 90 minutes at RT then diluted with ethyl acetate, washed once with saturated copper sulfate solution, washed twice with water, then dried over MgSO₄, concentrated then chromatographed (20% ethyl acetate: hexane) to give **analog** 348.

Example 135. **Synthesis of Analog 353: Analog 334** was dissolved in anhydrous CH₂Cl₂ at 0°C and TFA added to a final concentration of 40% for 40 minutes to remove the Boc protecting group. The reaction was stopped by adding additional anhydrous CH₂Cl₂ to dilute the final TFA concentration to 10%, then solvent and TFA removed using a roto-evaporator without heat. Next the residue was dissolved in peptide synthesis grade DMF (so no solvent amines are present) and commercially available **analog 388** was added at 0°C, followed by triethylamine (8.0 equivalents) with stirring overnight. After concentration the material was purified by preparative HPLC. Solvent A (aqueous) was 0.1%TFA in water. Solvent B (organic) was 60% acetonitrile, 39.9% water, and 0.1% TFA. HPLC gradient was initially 60% B increasing to 100% B over 45 minutes, with **analog 353** eluting at approximately 40 minutes.

Example 136. **Synthesis of Analog 356:** Dissolve **analog 334** in CH₂Cl₂ at 0°C, then add ice cold TFA to a final concentration of 25%, for 30 minutes to remove Boc group. Excess TFA removed by evaporating to dryness under vacuum, then add 0.5 ml CH₂Cl₂, evaporating to dryness under vacuum, repeat CH₂Cl₂ addition and evaporation a total of 3 times and keep on ice/cold the entire time. Add 1 ml dry DMF, add triethylamine (5 equiv), and keep on ice. Part B: Take Fmoc-triglycine-OH peptide (1.1 equiv) and dissolve in 3 mls dry DMF at RT, then add EDC (1.1 equiv) and NHS (N-hydroxysuccinimide, 1.1 equiv) to form the active ester, and cool on ice. Add solution B to A on ice, then allow to warm in chiller to RT overnight to produce 356 (yield ~ 60%).

Example 137. **Synthesis of Analog 357:** Prepared by dissolving **analog 359** in CH₂Cl₂ at 0°C, then adding ice cold TFA to a final concentration of 25%, for 30 minutes. Excess TFA removed by evaporating to dryness under vacuum, adding 0.5 ml CH₂Cl₂, evaporating to dryness under vacuum total of 3 times.

Example 138. **Synthesis of Analog 359:** Part A: Dissolve **analog 334** in CH₂Cl₂ at 0°C, then adding ice cold TFA to a final concentration of 25%, for 30 minutes to remove Boc group. Excess TFA removed by evaporating to dryness under vacuum, adding 0.5 ml CH₂Cl₂, evaporating to dryness under vacuum, repeat CH₂Cl₂ addition and evaporation a total of 3 times, and keep on ice/cold the entire time. Add 1 ml dry DMF, add triethylamine (5 equiv), and keep on ice. Part B: Take Boc-triglycine-OH peptide (1.1 equiv) and dissolve in 3 ml dry DMF at RT, then add EDC (1.1 equiv) and NHS (N-hydroxysuccinimide, 1.1 equiv) to form the active ester, and cool on ice. Add solution from Part B to solution from Part A on ice, then allow to warm in chiller to RT overnight to produce **analog 359.**

Example 139. **Synthesis of Analog 361:** Add **analog 159** and N,N'-Disuccinimidyl carbonate (1.1 equiv) in anhydrous dichloromethane, DIPEA (1.1 equiv) and DMAP (1.1 equiv), reflux for four hours.

Example 140. **Synthesis of Analog 362:** Illudin S and 1.2 equivalents of mesylate anhydride, and 1.2 equivalents of 2,4,6-collidine into dry CH₂Cl₂, use molecular sieves. Start the reaction at 0oC (on ice) then let sit overnight, gradually warming up to RT. The reaction mixture remained colorless, and **analog 362** was isolated as a white crystalline material using ethyl acetate/hexane chromatography.

Example 141. **Synthesis of Analog 363:** Parik-Doering reaction was used with **analog 383** in which pyridine sulfur trioxide was used in combination with DIPEA and DMSO under strict anhydrous conditions. At 0° C the reaction mix contained a minor amount of product compatible with **analog 383** when analyzed by LC/MS. When the reaction was precooled to -20° C, allowed to react with illudin S, and then slowly warmed to 0° C, **analog 383** was produced at ~60% yield. **Analog 383** was reacted with NH₂-Boc in the presence of TFA and triethylsilane under the same conditions that **analog 002** was reacted with NH₂-Boc to produce **analog 366.** However, the yields were very low at < 15%. **Analog 383** and 1.1 equivalent of NH₂-Boc were dissolved in anhydrous DCM with molecular sieves, 0.2 equivalents of acetic acid added (to produce the -C=N-HR intermediate), and allowed to sit overnight. In the morning sodium triacetoxyborohydride was added (4 equivalents) and the reaction allowed to proceed for 6 hours to produce **analog 389. Analog 389** was converted to the primary amine **analog 363** using standard Boc deprotection method.

Example 142. **Synthesis of Analog 365.** Illudin S (150 mg) was reacted with Dess Martin Periodinane reagent (1.1 equiv) for 60 minutes at RT in 15 mls CH₂Cl₂ (no molecular sieves are used as traces of water accelerate the reaction). Solvent removed and **analog 365** (110 mg) recovered using ethyl acetate/hexane chromatography.

Example 143. **Synthesis of Analog 366.** Add 5 mg of **analog 002** into a small vial with 50 uL of acetone, added BocNH₂ 6.5 mg (2.5 equiv), started reaction with 20 ul of 1 N H₂SO₄, allowed to go overnight at RT. The yield was >80%.

Example 144. **Synthesis of Analog 367: Analog 010** (80 mg) was dissolved in 3 ml of anhydrous acetonitrile with molecular sieves. Add 102 mg NH₂Boc (3 equiv), 140 ul Et₃SiH (3 equiv) stirred at RT for 30 minutes with sieves under nitrogen, added 63 uL of TFA (2.9 equiv). Within 30 minutes solution turning from yellow to red. Eventually turns dark brown (amine product). The solvent was removed, and compound isolated using ethyl acetate/hexane chromatography.

Example 145. **Synthesis of Analog 368:** Illudin S (185 mg, 0.522 mmol), DIPEA 329 mg/444 ul (5 equiv), was dissolved in 5 ml THF, add acetic anhydride 260 ul (5 equiv) and DMAP 62 mg (1 equiv) and monitor reaction at RT. To recover and purify use an acetone/hexane column (0% acetone, then 2, 4, 6% etc.).

Example 146. **Synthesis of Analog 369.** Illudin S (1.025 grams) and 385.1 mg of imidazole (1.46 equiv) into 8 mls dry DMF with molecular sieves and stirred for 25 minutes at RT under nitrogen, then 686 mg of TBDMSCl added (1.17 equiv) in two equal portions. The reaction was over in 20 minutes per TLC. Add saturated sodium bicarbonate to quench reaction, extracted ethyl acetate three times, organic phase recovered and washed with brine, and dried with anhydrous magnesium sulfate, then solvent removed under high vacuum and temperature of 45°C.

Example 147. **Synthesis of Analog 370: Analog 369** was dissolved in DMF then added to 10 mls of CH₂Cl₂, then 316 mg of sodium acetate added (1 equiv), stirred for RT for 15 minutes, then acetic anhydride added (1.02 equiv), and allowed to react overnight. The reaction may not progress unless DMAP was added (0.2 equiv). The next day additional sodium acetate (150 mg, 0.3 equiv) and acetic anhydride (185 ul; 0.5 equiv) and DMAP (0.2 equiv) added. The next day solvent removed under high vacuum and temperature of 45 °C.

Example 148. **Synthesis of Analog 371: Analog 368** was dissolved in excess 7N NH₃/methanol and allow to react slowly for 48 to 72 hours at 40°C then the primary acetate was selectively removed and **analog 371** was primarily produced with a yield of ~65% (illudin S was the other product at ∼35%).

Example 149. **Synthesis of Analog 372:** Dissolve 320 mg of #371 and 620 mg of Dess Martin (1.2 equiv) into 4 mls CH₂Cl₂ (no molecular sieves are used as traces of water accelerate the reaction), allowed to react for 45 minutes. Then added 3 mls of saturated bicarbonate, 3 mls of saturated Na₂S₂O₃ (to quench Dess Martin reagent), then 15 mls of diethyl ether. Wash the reaction mixture twice more with diethyl ether, dry with anhydrous sodium sulfate and then remove the ether. Store overnight then purified with column (elutes at 15% ethyl acetate/hexane).

Example 150. **Synthesis of Analog 373:** Place 4.5 mg of **analog 372** in 200 ul anhydrous CH₂Cl₂ with several molecular sieves. Add triethylsilane (3.0 equiv), TFA (2.9 equiv) and FmocNH₂ (3.0 equiv) in dry THF at RT for 6 hours under nitrogen. Purify by ethyl acetate/hexane gradient column. Yield ~40%.

Example 151. **Synthesis of Analog 374:** The Fmoc moiety of **analog 373** was removed using 4-methylpyridine (20% final) in DMF at RT for 30 minutes.

Example 152. **Synthesis of Analog 377:** Dissolve **analog 159** into dry CH₂Cl₂ with molecular sieves, add imidazole (1.1 equiv) and stir for 30 minutes at RT. Then 1.2 equiv of TBSCl (chlorotributyl silane) added. After 15 minutes add 0.3 equiv of additional TBSCl.

Example 153. **Synthesis of Analog 378:** 4.5 mg of **analog 372** in 200 ul DMF with molecular sieves under nitrogen. Add triethylsilane (3.0 equiv), TFA (2.9 equiv) and BocNH₂ (3.0 equiv) at RT for 6 hours under nitrogen. Purify by ethyl acetate/hexane gradient column. Yield ~40%.

Example 154. **Synthesis of Analog 379: Analog 359:** Part A: Dissolving **analog 334** in CH₂Cl₂ at 0°C, then adding ice cold TFA to a final concentration of 25%, for 30 minutes to remove Boc group. Excess TFA removed by evaporating to dryness under vacuum, adding 0.5 ml CH₂Cl₂, evaporating to dryness under vacuum total of 3 times and keep on ice/cold the entire time. Add 1 ml dry DMF, triethylamine (5 equiv), and keep on ice. Part B: Take Fmoc-triglycine-Val-cit-PNP peptide (1.1 equiv) and dissolve in 3 mls of dry DMF at 0°C. Add solution B to A on ice, then allow to warm in chiller to RT overnight to produce 379.

Example 155. **Synthesis of Analog 380:** Prepare from **analog 379** by removing Fmoc using 4-methylpyridine (20% final) in DMF at RT for 30 minutes.

Example 156. **Synthesis of Analog 381:** Prepare from **analog 377** using lithium hydroxide (LiOH) (1.0 equiv) in 10%methanol/THF at 4°C. One must have methanol otherwise LiOH acts as a nucleophile and removes the TBS, not the acetate protective group. After 1 hour then add additional 0.2 equiv LiOH, then after 30 minutes remove THF/methanol. Purify immediately using ethyl acetate/hexane column to prevent degradation.

Example 157. **Synthesis of Analog 382.** Add 0.5 equiv of Dess Martin periodinane reagent to **analog 381** at RT in CH₂Cl₂. Add another 0.5 equiv after 30 minutes, then another 0.2 equiv after 30 minutes. Added 3 ml saturated bicarbonate, 3 ml of saturated Na₂S₂O₃ (to quench Dess Martin reagent), then 15 mls of diethyl ether. Wash reaction mix 2 more times with diethyl ether, dry with anhydrous sodium sulfate and remove ether. The product can be stored overnight then purified with column.

Example 158. **Synthesis of Analog 383:** Prepared from **Analog 382** by treating with 1.5% HCl/methanol at RT for 60 minutes. If one sees a smear by TLC, then leave at 4°C overnight, and in the morning one will see a main product by TLC that was **analog 383.** Yield was poor as normally less than 25%.

Example 159. **Synthesis of Analog 384: Analog 382** was dissolved in 1.5 ml anhydrous acetonitrile, then add BocNH₂ (3.0 equiv), then triethylsilane (3.0 equiv), followed by TFA (2.9 equiv) for 5 hours at RT. Best to add TFA in aliquots so as to not remove the protecting silyl group. Recover product using 10% acetone/hexane column (not ethyl acetate column), but still the yield was low at ∼15%.

Example 160. **Synthesis of Analog 385:** Illudin S (10 mg) was dissolved in anhydrous methanol with molecular sieves; add 0.5 equiv of Indium (II) chloride, 2.0 equiv of triethylsilane). Let sit at RT overnight, remove methanol, and purify using an ethyl acetate/hexane column.

Example 161. **Synthesis of Analog 387:** Dissolve the boc- triglycine (20 mg/ml) in DMF, then add EDC (0.98 equiv) and pentafluorophenol (1.15 equiv) at RT for 2 hours. Evaporate with high vacuum at 40°C then add ethyl acetate. If solids present remove by filtration. Wash with saturated sodium bicarbonate solution to remove unreacted triglycine acid and unreacted pentafluorophenol, then wash with brine, dry and evaporate to a flaky white solid. (when an oil was obtained simply add diethyl ether to precipitate a flaky white solid).

Example 162. **Synthesis of Analog 389: Analog 383** and 1.1 equivalent of BocNH₂ are dissolved in anhydrous CH₂Cl₂ with molecular sieves, 0.2 equivalents of acetic acid added (to produce the -C=N-HR intermediate), and allowed to sit overnight. In the morning sodium triacetoxyborohydride was added (4 equivalents) and the reaction allowed to proceed for 6 hours to produce **analog 389.** Product was purified using an ethyl acetate/hexane column. The yield was ~25%.

Example 163: **Synthesis of Analog 392:** Part A: Dissolve **analog 334** in CH₂Cl₂ at 0°C, then adding ice cold TFA to a final concentration of 25%, for 30 minutes to remove Boc group. Excess TFA removed by evaporating to dryness under vacuum, adding 0.5 ml CH₂Cl₂, evaporating to dryness under vacuum, repeat CH₂Cl₂ addition and evaporation a total of 3 times, and keep on ice/cold the entire time. Add 1 ml dry DMF, add triethylamine (5 equiv), and keep on ice. Part B: Take the Azide N₃-triglycine-OH peptide (1.1 equiv) and dissolve in 3 ml dry DMF at RT, then add EDC (1.1 equiv) and NHS (N-hydroxysuccinimide, 1.1 equiv) to form the active ester, and cool on ice. Add solution from Part B to solution from Part A on ice, then allow to warm in chiller to RT overnight to produce **analog 392.**

Example 164: **Synthesis of Analog 394:** Dissolve **analog 235** into CH₂CL₂, chill on ice then add TFA to a final concentration of 10%, and allow to incubate on ice for 60 minutes. Solvent is removed using high vacuum, then the product is purified using an ethyl acetate/hexane column. The yield is ~90%.

Example 165: **Synthesis of Analog 397:** Into a 50 ml round bottom flask is placed 400 mg of Illudin S and then 20 ml of anhydrous DCM added along with molecular sieves. The reaction is allowed to stir at RT for 30 minutes to extract any water contained in the Illudin S. Then 1.06 ml of DIPEA is added with stirring for 15 minutes. Next the mixture is cooled to - 70°C, then 1.0 g of sulfur trioxide pyridine complex (Sigma Aldrich catalog #S7556) is dissolved into 1 ml of anhydrous DMSO containing molecular sieves at RT. The sulfur trioxide pyridine /DMSO oxidizing complex is slowly added to the Illudin S solution at -70°C with stirring. The reaction is allowed to slowly warm to -20°C, and maintained at that temperature for 15 minutes. The reaction is quenched with 10 ml of water followed immediately by 10 ml of ethyl acetate. Next 300 µl of 20% aqueous ammonia hydroxide is added whereupon a bright red color appears in the aqueous portion. The organic portion is removed, and the aqueous portion is extracted 3 more times with the ammonia hydroxide/ethyl acetate procedure. The organic phases are combined, washed with saturated bicarbonate, and then washed with saturated brine. Finally the organic phase is dried then the solvent removed. The product is purified using by silica gel chromatography using an ethyl acetate/hexane gradient to yield the intermediate primary aldehyde. Yield is approximately 30%. This primary aldehyde of Illudin S is then dissolved in anhydrous THF, then 2 equivalents of anhydrous sodium sulfate added, and the mixture cooled to 4°C under nitrogen. Then 3.0 equivalents of N-Boc Hydroxylamine are added. After 15 minutes, 3.5 µl of glacial acetic acid are added, followed by 6.0 equivalents of sodium triacetoxy borohydride. The reaction is allowed to proceed for 18 hours at 4°C under nitrogen. The mixture is filtered and the organic solvent removed by vacuum. The product is purified using silica gel chromatography with an ethyl acetate/hexane gradient to yield the hydroxylamine Boc derivative **analog 397.**

Example 166: **Synthesis of Analog 398: Analog 397** is dissolved in anhydrous THF, without molecular sieves, under nitrogen and cooled to 0°C. Then TFA is added to a final concentration of 35% for 25 minutes. The THF and TFA are removed by high vacuum without external heat to yield the hydroxylamine **analog 398** (final yield approximately 20%).

Example 167: **Synthesis of Analog 399:** Illudin S (700 mg) is dissolved in 70 ml of anhydrous DCM and 5.0 ml of peptide synthesis grade DMF at RT. Then molecular sieves are added and the mixture is stirred at RT for 30 minutes under nitrogen, and 2,4,6-trimethylpyridine (5.7 equivalents) is slowly added until the Illudin S is dissolved. The mixture is then cooled to - 4°C and sulfamoyl chloride (1.2 equivalents) is slowly added over a 15 minute period. The reaction is allowed to proceed for 4 hours at -4°C, then allowed to slowly warm and react overnight at RT for 16 hours. The DCM/DMF reaction mixture is washed with saturated sodium bicarbonate to remove residual sulfonic and hydrochloric acids. The remaining organic phase is dried then removed with high vacuum.. The product is purified using silica gel chromatography with an ethyl acetate/hexane gradient to yield **analog 399.** Yield is approximately 25%. The product should be stored at -20°C.

Example 168: **Synthesis of Analog 401:** Illudin S (500 mg) is dissolved in a mixture of 300 mg of sodium acetate and 6.0 ml of acetic anhydride. The reaction is allowed to proceed at RT with stirring and monitored. After 3 to 4 hours the reaction is quenched by adding 100 ml of saturated sodium bicarbonate solution. The aqueous solution is extracted four times with ethyl acetate and the organic fractions are combined, dried and removed by vacuum. The desired mono-acetate product is separated from the undesired residual Illudin S, di-acetate and secondary mono-acetate contaminating compounds by silica gel chromatography using an ethyl acetate/hexane gradient. The recovered mono-acetate product is dissolved in anhydrous CH₂Cl₂ under nitrogen at O°C with 1.15 equivalents of p-nitrophenylchloroformate. Next 1.5 equivalents of DIPEA and 1.0 equivalents of DMAP are slowly added with stirring. Then the Boc-N,N'-dimethylaminoethane reagent (3.0 equivalents) is slowly added and the mixture is kept at O°C under nitrogen for 2 hours then allowed to warm up to RT and react overnight. The CH₂Cl₂ is removed and the product is purified by silica gel chromatography using an ethyl acetate/hexane gradient to yield the desired product. If not used immediately this intermediate should be stored at -70°C under an inert gas. The intermediate (41 mg) is dissolved in 1.5 ml of anhydrous CH₂Cl₂ under nitrogen at O°C into a 25 ml round bottom flask, and 50 µl of anisole is added. Next 1.2 ml of trifluoroacetic acid is slowly added in 6 equal aliquots. The reaction is left stirring on ice, under nitrogen, for 45 minutes. Then 10 ml of pre-cooled (-20°C) CHCl₃ is quickly added. All liquid is quickly removed under high vacuum. The residue is placed back into the ice bath and 1.5 ml of pre-cooled (-20°C) DMF added. After 15 minutes the Fmoc-Val-Cit-PAB-PNP linker (62 mg, Axis Pharma catalog #AP10017) is added with stirring. After 15 minutes triethylamine (217 µl) is added. The mixture is stirred under nitrogen at 0°C for 1 hour, then allowed to slowly warm to RT. If desired, the CH₂Cl₂ is removed and the product is purified by silica gel chromatography using an ethyl acetate/hexane gradient to yield the desired product **analog 401.**

Example 169: **Synthesis of Analog 404: Analog 401** can be redissolved in 1. 5 ml of anhydrous CH₂Cl₂ under nitrogen at O°C, then 200 µl of triethylamine added, the vial sealed and allowed to react at 4°C for 18 hours. The mixture is purified by silica gel chromatography using an ethanol/chloroform gradient to yield the desired product **analog 404** (final yield approximately 20%).

Example 170: **Synthesis of Analog 405:** 50 mg of VP-PEG4-Acid ( 1-(6-methyl-4-vinylpyridin-2-yl)-3-oxo-7,110,13,16-tetraoxa-4-azanonadecan-19-oic acid) and N-hydroxysuccinimide (16 mg, 1.20 equivalents) are dissolved in 20 ml of anhydrous CH₂Cl₂ under nitrogen. The EDC (1-ethyl-3-(3-dimethylaminopropyl)carbodiimide, 21 mg, 1.20 equivalents) is added and allowed to react at RT for 24 hours to produce the compound VP-PEG4-NHS (2,5-dioxopyrrolidin-1-yl 1-(6-methyl-4-vinylpyridin-22-yl)-3 -oxo-7,10,13,16-tetraoxa-4-azanondecane-19-oate). The CH₂Cl₂ mixture containing the VP-PEG4-NHS is washed with water, then quickly dried and the CH₂Cl₂ removed by vacuum. Next **analog 404** (74 mg) is dissolved in 3.0 ml of DMF, and then added to the VP-PEG-NHS compound. The mixture is placed under nitrogen, cooled to 0°C, then 210 µl of triethylamine added, and the reaction allowed to continue overnight in the cold. The desired product, **analog 405,** is recovered using reverse phase HPLC (approximately 50% yield of initial starting material analog 404).

Example 171: **Synthesis of Analog 402** and **Analog 403:** Illudin S (0.95 grams) is dissolved in 100 ml of water, and then 100 ml of hexane is added to form an upper organic layer. With rapid stirring, 2.06 ml of concentrated sulfuric acid is added slowly. After 1 hour the hexane is removed and another 100 ml of hexane is added. This process is repeated one more time. The hexane layers are combined washed with a saturated sodium bicarbonate solution to neutralize residual acid, dried with anhydrous magnesium, then solvent removed to yield a bright yellow oil (AF1). Next paraformaldehyde (0.66 g) is dissolved in 60 ml of water, and concentrated sulfuric acid (5.0 ml) is slowly added. The cloudy solution is slowly heated until the solution is clear. This paraformaldehyde solution is allowed to slowly cool to 30°C. Then the previously prepared bright yellow intermediate (AF1) is dissolved in acetone, and the mixture slowly added with continuous stirring for 24 hours. The reaction mixture is extracted three times with ethyl acetate (100 ml), the extracts are combined, washed with saturated sodium bicarbonate, dried over anhydrous magnesium sulfate, then the organic solvent removed by vacuum. The residue is purified by silica gel chromatography using an ethyl acetate/hexane gradient to yield a bright orange intermediate (IRO1). IRO1 is dissolved in anhydrous dichloromethane, under nitrogen, and 2,4,6-trimethylpyridine (3.0 equivalents) is added, then the mixture is cooled to O°C. Sulfamoyl chloride (1.15 equivalents) is dissolved in anhydrous peptide-grade DMF (1 ml), and is slowly added to the dichloromethane solution, with mixing. The solution is mixed for one hour at O°C, then slowly allowed to warm to RT, and continued to react for a total of 4 hours. The reaction mixture is concentrated by vacuum, then purified by silica gel chromatography using an ethyl acetate/hexane gradient to yield **analog 402** and **analog 403** (final yield of both products is approximately 15%).

Example 172: **Synthesis of Analog 407:** Illudin S (100 mg) is dissolved in 20 ml of anhydrous DCM and 5.0 ml of DMF at RT. Then molecular sieves are added and the mixture is stirred at RT for 30 minutes under nitrogen, and 2,4,6-trimethylpyridine (5.7 equivalents) is slowly added until the Illudin S is dissolved. The mixture is then cooled to -4°C and the trifluormethanesulfanyl chloride (Aldrich Catakog #164798, 1.2 equivalents) is slowly added over a 15 minute period. The reaction is allowed to proceed for 4 hours at -4°C, and then allowed to slowly warm and react overnight at RT for 16 hours. The DCM/DMF reaction mixture is washed with saturated sodium bicarbonate to remove residual sulfonic and hydrochloric acids. The remaining organic phase is dried then removed with high vacuum. The product is purified using by silica gel chromatography using an ethyl acetate/hexane gradient to yield **analog 407.** Yield is approximately 15%. The product is unstable and should be stored at - 70°C under an inert gas.

Example 173: **Synthesis of Analog 408:** Illudin S (500 mg) is dissolved in a mixture of 300 mg of sodium acetate and 6.0 ml of acetic anhydride. The reaction is allowed to proceed at RT with stirring and monitored. After 3 to 4 hours the reaction is quenched by adding 100 ml of saturated sodium bicarbonate solution. The aqueous solution is extracted four times with ethyl acetate and the organic fractions are combined, dried and removed by vacuum. The desired mono-acetate product is separated from the undesired residual Illudin S, di-acetate and secondary mono-acetate contaminating compounds by silica gel chromatography using an ethyl acetate/hexane gradient. The recovered mono-acetate product is dissolved in anhydrous CH₂Cl₂ under nitrogen at O°C with 1.15 equivalents of p-nitrophenylchloroformate. Next 1.5 equivalents of DIPEA and 1.0 equivalents of DMAP are slowly added with stirring. After the Boc-N,N'-dimethylaminoethane reagent (3.0 equivalents) is added and the mixture is kept at O°C under nitrogen for an 2 hours then allowed to warm up to RT and react overnight. The CH₂Cl₂ is removed and the product is purified by silica gel chromatography using an ethyl acetate/hexane gradient to yield the desired intermediate product. If not used immediately this intermediate should be stored at -70°C under an inert gas. The intermediate (41 mg) is dissolved in 1.5 ml of anhydrous CH₂Cl₂ under nitrogen at O°C into a 25 ml round bottom flask, and 50 µl of anisole is added. Next 1.2 ml of trifluoroacetic acid is slowly added in 6 equal aliquots. The reaction is left stirring on ice, under nitrogen, for 45 minutes. Then 10 ml of precooled (-20°C) CHCl₃ added. All liquid is removed under high vacuum. The residue is placed back in to the ice bath and 1.5 ml of pre-cooled (-20°C) DMF added. This solution is added to a solution of 3ml of DMF containing the Fmoc-Gly3-Val-Cit-PNP peptide (Levena Biopharma Catalog #H1002, 1.1 equivalent) at O°C. The solution is allowed to slowly warm to RT and react overnight. The desired intermediate is recovered by precipitation with excess diethyl ether. The precipitate is washed twice with ice cold ether, dried to remove the ether, then redissolved in 1.5 ml of DMF, 200 µl of triethylamine added, and the reaction allowed to proceed at 4°C for 48 hours to produce **analog 408** (final yield is approximately 20%).

Example 174: **Synthesis of Analog 409:** Illudin S (500 mg) is dissolved in a mixture of 300 mg of sodium acetate and 6.0 ml of acetic anhydride. The reaction is allowed to proceed at RT with stirring and monitored. The reaction is quenched after 3 - 4 hours by adding 100 ml of saturated sodium bicarbonate solution. The aqueous solution is extracted four times with ethyl acetate, the organic fractions are combined, dried and removed by vacuum. The desired mono-acetate product is separated from the undesired residual Illudin S, di-acetate and secondary mono-acetate contaminating compounds by silica gel chromatography using an ethyl acetate/hexane gradient. The recovered mono-acetate product is dissolved in anhydrous CH₂Cl₂ under nitrogen at O°C with 1.15 equivalents of p-nitrophenylchloroformate. Next is added with stirring 1.5 equivalents of DIPEA and 1.0 equivalents of DMAP. After the Boc-N,N'-dimethylaminoethane reagent (3.0 equivalents) is added and the mixture is kept at O°C under nitrogen for an additional 2 hours then allowed to warm up to RT and react overnight. The CH₂Cl₂ is removed and the product is purified by silica gel chromatography using an ethyl acetate/hexane gradient to yield the desired product. If not used immediately this intermediate should be stored at -70°C under an inert gas. This intermediate (41 mg) is dissolved in 1.5 ml of anhydrous CH₂Cl₂ under nitrogen at O°C into a 2 5 ml round bottom flask, and 50 µl of anisole is added. Next 1.2 ml of trifluoroacetic acid is slowly added in 6 equal aliquots. The reaction is left stirring on ice, under nitrogen, for 45 minutes. Then 10 ml of precooled (-20°C) CHCl₃ is added. All liquid is removed under high vacuum. The residue is placed back in to the ice bath and 1.5 ml of pre-cooled (-20°C) peptide synthesis grade DMF added. After 15 minutes the Fmoc-Val-Cit-PAB-PNP linker (62 mg. Axis Pharma catalog #AP10017) is added with stirring. After 15 minutes then triethylamine (217 µl) is added. The mixture is stired under nitrogen at 0°C for 1 hour, then allowed to slowly warm to RT. If desired, the CH₂Cl₂ is removed and the product is purified by silica gel chromatography using an ethyl acetate/hexane gradient to yield the desired intermediate product. The intermediate can be redissolved in 1. 5 ml of anhydrous CH₂Cl₂ under nitrogen at O°C, then 200 µl of triethylamine added, the vial sealed and allowed to react at 4C for 18 hours. The desired intermediate is recovered by precipitation with excess ice-cold diethyl ether. The precipitate is washed twice with ice cold ether, dried to remove the ether, then redissolved in 1.5 ml of DMF and 200 µl of triethylamine is added, and the reaction allowed to proceed at 4°C for 48 hours to produce **analog 409** (final yield approximately 30%).

Example 175: **Synthesis of Analog 410:** Illudin S (500 mg) is dissolved in a mixture of 300 mg of sodium acetate and 6.0 mls of acetic anhydride. The reaction is allowed to proceed at RT with stirring and monitored. The reaction is quenched after 3 - 4 hours by adding 100 ml of saturated sodium bicarbonate solution. The aqueous solution is extracted four times with ethyl acetate, the organic fractions are combined, dried and removed by vacuum. The desired mono-acetate product is separated from the undesired residual Illudin S, di-acetate and secondary mono-acetate contaminating compounds by silica gel chromatography using an ethyl acetate/hexane gradient. The recovered mono-acetate product is dissolved in anhydrous CH₂Cl₂ under nitrogen at O°C with 1.15 equivalents of p-nitrophenylchloroformate. Next is added with stirring 1.5 equivalents of DIPEA and 1.0 equivalents of DMAP. After the Boc-N,N'-dimethylaminoethane reagent (3.0 equivalents) is added , the mixture is kept at O°C under nitrogen for an additional 2 hours then allowed to warm up to RT and react overnight. The CH₂Cl₂ is removed and the product is purified by silica gel chromatography using an ethyl acetate/hexane gradient to yield the desired product. If not used immediately this intermediate should be stored at -70°C under an inert gas. This intermediate (41 mg) is dissolved in 1.5 mls of anhydrous CH₂Cl₂ under nitrogen at O°C into a 25 ml round bottom flask, and 50 µl of anisole is added. Next 1.2 ml of trifluoroacetic acid is slowly added in 6 equal aliquots. The reaction is left stirring on ice, under nitrogen, for 45 minutes. Then 10 ml of pre-cooled (-20°C) CHCl₃ is added. All liquid is removed under high vacuum. The residue is placed back in to the ice bath and 1.5 ml of pre-cooled (-20°C) DMF added. Next MC-Val-Cit-PAB-PNP (Levena Biopharma Catalog #VC1004, 1.2 equivalents) is directly added with stirring while maintaining the temperature at 0°C. Once all solid material is dissolved then triethylamine (8.0 equivalents) is added and the reaction continued at 4°C for 48 hours. **Analog 410** is recovered by preparative HPLC using an acetonitrile gradient (5% to 95%) with 0.05% trifluoroacetic acid (final yield approximately 15%).

Abbreviations used include: DMAP = 4-dimethylaminopyridine; DCC = N,N'-dicycyclohexylcarbodiimide; ODHBt = 3,4,-dihydroxy-4-oxo-1,2,3-benzo-triazine-3-yl ester; NMM = N-methylmorpholine; EDC = 1-(3-dimethylaminopropyl)-3-ethylcarbodiimide hydrochloride; DIAD = diisopropyl azodicarboxylate; DEAD = diethyl azodicarboxylate; and DIPC = N,N'-diisopropylcarbodiimide. Boc₂O = anhydrous Boc; BocNH₂ = t-butyl carbamate; DIPEA = N,N-diisopropylethylamine; ODHBt = 3,4,-dihydroxy-4-oxo-1,2,3-benzo-triazine-3-yl ester (refers to ester made from HOOBt); HOOBt = (Hydroxy-3,4-dihydro-4-oxo-1,2,3-benzotriazine); NMM = N-methylmorpholine; RT - room temperature; TBDMSCl = t-butyldimethyl silyl chloride; TEA = triethanolamine.

### SPECIFIC EMBODIMENTS

Embodiments contemplated herein include Embodiments P1(a)-P19(a) following.

Embodiment P1(a). A compound, comprising: an illudofulvene moiety selected from the group consisting of analog 317 (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl 4-(fluorosulfonyl)benzoate; analog 318 ((2'S,3'R,6'R)-3'-((4-(fluorosulfonyl)benzoyl)oxy)-6'-hydroxy-2',4',6'-trimethyl-7'-oxo-2',3',6',7'-tetrahydrospiro[cyclopropane-1,5'-inden]-2'-yl)methyl 4-(fluorosulfonyl)benzoate; analog 333 ((2'S,3'R,6'R)-6'-hydroxy-2',4',6'-trimethyl-3'-(((4-nitrophenoxy)carbonyl)oxy)-7'-oxo-2',3',6',7'-tetrahydrospiro[cyclopropane-1,5'-inden]-2'-yl)methyl acetate; analog 334 ((2'S,3'R,6'R)-3'-(((2-((tert-butoxycarbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 335 tert-butyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate); analog 339 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-methylbenzenesulfonate; analog 345 (2'S,3'R,6'R)-2'-(acetoxymethyl)-6'-hydroxy-2',4',6'-trimethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate; analog 346 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-(N-(2-(2-(2-(2-aminoacetamido)acetamido)acetamido)ethyl)-N-methylsulfamoyl)benzoate; analog 347 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-sulfamoylbenzoate; analog 348 (3'S,6'R)-6'-hydroxy-2',2',4',6'-tetramethyl-7'-oxo-2',3',6',7'-tetrahydrospiro[cyclopropane-1,5'-inden]-3'-yl 4-sulfamoylbenzoate; analog 351 (3'S,6'R)-6'-hydroxy-2',2',4',6'-tetramethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate; analog 353 ((6'R)-3'-(((2-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 356 ((2'S,3'S,6'R)-3'-(((1-(9H-fluoren-9-yl)-13-methyl-3,6,9,12-tetraoxo-2-oxa-4,7,10,13-tetraazapentadecan-15-yl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 357 ((2'S,3'S,6'R)-3'-(((2-(2-(2-(2-aminoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 359 ((2'S,3'S,6'R)-6'-hydroxy-2',4',6'-trimethyl-3'-((methyl(2,2,14-trimethyl-4,7,10,13-tetraoxo-3-oxa-5,8,11,14-tetraazahexadecan-16-yl)carbamoyl)oxy)-7'-oxo-2',3',6',7'-tetrahydrospiro[cyclopropane-1,5'-inden]-2'-yl)methyl acetate; analog 361 ((2'S,3'R,6'R)-3'-((((2,5-dioxopyrrolidin-1-yl)oxy)carbonyl)oxy)-6'-hydroxy-2',4',6'-trimethyl-7'-oxo-2',3',6',7'-tetrahydrospiro[cyclopropane-1,5'-inden]-2'-yl)methyl acetate; analog 362 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl methanesulfonate; analog 363 (2'S,3'R,6'R)-2'-(aminomethyl)-3',6'-dihydroxy-2',4',6'-trimethyl-2',3'-dihydrospiro[cyclopropane-1,5'-inden]-7'(6'H)-one; analog 364 (2'R,3'R,6'R)-3'-amino-6'-hydroxy-2'-(hydroxymethyl)-2',4',6'-trimethyl-2',3'-dihydrospiro[cyclopropane-1,5'-inden]-7'(6'H)-one; analog 368 ((2'S,3'R,6'R)-3'-acetoxy-6'-hydroxy-2',4',6'-trimethyl-7'-oxo-2',3',6',7'-tetrahydrospiro[cyclopropane-1,5'-inden]-2'-yl)methyl acetate; analog 369 (2'S,3'R,6'R)-2'-(((tert-butyldimethylsilyl)oxy)methyl)-3',6'-dihydroxy-2',4',6'-trimethyl-2',3'-dihydrospiro[cyclopropane-1,5'-inden]-7'(6'H)-one; analog 370 (2'S,3'R,6'R)-2'-(((tertbutyldimethylsilyl)oxy)methyl)-6'-hydroxy-2',4',6'-trimethyl-7'-oxo-2',3',6',7'-tetrahydrospiro[cyclopropane-1,5'-inden]-3'-yl acetate; analog 371 (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl acetate; analog 372 (2'R,3'R,6'R)-2'-formyl-6'-hydroxy-2',4',6'-trimethyl-7'-oxo-2',3',6',7'-tetrahydrospiro[cyclopropane-1,5'-inden]-3'-yl acetate; analog 373 (2'S,3'R,6'R)-2'-(((((9H-fluoren-9-yl)methoxy)carbonyl)amino)methyl)-6'-hydroxy-2',4',6'-trimethyl-7'-oxo-2',3',6',7'-tetrahydrospiro[cyclopropane-1,5'-inden]-3'-yl acetate; analog 374 (2'S,3'R,6'R)-2'-(aminomethyl)-6'-hydroxy-2',4',6'-trimethyl-7'-oxo-2',3',6',7'-tetrahydrospiro[cyclopropane-1,5'-inden]-3'-yl acetate; analog 377 ((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl acetate; analog 378 (2'S,3'R,6'R)-2'-(((tert-butoxycarbonyl)amino)methyl)-6'-hydroxy-2',4',6'-trimethyl-7'-oxo-2',3',6',7'-tetrahydrospiro[cyclopropane-1,5'-inden]-3'-yl acetate; analog 379 ((2'S,3'R,6'R)-3'-(((2-((((4-((14S,17S)-1-(9H-fluoren-9-yl)-14-isopropyl-3,6,9,12,15-pentaoxo-17-(3-ureidopropyl)-2-oxa-4,7,10,13,16-pentaazaoctadecan-18-amido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 380 ((2'S,3'R,6'R)-3'-(((2-((((4-((2S,5S)-14-amino-5-isopropyl-4,7,10,13-tetraoxo-2-(3-ureidopropyl)-3,6,9,12-tetraazatetradecanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 381 (2'S,3'R,6'R)-6'-hydroxy-2'-(hydroxymethyl)-2',4',6'-trimethyl-3'-((tributylsilyl)oxy)-2',3'-dihydrospiro[cyclopropane-1,5'-inden]-7'(6'H)-one; analog 382 (2'R,3'R,6'R)-6'-hydroxy-2',4',6'-trimethyl-7'-oxo-3'-((tributylsilyl)oxy)-2',3',6',7'-tetrahydrospiro[cyclopropane-1,5'-indene]-2'-carbaldehyde; analog 383 (2'R,3'R,6'R)-3',6'-dihydroxy-2',4',6'-trimethyl-7'-oxo-2',3',6',7'-tetrahydrospiro[cyclopropane-1,5'-indene]-2'-carbaldehyde; analog 384 tert-butyl (((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl)carbamate; analog 389 tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)carbamate; analog 392 ((2'S,3'S,6'R)-3'-(((2-(2-(2-(2-azidoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 393 ((2'S,3'R,6'R)-3'-(((2-(2,5-dioxo-2,5-dihydro-1H-pyrrol-1-yl)ethyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 397 tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)(hydroxy)carbamate; analog 398 (2'S,3'R,6'R)-3',6'-dihydroxy-2'-((hydroxyamino)methyl)-2',4',6'-trimethyl-2',3'-dihydrospiro[cyclopropane-1,5'-inden]-7'(6'H)-one; analog 399 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl sulfamate; analog 401 ((6'R)-3'-(((2-((((4-((S)-2-((S)-2-((((9H-fluoren-9-yl)methoxy)carbonyl)amino)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 403 ((2'S,6'R)-6'-hydroxy-2',6'-dimethyl-4'-methylene-7'-oxo-2',4',6',7'-tetrahydrospiro[cyclopropane-1,5'-inden]-2'-yl)methyl methanesulfonate; analog 404 ((6'R)-3'-(((2-((((4-((S)-2-((S)-2-amino-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 405 ((2'S,3'S,6'R)-6'-hydroxy-3'-(((2-((((4-((2S,5S)-5-isopropyl-25-(6-methyl-4-vinylpyridin-2-yl)-4,7,23-trioxo-2-(3-ureidopropyl)-10,13,16,19-tetraoxa-3,6,22-triazapentacosanamido)benzyl)oxy)carbonyl)(methyl)amino)ethyl)(methyl)carbamoyl)oxy)-2',4',6'-trimethyl-7'-oxo-2',3',6',7'-tetrahydrospiro[cyclopropane-1,5'-inden]-2'-yl)methyl acetate; analog 407 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl trifluoromethanesulfonate; analog 408 4-((2S,5S)-14-amino-5-isopropyl-4,7,10,13-tetraoxo-2-(3-ureidopropyl)-3,6,9,12-tetraazatetradecanamido)benzyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate); analog 409 4-((S)-2-((S)-2-amino-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate) and analog 410 4-((S)-2-((S)-2-(6-(2,5-dioxo-2,5-dihydro-1H-pyrrol-1-yl)hexanamido)-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate).

Embodiment P2(a). The compound of Embodiment P1(a), wherein the illudofulvene moiety is selected from the group consisting of analog 334; analog 362; analog 371; and analog 383.

Embodiment P3(a). The compound of Embodiment P1(a) or P2(a), wherein the compound comprising an illudofulvene moiety is an affinity medicant conjugate, optionally wherein the affinity medicant conjugate comprises a linker.

Embodiment P4(a). A composition comprising the compound of any of Embodiments P1(a)-P3(a), and a physiologically compatible excipient.

Embodiment P5(a). A composition comprising racemic mixtures of any enantiomers of the compound of any of Embodiments P1(a)-P3(a).

Embodiment P6(a). A composition comprising the compound of any of Embodiments P1(a)-P3(a), in the form of a liposomal particle, a nanoparticle, or a PEGylated compound.

Embodiment P7(a). A composition comprising the compound of any of Embodiments P1(a)-P3(a), and a physiologically compatible carrier.

Embodiment P8(a). A compound, comprising: an illudofulvene moiety selected from the group consisting of analog 332 (R)-6'-hydroxy-3'-((methoxyamino)methyl)-2',4',6'-trimethylspiro[cyclopropane-1,5'-inden]-7'(6'H)-one; analog 337 4-nitrophenyl (R)-((6'-hydroxy-2',4',6'-trimethyl-7'-oxo-6',7'-dihydrospiro[cyclopropane-1,5'-inden]-3'-yl)methyl)carbamate; analog 338 4-nitrophenyl (R)-(3-(6'-hydroxy-2',4',6'-trimethyl-7'-oxo-6',7'-dihydrospiro[cyclopropane-1,5'-inden]-3'-yl)propyl)carbamate; analog 354 4-((S)-2-((S)-2-(6-(2,5-dioxo-2,5-dihydro-1H-pyrrol-1-yl)hexanamido)-3-methylbutanamido)-5-ureidopentanamido)benzyl (2-(3-((S)-6'-hydroxy-2',4',6'-trimethyl-7'-oxo-6',7'-dihydrospiro[cyclopropane-1,5'-inden]-3'-yl)propylidene)hydrazine-1-carbonyl)carbamate; analog 366 tert-butyl (R)-((6'-hydroxy-2',4',6'-trimethyl-7'-oxo-6',7'-dihydrospiro[cyclopropane-1,5'-inden]-3'-yl)methyl)carbamate; analog 367 tert-butyl (R)-(3-(6'-hydroxy-2',4',6'-trimethyl-7'-oxo-6',7'-dihydrospiro[cyclopropane-1,5'-inden]-3'-yl)propyl)carbamate; analog 394 (R)-6'-hydroxy-3'-((hydroxyamino)methyl)-2',4',6'-trimethylspiro[cyclopropane-1,5'-inden]-7'(6'H)-one; and analog 402 (R)-(6'-hydroxy-2',4',6'-trimethyl-7'-oxo-6',7'-dihydrospiro[cyclopropane-1,5'-inden]-3'-yl)methyl sulfamate.

Embodiment P9(a). The compound of Embodiment P8(a), wherein the illudofulvene moity is analog 394.

Embodiment P10(a). The compound of Embodiment P8(a) or P9(a), wherein the compound comprising an illudofulvene moiety is an affinity medicant conjugate, optionally wherein the affinity medicant conjugate comprises a linker.

Embodiment P11(a). A composition comprising the compound of any of Embodiments P8(a)-P10(a), and a physiologically compatible excipient.

Embodiment P12(a). A composition comprising racemic mixtures of any enantiomers of the compound of any of Embodiment P8(a)-P10(a).

Embodiment P13(a). A composition comprising the compound of any of Embodiment P8(a)-P10(a), in the form of a liposomal particle, a nanoparticle, or a PEGylated compound.

Embodiment P14(a). A composition comprising the compound of any of Embodiment P8(a)-P10(a), and a physiologically compatible carrier.

Embodiment P15(a). A compound comprising an illudofulvene moiety for use in a method of treating cancer, wherein the compound is a compound of any of Embodiments P1(a)-P3(a) or P8(a)-P10(a).

Embodiment P16(a). A composition comprising a compound comprising an illudofulvene moiety for use in a method of treating cancer, wherein the composition is a composition of any of Embodiments P4(a)-P7(a) or P11(a)-P14(a).

Embodiment P17(a). An illudofulvene moiety for use in a method of treating a patient with cancer, wherein the method comprises: (a) determining from a patient test sample that the patient has a cancer; and (b) treating the patient with the composition comprising the compound of Embodiments P1(a) to P14(a).

Embodiment P18(a). An affinity drug conjugate for use in a method of treating a patient with cancer, wherein the method comprises: (a) determining from a patient test sample that the patient has a cancer; (b) determining an affinity moiety that targets a marker expressed on the cancer; (c) generating an affinity drug conjugate comprising: the affinity moiety; and the composition comprising the compound of Embodiments P1(a) to P14(a); and (d) treating the patient with the affinity drug conjugate.

Embodiment P19(a). An affinity drug conjugate for use in a method of treating a patient with cancer, wherein the method comprises: (a) determining from a patient test sample that the patient has a cancer; (b) determining an affinity moiety that targets a marker expressed on the cancer; (c) identifying a linker moiety to covalently attach the composition comprising the compound of Embodiments P1(a) to P14(a) to the affinity moiety; (d) generating an affinity drug conjugate comprising: the affinity moiety; the linker moiety; and the composition comprising the compound of Embodiments P1(a) to P14(a); and (e) treating the patient with the affinity drug conjugate.

Embodiments contemplated herein include Embodiments P1(b)-P17(b) following.

Embodiment P1(b). A compound, wherein the compound is an illudofulvene analog selected from the group consisting of analog 317 (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl 4-(fluorosulfonyl)benzoate; analog 318 ((2'S,3'R,6'R)-3'-((4-(fluorosulfonyl)benzoyl)oxy)-6'-hydroxy-2',4',6'-trimethyl-7'-oxo-2',3',6',7'-tetrahydrospiro[cyclopropane-1,5'-inden]-2'-yl)methyl 4-(fluorosulfonyl)benzoate; analog 333 ((2'S,3'R,6'R)-6'-hydroxy-2',4',6'-trimethyl-3'-(((4-nitrophenoxy)carbonyl)oxy)-7'-oxo-2',3',6',7'-tetrahydrospiro[cyclopropane-1,5'-inden]-2'-yl)methyl acetate; analog 334 ((2'S,3'R,6'R)-3'-(((2-((tert-butoxycarbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 335 tert-butyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate); analog 339 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-methylbenzenesulfonate; analog 345 (2'S,3'R,6'R)-2'-(acetoxymethyl)-6'-hydroxy-2',4',6'-trimethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate; analog 346 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-(N-(2-(2-(2-(2-aminoacetamido)acetamido)acetamido)ethyl)-N-methylsulfamoyl)benzoate; analog 347 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-sulfamoylbenzoate; analog 348 (3'S,6'R)-6'-hydroxy-2',2',4',6'-tetramethyl-7'-oxo-2',3',6',7'-tetrahydrospiro[cyclopropane-1,5'-inden]-3'-yl 4-sulfamoylbenzoate; analog 351 (3'S,6'R)-6'-hydroxy-2',2',4',6'-tetramethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate; analog 353 ((6'R)-3'-(((2-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 356 ((2'S,3'S,6'R)-3'-(((1-(9H-fluoren-9-yl)-13-methyl-3,6,9,12-tetraoxo-2-oxa-4,7,10,13-tetraazapentadecan-15-yl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 357 ((2'S,3'S,6'R)-3'-(((2-(2-(2-(2-aminoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 359 ((2'S,3'S,6'R)-6'-hydroxy-2',4',6'-trimethyl-3'-((methyl(2,2,14-trimethyl-4,7,10,13-tetraoxo-3-oxa-5,8,11,14-tetraazahexadecan-16-yl)carbamoyl)oxy)-7'-oxo-2',3',6',7'-tetrahydrospiro[cyclopropane-1,5'-inden]-2'-yl)methyl acetate; analog 361 ((2'S,3'R,6'R)-3'-((((2,5-dioxopyrrolidin-1-yl)oxy)carbonyl)oxy)-6'-hydroxy-2',4',6'-trimethyl-7'-oxo-2',3',6',7'-tetrahydrospiro[cyclopropane-1,5'-inden]-2'-yl)methyl acetate; analog 362 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl methanesulfonate; analog 363 (2'S,3'R,6'R)-2'-(aminomethyl)-3',6'-dihydroxy-2',4',6'-trimethyl-2',3'-dihydrospiro[cyclopropane-1,5'-inden]-7'(6'H)-one; analog 364 (2'R,3'R,6'R)-3'-amino-6'-hydroxy-2'-(hydroxymethyl)-2',4',6'-trimethyl-2',3'-dihydrospiro[cyclopropane-1,5'-inden]-7'(6'H)-one; analog 368 ((2'S,3'R,6'R)-3'-acetoxy-6'-hydroxy-2',4',6'-trimethyl-7'-oxo-2',3',6',7'-tetrahydrospiro[cyclopropane-1,5'-inden]-2'-yl)methyl acetate; analog 369 (2'S,3'R,6'R)-2'-(((tert-butyldimethylsilyl)oxy)methyl)-3',6'-dihydroxy-2',4',6'-trimethyl-2',3'-dihydrospiro[cyclopropane-1,5'-inden]-7'(6'H)-one; analog 370 (2'S,3'R,6'R)-2'-(((tertbutyldimethylsilyl)oxy)methyl)-6'-hydroxy-2',4',6'-trimethyl-7'-oxo-2',3',6',7'-tetrahydrospiro[cyclopropane-1,5'-inden]-3'-yl acetate; analog 371 (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl acetate; analog 372 (2'R,3'R,6'R)-2'-formyl-6'-hydroxy-2',4',6'-trimethyl-7'-oxo-2',3',6',7'-tetrahydrospiro[cyclopropane-1,5'-inden]-3'-yl acetate; analog 373 (2'S,3'R,6'R)-2'-(((((9H-fluoren-9-yl)methoxy)carbonyl)amino)methyl)-6'-hydroxy-2',4',6'-trimethyl-7'-oxo-2',3',6',7'-tetrahydrospiro[cyclopropane-1,5'-inden]-3'-yl acetate; analog 374 (2'S,3'R,6'R)-2'-(aminomethyl)-6'-hydroxy-2',4',6'-trimethyl-7'-oxo-2',3',6',7'-tetrahydrospiro[cyclopropane-1,5'-inden]-3'-yl acetate; analog 377 ((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl acetate; analog 378 (2'S,3'R,6'R)-2'-(((tert-butoxycarbonyl)amino)methyl)-6'-hydroxy-2',4',6'-trimethyl-7'-oxo-2',3',6',7'-tetrahydrospiro[cyclopropane-1,5'-inden]-3'-yl acetate; analog 379 ((2'S,3'R,6'R)-3'-(((2-((((4-((14S,17S)-1-(9H-fluoren-9-yl)-14-isopropyl-3,6,9,12,15-pentaoxo-17-(3-ureidopropyl)-2-oxa-4,7,10,13,16-pentaazaoctadecan-18-amido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 380 ((2'S,3'R,6'R)-3'-(((2-((((4-((2S,5S)-14-amino-5-isopropyl-4,7,10,13-tetraoxo-2-(3-ureidopropyl)-3,6,9,12-tetraazatetradecanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 381 (2'S,3'R,6'R)-6'-hydroxy-2'-(hydroxymethyl)-2',4',6'-trimethyl-3'-((tributylsilyl)oxy)-2',3'-dihydrospiro[cyclopropane-1,5'-inden]-7'(6'H)-one; analog 382 (2'R,3'R,6'R)-6'-hydroxy-2',4',6'-trimethyl-7'-oxo-3'-((tributylsilyl)oxy)-2',3',6',7'-tetrahydrospiro[cyclopropane-1,5'-indene]-2'-carbaldehyde; analog 383 (2'R,3'R,6'R)-3',6'-dihydroxy-2',4',6'-trimethyl-7'-oxo-2',3',6',7'-tetrahydrospiro[cyclopropane-1,5'-indene]-2'-carbaldehyde; analog 384 tert-butyl (((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl)carbamate; analog 389 tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)carbamate; analog 392 ((2'S,3'S,6'R)-3'-(((2-(2-(2-(2-azidoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 393 ((2'S,3'R,6'R)-3'-(((2-(2,5-dioxo-2,5-dihydro-1H-pyrrol-1-yl)ethyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 397 tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)(hydroxy)carbamate; analog 398 (2'S,3'R,6'R)-3',6'-dihydroxy-2'-((hydroxyamino)methyl)-2',4',6'-trimethyl-2',3'-dihydrospiro[cyclopropane-1,5'-inden]-7'(6'H)-one; analog 399 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl sulfamate; analog 401 ((6'R)-3'-(((2-((((4-((S)-2-((S)-2-((((9H-fluoren-9-yl)methoxy)carbonyl)amino)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 403 ((2'S,6'R)-6'-hydroxy-2',6'-dimethyl-4'-methylene-7'-oxo-2',4',6',7'-tetrahydrospiro[cyclopropane-1,5'-inden]-2'-yl)methyl methanesulfonate; analog 404 ((6'R)-3'-(((2-((((4-((S)-2-((S)-2-amino-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 405 ((2'S,3'S,6'R)-6'-hydroxy-3'-(((2-((((4-((2S,5S)-5-isopropyl-25-(6-methyl-4-vinylpyridin-2-yl)-4,7,23-trioxo-2-(3-ureidopropyl)-10,13,16,19-tetraoxa-3,6,22-triazapentacosanamido)benzyl)oxy)carbonyl)(methyl)amino)ethyl)(methyl)carbamoyl)oxy)-2',4',6'-trimethyl-7'-oxo-2',3',6',7'-tetrahydrospiro[cyclopropane-1,5'-inden]-2'-yl)methyl acetate; analog 407 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl trifluoromethanesulfonate; analog 408 4-((2S,5S)-14-amino-5-isopropyl-4,7,10,13-tetraoxo-2-(3-ureidopropyl)-3,6,9,12-tetraazatetradecanamido)benzyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate); analog 409 4-((S)-2-((S)-2-amino-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate) and analog 410 4-((S)-2-((S)-2-(6-(2,5-dioxo-2,5-dihydro-1H-pyrrol-1-yl)hexanamido)-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-l,5'-inden]-3'-yl) ethane-1,2-diylbis(methylcarbamate).

Embodiment P2(b). The compound of Embodiment P1(b), wherein the illudofulvene analog is selected from the group consisting of analog 334; analog 362; analog 371; and analog 383.

Embodiment P3(b). A composition comprising the compound of Embodiment P1(b) or P2(b), and a physiologically compatible excipient.

Embodiment P4(b). A composition comprising racemic mixtures of any enantiomers of the compound of Embodiment P1(b) or P2(b).

Embodiment P5(b). A composition comprising the compound of Embodiment P1(b) or P2(b), in the form of a liposomal particle, a nanoparticle, or a PEGylated compound.

Embodiment P6(b). A composition comprising the compound of Embodiment P1(b) or P2(b), and a physiologically compatible carrier.

Embodiment P7(b). A compound, wherein the compound is an illudofulvene analog selected from the group consisting of analog 332 (R)-6'-hydroxy-3'-((methoxyamino)methyl)-2',4',6'-trimethylspiro[cyclopropane-1,5'-inden]-7'(6'H)-one; analog 337 4-nitrophenyl (R)-((6'-hydroxy-2',4',6'-trimethyl-7'-oxo-6',7'-dihydrospiro[cyclopropane-1,5'-inden]-3'-yl)methyl)carbamate; analog 338 4-nitrophenyl (R)-(3-(6'-hydroxy-2',4',6'-trimethyl-7'-oxo-6',7'-dihydrospiro[cyclopropane-1,5'-inden]-3'-yl)propyl)carbamate; analog 354 4-((S)-2-((S)-2-(6-(2,5-dioxo-2,5-dihydro-1H-pyrrol-1-yl)hexanamido)-3-methylbutanamido)-5-ureidopentanamido)benzyl (2-(3-((S)-6'-hydroxy-2',4',6'-trimethyl-7'-oxo-6',7'-dihydrospiro[cyclopropane-1,5'-inden]-3'-yl)propylidene)hydrazine-1-carbonyl)carbamate; analog 366 tert-butyl (R)-((6'-hydroxy-2',4',6'-trimethyl-7'-oxo-6',7'-dihydrospiro[cyclopropane-1,5'-inden]-3'-yl)methyl)carbamate; analog 367 tert-butyl (R)-(3-(6'-hydroxy-2',4',6'-trimethyl-7'-oxo-6',7'-dihydrospiro[cyclopropane-1,5'-inden]-3'-yl)propyl)carbamate; analog 394 (R)-6'-hydroxy-3'-((hydroxyamino)methyl)-2',4',6'-trimethylspiro[cyclopropane-1,5'-inden]-7'(6'H)-one; and analog 402 (R)-(6'-hydroxy-2',4',6'-trimethyl-7'-oxo-6',7'-dihydrospiro[cyclopropane-1,5'-inden]-3'-yl)methyl sulfamate.

Embodiment P8(b). The compound of Embodiment P7(b), wherein the illudofulvene analog is analog 394.

Embodiment P9(b). A composition comprising the compound of Embodiment P7(b) or P8(b), and a physiologically compatible excipient.

Embodiment P10(b). A composition comprising racemic mixtures of any enantiomers of the compound of Embodiment P7(b) or P8(b).

Embodiment P11(b). A composition comprising the compound of Embodiment P7(b) or P8(b), in the form of a liposomal particle, a nanoparticle, or a PEGylated compound.

Embodiment P12(b). A composition comprising the compound of Embodiment P7(b) or P8(b), and a physiologically compatible carrier.

Embodiment P13(b). A compound comprising an illudofulvene moiety for use in a method of treating cancer, wherein the compound is a compound of any of Embodiments P1(b)-P2(b) or P7(b)-P8(b).

Embodiment P14(b). A composition comprising a compound comprising an illudofulvene moiety for use in a method of treating cancer, wherein the composition is a composition of any of Embodiments P3(b)-P6(b) or P9(b)-P12(b).

Embodiment P15(b). An illudofulvene moiety for use in a method of treating a patient with cancer, wherein the method comprises: (a) determining from a patient test sample that the patient has a cancer; and (b) treating the patient with the composition comprising the compound of Embodiments P1(b)to P12(b).

Embodiment P16(b). An affinity drug conjugate for use in a method of treating a patient with cancer, wherein the method comprises: (a) determining from a patient test sample that the patient has a cancer; (b) determining an affinity moiety that targets a marker expressed on the cancer; (c) generating an affinity drug conjugate comprising: the affinity moiety; and the composition comprising the compound of Embodiments P1(b) to P12(b); and (d) treating the patient with the affinity drug conjugate.

Embodiment P17(b). An affinity drug conjugate for use in a method of treating a patient with cancer, wherein the method comprises: (a) determining from a patient test sample that the patient has a cancer; (b) determining an affinity moiety that targets a marker expressed on the cancer; (c) identifying a linker moiety to covalently attach the composition comprising the compound of Embodiments P1(b)to P12(b) to the affinity moiety; (d) generating an affinity drug conjugate comprising: the affinity moiety; the linker moiety; and the composition comprising the compound of Embodiments P1(b) to P12(b); and (e) treating the patient with the affinity drug conjugate.

Embodiments contemplated herein also include Embodiments Q1-Q39 following.

Embodiment Q1. A compound, comprising: an illudofulvene moiety selected from the group consisting of analog 317, 333, 334, 335, 345, 348, 351, 356, 357, 359, 364, 377, 379, 380, 381, 382, 392, and 393

Embodiment Q2. The composition comprising the compound of Embodiment Q1, and a physiologically compatible excipient.

Embodiment Q3. The composition comprising racemic mixtures of any enantiomers of the compound of Embodiment Q1.

Embodiment Q4. The composition comprising the compound of Embodiment Q1, in the form ofa liposomal particle, a nanoparticle, or a PEGylated compound.

Embodiment Q5. The composition comprising the compound of Embodiment Q1, and a physiologically compatible carrier.

Embodiment Q6. The compound of Embodiment Q1, where the illudofulvene is selected from the group consisting of analog 317, 333, 334, 335, 345, 348, 351, 353, 356, 357, 359, 361, 364, 377, 379, 380, 381, 382, 384, 392, and 393.

Embodiment Q7. A compound, comprising: an illudofulvene moiety selected from the group consisting of analog 339, 346, 347, 362, 363, 369, 370, 371, 372, 373, 374, 378, 384, and 389.

Embodiment Q8. The composition comprising the compound of Embodiment Q7, and a physiologically compatible excipient.

Embodiment Q9. The composition comprising racemic mixtures of any enantiomers of the compound of Embodiment Q7.

Embodiment Q10. The composition comprising the compound of Embodiment Q7, in the form of a liposomal particle, a nanoparticle, or a PEGylated compound.

Embodiment Q11. The composition comprising the compound of Embodiment Q7, and a physiologically compatible carrier.

Embodiment Q12. The compound, comprising: an illudofulvene moiety selected from the group consisting of analog 318, 361, and 383.

Embodiment Q13. The composition comprising the compound of Embodiment Q12, and a physiologically compatible excipient.

Embodiment Q14. The composition comprising racemic mixtures of any enantiomers of the compound of Embodiment Q12.

Embodiment Q15. The composition comprising the compound of Embodiment Q12, in the form of a liposomal particle, a nanoparticle, or a PEGylated compound.

Embodiment Q16. The composition comprising the compound of Embodiment Q12, and a physiologically compatible carrier.

Embodiment Q17. A compound, comprising: an illudofulvene moiety selected from the group consisting of analog 320, 321, 323, 324, 332, 336, 337, 338, 352, 354, 366, 367, or 394.

Embodiment Q18. The composition comprising the compound of Embodiment Q17, and a physiologically compatible excipient.

Embodiment Q19. The composition comprising racemic mixtures of any enantiomers of the compound of Embodiment Q17.

Embodiment Q20. The composition comprising the compound of Embodiment Q17, in the form of a liposomal particle, a nanoparticle, or a PEGylated compound.

Embodiment Q21. The composition comprising the compound of Embodiment Q17, and a physiologically compatible carrier.

Embodiment Q22. An illudofulvene analog of the formula: 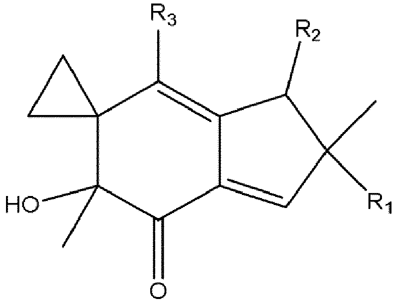 or a pharmaceutically acceptable salt thereof, where R₁, R₂, and R₃ each independently represent -H, -OH, -CH₃, -OCH₃, -C(=O)CH₃, -OC(=O)CH₃, -C(=O)OCH₃ -C(=O)CH₂CH₃,-OC(=O)CH₂CH₃, -C(=O)OCH₂CH₃, -CH₂CH₃, -CH(CH₃)₂, -C(CH₃)₃, -CH₂CH₂CH₃,-CH₂CH(CH₃)₂, -CHC(CH₃)₃, -CH₂OH, -NH₂, -CH₂NH₂, -N₃, and (C₁-C₄)alkyl.

Embodiment Q23. The illudofulvene analog of Embodiment Q22, further comprising an Affinity Moiety covalently attached to one or both R₁ and R₂.

Embodiment Q24. The illudofulvene analog of Embodiment Q23, where the Affinity Moiety is selected from the group consisting of an antibody, an antibody fragment, a receptor protein, a peptidic growth factor, an anti-angiogenic protein, a specific binding peptide, protease cleavable peptide, a glycopeptide, a peptide, a peptidic toxin, a protein toxin and an oligonucleotide.

Embodiment Q25. The illudofulvene analog of Embodiment Q23, further comprising a linker moiety adaptedto covalently attach the Affinity Moiety to the illudofulvene analog.

Embodiment Q26. The illudofulvene analog of Embodiment Q25, where the linker moiety is selected from the group consisting of a secondary amino group, a carbonyl group, an ester, and analcohol.

Embodiment Q27. The composition comprising racemic mixtures of the illudofulvene analog of Embodiment Q22.

Embodiment Q28. The composition comprising enantiomers of the illudofulvene analog of Embodiment Q22.

Embodiment Q29. The composition comprising enantiomers and racemic mixtures of the illudofulvene analog of Embodiment Q22.

Embodiment Q30. The composition comprising the illudofulvene analog of Embodiment Q22 and a physiologically compatible excipient.

Embodiment Q31. An illudofulvene analog of the formula: 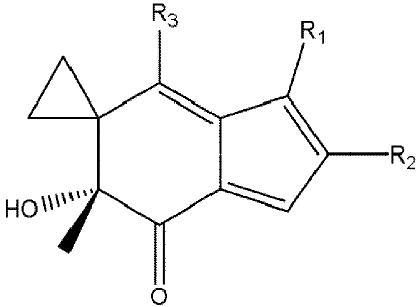 where R₁ represents -(CH₂)ₙR₄R₅, and n = an integer from 0 to 7, R₂ represents -H, -OH, -CH₃,-OCH₃, -C(=O)CH₃, -OC(=O)CH₃, -C(=O)OCH₃, -C(=O)CH₂CH₃, -OC(=O)CH₂CH₃,-C(=O)OCH₂CH₃, -CH₂CH₃, -CH(CH₃)₂, -C(CH₃)₃, -CH₂CH₂CH₃, -CH₂CH(CH₃)2, -CHC(CH₃)₃, -CH₂OH, -NH₂, -CH₂NH₂, -N₃, and (C₁-C₄)alkyl; where R₃ represents -CH₃, where R₄ = -H or-OH, and where R₅ = -H, -OH, -Boc, -Fmoc, or -C(=O)O(C₄H₆)NO₂.

Embodiment Q32. The illudofulvene analog of Embodiment Q31, further comprising an Affinity Moiety covalently attached to one or both R₁ and R₂.

Embodiment Q33. The illudofulvene analog of Embodiment Q32, where the Affinity Moiety is selected from the group consisting of an antibody, an antibody fragment, a receptor protein, a peptidic growth factor, an anti-angiogenic protein, a specific binding peptide, protease cleavable peptide, a glycopeptide, a peptide, a peptidic toxin, a protein toxin and an oligonucleotide.

Embodiment Q34. The illudofulvene analog of Embodiment Q32, further comprising a linker moiety adaptedto covalently attach the Affinity Moiety to the illudofulvene analog.

Embodiment Q35. The illudofulvene analog of Embodiment Q34, where the linker moiety is selected from the group consisting of a secondary amino group, a carbonyl group, an ester, and analcohol.

Embodiment Q36. A The composition comprising racemic mixtures of the illudofulvene analog of Embodiment Q31.

Embodiment Q37. The composition comprising enantiomers of the illudofulvene analog of Embodiment Q31.

Embodiment Q38. The composition comprising enantiomers and racemic mixtures of the illudofulvene analog of Embodiment Q31.

Embodiment Q39. The composition comprising the illudofulvene analog of Embodiment Q31 and a physiologically compatible excipient.

Embodiments contemplated herein also include Embodiments R1-R20 following

Embodiment R1. A compound, comprising: an illudofulvene moiety selected from the group consisting of analog 317, analog 318, analog 333, analog 334, analog 335, analog 339, analog 345, analog 346, analog 347, analog 348, analog 351, analog 353, analog 356, analog 357, analog 359, analog 361, analog 362, analog 363, analog 364, analog 368, analog 369, analog 370, analog 371, analog 372, analog 373, analog 374, analog 377, analog 378, analog 379, analog 380, analog 381, analog 382, analog 383, analog 384, analog 389, analog 392, analog 393, analog 397, analog 398, analog 399, analog 401, analog 403, analog 404, analog 405, analog 407, analog 408, analog 409, and analog 410.

Embodiment R2. The composition comprising the compound of Embodiment R1, and a physiologically compatible excipient.

Embodiment R3. The composition comprising racemic mixtures of any enantiomers of the compound of Embodiment R1.

Embodiment R4. The composition comprising the compound of Embodiment R1, in the form of a liposomal particle, a nanoparticle, or a PEGylated compound.

Embodiment R5. The composition comprising the compound of Embodiment R1, and a physiologically compatible carrier.

Embodiment R6. A compound, comprising: an illudofulvene moiety selected from the group consisting of analog 334, analog 362, analog 371, and analog 383.

Embodiment R7. The composition comprising the compound of Embodiment R6, and a physiologically compatible excipient.

Embodiment R8. The composition comprising racemic mixtures of any enantiomers of the compound of Embodiment R6

Embodiment R9. The composition comprising the compound of Embodiment R6, in the form of a liposomal particle, a nanoparticle, or a PEGylated compound.

Embodiment R10. The composition comprising the compound of Embodiment R6, and a physiologically compatible carrier.

Embodiment R11. A compound, comprising: an illudofulvene moiety selected from the group consisting of ((2'S,3'R,6'R)-3'-(((2-((tert-butoxycarbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl methanesulfonate; (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl acetate; and (2'R,3'R,6'R)-3',6'-dihydroxy-2',4',6'-trimethyl-7'-oxo-2',3',6',7'-tetrahydrospiro[cyclopropane-1,5'-indene]-2'-carbaldehyde.

Embodiment R12. The composition comprising the compound of Embodiment R11, and a physiologically compatible excipient.

Embodiment R13. The composition comprising racemic mixtures of any enantiomers of the compound of Embodiment R11.

Embodiment R14. The composition comprising the compound of Embodiment R11, in the form of a liposomal particle, a nanoparticle, or a PEGylated compound.

Embodiment R15. The composition comprising the compound of Embodiment R11, and a physiologically compatible carrier.

Embodiment R16. A compound, comprising: an illudofulvene moiety selected from the group consisting of tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)(hydroxy)carbamate; (2'S,3'R,6'R)-3',6'-dihydroxy-2'-((hydroxyamino)methyl)-2',4',6'-trimethyl-2',3'-dihydrospiro[cyclopropane-1,5'-inden]-7'(6'H)-one;((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl sulfamate; ((6'R)-3'-(((2-((((4-((S)-2-((S)-2-((((9H-fluoren-9-yl)methoxy)carbonyl)amino)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; ((2'S,6'R)-6'-hydroxy-2',6'-dimethyl-4'-methylene-7'-oxo-2',4',6',7'-tetrahydrospiro[cyclopropane-1,5'-inden]-2'-yl)methyl methanesulfonate; ((6'R)-3'-(((2-((((4-((S)-2-((S)-2-amino-3 - methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate, ((2'S,3'S,6'R)-6'-hydroxy-3'-(((2-((((4-((2S,5S)-5-isopropyl-25-(6-methyl-4-vinylpyridin-2-yl)-4,7,23-trioxo-2-(3-ureidopropyl)-10,13,16,19-tetraoxa-3,6,22-triazapentacosanamido)benzyl)oxy)carbonyl)(methyl)amino)ethyl)(methyl)carbamoyl)oxy)-2',4',6'-trimethyl-7'-oxo-2',3',6',7'-tetrahydrospiro[cyclopropane-1,5'-inden]-2'-yl)methyl acetate; ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl trifluoromethanesulfonate, 4-((2S,5S)-14-amino-5-isopropyl-4,7,10,13-tetraoxo-2-(3-ureidopropyl)-3,6,9,12-tetraazatetradecanamido)benzyl ((2'S,3'R,6'R)-6'-hydroxy-2'-(hydroxymethyl)-2',4',6'-trimethyl-7'-oxo-2',3',6',7'-tetrahydrospiro[ cyclopropane-1 ,5'-inden]-3'-yl) ethane-1,2-diylbis(methylcarbamate); and 4-((S)-2-((S)-2-amino-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate), and 4-((S)-2-((S)-2-(6-(2,5-dioxo-2,5-dihydro-1H-pyrrol-1-yl)hexanamido)-3-methylbutanamido)-5-ureidopentanamido)benzyl ((6'R)-6'-hydroxy-2'-(hydroxymethyl)-2',4',6'-tiimethyl-7'-oxo-2',3',6',7'-tetrahydrospiro[cyclopropane-1,5'-inden]-3'-yl) ethane-1,2-diylbis(methylcarbamate).

Embodiment R17. The composition comprising the compound of Embodiment R16, and a physiologically compatible excipient.

Embodiment R18. The composition comprising racemic mixtures of any enantiomers of the compound of Embodiment R16.

Embodiment R19. The composition comprising the compound of Embodiment R16, in the form of a liposomal particle, a nanoparticle, or a PEGylated compound.

Embodiment R20. The composition comprising the compound of Embodiment R16, and a physiologically compatible carrier.

The foregoing description of embodiments of the methods, systems, and components of the present invention has been provided for the purposes of illustration and description. It is not intended to be exhaustive or to limit the invention to the precise forms disclosed. Many modifications and variations will be apparent to one of ordinary skill in the relevant arts. For example, steps performed in the embodiments of the invention disclosed can be performed in alternate orders, certain steps can be omitted, and additional steps can be added. The embodiments were chosen and described in order to best explain the principles of the invention and its practical application, thereby enabling others skilled in the art to understand the invention for various embodiments and with various modifications that are suited to the particular used contemplated. Other embodiments are possible and are covered by the invention. Such embodiments will be apparent to persons skilled in the relevant art(s) based on the teachings contained herein. The breadth and scope of the present invention should not be limited by any of the above-described exemplary embodiments, but should be defined only in accordance with the following claims and their equivalents.

**Table I. Cytotoxic data IC₅₀ values (micromolar, 2 hour exposure, N =3, mean + SD) for Illudin M, analog 108 and analog 110 for cells expressing the estrogen receptor (ER) (MCF7) and cells not expressing the ER (HT29).**

| Analog | HT29 (ER Negative) | MCF7 (ER positive) |
|---|---|---|
| Illudin M | 0.52 ± 0.10 | 0.48 ± 0.13 |
| **108** | > 55 | 14.1 ± 2.8 |
| **110** | > 19 | 2.0 ± 0.1 |

**Table II. Activity of PSA cleavable illudofulvene analogs (210, 215, 216, 221) compared with Illudin S and illudofulvene precursor analogs (204, 207, 211, 212, 213, 214) against PSA negative and PSA positive cell line (48 hour exposure, N=3; mean + SD; IC50 values in nM).**

| Analog | Prostate PC3 (negative PSA) | Prostate DuPro (trace PSA) | Prostate LnCAP (positive PSA) |
|---|---|---|---|
| Illudin S | 16 ± 5 | 11 ± 3 | 15 ± 3 |
| **204** | n.t. | n.t. | 3,300 ± 1,000 |
| **207** | 880 ± 330 | 450 ± 40 | 560 ± 60 |
| **211** | 350 ± 80 | 280 ± 20 | 270 ± 50 |
| **212** | 120 ± 20 | 20 ± 2 | 120 ± 30 |
| **213** | 2,200 ± 100 | 360 ± 80 | 900 ± 200 |
| **214** | 300 ± 50 | 90 ± 10 | 190 ± 30 |
| **210** | 4,700 ± 500 | 3,500 ± 400 | 810 ± 130 |
| **215** | n.t. | n.t. | > 20,000 |
| **216** | 190 ± 10 | 280 ± 60 | 190 ± 30 |
| **221** | > 21,000 | 13,000 ± 1,000 | 800 ± 100 |

| | | | |
|---|---|---|---|
| n.t. denotes not tested | | | |

**Table III. Peptides cleaved by various proteases.**

| Protease | Peptide | SEQ. ID's |
|---|---|---|
| PSA | His-Ser-Ser-Lys-Leu-Gln-X Mu-His-Ser-Ser-Lys-Leu-Gln-X | SEQ. ID. 104 |
| | Mu-His-Ser-Ser-Lys-Leu-Gln-Lys-X | SEQ. ID. 106 |
| | Mu-His-Ser-Ser-Lys-Leu-EDA-Lys-X Mc-His-Ser-Ser-Lys-Leu-Gln-X Mc-His-Ser-Ser-Lys-Leu-Gln-X | SEQ. ID. 108 |
| | Hyp-Ala-Ser-Chg-Gln-Ser-X | SEQ. ID. 111 |
| | Hyp-Ala-Ser-Chg-Gln-Ser-Leu-X Mu-Hyp-Ala-Ser-Chg-Gln-Ser-X Mu-Hyp-Ala-Ser-Chg-Gln-Ser-Leu-X Mc-Hyp-Ala-Ser-Chg-Gln-Ser-X Mc-Hyp-Ala-Ser-Chg-Gln-Ser-Leu-X | SEQ. ID. 116 |
| | Hyp-Ser-Ser-Chg-Gln-Ser-Ser-Pro-X Mu-Hyp-Ser-Ser-Chg-Gln-Ser-Ser-Pro-X Mc-Hyp-Ser-Ser-Chg-Gln-Ser-Ser-Pro-X | SEQ. ID. 127 |
| | 4-O-Ac-Hyp-Ser-Ser-Chg-Gln-Ser-Ser-Pro-X | SEQ. ID. 131 |
| | Arg-Arg-Ser-Ser-Tyr-Tyr-Ser-Gly-X Mu-Arg-Arg-Ser-Ser-Tyr-Tyr-Ser-Gly-X Mc-Arg-Arg-Ser-Ser-Tyr-Tyr-Ser-Gly-X | SEQ. ID. 132 |
| | Mc-Ser-Ser-Lys-Tyr-Gln-Leu-X Mu-Ser-Ser-Lys-Tyr-Gln-Leu-X | SEQ. ID. 136 |
| | N-glutaryl-Hyp-Ala-Ser-chGly-Gln-Ser-Leu Mu-N-glutaryl-Hyp-Ala-Ser-chGly-Gln-Ser-Leu Mc-N-glutaryl-Hyp-Ala-Ser-chGly-Gln-Ser-Leu | SEQ. ID. 137 |
| Caspase-3 | Asp-Glu-Val-Asp-Pro-X Mu-Asp-Glu-Val-Asp-Pro-X Mc-Asp-Glu-Val-Asp-Pro-X | SEQ. ID. 138 |
| | Lys-Gly-Ser-Gly-Asp-Val-Glu-Gly-X Mu-Lys-Gly-Ser-Gly-Asp-Val-Glu-Gly-X Mc-Lys-Gly-Ser-Gly-Asp-Val-Glu-Gly-X | SEQ. ID. 139 |
| Cathepsin B | PLE-X | |
| | Gly-Phe-Leu-Gly-X | SEQ. ID. 141 |
| | Lys-Lys-Phe-D-Ala-X | SEQ. ID. 142 |
| | D-Ala-Phe-Lys-Lys-X Mc-Poly-L-glutamic acid-X | SEQ. ID. 144 |
| | Mc-Gly-Phe-Leu-Gly-X Mc-Lys-Lys-Phe-D-Ala-X Mc-D-Ala-Phe-Lys-Lys-X Mu-Poly-L-glutamic acid-X Mu-Gly-Phe-Leu-Gly-X Mu-Lys-Lys-Phe-D-Ala-X Mu-D-Ala-Phe-Lys-Lys-X | SEQ. ID. 145 |
| | Val-Cit-X | |
| FAP | Lys-Gln-Glu-Gln-Asn-Pro-Gly-Ser-Thr-X Mu-Lys-Gln-Glu-Gln-Asn-Pro-Gly-Ser-Thr-X Mc-Lys-Gln-Glu-Gln-Asn-Pro-Gly-Ser-Thr-X | SEQ. ID. 146 |
| Kallikrein 2 | Gly-Lys-Ala-Phe-Arg-Arg-X Mu-Gly-Lys-Ala-Phe-Arg-Arg-X Mc-Gly-Lys-Ala-Phe-Arg-Arg-X | SEQ. ID. 171 |
| MMP-2/-9/ | Glu-Pro-Cit-Gly-Hof-Tyr-Leu-X Mu-Glu-Pro-Cit-Gly-Hof-Tyr-Leu-X Mc-Glu-Pro-Cit-Gly-Hof-Tyr-Leu-X | SEQ. ID. 172 |
| | Gly-Ile-Leu-Gly-Val-Pro-X Mu-Gly-Ile-Leu-Gly-Val-Pro-X | SEQ. ID. 173 |
| | Mc-Gly-Ile-Leu-Gly-Val-Pro-X | |
| | Gly-Pro-Leu-Gly-Ile-Ala-Gly-Gln-X Mu-Gly-Pro-Leu-Gly-Ile-Ala-Gly-Gln-X Mc-Gly-Pro-Leu-Gly-Ile-Ala-Gly-Gln-X | SEQ. ID. 174 |
| MMP-7 | Lys-Arg-Ala-Leu-Gly-Leu-Pro-Gly Mu-Lys-Arg-Ala-Leu-Gly-Leu-Pro-Gly Mc-Lys-Arg-Ala-Leu-Gly-Leu-Pro-Gly | SEQ. ID. 175 |
| | Arg-Pro-Leu-Ala-Leu-Trp-Arg-Ser Mu-Arg-Pro-Leu-Ala-Leu-Trp-Arg-Ser Mc-Arg-Pro-Leu-Ala-Leu-Trp-Arg-Ser | SEQ. ID. 176 |
| TOP | Ala-L-L-Ala-L-Ile Mu-Ala-L-L-Ala-L-Ile Mc-Ala-L-L-Ala-L-Ile | |
| uPA | D-Ala-Phe-Lys or D-Ala-Phe-Lys-PABC | SEQ. ID. 177 |
| Cathepsin K | Gly-Gly-Pro-Nle-X | SEQ. ID. 178 |
| | Mu-Gly-Gly-Pro-Nle-X Mc-Gly-Gly-Pro-Nle-X | |
| Plasmin | D-Ala-Phe-Lys-Lys-X | SEQ. ID. 179 |
| | Mu-D-Ala-Phe-Lys-Lys-X Mc-D-Ala-Phe-Lys-Lys-X | |
| | D-Ala-Phe-Lys-X Mu-D-Ala-Phe-Lys-X Mc-D-Ala-Phe-Lys-X | |
| Thrombin | Poly-L-Lys-Gly-D-Phe-Pip-Arg-Ser-Gly-Gly- Gly-Gly-Gly-X | SEQ. ID. 180 |
| Trypsin | Poly-L-Lysine-Gly-Ala-Ser-D-Arg-Phe-Thr-Gly- X | SEQ. ID. 181 |

In **Table III**, the letter 'X' denotes the end attached to the medicant, Chg denotes cyclohexyl glycine, Cit denotes citrulline, EDA denotes ethanyl-D-Alanine, Hof denotes homophenylalanine, Hyp denotes 4-hydroxyproline, Mc denotes morpholinocarbonyl (carboxy-terminal protecting group), Mu denotes 4-morpholine-carbonyl (amino-terminal protecting group), Nle denotes norleucine, PABC denotes para-aminobenzoylcarboxyl, PLE denotes Poly-L-glutamic acid, and Pip denotes piperidine.

**Table IV. Mechanisms of Drug Resistance.**

| **Mechanism of Multi-drug Resistance** | **Resistance to** illudofulvenes |
|---|---|
| Gp170/MDR1 | No |
| Gp180/MRP | No |
| Topoisomerase I | No |
| Topoisomerase II | No |
| MVP/LRP (vault) | No |
| Thiol content/GST pi | No |
| DNA repair | No |
| Myc expression | No |
| Bcl-2 expression | No |
| BRCA status | No |
| P53 status | No |
| P21 status | No |
| MGMT expression | No |
| Microtubulin alteration | No |

**Table V. IUPAC names of the Illudofulvene analogs.**

| Entry # | Illudofulvene Analog | IUPAC Name of Illudofulvene Analog |
|---|---|---|
| 1 | 001 | (R)-6'-hydroxy-2',4',6'-trimethylspiro[cyclopropane-1,5'-inden]-7'(6'H)-one |
| 2 | 002 | (6'*R*)-6'-hydroxy-3'-(hydroxymethyl)-2',4',6'-trimethylspiro[cyclopropane-1,5'-inden]-7'(6'*H*)-one |
| 3 | 003 | (6'R,6‴R)-3',3‴-methylenebis(6'-hydroxy-2',4',6'-trimethylspiro[cyclopropane-1,5'-inden]-7'(6'H)-one) |
| 4 | 004 | (R)-3'-bromo-6'-hydroxy-2',4',6'-trimethylspiro[cyclopropane-1,5'-inden]-7'(6'H)-one |
| 5 | 005 | (R)-6'-hydroxy-3'-iodo-2',4',6'-trimethylspiro[cyclopropane-1,5'-inden]-7'(6'H)-one |
| 6 | 006 | (R)-6'-hydroxy-3 '-(4-hydroxybenzyl)-2',4',6'-trimethylspiro[cyclopropane-1,5'-inden]-7'(6'H)-one |
| 7 | 007 | (R)-6'-hydroxy-3'-(4-methoxybenzyl)-2',4',6'-trimethylspiro[cyclopropane-1,5'-inden]-7'(6'H)-one |
| 8 | 008 | (R)-((6'-hydroxy-2',4',6'-trimethyl-7'-oxo-6',7'-dihydrospiro[cyclopropane-1,5'-inden]-3'-yl)methoxy)methyl acetate |
| 9 | 009 | (R)-6'-hydroxy-3'-(3-hydroxypropyl)-2',4',6'-trimethylspiro[cyclopropane-1,5'-inden]-7'(6'H)-one |
| 10 | 010 | (R)-3-(6'-hydroxy-2',4',6'-trimethyl-7'-oxo-6',7'-dihydrospiro[cyclopropane-1,5'-inden]-3'-yl)propanal |
| 11 | 011 | (R)-6'-hydroxy-2',4',6'-trimethyl-7'-oxo-6',7'-dihydrospiro[cyclopropane-1,5'-indene]-3'-carbaldehyde |
| 12 | 012 | (R)-6'-hydroxy-2',4',6'-trimethyl-3'-nitrospiro[cyclopropane-1,5'-inden]-7'(6'H)-one |
| 13 | 013 | 4-hydroxy-5-(((R)-6'-hydroxy-2',4',6'-trimethyl-7'-oxo-6',7'-dihydrospiro[cyclopropane-1,5'-inden]-3'-yl)methyl)cyclohexane-1,3-dicarbaldehyde |
| 14 | 014 | (4a'S,7'R,9b'S)-7'-hydroxy-4a',7',9'-trimethyl-4a',9b'-dihydro-4'H-spiro[cyclopropane-1,8'-indeno[1,2-d][1,3]dioxin]-6'(7'H)-one |
| 15 | 015 | (R)-3'-(hydroxymethyl)-2',4',6'-trimethyl-7'-oxo-6',7'-dihydrospiro[cyclopropane-1,5'-inden]-6'-yl acetate |
| 16 | 016 | (R)-3'-(ethoxymethyl)-6'-hydroxy-2',4',6'-trimethylspiro[cyclopropane-1,5'-inden]-7'(6'H)-one |
| 17 | 017 | (6'R,6‴R)-3',3‴-(oxybis(methylene))bis(6'-hydroxy-2',4',6'-trimethylspiro[cyclopropane-1,5'-inden]-7'(6'H)-one) |
| 18 | 018 | (R)-6'-hydroxy-2',4',6'-trimethyl-3'-((((2R,3S,4R,5R,6S)-3,4,5-trihydroxy-6-(hydroxymethyl)tetrahydro-2H-pyran-2-yl)oxy)methyl)spiro[cyclopropane-1,5'-inden]-7'(6'H)-one |
| 19 | 019 | (6'R)-3'-((2,3-dihydroxypropoxy)methyl)-6'-hydroxy-2',4',6'-trimethylspiro[cyclopropane-1,5'-inden]-7'(6'H)-one |
| 20 | 020 | (R)-3'-((2-bromoethoxy)methyl)-6'-hydroxy-2',4',6'-trimethylspiro[cyclopropane-1,5'-inden]-7'(6'H)-one |
| 21 | 021 | (R)-6'-hydroxy-3'-(((2-methoxypropan-2-yl)oxy)methyl)-2',4',6'-trimethylspiro[cyclopropane-1,5'-inden]-7'(6'H)-one |
| 22 | 022 | (R)-6'-hydroxy-3'-((2-hydroxyethoxy)methyl)-2',4',6'-trimethylspiro[cyclopropane-1,5'-inden]-7'(6'H)-one |
| 23 | 023 | (R)-6'-hydroxy-3'-(((4-hydroxyphenyl)thio)methyl)-2',4',6'-trimethylspiro[cyclopropane-1,5'-inden]-7'(6'H)-one |
| 24 | 024 | (R)-3'-((benzylthio)methyl)-6'-hydroxy-2',4',6'-trimethylspiro[cyclopropane-1,5'-inden]-7'(6'H)-one |
| 25 | 025 | methyl (R)-2-(((6'-hydroxy-2',4',6'-trimethyl-7'-oxo-6',7'-dihydrospiro[cyclopropane-1,5'-inden]-3'-yl)methyl)thio)acetate |
| 26 | 026 | (R)-6'-hydroxy-2',4',6'-trimethyl-3'-((p-tolylthio)methyl)spiro[cyclopropane-1,5'-inden]-7'(6'H)-one |
| 27 | 027 | (R)-(6'-hydroxy-2',4',6'-trimethyl-7'-oxo-6',7'-dihydrospiro[cyclopropane-1,5'-inden]-3'-yl)methyl phenyl carbonate |
| 28 | 028 | (R)-(6'-hydroxy-2',4',6'-trimethyl-7'-oxo-6',7'-dihydrospiro[cyclopropane-1,5'-inden]-3'-yl)methyl benzoate |
| 29 | 029 | (R)-2-(((6'-hydroxy-2',4',6'-trimethyl-7'-oxo-6',7'-dihydrospiro[cyclopropane-1,5'-inden]-3'-yl)methyl)thio)acetic acid |
| 30 | 030 | methyl (R)-2-(((6'-hydroxy-1 '-((2-methoxy-2-oxoethyl)thio)-2',4',6'-trimethyl-7'-oxo-6',7'-dihydrospiro[cyclopropane-1,5'-inden]-3'-yl)methyl)thio)acetate |
| 31 | 031 | methyl 2-((((6'R)-6',7a'-dihydroxy-1-((2-methoxy-2-oxoethyl)thio)-2',4',6'-trimethyl-7'-oxo-1',6',7',7a'-tetrahydrospiro[cyclopropane-1,5'-inden]-3'-yl)methyl)thio)acetate |
| 32 | 032 | (6'R)-3'-(((2,3-dihydroxypropyl)thio)methyl)-6'-hydroxy-2',4',6'-trimethylspiro[cyclopropane-1,5'-inden]-7'(6'H)-one |
| 33 | 033 | 7'-methyl-4H-dispiro[cyclopropane-1,6'-indene-5',2"-[1,3]dioxolan]-4'-one |
| 34 | 034 | (R)-(6'-hydroxy-2',4',6'-trimethyl-7'-oxo-6',7'-dihydrospiro[cyclopropane-1,5'-inden]-3'-yl)methyl acetate |
| 35 | 035 | 6'-hydroxy-4'-methylspiro[cyclopropane-1,5'-inden]-7'(6'H)-one |
| 36 | 036 | (R)-3'-((1H-imidazol-1-yl)methyl)-6'-hydroxy-2',4',6'-trimethylspiro[cyclopropane-1,5'-inden]-7'(6'H)-one |
| 37 | 037 | 1-carboxy-2-((((R)-6'-hydroxy-2',4',6'-trimethyl-7'-oxo-6',7'-dihydrospiro[cyclopropane-1,5'-inden]-3'-yl)methyl)thio)ethan-1-aminium |
| 38 | 038 | (R)-3-(6'-hydroxy-2',4',6'-trimethyl-7'-oxo-6',7'-dihydrospiro[cyclopropane-1,5'-inden]-3'-yl)propanoic acid |
| 39 | 039 | (R)-3'-(3,3-dimethoxypropyl)-6'-hydroxy-2',4',6'-trimethylspiro[cyclopropane-1,5'-inden]-7'(6'H)-one |
| 40 | 040 | (R)-3'-(3,3-diethoxypropyl)-6'-hydroxy-2',4',6'-trimethylspiro[cyclopropane-1,5'-inden]-7'(6'H)-one |
| 41 | 041 | (R,Z)-3-(6'-hydroxy-2',4',6'-trimethyl-7'-oxo-6',7'-dihydrospiro[cyclopropane-1,5'-inden]-3'-yl)acrylaldehyde |
| 42 | 042 | (R)-3'-(hydroxymethyl)-4',6'-dimethyl-6'-((triethylsilyl)oxy)spiro[cyclopropane-1,5'-inden]-7'(6'H)-one |
| 43 | 043 | (R)-6'-hydroxy-2',4',6'-trimethyl-3'-(((triethylsilyl)oxy)methyl)spiro[cyclopropane-1,5'-inden]-7'(6'H)-one |
| 44 | 044 | (R)-2',4',6'-trimethyl-6'-((triethylsilyl)oxy)-3'-(((triethylsilyl)oxy)methyl)spiro[cyclopropane-1,5'-inden]-7'(6'H)-one |
| 45 | 045 | methyl 2-((7-hydroxy-5-(2-hydroxyethyl)-3-(hydroxymethyl)-2,4,6-trimethyl-1H-inden-1-yl)thio)acetate |
| 46 | 046 | (R)-3-(6'-hydroxy-2',4',6'-trimethyl-7'-oxo-6',7'-dihydrospiro[cyclopropane-1,5'-inden]-3'-yl)propyl acetate |
| 47 | 047 | (6-R)-3'-(2-(1,7-dihydroxy-2,4,6-trimethyl-1H-inden-5-yl)ethyl)-6'-hydroxy-2',4',6'-trimethylspiro[cyclopropane-1,5'-inden]-7'(6'H)-one |
| 48 | 048 | (R)-6'-hydroxy-2',4',6'-trimethyl-1'-(p-tolylthio)-3'-((p-tolylthio)methyl)spiro[cyclopropane-1,5'-inden]-7'(6'H)-one |
| 49 | 049 | (R)-6'-hydroxy-2',4',6'⁻trimethyl-3'-(p-tolylthio)spiro[cyclopropane-1,5'-inden]-7'(6'H)-one |
| 50 | 050 | (R)-6'-hydroxy-2',4',6'-trimethyl-1',3'-bis(p-tolylthio)spiro[cyclopropane-1,5'-inden]-7'(6'H)-one |
| 51 | 051 | (R)-2-(2-(((6'-hydroxy-2',4',6'-trimethyl-7'-oxo-6',7'-dihydrospiro[cyclopropane-1,5'-inden]-3'-yl)methyl)thio)acetoxy)ethyl2-mercaptoacetate |
| 52 | 052 | ethane-1,2-diyl bis(2-((((R)-6'-hydroxy-2',4',6'-trimethyl-7'-oxo-6',7'-dihydrospiro[cyclopropane-1,5'-inden]-3'-yl)methyl)thio)acetate) |
| 53 | 053 | (R)-3'-((2-(2-bromoethoxy)ethoxy)methyl)-6'-hydroxy-2',4',6'-trimethylspiro[cyclopropane-1,5'-inden]-7'(6'H)-one |
| 54 | 054 | (R)-6'-hydroxy-1'-(((R)-6'-hydroxy-2',4',6'-trimethyl-7'-oxo-6',7'-dihydrospiro[cyclopropane-l,5'-inden]-3'-yl)methyl)-3'-(hydroxymethyl)-2',4',6'-trimethylspiro[cyclopropane-1,5'-inden]-7'(6'H)-one |
| 55 | 055 | 5-(2-hydroxyethyl)-1-((4-hydroxyphenyl)thio)-3-(((4-hydroxyphenyl)thio)methyl)-2,4,6-trimethyl-1H-inden-7-ol |
| 56 | 056 | (R)-6'-hydroxy-3'-((4-hydroxyphenyl)thio)-2',4',6'-trimethylspiro[cyclopropane-1,5'-inden]-7'(6'H)-one |
| 57 | 057 | (R)-6'-hydroxy-1'-((4-hydroxyphenyl)thio)-2',4',6'-trimethylspiro[cyclopropane-1,5'-inden]-7'(6'H)-one |
| 58 | 058 | (R)-6'-hydroxy-1',3'-bis((4-hydroxyphenyl)thio)-2',4',6'-trimethylspiro[cyclopropane-1,5'-inden]-7'(6'H)-one |
| 59 | 059 | (6'S,7'R)-4'-methyl-6'-((triethylsilyl)oxy)-6',7'-dihydrospiro[cyclopropane-1,5'-inden]-7'-ol |
| 60 | 060 | (R)-7'-methyl-4'H-dispiro[cyclopropane-1,6'-indene-5',2"-[1,3]dioxolan]-4'-ol |
| 61 | 061 | (S)-4'-methyl-6'-((triethylsilyl)oxy)spiro[cyclopropane-1,5'-inden]-7'(6'H)-one |
| 62 | 062 | (R)-6'-hydroxy-2'-(hydroxymethyl)-4',6'-dimethylspiro[cyclopropane-1,5'-inden]-7'(6'H)-one |
| 63 | 063 | (R)-6'-hydroxy-2',3'-bis(hydroxymethyl)-4',6'-dimethylspiro[cyclopropane-1,5'-inden]-7'(6'H)-one |
| 64 | 064 | N-acetyl-S-(((R)-6'-hydroxy-2',4',6'-trimethyl-7'-oxo-6',7'-dihydrospiro[cyclopropane-1,5'-inden]-3'-yl)methyl)-L-cysteine |
| 65 | 065 | (R)-2-acetamido-3-((((R)-6'-hydroxy-4',6'-dimethyl-7'-oxo-6',7'-dihydrospiro[cyclopropane-1,5'-inden]-3'-yl)methyl)thio)-N-((S)-1-phenylethyl)propanamide |
| 66 | 066 | (S)-2-acetamido-3-((((R)-6'-hydroxy-2',4',6'-trimethyl-7'-oxo-6',7'-dihydrospiro[cyclopropane-1,5'-inden]-3'-yl)methyl)thio)-N-((S)-1-phenylethyl)propanamide |
| 67 | 067 | 4-methyl-2,3-dihydro-5H-indeno[5,6-b]furan-5-one |
| 68 | 068 | 5-hydroxy-6-(2-hydroxyethyl)-7-methyl-1H-inden-1-one |
| 69 | 069 | 5-(2-hydroxyethoxy)-6-(2-hydroxyethyl)-7-methyl-1H-inden-1-one |
| 70 | 070 | (3a'R,4'R)-4'-hydroxy-7'-methyl-3a',4'-dihydro-1'H-dispiro[cyclopropane-1,6'-indene-5',2"-[1,3]dioxolan]-1'-one |
| 71 | 071 | (3a'R,4'R)-7'-methyl-4'-((triethylsilyl)oxy)-3a',4'-dihydro-1'H-dispiro[cyclopropane-1,6'-indene-5',2"-[1,3]dioxolan]-1'-one |
| 72 | 072 | (7'R,7a'R)-7'-hydroxy-4'-methyl-7',7a'-dihydrospiro[cyclopropane-1,5'-indene]-3',6'-dione |
| 73 | 073 | (7'R,7a'R)-4'-methyl-7'-((triethylsilyl)oxy)-7',7a'-dihydrospiro[cyclopropane-1,5'-indene]-3',6'-dione |
| 74 | 074 | (6'R)-3'-((((2,2-dimethyl-1,3-dioxolan-4-yl)methyl)thio)methyl)-6'-hydroxy-2',4',6'-trimethylspiro[cyclopropane-1,5'-inden]-7'(6'H)-one |
| 75 | 075 | (R)-(6'-hydroxy-4',6'-dimethyl-7'-oxo-6',7'-dihydrospiro[cyclopropane-1,5'-inden]-2'-yl)methyl acetate |
| 76 | 076 | (R)-(6'-hydroxy-4',6'-dimethyl-7'-oxo-6',7'-dihydrospiro[cyclopropane-1,5'-indene]-2',3'-diyl)bis(methylene) diacetate |
| 77 | 077 | (R)-(6'-hydroxy-3'-(hydroxymethyl)-4',6'-dimethyl-7'-oxo-6',7'-dihydrospiro[cyclopropane-1,5'-inden]-2'-yl)methyl acetate |
| 78 | 078 | (R)-(6'-hydroxy-2'-(hydroxymethyl)-4',6'-dimethyl-7'-oxo-6',7'-dihydrospiro[cyclopropane-1,5'-inden]-3'-yl)methyl acetate |
| 79 | 079 | (R)-6'-hydroxy-2'-(methoxymethyl)-4',6'-dimethylspiro[cyclopropane-1,5'-inden]-7'(6'H)-one |
| 80 | 080 | (R)-6'-hydroxy-3'-(methoxymethyl)-2',4',6'-trimethylspiro[cyclopropane-1,5'-inden]-7'(6'H)-one |
| 81 | 081 | (R)-6'-hydroxy-2'-(hydroxymethyl)-3'-(methoxymethyl)-4',6'-dimethylspiro[cyclopropane-1,5'-inden]-7'(6'H)-one |
| 82 | 082 | (R)-6'-hydroxy-2',3'-bis(methoxymethyl)-4',6'-dimethylspiro[cyclopropane-1,5'-inden]-7'(6'H)-one |
| 83 | 083 | (R)-2-acetamido-3-((((R)-6'-hydroxy-2',4',6'-trimethyl-7'-oxo-6',7'-dihydrospiro[cyclopropane-1,5'-inden]-3'-yl)methyl)thio)-N-((S)-1-((2-(((S)-4-methyl-1-oxopentan-2-yl)amino)-2-oxoethyl)amino)-1-oxo-3-phenylpropan-2-yl)propanamide |
| 84 | 084 | (S)-2-((R)-2-acetamido-3-((((R)-6'-hydroxy-2',4',6'-trimethyl-7'-oxo-6',7'-dihydrospiro[cyclopropane-1,5'-inden]-3'-yl)methyl)thio)propanamido)-4-methyl-N-(2-oxo-2-(((R)-1-oxo-3-phenylpropan-2-yl)amino)ethyl)pentanamide |
| 85 | 085 | (S)-2-((R)-2-acetamido-3-((((R)-6'-hydroxy-2',4',6'-trimethyl-7'-oxo-6',7'-dihydrospiro[cyclopropane-1,5'-inden]-3'-yl)methyl)thio)propanamido)-4-methyl-N-((S)-4-methyl-1-oxo-1-(((R)-1-oxo-3 -phenylpropan-2-yl)amino)pentan-2-yl)pentanamide |
| 86 | 086 | (R)-2-acetamido-3-((((R)-6'-hydroxy-2',4',6'-trimethyl-7'-oxo-6',7'-dihydrospiro[cyclopropane-1,5'-inden]-3'-yl)methyl)thio)-N-(2-oxo-2-(((R)-1-oxo-3-phenylpropan-2-yl)amino)ethyl)propanamide |
| 87 | 087 | (S)-2-((R)-2-acetamido-3-((((R)-6'-hydroxy-2',4',6'-trimethyl-7'-oxo-6',7'-dihydrospiro[cyclopropane-1,5'-inden]-3'-yl)methyl)thio)propanamido)-4-methyl-N-((R)-4-methyl-1-(((S)-4-methyl-1-oxopentan-2-yl)amino)-1-oxopentan-2-yl)pentanamide |
| 88 | 088 | (R)-(6'-acetoxy-2',4',6'-trimethyl-7'-oxo-6',7'-dihydrospiro[cyclopropane-1,5'-inden]-3'-yl)methyl acetate |
| 89 | 089 | N5-((R)-1-((carboxymethyl)amino)-3-((((R)-6'-hydroxy-2',4',6'-trimethyl-7'-oxo-6',7'-dihydrospiro[cyclopropane-1,5'-inden]-3'-yl)methyl)thio)-1-oxopropan-2-yl)-D-glutamine |
| 90 | 090 | (R)-2'-(hydroxymethyl)-4',6'-dimethyl-7'-oxo-6',7'-dihydrospiro[cyclopropane-1,5'-inden]-6'-ylacetate |
| 91 | 091 | (R)-3-(6'-hydroxy-2',4',6'-trimethyl-7'-oxo-6',7'-dihydrospiro[cyclopropane-1,5'-inden]-3'-yl)propanoic acid |
| 92 | 092 | (R)-6'-hydroxy-3'-(3-methoxypropyl)-2',4',6'-trimethylspiro[cyclopropane-1,5'-inden]-7'(6'H)-one |
| 93 | 093 | (R)-3'-(3,3-diethoxypropyl)-6'-hydroxy-2',4',6'-trimethylspiro[cyclopropane-1,5'-inden]-7'(6'H)-one |
| 94 | 094 | (R)-3-(6'-hydroxy-2',4',6'-trimethyl-7'-oxo-6',7'-dihydrospiro[cyclopropane-1,5'-inden]-3'-yl)propylacetate |
| 95 | 095 | (R)-6'-hydroxy-3'-(3-methoxypropyl)-2',4',6'-trimethylspiro[cyclopropane-1,5'-inden]-7'(6'H)-one |
| 96 | 096 | (R)-6'-hydroxy-2',4',6'-trimethyl-3'-(((1-methyl-1H-imidazol-2-yl)thio)methyl)spiro[cyclopropane-1,5'-inden]-7'(6'H)-one |
| 97 | 097 | S-(((R)-6'-hydroxy-2',4',6'-trimethyl-7'-oxo-6',7'-dihydrospiro[cyclopropane-1,5'-inden]-3'-yl)methyl)homocysteine |
| 98 | 098 | ((S)-3-((((R)-6'-hydroxy-2',4',6'-trimethyl-7'-oxo-6',7'-dihydrospiro[cyclopropane-1,5'-inden]-3'-yl)methyl)thio)-2-methylpropanoyl)proline |
| 99 | 099 | (2'S,6'R)-6'-hydroxy-2'-(hydroxymethyl)-2',4',6'-trimethylspiro[cyclopropane-1,5'-indene]-3',7'(2'H,6'H)-dione |
| 100 | 100 | S-(((R)-6'-hydroxy-2',4',6'-trimethyl-7'-oxo-6',7'-dihydrospiro[cyclopropane-1,5'-inden]-3'-yl)methyl)-N-S-(((R)-6'-hydroxy-2',4',6'-trimethyl-7'-oxo-6',7'-dihydrospiro[cyclopropane-1,5'-inden]-3'-yl)methyl)cysteinyl-L-asparaginylglycyl-L-arginylcysteine |
| 101 | 101 | S-(((R)-6'-hydroxy-2',4',6'-trimethyl-7'-oxo-6',7'-dihydrospiro[cyclopropane-1,5'-inden]-3'-yl)methyl)-N-S-(((R)-6'-hydroxy-2',4',6'-trimethyl-7'-oxo-6',7'-dihydrospiro[cyclopropane-1,5'-inden]-3'-yl)methyl)cysteinyl-L-arginylglycyl-L-asparaginyl cysteine |
| 102 | 102 | S-(((R)-6'-hydroxy-2',4',6'-trimethyl-7'-oxo-6',7'-dihydrospiro[cyclopropane-1,5'-inden]-3'-yl)methyl)cysteinyl-L-asparaginylglycyl-L-arginylcysteine |
| 103 | 103 | (R)-(6'-acetoxy-2'-(hydroxymethyl)-4',6'-dimethyl-7'-oxo-6',7'-dihydrospiro[cyclopropane-1,5'-inden]-3'-yl)methyl acetate |
| 104 | 104 | (R)-8'-hydroxy-6',8'-dimethyl-1',5'-dihydrospiro[cyclopropane-1,7'-indeno[1,2-e][1,3]dioxepin]-9'(8'H)-one |
| 105 | 105 | (E)-2-((2R,4S)-4-hydroxy-2-((1R,2S)-2-hydroxy-4,4-dimethylcyclopentyl)-2-methylcyclobutylidene)propanal |
| 106 | 106 | 5-(((3'S,6'R)-6'-hydroxy-2',2',4',6;-tetramethyl-7'-oxo-2',3',6',7'-tetrahydrospiro[cyclopropane-1,5'-inden]-3'-yl)oxy)-5-oxopentanoic acid |
| 107 | 107 | (3'S,6'R)-6'-hydroxy-2',2',4',6'-tetramethyl-7'-oxo-2',3',6',7'-tetrahydrospiro[cyclopropane-1,5'-inden]-3'-yl((13S)-13-methyl-17-oxo-7,8,9,11,12,13,14,15,16,17-decahydro-6H-cyclopenta[a]phenanthren-3 -yl) glutarate |
| 108 | 108 | (13S)-17-hydroxy-13-methyl-7,8,9,11,12,13,14,15,16,17-decahydro-6H-cyclopenta[a]phenanthren-3-yl 3-((R)-6'-hydroxy-2',4',6'-trimethyl-7'-oxo-6',7'-dihydrospiro[cyclopropane-1,5'-inden]-3'-yl)propanoate |
| 109 | 109 | (13S)-17-hydroxy-13-methyl-7,8,9,11,12,13,14,15,16,17-decahydro-6H-cyclopenta[a]phenanthren-3-yl ((3'S,6'R)-6'-hydroxy-2',2',4',6'-tetramethyl-7'-oxo-2',3 ',6',7'-tetrahydrospiro[cyclopropane-1,5'-inden]-3'-yl) glutarate |
| 110 | 110 | (13 S)-13-methyl-17-oxo-7,8,9,11,12,13,14,15,16,17-decahydro-6H-cyclopenta[a]phenanthren-3-yl3-((R)-6'-hydroxy-2',4',6'-trimethyl-7'-oxo-6',7'-dihydrospiro[cyclopropane-l,5'-inden]-3'-yl)propanoate |
| 111 | 111 | (10R,13S)-10,13-dimethyl-3-oxo-2,3,6,7,8,9,10,11,12,13,14,15,16,17-tetradecahydro-1H-cyclopenta[a]phenanthren-17-yl 3-(6'-hydroxy-2',4'-dimethyl-7'-oxo-6',7'-dihydrospiro[cyclopropane-1,5'-inden]-3'-yl)propanoate |
| 112 | 112 | (13 S)-1 0, 13-dimethyl-17-oxohexadecahydro-1H-cyclopenta[a]phenanthren-3-yl 3-((R)-6'-hydroxy-2',4',6'-trimethyl-7'-oxo-6',7'-dihydrospiro[cyclopropane-1,5'-inden]-3'-yl)propanoate |
| 113 | 113 | (R)-3'-(but-3-en-l-yl)-6'-hydroxy-2',4',6'-trimethylspiro[cyclopropane-1,5'-inden]-7'(6'H)-one |
| 114 | 114 | (6'R)-6'-hydroxy-2',4',6'-trimethyl-3'-(2-(oxiran-2-yl)ethyl)spiro[cyclopropane-1,5'-inden]-7'(6'H)-one |
| 115 | 115 | (R)-3-(6'-hydroxy-2',4',6'-trimethyl-7'-oxo-6',7'-dihydrospiro[cyclopropane-1,5'-inden]-3'-yl)propanal oxime |
| 116 | 116 | (R)-3'-(tert-butoxymethyl)-6'-hydroxy-2',4',6'-trimethylspiro[cyclopropane-1,5'-inden]-7'(6'H)-one |
| 117 | 117 | 5-(((2'S,6'R)-3'-((4-carboxybutanoyl)oxy)-6'-hydroxy-2',4',6'-trimethyl-7'-oxo-2',3',6',7'-tetrahydrospiro[cyclopropane-1,5'-inden]-2'-yl)methoxy)-5-oxopentanoic acid |
| 118 | 118 | 5-(((2'S,6'R)-2'-(((3,5-dinitrobenzoyl)oxy)methyl)-6'-hydroxy-2',4',6'-trimethyl-7'-oxo-2',3',6',7'-tetrahydrospiro[cyclopropane-1,5'-inden]-3'-yl)oxy)-5-oxopentanoic acid |
| 119 | 119 | (6'R)-3'-(3,4-dihydroxybutyl)-6'-hydroxy-2',4',6'-trimethylspiro[cyclopropane-1,5'-inden]-7'(6'H)-one |
| 120 | 120 | (R)-6'-hydroxy-3'-(3-((3-((S)-6'-hydroxy-2',4',6'-trimethyl-7'-oxo-6',7'-dihydrospiro[cyclopropane-1,5'-inden]-3 yl)propylidene)hydrazineylidene)propyl)-2',4',6'-trimethylspiro[cyclopropane-1,5'-inden]-7'(6'H)-one |
| 121 | 121 | (R)-2-(3-(6'-hydroxy-2',4',6'-trimethyl-7'-oxo-6',7'-dihydrospiro[cyclopropane-1,5'-inden]-3'-yl)propylidene)hydrazine-1-carboxamide |
| 122 | 122 | (R)-6'-hydroxy-2',4',6'-trimethyl-3'-(3-(2-phenylhydrazineylidene)propyl)spiro[cyclopropane-1,5'-inden]-7'(6'H)-one |
| 123 | 123 | (R)-N'-(3-(6'-hydroxy-2',4',6'-trimethyl-7'-oxo-6',7'-dihydrospiro[cyclopropane-1,5'-inden]-3'-yl)propylidene)-4-methylbenzenesulfonohydrazide |
| 124 | 124 | (R)-3-(6'-hydroxy-2',4',6'-trimethyl-7'-oxo-6',7'-dihydrospiro[cyclopropane-1,5'-inden]-3'-yl)propanal O-acetyl oxime |
| 125 | 125 | (R)-3'-(3-(2-(2,4-dinitrophenyl)hydrazineylidene)propyl)-6'-hydroxy-2',4',6'-trimethylspiro[cyclopropane-1,5'-inden]-7'(6'H)-one |
| 126 | 126 | (R)-6'-hydroxy-2',4',6'-trimethyl-7'-oxo-6',7'-dihydrospiro[cyclopropane-1,5'-indene]-3'-carbaldehyde oxime |
| 127 | 127 | 2-hydroxy-4-((R)-6'-hydroxy-2',4',6'-trimethyl-7'-oxo-6',7'-dihydrospiro[cyclopropane-1,5'-inden]-3'-yl)butanenitrile |
| 128 | 128 | (6'R)-6'-hydroxy-3'-(3-hydroxybutyl)-2',4',6'-trimethylspiro[cyclopropane-1,5'-inden]-7'(6'H)-one |
| 129 | 129 | (6'R)-2',4',6'-trimethyl-6',7'-dihydrospiro[cyclopropane-1,5'-indene]-6',7'-diol |
| 130 | 130 | (R)-6'-hydroxy-3'-(3-(hydroxyamino)propyl)-2',4',6'-trimethylspiro[cyclopropane-1,5'-inden]-7'(6'H)-one |
| 131 | 131 | (R)-N-benzyl-3-(6'-hydroxy-2',4',6'-trimethyl-7'-oxo-6',7'-dihydrospiro[cyclopropane-1,5'-inden]-3'-yl)propanamide |
| 132 | 133 | (E)-7-(chloromethylene)-5-hydroxy-5,9-dimethylspiro[3.5]non-8-en-6-one |
| 133 | 134 | (E)-6-(chloromethylene)-4-hydroxy-4,8-dimethylspiro[2.5]oct-7-en-5-one |
| 134 | 135 | ((2'S,6'R)-3',6'-dihydroxy-2',4',6'-tnmethyl-7'-oxo-2',3',6',7'-tetrahydrospiro[cyclopropane-1,5'-inden]-2'-yl)methyl 4-nitrobenzoate |
| 135 | 136 | ((2'S,6'R)-3',6'-dihydroxy-2',4',6'-trimethyl-7'-oxo-2',3',6',7'-tetrahydrospiro[cyclopropane-1,5'-inden]-2'-yl)methyl4-(N-acetoxyacetamido)benzoate |
| 136 | 137 | ((2'S,6'R)-6'-hydroxy-2',4',6'-trimethyl-3',7'-dioxo-2',3',6',7'-tetrahydrospiro[cyclopropane-1,5'-inden]-2'-yl)methyl 4-nitrobenzoate |
| 137 | 138 | ((2'S,6'R)-6'-hydroxy-2',4',6'-trimethyl-3',7'-dioxo-2',3',6',7'-tetrahydrospiro[cyclopropane-1,5'-inden]-2'-yl)methyl4-(N-acetoxyacetamido)benzoate |
| 138 | 139 | (2'S,6'R)-6-hydroxy-2',4',6'-trimethyl-2'-(((4-nitrobenzoyl)oxy)methyl)-7'-oxo-2',3',6',7'-tetrahydrospiro[cyclopropane-1,5'-inden]-3'-yl4-nitrobenzoate |
| 139 | 140 | ((2'S,6'R)-3'-((4-(N-acetoxyacetamido)benzoyl)oxy)-6'-hydroxy-2',4',6'-trimethyl-7'-oxo-2',3',6',7'-tetrahydrospiro[cyclopropane-1,5'-inden]-2'-yl)methyl 4-(N-acetoxyacetamido)benzoate |
| 140 | 141 | dimethyl (5'R)-4',5'-dihydroxy-5',7',9'-trimethyl-4',5'-dihydro-1'H-spiro[cyclopropane-1,6'-[1,3a]ethenoindene]-2',3'-dicarboxylate |
| 141 | 142 | dimethyl (5'R)-5'-hydroxy-5',7',9'-trimethyl-4'-oxo-4',5'-dihydro-1'H-spiro[cyclopropane-1,6'-[1,3a]ethenoindene]-2',3'-dicarboxylate |
| 142 | 143 | (R)-6'-hydroxy-1,2',4',6'-tetramethylspiro[cyclopropane-1,5'-inden]-7'(6'H)-one |
| 143 | 144 | (R)-2-((2'-ethyl-6'-hydroxy-4',6'-dimethyl-7'-oxo-6',7'-dihydrospiro[cyclopropane-1,5'-inden]-3'-yl)methoxy)ethyl acetate |
| 144 | 145 | (R)-5-((6'-hydroxy-2',4',6'-trimethyl-7'-oxo-6',7'-dihydrospiro[cyclopropane-1,5'-inden]-3'-yl)methoxy)-5-oxopentanoic acid |
| 145 | 146 | (R)-4-(6'-hydroxy-2',4',6'-trimethyl-7'-oxo-6',7'-dihydrospiro[cyclopropane-1,5'-inden]-3'-yl)butanenitrile |
| 146 | 147 | (R)-3'-((benzo[d]thiazol-2-ylthio)methyl)-6'-hydroxy-2',4',6'-trimethylspiro[cyclopropane-1,5'-inden]-7'(6'H)-one |
| 147 | 148 | (R)-3'-((benzo[d]oxazol-2-ylthio)methyl)-6'-hydroxy-2',4',6'-trimethylspiro[cyclopropane-1,5'-inden]-7'(6'H)-one |
| 148 | 149 | (R)-6'-hydroxy-2',4',6'-trimethyl-3'-(((1-methyl-1H-tetrazol-5-yl)thio)methyl)spiro[cyclopropane-1,5'-inden]-7'(6'H)-one |
| 149 | 150 | (R)-6'-hydroxy-2',4',6'-trimethyl-3'-(((5-methyl-1H-benzo[d]imidazol-2-yl)thio)methyl)spiro[cyclopropane-1,5'-inden]-7'(6'H)-one |
| 150 | 151 | (R)-6'-hydroxy-2',4',6'-trimethyl-3'-(((1-phenyl-1H-tetrazol-5-yl)thio)methyl)spiro[cyclopropane-1,5'-inden]-7'(6'H)-one |
| 151 | 152 | (R)-6'-hydroxy-2',4',6'-trimethyl-3'-(((5-nitro-1H-benzo[d]imidazol-2-yl)thio)methyl)spiro[cyclopropane-,5'-inden]-7'(6'H)-one |
| 152 | 153 | (R)-3'-(((1H-1,2,4-triazol-3-yl)thio)methyl)-6'-hydroxy-2',4',6'-trimethylspiro[cyclopropane-1,5'-inden]-7'(6'H)-one |
| 153 | 154 | (R)-6'-hydroxy-3'-(((4-hydroxypteridin-2-yl)thio)methyl)-2',4',6'-trimethylspiro[cyclopropane-1,5'-inden]-7'(6'H)-one |
| 154 | 155 | (R)-6'-hydroxy-3'-(((1-(4-hydroxyphenyl)-1H-tetrazol-5-yl)thio)methyl)-2',4',6'-trimethylspiro[cyclopropane-1,5'-inden]-7'(6'H)-one |
| 155 | 156 | (R)-4-(5-(((6'-hydroxy-2',4',6'-trimethyl-7'-oxo-6',7'-dihydrospiro[cyclopropane-1,5'-inden]-3'-yl)methyl)thio)-1H-tetrazol-1-yl)phenyl acetate |
| 156 | 157 | 7'-methyl-4'H-dispiro[cyclobutane-1,6'-indene-5',2"-[1,3]dioxolan]-4'-one |
| 157 | 158 | 5-hydroxy-2,2,6,8a-tetramethyl-2,3,3a,8,8a,8b-hexahydro-1H-cyclobuta[d]cyclopenta[b]oxepin-7(5H)-one |
| 158 | 159 | ((6'R)-3',6'-dihydroxy-2',4',6'-trimethyl-7'-oxo-2',3',6',7'-tetrahydrospiro[cyclopropane-1,5'-inden]-2'-yl)methylacetate |
| 159 | 160 | 5-(((6'R)-2'-(acetoxymethyl)-6'-hydroxy-2',4',6'-trimethyl-7'-oxo-2',3',6',7'-tetrahydrospiro[cyclopropane-1,5'-inden]-3'-yl)oxy)-5-oxopentanoic acid |
| 160 | 161 | ((6'R)-6'-hydroxy-2',4',6'-trimethyl-3',7'-dioxo-2',3',6',7'-tetrahydrospiro[cyclopropane-1,5'-inden]-2'-yl)methyl acetate |
| 161 | 162 | 5-(((6'R)-6'-hydroxy-2',4',6'-trimethyl-3',7'-dioxo-2',3',6',7'-tetrahydrospiro[cyclopropane-1,5'-inden]-2'-yl)methoxy)-5-oxopentanoic acid |
| 162 | 163 | (6'R)-2'-(acetoxymethyl)-6'-hydroxy-2',4',6'-trimethyl-7'-oxo-2',3',6',7'-tetrahydrospiro[cyclopropane-1,5'-inden]-3'-yl 2-chloroacetate |
| 163 | 164 | (R)-(6'-hydroxy-2',4',6'-trimethyl-7'-oxo-6',7'-dihydrospiro[cyclopropane-1,5'-inden]-3'-yl)methyl 2-chloroacetate |
| 164 | 165 | (R)-(6'-hydroxy-2',4',6'-trimethyl-7'-oxo-6',7'-dihydrospiro[cyclopropane-1,5'-inden]-3'-yl)methyl 2-morpholinoacetate |
| 165 | 166 | (6'R)-2'-(acetoxymethyl)-6'-hydroxy-2',4',6'-trimethyl-7'-oxo-2',3',6',7'-tetrahydrospiro[cyclopropane-1,5'-inden]-3'-yl methyl glutarate |
| 166 | 167 | (6'R)-6'-hydroxy-2'-(hydroxymethyl)-2',4',6'-trimethyl-7'-oxo-2',3',6',7'-tetrahydrospiro[cyclopropane-1,5'-inden]-3'-yl methyl glutarate |
| 167 | 168 | ((6'R)-6'-hydroxy-2',4',6'-trimethyl-3',7'-dioxo-2',3',6',7'-tetrahydrospiro[cyclopropane-1,5'-inden]-2'-yl)methyl methyl glutarate |
| 168 | 169 | ((6'R)-6'-hydroxy-2',4',6'-trimethyl-3',7'-dioxo-2',3',6',7'-tetrahydrospiro[cyclopropane-1,5'-inden]-2'-yl)methyl 2-chloroacetate |
| 169 | 170 | 5-(2-hydroxyethyl)-3-(hydroxymethyl)-2,4,6-trimethyl-1H-inden-7-ol |
| 170 | 171 | 6-(2-hydroxyethyl)-2,5,7-trimethyl-1-methylene-1H-inden-4-ol |
| 171 | 172 | 6-ethyl-2,5,7-trimethyl-1-methylene-1H-inden-4-ol |
| 172 | 173 | 2-(4-hydroxy-2,5,7-trimethyl-1-methylene-1H-inden-6-yl)ethyl acetate |
| 173 | 174 | 5-(2-hydroxyethyl)-3-(hydroxymethyl)-2,4,6-trimethyl-1H-indene-1,7-diol |
| 174 | 175 | (2S,3S,4R,5S,6R)-2-(acetoxymethyl)-6-(((R)-6'-hydroxy-2',4',6'-trimethyl-7'-oxo-6',7'-dihydrospiro[cyclopropane-1,5'-inden]-3'-yl)methoxy)tetrahydro-2H-pyran-3,4,5-triyl triacetate |
| 175 | 176 | 3-((R)-6'-hydroxy-2',4',6'-trimethyl-7'-oxo-6',7'-dihydrospiro[cyclopropane-1,5'-inden]-3'-yl)propyl leucinate |
| 176 | 177 | (R)-5-(3-(6'-hydroxy-2',4',6'-trimethyl-7'-oxo-6',7'-dihydrospiro[cyclopropane-1,5'-inden]-3'-yl)propoxy)-5-oxopentanoic acid |
| 177 | 178 | (R)-4-(3-(6'-hydroxy-2',4',6'-trimethyl-7'-oxo-6',7'-dihydrospiro[cyclopropane-1,5'-inden]-3'-yl)propoxy)-4-oxobutanoic acid |
| 178 | 179 | (R)-3-(6'-hydroxy-2',4',6'-trimethyl-7'-oxo-6',7'-dihydrospiro[cyclopropane-1,5'-inden]-3'-yl)propyl glycinate |
| 179 | 180 | (1a'R,3'R,7'S,7a'R)-3',7'-dihydroxy-1a',3',6',6'-tetramethyl-6',7'-dihydro-1a'H-spiro[cyclopropane-1,2'-indeno[3a,4-b]oxiren]-4'(3'H)-one |
| 180 | 181 | ((1a'R,3'R,6'S,7'S,7a'R)-3',7'-dihydroxy-1a',3',6'-trimethyl-4'-oxo-3',4',6',7'-tetrahydro-1a'H-spiro[cyclopropane-1,2'-indeno[3a,4-b]oxiren]-6'-yl)methylacetate |
| 181 | 182 | (2'R,7'S,7a'S)-2'-chloro-7'-hydroxy-2',4'-dimethyl-1',2',7',7a'-tetrahydrospiro[cyclopropane-1,5'-indene]-3',6'-dione |
| 182 | 183 | (2'S,7'S,7a'S)-7'-hydroxy-2'-sopropoxy-2',4'-dimethyl-1',2',7',7a'-tetrahydrospiro[cyclopropane-1,5'-indene]-3',6'-dione |
| 183 | 184 | (R)-1-hydroxy-1-((6'-hydroxy-2',4',6'-trimethyl-7'-oxo-6',7'-dihydrospiro[cyclopropane-1,5'-inden]-3'-yl)methyl)urea |
| 184 | 185 | (S)-6'-hydroxy-2',4',6'-trimethylspiro[cyclopropane-1,5'-inden]-7'(6'H)-one |
| 185 | 186 | (6'S,7'R)-6',7'-dihydroxy-2',4',6'-trimethyl-7',7a'-dihydrospiro[cyclopropane-1,5'-inden]-3'(6'H)-one |
| 186 | 187 | (S)-6'-hydroxy-3'-(hydroxymethyl)-2',4',6'-trimethylspiro[cyclopropane-1,5'-inden]-7'(6'H)-one |
| 187 | 188 | (6'S,6‴S)-3',3‴-methylenebis(6'-hydroxy-2',4'-6'-trimethylspiro[cyclopropane-1,5'-inden]-7'(6'H)-one) |
| 188 | 189 | (R)-1-((6'-hydroxy-2',4',6'-trimethyl-7'-oxo-6',7'-dihydrospiro[cyclopropane-1,5'-inden]-3'-yl)methyl)-1H-pyrrole-2,5-dione |
| 189 | 190 | (R)-1-(3-(6'-hydroxy-2',4',6'-trimethyl-7'-oxo-6',7'-dihydrospiro[cyclopropane-1,5'-inden]-3'-yl)propyl)-1H-pyrrole-2,5-dione |
| 190 | 191 | 6'-hydroxy-4',6'-dimethylspiro[cyclobutane-1,5'-inden]-7'(6'H)-one |
| 191 | 192 | (R)-2-((6'-hydroxy-2',4',6'-trimethyl-7'-oxo-6',7'-dihydrospiro[cyclopropane-1,5'-inden]-3'-yl)methyl)isoindoline-1,3-dione |
| 192 | 193 | (R)-3'-(azidomethyl)-6'-hydroxy-2',4',6'-trimethylspiro[cyclopropane-1,5'-inden]-7'(6'H)-one |
| 193 | 194 | (R)-3'-(((R)-3'-(azidomethyl)-6'-hydroxy-2',4',6'-trimethyl-7'-oxo-6',7'-dihydrospiro[cyclopropane-1,5'-inden]-1'-yl)methyl)-6'-hydroxy-2',4',6'-trimethylspiro[cyclopropane-1,5'-inden]-7'(6'H)-one |
| 194 | 195 | (R)-3'-(3-azidopropyl)-6'-hydroxy-4',6'-dimethylspiro[cyclopropane-1,5'-inden]-7'(6'H)-one |
| 195 | 196 | 3-((R)-6'-hydroxy-2',4',6'-trimethyl-7'-oxo-6',7'-dihydrospiro[cyclopropane-1,5'-inden]-3'-yl)propyl L-prolinate |
| 196 | 197 | (R)-2-(3-(6'-hydroxy-2',4',6'-trimethyl-7'-oxo-6',7'-dihydrospiro[cyclopropane-1,5'-inden]-3'-yl)propyl)isoindoline-1,3-dione |
| 197 | 198 | (R)-6'-hydroxy-2',4',6'-trimethyl-3'-((4-nitrophenoxy)methyl)spiro[cyclopropane-1,5'-inden]-7'(6'H)-one |
| 198 | 199 | (R)-6'-hydroxy-2',4',6'-trimethyl-3'-(phenoxymethyl)spiro[cyclopropane-1,5'-inden]-7'(6'H)-one |
| 199 | 200 | (R)-6'-hydroxy-3'-(2-hydroxybenzyl)-2',4',6'-trimethylspiro[cyclopropane-1,5'-inden]-7'(6'H)-one |
| 200 | 201 | (R)-N-(3-(6'-hydroxy-2',4',6'-trimethyl-7'-oxo-6',7'-dihydrospiro[cyclopropane-1,5'-inden]-3'-yl)propyl)acetamide |
| 201 | 202 | (S)-N-(3-((R)-6'-hydroxy-2',4',6'-trimethyl-7'-oxo-6',7'-dihydrospiro[cyclopropane-1,5'-inden]-3'-yl)propyl)pyrrolidine-2-carboxamide |
| 202 | 203 | 3-((R)-6'-hydroxy-2',4',6'-trimethyl-7'-oxo-6',7'-dihydrospiro[cyclopropane-1,5'-inden]-3'-yl)propyl L-seryl-L-prolinate |
| 203 | 204 | 2'-(((tert-butyldimethylsilyl)oxy)methyl)-3',6'-dihydroxy-2',4',6'-trimethyl-2',3'-dihydrospiro[cyclopropane-1,5'-inden]-7'(6'H)-one |
| 204 | 205 | (2'R,3'S,6'R)-3'-amino-2'-(((tert-butyldimethylsilyl)oxy)methyl)-6'-hydroxy-2',4',6'-trimethyl-2',3'-dihydrospiro[cyclopropane-1,5'-inden]-7'(6'H)-one |
| 205 | 206 | (2'R,3'S,6'R)-3'-amino-6'-hydroxy-2'-(hydroxymethyl)-2',4',6'-trimethyl-2',3'-dihydrospiro[cyclopropane-1,5'-inden]-7'(6'H)-one |
| 206 | 207 | (S)-2-amino-N-(3-((R)-6'-hydroxy-2',4',6'-trimethyl-7'-oxo-6',7'-dihydrospiro[cy clopropane-1,5 '-inden] -3 '-yl)propyl)-4-methylpentanamide |
| 207 | 208 | 3-((R)-6'-hydroxy-2',4',6'-trimethyl-7'-oxo-6',7'-dihydrospiro[cyclopropane-1,5'-inden]-3'-yl)propyl (tert-butoxycarbonyl)-L-seryl-L-prolinate |
| 208 | 209 | 3-((R)-6'-hydroxy-2',4',6'-trimethyl-7'-oxo-6',7'-dihydrospiro[cyclopropane-1,5'-inden]-3'-yl)propyl L-seryl-L-seryl-L-prolinate |
| 209 | 210 | 3-((R)-6'-hydroxy-2',4',6'-trimethyl-7'-oxo-6',7'-dihydrospiro[cyclopropane-1,5'-inden]-3'-yl)propyl ((S)-2-((S)-2-((S)-2-((2S,4R)-1-acetyl-4-hydroxypyrrolidine-2-carboxamido)-3-hydroxypropanamido)-3-hydroxypropanamido)-2-cyclohexylacetyl)-L-glutaminyl-L-seryl-L-seryl-L-prolinate |
| 210 | 211 | (R)-3'-(3-aminopropyl)-6'-hydroxy-2',4',6'-trimethylspiro[cyclopropane-1,5'-inden]-7'(6'H)-one |
| 211 | 212 | (3'S,6'R)-6'-hydroxy-2',2',4',6'-tetramethyl-7'-oxo-2',3',6',7'-tetrahydrospiro[cyclopropane-1,5'-inden]-3'-yl L-prolinate |
| 212 | 213 | (2'S,3'R,6'R)-2'-(((tert-butyldimethylsilyl)oxy)methyl)-6'-hydroxy-2',4',6'-trimethyl-7'-oxo-2',3',6',7'-tetrahydrospiro[cyclopropane-1,5'-inden]-3'-yl L-prolinate |
| 213 | 214 | (2'S,3'R,6'R)-2'-(((tert-butyldimethylsilyl)oxy)methyl)-6'-hydroxy-2',4',6'-trimethyl-7'-oxo-2',3',6',7'-tetrahydrospiro[cyclopropane-1,5'-inden]-3'-yl L-seryl-L-seryl-L-prolinate |
| 214 | 215 | (2'S,3'R,6'R)-2'-(((tert-butyldimethylsilyl)oxy)methyl)-6'-hydroxy-2',4',6'-trimethyl-7'-oxo-2',3',6',7'-tetrahydrospiro[cyclopropane-1,5'-inden]-3'-yl((S)-2-((S)-2-((S)-2-((2S,4R)-1-acetyl-4-hydroxypyrrolidine-2-carboxamido)-3-hydroxypropanamido)-3-hydroxypropanamido)-2-cyclohexylacetyl)-L-glutaminyl-L-seryl-L-seryl-L-prolinate |
| 215 | 216 | (3'S,6'R)-6'-hydroxy-2',2',4',6'-tetramethyl-7'-oxo-2',3',6',7'-tetrahydrospiro[cyclopropane-1,5'-inden]-3'-yl((S)-2-((S)-2-((S)-2-((2S,4R)-1-acetyl-4-hydroxypyrrolidine-2-carboxamido)-3-hydroxypropanamido)-3-hydroxypropanamido)-2-cyclohexylacetyl)-L-glutaminyl-L-seryl-L-seryl-L-prolinate |
| 216 | 217 | (R)-(6'-hydroxy-2',4',6'-trimethyl-7'-oxo-6',7'-dihydrospiro[cyclopropane-1,5'-inden]-3'-yl)methyl 6-(2,5-dioxo-2,5-dihydro-1H-pyrrol-1-yl)hexanoate |
| 217 | 218 | (3'S,6'R)-6'-hydroxy-2',2',4',6'-tetramethyl-7'-oxo-2',3',6',7'-tetrahydrospiro[cyclopropane-1,5'-inden]-3'-yl 6-(2,5-dioxo-2,5-dihydro-1H-pyrrol-1-yl)hexanoate |
| 218 | 219 | (2'S,3'R,6'R)-2'-(((tert-butyldimethylsilyl)oxy)methyl)-6'-hydroxy-2',4',6'-trimethyl-7'-oxo-2',3',6',7'-tetrahydrospiro[cyclopropane-1,5'-inden]-3'-yl 4-(fluorosulfonyl)benzoate |
| 219 | 220 | (R)-1-acetoxy-1-((6'-hydroxy-2',4',6'-trimethyl-7'-oxo-6',7'-dihydrospiro[cyclopropane-1,5'-inden]-3'-yl)methyl)urea |
| 220 | 221 | (S)-2-((3S,6S,9S,12S,15S)-3-((1H-imidazol-4-yl)methyl)-12-(4-aminobutyl)-6,9-bis(hydroxymethyl)-15-isobutyl-1-morpholino-1,4,7,10,13-pentaoxo-2,5,8,11,14-pentaazahexadecan-16-amido)-N1-((S)-1-((3-((R)-6'-hydroxy-2',4',6'-trimethyl-7'-oxo-6',7'-dihydrospiro[cyclopropane-1,5'-inden]-3'-yl)propyl)amino)-4-methyl-1-oxopentan-2-yl)pentanediamide |
| 221 | 222 | (3'S,6'R)-6'-hydroxy-2',2',4',6'-tetramethyl-7'-oxo-2',3',6',7'-tetrahydrospiro[cyclopropane-1,5'-inden]-3'-yl 4-(fluorosulfonyl)benzoate |
| 222 | 223 | 3-((R)-6'-hydroxy-2',4',6'-trimethyl-7'-oxo-6',7'-dihydrospiro[cyclopropane-1,5'-inden]-3'-yl)propyl ((4R,7S,13S)-13-(2-amino-2-oxoethyl)-7-(3-guanidinopropyl)-6,9,12,15-tetraoxo-1,2-dithia-5,8,14-triazacycloheptadecane-4-carbonyl)glycinate |
| 223 | 224 | (R,E)-6'-hydroxy-2',4',6'-trimethylspiro[cyclopropane-1,5 '-inden]-7'(6'H)-one oxime |
| 224 | 225 | (2'R,3'R,6'R,E)-2',3',6'-trihydroxy-2',4',6'-trimethyl-2',3'-dihydrospiro[cyclopropane-1,5'-inden]-7'(6'H)-one oxime |
| 225 | 226 | (R)-(6'-hydroxy-2',4',6'-trimethyl-7'-oxo-6',7'-dihydrospiro[cyclopropane-1,5'-inden]-3'-yl)methyl (E)-octadec-9-enoate |
| 226 | 227 | (R)-3-(6'-hydroxy-2',4',6'-trimethyl-7'-oxo-6',7'-dihydrospiro[cyclopropane-1,5'-inden]-3'-yl)propyl (E)-octadec-9-enoate |
| 227 | 228 | (2'S,6'R)-6'-hydroxy-2',4',6'-trimethyl-2'-((((E)-octadec-9-enoyl)oxy)methyl)-7'-oxo-2',3',6',7'-tetrahydrospiro[cyclopropane-1,5'-inden]-3'-yl (E)-octadec-9-enoate |
| 228 | 229 | (R,E)-N-(3-(6'-hydroxy-2',4',6'-trimethyl-7'-oxo-6',7'-dihydrospiro[cyclopropane-1,5'-inden]-3'-yl)propyl)octadec-9-enamide |
| 229 | 230 | N-((3'R,6'R)-6'-hydroxy-2',2',4',6'-tetramethyl-7'-oxo-2',3',6',7'-tetrahydrospiro[cyclopropane-1,5'-inden]-3'-yl)methanesulfonamide |
| 230 | 231 | N-((3'R,6'R)-6'-hydroxy-2',2',4',6'-tetramethyl-7'-oxo-2',3',6',7'-tetrahydrospiro[cyclopropane-1,5 '-inden]-3 '-yl)-4-methylbenzenesulfonamide |
| 231 | 232 | (R)-3-(6'-hydroxy-2',4',6'-trimethyl-7'-oxo-6',7'-dihydrospiro[cyclopropane-1,5'-inden]-3'-yl)propyl hydroxycarbamate |
| 232 | 233 | ethyl (R)-hydroxy((6'-hydroxy-2',4',6'-trimethyl-7'-oxo-6',7'-dihydrospiro[cyclopropane-1,5'-inden]-3'-yl)methyl)carbamate |
| 233 | 234 | benzyl (R)-hydroxy((6'-hydroxy-2',4',6'-trimethyl-7'-oxo-6',7'-dihydrospiro[cyclopropane-1,5'-inden]-3'-yl)methyl)carbamate |
| 234 | 235 | tert-butyl (R)-hydroxy((6'-hydroxy-2',4',6'-trimethyl-7'-oxo-6',7'-dihydrospiro[cyclopropane-1,5'-inden]-3'-yl)methyl)carbamate |
| 235 | 236 | (R)-6'-hydroxy-3'-(hydroxymethyl)-1',2',4',6'-tetramethylspiro[cyclopropane-1,5'-inden]-7'(6'H)-one |
| 236 | 237 | (R)-3-(6'-hydroxy-2',4',6'-trimethyl-7'-oxo-6',7'-dihydrospiro[cyclopropane-1,5'-inden]-3'-yl)propyl (2-bromoethyl)carbamate |
| 237 | 238 | (R)-3-(6'-hydroxy-2',4',6'-trimethyl-7'-oxo-6',7'-dihydrospiro[cyclopropane-1,5'-inden]-3'-yl)propyl (2-chloroethyl)carbamate |
| 238 | 239 | (R)-3-(6'-hydroxy-2',4',6'-trimethyl-7'-oxo-6',7'-dihydrospiro[cyclopropane-1,5'-inden]-3'-yl)propyl (2-hydroxyethyl)carbamate |
| 239 | 240 | (R)-3-(6'-hydroxy-2',4',6'-trimethyl-7'-oxo-6',7'-dihydrospiro[cyclopropane-1,5'-inden]-3'-yl)propyl acetoxy(acetyl)carbamate |
| 240 | 241 | (R)-N-(3-(6'-hydroxy-2',4',6'-trimethyl-7'-oxo-6',7'-dihydrospiro[cyclopropane-1,5'-inden]-3'-yl)propyl)methanesulfonamide |
| 241 | 242 | (R)-N-(3-(6'-hydroxy-2',4',6'-trimethyl-7'-oxo-6',7'-dihydrospiro[cyclopropane-1,5'-inden]-3'-yl)propyl)-4-methylbenzenesulfonamide |
| 242 | 243 | (R)-3-(6'-hydroxy-2',4',6'-trimethyl-7'-oxo-6',7'-dihydrospiro[cyclopropane-1,5'-inden]-3'-yl)propyl (2-fluoroethyl)carbamate |
| 243 | 244 | (R)-1-hydroxy-1-(3-(6'-hydroxy-2',4',6'-trimethyl-7'-oxo-6',7'-dihydrospiro[cyclopropane-1,5'-inden]-3'-yl)propyl)urea |
| 244 | 245 | (R)-1-hydroxy-1-(3-(6'-hydroxy-2',4'-6'-trimethyl-7'-oxo-6',7'-dihydrospiro[cyclopropane-1,5'-inden]-3'-yl)propyl)thiourea |
| 245 | 246 | (R)-3-(6'-hydroxy-2',4',6'-trimethyl-7'-oxo-6',7'-dihydrospiro[cyclopropane-1,5'-inden]-3'-yl)propyl morpholine-4-carboxylate |
| 246 | 247 | (R)-3-(6'-hydroxy-2',4',6'-trimethyl-7'-oxo-6',7'-dihydrospiro[cyclopropane-1,5'-inden]-3'-yl)propyl morpholine-4-carboxylate |
| 247 | 248 | (R)-3-(6'-hydroxy-2',4',6'-trimethyl-7'-oxo-6',7'-dihydrospiro[cyclopropane-1,5'-inden]-3'-yl)propyl [1,4'-bipiperidine]-1'-carboxylate |
| 248 | 249 | (R)-6'-hydroxy-2'-(hydroxymethyl)-3',4',6'-trimethylspiro[cyclopropane-1,5'-inden]-7'(6'H)-one |
| 249 | 250 | ((1a'R,2'S,3'R,6'R,7a'S)-3'-acetoxy-6'-hydroxy-2',4',6'-trimethyl-7'-oxo-1',1a',2',3',6',7'-hexahydrospiro[cyclopropane-1,5'-cyclopropa[c]inden]-2'-yl)methyl acetate |
| 250 | 251 | ((1a'S,2'S,3'R,6'R,7a'R)-3'-acetoxy-6'-hydroxy-2',4',6'-trimethyl-7'-oxo-1',1a',2',3',6',7'-hexahydrospiro[cyclopropane-1,5'-cyclopropa[c]inden]-2'-yl)methyl acetate |
| 251 | 252 | (1a'R,3'S,6'R,7a'S)-3',6'-dihydroxy-2',2',4',6'-tetramethyl-1',1a',2',3'-tetrahydrospiro[cyclopropane-1,5'-cyclopropa[c]inden]-7'(6'H)-one |
| 252 | 253 | (1a'S,3'S,6'R,7a'R)-3',6'-dihydroxy-2',2',4',6'-tetramethyl-1',1a',2',3'-tetrahydrospiro[cyclopropane-1,5'-cyclopropa[c]inden]-7'(6'H)-one |
| 253 | 254 | (R)-3-(6'-hydroxy-2',4',6'-trimethyl-7'-oxo-6',7'-dihydrospiro[cyclopropane-1,5'-inden]-3'-yl)propyl 4-sulfamoylbenzoate |
| 254 | 255 | (R)-3-(6'-hydroxy-2',4',6'-trimethyl-7'-oxo-6',7'-dihydrospiro[cyclopropane-1,5'-inden]-3'-yl)propyl sulfamate |
| 255 | 256 | 3-((R)-6'-hydroxy-2',4',6'-trimethyl-7'-oxo-6',7'-dihydrospiro[cyclopropane-1,5'-inden]-3'-yl)propyl 1-(3-((R)-6'-hydroxy-2',4',6'-trimethyl-7'-oxo-6',7'-dihydrospiro[cyclopropane-1,5'-inden]-3'-yl)propyl)-1H-1,2,3-triazole-4-carboxylate |
| 256 | 257 | 3-((R)-6'-hydroxy-2',4',6'-trimethyl-7'-oxo-6',7'-dihydrospiro[cyclopropane-1,5'-inden]-3'-yl)propyl 1-(((R)-6'-hydroxy-2',4',6'-trimethyl-7'-oxo-6',7'-dihydrospiro[cyclopropane-1,5'-inden]-3'-yl)methyl)-1H-1,2,3-triazole-4-carboxylate |
| 257 | 258 | (4-carboxy-4-(4-carboxy-4-((3-((R)-6'-hydroxy-2',4',6'-trimethyl-7'-oxo-6',7'-dihydrospiro[cyclopropane-1,5'-inden]-3'-yl)propyl)amino)butanamido)butanoyl)glutamic acid |
| 258 | 259 | (R)-3'-((S)-2,2-dioxido-1,2,3-oxathiazinan-4-yl)-6'-hydroxy-2',4',6'-trimethylspiro[cyclopropane-1,5'-inden]-7'(6'H)-one |
| 259 | 260 | (R)-N-(3-(6'-hydroxy-2',4',6'-trimethyl-7'-oxo-6',7'-dihydrospiro[cyclopropane-1,5'-inden]-3-yl)propyl)-[1,4'-bipiperidine]-1'-carboxamide |
| 260 | 261 | (R)-3-(6'-hydroxy-2',4',6'-trimethyl-7'-oxo-6',7'-dihydrospiro[cyclopropane-1,5'-inden]-3'-yl)propyl 1H-imidazole-1-carboxylate |
| 261 | 262 | methyl (R)-2-(((6'-hydroxy-2',4',6'-trimethyl-7'-oxo-6',7'-dihydrospiro[cyclopropane-1,5'-inden]-3'-yl)methyl)sulfonyl)acetate |
| 262 | 263 | methyl 2-((((R)-6'-hydroxy-2',4',6'-trimethyl-7'-oxo-6',7'-dihydrospiro[cyclopropane-1,5'-inden]-3'-yl)methyl)sulfinyl)acetate |
| 263 | 264 | N-[3-(6'-hydroxy-2',6'-dimethyl-7'-oxo-6',7'-dihydrospiro[cyclopropane-1,5'-inden]-3'-yl)propyl]sulfuric diamide |
| 264 | 265 | N-hydroxy-N'-[3-(6'-hydroxy-2',6'-dimethyl-7'-oxo-6',7'-dihydrospiro[cyclopropane-1,5'-inden]-3'-yl)propyl]sulfuric diamide |
| 265 | 266 | N-[3-(6'-hydroxy-2',6'-dimethyl-7'-oxo-6',7'-dihydrospiro[cyclopropane-1,5'-inden]-3'-yl)propyl]-N-methoxysulfuric diamide |
| 266 | 267 | (R)-2-amino-N-((6'-hydroxy-2',4',6'-trimethyl-7'-oxo-6',7'-dihydrospiro[cyclopropane-1,5'-inden]-3'-yl)methyl)-N-methoxyacetamide |
| 267 | 268 | (R)-2,2,2-trifluoro-N-(3-(6'-hydroxy-2',4',6'-trimethyl-7'-oxo-6',7'-dihydrospiro[cyclopropane-1,5'-inden]-3'-yl)propyl)acetamide |
| 268 | 269 | (R)-(6'-hydroxy-2',4',6'-trimethyl-7'-oxo-6',7'-dihydrospiro[cyclopropane-1,5'-inden]-3'-yl)methyl (4-methoxyphenyl)sulfamate |
| 269 | 270 | (R)-3'-(aminomethyl)-6'-hydroxy-2',4',6'-trimethylspiro[cyclopropane-1,5'-inden]-7'(6'H)-one |
| 270 | 271 | (R)-N-((6'-hydroxy-2',4',6'-trimethyl-7'-oxo-6',7'-dihydrospiro[cyclopropane-1,5'-inden]-3'-yl)methyl)methanesulfonami de |
| 271 | 272 | (5S,6S,7S)-3-(((3-((R)-6'-hydroxy-2',4'-6'-trimethyl-7'-oxo-6',7'-dihydrospiro[cyclopropane-1,5'-inden]-3'-yl)propanoyl)oxy)methyl)-8-oxo-7-(2-(thiophen-2-yl)acetamido)-5-thia-1-azabicyclo[4.2.0]oct-2-ene-2-carboxylic acid 5-oxide |
| 272 | 273 | (5S,6S,7S)-3-((((3-((R)-6'-hydroxy-2',4',6'-trimethyl-7'-oxo-6',7'-dihydrospiro[cyclopropane-1,5'-inden]-3'-yl)propyl)carbamoyl)oxy)methyl)-8-oxo-7-(2-(thiophen-2-yl)acetamido)-5-thia-1-azabicyclo[4.2.0]oct-2-ene-2-carboxylic acid 5-oxide |
| 273 | 274 | (6S,7S)-3-((((3-((R)-6'-hydroxy-2',4',6'-trimethyl-7'-oxo-6',7'-dihydrospiro[cyclopropane-1,5'-inden]-3'-yl)propyl)carbamoyl)oxy)methyl)-8-oxo-7-(2-(thiophen-2-yl)acetamido)-5-thia-1-azabicyclo[4.2.0]oct-2-ene-2-carboxylic acid 5,5-dioxide |
| 274 | 275 | N-(((R)-6'-hydroxy-2',4',6'-trimethyl-7'-oxo-6',7'-dihydrospiro[cyclopropane-1,5'-inden]-3'-yl)methyl)pyrrolidine-2-carboxamide |
| 275 | 276 | 2-amino-N-(((R)-6'-hydroxy-2',4',6'-trimethyl-7'-oxo-6',7'-dihydrospiro[cyclopropane-1,5'-inden]-3'-yl)methyl)-4-methylpentanamide |
| 276 | 277 | (R)-1-hydroxy-3-((6'-hydroxy-2',4',6'-trimethyl-7'-oxo-6',7'-dihydrospiro[cyclopropane-1,5'-inden]-3'-yl)methyl)urea |
| 277 | 278 | (R)-1-((6'-hydroxy-2',4',6'-trimethyl-7'-oxo-6',7'-dihydrospiro[cyclopropane-1,5'-inden]-3'-yl)methyl)-3-methoxyurea |
| 278 | 279 | (R)-1-((6'-hydroxy-2',4',6'-trimethyl-7'-oxo-6',7'-dihydrospiro[cyclopropane-1,5'-inden]-3'-yl)methyl)-3-(2-hydroxyethyl)urea |
| 279 | 280 | (R)-1-(3-(6'-hydroxy-2',4',6'-trimethyl-7'-oxo-6',7'-dihydrospiro[cyclopropane-1,5'-inden]-3'-yl)propyl)-3-(2-hydroxyethyl)urea |
| 280 | 281 | (R)-1-(2-chloroethyl)-3-(3-(6'-hydroxy-2',4',6'-trimethyl-7'-oxo-6',7'-dihydrospiro[cyclopropane-1,5'-inden]-3'-yl)propyl)urea |
| 281 | 282 | (R)-1-(2-chloroethyl)-3-((6'-hydroxy-2',4',6'-trimethyl-7'-oxo-6',7'-dihydrospiro[cyclopropane-1,5'-inden]-3'-yl)methyl)urea |
| 282 | 283 | N-[(6'-hydroxy-2',6'-dimethyl-7'-oxo-6',7'-dihydrospiro[cyclopropane-1,5'-inden]-3'-yl)methyl]sulfuric diamide |
| 283 | 284 | (R)-3-((6'-hydroxy-2',4',6'-trimethyl-7'-oxo-6',7'-dihydrospiro[cyclopropane-1,5'-inden]-3'-yl)methyl)pyrimidine-2,4(1H,3H)-dione |
| 284 | 285 | N-hydroxy-N-[(6'-hydroxy-2',6'-dimethyl-7'-oxo-6',7'-dihydrospiro[cyclopropane-1,5'-inden]-3'-yl)methyl]sulfuric diamide |
| 285 | 286 | (R)-5-fluoro-1-((6'-hydroxy-2',4',6'-trimethyl-7'-oxo-6',7'-dihydrospiro[cyclopropane-1,5'-inden]-3'-yl)methyl)pyrimidine-2,4(1H,3H)-dione |
| 286 | 287 | (R)-5-fluoro-3-((6'-hydroxy-2',4',6'-trimethyl-7'-oxo-6',7'-dihydrospiro[cyclopropane-1,5'-inden]-3'-yl)methyl)pyrimidine-2,4(1H,3H)-dione |
| 287 | 288 | (R)-1-hydroxy-3-(3-(6'-hydroxy-2',4',6'-trimethyl-7'-oxo-6',7'-dihydrospiro[cyclopropane-1,5'-inden]-3'-yl)propyl)urea |
| 288 | 289 | (R)-1-((6'-hydroxy-2',4',6'-trimethyl-7'-oxo-6',7'-dihydrospiro[cyclopropane-1,5'-inden]-3'-yl)methyl)pyrimidine-2,4(1H,3H)-dione |
| 289 | 290 | ((S)-6'-hydroxy-2',4',6'-trimethyl-7'-oxo-6',7'-dihydrospiro[cyclopropane-1,5'-inden]-3'-yl)methyl 5-oxo-5-(((S)-1,2,3,10-tetramethoxy-9-oxo-5,6,7,9-tetrahydrobenzo[a]heptalen-7-yl)amino)pentanoate |
| 290 | 291 | 3-((S)-6'-hydroxy-2',4',6'-trimethyl-7'-oxo-6',7'-dihydrospiro[cyclopropane-1,5'-inden]-3'-yl)propyl 5-oxo-5-(((S)-1,2,3,10-tetramethoxy-9-oxo-5,6,7,9-tetrahydrobenzo[a]heptalen-7-yl)amino)pentanoate |
| 291 | 292 | N1-(((S)-6'-hydroxy-2',4',6'-trimethyl-7'-oxo-6',7'-dihydrospiro[cyclopropane-1,5'-inden]-3'-yl)methyl)-N5-((S)-1,2,3,10-tetramethoxy-9-oxo-5,6,7,9-tetrahydrobenzo[a]heptalen-7-yl)glutaramide |
| 292 | 293 | 3-((S)-6'-hydroxy-2',4',6'-trimethyl-7'-oxo-6',7'-dihydrospiro[cyclopropane-1,5'-inden]-3 -yl)-N-((S)-1,2,3,10-tetramethoxy-9-oxo-5,6,7,9-tetrahydrobenzo[a]heptalen-7-yl)propanamide |
| 293 | 294 | 2-amino-N-(((R)-6'-hydroxy-2',4',6'-trimethyl-7'-oxo-6',7'-dihydrospiro[cyclopropane-1,5'-inden]-3'-yl)methyl)-N-methoxypropanamide |
| 294 | 295 | 2-amino-N-(((R)-6'-hydroxy-2',4',6'-trimethyl-7'-oxo-6',7'-dihydrospiro[cyclopropane-1,5'-inden]-3'-yl)methyl)-N-methoxy-4-methylpentanamide |
| 295 | 296 | 2-amino-N-(((R)-6'-hydroxy-2',4',6'-trimethyl-7'-oxo-6',7'-dihydrospiro[cyclopropane-1,5'-inden]-3'-yl)methyl)-N-methoxy-4-(methylthio)butanamide |
| 296 | 297 | 2-amino-N-(((R)-6'-hydroxy-2',4',6'-trimethyl-7'-oxo-6',7'-dihydrospiro[cyclopropane-1,5'-inden]-3'-yl)methyl)-3-(1H-indol-3-yl)-N-methoxypropanamide |
| 297 | 298 | (R)-3-(6'-hydroxy-2',4',6'-trimethyl-7'-oxo-6',7'-dihydrospiro[cyclopropane-1,5'-inden]-3'-yl)propyl (tert-butoxycarbonyl)glycinate |
| 298 | 299 | (2'S,3'R,6'R)-2'-(azidomethyl)-3',6'-dihydroxy-2',4'6'-trimethyl-2',3'-dihydrospiro[cyclopropane-1,5'-inden]-7'(6'H)-one |
| 299 | 300 | (2'R,3'R,6'R)-3'-azido-6'-hydroxy-2'-(hydroxymethyl)-2',4',6'-trimethyl-2',3'-dihydrospiro[cyclopropane-1,5'-inden]-7'(6'H)-one |
| 300 | 301 | (R)-6'-hydroxy-2',4',6'-trimethyl-7'-oxo-6',7'-dihydrospiro[cyclopropane-1,5'-inden]-3'-yl 6-oxo-6-phenylhexanoate |
| 301 | 302 | ((2'S3'R,6'R)-3',6'-dihydroxy-2',4',6'-trimethyl-7'-oxo-2',3',6',7'-tetrahydrospiro[cyclopropane-1,5'-inden]-2'-yl)methyl 3,5-dinitrobenzoate |
| 302 | 303 | (2'S,3'R,6'R)-2'-(((3,5-dinitrocyclohexa-2,4-diene-1-carbonyl)oxy)methyl)-6'-hydroxy-2',4',6'-trimethyl-7'-oxo-2',3',6',7'-tetrahydrospiro[cyclopropane-1,5'-inden]-3'-yl 3,5-dinitrobenzoate |
| 303 | 304 | (R)-3-(6'-hydroxy-2',4',6'-trimethyl-7'-oxo-6',7'-dihydrospiro[cyclopropane-1,5'-inden]-3'-yl)propyl propiolate |
| 304 | 305 | (R)-3-(6'-hydroxy-2',4',6'-trimethyl-7'-oxo-6',7'-dihydrospiro[cyclopropane-1,5'-inden]-3'-yl)propyl (4-nitrophenyl) carbonate |
| 305 | 306 | (R)-3-(6'-hydroxy-2',4',6'-trimethyl-7'-oxo-6',7'-dihydrospiro[cyclopropane-1,5'-inden]-3'-yl)propyl 4-methylbenzenesulfonate |
| 306 | 307 | (3'R,6'R)-3'-azido-6'-hydroxy-2',2',4',6'-tetramethyl-2',3'-dihydrospiro[cyclopropane-1,5'-inden]-7'(6'H)-one |
| 307 | 308 | (3'R,6'R)-3'-amino-6'-hydroxy-2',2',4',6'-tetramethyl-2',3'-dihydrospiro[cyclopropane-1,5'-inden]-7'(6'H)-one |
| 308 | 309 | (2'R,3'S,6'R)-3'-azido-2'-(((tert-butyldimethylsilyl)oxy)methyl)-6'-hydroxy-2',4',6'-trimethyl-2',3'-dihydrospiro[cyclopropane-1,5'-inden]-7'(6'H)-one |
| 309 | 310 | (R)-(6'-hydroxy-2',4',6'-trimethyl-7'-oxo-6',7'-dihydrospiro[cyclopropane-1,5'-inden]-3'-yl)methyl 4-sulfamoylbenzoate |
| 310 | 311 | (3'S,6'R)-6'-hydroxy-2',2',4',6'-tetramethyl-7'-oxo-2',3',6',7'-tetrahydrospiro[cyclopropane-1, 5'-inden]-3'-yl 4-sulfamoylbenzoate |
| 311 | 312 | (R)-(6'-hydroxy-2',4',6'-trimethyl-7'-oxo-6',7'-dihydrospiro[cyclopropane-1,5'-inden]-3'-yl)methyl 4-methylbenzenesulfonate |
| 312 | 313 | 2,3,4,5,6-pentafluoro-N-((3'R,6'R)-6'-hydroxy-2',2',4',6'-tetramethyl-7'-oxo-2',3',6',7'-tetrahydrospiro[cyclopropane-1,5'-inden]-3'-yl)benzenesulfonamide |
| 313 | 314 | (R)-3-(6'-hydroxy-2',4',6'-trimethyl-7'-oxo-6',7'-dihydrospiro[cyclopropane-1,5'-inden]-3'-yl)propyl 4-methylbenzenesulfonate |
| 314 | 315 | (R)-3'-((2,5-dimethyl-1H-pyrrol-3-yl)methyl)-6'-hydroxy-2',4',6'-trimethylspiro[cyclopropane-1,5'-inden]-7'(6'H)-one |
| 315 | 316 | ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-(fl uorosulfony l)benzoate |
| 316 | 317 | (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl 4-(fluorosulfonyl)benzoate |
| 317 | 318 | ((2-S,3'R,6'R)-3'-((4-(fluorosulfonyl)benzoyl)oxy)-6'-hydroxy-2',4',6'-trimethyl-7'-oxo-2',3',6',7'-tetrahydrospiro[cyclopropane-1,5'-inden]-2'-yl)methyl4-(fluorosulfonyl)benzoate |
| 318 | 332 | (R)-6'-hydroxy-3'-((methoxyamino)methyl)-2',4',6'-trimethylspiro[cyclopropane-1,5'-inden]-7'(6'H)-one |
| 319 | 333 | ((2'S,3'R,6'R)-6'-hydroxy-2',4',6'-trimethyl-3'-(((4-nitrophenoxy)carbonyl)oxy)-7'-oxo-2',3',6',7'-tetrahydrospiro[cyclopropane-1,5'-inden]-2'-yl)methyl acetate |
| 320 | 334 | ((2'S,3'R,6'R)-3'-(((2-((tert-butoxycarbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5' - inden]- 2' - yl)methylacetate |
| 321 | 335 | tert-butyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl)ethane-1,2-diylbis(methylcarbamate) |
| 322 | 337 | 4-nitrophenyl (R)-((6'-hydroxy-2',4',6'-trimethyl-7'-oxo-6',7'-dihydrospiro[cyclopropane-1,5'-inden]-3'-yl)methyl)carbamate |
| 323 | 338 | 4-nitrophenyl (R)-(3-(6'-hydroxy-2',4',6'-trimethyl-7'-oxo-6',7'-dihydrospiro[cyclopropane-1,5'-inden]-3'-yl)propyl)carbamate |
| 324 | 339 | ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-methylbenzenesulfonate |
| 325 | 340 | (R)-N-((6'-hydroxy-2',4',6'-trimethyl-7'-oxo-6',7'-dihydrospiro[cyclopropane-1,5'-inden]-3'-yl)methy l)methanesulfonamide |
| 326 | 345 | (2-S,3'R,6'R)-2'-(acetoxymethyl)-6'-hydroxy-2',4',6'-trimethyl-7'-oxo-2',3 ',6', 7'-tetrahydrospiro[cyclopropane-1,5'-inden]-3 '-yl 4-(N-(2-(2-(2-(2-aminoacetamido)acetamido)acetamido)ethyl)-N-methy lsulfamoyl) benzoate |
| 327 | 346 | ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-(N-(2-(2-(2-(2-aminoacetamido)acetamido)acetamido)ethyl)-N-methylsulfamoyl)benzoate |
| 328 | 347 | ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-sulfamoylbenzoate |
| 330 | 348 | (3'S,6'R-6'-hydroxy-2',2',4',6'-tetramethyl-7'-oxo-2',3',6',7'-tetrahydrospiro[cyclopropane-1, 5'-inden]-3'-yl 4-sulfamoy lbenzoate |
| 331 | 351 | (3'S,6'R)-6'-hydroxy-2',2',4',6'-tetramethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate |
| 332 | 353 | ((6'R)-3'-(((2-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)(methyl)amino)ethyl)(met hyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate |
| 333 | 354 | 4-((S)-2-((S)-2-(6-(2,5-dioxo-2,5-dihydro-1H-pyrrol-1-yl)hexanamido)-3-methylbutanamido)-5-ureidopentanamido)benzyl (2-(3-((S)-6'-hydroxy-2',4',6'-trimethyl-7'-oxo-6',7'-dihydrospiro[cyclopropane-1,5'-inden]-3'-yl)propylidene)hydrazine-1-carbonyl)carbamate |
| 334 | 356 | ((2'S,3'S,6'R)-3'-(((1-(9H-fluoren-9-yl)-13-methyl-3,6,9,12-tetraoxo-2-oxa-4,7,10,13-tetraazapentadecan-15-yl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate |
| 335 | 357 | ((2'S,3'S,6'R)-3'-(((2-(2-(2-(2-aminoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate |
| 336 | 359 | ((2'S,3'S,6'R)-6'-hydroxy-2',4',6'-trimethyl-3'-((methyl(2,2,14-trimethyl-4,7,10,13-tetraoxo-3-oxa-5,8,11,14-tetraazahexadecan-16-yl)carbamoyl)oxy)-7'-oxo-2',3',6',7'-tetrahydrospiro[cyclopropane-1,5' - inden]- 2' - y l)methy lacetate |
| 337 | 361 | ((2'S,3'R,6'R)-3'-((((2,5-dioxopyrrolidin-1-yl)oxy)carbonyl)oxy)-6'-hydroxy-2',4',6'-trimethyl-7'-oxo-2',3',6',7'-tetrahydrospiro[cyclopropane-1,5' - inden]- 2' - yl)methylacetate |
| 338 | 362 | ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl methanesulfonate |
| 339 | 363 | (2'S,3'R,6'R)-2'-(aminomethyl)-3',6'-dihydroxy-2',4',6'-trimethyl-2',3'-dihydrospiro[cyclopropane-1,5'-inden]-7'(6'H)-one |
| 340 | 364 | (2'R,3'R,6'R)-3'-amino-6'-hydroxy-2'-(hydroxymethyl)-2',4',6'-trimethyl-2',3'-dihydrospiro[cyclopropane-1,5'-inden]-7'(6'H)-one |
| 341 | 366 | tert-butyl (R)-((6'-hydroxy-2',4',6'-trimethyl-7'-oxo-6',7'-dihydrospiro[cyclopropane-1,5'-inden]-3'-yl)methyl)carbamate |
| 342 | 367 | tert-butyl (R)-(3-(6'-hydroxy-2',4',6'-trimethyl-7'-oxo-6',7'-dihydrospiro[cyclopropane-1,5'-inden]-3'-yl)propyl)carbamate |
| 343 | 368 | ((2'S,3'R,6'R)-3'-acetoxy-6'-hydroxy-2',4',6'-trimethyl-7'-oxo-2',3',6',7'-tetrahydrospiro[cyclopropane-1,5'-inden]-2'-yl)methyl acetate |
| 344 | 369 | (2'S,3'R,6'R)-2'-(((tert-butyldimethylsilyl)oxy)methyl)-3',6'-dihydroxy-2',4',6'-trimethyl-2',3'-dihydrospiro[cyclopropane-1,5'-inden]-7'(6'H)-one |
| 345 | 370 | (2'S,3'R,6'R)-2'-(((tert-butyldimethylsilyl)oxy)methyl)-6'-hydroxy-2',4',6'-trimethyl-7'-oxo-2',3',6',7'-tetrahydrospiro[cyclopropane-1,5'-inden]-3'-yl acetate |
| 346 | 371 | (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl acetate |
| 347 | 372 | (2'R,3'R,6'R)-2'-formyl-6'-hydroxy-2',4',6'-trimethyl-7'-oxo-2',3',6',7'-tetrahydrospiro[cyclopropane-1,5'-inden]-3'-yl acetate |
| 348 | 373 | (2'S,3'R,6'R)-2'-(((((9H-fluoren-9-yl)methoxy)carbonyl)amino)methyl)-6'-hydroxy-2',4',6'-trimethyl-7'-oxo-2',3',6',7'-tetrahydrospiro[cyclopropane-1,5'-inden]-3'-yl acetate |
| 349 | 374 | (2'S,3'R,6'R)-2'-(aminomethyl)-6'-hydroxy-2',4',6'-trimethyl-7'-oxo-2',3',6',7'-tetrahydrospiro[cyclopropane-1,5'-inden]-3'-yl acetate |
| 350 | 377 | ((2'S,3'R,6'R)-6'-hydroxy-2',4',6'⁻trimethyl-7'-oxo-3'-((tributylsilyl)oxy)-2',3',6',7'-tetrahydrospiro[cyclopropane-1,5'-inden]-2'-yl)methyl acetate |
| 351 | 378 | (2'S,3'R,6'R)-2'-(((tert-butoxycarbonyl)amino)methyl)-6'-hydroxy-2',4',6'-trimethyl-7'-oxo-2',3',6',7'-tetrahydrospiro[cyclopropane-1,5'-inden]-3'-yl acetate |
| 352 | 379 | ((2'S,3'R,6'R)-3'-(((2-((((4-((14S,17S)-1-(9H-fluoren-9-yl)-14-isopropyl-3,6,9,12, 15 -pentaoxo-17-(3 -ureidopropyl)-2-oxa-4,7, 10, 13, 16-pentaazaoctadecan-18-amido)benzyl)oxy)carbonyl)(methyl)amino)ethyl)(methyl)carbamo yl)oxy)-6'-hydroxy-2',4',6'-trimethyl-7'-oxo-2',3',6',7'-tetrahydrospiro[cyclopropane-1,5' - inden]- 2' - yl)methylacetate |
| 353 | 380 | ((2'S,3'R,6'R)-3 '-(((2-((((4-((2S, 5S)-14-amino-5-isopropyl-4,7,10,13-tetraoxo-2-(3-ureidopropyl)-3,6,9,12-tetraazatetradecanamido)benzyl)oxy)carbonyl)(methyl)amino)ethyl )(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate |
| 354 | 381 | (2⁻S,3'R,6'R)-6'-hydroxy-2'-(hydroxymethyl)-2',4',6'-trimethyl-3'-((tributylsilyl)oxy)-2',3'-dihydrospiro[cyclopropane-1,5'-inden]-7'(6'H)-one |
| 355 | 382 | (2'R,3'R,6'R)-6'-hydroxy-2',4',6'-trimethyl-7'-oxo-3'-((tributylsilyl)oxy)-2',3',6',7'-tetrahydrospiro[cyclopropane-1,5'-indene]-2'-carbaldehyde |
| 356 | 383 | (2'R,3'R,6'R)-3',6'-dihydroxy-2',4',6'-trimethyl-7'-oxo-2',3',6',7'-tetrahydrospiro[cyclopropane-1,5'-indene]-2'-carbaldehyde |
| 357 | 384 | tert-butyl (((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl)carbamate |
| 358 | 389 | tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)carbamate |
| 359 | 392 | ((2'S,3'S,6'R)-3-(((2-(2-(2-(2-azidoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate |
| 360 | 393 | ((2'S,3'R,6'R)-3'-(((2-(2,5-dioxo-2,5-dihydro-1H-pyrrol-1-yl)ethyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate |
| 361 | 394 | (R)-6'-hydroxy-3'-((hydroxyamino)methyl)-2',4',6'-trimethylspiro[cyclopropane-1,5'-inden]-7'(6'H)-one |
| 362 | 397 | tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)(hydroxy)carbamate |
| 363 | 398 | (2'S,3'R,6'R)-3',6'-dihydroxy-2'-((hydroxyamino)methyl)-2',4',6'-trimethyl-2',3'-dihydrospiro[cyclopropane-1,5'-inden]-7'(6'H)-one |
| 364 | 399 | ((2'S,3'R,6'R)-3',6'-dihydroxy-2',4',6'-trimethyl-7'-oxo-2',3',6',7'-tetrahydrospiro[cyclopropane-1,5' - inden]- 2' - y l)methy lsulfamate |
| 365 | 401 | ((6'R)-3'-(((2-((((4-((S)-2-((S)-2-((((9H-fluoren-9-yl)methoxy)carbonyl)amino)-3-methylbutanamido)-5-ureidopentanamido)benzyl)oxy)carbonyl)(methyl)amino)ethyl)(met hyl)carbamoyl)oxy)-6'-hydroxy-2',4',6'-trimethyl-7'-oxo-2',3',6',7'-tetrahydrospiro[cyclopropane-1,5' - inden]- 2' - yl)methylacetate |
| 366 | 402 | (R)-(6'-hydroxy-2',4',6'-trimethyl-7'-oxo-6',7'-dihydrospiro[cyclopropane-1,5'-inden]-3'-yl)methyl sulfamate |
| 367 | 403 | ((2'S,6'R)-6'-hydroxy-2',6'-dimethyl-4'-methylene-7'-oxo-2',4',6',7'-tetrahydrospiro[cyclopropane-1,5'-inden]-2'-yl)methyl methanesulfonate |
| 368 | 404 | ((6'R)-3'-(((2-((((4-((S)-2-((S)-2-amino-3-methylbutanamido)-5-ureidopentanamido)benzyl)oxy)carbonyl)(methyl)amino)ethyl)(met hyl)carbamoyl)oxy)-6'-hydroxy-2',4',6'-trimethyl-7'-oxo-2',3',6',7'-tetrahydrospiro[cyclopropane-1,5' - inden]- 2' - yl)methylacetate |
| 369 | 405 | ((2'S,3'S,6'R)-6'-hydroxy-3'-(((2-((((4-((2S,5S)-5-isopropyl-25-(6-methyl-4-vinylpyridin-2-yl)-4,7,23-trioxo-2-(3-ureidopropyl)-10,13,16,19-tetraoxa-3,6,22-triazapentacosanamido)benzyl)oxy)carbonyl)(methyl)amino)ethyl)( methyl)carbamoyl)oxy)-2',4',6'-trimethyl-7'-oxo-2',3',6',7'-tetrahydrospiro[cyclopropane-1,5' - inden]- 2' - yl)methylacetate |
| 370 | 407 | ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl trifluoromethanesulfonate |
| 371 | 408 | 4-((2S,5S)-14-amino-5-isopropyl-4,7,10,13-tetraoxo-2-(3-ureidopropyl)-3,6,9,12-tetraazatetradecanamido)benzyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate) |
| 372 | 409 | 4-((S)-2-((S)-2-amino-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl)ethane-1,2-diylbis(methylcarbamate) |
| 373 | 410 | 4-((S)-2-((S)-2-(6-(2,5-dioxo-2,5-dihydro-1H-pyrrol-1-yl)hexanamido)-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate) |

**Table VI. Summary NCI DTP 60 Cell Line Data.**

| NAME/NSC | Mean GI50 inhibition | Mean TGI cytostatic | Mean LD50 cytotoxic |
|---|---|---|---|
| Pyrrolobenzodiazepines 694501 | 7 nM | 302 nM | > 23,000 nM^{∗} |
| Maytansine^{∗∗} 153858 | 19 nM | 318 nM | 49,200 nM |
| Fumagillol 642492 | 6,130 nM | 9,850 nM | > 50,000 nM |
| Dolstatin-10 376128 | 17 nM | 2,680 nM | > 50,000 nM |
| Auristatins 654663 | 1.4 nM | 902^{∗∗} nM | > 5,000 nM^{∗∗} |
| Enadiyne 157365 | 2,900 nM | > 100,000 nM | > 100,000 nM |
| Halichondrin B 609395 | 1.2 nM | 199 nM | > 1,000 nM |
| Tubulysin A | 12 nM | 1,318 nM | > 10,000 nM |
| | | | |
| Illudin S | 10 nM | 64 nM | 511 nM |
| Illudin M | 3 nM | 20 nM | 291 nM |

**Table VII. Ability of Illudofulvene analogs to inhibit tumor cell growth.**

| Analog Number | Mean IC50 value (nM) ± SD, N=3 unless otherwise indicated | | | |
|---|---|---|---|---|
| | MV522 Target Cell Line | | 8392B Nontarget Cell Line | |
| | 2 hr exposure | 48 hr exposure | 2 hr exposure | 48 hr exposure |
| 001 | 2200 ± 100 | 350± 20 | | 830± 100 |
| 002 | 110 ± 40 | 70±10 | 26000±4500 | 800±100 |
| 004 | 4200 | 600 | | |
| 008 | 870 ± 90 | 630 ± 80 | 12200 ± 700 | 15100 ± 2200 |
| 009 | 500 ± 30 | 850 ± 180 | 47100 ± 11000 | 43200 ± 2300 |
| 010 | 8900 ± 1500 | 170 ± 60 | 29400 ± 1600 | 14500 ± 1700 |
| 011 | 4900 ± 900 | 1200 (N=2) | > 100000 | 40400 ± 6700 |
| 012 | 5150 ± 1350 | 320 ± 90 | 42200 ± 5000 | 18800 ± 2800 |
| 013 | 5100 ± 700 | 270 ± 130 | 11900 ± 1300 | 4200 ± 400 |
| 014 | 115 ± 30 | 460 ± 120 | 9650 ± 200 | 1100 ± 300 |
| 015 | 1800 ± 200 | 480 ± 110 | 810 ± 260 | 1300 ± 150 |
| 016 | 490 ± 130 | 440 ± 90 | > 100000 | 870 ± 60 |
| 017 | 2400 ± 360 | 320 ± 60 | 14700 ± 900 | |
| 018 | 8800 ± 2900 | | 4200± 1300 | |
| 019 | 470 ± 60 | 660 ± 80 | > 75000 | |
| 020 | 530 ± 140 | 230 ± 10 | 25000 ± 3100 | |
| 021 | 2400± 1000 | 930 ± 250 | 34400 ± 9400 | |
| 022 | 700 ± 200 | 680 ± 180 | 31700 ± 1400 | |
| 023 | 2900 ± 1140 | 2750 ± 500 | > 138000 | |
| 024 | 1800 ± 200 | 1200 ± 300 | 12800 ± 2100 | |
| 025 | 1300 ± 310 | 1200 ± 100 | > 25000 | |
| 030 | | > 3000 | | |
| 031 | | > 3000 | | |
| 032 | 600 ± 190 | 210 ± 30 | > 30000 | |
| 033 | 10000 ± 1100 | 4600 ± 200 | 29900 ± 3300 | |
| 034 | 1400 ± 170 | 490 ± 40 | > 100000 | 4400 ± 200 |
| 035 | 5600 ± 600 | | > 150000 | |
| 037 | 26000 ± 5000 | 29200 ± 2300 | > 85000 | |
| 038 = 091 | 750 ± 60 | | 24900 ± 8000 | |
| 039 = 092 | 1500 ± 240 | 600 ± 40 | 24600 ± 2400 | 820 ± 250 |
| 040 = 093 | 3400 ± 360 | 700 ± 90 | 24000 ± 3300 | 5200 ± 470 |
| 060 | 19400 ± 1800 | | 27600 ± 3000 | |
| 062 | 2600 ± 300 | 660 ± 200 | 37100 ± 2300 | |
| 063 | 43000 ± 5700 | 580 ± 250 | | |
| 064 | 28000 ± 4600 | 1200 ± 300 | | |
| 065 | 6200 ± 1100 | 2500 ± 1200 | | |
| 075 | 19600 ± 9700 | | 62000 ± 3600 | |
| 076 | 24000 ± 6100 | | 39500 ± 7200 | |
| 077 | 9200±1200 | | | |
| 078 | 20400 ± 6300 | | > 100000 | |
| 079 | 7700 ± 3500 | | > 100000 | |
| 080 | 8800 ± 2400 | | > 100000 | |
| 081 | > 80000 | | > 80000 | |
| 082 | 50600 ± 7100 | | > 100000 | |
| 083 | | 37200 ± 2900 | | > 42000 |
| 084 | | 28200 ± 1400 | | > 42000 |
| 085 | > 40000 | > 40000 | | |
| 087 | > 40000 | 24700 ± 3900 | > 40000 | |
| 089 | 19300 ± 5700 | 15500 ± 2800 | > 60000 | |
| 090 | 2500 ± 400 | 2900 ± 400 | 1600 ± 200 | 3800 ± 300 |
| 094 | 800 ± 100 | 210 ± 20 | 9000 ± 1700 | 110 ± 10 |
| 096 | 2700 ± 400 | 6200 ± 600 | > 88000 | > 3000 |
| 097 | 2900 ± 100 | | > 82000 | |
| 098 | 18800 ± 2500 | 4600 ± 250 | > 65000 | 11700 ± 1800 |
| 099 | 8400 ± 1100 | 1800 ± 200 | 4000 ± 400 | 300 ± 20 |
| 100 | > 10000 | 1700 ± 500 | | |
| 101 | > 8000 | > 7500 | | |
| 102 | > 13000 | 1300± 100 | | |
| 103 | 31800 ±4900 | 5900 ± 400 | 12100 ± 2000 | 2300 ± 200 |
| 104 | 6300 ± 400 | 6000 ± 500 | 36400 ± 6500 | 2700 ± 600 |
| 105 | 7300 ± 1200 | 2100 ± 400 | > 100000 | |
| 106 | 5200 ± 1000 | | > 83000 | |
| 107 | > 50000 | 1600 ± 100 | > 50000 | |
| 108 | 12300 ± 2300 | 520 ± 50 | > 55000 | 6000 ± 1600 |
| 109 | > 50000 | | > 50000 | |
| 110 | > 55000 | 1400 ± 100 | > 55000 | 25300 ±2100 |
| 111 | 16700 ± 2100 | 11900 ± 2800 | 34600 ± 2100 | 10200 ± 1000 |
| 112 | 10000 ± 2000 | 6700 ± 1200 | 14900 ± 100 | 5200 ± 300 |
| 113 | 85000 ± 700 | 14100 ± 3000 | > 93000 | 7800± 1000 |
| 114 | 1500 ± 100 | 260 ± 70 | 25100 ± 1000 | 700 ± 100 |
| 115 | 1500 ± 100 | 70 ± 5 | 1600 ± 700 | 630 ± 60 |
| 116 | 400 ± 100 | 1000 ± 50 | 7000 ± 400 | 170 ± 30 |
| 117 | 1100 ± 100 | 100 ± 30 | 7900 ± 1600 | 10 ± 2 |
| 118 | 14000 ± 2000 | 740 ± 120 | 24500 ± 4500 | 2000 ± 400 |
| 119 | 1100 ± 70 | 270 ± 40 | > 33000 | > 10000 |
| 120 | 2800 ± 900 | 600 ± 100 | 19100 ± 4600 | 510 ± 110 |
| 121 | 300 ± 10 | 90 ± 10 | 15200 ± 6000 | 1300 ± 500 |
| 122 | 6400 ± 300 | 2400 ± 300 | 14500 ± 1200 | 1100 ± 300 |
| 123 | 1900 ± 400 | 600 ± 60 | 450 ± 30 | 2400 ± 500 |
| 124 | 2800 ± 700 | 870 ± 350 | > 30000 | 2400 ± 550 |
| 125 | 3700 ± 600 | 1200 ± 200 | 15500 ± 1400 | 600 ± 100 |
| 126 | 2100 ± 500 | 900 ± 100 | > 30000 | 330 ± 80 |
| 127 | 870 ± 30 | 340 ± 90 | > 30000 | 100 ± 40 |
| 128 | 840 ± 230 | 370 ± 50 | > 35000 | 800 ± 70 |
| 129 | > 136000 | 19700 ± 1900 | > 136000 | 39400 ± 9200 |
| 130 | 700 ± 100 | 130 ± 40 | 27,000 ± 7000 | 4400 ± 500 |
| 133 | 58800 ± 6600 | 15800 ± 2600 | 12200 ± 2300 | 2700 ± 400 |
| 134 | 50000 ± 6000 | 28000 ± 4000 | 43900 ± 5100 | 8500 ± 2000 |
| 135 | 1600 ± 300 | 22 ± 4 | 70 ± 20 | 22 ± 2 |
| 136 | 430 ± 10 | 130 ± 10 | > 6200 | 25 ± 2 |
| 137 | 850 ± 110 | 1200 ± 100 | 8500 ± 1200 | 710 ± 60 |
| 138 | 2100 ± 200 | 1000 ± 200 | 5400 ± 200 | 820 ± 230 |
| 139 | 6400 ± 900 | 3400 ± 500 | 11600 ± 900 | 2600 ± 1000 |
| 140 | 17100 ± 5100 | > 14000 | 12700 ± 300 | > 14000 |
| 141 | 11400 ± 1000 | 3700 ± 800 | 13700 ± 1900 | 1100 ± 140 |
| 142 | 90 ± 10 | 24 ± 7 | 6400 ± 1100 | 80 ± 6 |
| 143 | 43500 ± 11300 | 11400 ± 1800 | 56500 ± 20000 | 3600 ± 700 |
| 146 | 2500±400 | 740±280 | 13,000±1200 | |
| 147 | > 76000 | 26100 ± 12900 | > 76000 | 43800 ±3000 |
| 148 | 17100 ± 1100 | 6800 ± 1100 | 61000 ± 11600 | 6700± 1600 |
| 149 | 2900 ± 1000 | 1500 500 | 44600 ± 1400 | 4100 ± 900 |
| 150 | 9500 ± 1600 | 1400 ± 400 | 59000 ± 5500 | 10600 ± 800 |
| 151 | 7900 ± 400 | 4200 ± 1600 | 25500 ± 1200 | 6600 ± 2300 |
| 152 | | 6400 ± 1200 | 49000 ± 7700 | 9100 ± 100 |
| 153 | 8700 ±2700 | 10900 ± 3400 | > 90000 | 15800 ± 9600 |
| 154 | > 70000 | 61300 ± 10000 | > 70000 | 46,700 ± 13100 |
| 155 | 8200±1200 | 3600±400 | 17,000±4000 | 9100 ± 1100 |
| 156 | 7200±500 | 3100±100 | 32,300±9,400 | 5500 ± 1200 |
| 157 | > 400,000 | > 123,000 | > 350,000 | 13100± 1600 |
| 158 | > 175,000 | >175,000 | > 200,000 | 61,000 ± 9,000 |
| 159 | 2700±400 | 120±10 | 13,700±4,200 | < 10 nM |
| 160 | 1900±200 | 500±200 | 52,400 ± 17,800 | 3200± 1100 |
| 161 | 2800±500 | 3300±700 | 13,800 ± 3,400 | > 10,000 |
| 163 | 3500±800 | 820±40 | 18600±800 | 910±100 |
| 164 | | 70±10 | 3500±1600 | 130±40 |
| 165 | 7700±1100 | 290±40 | 11000±3300 | 11000±1000 |
| 166 | 6500±600 | 7200±1900 | 6500±2100 | 6000±1500 |
| 167 | 14800±2200 | | 18500±2300 | |
| 169 | 7100±600 | | 2300±600 | |
| 177 | 7500±800 | 1900 ± 800 | 73000±5000 | 4100±1300 |
| 178 | 21000±4000 | 1000 ± 100 | 32000±9000 | > 8000 |
| 180 | 19900±300 | > 4000 | 5200±1800 | 660 ± 50 |
| 182 | 99000±12000 | 38000±8200 | 39000±7000 | 18700±2700 |
| 183 | >120,000 | > 275,000 | >120,000 | > 235,000 |
| 184 | 800 ± 300 | 210±20 | > 100,000 | > 10000 |
| 185 | 1700 ± 600 | 1900±100 | | |
| 186 | 144000±32000 | 70000±16000 | 79000±24000 | 48000±2000 |
| 187 | 1300 ± 400 | 900 ± 200 | 3200 ± 800 | 3200 ± 700 |
| 189 | 8900 ±2500 | 6100± 2600 | 41,000±3700 | |
| 190 | 19,000±4000 | >9,000 | 56,000±2000 | >9,000 |
| 191 | > 140,000 | 49,000 ± 13000 | > 140,000 | 15000±4000 |
| 192 | 1,600±200 | 700±100 | 8700±1700 | 200±30 |
| 193 | 1400±400 | 2500±600 | 48,000±7000 | >11,000 |
| 195 | 1400±200 | 390±120 | 21,000±6000 | 4300±1200 |
| 196 | 840±100 | 450±120 | 80,000±5000 | >9,200 |
| 197 | 950±70 | 500±100 | 9500±400 | 11,300±100 |
| 198 | 700±100 | 2800±600 | >8,200 | >82,000 |
| 199 | 4700±600 | 2500±1100 | >93,000 | >9,300 |
| 201 | 360±110 | 260±70 | 13,000±1700 | 26,000±7000 |
| 202 | 1200± 100 | 650±100 | > 62,000 | > 6200 |
| 203 | 760 ± 170 | 940±330 | 48,000±6000 | > 5500 |
| 204 | 220±40 | 1600±300 | 4100±800 | 8600±800 |
| 205 | 8400±2200 | 1200±400 | > 185,000 | >2,600 |
| 206 | 610±40 | 230±20 | 20,000±1000 | 8200±200 |
| 207 | 570±60 | 410±60 | | |
| 208 | 1200±100 | 930±160 | 25,000±3000 | |
| 209 | 3900±1100 | 610±100 | > 90,000 | |
| 210 | 40,000±4000 | 5500±600 | | |
| 211 | 470±120 | 430±100 | 59,000±9000 | |
| 212 | 80±10 | 55± 5 | | |
| 213 | 2300±700 | 1700±700 | | |
| 214 | 2900±800 | 360±30 | | |
| 215 | 26,000±3000 | 490±120 | | |
| 216 | 460±60 | 150±40 | | |
| 217 | 2,200±100 | 2,200±100 | 43,000±4,000 | >7,000 |
| 218 | 10,000±3,000 | 600±200 | 15,000±6,000 | 600±100 |
| 219 | >52,000 | >52,00 | >52,000 | >52,000 |
| 220 | 90±10 | 130±10 | 101,000±18,000 | 40,000±3,000 |
| 221 | >21,000 | 2,500±200 | >21,000 | >21,000 |
| 222 | 5,000±100 | 1,100±100 | 9,300±200 | 330±60 |
| 223 | 20,000±3,700 | 2,700±300 | >185,000 | >55,000 |
| 224 | >200,000 | >130,000 | >200,000 | >130,000 |
| 225 | 47,000±4,000 | 55,000±11,000 | >350,000 | 33,000±13,000 |
| 226 | >59,000 | >59,000 | >59,000 | >59,000 |
| 227 | >57,000 | 4,400±700 | >57,000 | 16,000±4,000 |
| 228 | >38,000 | >38,000 | 24,000±3,000 | >38,000 |
| 229 | >56,000 | >2,000 | >56,000 | >2,000 |
| 230 | 620±80 | 100±10 | 38,000±5,000 | 1,000±200 |
| 231 | 1,500±100 | 280±10 | 14,000±4,000 | |
| 232 | 700±100 | 460±60 | 42,000±6,000 | 3,300±600 |
| 233 | 3,200±300 | 350±80 | >150,000 | 2,400±700 |
| 234 | 3,000±300 | 1,100±400 | 24,000±6,000 | 9,000±1,000 |
| 235 | 3,500±400 | 2,200±400 | 49,000±6,000 | 6,500±1,600 |
| 236 | 49,000±11,000 | 29,000±5,000 | 48,000±10,000 | |
| 237 | 1,200±300 | 730±140 | 22,000±1,000 | 6,600±900 |
| 238 | 780±190 | 57±8 | 23,000±2,000 | 4,700±1,200 |
| 239 | 420±60 | 70±20 | 39,000±3,000 | 28,000±4,000 |
| 240 | 2,900±100 | 1,300±200 | >24,000 | 1,300±100 |
| 241 | 560±90 | 110±20 | >28,000 | 18,000±4,000 |
| 242 | 2,400±400 | 580±150 | 18,000±2,000 | 2,900±600 |
| 243 | 2,200±500 | 670±240 | 64,000±10,000 | 26,000±6,000 |
| 244 | 1,600±400 | 150±10 | 87,000±11,000 | 35,000±7,000 |
| 245 | 3,400±1000 | 440±90 | 79,000±7,000 | 14,000±1,700 |
| 246 | 2,800±260 | 1,900±450 | 14,000±2,000 | 6,200±1,300 |
| 247 | 6,100±2,000 | 1,200±250 | 10,000±1,400 | 7,100±1,700 |
| 248 | 830±100 | 200±25 | 23,000±1,000 | 610±120 |
| 249 | 4,100±820 | 420±100 | 18,000±3,500 | 19,000±3,800 |
| 250 | 99,000±21,000 | 137,000±14,000 | >275,000 | 137,000±10,000 |
| 251 | 128,000±4,000 | 51,000±1,000 | >275,000 | 82,000±8,000 |
| 252 | >380,000 | 33,000±3,000 | >380,000 | >380,000 |
| 253 | >380,000 | >38,000 | >380,000 | >380,000 |
| 254 | 2,700±800 | 1,100±100 | 43,000±6,000 | >65,000 |
| 255 | 2,900±500 | 55±2 | 119,000±15,000 | 99,000±4,000 |
| 256 | 1,500±200 | 880±200 | 7,500±800 | 7,100±300 |
| 257 | 2,800±600 | 320±30 | 25,000±2,000 | 26,000±3,000 |
| 258 | >45,000 | >45,000 | >45,000 | >45,000 |
| 259 | 16000±3000 | 2400±200 | >85,000 | 4700±400 |
| 260 | 1600±500 | 150±20 | >64,000 | 19000±4500 |
| 261 | 6300±1100 | 1000±150 | 64000±2000 | 38000±2100 |
| 262 | 8700±1300 | 3900±570 | 287000±14000 | 73000±17000 |
| 263 | 2000±300 | 1400±200 | 124000±18000 | 39000±7000 |
| 264 | 1400±100 | 76±17 | >85,000 | 54000±20000 |
| 265 | 810±20 | 8±1 | 1100±200 | 250±80 |
| 266 | 140±20 | 70±18 | 56000±15000 | 32000±7000 |
| 267 | 900±160 | 160±20 | >90,000 | 28000±8000 |
| 268 | 2100±200 | 330±90 | 54,000±16000 | >8,000 |
| 269 | 11000±3000 | 850±320 | 52000±4000 | >7,000 |
| 270 | 8000±1500 | 1300±100 | >84,000 | 7100±700 |
| 271 | 1700±200 | 200±90 | >93,000 | >9,300 |
| 272 | >46,000 | >4,700 | >47,000 | >4,700 |
| 273 | 30000±5000 | >1,500 | >45,000 | >4,500 |
| 274 | 39000±3000 | 1200±300 | >46,000 | >4,500 |
| 275 | 1500±300 | 370±40 | >62,000 | >6,200 |
| 276 | 1500±200 | 760±100 | >61,000 | >6,100 |
| 277 | 760±70 | 190±20 | 31,000±6000 | 9,800±1000 |
| 278 | 1000±100 | 270±10 | >94000 | >9,400 |
| 279 | 1700±400 | 190±20 | >90000 | >9,000 |
| 280 | 2400±800 | <80 | >83000 | >2,800 |
| 281 | 1800±700 | 170±10 | 27000±2000 | 5000±700 |
| 282 | 680±60 | 110±10 | >85000 | >8,500 |
| 283 | 2900±1200 | 300±20 | 40000±4000 | >9,300 |
| 284 | 13,600(N=2) | 340±20 | | >8,800 |
| 285 | 3800±1100 | 310±20 | 84000±9000 | 2000±100 |
| 286 | 48000±10000 | 6300±200 | 51000±1700 | >8,800 |
| 287 | 455000±22000 | 1100±100 | 567000±17000 | 4700±400 |
| 288 | 1800±600 | 150±20 | 11000±3200 | ∼9,000 |
| 289 | 51 ± 4 | 530±150 | >290000 | >8,800 |
| 294 | 960 ±170 | | | |
| 295 | 200 ± 44 | | | |
| 296 | 250 (N=2) | | | |
| 297 | 2200 (N=1) | | | |
| 298 | > 7000 | | | |

**Table VIII. Screening of Analogs from the National Cancer Institute (NCI) Developmental Therapeutics Program (DTP) NCI 60 cell line screen assay.**

| Analog | Activity Value^{∗} | Cytotoxicity | MDR Activity |
|---|---|---|---|
| Epothilone A | +31 | No | No |
| MMAE | +21 | No | No |
| DM1 | +8 | No | No |
| 002 | -15 | Yes | Yes |
| 142 | -5 | Yes | Yes |
| 159 | -25 | Yes | Yes |
| 176 | -11 | Yes | Yes |
| 334 | -11 | Yes | Yes |
| 362 | -9 | Yes | Yes |
| 371 | -18 | Yes | Yes |
| 383 | -1 | Yes | Yes |
| 394 | -2 | Yes | Yes |

| | | | |
|---|---|---|---|
| ^{∗} Activity Value = the lower or more negative the activity value is an indication of the more active the compound was against tumor cells in the 60 cell line panel. A positive value indicates tumor cells could grow but at a slower rate. A value of Zero (0) indicates that while no cell growth was detected there was no evidence of cell death occurring (i.e., when the drug is removed the cells could grow again). A negative value indicates that the compounds actually lyse tumor cells. | | | |

## Claims

1. A compound, (a) said compound comprising an illudofulvene moiety, or (b) said compound being an illudofulvene analog, wherein the illudofulvene moiety or the illudofulvene analog is selected from the group consisting of analog 317 (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl 4-(fluorosulfonyl)benzoate; analog 318 ((2'S,3'R,6'R)-3'-((4-(fluorosulfonyl)benzoyl)oxy)-6'-hydroxy-2',4',6'-trimethyl-7'-oxo-2',3',6',7'-tetrahydrospiro[cyclopropane-1,5'-inden]-2'-yl)methyl 4-(fluorosulfonyl)benzoate; analog 333 ((2'S,3'R,6'R)-6'-hydroxy-2',4',6'-trimethyl-3'-(((4-nitrophenoxy)carbonyl)oxy)-7'-oxo-2',3',6',7'-tetrahydrospiro[cyclopropane-1,5'-inden]-2'-yl)methyl acetate; analog 334 ((2'S,3'R,6'R)-3'-(((2-((tert-butoxycarbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 335 tert-butyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate); analog 339 ((2'S,3'R,6'R)-3 ',6'-dihydroxy-2',4',6'-trimethyl-7'-oxo-2',3',6',7'-tetrahydrospiro[cyclopropane-1,5'-inden]-2'-yl)methyl 4-methylbenzenesulfonate; analog 345 (2'S,3'R,6'R)-2'-(acetoxymethyl)-6'-hydroxy-2',4',6'-trimethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate; analog 346 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl 4-(N-(2-(2-(2-(2-aminoacetamido)acetamido)acetamido)ethyl)-N-methylsulfamoyl)benzoate; analog 347 ((2'S,3'R,6'R)-3 ',6'-dihydroxy-2',4',6'-trimethyl-7'-oxo-2',3',6',7'-tetrahydrospiro[cyclopropane-1,5'-inden]-2'-yl)methyl 4-sulfamoylbenzoate; analog 348 (3'S,6'R)-6'-hydroxy-2',2',4',6'-tetramethyl-7'-oxo-2',3',6',7'-tetrahydrospiro[cyclopropane-1,5'-inden]-3'-yl4-sulfamoylbenzoate; analog 351 (3'S,6'R)-6'-hydroxy-2',2',4',6'-tetramethyl-7'-oxo-2',3',6',7'-tetrahydrospiro[cyclopropane-1,5'-inden]-3'-yl 4-(N-(2-(2-(2-(2-aminoacetamido)acetamido)acetamido)ethyl)-N-methylsulfamoyl)benzoate; analog 353 ((6'R)-3'-(((2-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 356 ((2'S,3'S,6'R)-3'-(((1-(9H-fluoren-9-yl)-13-methyl-3,6,9,12-tetraoxo-2-oxa-4,7,10,13-tetraazapentadecan-15-yl)(methyl)carbamoyl)oxy)-6'-hydroxy-2',4',6'-trimethyl-7'-oxo-2',3',6',7'-tetrahydrospiro[cyclopropane-l,5'-inden]-2'-yl)methyl acetate; analog 357 ((2'S,3'S,6'R)-3'-(((2-(2-(2-(2-aminoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 359 ((2'S,3'S,6'R)-6'-hydroxy-2',4',6'-trimethyl-3'-((methyl(2,2,14-trimethyl-4,7,10,13-tetraoxo-3-oxa-5,8,11,14-tetraazahexadecan-16-yl)carbamoyl)oxy)-7'-oxo-2',3',6',7'-tetrahydrospiro[cyclopropane-1,5'-inden]-2'-yl)methyl acetate; analog 361 ((2'S,3'R,6'R)-3'-((((2,5-dioxopyrrolidin-1-yl)oxy)carbonyl)oxy)-6'-hydroxy-2',4',6'-trimethyl-7'-oxo-2',3',6',7'-tetrahydrospiro[cyclopropane-1,5'-inden]-2'-yl)methyl acetate; analog 362 ((2'S,3R,6'R)-3',6'-dihydroxy-2',4',6'-trimethyl-7'-oxo-2',3',6',7'-tetrahydrospiro[cyclopropane-1,5'-inden]-2'-yl)methyl methanesulfonate; analog 363 (2'S,3'R,6'R)-2'-(aminomethyl)-3',6'-dihydroxy-2',4',6'-trimethyl-2',3'-dihydrospiro[cyclopropane-1,5'-inden]-7'(6'H)-one; analog 364 (2'R,3'R,6'R)-3'-amino-6'-hydroxy-2'-(hydroxymethyl)-2',4',6'-trimethyl-2',3'-dihydrospiro[cyclopropane-1,5'-inden]-7'(6'H)-one; analog 368 ((2'S,3'R,6'R)-3'-acetoxy-6'-hydroxy-2',4',6'-trimethyl-7'-oxo-2',3',6',7'-tetrahydrospiro[cyclopropane-1,5'-inden]-2'-yl)methyl acetate; analog 369 (2'S,3'R,6'R)-2'-(((tert-butyldimethylsilyl)oxy)methyl)-3',6'-dihydroxy-2',4',6'-trimethyl-2',3'-dihydrospiro[cyclopropane-1,5'-inden]-7'(6'H)-one; analog 370 (2'S,3'R,6'R)-2'-(((tertbutyldimethylsilyl)oxy)methyl)-6'-hydroxy-2',4',6'-trimethyl-7'-oxo-2',3',6',7'-tetrahydrospiro[cyclopropane-1,5'-inden]-3'-yl acetate; analog 371 (2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl acetate; analog 372 (2'R,3'R,6'R)-2'-formyl-6'-hydroxy-2',4',6'-trimethyl-7'-oxo-2',3',6',7'-tetrahydrospiro[cyclopropane-1,5'-inden]-3'-yl acetate; analog 373 (2'S,3'R,6'R)-2'-(((((9H-fluoren-9-yl)methoxy)carbonyl)amino)methyl)-6'-hydroxy-2',4',6'-trimethyl-7'-oxo-2',3',6',7'-tetrahydrospiro[cyclopropane-1,5'-inden]-3'-yl acetate; analog 374 (2'S,3'R,6'R)-2'-(aminomethyl)-6'-hydroxy-2',4',6'-trimethyl-7'-oxo-2',3',6',7'-tetrahydrospiro[cyclopropane-1,5'-inden]-3'-yl acetate; analog 377 ((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl acetate; analog 378 (2'S,3'R,6R)-2'-(((tert-butoxycarbonyl)amino)methyl)-6'-hydroxy-2',4',6'-trimethyl-7'-oxo-2',3',6',7'-tetrahydrospiro[cyclopropane-1,5'-inden]-3'-yl acetate; analog 379 ((2'S,3'R,6'R)-3'-(((2-((((4-((14S,17S)-1-(9H-fluoren-9-yl)-14-isopropyl-3,6,9,12,15-pentaoxo-17-(3-ureidopropyl)-2-oxa-4,7,10,13,16-pentaazaoctadecan-18-amido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 380((2'S,3'R,6'R)-3'-(((2-((((4-((2S,5S)-14-amino-5-isopropyl-4,7,10,13-tetraoxo-2-(3-ureidopropyl)-3,6,9,12-tetraazatetradecanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 381 (2'S,3'R,6'R)-6'-hydroxy-2'-(hydroxymethyl)-2',4',6'-trimethyl-3'-((tributylsilyl)oxy)-2',3'-dihydrospiro[cyclopropane-1,5'-inden]-7'(6'H)-one; analog 382 (2'R,3'R,6'R)-6'-hydroxy-2',4',6'-trimethyl-7'-oxo-3'-((tributylsilyl)oxy)-2',3',6',7'-tetrahydrospiro[cyclopropane-1,5'-indene]-2'-carbaldehyde; analog 383 (2'R,3'R,6'R)-3',6'-dihydroxy-2',4',6'-trimethyl-7'-oxo-2',3',6',7'-tetrahydrospiro[cyclopropane-1,5'-indene]-2'-carbaldehyde; analog 384 tert-butyl (((2'S,3'R,6'R)-6'-hydroxy-2',4',6'-trimethyl-7'-oxo-3'-((tributylsilyl)oxy)-2',3',6',7'-tetrahydrospiro[cyclopropane-1,5'-inden]-2'-yl)methyl)carbamate; analog 389 tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)carbamate; analog 392 ((2'S,3'S,6'R)-3'-(((2-(2-(2-(2-azidoacetamido)acetamido)-N-methylacetamido)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 393 ((2'S,3'R,6'R)-3'-(((2-(2,5-dioxo-2,5-dihydro-1H-pyrrol-1-yl)ethyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 397 tert-butyl (((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl)(hydroxy)carbamate; analog 398 (2'S,3'R,6'R)-3',6'-dihydroxy-2'-((hydroxyamino)methyl)-2',4',6'-trimethyl-2',3'-dihydrospiro[cyclopropane-1,5'-inden]-7'(6'H)-one; analog 399 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl sulfamate; analog 401 ((6'R)-3'-(((2-((((4-((S)-2-((S)-2-((((9H-fluoren-9-yl)methoxy)carbonyl)amino)-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 403 ((2'S,6'R)-6'-hydroxy-2',6'-dimethyl-4'-methylene-7'-oxo-2',4',6',7'-tetrahydrospiro[cyclopropane-1,5'-inden]-2'-yl)methyl methanesulfonate; analog 404 ((6'R)-3'-(((2-((((4-((S)-2-((S)-2-amino-3-methylbutanamido)-5-ureidopentanamido)benzyl)oxy)carbonyl)(methyl)amino)ethyl)(methyl)carbamoyl)oxy)-6'-hydroxy-2',4',6'-trimethyl-7'-oxo-2',3',6',7'-tetrahydrospiro[cyclopropane-1,5'-inden]-2'-yl)methyl acetate; analog 405 ((2'S,3'S,6'R)-6'-hydroxy-3'-(((2-((((4-((2S,5S)-5-isopropyl-25-(6-methyl-4-vinylpyridin-2-yl)-4,7,23 -trioxo-2-(3-ureidopropyl)-10,13,16,19-tetraoxa-3,6,22-triazapentacosanamido)benzyl)oxy)carbonyl)(methyl)amino)ethyl)(methyl)carbamoyl)oxy)-2',4',6'-trimethyl-7'-oxo-2',3',6',7'-tetrahydrospiro[cyclopropane-1,5'-inden]-2'-yl)methyl acetate; analog 407 ((2'S,3'R,6'R)-3',6'-dihydroxy-2',4',6'-trimethyl-7'-oxo-2',3',6',7'-tetrahydrospiro[cyclopropane-1,5'-inden]-2'-yl)methyl trifluoromethanesulfonate; analog 408 4-((2S,5S)-14-amino-5-isopropyl-4,7,10,13-tetraoxo-2-(3-ureidopropyl)-3,6,9,12-tetraazatetradecanamido)benzyl ((2'S,3'R,6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate); analog 409 4-((S)-2-((S)-2-amino-3-methylbutanamido)-5-ureidopentanamido)benzyl ((6'R)-6'-hydroxy-2'-(hydroxymethyl)-2',4',6'-tnmethyl-7'-oxo-2',3',6',7'-tetrahydrospiro[cyclopropane-1,5'-inden]-3'-yl) ethane-1,2-diylbis(methylcarbamate) and analog 410 4-((S)-2-((S)-2-(6-(2,5-dioxo-2,5-dihydro-1H-pyrrol-1-yl)hexanamido)-3-methylbutanamido)-5-ureidopentanamido)benzyl ((6'R)-6'-hydroxy-2'-(hydroxymethyl)-2',4',6'-trimethyl-7'-oxo-2',3',6',7'-tetrahydrospiro[cyclopropane-1,5'-inden]-3'-yl) ethane-1,2-diylbis(methylcarbamate).

2. The compound of claim 1, wherein the compound comprising an illudofulvene moiety is an affinity medicant conjugate, optionally wherein the affinity medicant conjugate comprises a linker.

3. A composition comprising the compound of claim 1 or 2, and a physiologically compatible excipient.

4. A composition comprising racemic mixtures of any enantiomers of the compound of claims 1 or 2.

5. A composition comprising the compound of claim 1 or 2, in the form of a liposomal particle, a nanoparticle, or a PEGylated compound.

6. A composition comprising the compound of claim 1 or 2, and a physiologically compatible carrier.

7. A compound, (a) said compound comprising an illudofulvene moiety, or (b) said compound being an illudofulvene analog, wherein the illudofulvene moiety or the illudofulvene analog is selected from the group consisting of analog 332 (R)-6'-hydroxy-3'-((methoxyamino)methyl)-2',4',6'-trimethylspiro[cyclopropane-1,5'-inden]-7'(6'H)-one; analog 337 4-nitrophenyl (R)-((6'-hydroxy-2',4',6'-trimethyl-7'-oxo-6',7'-dihydrospiro[cyclopropane-1,5'-inden]-3'-yl)methyl)carbamate; analog 338 4-nitrophenyl (R)-(3-(6'-hydroxy-2',4',6'-trimethyl-7'-oxo-6',7'-dihydrospiro[cyclopropane-1,5'-inden]-3'-yl)propyl)carbamate; analog 354 4-((S)-2-((S)-2-(6-(2,5-dioxo-2, 5-dihydro-1H-pyrrol-1-yl)hexanamido)-3-methylbutanamido)-5-ureidopentanamido)benzyl (2-(3-((S)-6'-hydroxy-2',4',6'-trimethyl-7'-oxo-6',7'-dihydrospiro[cyclopropane-1,5'-inden]-3'-yl)propylidene)hydrazine-1-carbonyl)carbamate; analog 366 tert-butyl (R)-((6'-hydroxy-2',4',6'-trimethyl-7'-oxo-6',7'-dihydrospiro[cyclopropane-1,5'-inden]-3'-yl)methyl)carbamate; analog 367 tert-butyl (R)-(3-(6'-hydroxy-2',4',6'-trimethyl-7'-oxo-6',7'-dihydrospiro[cyclopropane1,5'-inden]-3'-yl)propyl)carbamate; analog 394 (R)-6'-hydroxy-3'-((hydroxyamino)methyl)-2',4',6'-trimethylspiro[cyclopropane-1,5'-inden]-7'(6'H)-one; and analog 402 (R)-(6'-hydroxy-2',4',6'-trimethyl-7'-oxo-6',7'-dihydrospiro[cyclopropane-1,5'-inden]-3'-yl)methyl sulfamate.

8. The compound of claim 7, wherein the compound comprising an illudofulvene moiety is an affinity medicant conjugate, optionally wherein the affinity medicant conjugate comprises a linker.

9. A composition comprising the compound of claim 7 or 8, and a physiologically compatible excipient.

10. A composition comprising racemic mixtures of any enantiomers of the compound of claim 7 or 8.

11. A composition comprising the compound of claim 7 or 8, in the form of a liposomal particle, a nanoparticle, or a PEGylated compound.

12. A composition comprising the compound of claim 7 or 8, and a physiologically compatible carrier.

13. A compound, (a) said compound comprising an illudofulvene moiety, or (b) said compound being an illudofulvene analog for use in a method of treating cancer, wherein the compound is a compound of any of claims 1 or 2, or of any of claims 7 or 8.

14. A composition for use in a method of treating cancer, wherein the composition is a composition of any of claims 3 to 6 or 9 to 12.
